(19) Europäisches Patentamt
European Patent Office
Office européen des brevets

(11) EP 4 450 493 A1

(12) **EUROPEAN PATENT APPLICATION**
published in accordance with Art. 153(4) EPC

(43) Date of publication:
**23.10.2024 Bulletin 2024/43**

(21) Application number: **22906496.9**

(22) Date of filing: **12.12.2022**

(51) International Patent Classification (IPC):
***C07D 401/12*** *(2006.01)* ***A61K 31/506*** *(2006.01)*
***A61P 35/00*** *(2006.01)*

(52) Cooperative Patent Classification (CPC):
**A61K 31/506; A61P 35/00; C07D 401/12;**
Y02P 20/55

(86) International application number:
**PCT/CN2022/138383**

(87) International publication number:
**WO 2023/109751 (22.06.2023 Gazette 2023/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.12.2021 CN 202111522765**

(71) Applicant: **Signet Therapeutics Inc.**
**Shenzhen, Guangdong 518048 (CN)**

(72) Inventors:
- **ZHANG, Haisheng**
  **Shenzhen, Guangdong 518048 (CN)**
- **ZHUO, Shu**
  **Shenzhen, Guangdong 518048 (CN)**
- **HU, Yabing**
  **Shenzhen, Guangdong 518048 (CN)**
- **DAI, Changgui**
  **Shenzhen, Guangdong 518048 (CN)**
- **CHENG, Huimin**
  **Shenzhen, Guangdong 518048 (CN)**
- **NIU, Chunyi**
  **Shenzhen, Guangdong 518048 (CN)**
- **FANG, Lei**
  **Shenzhen, Guangdong 518048 (CN)**
- **CHEN, Yu**
  **Shenzhen, Guangdong 518048 (CN)**

(74) Representative: **Latscha Schöllhorn Partner AG**
**Grellingerstrasse 60**
**4052 Basel (CH)**

(54) **PYRIMIDINE OR PYRIDINE DERIVATIVE AND MEDICINAL USE THEREOF**

(57) A focal adhesion kinase (FAK) inhibitor compound, a composition containing the compound, and a use of the compound. The compound has a good treatment effect on cancer, pulmonary hypertension, and pathological angiogenesis, and has good clinical application prospects.

EP 4 450 493 A1

## Description

### CROSS-REFERENCE TO RELATED APPLICATION

This application claims the priority of the Chinese application No. 202111522765.8 filed on December 13, 2021, which is incorporated herein by reference in its entirety.

### TECHNICAL FIELD

The present application belongs to the field of pharmaceuticals, and specifically relates to a pyrimidine or pyridine derivative and its pharmaceutical use, particularly the use in the preparation of medicament for the treatment or prevention of cancer, pulmonary arterial hypertension, and pathological angiogenesis.

### BACKGROUND

Focal adhesion kinase (FAK), also known as PTK2 (protein tyrosine kinase 2), is a non-receptor tyrosine kinase located at the intersection of multiple signaling pathways, which can be activated by integrins, growth factor receptors, G protein coupled receptors, and cytokines. In addition to serving as a cytoplasmic kinase involved in signal transduction, related studies have also shown that FAK plays an important role in the nucleus. FAK can promote p53 degradation through ubiquitination, leading to cancer cell growth and proliferation. Tang *et al.* reported that FAK can also regulate the expression of GATA4 and IL-33, thereby reducing inflammatory response and immune escape. In the microenvironment of tumors, FAK in the nucleus can regulate the formation of new blood vessels and affect the blood supply of tumors.

FAK is widely expressed *in vivo* and plays an important role in cell growth, proliferation, migration, and adhesion, participating in embryonic development and the occurrence and development of diseases such as cancer and cardiovascular diseases. Overexpression of FAK is found in many kinds of cancers, including colon cancer, breast cancer, prostatic cancer, thyroid cancer, neuroblastoma, ovarian cancer, cervical cancer, brain cancer, head and neck cancer, liver cancer, esophageal cancer, pancreatic cancer, lung cancer, gastric cancer and acute leukemia. High expression of FAK often indicates poor prognosis. For example, studies have found that the $RHOA^{Y42C}$ mutation of GTP enzyme is one of the most common functional acquired mutations in diffuse gastric cancer, and mice having $RHOA^{Y42C}$ mutation are sensitive to FAK inhibitors, indicating that the inhibition of FAK activity may become a new strategy for treating diffuse gastric cancer.

During the process of FAK exerting its function, the binding of transmembrane integrin receptors to the extracellular matrix (ECM) recruits FAK to the site of integrin aggregation. FAK does not directly interact with integrins, but rather binds to cell membranes and other adhesion proteins through its carboxyl terminal FAT domain. Once recruited, non-activated FAK activates its catalytic activity through self-phosphorylation of Y397. After phosphorylation, FAK as a molecular scaffold can recruit Src family kinases. Src can phosphorylate Y576 and Y577 sites of FAK, further enhancing the activity of FAK and simultaneously promoting FAK's recruitment of downstream proteins comprising SH2 domains, such as Grb2 and PI3K. When Grb2 binds to FAK, it can further recruit SOS to form complexes, thereby further activating the downstream Ras-MAPK signaling pathway.

Based on the above, FAK and its signaling pathway related targets are considered as potential targets for the development of anti-cancer drugs. However, there are no drugs commercially available for FAK inhibitors to date, and only some drugs have entered the clinical stage, such as Defactinib, IN10018, GSK-2256098, etc. Therefore, it is crucial to develop new compounds that regulate the FAK signaling pathway.

YAP (Yes-associated protein), as a downstream signaling pathway of FAK, is a Yes-associated protein, which is a transcriptional coactivator of the Hippo pathway, located on human chromosome 11q22 and promotes gene expression by enhancing the activity of transcription factors. MST1/2 is activated by external signals, binds to regulatory protein SAV1 and phosphorylates LATS1/2 and MOB, followed by directly phosphorylating YAP/TAZ. The phosphorylated YAP/TAZ remains in the cytoplasm and transcription is inhibited. When this signaling pathway is blocked or inactivated, unphosphorylated YAP/TAZ is transferred from the cytoplasm to the nucleus, and binds to transcription factors such as TEADs, Smad, Runx1/2, p63/p73, ErbB4 to promote gene transcription.

The Hippo-YAP pathway is a signaling pathway discovered in recent years that regulates organ volume and maintains a balance between cell proliferation and apoptosis, and is closely related to uncontrolled proliferation of tumor cells. The main function of the Hippo-YAP pathway in mammals is to inhibit the activity of transcription regulatory factors YAP and TAZ and negatively regulate tumor progression, thus the Hippo pathway is also considered as an anticancer pathway. Specifically, the core members of this pathway include serine/threonine kinases MST1, MST2, LATS1, and LATS2, scaffold proteins SAV1 (binding to MST1 and MST2), MOB1 (binding to LATS1 and LATS2), transcriptional coactivator YAP, and transcription factor TEAD comprising the TEA binding domain. In short, when the Hippo pathway is activated, MST1/2 kinase phosphorylates LATS1/2 which in turn phosphorylates YAP. The phosphorylated YAP

becomes inactive and transfers out of the nucleus, while YAP in the cytoplasm is degraded by proteasomes.

Research has confirmed that the Hippo pathway is involved in the progression of various tumors such as lung cancer, colon cancer, ovarian cancer, prostatic cancer, liver cancer, etc. However, gene mutations in the Hippo pathway less occur in human tumors. Based on this, it can be concluded that the dysregulation of the Hippo pathway in human tumors is not only caused by mutations in key proteins of the Hippo pathway itself, but also by cross talk between other abnormally expressed proteins or signaling pathways in tumor cells and the Hippo pathway. In addition, the role of the Hippo signaling pathway in tumors is closely related to the nuclear translocation of YAP/TAZ. Recently, high expression of YAP has been found in various tumors, which is related to high pathological grading, advanced TNM stage, lymph node metastasis, and there is a phenomenon of nuclear localization.

Based on this, inhibition of active YAP can block related signaling pathways and potentially inhibit tumors, providing guidance for drug development.

## SUMMARY OF THE INVENTION

The present application provides a compound that is useful as an inhibitor of focal adhesion kinase (FAK), as well as a composition comprising such compounds, and the use of such compounds. The compound of the present application surprisingly has a good effect of inhibition of YAP in addition to a good effect of inhibition of FAK. The compound provided by the present application has good therapeutic effects and good clinical application prospects for cancer, pulmonary arterial hypertension, and pathological angiogenesis.

In one aspect, the present application provides a compound, which is a compound of formula I or a stereoisomer, a tautomer, an enantiomer, a diastereomer, a racemate, a geometric isomer, a nitrogen oxide, a solvate, a hydrate, a crystal form, an ester, an isotope labeled compound (preferably a deuterate), a metabolite, a pharmaceutically acceptable salt or a prodrug thereof:

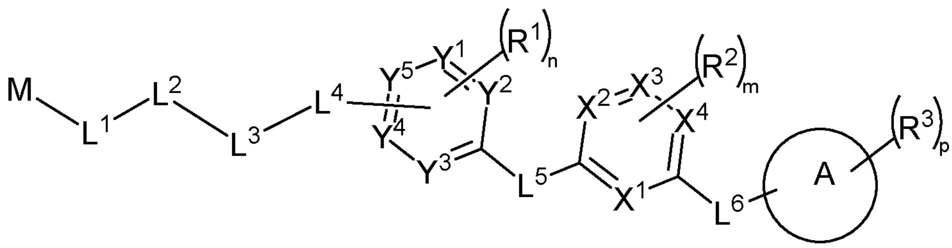

I

wherein,

M is a covalent warhead; A is an aryl or heteroaryl group;

$L^1$ is a bond, -C(=O)-, -S(=O)-, -S(=O)$_2$-, -C(=O)NR$^{L1a}$-, -NR$^{L1a}$C(=O)-, -S(=O)$_2$NR$^{L1a}$ , or -NR$^{L1a}$S(=O)$_2$-; wherein, each $R^{L1a}$ is independently hydrogen, alkyl, alkenyl, alkynyl, heteroalkyl, haloalkyl, haloheteroalkyl, or a nitrogen protecting group; wherein the alkyl, alkenyl, alkynyl, heteroalkyl, haloalkyl, haloheteroalkyl involved in $R^{L1a}$ each optionally are independently substituted by one or more substituents selected from the group consisting of hydrogen (such as deuterium), halogen, hydroxyl, amino, cyano, and oxo;

$L^2$ is a bond, alkyl, heteroalky, saturated or partially unsaturated carbocyclyl, saturated or partially unsaturated heterocyclyl, aryl, or heteroaryl; wherein, the alkyl, heteroalkyl, saturated or partially unsaturated carbocyclyl, saturated or partially unsaturated heterocyclyl, aryl, or heteroaryl involved in $L^2$ each optionally are independently substituted by one or more substituents selected from the group consisting of hydrogen (such as deuterium), hydroxyl, amino, cyano, halogen, oxo, alkyl, alkenyl, alkynyl, heteroalkyl, and haloheteroalkyl;

$L^3$ is a bond, aryl, or heteroaryl; wherein, the aryl, or heteroaryl involved in $L^3$ each optionally are independently substituted by one or more substituents selected from the group consisting of hydrogen (such as deuterium), hydroxyl, amino, cyano, halogen, alkyl, alkenyl, alkynyl, heteroalkyl, haloalkyl, and haloheteroalkyl;

$L^4$ is a carbon chain or heterochain with 2-6 atoms; wherein, the carbon chain or heterochain involved in $L^4$ each optionally are independently substituted by one or more substituents selected from the group consisting of hydrogen (such as deuterium), hydroxyl, amino, cyano, halogen, oxo, alkenyl, alkynyl, alkyl, haloalkyl, hydroxyalkyl, aminoalkyl, alkoxyl, haloalkoxyl, hydroxyalkoxyl, aminoalkoxyl, alkylamino, and a nitrogen protecting group;

$L^5$ is -NR$^{L5a}$-, -O-, -S-, alkyl or heteroalkyl; wherein, the alkyl and heteroalkyl involved in $L^5$ each optionally are independently substituted by one or more substituents selected from the group consisting of hydrogen (such as deuterium), amino, hydroxyl, oxo, cyano, halogen, alkenyl and alkynyl; each $R^{L5a}$ is independently hydrogen, alkyl, alkenyl, alkynyl, heteroalkyl, haloalkyl, or a nitrogen protecting group;

$L^6$ is -NR$^{L6a}$-, -O-, -S-, alkyl, or heteroalkyl; wherein, the alkyl and heteroalkyl involved in $L^6$ each optionally are

independently substituted by one or more substituents selected from the group consisting of hydrogen, amino, hydroxyl, oxo, cyano, halogen, alkenyl or alkynyl; each $R^{L6a}$ is independently hydrogen, alkyl, alkenyl, alkynyl, heteroalkyl, haloalkyl, or a nitrogen protecting group;

$X^1$, $X^2$, $X^3$, $X^4$, $Y^1$, $Y^2$, $Y^3$, $Y^4$, and $Y^5$ each are independently CH or N;

each $R^1$ is independently hydrogen, amino, hydroxyl, cyano, halogen, alkyl, alkenyl, alkynyl, heteroalkyl, haloalkyl, haloheteroalkyl, alkoxyl, haloalkoxyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl; if there are two adjacent $R^1$, the two adjacent $R^1$ and their connected atoms optionally form saturated or partially unsaturated carbocyclyl, saturated or partially unsaturated heterocyclyl, aryl or heteroaryl; wherein, the alkyl, alkenyl, alkynyl, heteroalkyl, haloalkyl, haloheteroalkyl, alkoxyl, haloalkoxyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, heteroarylalkyl involved in $R^1$ each optionally are independently substituted by one or more substituents selected from the group consisting of hydrogen (such as deuterium), hydroxyl, amino, cyano, halogen, oxo, and alkyl;

each $R^2$ is independently hydrogen, amino, hydroxyl, cyano, halogen, alkyl, alkenyl, alkynyl, heteroalkyl, haloalkyl, haloheteroalkyl, alkoxyl, haloalkoxyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl; if there are two adjacent $R^2$, the two adjacent $R^2$ and their connected atoms optionally form saturated or partially unsaturated carbocyclyl, saturated or partially unsaturated heterocyclyl, aryl, or heteroaryl; wherein, the alkyl, alkenyl, alkynyl, heteroalkyl, haloalkyl, haloheteroalkyl, alkoxyl, haloalkoxyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^2$ each optionally are independently substituted by one or more substituents selected from the group consisting of hydroxyl, amino, cyano, halogen, oxo, and alkyl;

each $R^3$ is independently hydrogen, amino, hydroxyl, cyano, halogen, alkyl, alkenyl, alkynyl, heteroalkyl, haloalkyl, haloheteroalkyl, alkoxyl, haloalkoxyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl; if there are two adjacent $R^3$, the two adjacent $R^3$ and their connected atoms optionally form saturated or partially unsaturated carbocyclyl, saturated or partially unsaturated heterocyclyl, aryl or heteroaryl; wherein, the alkyl, alkenyl, alkynyl, heteroalkyl, haloalkyl, haloheteroalkyl, alkoxyl, haloalkoxyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^3$ each optionally are independently substituted by one or more substituents selected from the group consisting of hydroxyl, amino, cyano, halogen, oxo, and alkyl; and

m and p each are independently 0, 1, 2, 3 or 4.

In some examples, the compound provided by the present application is a compound of formula I, or a stereoisomer, a tautomer, a nitrogen oxide, a solvate, a metabolite, a pharmaceutically acceptable salt, or a prodrug thereof:

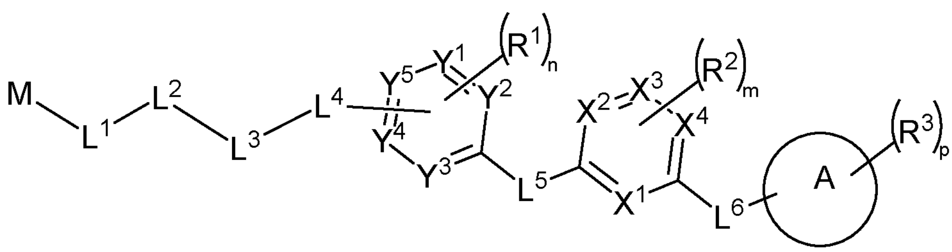

I

wherein,

M is a covalent warhead;

A is aryl or heteroaryl;

$L^1$ is a bond, -C(=O)-, -S(=O)-, -S(=O)z-, -C(=O)$NR^{L1a}$ , -$NR^{L1a}$C(=O)-, -$S(=O)_2NR^{L1a}$, or -$NR^{L1a}S(=O)_2$-; wherein, each $R^{L1a}$ is independently hydrogen, alkyl, alkenyl, alkynyl, heteroalkyl, haloalkyl, haloheteroalkyl, or a nitrogen protecting group; wherein the alkyl, alkenyl, alkynyl, heteroalkyl, haloalkyl, haloheteroalkyl involved in $R^{L1a}$ each optionally are independently substituted by one or more substituents selected from the group consisting of hydrogen (such as deuterium), halogen, hydroxyl, amino, cyano, and oxo;

$L^2$ is a bond, alkyl, heteroalky, saturated or partially unsaturated carbocyclyl, saturated or partially unsaturated

heterocyclyl, aryl or heteroaryl; wherein, the alkyl, heteroalkyl, saturated or partially unsaturated carbocyclyl, saturated or partially unsaturated heterocyclyl, aryl, or heteroaryl involved in $L^2$ each optionally are independently substituted by one or more substituents selected from the group consisting of hydrogen (such as deuterium), hydroxyl, amino, cyano, halogen, oxo, alkyl, alkenyl, alkynyl, heteroalkyl, and haloheteroalkyl;

$L^3$ is a bond, aryl or heteroaryl; wherein, the aryl, or heteroaryl involved in $L^3$ each optionally are independently substituted by one or more substituents selected from the group consisting of hydrogen (such as deuterium), hydroxyl, amino, cyano, halogen, alkyl, alkenyl, alkynyl, heteroalkyl, haloalkyl, and haloheteroalkyl;

$L^4$ is a carbon chain or heterochain with 2-6 atoms; wherein, the carbon chain or heterochain involved in $L^4$ each optionally are independently substituted by one or more substituents selected from the group consisting of hydrogen (such as deuterium), hydroxyl, amino, cyano, halogen, oxo, alkenyl, alkynyl, alkyl, haloalkyl, hydroxyalkyl, aminoalkyl, alkoxyl, haloalkoxyl, hydroxyalkoxyl, aminoalkoxyl, alkylamino, and a nitrogen protecting group;

$L^5$ is $-NR^{L5a}-$, -O-, -S-, alkyl or heteroalkyl; wherein, the alkyl and heteroalkyl involved in $L^5$ each optionally are independently substituted by one or more substituents selected from the group consisting of hydrogen, amino, hydroxyl, oxo, cyano, halogen, alkenyl, and alkynyl; each $R^{L5a}$ is independently hydrogen (such as deuterium), alkyl, alkenyl, alkynyl, heteroalkyl, haloalkyl, or a nitrogen protecting group;

$L^6$ is $-NR^{L6a}-$, -O-, -S-, alkyl, or heteroalkyl; wherein, the alkyl and heteroalkyl involved in $L^6$ each optionally are independently substituted by one or more substituents selected from the group consisting of hydrogen (such as deuterium), amino, hydroxyl, oxo, cyano, halogen, alkenyl, or alkynyl; each $R^{L6a}$ is independently hydrogen, alkyl, alkenyl, alkynyl, heteroalkyl, haloalkyl, or a nitrogen protecting group;

$X^1$, $X^2$, $X^3$, $X^4$, $Y^1$, $Y^2$, $Y^3$, $Y^4$ and $Y^5$ each are independently CH or N;

each $R^1$ is independently hydrogen, amino, hydroxyl, cyano, halogen, alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl; if there are two adjacent $R^1$, the two adjacent $R^1$ and their connected atoms optionally form saturated or partially unsaturated carbocyclyl, saturated or partially unsaturated heterocyclyl, aryl or heteroaryl; wherein, the alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, heteroarylalkyl involved in $R^1$ each optionally are independently substituted by one or more substituents selected from the group consisting of hydrogen (such as deuterium), hydroxyl, amino, cyano, halogen, oxo, and alkyl;

each $R^2$ is independently hydrogen, amino, hydroxyl, cyano, halogen, alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl; if there are two adjacent $R^2$, the two adjacent $R^2$ and their connected atoms optionally form saturated or partially unsaturated carbocyclyl, saturated or partially unsaturated heterocyclyl, aryl or heteroaryl; wherein, the alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^2$ each optionally are independently substituted by one or more substituents selected from the group consisting of hydroxyl, amino, cyano, halogen, oxo and alkyl;

each $R^3$ is independently hydrogen, amino, hydroxyl, cyano, halogen, alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl; if there are two adjacent $R^3$, the two adjacent $R^3$ and their connected atoms optionally form saturated or partially unsaturated carbocyclyl, saturated or partially unsaturated heterocyclyl, aryl or heteroaryl; wherein, the alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^3$ each optionally are independently substituted by one or more substituents selected from the group consisting of hydroxyl, amino, cyano, halogen, oxo, and alkyl; and m and p each are independently 0, 1, 2, 3 or 4.

In some examples, at least one of $X^1$, $X^2$, $X^3$, $X^4$, $Y^1$, $Y^2$, $Y^3$, $Y^4$, and $Y^5$ is an N atom. In some examples, at least two of $X^1$, $X^2$, $X^3$, $X^4$, $Y^1$, $Y^2$, $Y^3$, $Y^4$, and $Y^5$ are N atoms.

In some examples, at least one of $X^1$, $X^2$, $X^3$, and $X^4$ is an N atom. In some examples, at least two of $X^1$, $X^2$, $X^3$, and $X^4$ are N atoms. In some examples, at least one of $X^1$, $X^2$, $X^3$, and $X^4$ is an N atom. In some examples, at least two of $X^1$, $X^2$, $X^3$, and $X^4$ are N atoms.

In some examples, at least one of $Y^1$, $Y^2$, $Y^3$, $Y^4$, and $Y^5$ is an N atom. In some examples, at least two of $Y^1$, $Y^2$, $Y^3$, $Y^4$ and, $Y^5$ are N atoms.

In some examples, M is

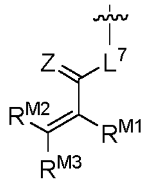
i-1
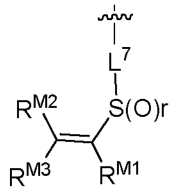
i-2
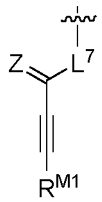
i-3
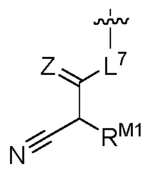
i-4
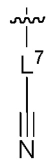
i-5
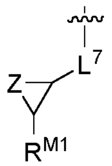
i-6
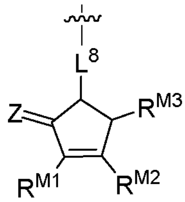
i-7
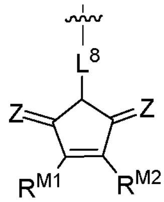
i-8
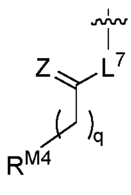
i-9
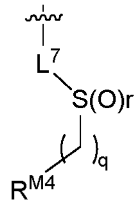
i-10
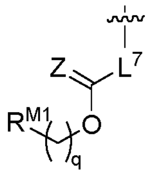
i-11
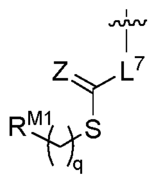
i-12
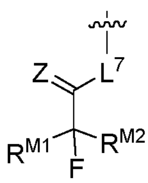
i-13
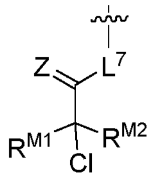
i-14
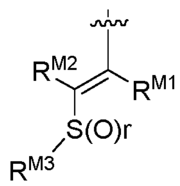
i-15
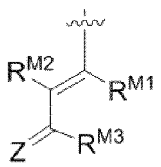
i-16
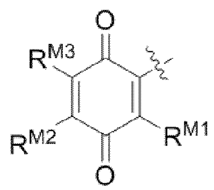
i-17
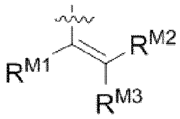
i-18
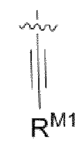
i-19
or
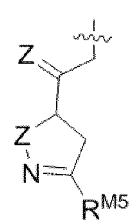
i-20
;

wherein,

$L^7$ is a bond, -O-, -S-, -$NR^{L7a}$-, or $C_{1-4}$ alkyl, wherein, one or more carbon units of the $C_{1-4}$ alkyl optionally are independently replaced with -O-, -S-, -$NR^{L7a}$-, -$NR^{L7a}$C(=O)-, -C(=O)$NR^{L7a}$-, -SC(=O)-, -C(=O)S-, -OC(=O)-, -C(=O)O-, -$NR^{L7a}$C(=S)-, -C(=S)$NR^{L7a}$-, *trans* $CR^{L7b}$=$CR^{L7b}$-, *cis* $CR^{L7b}$=$CR^{L7b}$-, -C≡C-, -S(=O)-, -S(=O)O-, -OS(=O)-, -S(=O)$NR^{L7a}$-, -$NR^{L7a}$S(=O)-, -$S(=O)_2$-, -$S(=O)_2$O-, -$OS(=O)_2$-, -$S(=O)_2NR^{L7a}$- or, -$NR^{L7a}S(=O)_2$-; wherein each $R^{L7a}$ is independently hydrogen, $C_{1-4}$alkyl, or a nitrogen protecting group; each $R^{L7b}$ is independently hydrogen, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two adjacent $R^{L7b}$ groups bind with their connected atoms to form 3-6 membered saturated or partially unsaturated carbocyclyl, or 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $L^7$, $R^{L7a}$, and $R^{L7b}$ each optionally are independently substituted by one or more substituents selected from the group consisting of hydrogen (such as deuterium), halogen, hydroxyl, amino, cyano, oxo, and $C_{1-4}$alkyl;

$L^8$ is a bond or $C_{1-4}$alkyl, wherein the alkyl involved in $L^8$ optionally is substituted by one or more substituents selected from the group consisting of hydrogen (such as deuterium), halogen, hydroxyl, amino, cyano, and oxo; each $R^{M1}$

is independently hydrogen, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, $-CH_2OR^{M1a}$, $-CH_2N(R^{M1a})_2$, $-CH_2SR^{M1a}$, $-OR^{M1a}$, $-N(R^{M1a})_2$, $-Si(R^{M1a})_3$, or $-SR^{M1a}$, wherein each $R^{M1a}$ is independently hydrogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl; or two $R^{M1a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M1}$ and $R^{M1a}$ each optionally are independently substituted by one or more substituents selected from the group consisting of hydrogen (such as deuterium), halogen, hydroxyl, amino, cyano, oxo, and $C_{1-4}$alkyl;

each $R^{M2}$ is independently hydrogen, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, $-CH_2ORM^{2a}$, $-CH_2N(R^{M2a})_2$, $-CH_2SR^{M2a}$, $-OR^{M2a}$, $-N(R^{M2a})_2$ or $-SR^{M2a}$, wherein each $R^{M2a}$ is independently hydrogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl; or two $R^{M2a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M2}$ and $R^{M2a}$ each optionally are independently substituted by one or more substituents selected from the group consisting of hydrogen (such as deuterium), halogen, hydroxyl, amino, cyano, oxo, and $C_{1-4}$alkyl;

each $R^{M3}$ is independently hydrogen, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, $-CH_2ORM^{3a}$, $-CH_2N(R^{M3a})_2$, $-CH_2SR^{M3a}$, $-OR^{M3a}$, $-N(R^{M3a})_2$, or $-SR^{M3a}$, wherein each $R^{M3a}$ is independently hydrogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl; or two $R^{M3a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M3}$ and $R^{M3a}$ each optionally are independently substituted by one or more substituents selected from the group consisting of hydrogen (such as deuterium), halogen, hydroxyl, amino, cyano, oxo, and $C_{1-4}$alkyl;

optionally, $R^{M1}$ and $R^{M3}$, or $R^{M2}$ and $R^{M3}$, or $R^{M1}$ and $R^{M2}$ optionally connect to form saturated or partially unsaturated carbocyclyl or saturated or partially unsaturated heterocyclyl, wherein the saturated or partially unsaturated carbocyclyl or saturated or partially unsaturated heterocyclyl each optionally are independently substituted by one or more substituents selected from the group consisting of hydrogen (such as deuterium), halogen, hydroxyl, amino, cyano, oxo, and $C_{1-4}$alkyl;

$R^{M4}$ is a leaving group; $R^{M5}$ is halogen; Z is O, S or $NR^Z$; wherein $R^Z$ is hydrogen, deuterium, $C_{1-4}$alkyl, or a nitrogen protecting group; the alkyl involved in $R^Z$ optionally is substituted by one or more substituents selected from the group consisting of hydrogen (such as deuterium), halogen, hydroxyl, amino, cyano, and oxo; q is 0, 1, 2, 3, 4, 5, or 6; r is 1 or 2.

In some examples, M is

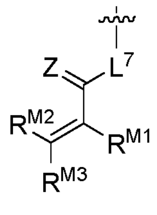
i-1 ,
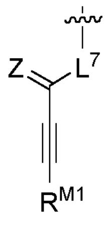
i-3 ,
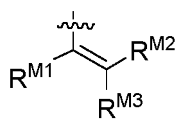
i-18 or
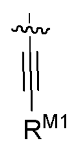
i-19 ;

wherein,

$L^7$ is a bond, -O-, -S-, $-NR^{L7a}-$, or $C_{1-4}$alkyl, wherein, one or more carbon units of the $C_{1-4}$alkyl group optionally are independently replaced with -O-, -S-, $-NR^{L7a}-$, $-NR^{L7a}C(=O)-$, $-C(=O)NR^{L7a}$, -SC(=O)-, -C(=O)S-, -OC(=O)-, -C(=O)O-, $-NR^{L7a}C(=S)-$, $-C(=S)NR^{L7a}-$, *trans* $CR^{L7b}=CR^{L7b}-$, *cis* $CR^{L7b}=CR^{L7b}-$, -C≡C-, -S(=O)-, -S(=O)O-, -OS(=O)-, $-S(=O)NR^{L7a}-$, $-NR^{L7a}S(=O)-$, $-S(=O)_2-$, $-S(=O)_2O-$, $-OS(=O)_2-$, $-S(=O)_2NR^{L7a}-$, or $-NR^{L7a}S(=O)_2-$; where-

in each $R^{L7a}$ is independently hydrogen, deuterium, $C_{1-4}$alkyl, or a nitrogen protecting group; each $R^{L7b}$ is independently hydrogen, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two adjacent $R^{L7b}$ groups optionally bind with their connected atoms to form 3-6 membered saturated or partially unsaturated carbocyclyl or 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $L^7$, $R^{L7a}$ and $R^{L7b}$ each optionally are independently substituted by one or more substituents selected from the group consisting of hydrogen (such as deuterium), halogen, hydroxyl, amino, cyano, oxo, and $C_{1-4}$alkyl;

Z is O, S, or $NR^Z$; wherein $R^Z$ is hydrogen, $C_{1-4}$alkyl, or a nitrogen protecting group; alkyl involved in $R^Z$ optionally is substituted by one or more substituents selected from the group consisting of hydrogen (such as deuterium), halogen, hydroxyl, amino, cyano, and oxo;

each $R^{M1}$ is independently hydrogen, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, $-CH_2OR^{M1a}$, $-CH_2N(R^{M1a})_2$, $-CH_2SR^{M1a}$, $-OR^{M1a}$, $-N(R^{M1a})_2$, $-Si(R^{M1a})_3$ or $-SR^{M1a}$, wherein each $R^{M1a}$ is independently hydrogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two $R^{M1a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M1}$ and $R^{M1a}$ each optionally are independently substituted by one or more substituents selected from the group consisting of deuterium, halogen, hydroxyl, amino, cyano, oxo, and $C_{1-4}$alkyl;

each $R^{M2}$ is independently hydrogen, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, $-CH_2ORM^{2a}$, $-CH_2N(R^{M2a})_2$, $-CH_2SR^{M2a}$, $-OR^{M2a}$, $-N(R^{M2a})_2$ or $-SR^{M2a}$, wherein each $R^{M2a}$ is independently hydrogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two $R^{M2a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M2}$ and $R^{M2a}$ each optionally are independently substituted by one or more substituents selected from the group consisting of hydrogen (such as deuterium), halogen, hydroxyl, amino, cyano, oxo, and $C_{1-4}$alkyl;

each $R^{M3}$ is independently hydrogen, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, $-CH_2ORM^{3a}$, $-CH_2N(R^{M3a})_2$, $-CH_2SR^{M3a}$, $-OR^{M3a}$, $-N(R^{M3a})_2$ or $-SR^{M3a}$, wherein each $R^{M3a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two $R^{M3a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M3}$ and $R^{M3a}$ each optionally are independently substituted by one or more substituents selected from the group consisting of hydrogen (such as deuterium), halogen, hydroxyl, amino, cyano, oxo, and $C_{1-4}$alkyl;

In some examples, M is

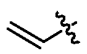 , 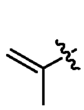 , 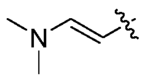 , 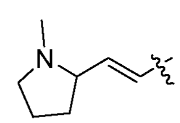 , 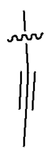 , 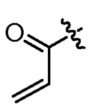 , 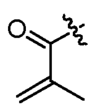 , 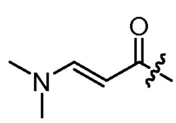 ,

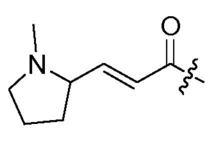 or 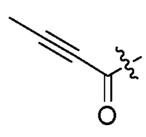 .

In some examples, A is 6-10 membered aryl, or 5-10 membered heteroaryl. In some examples, A is 6-10 membered aryl, or 5-10 membered azaaryl. In some examples, A is phenyl or 6-membered azaaryl. In some examples, A is phenyl, imidazolyl, pyrazolyl, triazolyl, tetraazolyl, oxazolyl, thiazolyl, furyl, thienyl, pyrrolyl, pyridinyl, pyrimidinyl, pyrazinyl, pyridazinyl, or the following groups:

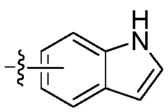 , 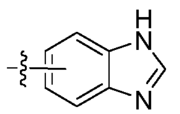 , 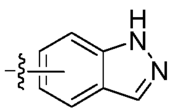 , 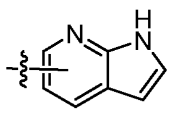 , 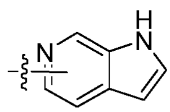 , 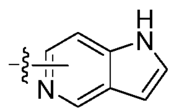 ,

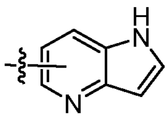 , 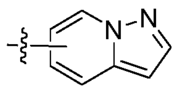 , 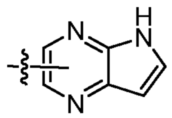 , 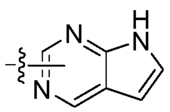 , 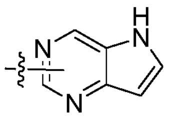 , 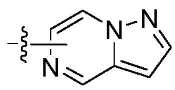 ,

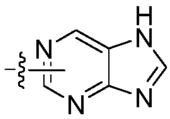 , 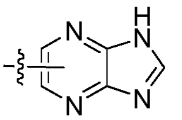 , 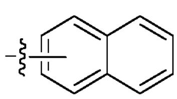 , 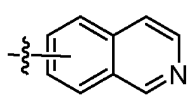 , 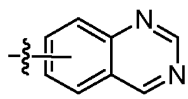 or 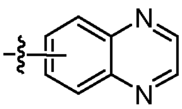 ,

in which 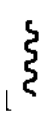 means an end of A connected to $L^6$.

In some examples, A is phenyl, pyridinyl, pyrimidinyl, pyrazinyl, or pyridazinyl. In some examples, A is phenyl or pyrazinyl.

In some examples, $L^1$ is a bond, -C(=O)-, -S(=O)-, $-S(=O)_2-$, $-C(=O)NR^{L1a}-$, $-NR^{L1a}C(=O)-$, $-S(=O)_2NR^{L1a}-$, or $-NR^{L1a}S(=O)_2-$; wherein, each $R^{L1a}$ is independently hydrogen, $C_{1-4}$alkyl, $C_{2-4}$alkenyl, $C_{2-4}$alkynyl, $C_{1-4}$heteroalkyl, $C_{1-4}$haloalkyl, $C_{1-4}$haloheteroalkyl, or a nitrogen protecting group; wherein the alkyl, alkenyl, alkynyl, heteroalkyl, haloalkyl, haloheteroalkyl involved in $R^{L1a}$ each optionally are independently substituted by one or more substituents selected from the group consisting of hydrogen (such as deuterium), halogen, hydroxyl, amino, cyano, and oxo; $L^2$ is a bond, $C_{1-6}$alkyl, $C_{1-6}$heteroalkyl, 3-6 membered saturated or partially unsaturated carbocyclyl, 3-6 membered saturated or partially unsaturated heterocyclyl, 6-10 membered aryl, or 5-10 membered heteroaryl; wherein, the alkyl, heteroalkyl, saturated or partially unsaturated carbocyclyl, saturated or partially unsaturated heterocyclyl, aryl, or heteroaryl involved in $R^2$ each optionally are independently substituted by one or more substituents selected from the group consisting of hydrogen (such as deuterium), hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, or $C_{1-4}$haloheteroalkyl; $L^3$ is a bond, 6-10 membered aryl, or 5-10 membered heteroaryl; wherein, the aryl, or heteroaryl involved in $L^3$ each optionally are independently substituted by one or more substituents selected from the group consisting of hydrogen (such as deuterium), hydroxyl, amino, cyano, halogen, $C_{1-4}$alkyl, $C_{2-4}$alkenyl, $C_{2-4}$alkynyl, $C_{1-4}$heteroalkyl, $C_{1-4}$haloalkyl, or $C_{1-4}$haloheteroalkyl; $L^4$ is a carbon chain or heterochain with 2-6 atoms; wherein, the carbon chain or heterochain involved in $L^4$ each optionally are independently substituted by one or more substituents selected from the group consisting of hydrogen (such as deuterium), hydroxyl, amino, cyano, halogen, oxo, $C_{2-4}$alkenyl, $C_{2-4}$alkynyl, $C_{1-4}$alkyl, $C_{1-4}$haloalkyl, $C_{1-4}$hydroxyalkyl, $C_{1-4}$aminoalkyl, $C_{1-4}$alkoxyl, $C_{1-4}$haloalkoxyl, $C_{1-4}$hydroxyalkoxyl, $C_{1-4}$aminoalkoxyl, $C_{1-4}$alkylamino, and a nitrogen protecting group.

In some examples, $L^1$ is a bond, -C(=O)-, -S(=O)-, $-S(=O)_2-$, $-C(=O)NR^{L1a}-$, $-NR^{L1a}C(=O)-$, $-S(=O)_2NR^{L1a}-$, or $-NR^{L1a}S(=O)_2-$; wherein, each $R^{L1a}$ is independently hydrogen, methyl, ethyl, ethenyl, ethynyl or trifluoromethyl; $L^2$ is a bond, $C_{1-6}$alkyl, $C_{1-6}$heteroalkyl, 3-6 membered saturated or partially unsaturated carbocyclyl, 3-6 membered saturated or partially unsaturated heterocyclyl, 6-10 membered aryl, or 5-10 membered heteroaryl; wherein, the alkyl, heteroalkyl, saturated or partially unsaturated carbocyclyl, saturated or partially unsaturated heterocyclyl, aryl, or heteroaryl involved in $L^2$ each optionally are independently substituted by a substituent selected from the group consisting of hydrogen (such as deuterium), hydroxyl, amino, cyano, fluoro, chloro, oxo, ethenyl, ethynyl, methoxy, ethoxy, trifluoromethoxy and methylamino; $L^3$ is a bond, 6-10 membered aryl, or 5-10 membered heteroaryl; wherein, aryl, or heteroaryl involved in $L^3$ each optionally are independently substituted by a substituent selected from the group consisting of hydrogen (such as deuterium), hydroxyl, amino, cyano, fluoro, chloro, methyl, ethyl, ethenyl, ethynyl, methoxy, ethoxy, trifluoromethoxy and methylamino; $L^4$ is a carbon chain or heterochain with 2-4 atoms; wherein, the carbon chain or heterochain involved in $L^4$ each optionally are independently substituted by one or more substituents selected from the group consisting of hydrogen (such as deuterium), hydroxyl, amino, cyano, halogen, oxo, ethenyl, ethynyl, methyl, ethyl, isopropyl, methoxy, ethoxy, trifluoromethoxy, methylamino, and a nitrogen protecting group.

In some examples, $L^1$ is a bond, -C(=O)-, -S(=O)-, $-S(=O)_2-$, -C(=O)NH-, -NHC(=O)-, $-S(=O)_2NH-$ or $-NHS(=O)_2-$; $L^2$ is a bond, 3-6 membered saturated or partially unsaturated carbocyclyl or 3-6 membered saturated or partially unsaturated heterocyclyl; wherein, saturated or partially unsaturated carbocyclyl, saturated or partially unsaturated heterocyclyl involved in $L^2$ each optionally are independently substituted by one or more substituents selected from the group consisting of hydroxyl, amino, cyano, fluoro, chloro, oxo, ethenyl, ethynyl, methoxy, ethoxy, trifluoromethoxy and methylamino; $L^3$ is a bond, 6-10 membered aryl, or 5-10 membered heteroaryl; wherein, aryl, or heteroaryl involved in $L^3$ each optionally are substituted by one or more substituents selected from the group consisting of hydroxyl, amino, cyano, fluoro, chloro, methyl, ethyl, ethenyl, ethynyl, methoxy, ethoxy, trifluoromethoxy, methylamino; $L^4$ is a carbon chain or heterochain with 2-4 atoms; wherein, the carbon chain or heterochain involved in $L^4$ each optionally are independently substituted by one or more substituents selected from the group consisting of hydrogen (such as deuterium), hydroxyl, amino, cyano, halogen, oxo, ethenyl, ethynyl, methyl, ethyl, isopropyl, methoxy, ethoxy, trifluoromethoxy, methylamino, and a nitrogen protecting group.

In some examples, $L^1$ is a bond, -C(=O)- or -C(=O)NH-; $L^2$ is a bond, cyclohexyl, piperidyl, phenyl or pyridinyl; $L^3$ is a bond, phenyl or pyridinyl; $L^4$ is $-NHCH_2-$, $-CH_2NH-$, $-OCH_2-$, $-CH_2O-$, $-SCH_2-$, $-CH_2S-$, $-NHCH_2CH_2-$, $-CH_2NHCH_2-$, $-CH_2CH_2NH-$, $-OCH_2CH_2-$, $-CH_2OCH_2-$, $-CH_2CH_2O-$, $-SCH_2CH_2-$, $-CH_2SCH_2-$, $-CH_2CH_2S-$, $-NHCH_2CH_2CH_2-$, $-CH_2NHCH_2CH_2-$, $-CH_2CH_2NHCH_2-$, $-CH_2CH_2CH_2NH-$, $-OCH_2CH_2CH_2-$, $-CH_2OCH_2CH_2-$, $-CH_2CH_2OCH_2-$, $-CH_2CH_2CH_2O-$, $-SCH_2CH_2CH_2-$, $-CH_2SCH_2CH_2-$, $-CH_2CH_2SCH_2-$, $-CH_2CH_2CH_2S-$, $-CH_2OCH_2O-$, $-OCH_2OCH_2-$, $-OCH_2CH_2O-$, $-NHCH_2CH_2O-$, $-CH_2NHCH_2O-$ or $-NHCH_2OCH_2-$; wherein the hydrogen in $CH_2$ involved in $L^4$ each optionally are independently replaced by a substituent selected from the group consisting of hydrogen (such as deuterium), hydroxyl, amino, cyano, halogen, oxo, ethenyl, ethynyl, methyl, ethyl, isopropyl, methoxy, ethoxy, trifluoromethoxy and methylamino; the hydrogen in NH involved in $L^4$ each optionally are independently replaced by a substituent selected from the group consisting of deuterium, hydroxyl, cyano, halogen, oxo, ethenyl, ethynyl, methyl, ethyl, isopropyl, methoxy, ethoxy, trifluoromethoxy, and a nitrogen protecting group.

In some examples, $L^1$ is a bond, -C(=O)- or -C(=O)NH-; $L^2$ is a bond, cyclohexyl, piperidyl, phenyl or pyridinyl; $L^3$ is a bond, phenyl or pyridinyl; $L^4$ is $-NHCH_2-$, $-CH_2NHCH_2-$, -NHC(=O)-, $-CH_2NHC(=O)-$, $-C(=O)NHCH_2-$, or $-NH-CH(CH_3)-$.

In some examples, $L^5$ is $-NR^{L5a}-$, -O-, -S-, $C_{1-6}$alkyl or $C_{1-6}$heteroalkyl; wherein, the alkyl and heteroalkyl involved in $L^5$ each optionally are independently substituted by one or more substituents selected from the group consisting of hydrogen (such as deuterium), amino, hydroxyl, oxo, cyano, halogen, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; each $R^{L5a}$ is independently hydrogen, deuterium, $C_{1-6}$alkyl, $C_{2-6}$alkenyl, $C_{2-6}$alkynyl, $C_{1-6}$heteroalkyl, $C_{1-6}$haloalkyl, or a nitrogen protecting group; $L^6$ is $-NR^{L6a}-$, -O-, -S-, $C_{1-6}$alkyl or $C_{1-6}$heteroalkyl; wherein, the alkyl and heteroalkyl involved in $L^6$ each optionally are independently substituted by one or more substituents selected from the group consisting of hydrogen (such as deuterium), amino, hydroxyl, oxo, cyano, halogen, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; each $R^{L6a}$ is independently hydrogen, $C_{1-6}$alkyl, $C_{2-6}$alkenyl, $C_{2-6}$alkynyl, $C_{1-6}$heteroalkyl, $C_{1-6}$haloalkyl, or a nitrogen protecting group.

In some examples, $L^5$ is $-NR^{L5a}$, $-CR^{L5b}R^{L5c}-$, -O-, -S-, $-CR^{L5b}R^{L5c}NR^{L5a}-$, $-NR^{L5a}CR^{L5b}R^{L5c}-$, $-CR^{L5b}R^{L5c}O-$, $-OCR^{L5b}R^{L5c}-$, $-CR^{L5b}R^{L5c}S-$, or $-SCR^{L5b}R^{L5c}-$; wherein, each $R^{L5b}$ and $R^{L5c}$ are independently hydrogen, amino, hydroxyl, oxo, cyano, halogen, $C_{1-4}$alkyl, $C_{2-4}$alkenyl, $C_{2-4}$alkynyl, $C_{1-4}$heteroalkyl, $C_{1-4}$haloalkyl, or $C_{1-4}$haloheteroalkyl; each $R^{L5a}$ is independently hydrogen, deuterium, $C_{1-4}$alkyl, $C_{2-4}$alkenyl, $C_{2-4}$alkynyl, $C_{1-4}$heteroalkyl, $C_{1-4}$haloalkyl, or a nitrogen protecting group; $L^6$ is $-NR^{L6a}$, $-CR^{L6b}R^{L6c}-$, -O-, -S-, $-CR^{L6b}R^{L6c}NR^{L6a}-$, $-NR^{L6a}CR^{L6b}R^{L6c}-$, $-CR^{L6b}R^{L6c}O-$, $-OCR^{L6b}R^{L6c}-$, $-CR^{L6b}R^{L6c}S-$, or $-SCR^{L6b}R^{L6c}-$; wherein, each $R^{L6b}$ and $R^{L6c}$ are independently hydrogen, amino, hydroxyl, oxo, cyano, halogen, $C_{1-4}$alkyl, $C_{2-4}$alkenyl, $C_{2-4}$alkynyl, $C_{1-4}$heteroalkyl, $C_{1-4}$haloalkyl, or $C_{1-4}$haloheteroalkyl; each $R^{L6a}$ is independently hydrogen, $C_{1-4}$alkyl, $C_{2-4}$alkenyl, $C_{2-4}$alkynyl, $C_{1-4}$heteroalkyl, $C_{1-4}$haloalkyl, or a nitrogen protecting group.

In some examples, $L^5$ is -NH-, -O-, -S-, $-NHCH_2-$, $-OCH_2-$, $-SCH_2-$, $-CH_2NH-$, $-CH_2O-$ or $-CH_2S-$; $L^6$ is -NH-, -O-, -S-, $-NHCH_2-$, $-OCH_2-$, $-SCH_2-$, $-CH_2NH-$, $-CH_2O-$, or $-CH_2S-$. In some examples, $L^5$ is -NH-; $L^6$ is -NH-, or $-NHCH_2-$.

In some examples, each $R^1$ is independently hydrogen, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, $C_{1-6}$alkoxyl, $C_{1-6}$haloalkoxyl, $C_{1-6}$haloheteroalkyl, 3-6 membered saturated or partially unsaturated carbocyclyl, 3-6 membered saturated or partially unsaturated carbocyclyl$C_{1-6}$alkyl, 3-6 membered saturated or partially unsaturated heterocyclyl, 3-6 membered saturated or partially unsaturated heterocyclyl$C_{1-6}$alkyl, 6-10 membered aryl, 6-10 membered aryl$C_{1-6}$alkyl, 6-10 membered heteroaryl, or 6-10 membered heteroaryl$C_{1-6}$alkyl; if there are two adjacent $R^1$, the two adjacent $R^1$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^1$

each optionally are independently substituted by one or more substituents selected from the group consisting of hydrogen (such as deuterium), hydroxyl, amino, cyano, halogen, oxo, and $C_{1-4}$alkyl;

each $R^2$ is independently hydrogen, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, $C_{1-6}$haloheteroalkyl, $C_{1-6}$alkoxyl, $C_{1-6}$haloalkoxyl, 3-6 membered saturated or partially unsaturated carbocyclyl, 3-6 membered saturated or partially unsaturated carbocyclyl$C_{1-6}$alkyl, 3-6 membered saturated or partially unsaturated heterocyclyl, 3-6 membered saturated or partially unsaturated heterocyclyl$C_{1-6}$alkyl, 6-10 membered aryl, 6-10 membered aryl$C_{1-6}$alkyl, 6-10 membered heteroaryl or 6-10 membered heteroaryl$C_{1-6}$alkyl; if there are two adjacent $R^2$, the two adjacent $R^2$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^2$ each optionally are independently substituted by one or more substituents selected from the group consisting of hydrogen (such as deuterium), hydroxyl, amino, cyano, halogen, oxo, and $C_{1-4}$alkyl; and

each $R^3$ is independently hydrogen, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, $C_{1-6}$haloheteroalkyl, $C_{1-6}$alkoxyl, $C_{1-6}$haloalkoxyl, 3-6 membered saturated or partially unsaturated carbocyclyl, 3-6 membered saturated or partially unsaturated carbocyclyl$C_{1-6}$alkyl, 3-6 membered saturated or partially unsaturated heterocyclyl, 3-6 membered saturated or partially unsaturated heterocyclyl$C_{1-6}$alkyl, 6-10 membered aryl, 6-10 membered aryl$C_{1-6}$alkyl, 6-10 membered heteroaryl or 6-10 membered heteroaryl$C_{1-6}$alkyl; if there are two adjacent $R^3$, the two adjacent $R^3$ and their connected atoms optionally form3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^3$ each optionally are independently substituted by one or more substituents selected from the group consisting of hydrogen (such as deuterium), hydroxyl, amino, cyano, halogen, oxo, and $C_{1-4}$alkyl.

In some examples, each $R^1$ is independently hydrogen, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, or $C_{1-6}$haloheteroalkyl; if there are two adjacent $R^1$, the two adjacent $R^1$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, saturated or partially unsaturated carbocyclyl, saturated or partially unsaturated heterocyclyl, aryl, or heteroaryl involved in $R^1$ each optionally are independently substituted by one or more substituents selected from the group consisting of hydrogen (such as deuterium), hydroxyl, amino, cyano, halogen, oxo, and $C_{1-4}$alkyl; each $R^2$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, and $C_{1-6}$haloheteroalkyl; if there are two adjacent $R^2$, the two adjacent $R^2$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, saturated or partially unsaturated carbocyclyl, saturated or partially unsaturated heterocyclyl, aryl, or heteroaryl involved in $R^2$ each optionally are independently substituted by one or more substituents selected from the group consisting of hydrogen (such as deuterium), hydroxyl, amino, cyano, halogen, oxo, and $C_{1-4}$alkyl; and each $R^3$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, $C_{1-6}$haloheteroalkyl; if there are two adjacent $R^3$, the two adjacent $R^3$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, saturated or partially unsaturated carbocyclyl, saturated or partially unsaturated heterocyclyl, aryl, or heteroaryl involved in $R^3$ each optionally are independently substituted by one or more substituents selected from the group consisting of hydrogen (such as deuterium), hydroxyl, amino, cyano, halogen, oxo, and $C_{1-4}$alkyl.

In some examples, each $R^1$ is independently hydrogen, amino, hydroxyl, cyano, halogen, $C_{1-6}$alkyl, $C_{1-6}$heteroalkyl, $C_{1-6}$haloalkyl, or $C_{1-6}$haloheteroalkyl; wherein the alkyl, heteroalkyl, haloalkyl, haloheteroalkyl involved in $R^1$ each optionally are independently substituted by one or more substituents selected from the group consisting of deuterium, hydroxyl, amino, cyano, halogen, and oxo; each $R^2$ is independently hydrogen, amino, hydroxyl, cyano, halogen, $C_{1-6}$alkyl, $C_{1-6}$heteroalkyl, $C_{1-6}$haloalkyl, and $C_{1-6}$haloheteroalkyl; wherein the alkyl, heteroalkyl, haloalkyl, haloheteroalkyl involved in $R^2$ each optionally are independently substituted by one or more substituents selected from

the group consisting of hydrogen (such as deuterium), hydroxyl, amino, cyano, halogen, and oxo; and each $R^3$ is independently hydrogen, amino, hydroxyl, cyano, halogen, $C_{1-6}$alkyl, $C_{1-6}$heteroalkyl, $C_{1-6}$haloalkyl, and $C_{1-6}$haloheteroalkyl; if there are two adjacent $R^3$, the two adjacent $R^3$ and their connected atoms optionally form 3-6 membered saturated or partially unsaturated carbocyclyl, 3-6 membered saturated or partially unsaturated heterocyclyl; wherein the alkyl, heteroalkyl, haloalkyl, haloheteroalkyl, 3-6 membered saturated or partially unsaturated carbocyclyl, 3-6 membered saturated or partially unsaturated heterocyclyl involved in $R^3$ each optionally are independently substituted by one or more substituents selected from the group consisting of hydrogen (such as deuterium), hydroxyl, amino, cyano, halogen, oxo, and $C_{1-4}$alkyl.

In some examples, each $R^1$ is independently hydrogen, amino, hydroxyl, cyano, halogen, $C_{1-4}$alkyl, $C_{1-4}$heteroalkyl, $C_{1-4}$haloalkyl, or $C_{1-4}$haloheteroalkyl; wherein the alkyl, heteroalkyl, haloalkyl, haloheteroalkyl involved in $R^1$ each optionally are independently substituted by one or more substituents selected from the group consisting of hydrogen (such as deuterium), hydroxyl, amino, cyano, halogen, and oxo; each $R^2$ is independently hydrogen, deuterium, amino, hydroxyl, cyano, halogen, $C_{1-4}$alkyl, $C_{1-4}$heteroalkyl, $C_{1-4}$haloalkyl, and $C_{1-4}$haloheteroalkyl; wherein the alkyl, heteroalkyl, haloalkyl, haloheteroalkyl involved in $R^2$ each optionally are independently substituted by one or more substituents selected from the group consisting of hydrogen (such as deuterium), hydroxyl, amino, cyano, halogen, and oxo; and each $R^3$ is independently hydrogen, amino, hydroxyl, cyano, halogen, $C_{1-4}$alkyl, $C_{1-4}$heteroalkyl, $C_{1-4}$haloalkyl, and $C_{1-4}$haloheteroalkyl; if there are two adjacent $R^3$, the two adjacent $R^3$ and their connected atoms optionally form 3-6 membered saturated or partially unsaturated carbocyclyl, 3-6 membered saturated or partially unsaturated heterocyclyl; wherein the alkyl, heteroalkyl, haloalkyl, haloheteroalkyl, 3-6 membered saturated or partially unsaturated carbocyclyl, 3-6 membered saturated or partially unsaturated heterocyclyl involved in $R^3$ each optionally are independently substituted by one or more substituents selected from the group consisting of hydrogen (such as deuterium), hydroxyl, amino, cyano, halogen, oxo, methyl, ethyl, and isopropyl.

In some examples, each $R^1$ is independently hydrogen, amino, hydroxyl, cyano, fluoro, chloro, methyl, ethyl, propyl, isopropyl, *t*-butyl, methoxy, ethoxy, *i*-propoxy, *t*-butoxy, trifluoromethyl, trifluoromethoxy, $CH_3C(=O)-$, $CH_3CH_2C(=O)-$, $(CH_3)_2CHC(=O)-$, $CH_3C(=O)O-$, $CH_3CH_2C(=O)O-$, $(CH_3)_2CHC(=O)O-$, $CH_3C(=O)NH-$, $CH_3CH_2C(=O)NH-$, $(CH_3)_2CHC(=O)NH-$, $CH_3NHC(=O)-$, $CH_3CH_2NHC(=O)-$, $(CH_3)_2CHNHC(=O)-$, $CH_3ONHC(=O)-$, $CH_3CH_2ONHC(=O)-$, $(CH_3)_2CHONHC(=O)-$, $CH_3S(=O)_2-$, $CH_3CH_2S(=O)_2-$, $(CH_3)_2CHS(=O)_2-$, $CH_3S(=O)_2O-$, $CH_3CH_2S(=O)_2O-$, $(CH_3)_2CHS(=O)_2O-$, $CH_3S(=O)_2NH-$, $CH_3CH_2S(=O)_2NH-$, $(CH_3)_2CHS(=O)_2NH-$, $CH_3NHS(=O)_2-$, $CH_3CH_2NHS(=O)_2-$, $(CH_3)_2CHNHS(=O)_2-$, $CH_3C(=O)N(CH_3)-$, $CH_3CH_2C(=O)N(CH_3)-$, $(CH_3)_2CHC(=O)N(CH_3)-$, $CH_3N(CH_3)C(=O)-$, $CH_3CH_2N(CH_3)C(=O)-$, $(CH_3)_2CHN(CH_3)C(=O)-$, $CH_3ON(CH_3)C(=O)-$, $CH_3CH_2ON(CH_3)C(=O)-$, $(CH_3)_2CHON(CH_3)C(=O)-$, $CH_3S(=O)_2N(CH_3)-$, $CH_3CH_2S(=O)_2N(CH_3)-$, $(CH_3)_2CHS(=O)_2N(CH_3)-$, $CH_3N(CH_3)S(=O)_2-$, $CH_3CH_2N(CH_3)S(=O)_2-$, $(CH_3)_2CHN(CH_3)S(=O)_2-$, $CH_3C(=O)N(CH_2CH_3)-$, $CH_3CH_2C(=O)N(CH_2CH_3)-$, $(CH_3)_2CHC(=O)N(CH_2CH_3)-$, $CH_3N(CH_2CH_3)C(=O)-$, $CH_3CH_2N(CH_2CH_3)C(=O)-$, $(CH_3)_2CHN(CH_2CH_3)C(=O)-$, $CH_3ON(CH_2CH_3)C(=O)-$, $CH_3CH_2ON(CH_2CH_3)C(=O)-$, $(CH_3)_2CHON(CH_2CH_3)C(=O)-$, $CH_3S(=O)_2N(CH_2CH_3)-$, $CH_3CH_2S(=O)_2N(CH_2CH_3)-$, $(CH_3)_2CHS(=O)_2N(CH_2CH_3)-$, $CH_3N(CH_2CH_3)S(=O)_2-$, $CH_3CH_2N(CH_2CH_3)S(=O)_2-$, $(CH_3)_2CHN(CH_2CH_3)S(=O)_2-$, $(CH_3)_2P(=O)-$, $CH_3CH_2(CH_3)P(=O)-$, $(CH_3)_2CH(CH_3)P(=O)-$, $(CH_3CH_2)_2P(=O)-$, $CH_3CH_z(CH_3)P(=O)-$, or $(CH_3)_2CH(CH_3CH_2)P(=O)-$;

each $R^2$ is independently hydrogen, amino, hydroxyl, cyano, fluoro, chloro, bromo, methyl, ethyl, propyl, isopropyl, *t*-butyl, methoxy, ethoxy, *i*-propoxy, *t*-butoxy, trifluoromethyl, trifluoromethoxy, $CH_3C(=O)-$, $CH_3CH_2C(=O)-$, $(CH_3)_2CHC(=O)-$, $CH_3C(=O)O-$, $CH_3CH_2C(=O)O-$, $(CH_3)_2CHC(=O)O-$, $CH_3C(=O)NH-$, $CH_3CH_2C(=O)NH-$, $(CH_3)_2CHC(=O)NH-$, $CH_3NHC(=O)-$, $CH_3CH_2NHC(=O)-$, $(CH_3)_2CHNHC(=O)-$, $CH_3ONHC(=O)-$, $CH_3CH_2ONHC(=O)-$, $(CH_3)_2CHONHC(=O)-$, $CH_3S(=O)_2-$, $CH_3CH_2S(=O)_2-$, $(CH_3)_2CHS(=O)_2-$, $CH_3S(=O)_2O-$, $CH_3CH_2S(=O)_2O-$, $(CH_3)_2CHS(=O)_2O-$, $CH_3S(=O)_2NH-$, $CH_3CH_2S(=O)_2NH-$, $(CH_3)_2CHS(=O)_2NH-$, $CH_3NHS(=O)_2-$, $CH_3CH_2NHS(=O)_2-$, $(CH_3)_2CHNHS(=O)_2-$, $CH_3C(=O)N(CH_3)-$, $CH_3CH_2C(=O)N(CH_3)-$, $(CH_3)_2CHC(=O)N(CH_3)-$, $CH_3N(CH_3)C(=O)-$, $CH_3CH_2N(CH_3)C(=O)-$, $(CH_3)_2CHN(CH_3)C(=O)-$, $CH_3ON(CH_3)C(=O)-$, $CH_3CH_2ON(CH_3)C(=O)-$, $(CH_3)_2CHON(CH_3)C(=O)-$, $CH_3S(=O)_2N(CH_3)-$, $CH_3CH_2S(=O)_2N(CH_3)-$, $(CH_3)_2CHS(=O)_2N(CH_3)-$, $CH_3N(CH_3)S(=O)_2-$, $CH_3CH_2N(CH_3)S(=O)_2-$, $(CH_3)_2CHN(CH_3)S(=O)_2-$, $CH_3C(=O)N(CH_2CH_3)-$, $CH_3CH_2C(=O)N(CH_2CH_3)-$, $(CH_3)_2CHC(=O)N(CH_2CH_3)-$, $CH_3N(CH_2CH_3)C(=O)-$, $CH_3CH_2N(CH_2CH_3)C(=O)-$, $(CH_3)_2CHN(CH_2CH_3)C(=O)-$, $CH_3ON(CH_2CH_3)C(=O)-$, $CH_3CH_2ON(CH_2CH_3)C(=O)-$, $(CH_3)_2CHON(CH_2CH_3)C(=O)-$, $CH_3S(=O)_2N(CH_2CH_3)-$, $CH_3CH_2S(=O)_2N(CH_2CH_3)-$, $(CH_3)_2CHS(=O)_2N(CH_2CH_3)-$, $CH_3N(CH_2CH_3)S(=O)_2-$, $CH_3CH_2N(CH_2CH_3)S(=O)_2-$, $(CH_3)_2CHN(CH_2CH_3)S(=O)_2-$, $(CH_3)_2P(=O)-$, $CH_3CH_2(CH_3)P(=O)-$, $(CH_3)_2CH(CH_3)P(=O)-$, $(CH_3CH_2)_2P(=O)-$, $CH_3CH_2(CH_3)P(=O)-$, or $(CH_3)_2CH(CH_3CH_2)P(=O)-$; and

each $R^3$ is independently hydrogen, amino, hydroxyl, cyano, fluoro, chloro, methyl, ethyl, propyl, isopropyl, *t*-butyl, methoxy, ethoxy, *i*-propoxy, *t*-butoxy, trifluoromethyl, trifluoromethoxy, $CH_3C(=O)-$, $CH_3CH_2C(=O)-$, $(CH_3)_2CHC(=O)-$, $CH_3C(=O)O-$, $CH_3CH_2C(=O)O-$, $(CH_3)_2CHC(=O)O-$, $CH_3C(=O)NH-$, $CH_3CH_2C(=O)NH-$, $(CH_3)_2CHC(=O)NH-$, $CH_3NHC(=O)-$, $CH_3CH_2NHC(=O)-$, $(CH_3)_2CHNHC(=O)-$, $CH_3ONHC(=O)-$,

$CH_3CH_2ONHC(=O)$-, $(CH_3)_2CHONHC(=O)$-, $CH_3S(=O)_2$-, $CH_3CH_2S(=O)_2$-, $(CH_3)_2CHS(=O)_2$-, $CH_3S(=O)_2O$-, $CH_3CH_2S(=O)_2O$-, $(CH_3)_2CHS(=O)_2O$-, $CH_3S(=O)_2NH$-, $CH_3CH_2S(=O)_2NH$-, $(CH_3)_2CHS(=O)_2NH$-, $CH_3NHS(=O)_2$-, $CH_3CH_2NHS(=O)_2$-, $(CH_3)_2CHNHS(=O)_2$-, $CH_3C(=O)N(CH_3)$-, $CH_3CH_2C(=O)N(CH_3)$-, $(CH_3)_2CHC(=O)N(CH_3)$-, $CH_3N(CH_3)C(=O)$-, $CH_3CH_2N(CH_3)C(=O)$-, $(CH_3)_2CHN(CH_3)C(=O)$-, $CH_3ON(CH_3)C(=O)$-, $CH_3CH_2ON(CH_3)C(=O)$-, $(CH_3)_2CHON(CH_3)C(=O)$-, $CH_3S(=O)_2N(CH_3)$-, $CH_3CH_2S(=O)_2N(CH_3)$-, $(CH_3)_2CHS(=O)_2N(CH_3)$-, $CH_3N(CH_3)S(=O)_2$-, $CH_3CH_2N(CH_3)S(=O)_2$-, $(CH_3)_2CHN(CH_3)S(=O)_2$-, $CH_3C(=O)N(CH_2CH_3)$-, $CH_3CH_2C(=O)N(CH_2CH_3)$-, $(CH_3)_2CHC(=O)N(CH_2CH_3)$-, $CH_3N(CH_2CH_3)C(=O)$-, $CH_3CH_2N(CH_2CH_3)C(=O)$-, $(CH_3)_2CHN(CH_2CH_3)C(=O)$-, $CH_3ON(CH_2CH_3)C(=O)$-, $CH_3CH_2ON(CH_2CH_3)C(=O)$-, $(CH_3)_2CHON(CH_2CH_3)C(=O)$-, $CH_3S(=O)_2N(CH_2CH_3)$-, $CH_3CH_2S(=O)_2N(CH_2CH_3)$-, $(CH_3)_2CHS(=O)_2N(CH_2CH_3)$-, $CH_3N(CH_2CH_3)S(=O)_2$-, $CH_3CH_2N(CH_2CH_3)S(=O)_2$-, $(CH_3)_2CHN(CH_2CH_3)S(=O)_2$-, $(CH_3)_2P(=O)$-, $CH_3CH_2(CH_3)P(=O)$-, $(CH_3)_2CH(CH_3)P(=O)$-, $(CH_3CH_2)_2P(=O)$-, $CH_3CH_2(CH_3)P(=O)$-, or $(CH_3)_2CH(CH_3CH_2)P(=O)$-; if there are two adjacent $R^3$, the two adjacent $R^3$ and their connected atoms optionally form the following groups:

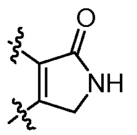, 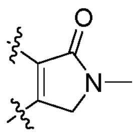, 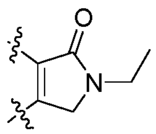, 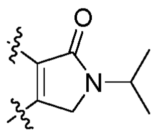, 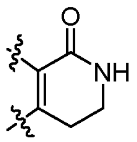, 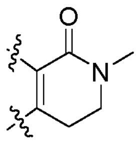, 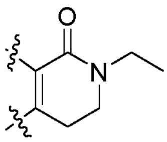,

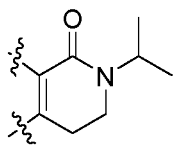, 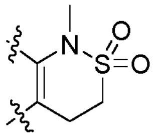 or 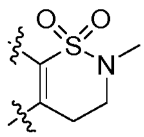.

In some examples, the saturated or partially unsaturated carbocyclyl is selected from formulae ii-1 to ii-6; when formulae ii-1 to ii-6 are connected to the rest of the molecule through one bond, formulae ii-1 to ii-6 are monovalent; when formulae ii-1 to ii-6 are connected to the rest of the molecule through two bonds, formulae ii-1 to ii-6 are bivalent; the saturated or partially unsaturated carbocyclyl represented by formulae ii-1 to ii-6 each optionally are independently substituted by one or more substituents defined by the aforementioned corresponding groups of the present application;

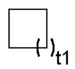
ii-1,
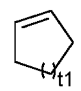
ii-2,
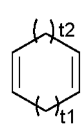
ii-3,
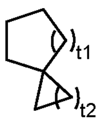
ii-4,
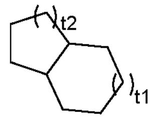
ii-5 or
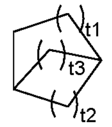
ii-6;

The saturated or partially unsaturated heterocyclyl is selected from formulae iii-1 to iii-16; when formulae iii-1 to iii-16 are connected to the rest of the molecule through one bond, formulae iii-1 to iii-16 are monovalent; when formulae iii-1 to iii-16 are connected to the rest of the molecule through two bonds, formulae iii-1 to iii-16 are bivalent; the saturated or partially unsaturated heterocyclyl represented by formulae iii-1 to iii-16 each optionally are independently substituted by one or more substituents defined by the aforementioned corresponding groups of the present application;

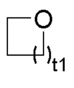
iii-1,
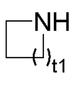
iii-2,
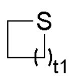
iii-3,
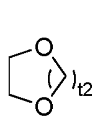
iii-4,
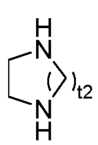
iii-5,
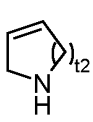
iii-6,
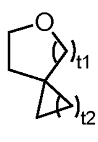
iii-7,
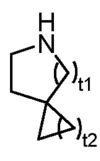
iii-8,
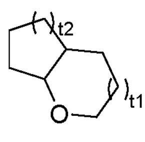
iii-9,
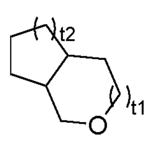
iii-10,

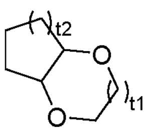
iii-11 ,
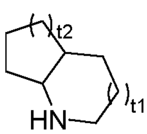
iii-12 ,
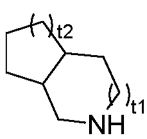
iii-13 ,
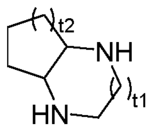
iii-14 ,
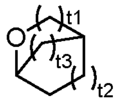
iii-15 or
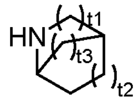
iii-16 ;

wherein, each t1 is independently 0, 1, 2, 3, 4, or 5; each t2 and t3 are independently 1, 2, 3, 4, or 5.

The phrase "substituted by substituents defined by the aforementioned corresponding groups of the present application" refers to substitution of the substituent mentioned in the previous sections involving "saturated or partially unsaturated carbocyclyl" and "saturated or partially unsaturated heterocyclyl".

In some examples, the saturated or partially unsaturated carbocyclyl is selected the following groups; when the saturated or partially unsaturated carbocyclyl is connected to the rest of the molecule through one bond, the following groups are monovalent; when the saturated or partially unsaturated carbocyclyl is connected to the rest of the molecule through two bonds, the following groups are bivalent; the following saturated or partially unsaturated carbocyclyl each optionally are independently substituted by one or more substituents defined by the aforementioned corresponding groups of the present application;

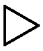 , 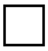 , 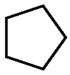 , 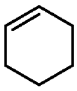 , 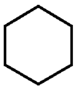 , 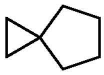 , 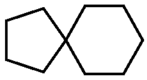 , 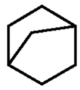 或 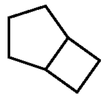 ;

The saturated or partially unsaturated heterocyclyl is selected the following groups; when the saturated or partially unsaturated heterocyclyl is connected to the rest of the molecule through one bond, the following groups are monovalent; when the saturated or partially unsaturated heterocyclyl is connected to the rest of the molecule through two bonds, the following groups are bivalent; the following saturated or partially unsaturated heterocyclyl each optionally are independently substituted by one or more substituents defined by the aforementioned corresponding groups of the present application;

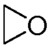 , 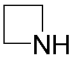 , 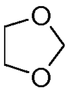 , 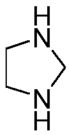 , 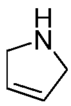 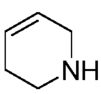 , 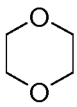 , 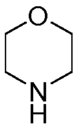 , 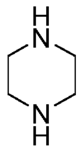 ,

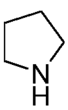 , 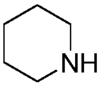 , 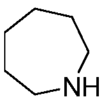 , 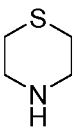 , 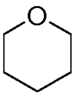 , 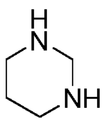 , 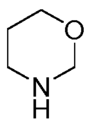 ,

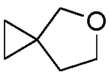 , 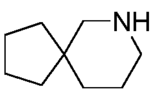 , 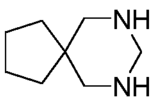 , 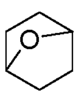 , 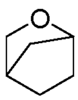 , 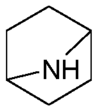 ,

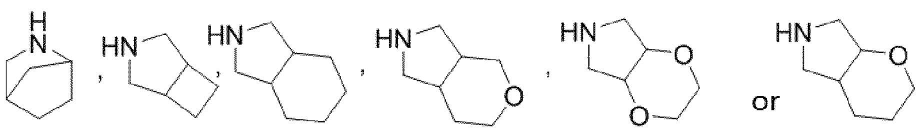
, , , , or .

The phrase "substituted by one or more substituents defined by the aforementioned corresponding groups of the present application" refers to the substituent mentioned in the previous sections that involve "saturated or partially unsaturated carbocyclyl" and "saturated or partially unsaturated heterocyclyl".

In some examples, the compound of the present application is a compound of any one of formulae II-1 to II-6

or a stereoisomer, a tautomer, an enantiomer, a diastereomer, a racemate, a geometric isomer, a nitrogen oxide, a solvate, a hydrate, a crystal form, an ester, an isotope labeled compound (preferably a deuterate), a metabolite, a pharmaceutically acceptable salt, or a prodrug thereof:

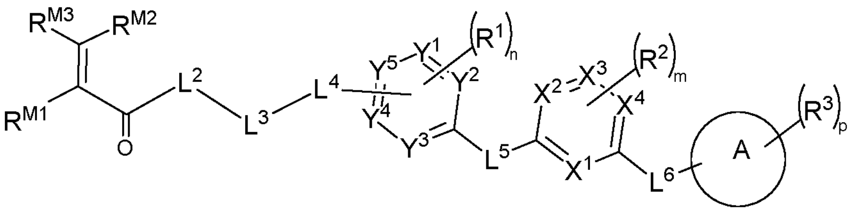

II-1,

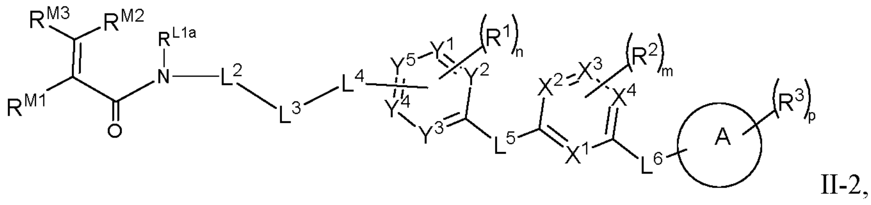

II-2,

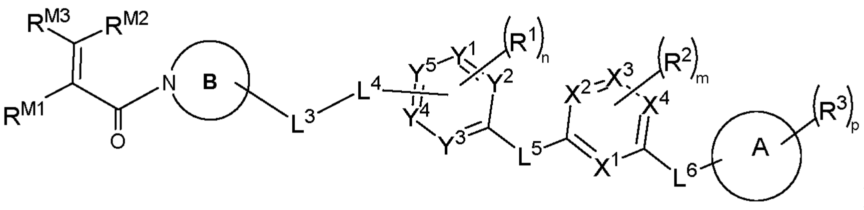

II-3,

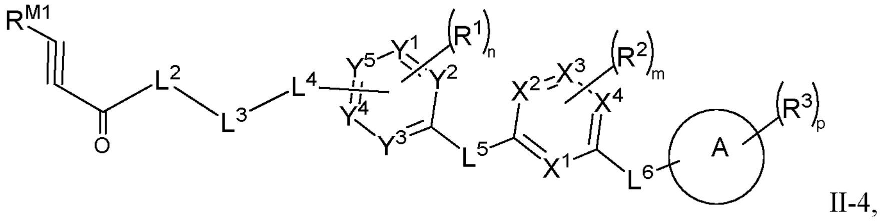

II-4,

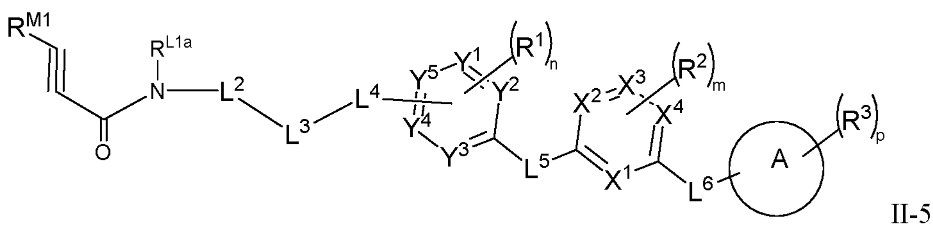

II-5,

or

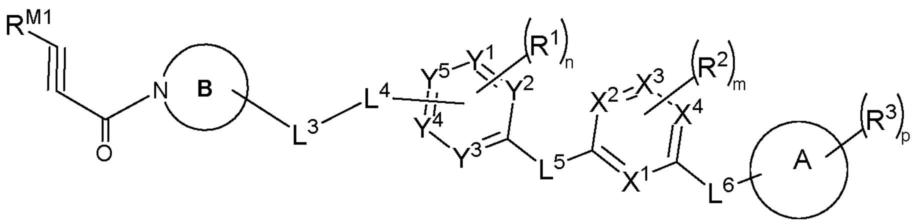

II-6;

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of hydrogen (such as deuterium), hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl.

In some examples, the compound of the present application is a compound of formula III or a stereoisomer, a tautomer, an enantiomer, a diastereomer, a racemate, a geometric isomer, a nitrogen oxide, a solvate, a hydrate, a crystal form, an ester, an isotope labeled compound (preferably a deuterate), a metabolite, a pharmaceutically acceptable salt, or a prodrug thereof:

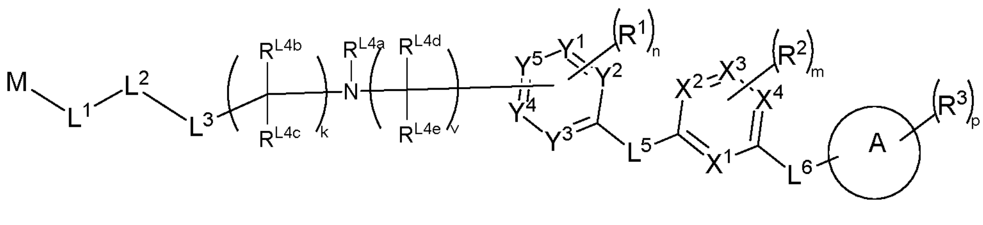

III

wherein, $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; k and v each are independently 0, 1 or 2.

In some examples, the compound of the present application is a compound of any one of formulae IV-1 to IV-6 or a stereoisomer, a tautomer, an enantiomer, a diastereomer, a racemate, a geometric isomer, a nitrogen oxide, a solvate, a hydrate, a crystal form, an ester, an isotope labeled compound (preferably a deuterate), a metabolite, a pharmaceutically acceptable salt, or a prodrug thereof:

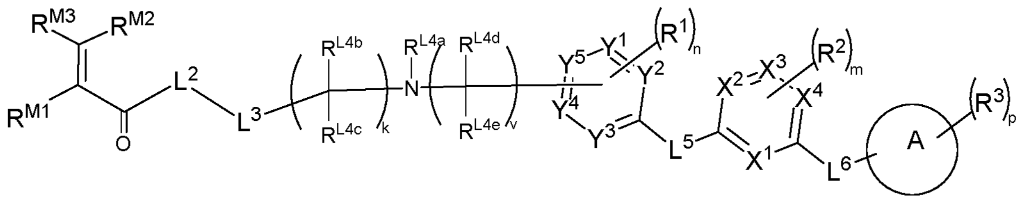

IV-1,

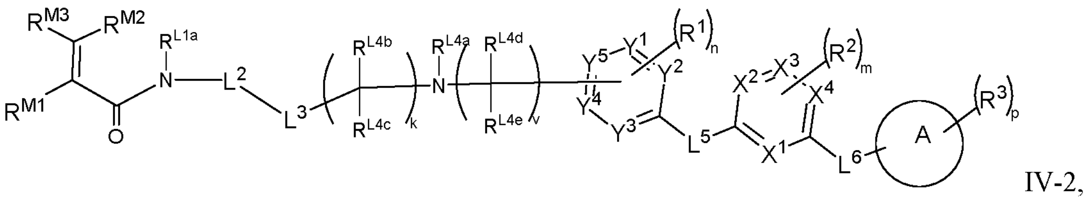

IV-2,

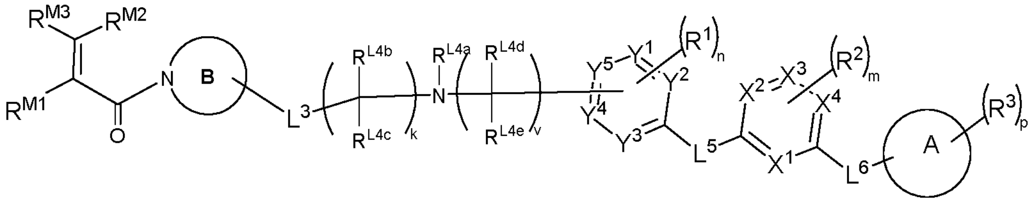

IV-3,

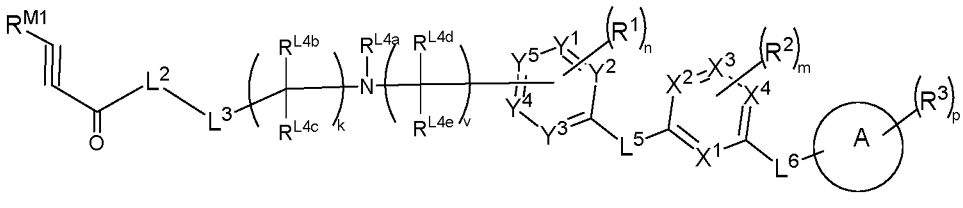

IV-4,

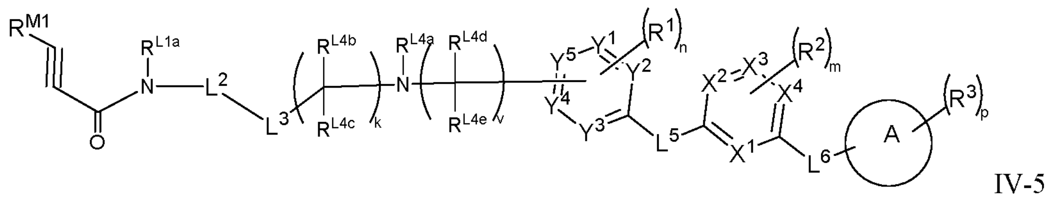

IV-5;

or

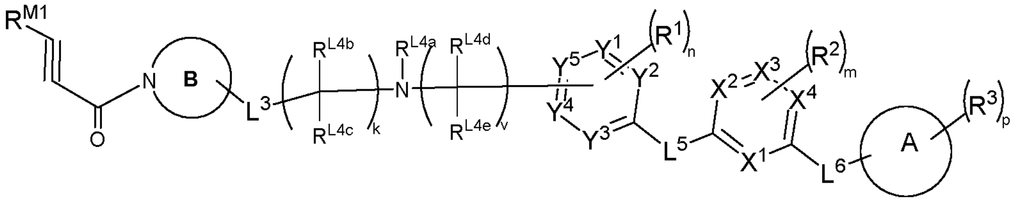

IV-6;

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of hydrogen (such as deuterium), hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; k and v each are independently 0, 1, or 2.

In some examples, the compound of the present application is a compound of any one of formulae V-1 to V-7 or a stereoisomer, a tautomer, an enantiomer, a diastereomer, a racemate, a geometric isomer, a nitrogen oxide, a solvate, a hydrate, a crystal form, an ester, an isotope labeled compound (preferably a deuterate), a metabolite, a pharmaceutically acceptable salt, or a prodrug thereof:

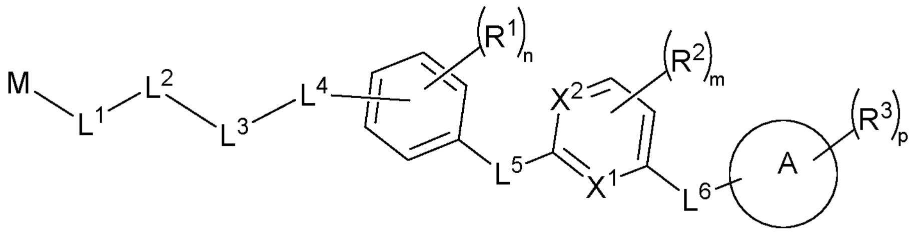

V-1,

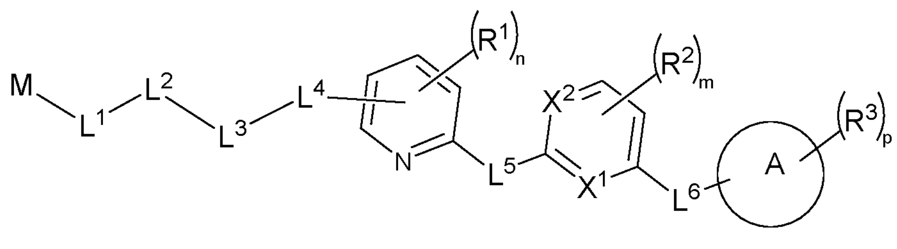

V-2,

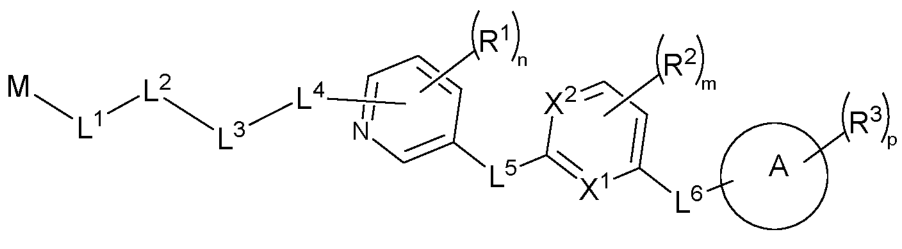

V-3,

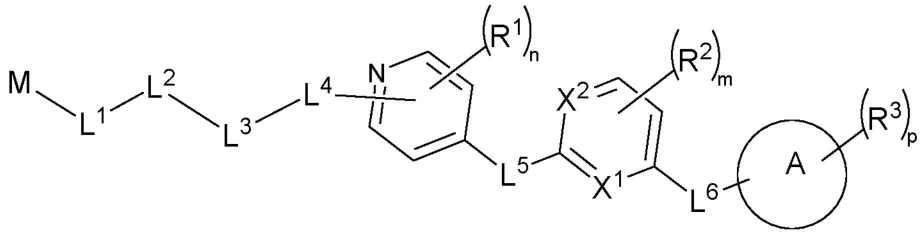

V-4,

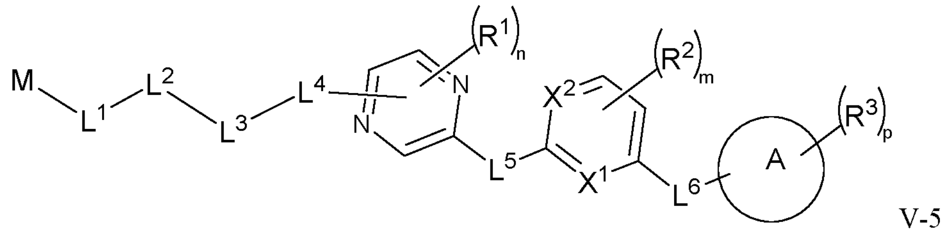

V-5,

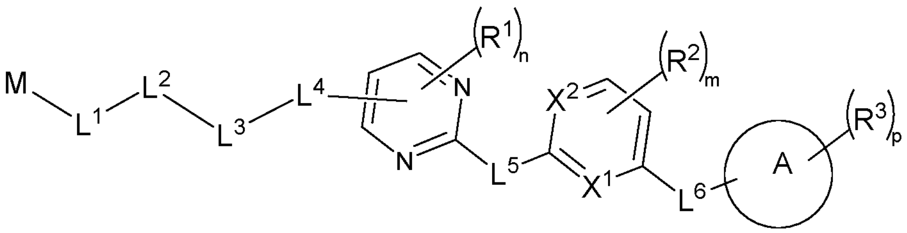

V-6,

or

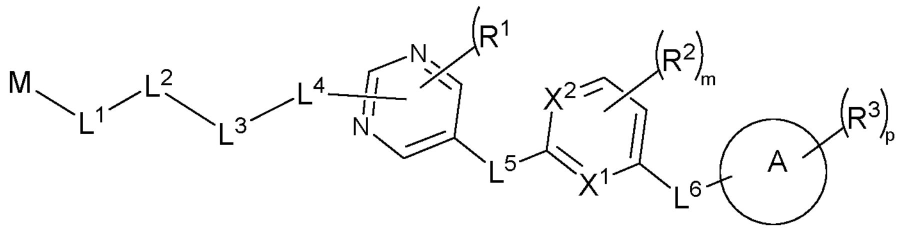

V-7.

In some examples, the compound of the present application is a compound of any one of formulae VI-1 to VI-5, or a stereoisomer, a tautomer, an enantiomer, a diastereomer, a racemate, a geometric isomer, a nitrogen oxide, a solvate, a hydrate, a crystal form, an ester, an isotope labeled compound (preferably a deuterate), a metabolite, a pharmaceutically acceptable salt, or a prodrug thereof:

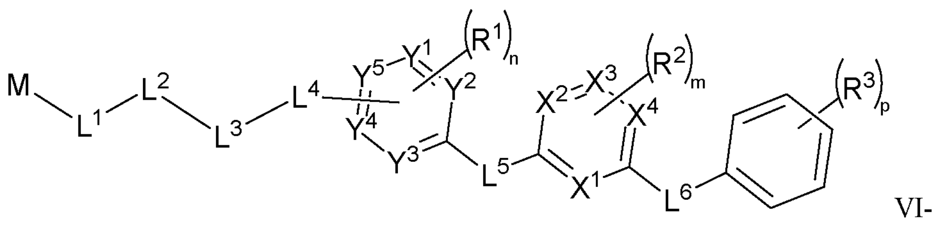

VI-1,

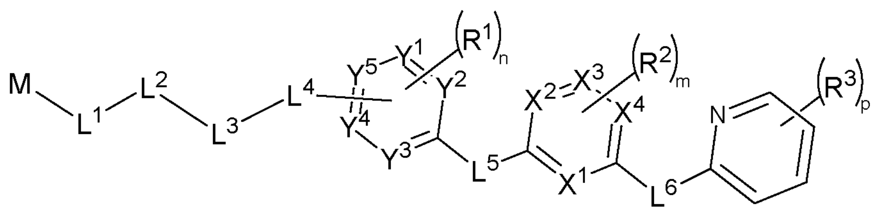

VI-2,

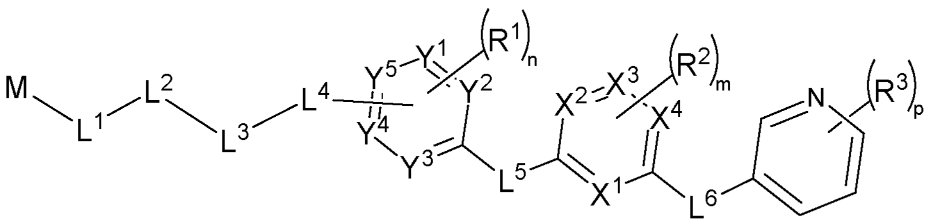

VI-3,

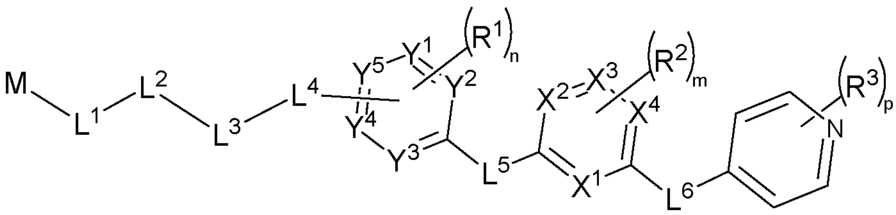

VI-4,

or

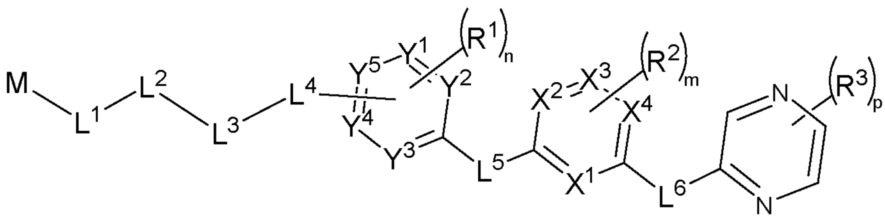

VI-5.

In some examples, the compound of the present application is a compound of any one of formulae VII-1 to VII-6, or a stereoisomer, a tautomer, an enantiomer, a diastereomer, a racemate, a geometric isomer, a nitrogen oxide, a solvate, a hydrate, a crystal form, an ester, an isotope labeled compound (preferably a deuterate), a metabolite, a pharmaceutically acceptable salt, or a prodrug thereof:

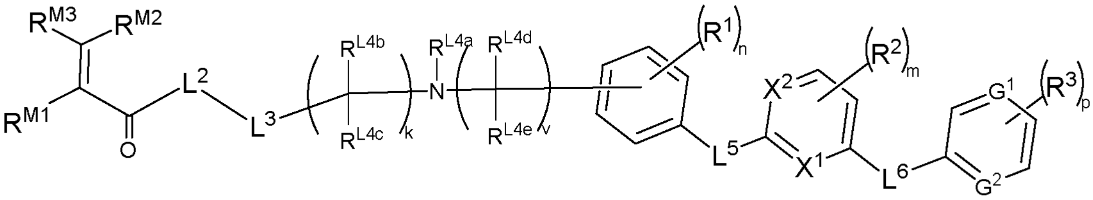

VII-1,

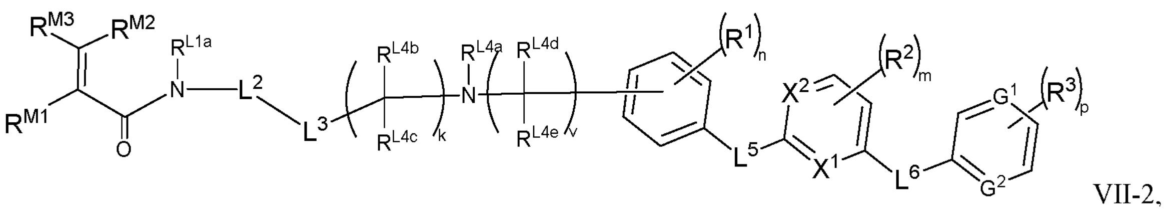

VII-2,

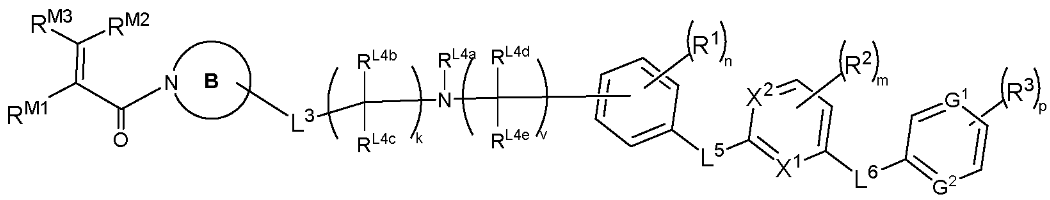

VII-3.

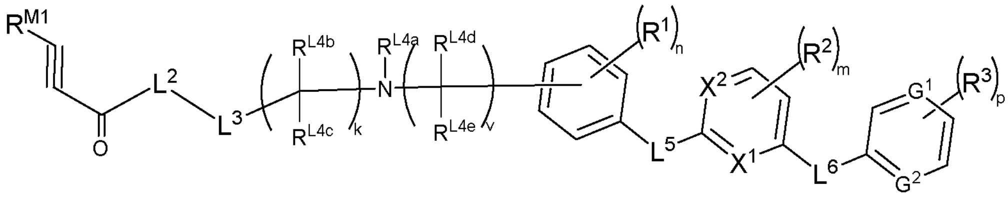

VII-4,

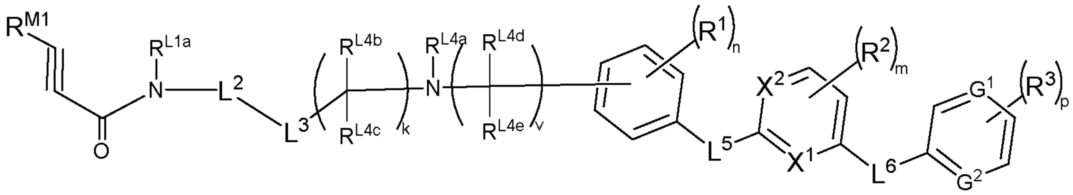

VII-5,

or

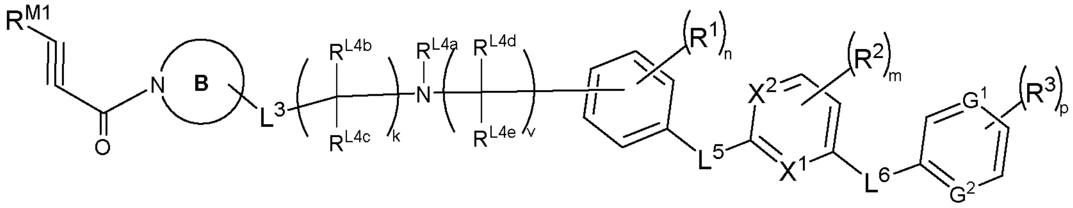

VII-6;

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of hydrogen (such as deuterium), hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; $G^1$ and $G^2$ each are independently CH or N; k and v each are independently 0, 1, or 2.

In some examples, the compound of the present application is a compound of any one of formulae VIII-1 to VIII-6, or a stereoisomer, a tautomer, an enantiomer, a diastereomer, a racemate, a geometric isomer, a nitrogen oxide, a solvate, a hydrate, a crystal form, an ester, an isotope labeled compound (preferably a deuterate), a metabolite, a pharmaceutically acceptable salt, or a prodrug thereof:

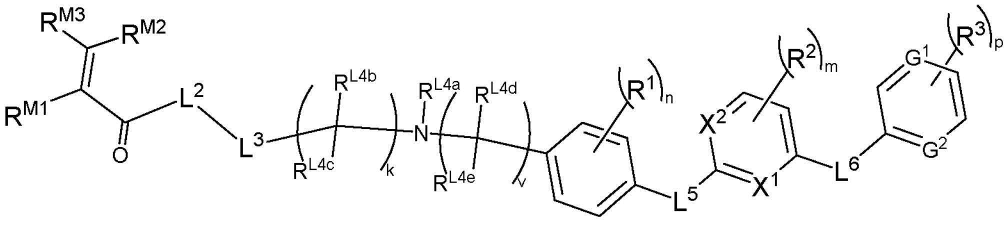

VIII-1,

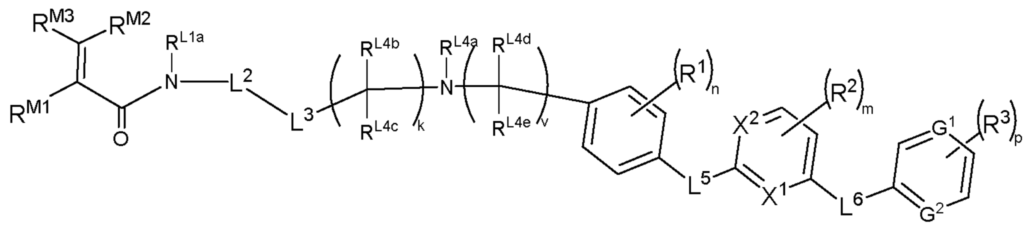
VIII-2,

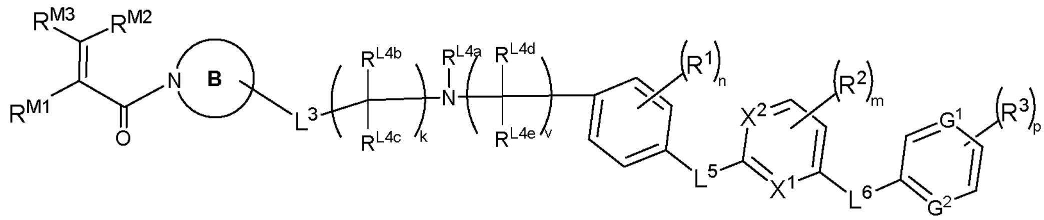
VIII-3,

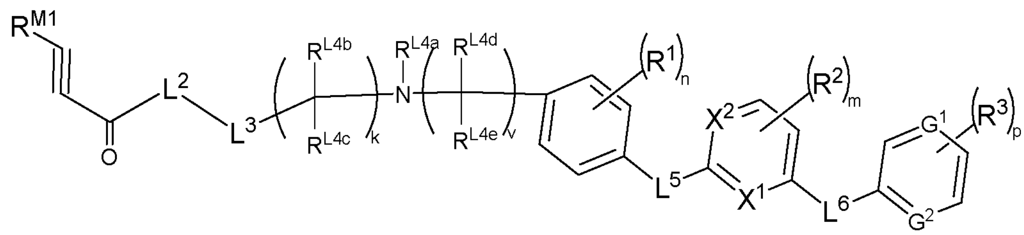
VIII-4,

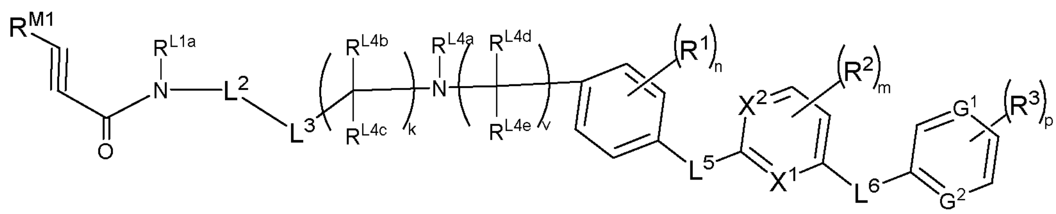
VIII-5,

or

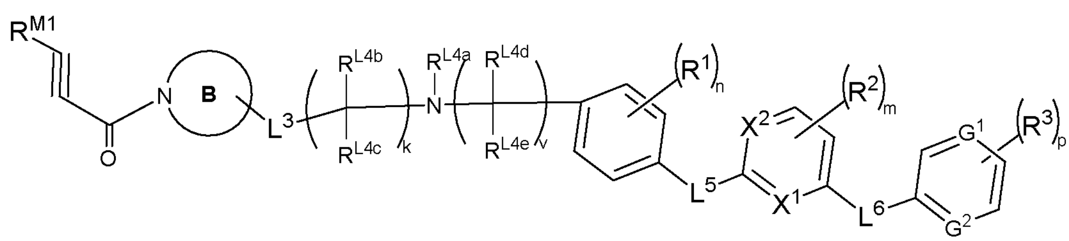
VIII-6;

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; $G^1$ and $G^2$ each are independently CH or N; k and v each are independently 0, 1, or 2.

The present application also relates to a method for preparing, isolating, and purifying the compound of the present application.

The present application also relates to a method for preparing the compound of the present application, which

comprises one or more of steps (1) to (3) as follows:

(1) reacting the compound of formula a1 with compound a2 to produce the compound of formula a3; (2) reacting the compound of formula a3 with compound a4 to produce the compound of formula a5; (3) reacting the compound of formula a5 with the compound of formula a6 to produce the compound of formula a7;

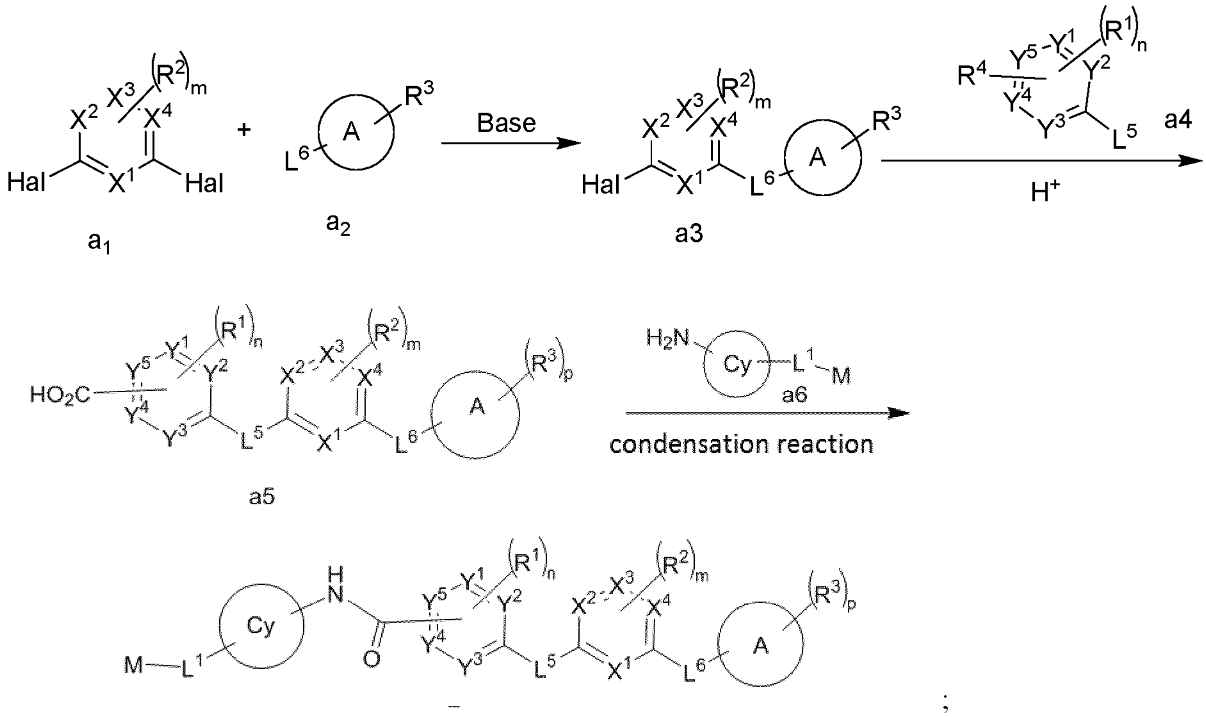


$a_1$ $a_2$ a3 a5

;

Alternatively, the preparation method comprises one or more of steps (4) to (9) as follows:

(4) reacting the compound of formula b1 with the compound of formula b2 to produce the compound of formula b3; (5) converting the compound of formula b3 into the compound of formula b4; (6) converting the compound of formula b4 into the compound of formula b5; (7) reacting the compound of formula b5 with the compound of formula b3 to produce the compound of formula b6; (8) converting the compound of formula b6 into the compound of formula b7; (9) converting the compound of formula b7 into the compound of formula b8,

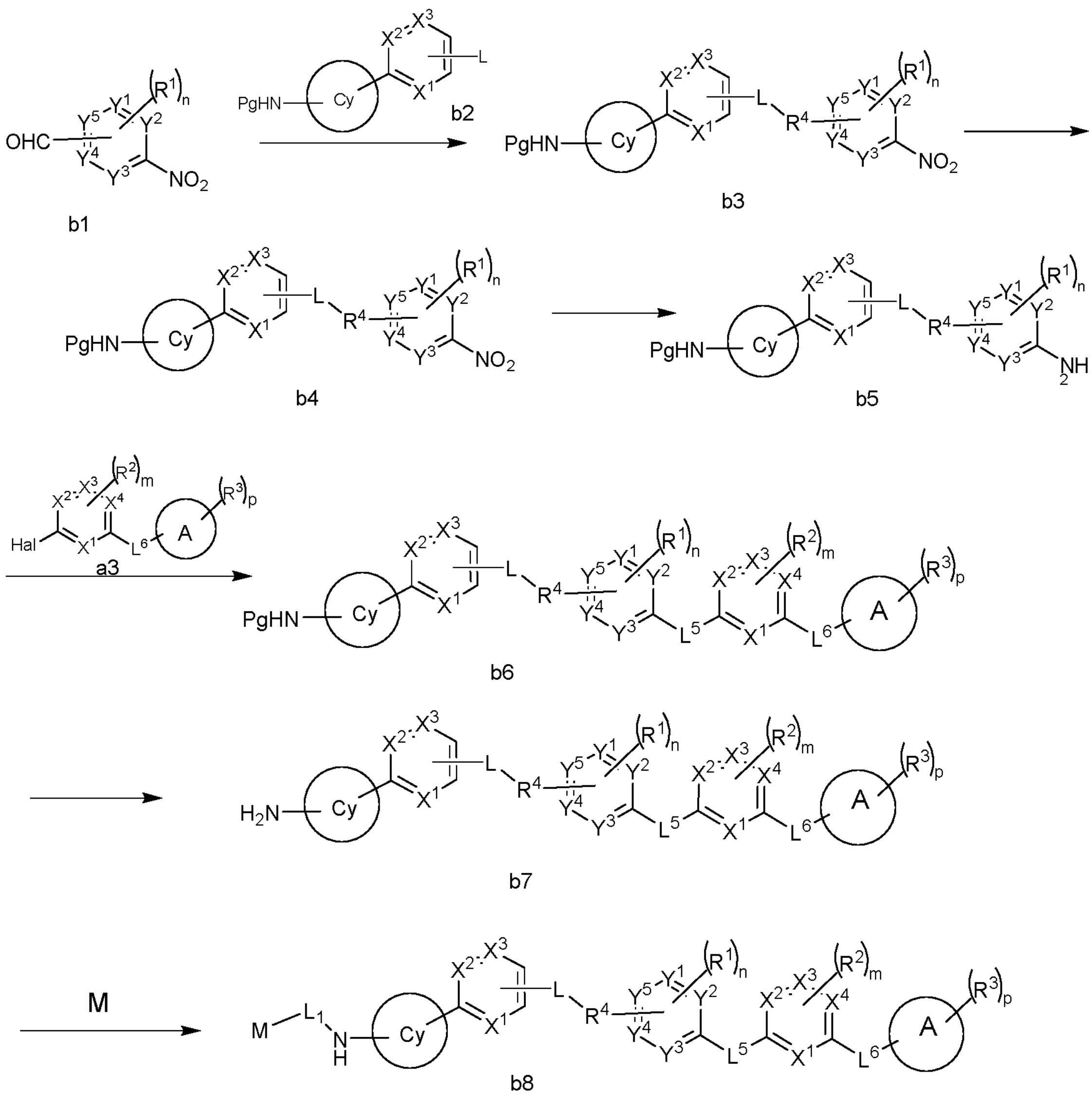


b1, b2, a3, b3, b4, b5, b6, b7, b8

wherein Hal is halogen, Cy represents a structure of various ring systems (including but not limited to 3-6 membered saturated or partially unsaturated carbocyclyl, 3-6 membered saturated or partially unsaturated heterocyclyl, 6-10 membered aryl, or 5-10 membered heteroaryl), Pg represents a protecting group; A, $R^1$, $R^2$, $R^3$, $R^4$, $X^1$, $X^2$, $X^3$, $X^4$, $Y^1$, $Y^2$, $Y^3$, $Y^4$, $Y^5$, $L^1$, $L^2$, $L^5$, $L^6$, M, n, m, and p are defined as described in the present application.

In another aspect, the present application relates to the preparation of intermediates for compounds represented by formulae I, II-1 to II-6, III, IV-1 to IV-6, V-1 to V-6, VI-1 to VI-5, VII-1 to VII-6, and VIII-1 to VIII-6.

In another aspect, the present application provides a pharmaceutical composition comprising a compound of the present application and a pharmaceutically acceptable excipient thereof.

In another aspect, the present application provides use of the compound or pharmaceutical composition described herein in the preparation of medicaments for the prevention or treatment of FAK related diseases.

In another aspect, the present application provides use of the compound or pharmaceutical composition described herein for the prevention or treatment of FAK related diseases.

In another aspect, the present application provides a method for preventing or treating FAK related diseases using a compound or pharmaceutical composition of the present application.

In another aspect, the present application provides a method of administering an effective amount of the compound or pharmaceutical composition of the present application to prevent or treat FAK related diseases.

In some embodiments, the FAK related disease includes cancer, pulmonary arterial hypertension, and pathological angiogenesis.

In some embodiments, FAK related diseases include lung cancer, bone cancer, pancreatic cancer, skin cancer, head and neck cancer, skin or intraocular melanoma, uterine cancer, ovarian cancer, rectal cancer, cancer in the anal

region, gastric cancer, colon cancer, breast cancer, fallopian tube cancer, endometrial cancer, cervical cancer, sheath cancer, vulva cancer, Hodgkin's disease, esophageal cancer, small intestine cancer, endocrine system cancer, thyroid cancer, parathyroid cancer, adrenal cancer, soft tissue sarcoma, urethral cancer, penis cancer, prostatic cancer, chronic or acute leukemia, lymphocytic lymphoma, bladder cancer, kidney or ureteral cancer, renal cell carcinoma, renal pelvis carcinoma, central nervous system (CNS) tumor, primary CNS lymphoma, spinal axis carcinoma, brain stem glioma, and pituitary adenoma or the combination of one or more of above cancers.

In another aspect, the present application provides a method for regulating the Hippo-YAP signaling pathway, comprising administering the compound or pharmaceutical composition of the present application.

In another aspect, the present application provides use of the compound or pharmaceutical composition described herein in the preparation of inhibitor of Hippo-YAP signaling pathway.

In another aspect, the present application provides use of the compound or pharmaceutical composition described herein in the preparation of medicaments for the prevention or treatment of YAP related diseases.

In another aspect, the present application provides use of the compound or pharmaceutical composition described herein for the prevention or treatment of YAP related diseases.

In another aspect, the present application provides a method for preventing or treating YAP related diseases, comprising administering an effective amount of the compound or pharmaceutical composition of the present application.

In some embodiments, the YAP related disease is selected from cancer. In some embodiments, the YAP related disease includes the combination of one or more of skin cancer, bone cancer, glioma, breast cancer, adrenal cancer, bladder cancer, esophageal cancer, head or neck cancer, liver cancer, parathyroid cancer, penis cancer, small intestine cancer, thyroid cancer, urethral cancer, cervical cancer, endometrial cancer, fallopian tube cancer, renal pelvis cancer, vaginal cancer, vulva cancer, chronic or acute leukemia, colon cancer, melanoma, hematological malignancy, Hodgkin's lymphoma, lung cancer, lymphocytic lymphoma, central nervous system (CNS) tumor, ovarian cancer, pancreatic cancer, pituitary adenoma, prostatic cancer, soft tissue sarcoma, gastric cancer, and uterine cancer.

In another aspect, the present application provides a method for inhibiting FAK kinase and/or YAP protein activity in cells or subjects, comprising a step of contacting the cell with the compound or pharmaceutical composition as described herein or administering the compound or pharmaceutical composition as described herein to the subject.

In some embodiments, the cells are mammalian cells. In some embodiments, the subjects are mammals, preferably humans.

## DETAILED DESCRIPTION OF INVENTION

Now provide a detailed description of certain embodiments of the present application, with examples explained by the accompanying structural or chemical formulae. the present application is intended to cover all alternative, modified, and equivalent technical solutions, all of which are within the scope of the present application as defined in claims. Those skilled in the art should recognize that many methods and materials similar or equivalent to those described herein can be used to practice the present application. the present application is not limited to the methods and materials described herein. If one or more of the literature, patents, and similar materials incorporated are different or contradictory to this application (including but not limited to defined terms, terminology applications, described technology, etc.), this application shall prevail.

It should be further recognized that certain features of the present application have been described in multiple independent embodiments for clarity, but can also be provided in combination in a single embodiment. On the contrary, the various features of the present application have been described in a single embodiment for simplicity, but can also be provided separately or in any suitable sub combination.

### Definition and General Terms

Unless otherwise stated, all technical terms used herein have the same meanings as those commonly understood by those skilled in the art to which the present application belongs. All patents and publications related to the present application are incorporated herein by reference in its entirety.

Unless otherwise stated or there is a clear conflict in the context, the articles "a", "an", and "the" used herein are intended to encompass "at least one" or "one or more". Therefore, the articles used herein refer to articles of one or more (i.e. at least one) objects. For example, "a component" refers to one or more components, that is, there may be more than one component considered for adoption or use in the implementation of the embodiment.

The term "one or more" used herein refers to one or more than one. In some examples, "one or more" represents 1-5; in some examples, "one or more" represents 1-4; in some examples, "one or more" represents 1-3; in some examples, "one or more" represents 1-2; in some examples, "one or more" represents one; in other examples, "one or more" represents 1, 2, 3, 4, or 5.

The term "subject" used herein refers to animals. Typically, the animal is a mammal. The term "subject" also

refer to, such as primates (such as humans, males or females), cows, sheep, goats, horses, dogs, cats, rabbits, rats, mice, fish, birds, etc. In some embodiments, the subject is primates. In other embodiments, the subject is a human.

The term "patient" used herein refers to humans (including adults and children) or other animals. In some embodiments, "patient" refers to a human.

The term "comprise" or "include" used herein is an open-ended expression that includes the contents specified in the present application, but does not exclude contents of other aspects.

Unless otherwise explicitly stated, the phrases "each ... independently", "... each independently" and "... independently" used in the present application are interchangeable and should be broadly understood, which indicate that in different groups, the specific options expressed by the same symbols do not affect each other, or in the same group, the specific options expressed by the same symbols do not affect each other.

Unless otherwise explicitly stated, the phrases "optionally" or "arbitrarily" used herein refer to subsequent actions that may occur or may not occur. For example, "optionally substituted", "arbitrarily substituted", "optionally substituted with... " indicate that the group can be substituted or not substituted. Unless otherwise indicated, an optional substituent can substitute at various substitutable positions of the group.

Unless otherwise explicitly stated, when "each ... independently", "... each independently" and "... independently" can be used in combination with "optionally" or "arbitrarily", the combination does not need to consider the order and has the same meaning. For example, "each optionally ... independently" and "optionally ... independently" indicate that in different groups, the specific options expressed by the same symbol do not affect each other, and subsequent actions may occur or may not occur.

The term "$C_{m-n}$" used as a prefix alone refers to any group having m to n carbon atoms, while $C_m$ refers to any group having m carbon atoms. For example, $C_{1-20}$alkyl refers to an alkyl group containing 1 to 20 carbon atoms; $C_{1-8}$alkoxy group refers to an alkoxy group containing 1 to 8 carbon atoms; $C_{2-6}$alkenyl refers to an alkenyl group containing 2 to 6 carbon atoms; $C_6$haloalkyl refers to a haloalkyl group containing 6 carbon atoms.

The term "m-n membered" used as a prefix alone refers to a ring having m to n ring atoms, such as "3-6 membered saturated or partially unsaturated carbocyclyl" represents carbocyclyl having 3 to 6 ring atoms, "3-6 membered saturated or partially unsaturated heterocyclyl" represents heterocyclyl having 3 to 6 ring atoms; "6-10 membered aryl" represents aryl having 6 to 10 ring atoms; "5-10 membered heteroaryl" represents heteroaryl having 5 to 10 ring atoms.

The term "replaced" used herein indicates that one or more carbon units in a given structure have been replaced by a specific replacement group. When more than one carbon unit in a given structural formula can be replaced by one or more replacement groups selected from specific groups, the replacement groups can occur at various positions and are the same or different. The replacement groups described therein may be, but are not limited to, -O-, -S-, $-NR^{L7a}-$, $-NR^{L7a}C(=O)-$, $-C(=O)NR^{L7a}-$, -SC(=O)-, -C(=O)S-, -OC(=O)-, -C(=O)O-, $-NR^{L7a}C(=S)-$, $-C(=S)NR^{L7a}-$, *trans* $CR^{L7b}=CR^{L7b}-$, *cis* $CR^{L7b}=CR^{L7b}-$, -C≡C-, -S(=O)-, -S(=O)O-, -OS(=O)-, $-S(=O)NR^{L7a}-$, $-NR^{L7a}S(=O)-$, $-S(=O)_2-$, $-S(=O)_2O-$, $-OS(=O)_2-$, $-S(=O)_2NR^{L7a}-$, or $-NR^{L7a}S(=O)_2-$, wherein $R^{L7a}$ is hydrogen, deuterium, $C_{1-4}$alkyl, or a nitrogen protecting group; $R^{L7b}$ is hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two $R^{L7b}$ groups arbitrarily connected to form 3-6 membered saturated or partially unsaturated carbocyclyl or 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in $L^7$, $R^{L7a}$ and $R^{L7b}$ each arbitrarily are independently substituted by one or more substituents selected from the group consisting of deuterium, halogen, hydroxyl, amino, cyano, oxo, and $C_{1-4}$alkyl;

The term "substitute" or "substituted" used herein indicates that one or more hydrogen atoms in a given structure have been replaced by a specific substituent. When more than one position in a given structure formula can be replaced by one or more substituents selected from specific groups, the substituent can occur at various positions and are the same or different. The substituents described therein may be, but are not limited to, deuterium, fluoro, chloro, bromo, iodo, amino, hydroxyl, carboxyl, amido, aminoacyl, sulfonyl, cyano, oxo(=O), nitro, $C_{1-6}$alkyl, $C_{2-6}$alkenyl, $C_{2-6}$alkynyl, $C_{2-6}$alkenyloxy, $C_{1-6}$alkoxyl, $C_{1-6}$alkylamino, di($C_{1-6}$alkyl)amino, $C_{1-6}$haloalkyl, 3-12 membered heterocyclyl, 3-10 membered carbocyclyl, $C_{6-10}$aryl, $C_{1-9}$heteroaryl, 3-12 membered heterocyclyl$C_{1-6}$alkyl, $C_{6-10}$aryl$C_{1-6}$alkyl, $C_{1-9}$heteroaryl$C_{1-6}$alkyl, $C_{1-6}$hydroxyalkyl, $C_{1-6}$alkoxy$C_{1-6}$alkyl, etc.

The present application uses a certain "group involved in a certain Markush variable" in some group descriptions to distinguish the same group in different Markush variables, such as "alkyl involved in $R^{L1a}$", which refers to the alkyl defined in $R^{L1a}$. When the alkyl uses the prefix "$C_{m-n}$", i.e. $C_{m-n}$ alkyl, for simplicity, "$C_{m-n}$ alkyl involved in $R^{L1a}$" is written as "alkyl involved in $R^{L1a}$", and "alkyl involved in $R^{L1a}$" refers to $C_{m-n}$ alkyl defining $R^{L1a}$.

The term "stereoisomer" used herein refers to compounds having the same chemical structure but with different spatial arrangements of atoms or groups. Stereoisomers include enantiomers, diastereomers, conformational isomers (rotational isomers), geometric isomers (*cis*/*trans* isomers), hindered isomers, and so on. Unless otherwise indicated, all stereoisomers or mixtures of stereoisomers of the structural formula described in the present application fall within the scope of the present application. The term "enantiomer" used herein refers to stereoisomers that are mutually an

actual object and a mirror image that cannot overlap. The term "diastereomer" refers to a stereoisomer of a compound having two or more chiral centers that is not a mirror image of another stereoisomer of the same compound.

The term "geometric isomer" used herein includes: *cis*/*trans* isomers, homostereoisomeric polymers and metastereoisomeric polymers, rotational isomers caused by steric hindrance, etc.

The definition and rules of stereochemistry used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994.

Any mixture of stereoisomers obtained can be separated into pure or essentially pure geometric isomers, enantiomers, and diastereomers based on differences in the physical and chemical properties of the components, for example, by chromatography and/or fractional crystallization.

The term "tautomer" or "tautomeric form" used herein refers to structural isomers having different energies that can be converted to each other through low energy barriers. If tautomerism is possible (such as in a solution), chemical equilibrium of the tautomers can be achieved. For example, proton tautomers (also known as prototropic tautomer) include mutual transformations that occur through proton transfer, such as keto-enol tautomerism and imine-enamine tautomerism. Valence tautomers involve mutual transformations through the recombination of some bond-formation electrons. A specific example of keto-enol tautomerism is the tautomerism of pentane-2,4-dione and 4-hydroxy-pentan-3-ene-2-one. Another example of tautomerism is phenol-keto tautomerism. A specific example of phenol-keto tautomerism is the tautomerization of pyridine-4-ol and pyridine-4(1H)-one. Unless otherwise detailed, all tautomeric forms of the compounds of the present application are within the scope of the present application.

The term "nitrogen oxide" used in the present application refers to such a compound that, when it contains several amine functional groups, one or more nitrogen atoms are oxidized to form N-oxides. Special examples of N-oxides are N-oxides of tertiary amines or N-oxides of a nitrogen atom in nitrogen-containing heterocycles. Oxidants such as hydrogen peroxide or peracid (such as peroxycarboxylic acid) can be used to treat corresponding amines to form N-oxides (see Advanced Organic Chemistry, WileyInterscience, 4th edition, Jerry March, pages). Especially, N-oxides can be prepared according to a method of L. W. Deady (Syn. Comm. 1977, 7509-514), for example, an amine compound is reacted with m-chloroperoxybenzoic acid (MCPBA) in an inert solvent such as dichloromethane.

The term "solvate" used herein refers to an associate formed by one or more solvent molecules and a compound of the present application. A solvent used to form solvates includes, but not limited to, water, isopropanol, ethanol, methanol, dimethyl sulfoxide, ethyl acetate, acetic acid, and aminoethanol. The term "hydrate" refers to a associate derived from water as a solvent molecule.

The term "metabolite" used herein refers to a product resulted from metabolism of a specific compound or its salt in the body. The metabolite of a compound can be identified by well-known techniques in the art, and its activity can be characterized by experimental methods as described in the present application. Such products can be obtained through oxidation, reduction, hydrolysis, amidation, desamidation, esterification, deesterification, enzymatic cleavage, and other processes of the administered compound. Correspondingly, the present application encompasses metabolites of the compound, including metabolites produced by contacting the compounds of the present application with mammals sufficiently for a period of time.

The term "ester" includes compounds or fragments containing carbon or heteroatoms bonded to an oxygen atom that is bonded to a carbon of a carbonyl group. The term "ester" includes alkoxycarbonyl groups, such as methoxycarbonyl, ethoxycarbonyl, propoxycarbonyl, butoxycarbonyl, pentoxycarbonyl, etc.

It should be understood that the compounds of the present application can be described as different tautomers. It should also be understood that when a compound has tautomeric forms, all tautomeric forms are intended to be encompassed within the scope of the present application, and the nomenclature of the compound does not exclude any tautomeric forms. It should be understood that certain tautomers may have a higher level of activity than other tautomers.

The term "crystal form" refers to crystalline structures of a compound (or its salts or solvates) crystallized in different crystal stacking arrangements, all of which have the same elemental composition. Different crystal forms typically exhibit different X-ray diffraction patterns, infrared spectra, melting points, densities, hardness, crystal shapes, optical and electrical properties, stabilities, and solubilities. Recrystallization solvents, crystallization rate, storage temperature and other factors may lead to one crystal form that dominates. The crystal form of a compound can be obtained through crystallization under different conditions.

The term "isotope labeled" refers to a procedure of replacing an element in a molecule of a compound with a corresponding isotope. "Isotope" refers to the same element with different masses but identical chemical properties. The method of replacing an atom in a molecule with its isotope is called as isotope labeling.

The term "pharmaceutically acceptable" used herein refers to such compounds, raw materials, compositions, and/or dosage forms that are within reasonable medical judgment, suitable for contact with tissues of a patient, without excessive toxicity, irritation, allergic reactions, or other issues and complications that are commensurate with reasonable benefit/risk ratios, and effectively used for their intended use.

The term "pharmaceutically acceptable salt" used herein refers to organic and inorganic salts of the compound

of the present application. Pharmaceutically acceptable salts are well-known in the art, such as pharmaceutically acceptable sales described in detail in reference S. M. Berge et al., J. Pharmaceutical Sciences, 1977, 66:1-19. The salts formed from non-toxic pharmaceutically acceptable acid include, but are not limited to, inorganic salts such as hydrochloride, hydrobromate, phosphate, sulfate, perchlorate, and organic salts such as acetate, oxalate, maleic acid, tartrate, citrate, succinate, malonate, or salts obtained through other methods, such as ion exchange recorded in books and literature. Other pharmaceutically acceptable salts include adipate, alginate, ascorbate, aspartate, benzenesulfonate, benzoate, bisulfate, borate, butyrate, camphorate, camphorsulfonate, cyclopentyl propionate, digluconate, dodecylsulfate, ethanesulfonate, formate, fumarate, gluconate, glycerophosphate, gluconate, hemisulfate, heptaneate, hexanoate, hydroiodate, 2-hydroxy-ethanesulfonate, lacturonate, lactate, lauryl sulfate, malate, malonate, methanesulfonate, 2-naphthalene sulfonate, niacinate, nitrate, oleate, palmitic acid, pamoate, pectinate, persulfate, 3-phenylpropionate, picrate, valerate, propionate, stearate, thiocyanate, p-toluenesulfonate, undecanoate, valerate, and so on. The salts obtained from appropriate bases include salts of alkali metals, alkali earth metals, ammonium and $N^+(R)_4$, such as R is H, $C_{1-4}$alkyl, $C_{6-10}$aryl, etc., and salts of cyclic ammonia, such as pyridine, morpholine, and piperazine. the present application also is intended to conceive quaternary ammonium salts formed by any compound containing groups of N atoms. Water soluble, oil soluble, or dispersed products can be obtained through quaternization. Alkali metal salts or alkaline earth metal salts include salts of sodium, lithium, potassium, calcium, magnesium, and so on. Pharmaceutically acceptable salts further include appropriate, non-toxic ammonium, and amine cations formed by quaternary ammonium salts and counter ions, such as halides, hydroxides, carboxylates, sulfates, phosphates, nitrates, $C_{1-8}$sulfonates, and aromatic sulfonates.

The term "prodrug" used in the present application represents a compound that will transform *in vivo* into the compound of formulae I, II-1 to II-6, III, IV-1 to IV-6, V-1 to V-6, VI-1 to VI-5, VII-1 to VII-6, VIII-1 to VIII-6. This transformation is affected by the hydrolysis of precursor drugs in the blood or enzymatic conversion into the parent structure in the blood or tissue. The prodrug of the present application can be esters. In existing technology, esters that can be used as prodrugs include phenylesters, aliphatic($C_{1-24}$)esters, acyloxymethyl esters, carbonates, carbamate esters, and amino acid esters. For example, if the compound in the present application contains hydroxyl groups, it can be acylated to form a compound in a prodrug form. Other prodrug forms include phosphate, such as those obtained by phosphorylation of hydroxyl groups in the parent molecule. A complete discussion on prodrugs can refer to the following literatures: T. Higuchi and V Stella, Pro-drugs as Novel Delivery Systems, Vol. 14 of the A. C. S. Symposium Series, Edward B. Roche, ed., Bioreversible Carriers in Drug Design, American Pharmaceutical Association and Pergamon Press, 1987, J. Rautio et al, Prodrugs: Design and Clinical Applications, Nature Review Drug Discovery, 2008, 7, 255-270, and S. J. Hecker et al, Prodrugs of Phosphates and Phosphonates, Journal of Medicinal Chemistry, 2008, 51, 2328-2345.

As used in the present application, the term "treat" or "treatment" refers to the improvement of a disease or condition (i.e. slowing or preventing or alleviating the development of the disease or at least one clinical symptom thereof). In some embodiments, the term "treat" or "treatment" refers to alleviation or improvement of at least one bodily parameter, including bodily parameters that may not be perceived by a patient. In other embodiments, the term "treat" or "treatment" refers to regulating a disease or condition from a physical aspect (such as stable and perceptible symptoms) or a physiological aspect (such as stable body parameters) or both. In other embodiments, the term "treat" or "treatment" refers to preventing or delaying the onset, occurrence, or deterioration of a disease or condition.

In various parts of this specification, the substituents of the present compounds are disclosed according to the type or range of functional groups. It is particularly pointed out that the present application encompasses each independent sub-combination of separate members of these functional group types and ranges. For example, the term "$C_{1-6}$alkyl" particularly refers to methyl, ethyl, $C_3$alkyl, $C_4$alkyl, $C_5$alkyl and $C_6$alkyl disclosed separately.

In various parts of this specification, a connecting group is described. When the structure clearly requires a connecting group, the Markush variables listed for that connecting group should be understood as a connecting group. For example, if a structure requires a connecting group and the Markush group definition for the variable lists "alkyl" or "aryl", it should be understood that "alkyl" or "aryl" respectively represent the connected group of alkylene or arylene.

The term "covalent warhead" refers to a group that can interact with a specific target protein to form a covalent bond, and includes but not limited to:

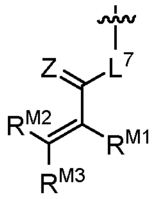

i-1

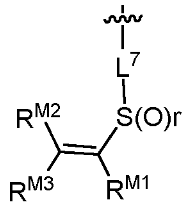

i-2

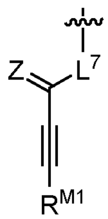

i-3

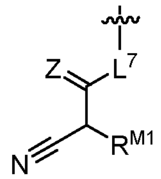

i-4

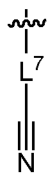

i-5

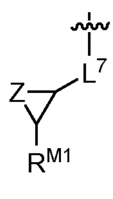 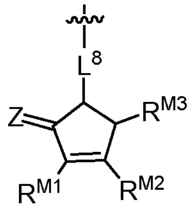 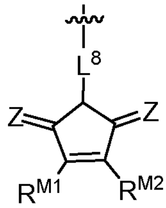 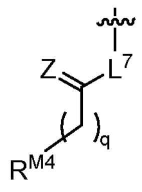 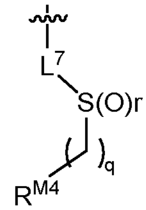

i-6 i-7 i-8 i-9 i-10

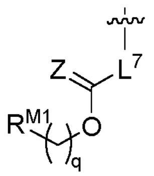 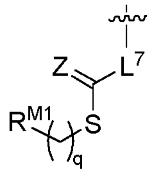 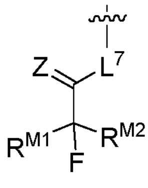 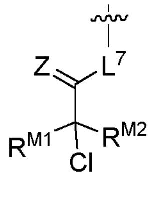 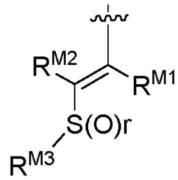

i-11 i-12 i-13 i-14 i-15

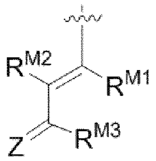 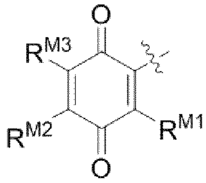 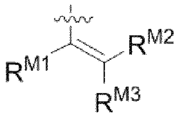 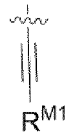 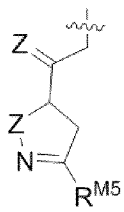

i-16 i-17 i-18 i-19 or i-20

;

wherein,

$L^7$ is a bond, -O-, -S-, $-NR^{L7a}-$ or $C_{1-4}$alkyl, wherein, one or more carbon units of the $C_{1-4}$alkyl group optionally are independently replaced with -O-, -S-, $-NR^{L7a}-$, $-NR^{L7a}C(=O)-$, $-C(=O)NR^{L7a}-$, -SC(=O)-, -C(=O)S-, -OC(=O)-, -C(=O)O-, $-NR^{L7a}C(=S)-$, $-C(=S)NR^{L7a}-$, *trans* $CR^{L7b}=CR^{L7b}-$, *cis* $CR^{L7b}=CR^{L7b}-$, -C≡C-, -S(=O)-, -S(=O)O-, -OS(=O)-, $-S(=O)NR^{L7a}-$, $-NR^{L7a}S(=O)-$, $-S(=O)_2-$, $-S(=O)_2O-$, $-OS(=O)_2-$, $-S(=O)_2NR^{L7a}-$, or $-NR^{L7a}S(=O)_2-$; wherein each $R^{L7a}$ is independently hydrogen, $C_{1-4}$alkyl, or a nitrogen protecting group; each $R^{L7b}$ is independently hydrogen, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two adjacent $R^{L7b}$ groups bind with their connected atoms to form 3-6 membered saturated or partially unsaturated carbocyclyl or 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $L^7$, $R^{L7a}$ and $R^{L7b}$ each optionally are independently substituted by one or more substituents selected from the group consisting of hydrogen (such as deuterium), halogen, hydroxyl, amino, cyano, oxo, and $C_{1-4}$alkyl;

$L^8$ is a bond or $C_{1-4}$alkyl, wherein the alkyl involved in $L^8$ optionally is substituted by one or more substituents selected from the group consisting of hydrogen (such as deuterium), halogen, hydroxyl, amino, cyano, and oxo;

each $R^{M1}$ is independently hydrogen, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, $-CH_2OR^{M1a}$, $-CH_2N(R^{M1a})_2$, $-CH_2SR^{M1a}$, $-OR^{M1a}$, $-N(R^{M1a})_2$, $-Si(R^{M1a})_3$ or $-SR^{M1a}$, wherein each $R^{M1a}$ is independently hydrogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl; or two $R^{M1a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M1}$ and $R^{M1a}$ each optionally are independently substituted by one or more substituents selected from the group consisting of hydrogen (such as deuterium), halogen, hydroxyl, amino, cyano, oxo, and $C_{1-4}$alkyl;

each $R^{M2}$ is independently hydrogen, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, $-CH_2OR^{M2a}$, $-CH_2N(R^{M2a})_2$, $-CH_2SR^{M2a}$, $-OR^{M2a}$, $-N(R^{M2a})_2$ or $-SR^{M2a}$, wherein each $R^{M2a}$ is independently hydrogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl; or two $R^{M2a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M2}$ and $R^{M2a}$ each optionally are independently substituted by one or more substituents selected from the group consisting of hydrogen (such as deuterium), halogen, hydroxyl, amino, cyano, oxo, and $C_{1-4}$alkyl;

each $R^{M3}$ is independently hydrogen, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, $-CH_2OR^{M3a}$, $-CH_2N(R^{M3a})_2$, $-CH_2SR^{M3a}$, $-OR^{M3a}$, $-N(R^{M3a})_2$ or $-SR^{M3a}$, wherein each $R^{M3a}$ is independently hydrogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl; or two $R^{M3a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M3}$ and $R^{M3a}$ each optionally are independently substituted by one or more substituents selected from the group consisting of hydrogen (such as deuterium), halogen, hydroxyl, amino, cyano, oxo, and $C_{1-4}$alkyl;

Optionally, $R^{M1}$ and $R^{M3}$, or $R^{M2}$ and $R^{M3}$, or $R^{M1}$ and $R^{M2}$ optionally connect to form saturated or partially unsaturated carbocyclyl or saturated or partially unsaturated heterocyclyl, wherein saturated or partially unsaturated carbocyclyl or saturated or partially unsaturated heterocyclyl each optionally are independently substituted by one or more substituents selected from the group consisting of hydrogen (such as deuterium), halogen, hydroxyl, amino, cyano, oxo, and $C_{1-4}$alkyl;

$R^{M4}$ is a leaving group; $R^{M5}$ is halogen; Z is O, S, or $NR^Z$; wherein $R^Z$ is hydrogen, $C_{1-4}$alkyl, or a nitrogen protecting group; alkyl involved in $R^Z$ optionally is substituted by one or more substituents selected from the group consisting of hydrogen (such as deuterium), halogen, hydroxyl, amino, cyano, and oxo; q is 0, 1, 2, 3, 4, 5 or 6; r is 1 or 2.

The term "alkyl" or "alkyl group" represents a saturated linear or branched hydrocarbyl, where the alkyl can be optionally substituted by one or more substituents described in the present application. In some embodiments, alkyl is $C_{1-20}$alkyl; in some embodiments, alkyl is $C_{1-10}$alkyl; in another embodiment, alkyl is $C_{1-6}$alkyl; in yet another embodiment, alkyl is $C_{1-4}$alkyl; in yet another embodiment, alkyl is $C_{1-3}$alkyl. In some specific structures, when the alkyl group is clearly represented as a connecting group, the alkyl group represents the connected alkylene, for example, $C_{1-6}$alkyl in the structure formula ($C_{6-10}$aryl)-($C_{1-6}$alkyl)- should be understood as $C_{1-6}$alkene. Examples of alkyl include, but are not limited to, methyl (Me, $-CH_3$), ethyl (Et, $-CH_2CH_3$), n-propyl (n-Pr, $-CH_2CH_2CH_3$), isopropyl (*i*-Pr, $-CH(CH_3)_2$), n-butyl (n-Bu, $-CH_2CH_2CH_2CH_3$), 2-methylpropyl or *i*-butyl (i-Bu, $-CH_2CH(CH_3)_2$), 1-methylpropyl or s-butyl (s-Bu, $-CH(CH_3)CH_2CH_3$), *t*-butyl (*t*-Bu, $-C(CH_3)_3$), n-pentyl ($-CH_2CH_2CH_2CH_2CH_3$), 2-pentyl ($-CH(CH_3)CH_2CH_2CH_3$), 3-pentyl ($-CH(CH_2CH_3)_2$), 2-methyl-2-butyl ($-C(CH_3)_2CH_2CH_3$), 3-methyl-2-butyl ($-CH(CH_3)CH(CH_3)_2$), 3-methyl-1-butyl ($-CH_2CH_2CH(CH_3)_2$), 2-methyl-1-butyl ($-CH_2CH(CH_3)CH_2CH_3$), n-hexyl ($-CH_2CH_2CH_2CH_2CH_2CH_3$), 2-hexyl ($-CH(CH_3)CH_2CH_2CH_2CH_3$), 3-hexyl ($-CH(CH_2CH_3)(CH_2CH_2CH_3)$), 2-methyl-2-pentyl ($-C(CH_3)_2CH_2CH_2CH_3$), 3-methyl-2-pentyl ($-CH(CH_3)CH(CH_3)CH_2CH_3$), 4-methyl-2-pentyl ($-CH(CH_3)CH_2CH(CH_3)_2$), 3-methyl-3-pentyl ($-C(CH_3)(CH_2CH_3)_2$), 2-methyl-3-pentyl ($-CH(CH_2CH_3)CH(CH_3)_2$), 2,3-dimethyl-2-butyl ($-C(CH_3)_2CH(CH_3)_2$), 3,3-dimethyl-2-butyl ($-CH(CH_3)C(CH_3)_3$), n-heptyl, n-octyl, and so on.

The term "heteroalkyl" refers to a group obtained by inserting one or more heteroatoms into alkyl, where the alkyl group and heteroatoms have the meaning described in the present application. The heteroalkyl can be optionally substituted by one or more substituents described in the present application. Heteroalkyl can be connected to the rest of the molecule through its carbon atoms, or to the rest of the molecule through its heteroatoms. In some embodiments, heteroalkyl is $C_{1-20}$heteroalkyl; in some embodiments, heteroalkyl is $C_{1-10}$heteroalkyl; in another embodiment, heteroalkyl is $C_{1-6}$heteroalkyl; in yet another embodiment heteroalkyl is $C_{1-4}$heteroalkyl; in yet another embodiment heteroalkyl is $C_{1-3}$heteroalkyl. Examples of heteroalkyl include, but are not limited to, methoxy, ethoxy, *i*-propoxy, *t*-butoxy, trifluoromethoxy, $CH_3C(=O)O-$, $CH_3CH_2C(=O)O-$, $(CH_3)_2CHC(=O)O-$, $CH_3C(=O)NH-$, $CH_3CH_2C(=O)NH-$, $(CH_3)_2CHC(=O)NH-$, $CH_3NHC(=O)-$, $CH_3CH_2NHC(=O)-$, $(CH_3)_2CHNHC(=O)-$, $CH_3ONHC(=O)-$, $CH_3CH_2ONHC(=O)-$, $(CH_3)_2CHONHC(=O)-$, $CH_3S(=O)_2-$, $CH_3CH_2S(=O)_2-$, $(CH_3)_2CHS(=O)_2-$, $CH_3S(=O)_2O-$, $CH_3CH_2S(=O)_2O-$, $(CH_3)_2CHS(=O)_2O-$, $CH_3S(=O)_2NH-$, $CH_3CH_2S(=O)_2NH-$, $(CH_3)_2CHS(=O)_2NH-$, $CH_3NHS(=O)_2-$, $CH_3CH_2NHS(=O)_2-$, $(CH_3)_2CHNHS(=O)_2-$, $CH_3C(=O)N(CH_3)-$, $CH_3CH_2C(=O)N(CH_3)-$, $(CH_3)_2CHC(=O)N(CH_3)-$, $CH_3N(CH_3)C(=O)-$, $CH_3CH_2N(CH_3)C(=O)-$, $(CH_3)_2CHN(CH_3)C(=O)-$, $CH_3ON(CH_3)C(=O)-$, $CH_3CH_2ON(CH_3)C(=O)-$, $(CH_3)_2CHON(CH_3)C(=O)-$, $CH_3S(=O)_2N(CH_3)-$, $CH_3CH_2S(=O)_2N(CH_3)-$, $(CH_3)_2CHS(=O)_2N(CH_3)-$,

$CH_3N(CH_3)S(=O)_2$-, $CH_3CH_2N(CH_3)S(=O)_2$-, $(CH_3)_2CHN(CH_3)S(=O)_2$-, $CH_3C(=O)N(CH_2CH_3)$-, $CH_3CH_2C(=O)N(CH_2CH_3)$-, $(CH_3)_2CHC(=O)N(CH_2CH_3)$-, $CH_3N(CH_2CH_3)C(=O)$-, $CH_3CH_2N(CH_2CH_3)C(=O)$-, $(CH_3)_2CHN(CH_2CH_3)C(=O)$-, $CH_3ON(CH_2CH_3)C(=O)$-, $CH_3CH_2ON(CH_2CH_3)C(=O)$-, $(CH_3)_2CHON(CH_2CH_3)C(=O)$-, $CH_3S(=O)_2N(CH_2CH_3)$-, $CH_3CH_2S(=O)_2N(CH_2CH_3)$-, $(CH_3)_2CHS(=O)_2N(CH_2CH_3)$-, $CH_3N(CH_2CH_3)S(=O)_2$-, $CH_3CH_2N(CH_2CH_3)S(=O)_2$-, $(CH_3)_2CHN(CH_2CH_3)S(=O)_2$-, $(CH_3)_2P(=O)$-, $CH_3CH_2(CH_3)P(=O)$-, $(CH_3)_2CH(CH_3)P(=O)$-, $(CH_3CH_2)_2P(=O)$-, $CH_3CH_2(CH_3)P(=O)$-, or $(CH_3)_2CH(CH_3CH_2)P(=O)$-. When heteroalkyl is a connecting group, and "heteroalkyl" is listed for the Markush group definition, "heteroalkyl" represents the connected heteroalkene. Examples of heteroalkene include, but are not limited to, $-NHCH_2$-, $-CH_2NH$-, $-OCH_2$-, $-CH_2O$-, $-SCH_2$-, $-CH_2S$-, $-NHCH_2CH_2$-, $-CH_2NHCH_2$-, $-CH_2CH_2NH$-, $-OCH_2CH_2$-, $-CH_2OCH_2$-, $-CH_2CH_2O$-, $-SCH_2CH_2$-, $-CH_2SCH_2$-, $-CH_2CH_2S$-, $-NHCH_2CH_2CH_2$-, $-CH_2NHCH_2CH_2$-, $-CH_2CH_2NHCH_2$-, $-CH_2CH_2CH_2NH$-, $-OCH_2CH_2CH_2$-, $-CH_2OCH_2CH_2$-, $-CH_2CH_2OCH_2$-, $-CH_2CH_2CH_2O$-, $-SCH_2CH_2CH_2$-, $-CH_2SCH_2CH_2$-, $-CH_2CH_2SCH_2$-, $-CH_2CH_2CH_2S$-, $-CH_2OCH_2O$-, $-OCH_2OCH_2$-, $-OCH_2CH_2O$-, $-NHCH_2CH_2O$-, $-CH_2NHCH_2O$- or $-NHCH_2OCH_2$-.

The term "alkenyl" represents a straight or branched hydrocarbon group, wherein there is at least one unsaturated site, i.e. a carbon-carbon *sp*$^2$ double bond, where the alkenyl group can be optionally substituted by one or more substituents described in the present application, including the orientations of "cis" and "tans", or the orientations of "E" and "Z"; the alkenyl group can be connected to the rest of the molecule by atoms that form double bonds, or to the rest of the molecule by atoms that do not form double bonds. In some embodiments, alkenyl is $C_{2-12}$alkenyl; in one embodiment, alkenyl is $C_{2-8}$alkenyl; in another embodiment, alkenyl is $C_{2-6}$alkenyl; in yet another embodiment, alkenyl is $C_{2-4}$alkenyl. Examples of alkenyl groups include, but are not limited to, ethenyl($-CH=CH_2$), allyl($-CH_2CH=CH_2$), etc. When alkenyl is a connecting group and "alkenyl" is listed for the Markush group definition, then "alkenyl" represents a connected alkenylene group. Examples of the alkenyl group represented as the connected alkenylene group include, but are not limited to: $-C\equiv C$-, $-CH_2C\equiv C$-, $-CH_2C\equiv CCH_2$-, and so on.

The term "alkynyl" represents a straight or branched hydrocarbon group, wherein there is at least one unsaturated site, i.e. a carbon-carbon *sp* triple bond, where the alkynyl group can be optionally substituted by one or more substituents described in the present application; the alkynyl group can be connected to the rest of the molecule by atoms that form triple bonds, or to the rest of the molecule by atoms that do not form triple bonds. In some embodiments, alkynyl is $C_{2-12}$alkynyl; in one embodiment, alkynyl is $C_{2-8}$alkynyl; in another embodiment, alkynyl is $C_{2-6}$alkynyl; in yet another embodiment, alkynyl is $C_{2-4}$alkynyl. Examples of alkynyl groups include, but are not limited to, ethynyl ($-C\equiv CH$), propargyl ($-CH_2C\equiv CH$), 1-propyne group ($-C\equiv C-CH_3$), etc. When alkynyl is a connecting group and "alkynyl" is listed for the Markush group definition, then "alkynyl" represents a connected alkynylene group. Examples of alkynyl groups represented as the connected alkynylene group include, but are not limited to: $-C\equiv C$-, $-CH_2C\equiv C$-, $-CH_2C\equiv CCH_2$-, and so on.

The term "alkoxy" indicates that an alkyl group is connected to the rest of the molecule through an oxygen atom, where the alkyl group has the meaning described in the present application. The alkoxy group can be optionally substituted by one or more substituents described in the present application. Unless otherwise detailed, in some embodiments, alkoxy is $C_{1-20}$alkoxy; in some embodiments, alkoxy is $C_{1-10}$alkoxy; in another embodiment, alkoxy is $C_{1-6}$alkoxy; in yet another embodiment, alkoxy is $C_{1-4}$alkoxy; in yet another embodiment, alkoxy is $C_{1-3}$alkoxy. When alkoxy is a connecting group and "alkoxy" is listed for the Markush group definition, then "alkoxy" represents a connected alkoxylene group. Examples of alkoxy include, but are not limited to, methoxy (MeO, $-OCH_3$), ethoxy (EtO, $-OCH_2CH_3$), 1-propoxy (n-PrO, n-propoxy, $-OCH_2CH_2CH_3$), 2-propoxy (*i*-PrO, *i*-propoxy, $-OCH(CH_3)_2$), 1-butoxy (n-BuO, n-butoxy, $-OCH_2CH_2CH_2CH_3$), 2-methyl-l-propoxy (*i*-BuO, *i*-butoxy, $-OCH_2CH(CH_3)_2$), 2-butoxy (s-BuO, s-butoxy, $-OCH(CH_3)CH_2CH_3$), 2-methyl-2-propoxy (*t*-BuO, *t*-butoxy, $-OC(CH_3)_3$), 1-pentoxy (n-pentoxy, $-OCH_2CH_2CH_2CH_2CH_3$), 2-pentoxy ($-OCH(CH_3)CH_2CH_2CH_3$), 3-pentoxy ($-OCH(CH_2CH_3)_2$), 2-methyl-2-butoxy ($-OC(CH_3)_2CH_2CH_3$), 3-methyl-2-butoxy ($-OCH(CH_3)CH(CH_3)_2$), 3-methyl-l-butoxy ($-OCH_2CH_2CH(CH_3)_2$), 2-methyl-l-butoxy ($-OCH_2CH(CH_3)CH_2CH_3$), etc.

The term "alkylamino" indicates that an alkyl group is connected to the rest of the molecule through a nitrogen atom, where the alkyl group has the meaning described in the present application. The alkylamino group can be optionally substituted by one or more substituents described in the present application. In some embodiments, alkylamino is $C_{1-20}$alkylamino; in some embodiments, alkylamino is Ci-ioalkylamino; in another embodiment, alkylamino is $C_{1-6}$alkylamino; in yet another embodiment, alkylamino is $C_{1-4}$alkylamino; in yet another embodiment, alkylamino is $C_{1-3}$alkylamino. When alkylamino is a connecting group and "alkylamino" is listed for the Markush group definition, then "alkylamino" represents a connected alkylene-amino group. Examples of alkylamino include, but are not limited to, methylamine, ethylamine, n-propylamine, and isopropylamino.

The term "haloalkyl" indicates that the alkyl group has been substituted by one or more halogen atoms, where alkyl has the meaning described in the present application. In some embodiments, haloalkyl is $C_{1-20}$haloalkyl; in some embodiments, haloalkyl is $C_{1-10}$haloalkyl; in another embodiment, haloalkyl is $C_{1-6}$haloalkyl; in yet another embodiment, haloalkyl is $C_{1-4}$haloalkyl; in yet another embodiment, haloalkyl is $C_{1-3}$haloalkyl. When haloalkyl is a connecting group and "haloalkyl" is listed for the Markush group definition, then "haloalkyl" represents a connected haloalkylene group.

Examples of haloalkyl include, but are not limited to, monofluoromethyl, difluoromethyl, trifluoromethyl, 1-fluoroethyl, 2-fluoroethyl, 1,2-difluoroethyl, 1,1-difluoroethyl, 2,2-difluoroethyl, 2,2-difluoroethyl, monochloromethyl, dichloromethyl, trichloromethyl, 2-chloroethyl, 1-chloroethyl, 1,2-dichloroethyl, 1,1-dichloroethyl, 2,2-dichloroethyl, 1,1-dibromoethyl, etc.

The term "haloalkoxy" indicates that an alkoxy group is substituted by one or more halogen atoms, where alkoxy has the meaning described in the present application. In some embodiments, haloalkoxy is $C_{1-20}$haloalkoxy; in some embodiments, haloalkoxy is $C_{1-10}$haloalkoxy; in another embodiment, haloalkoxy is $C_{1-6}$haloalkoxy; in yet another embodiment, haloalkoxy is $C_{1-4}$haloalkoxy; in yet another embodiment, haloalkoxy is $C_{1-3}$haloalkoxy. When haloalkoxy is a connecting group and "haloalkoxy" is listed for the Markush group definition, then "haloalkoxy" represents a connected haloalkoxylene group. Examples of haloalkoxy include, but are not limited to, monofluoromethoxy, difluoromethoxy, trifluoromethoxy, 1-fluoroethoxy, 2-fluoroethoxy, 1,2-difluoroethoxy, 1,1-difluoroethoxy, 2,2-difluoroethoxy, monochloromethoxy, dichloromethoxy, trichloromethoxy, 2-chloroethoxy, 1,2-dichloroethoxy, 1,1-dichloroethoxy, 2,2-dichloroethoxy, 1,1-dichloroethoxy, 2,2-dichloroethoxy, 1,1-dibromoethoxy, etc.

The term "haloheteroalkyl" indicates that a heteroalkyl group is substituted by one or more halogen atoms, where heteroalkyl has the meaning described in the present application. In some embodiments, haloheteroalkyl is $C_{1-2}$ohaloheteroalkyl; in some embodiments, haloheteroalkyl is $C_{1-10}$haloheteroalkyl; in another embodiment, haloheteroalkyl is $C_{1-6}$haloheteroalkyl; in yet another embodiment, haloheteroalkyl is $C_{1-4}$haloheteroalkyl; in yet another embodiment, haloheteroalkyl is $C_{1-3}$haloheteroalkyl. When haloheteroalkyl is a connecting group and "haloheteroalkyl" is defined and listed for that Markush group, then "haloheteroalkyl" represents a connected haloheteroalkylene group.

The term "halogen" refers to fluorine (F), chlorine (Cl), bromine (Br), or iodine (I). The term "hydroxyl" refers to "-OH". The term "amino" refers to "$-NH_2$". The term "cyano" refers to "-CN" or "-C≡N". The term "hydrogen" refers to H, which includes $^1H$, $^2H$, $^3H$. In some examples, hydrogen refers to $^1H$. In some examples, hydrogen refers to $^2H$ (i.e. deuterium). The term "oxo" refers to "=O". The term "heteroatom" refers to N, O, S, P. In some examples, heteroatoms refer to N, O, and S. In some examples, the heteroatom refers to N.

The term "saturated or partially unsaturated carbocyclyl" and "saturated or partially unsaturated carbocycle" can be interchangeably used to represent non-aromatic saturated or partially unsaturated monocyclic or multicyclic ring systems composed of carbon atoms as ring members. In some examples, the multicyclic ring system is a bicyclic or tricyclic ring system. The saturated or partially unsaturated carbocyclyl includes saturated or partially unsaturated monocyclic carbocyclyl, saturated or partially unsaturated spirocyclic carbocyclyl, saturated or partially unsaturated fused cyclic carbocyclyl, saturated or partially unsaturated bridged cyclic carbocyclyl. In some embodiments, saturated or partially unsaturated carbocyclyl represents 3-12 membered saturated or partially unsaturated carbocyclyl; in some other embodiments, saturated or partially unsaturated carbocyclyl represents 3-10 membered saturated or partially unsaturated carbocyclyl; in some other embodiments, saturated or partially unsaturated carbocyclyl represents 3-7 membered saturated or partially unsaturated carbocyclyl. The saturated or partially unsaturated carbocyclyl can be optionally substituted independently by one or more substituents described in the present application. When saturated or partially unsaturated carbocyclyl is a connecting group, the term "saturated or partially unsaturated carbocyclyl" represents "saturated or partially unsaturated carbocyclylene".

In some other embodiments, saturated or partially unsaturated carbocyclyl represents 3-6 membered saturated or partially unsaturated carbocyclyl. In some other embodiments, "saturated or partially unsaturated carbocyclyl" is selected from formulae ii-1 to ii-6; when formulae ii-1 to ii-6 are connected to the rest of the molecule through one bond, formulae ii-1 to ii-6 are monovalent; when formulae ii-1 to ii-6 are connected to the rest of the molecule through two bonds, formulae ii-1 to ii-6 are bivalent; the saturated or partially unsaturated carbocyclyl represented by formulae ii-1 to ii-6 each optionally are independently substituted by one or more substituents of the present application;

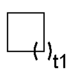
ii-1 ,
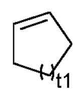
ii-2 ,
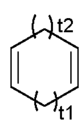
ii-3 ,
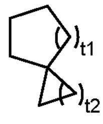
ii-4 ,
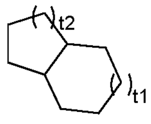
ii-5 or
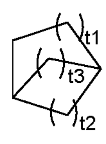
ii-6 ;

wherein, each t1 is independently 0, 1, 2, 3, 4, or 5; t2 and t3 each are independently 1, 2, 3, 4, or 5.

In some other examples, the saturated or partially unsaturated carbocyclyl is selected from the following groups; when the saturated or partially unsaturated carbocyclyl is connected to the rest of the molecule through one bond, the following groups are monovalent; when the saturated or partially unsaturated carbocyclyl is connected to the rest of the molecule through two bonds, the following groups are bivalent; the following saturated or partially unsaturated carbocyclyl each optionally are independently substituted by one or more substituents of the present application;

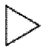 , 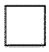 , 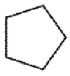 , 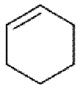 , 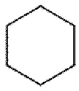 , 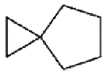 , 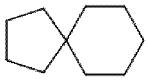 , 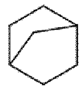 or 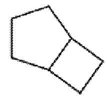 .

The term "saturated or partially unsaturated monocyclic carbocyclyl" is a monocyclic ring system, and can be interchangeably used with "saturated or partially unsaturated monocyclic carbocycle". In some embodiments, saturated or partially unsaturated monocyclic carbocyclyl is 3-8 membered saturated or partially unsaturated monocyclic carbocyclyl; in some other embodiments, saturated or partially unsaturated monocyclic carbocyclyl is 3-7 membered saturated or partially unsaturated monocyclic carbocyclyl; in some other embodiments, saturated or partially unsaturated monocyclic carbocyclyl is 3-6 membered saturated or partially unsaturated monocyclic carbocyclyl; in some other embodiments, saturated or partially unsaturated monocyclic carbocyclyl is 5-6 membered saturated or partially unsaturated monocyclic carbocyclyl. Examples of saturated or partially unsaturated monocyclic carbocyclyl include, but are not limited to: cyclopropyl, cyclobutyl, cyclopentyl, cyclohexyl, cyclohexyl, cyclopentadienyl, cyclohexene, etc. When saturated or partially unsaturated monocyclic carbocyclyl is a connecting group, saturated or partially unsaturated monocyclic carbocyclyl represents the connected saturated or partially unsaturated monocyclic-carbocyclylene. The saturated or partially unsaturated monocyclic carbocyclyl can be optionally and independently substituted by one or more substituents described in the present application.

The term "saturated or partially unsaturated spirocyclic carbocyclyl" is a multicyclic ring system, and can be interchangeably used with "saturated or partially unsaturated spirocyclic carbocycle", which contains at least one such a ring system that is a saturated or partially unsaturated non-aromatic ring system formed by two carbon rings sharing one carbon atom. In some embodiments, saturated or partially unsaturated spirocyclic carbocyclyl is 7-12 membered saturated or partially unsaturated spirocyclic carbocyclyl; in some other embodiments, saturated or partially unsaturated spirocyclic carbocyclyl is 7-10 membered saturated or partially unsaturated spirocyclic carbocyclyl. Examples of saturated or partially unsaturated spirocyclic carbocyclyl include, but are not limited to: spiro[4.4]nonyl, spiro[3.4]octyl, spiro[4.5]decyl, etc. When saturated or partially unsaturated spirocyclic carbocyclyl is a connecting group, saturated or partially unsaturated spirocyclic carbocyclyl represents the connected saturated or partially unsaturated spirocyclic-carbocyclylene. The saturated or partially unsaturated spirocyclic carbocyclyl can be optionally and independently substituted by one or more substituents described in the present application.

The term "saturated or partially unsaturated fused cyclic carbocyclyl" is a multicyclic ring system, and can be interchangeably used with "saturated or partially unsaturated fused cyclic carbocycle", which contains at least one such a ring system that is a saturated or partially unsaturated non-aromatic ring system where two carbon rings share two carbon atoms. In some embodiments, saturated or partially unsaturated fused cyclic carbocyclyl is 6-12 membered saturated or partially unsaturated fused cyclic carbocyclyl; in some other embodiments, saturated or partially unsaturated fused cyclic carbocyclyl is 6-10 membered saturated or partially unsaturated fused cyclic carbocyclyl; in some other embodiments, saturated or partially unsaturated fused cyclic carbocyclyl is 8-10 membered saturated or partially unsaturated fused cyclic carbocyclyl. Examples of saturated or partially unsaturated fused cyclic carbocyclyl include, but are not limited to: dicyclic[3.1.0]hexyl, dicyclic[3.2.0]heptyl, dicyclic[3.3.0]octyl, dicyclic[4.3.0]nonyl, etc. When saturated or partially unsaturated fused cyclic carbocyclyl is a connecting group, saturated or partially unsaturated fused cyclic carbocyclyl represents the connected saturated or partially unsaturated fused-cyclic-carbocyclylene. The saturated or partially unsaturated fused cyclic carbocyclyl can be optionally and independently substituted by one or more substituents described in the present application.

The term "saturated or partially unsaturated bridged cyclic carbocyclyl" is a multicyclic ring system, and can be interchangeably used with "saturated or partially unsaturated bridged cyclic carbocycle", which contains at least one such a ring system that is a saturated or partially unsaturated non-aromatic ring system where two carbon rings share three or more carbon atoms. In some embodiments, saturated or partially unsaturated bridged cyclic carbocyclyl is 5-12 membered saturated or partially unsaturated bridged cyclic carbocyclyl; in some other embodiments, saturated or partially unsaturated bridged cyclic carbocyclyl is 5-10 membered saturated or partially unsaturated bridged cyclic carbocyclyl; in some other embodiments, saturated or partially unsaturated bridged cyclic carbocyclyl is 5-8 membered saturated or partially unsaturated bridged cyclic carbocyclyl. Examples of saturated or partially unsaturated bridged cyclic carbocyclyl include, but are not limited to: dicyclic[3.1.1]heptyl, dicyclic[3.2.1]octyl, dicyclic[2.2.2]octyl, etc. When saturated or partially unsaturated bridged cyclic carbocyclyl is a connecting group, saturated or partially unsaturated bridged cyclic carbocyclyl represents the connected saturated or partially unsaturated bridged-cyclic-carbocyclylene. The saturated or partially unsaturated bridged cyclic carbocyclyl can be optionally and independently substituted by one or more substituents described in the present application.

The terms "saturated or partially unsaturated heterocyclyl" and "saturated or partially unsaturated heterocycle" can be used interchangeably and represent a saturated or partially unsaturated non-aromatic monocyclic or multicyclic ring system, in which the ring atoms contain at least one carbon atom and one or more heteroatoms. The heteroatoms

have the meaning described in the present application. Unless otherwise detailed, saturated or partially unsaturated heterocyclyl can be connected to the rest of the molecule through its carbon atoms, or to the rest of the molecule through its heteroatoms. In some examples, the multicyclic ring system is a bicyclic or tricyclic ring. Saturated or partially unsaturated heterocyclyl includes saturated or partially unsaturated monocyclic heterocyclyl, saturated or partially unsaturated spirocyclic heterocyclyl, saturated or partially unsaturated fused cyclic heterocyclyl, saturated or partially unsaturated bridged cyclic heterocyclyl. In some embodiments, saturated or partially unsaturated heterocyclyl represents 3-12 membered saturated or partially unsaturated heterocyclyl; in some other embodiments, saturated or partially unsaturated heterocyclyl represents 3-10 membered saturated or partially unsaturated heterocyclyl; In some other embodiments, saturated or partially unsaturated heterocyclyl represents 3-7 membered saturated or partially unsaturated heterocyclyl; in some other embodiments, saturated or partially unsaturated heterocyclyl represents 3-6-membered saturated or partially unsaturated heterocyclyl. The saturated or partially unsaturated heterocyclyl can be optionally and independently substituted by one or more substituents described in the present application. Wen saturated or partially unsaturated heterocyclyl is a connecting group, the term "saturated or partially unsaturated heterocyclyl" represents "saturated or partially unsaturated heterocyclylene".

In some other embodiments, saturated or partially unsaturated heterocyclyl is selected from formulae iii-1 to iii-16; when formulae iii-1 to iii-16 are connected to the rest of the molecule through one bond, formulae iii-1 to iii-16 are monovalent; when formulae iii-1 to iii-16 are connected to the rest of the molecule through two bonds, formulae iii-1 to iii-16 are bivalent; saturated or partially unsaturated heterocyclyl represented by formulae iii-1 to iii-16 each optionally are independently substituted by one or more substituents defined by the aforementioned corresponding groups of the present application;

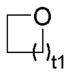
iii-1 ,
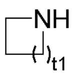
iii-2 ,
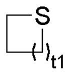
iii-3 ,
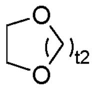
iii-4 ,
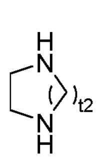
iii-5 ,
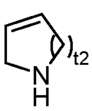
iii-6 ,
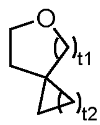
iii-7 ,
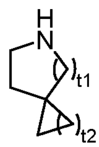
iii-8 ,
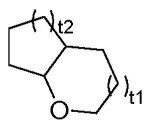
iii-9 ,
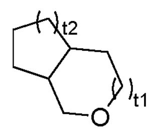
iii-10 ,

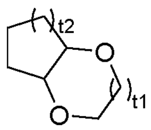
iii-11 ,
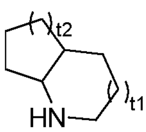
iii-12 ,
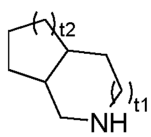
iii-13 ,
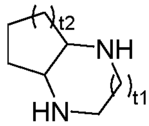
iii-14 ,
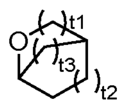
iii-15 or
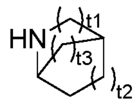
iii-16 ;

wherein, each t1 is independently 0, 1, 2, 3, 4, or 5; t2 and t3 each are independently 1, 2, 3, 4, or 5.

The phrase "substituted by substituents defined by the aforementioned corresponding groups of the present application" refers to substitution of the substituent mentioned in the aforementioned groups involving "saturated or partially unsaturated carbocyclyl" and "saturated or partially unsaturated heterocyclyl".

In some other examples, saturated or partially unsaturated heterocyclyl is selected from the following groups; when the saturated or partially unsaturated heterocyclyl is connected to the rest of the molecule through one bond, the following groups are monovalent; when the saturated or partially unsaturated heterocyclyl is connected to the rest of the molecule through two bonds, the following groups are bivalent; the following saturated or partially unsaturated heterocyclyl each optionally are independently substituted by one or more substituents defined by the aforementioned corresponding groups of the present application;

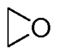 , 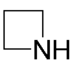 , 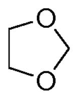 , 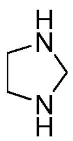 , 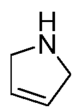 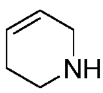 , 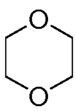 , 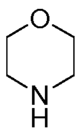 , 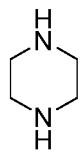 ,

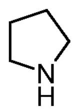, 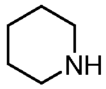, 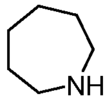, 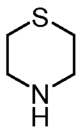, 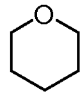, 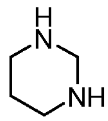, 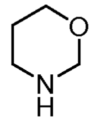,

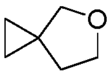, 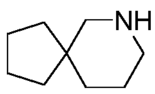, 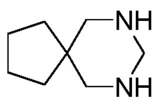, 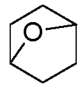, 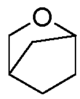, 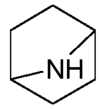,

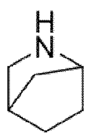, 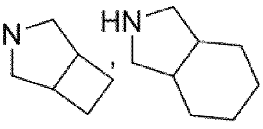, 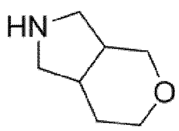, 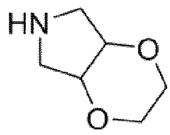 or 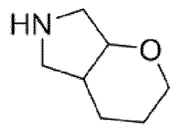.

The term "saturated or partially unsaturated monocyclic heterocyclyl" is a monocyclic ring system, and can be interchangeably used with "saturated or partially unsaturated monocyclic heterocycle". In some embodiments, saturated or partially unsaturated monocyclic heterocyclyl is 3-8 membered saturated or partially unsaturated monocyclic heterocyclyl; in some other embodiments, saturated or partially unsaturated monocyclic heterocyclyl is 3-7 membered saturated or partially unsaturated monocyclic heterocyclyl; in some other embodiments, saturated or partially unsaturated monocyclic heterocyclyl is 3-6 membered saturated or partially unsaturated monocyclic heterocyclyl; in some other embodiments, saturated or partially unsaturated monocyclic heterocyclyl is 5-6 membered saturated or partially unsaturated monocyclic heterocyclyl. Examples of saturated or partially unsaturated monocyclic heterocyclyl include, but are not limited to: oxiranyl, azetidinyl, oxetanyl, thietanyl, pyrrolidinyl, 2-pyrrolinyl, 3-pyrrolinyl, pyrazolinyl, imidazolinyl, imidazolidinyl, tetrahydrofuryl, dihydrofuryl, tetrahydrothienyl, dihydrothienyl, 1,3-dioxolanyl, dithiolanyl, tetrahydropyranyl, dihydropyranyl, 2H-pyranyl, 4H-pyranyl, tetrahydrothiopyranyl, piperidinyl, morpholinyl, thiomorpholinyl, piperazinyl, dioxinyl, dithianyl, thiazolyl, homopiperazinyl, homopiperidinyl, 1,1-dioxo-1,3-thiomorpholinyl, etc. When saturated or partially unsaturated monocyclic heterocyclyl is a connecting group, saturated or partially unsaturated monocyclic heterocyclyl represents the connected saturated or partially unsaturated monocyclic heterocyclylene. The saturated or partially unsaturated monocyclic heterocyclyl can be optionally and independently substituted by one or more substituents described in the present application.

The term "saturated or partially unsaturated spirocyclic heterocyclyl" is a multicyclic ring system, and can be interchangeably used with "saturated or partially unsaturated spirocyclic heterocycle", which contains at least one such a ring system that is a saturated or partially unsaturated non-aromatic ring system consisting of two heterocycles sharing one heteroatom. In some embodiments, saturated or partially unsaturated spirocyclic heterocyclyl is 7-12 membered saturated or partially unsaturated spirocyclic heterocyclyl; in some other embodiments, saturated or partially unsaturated spirocyclic heterocyclyl is 7-10 membered saturated or partially unsaturated spirocyclic heterocyclyl. Examples of saturated or partially unsaturated spirocyclic heterocyclyl include, but are not limited to: 4,7-diazospiro[2.5]octyl, 2,8-diazospiro[4.5]decyl, 2,7-diazospiro[4.5]decyl, 2,7-diazospiro[3.5]decyl, 2,6-diazospiro[3.3]heptyl, 2,7-diazospiro[4.4]nonyl, 3-diazospiro[5.5]undecyl, 2,7-diazospiro[4.4]nonyl-1-one, etc. When saturated or partially unsaturated spirocyclic heterocyclyl is a connecting group, saturated or partially unsaturated spirocyclic heterocyclyl represents the connected saturated or partially unsaturated spirocyclic-heterocyclylene. The saturated or partially unsaturated spirocyclic heterocyclyl can be optionally and independently substituted by one or more substituents described in the present application.

The term "saturated or partially unsaturated fused cyclic heterocyclyl" is a multicyclic ring system, and can be interchangeably used with "saturated or partially unsaturated fused cyclic heterocycle", which contains at least one such a ring system that is a saturated or partially unsaturated non-aromatic ring system consisting of two heterocycles sharing two heteroatoms. In some embodiments, saturated or partially unsaturated fused cyclic heterocyclyl is 6-12 membered saturated or partially unsaturated fused cyclic heterocyclyl; in some other embodiments, saturated or partially unsaturated fused cyclic heterocyclyl is 6-10 membered saturated or partially unsaturated fused cyclic heterocyclyl; in some other embodiments, saturated or partially unsaturated fused cyclic heterocyclyl is 8-10 membered saturated or partially unsaturated fused cyclic heterocyclyl. Examples of saturated or partially unsaturated fused cyclic heterocyclyl include, but are not limited to: octahydroimidazolo[1,5-c]pyrimidine, etc. When saturated or partially unsaturated fused cyclic heterocyclyl is a connecting group, saturated or partially unsaturated fused cyclic heterocyclyl represents the connected saturated or partially unsaturated fused-cyclic-heterocyclylene. The saturated or partially unsaturated fused cyclic heterocyclyl can be optionally and independently substituted by one or more substituents described in the present application.

The term "saturated or partially unsaturated bridged cyclic heterocyclyl" is a multicyclic ring system, and can

be interchangeably used with "saturated or partially unsaturated bridged cyclic heterocycle", which contains at least one such a ring system that is a saturated or partially unsaturated non-aromatic ring system consisting of two heterocycles sharing three or more heteroatoms. In some embodiments, saturated or partially unsaturated bridged cyclic heterocyclyl is 5-12 membered saturated or partially unsaturated bridged cyclic heterocyclyl; in some other embodiments, saturated or partially unsaturated bridged cyclic heterocyclyl is 5-10 membered saturated or partially unsaturated bridged cyclic heterocyclyl; in some other embodiments, saturated or partially unsaturated bridged cyclic heterocyclyl is 5-8 membered saturated or partially unsaturated bridged cyclic heterocyclyl. Examples of saturated or partially unsaturated bridged cyclic heterocyclyl include, but are not limited to: 3,6-diazabicyclo[3.1.1]heptane, 3,8-diazabicyclo[3.2.1]octane, 2-diazabicyclo[2.2.1]heptane, 6-diazabicyclo[3.1.1]heptane, 3-diazabicyclo[3.1.1]heptane, 8-diazabicyclo[3.2.1]octane, 3-diazabicyclo[3.2.1]octane, 2-diazabicyclo[2.2.2]octane, etc. When saturated or partially unsaturated bridged cyclic heterocyclyl is a connecting group, saturated or partially unsaturated bridged cyclic heterocyclyl represents the connected saturated or partially unsaturated bridged-cyclic heterocyclylene. The saturated or partially unsaturated bridged cyclic heterocyclyl can be optionally and independently substituted by one or more substituents described in the present application.

The terms "aryl" and "aromatic ring" can be used interchangeably, representing an aromatic monocyclic or multicyclic ring system composed of carbon atoms as ring atoms. In some examples, the multicyclic ring system is a bicyclic or tricyclic ring. In some examples, aryl is 6-14 membered aryl; in some other examples, aryl is 6-10 membered aryl; in some other examples, aryl is phenyl or naphthyl. When aryl is a connecting group, aryl represents the connected arylene. The aryl can be optionally and independently substituted by one or more substituents described in the present application.

The terms "heteroaryl" and "heteroaromatic ring" can be used interchangeably, representing an aromatic monocyclic or multicyclic ring system composed of at least one carbon atom and one or more heteroatoms as ring atoms. The heteroatoms have the meaning described in the present application. In some examples, the multicyclic ring system is a bicyclic or tricyclic ring. In some examples, heteroaryl is 5-14 membered heteroaryl; in some other examples, heteroaryl is 5-10 membered heteroaryl; in some other examples, heteroaryl is 5-6 membered heteroaryl. In some other examples, heteroaryl is 5-10 membered azaaryl; in some other examples, heteroaryl is 5-6-membered azaaryl; in some other examples, heteroaryl is 6-membered azaaryl. Examples of heteroaryl include, but are not limited to: 2-furyl, 3-furyl, N-imidazolyl, 2-imidazolyl, 4-imidazolyl, 5-imidazolyl, 3-isoxazolyl, 4-isoxazolyl, 5-isoxazolyl, 2-oxazolyl, 4-oxazolyl, 5-oxazolyl, N-pyrrolyl, 2-pyrrolyl, pyrazinyl, 3-pyrrolyl, 2-pyridinyl, 3-pyridinyl, 4-pyridinyl, 2-pyrimidinyl, 4-pyrimidinyl, 5-pyrimidinyl, pyridazinyl (such as 3-pyridazinyl), 2-thiazolyl, 4-thiazolyl, 5-thiazolyl, tetrazolyl (such as 5-tetrazolyl), triazolyl (such as 2-triazolyl and 5-triazolyl), 2-thienyl, 3-thienyl, pyrazolyl (such as 2-pyrazolyl), isothiazolyl, 1,2,3-oxadiazolyl, 1,2,5-oxadiazolyl, 1,2,4-oxadiazolyl, 1,2,3-triazolyl, 1,2,3-thiadiazolyl, 1,3,4-thiadiazolyl, 1,2,5-thiadiazolyl, pyrazinyl, 1,3,5-triazinyl, benzoimidazolyl, benzofuryl, benzothiophenyl, indolyl (such as 2-indolyl), purinyl, quinolinyl (such as 2-quinolinyl, 3-quinolinyl, 4-quinolinyl), isoquinolinyl (such as 1-isoquinolinyl, 3-isoquinolinyl or 4-isoquinolinyl), imidazo[1,2-a]pyridinyl, pyrazolo[1,5-a]pyridinyl, pyrazolo[1,5-a]pyrimidinyl, imidazo[1,2-b]pyridazinyl, [1,2,4]triazolo[4,3-b]pyridazinyl, [1,2,4]triazolo[1,5-a]pyrimidinyl, [1,2,4]triazolo[1,5-a]pyridinyl, etc. When aryl is a connecting group, aryl represents the connected arylene. The aryl group can be optionally and independently substituted by one or more substituents described in the present application.

The term "carbon chain" refers to a saturated or unsaturated functional group acting as a linker that is composed of carbon atoms. The carbon chain can be optionally substituted by one or more substituents described in the present application. In some embodiments, the carbon chain is saturated; in some other embodiments, the carbon chain contains one or more double or triple bonds. In some embodiments, the carbon chain is a carbon chain having 2-6 atoms; in some other embodiments, the carbon chain is a carbon chain having 2-4 atoms; in some other embodiments, the carbon chain is a carbon chain having 2-3 atoms; in some other embodiments, a carbon chain is a carbon chain having 2 atoms; in some other embodiments, the carbon chain is a carbon chain having 3 atoms; in some other embodiments, a carbon chain is a carbon chain having 4 atoms.

The term "heterochain" refers to a saturated or unsaturated functional group acting as a linker that is composed of carbon atoms and heteroatoms, comprising at least one heteroatom. The heterochain can be optionally substituted by one or more substituents described in the present application. In some embodiments, the heterochain is saturated; in some other embodiments, the heterochain contains one or more double or triple bonds. In some embodiments, the heterochain is a heterochain having 2-6 atoms; in some other embodiments, the heterochain is a heterochain having 2-4 atoms; in some other embodiments, the heterochain is a heterochain having 2-3 atoms; in some other embodiments, the heterochain is a heterochain having 2 atoms; in some other embodiments, heterochain is a heterochain having 3 atoms; in some other embodiments, heterochain is a heterochain having 4 atoms.

The term "nitrogen protecting group" has the same meaning as "amino protecting group" and can be interchanged with "amino protecting group". The nitrogen protection group refers to a substituent group that is connected to an amino group to block or protect the functionality of the amino group in a compound. Suitable amino protection groups include acetyl, trifluoroacetyl, *t*-butoxycarbonyl (BOC, Boc), benzyloxycarbonyl (CBZ, Cbz), and 9-fluorenylmethyloxycarbonyl (Fmoc).

The term "leaving group" refers to an atom or functional group that detaches from a larger molecule during a chemical reaction, and is a term used in nucleophilic substitution and elimination reactions. In nucleophilic substitution reactions, the reactants attacked by nucleophilic reagents are called as a substrate, while atoms or atomic groups that carry a pair of electrons and detach from the substrate molecule are called a leaving group. Common leaving groups include, but are not limited to, halogen atoms, ester groups, sulfonate ester groups, nitro groups, azide groups, or hydroxyl groups.

The term "carbon member" refers to $CH_2$, or a substituted $CH_2$.

As described in the present application, unless otherwise stated in detail, in the combined groups, such as hydroxyalkyl, aminoalkyl, hydroxyalkoxyl, aminoalkoxyl, alkylamino, saturated or partially unsaturated carbocyclylalkyl, saturated or partially unsaturated heterocyclylalkyl, arylalkyl, heteroarylalkyl used in the present application, the groups involved therein, such as amino, hydroxyl, alkyl, alkoxyl, saturated or partially unsaturated carbocyclyl, saturated or partially unsaturated heterocyclyl, aryl, heteroaryl have the definitions described in the present application.

As described in the present application, unless otherwise stated in detail, cyclic substituents, such as saturated or partially unsaturated carbocyclyl, saturated or partially unsaturated heterocyclyl, aryl, heteroaryl, can be connected to the rest of the molecule at any linkable position on the ring. For example, piperidyl contains piperidin-1-yl, piperidin-2-yl, piperidin-3-yl, and piperidin-4-yl. As described in the present application, a ring system formed by connecting a substituent R to the center of a ring through a bond represents that the substituent R can be substituted at any substitutable or reasonable position on the ring it is connected to. As described in the present application, a ring system formed by connecting a bond to the center of a ring represents that the bond can be connected to the rest of the molecule at any linkable position on the ring system. As described in the present application, if there are two attachment sites on a ring connected to the rest of a molecule, these two attachment sites can be connected to the rest of the molecule at any available position on the ring, and the two attachment positions can be interchanged.

The term "pharmaceutically acceptable excipients" used in the present application refers to materials, mixtures, or vehicle that are pharmaceutically acceptable and consistently associated with the administered dosage form or pharmaceutical composition. Each excipient must be compatible with other components of the pharmaceutical composition when mixed to avoid interactions that significantly reduce the efficacy of the disclosed compound when administered to patients and avoid interactions other than those of the pharmaceutically acceptable composition. In addition, each excipient must be pharmaceutically acceptable, for example, have sufficiently high purity. The appropriate pharmaceutically acceptable excipients will vary depending on the specific dosage form chosen. In addition, pharmaceutically acceptable excipients can be selected based on their specific functions in the composition. For example, certain pharmaceutically acceptable excipients that can help produce uniform dosage forms can be selected. Certain pharmaceutically acceptable excipients that can contribute to the production of stable dosage forms can be selected. Certain pharmaceutically acceptable excipients that can aid in carrying or transporting the present compound administered to patients from one organ or part of the body to another can be selected. Certain pharmaceutically acceptable excipients that can enhance patient's compliance can be selected.

Some suitable examples of excipients include lactose, glucose, sucrose, sorbitol, mannitol, starch, arabic gum, calcium phosphate, alginate, tragacanth gum, gelatin, calcium silicate, microcrystalline cellulose, polyvinylpyrrolidone, cellulose, water, syrup, and methylcellulose. Suitable pharmaceutically acceptable excipients also include the following types of excipients: vehicle, propellants, solubilizers, cosolvents, emulsifiers, colorants, binders, disintegrants, fillers, lubricants, wetting agents, osmotic pressure regulators, stabilizers, glidants, flavor correction agents, preservatives, suspending agents, coating materials, aromatics, antiadhesive agents, antioxidants, chelating agents, penetration enhancers, pH regulators, plasticizers, surfactants, foaming agents, defoamers, thickeners, encapsulation agents, moisturizing agents, absorbers, diluents, flocculants and anti-coagulants and filter aids. Technicians can recognize that certain pharmaceutically acceptable excipients can provide more than one function and offer alternative functions, depending on how much of that excipient is present in the formulation and which other excipients are present in the formulation. The compounds of the present application can be prepared using known methods in the art, in order to achieve rapid, sustained, or delayed release of active components after administration to patients.

Technicians possess knowledge and skills in the art to enable them to choose appropriate amounts of pharmaceutically acceptable excipients for use in the present application. In addition, there are numerous resources available to technical personnel, which describe pharmaceutically acceptable excipients and are used to select appropriate pharmaceutically acceptable excipients. Examples thereof include Remington"s Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

As used in the present application, "pharmaceutically acceptable carriers" include any and all solvents and solvent mixtures, coatings, complexing agents, solid carriers, dispersion media, surfactant excipients, antibacterial and antifungal drugs, isotonic and absorption delaying agent for drug active substances, and mixtures thereof, and all of which are also known in the art.

Various carriers for preparing pharmaceutically acceptable compositions and well-known techniques for their preparation are disclosed in Remington: The Science and Practice of Pharmacy, 21st edition, 2005, ed. D. B. Troy, Lippincott Williams & Wilkins, Philadelphia, and Encyclopedia of Pharmaceutical Technology, eds. J. Swarbrick and J.C.Boylan,1988-1999, Marcel Dekker, New York, and the contents of these literature are incorporated by reference into the present application. Except for any commonly used carrier that is incompatible with the compound of the present application due to any unexpected biological effects or harmful interactions with any other component in a pharmaceutically acceptable composition, the focus on its application falls within the scope of the present application.

A suitable pharmaceutically acceptable carrier depends on a form of medicaments and is known to those skilled in the art. Non-limiting examples of pharmaceutically acceptable carriers include those selected from the following: lactose, gelatin, sugar alcohols (such as starch, mannitol, corn starch, etc.), vegetable oil, talc, magnesium stearate, colloidal silica, carboxymethyl cellulose, microcrystalline cellulose, sodium dodecanesulfate, Tween buffer aqueous solution, copovidone, polysorbate, ethanol, propylene glycol, polyglycols (preferably polyethylene glycol, such as PEG400), 80 (i.e. PEG (20), sorbitol monooleate), DMSO, a mixture of water and cosolvents, such as aqueous solutions including alcohols such as ethanol and/or polyglycols such as polyethylene glycol, polyols such as glycerol and/or esters of polyethylene glycol and fatty acids, surfactants such as anionic, cationic, non-ionic, and zwitterionic surfactants, complexing agents such as cyclodextrin, for example, $\alpha$-cyclodextrin ($\alpha$-CD) or hydroxypropyl-$\beta$-cyclodextrin (HP-$\beta$-CD), bile acids or lipids, such as salts of animal or plant phospholipids, micelle forming agent, and oils such as corn oil, or mixtures of two or more components aforementioned.

Unless otherwise indicated, any structural formula provided by the present application is also intended to represent these compounds in the forms that have not been enriched by isotopes or have been enriched by isotopes. Isotope enriched compounds have the structure described by the general formula of the present application, with exception that one or more atoms are replaced by atoms with the selected atomic weight or mass number. Illustrative isotopes that can be incorporated into the compound of the present application include isotopes of hydrogen, carbon, nitrogen, oxygen, phosphorus, sulfur, fluorine, and chlorine, such as $^{2}H$, $^{3}H$, $^{11}C$, $^{13}C$, $^{14}C$, $^{15}N$, $^{17}O$, $^{18}O$, $^{18}F$, $^{31}P$, $^{32}P$, $^{35}S$, $^{36}Cl$ and $^{125}I$.

Compounds containing radioactive isotopes such as $^{3}H$, $^{14}C$, and $^{18}F$, or containing non-radioactive isotopes such as $^{2}H$ and $^{13}C$, as a compound enriched in isotopes, can be used for metabolic studies (using $^{14}C$), reaction kinetics studies (using e.g. $^{2}H$ or $^{3}H$), detection or imaging techniques such as positron emission tomography (PET) or single photon emission computed tomography (SPECT) including drug or substrate tissue distribution determination, or can be used in patient radiotherapy. Compounds enriched with $^{18}F$ are particularly ideal for PET or SPECT studies. The compounds of formulae I, II-1 to II-6, III, IV-1 to IV-6, V-1 to V-6, VI-1 to VI-5, VII-1 to VII-6, and VIII-1 to VIII-6 enriched with isotopes can be prepared by conventional techniques familiar to those skilled in the art, or prepared as described in the examples and preparation processes of the present application, using appropriate isotopes labeled reagents instead of previously used unlabeled reagents.

In addition, the substitution of heavier isotopes, especially deuterium (i.e., $^{2}H$ or D), can provide certain therapeutic advantages, which are brought about by higher metabolic stability, such as an increase in half-life in the body, a decrease in dose requirements, or an improvement in treatment index. It should be understood that deuterium in the present application is considered as a substituent for the compound of formulae I, II-1 to II-6, III, IV-1 to IV-6, V-1 to V-6, VI-1 to VI-5, VII-1 to VII-6, and VIII-1 to VIII-6. An isotope enrichment factor can be used to define the concentration of heavier isotopes, especially deuterium. The term "isotope enrichment factor" used in the present application refers to the ratio between the isotopic abundance and the natural abundance of a specified isotope. If the substituent of the compound of the present application is designated as deuterium, the compound has an isotopic enrichment factor of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation) for each designated deuterium atom. The pharmaceutically acceptable solvates of the present application include those solvates in which the crystalline solvent can be isotopic substituted, such as D2O, acetone-d6, DMSO-d6.

## Compounds, pharmaceutical compositions, formulations, and administration

The present application provides a compound as a focal adhesion kinase (FAK) inhibitor that has a good therapeutic effect on cancer, pulmonary arterial hypertension, and pathological angiogenesis.

In one aspect, the present application relates to a compound, which is a compound of formula I or a stereoisomer, a tautomer, a nitrogen oxide, a solvate, a metabolite, a pharmaceutically acceptable salt, or a prodrug thereof:

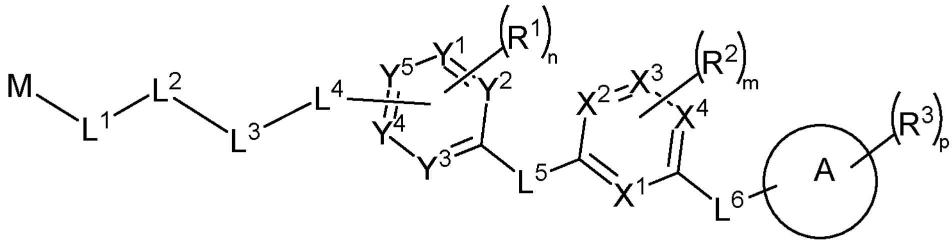

I

wherein, M, A, $L^1$, $L^2$, $L^3$, $L^4$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, n, m, and p are defined as described in the present application.

In some examples, M is a covalent warhead. In some examples, M is:

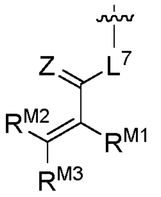
i-1

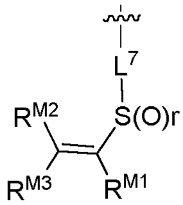
i-2

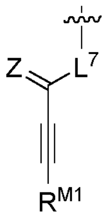
i-3

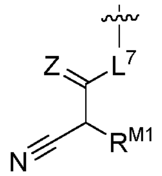
i-4

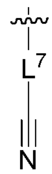
i-5

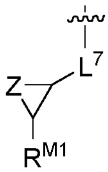
i-6

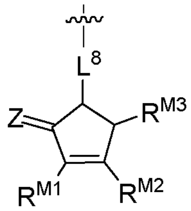
i-7

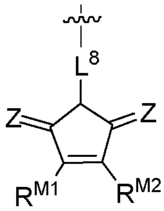
i-8

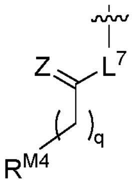
i-9

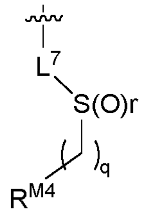
i-10

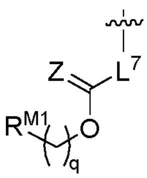
i-11

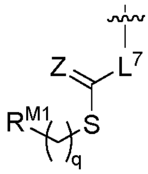
i-12

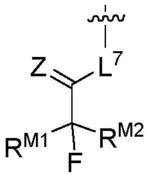
i-13

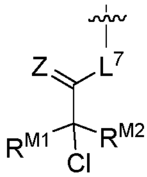
i-14

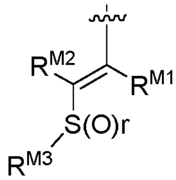
i-15

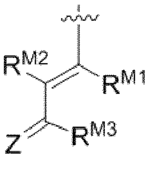
i-16

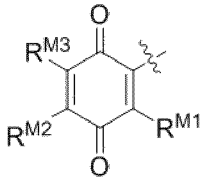
i-17

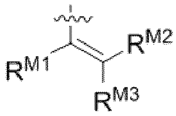
i-18

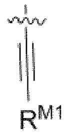
i-19 or

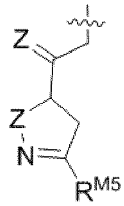
i-20 ;

wherein Z, $L^7$, $L^8$, $R^{M1}$, $R^{M2}$, $R^{M3}$, $R^{M4}$, $R^{M5}$, q, r are defined as described in the present application.

In some examples, M is:

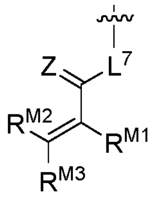 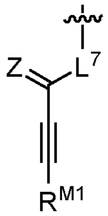 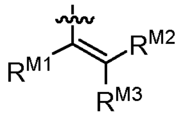 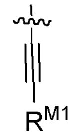

i-1, i-3, i-18 or i-19;

wherein Z, $L^7$, $R^{M1}$, $R^{M2}$, $R^{M3}$ are defined as described in the present application.

In some examples, $L^7$ is a bond, -O-, -S-, -$NR^{L7a}$- or $C_{1-4}$alkyl, wherein, one or more carbon units of the $C_{1-4}$alkyl group optionally are independently replaced with -O-, -S-, -$NR^{L7a}$-, -$NR^{L7a}$C(=O)-, -C(=O)$NR^{L7a}$-, -SC(=O)-, -C(=O)S-, -OC(=O)-, -C(=O)O-, -$NR^{L7a}$C(=S)-, -C(=S)$NR^{L7a}$-, *trans* $CR^{L7b}$=$CR^{L7b}$-, *cis* $CR^{L7b}$=$CR^{L7b}$-, -C≡C-, -S(=O)-, -S(=O)O-, -OS(=O)-, -S(=O)$NR^{L7a}$-, -$NR^{L7a}$S(=O)-, -S(=O)$_2$-, -S(=O)$_2$O-, -OS(=O)$_2$-, -S(=O)$_2$$NR^{L7a}$-, or -$NR^{L7a}$S(=O)$_2$-; wherein each $R^{L7a}$ is independently hydrogen, deuterium, $C_{1-4}$alkyl, or a nitrogen protecting group; each $R^{L7b}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two adjacent $R^{L7b}$ groups bind with their connected atoms to form 3-6 membered saturated or partially unsaturated carbocyclyl or 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $L^7$, $R^{L7a}$ and $R^{L7b}$ each optionally are independently substituted by one or more substituents selected from the group consisting of deuterium, halogen, hydroxyl, amino, cyano, oxo, and $C_{1-4}$alkyl.

In some examples, $L^7$ is a bond, -O-, -S-, -NH- or $C_{1-4}$alkyl, wherein, one or more carbon units of the $C_{1-4}$alkyl group optionally are independently replaced with -O-, -S-, -NH-, -NHC(=O)-, -C(=O)NH-, -SC(=O)-, -C(=O)S-, -OC(=O)-, -C(=O)O-, -NHC(=S)-, -C(=S)NH-, *trans* CH=CH-, *cis* CH=CH-, -C≡C-, -S(=O)-, -S(=O)O-, -OS(=O)-, -S(=O)NH-, -NHS(=O)-, -S(=O)$_2$-, -S(=O)$_2$O-, -OS(=O)$_2$-, -S(=O)zNH- or -NHS(=O)$_2$-.

In some examples, $L^8$ is a bond or $C_{1-4}$alkyl, wherein the alkyl involved in $L^8$ optionally is substituted by one or more substituents selected from the group consisting of deuterium, halogen, hydroxyl, amino, cyano, and oxo.

In some examples, each $R^{M1}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, -$CH_2OR^{M1a}$, -$CH_2N(R^{M1a})_2$, -$CH_2SR^{M1a}$, -$OR^{M1a}$, -$N(R^{M1a})_2$, -$Si(R^{M1a})_3$ or -$SR^{M1a}$, wherein each $R^{M1a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl; or two $R^{M1a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M1}$ and $R^{M1a}$ each optionally are independently substituted by one or more substituents selected from the group consisting of deuterium, halogen, hydroxyl, amino, cyano, oxo, and $C_{1-4}$alkyl.

In some examples, each $R^{M1}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, -$CH_2OR^{M1a}$, -$CH_2N(R^{M1a})_2$, -$CH_2SR^{M1a}$, -$OR^{M1a}$, -$N(R^{M1a})_2$, -$Si(R^{M1a})_3$ or -$SR^{M1a}$, wherein each $R^{M1a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two $R^{M1a}$ groups optionally bind with their connected atoms to form 3-6 membered saturated or partially unsaturated heterocyclyl.

In some examples, each $R^{M2}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, -$CH_2OR^{M2a}$, -$CH_2N(R^{M2a})_2$, -$CH_2SR^{M2a}$, -$OR^{M2a}$, -$N(R^{M2a})_2$ or -$SR^{M2a}$, wherein each $R^{M2a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl; or two $R^{M2a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M2}$ and $R^{M2a}$ each optionally are independently substituted by one or more substituents selected from the group consisting of deuterium, halogen, hydroxyl, amino, cyano, oxo, and $C_{1-4}$alkyl.

In some examples, each $R^{M2}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, $-CH_2OR^{M2a}$, $-CH_2N(R^{M2a})_2$, $-CH_2SR^{M2a}$, $-OR^{M2a}$, $-N(R^{M2a})_2$ or $-SR^{M2a}$, wherein each $R^{M2a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two $R^{M2a}$ groups optionally bind with their connected atoms to form 3-6 membered saturated or partially unsaturated heterocyclyl.

In some examples, each $R^{M3}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, $-CH_2OR^{M3a}$, $-CH_2N(R^{M3a})_2$, $-CH_2SR^{M3a}$, $-OR^{M3a}$, $-N(R^{M3a})_2$ or $-SR^{M3a}$, wherein each $R^{M3a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl; or two $R^{M3a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M3}$ and $R^{M3a}$ each optionally are independently substituted by one or more substituents selected from the group consisting of deuterium, halogen, hydroxyl, amino, cyano, oxo, and $C_{1-4}$alkyl.

In some examples, each $R^{M3}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, $-CH_2OR^{M3a}$, $-CH_2N(R^{M3a})_2$, $-CH_2SR^{M3a}$, $-OR^{M3a}$, $-N(R^{M3a})_2$ or $-SR^{M3a}$, wherein each $R^{M3a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two $R^{M3a}$ groups optionally bind with their connected atoms to form 3-6 membered saturated or partially unsaturated heterocyclyl.

In some examples, optionally, $R^{M1}$ and $R^{M3}$, or $R^{M2}$ and $R^{M3}$, or $R^{M1}$ and $R^{M2}$ optionally connect to form saturated or partially unsaturated carbocyclyl or saturated or partially unsaturated heterocyclyl, wherein saturated or partially unsaturated carbocyclyl or saturated or partially unsaturated heterocyclyl each optionally are independently substituted by one or more substituents selected from the group consisting of deuterium, halogen, hydroxyl, amino, cyano, oxo, and $C_{1-4}$alkyl.

In some examples, optionally, $R^{M1}$ and $R^{M3}$, or $R^{M2}$ and $R^{M3}$, or $R^{M1}$ and $R^{M2}$ optionally connect to form 3-6 membered saturated or partially unsaturated carbocyclyl or 3-6 membered saturated or partially unsaturated heterocyclyl, wherein 3-6 membered saturated or partially unsaturated carbocyclyl or 3-6 membered saturated or partially unsaturated heterocyclyl each optionally are independently substituted by one or more substituents selected from the group consisting of deuterium, halogen, hydroxyl, amino, cyano, oxo, and $C_{1-4}$alkyl.

In some examples, $R^{M4}$ is a leaving group. In some examples, $R^{M4}$ is halogen, p-tosyl, phenylsulfonyl, p-nitrophenylsulfonyl. In some examples, $R^{M5}$ is halogen.

In some examples, Z is O, S or $NR^Z$; wherein $R^Z$ is hydrogen, deuterium, $C_{1-4}$alkyl, or a nitrogen protecting group; alkyl involved in $R^Z$ optionally is substituted by one or more substituents selected from the group consisting of deuterium, halogen, hydroxyl, amino, cyano, and oxo. In some examples, Z is O, S or NH.

In some examples, q is 0, 1, 2, 3, 4, 5 or 6.

In some examples, r is 1 or 2.

In some examples, M is

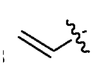, 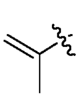, 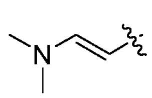, 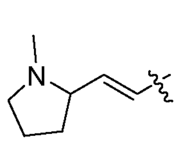, 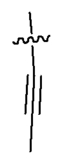, 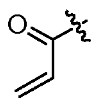, 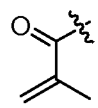,

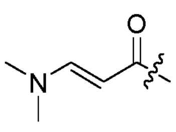, 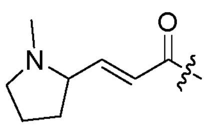 or 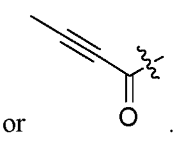.

In some examples, A is aryl or heteroaryl. In some examples, A is 6-10 membered aryl, or 5-10 membered heteroaryl.

In some other examples, A is 6-10 membered aryl or 5-10 membered azaaryl. In some other examples, A is phenyl or 6-membered azaaryl. In some other examples, A is phenyl, imidazolyl, pyrazolyl, triazolyl, tetraazolyl, oxazolyl,

thiazolyl, furyl, thienyl, pyrrolyl, pyridinyl, pyrimidinyl, pyrazinyl, pyridazinyl, or the following groups:

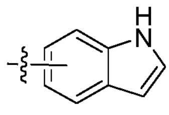 , 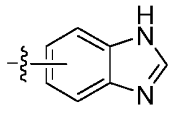 , 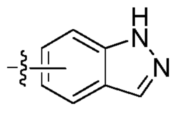 , 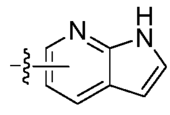 , 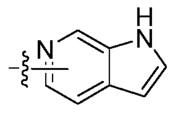 , 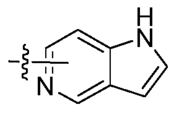 ,

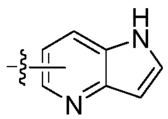 , 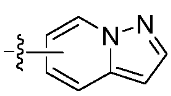 , 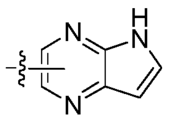 , 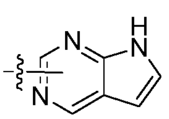 , 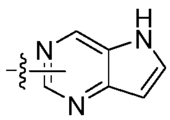 , 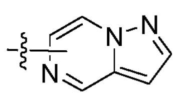 ,

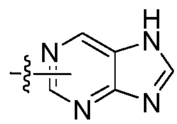 , 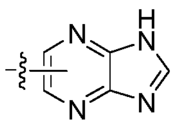 , 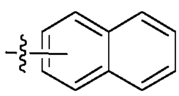 , 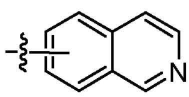 , 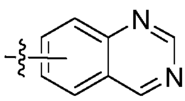 or 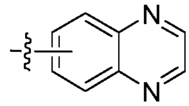 .

In some other examples, A is phenyl, pyridinyl, pyrimidinyl, pyrazinyl or pyridazinyl. In some other examples, A is phenyl or pyrazinyl.

In some examples, $L^1$ is a bond, -C(=O)-, -S(=O)-, $-S(=O)_2-$, $-C(=O)NR^{L1a}-$, $-NR^{L1a}C(=O)-$, $-S(=O)_2NR^{L1a}-$, or $-NR^{L1a}S(=O)_2-$; wherein, each $R^{L1a}$ is independently hydrogen, alkyl, alkenyl, alkynyl, heteroalkyl, haloalkyl, haloheteroalkyl, or a nitrogen protecting group; wherein the alkyl, alkenyl, alkynyl, heteroalkyl, haloalkyl, haloheteroalkyl involved in $R^{L1a}$ each optionally are independently substituted by one or more substituents selected from the group consisting of deuterium, halogen, hydroxyl, amino, cyano, and oxo.

In some examples, $L^1$ is a bond, -C(=O)-, -S(=O)-, $-S(=O)_2-$, $-C(=O)NR^{L1a}-$, $-NR^{L1a}C(=O)-$, $-S(=O)_2NR^{L1a}-$, or $-NR^{L1a}S(=O)_2-$; wherein, each $R^{L1a}$ is independently hydrogen, deuterium, $C_{1-6}$alkyl, $C_{2-6}$alkenyl, $C_{2-6}$alkynyl, $C_{1-6}$heteroalkyl, $C_{1-6}$haloalkyl, $C_{1-6}$haloheteroalkyl, or a nitrogen protecting group; wherein the alkyl, alkenyl, alkynyl, heteroalkyl, haloalkyl, haloheteroalkyl involved in $R^{L1a}$ each optionally are independently substituted by one or more substituents selected from the group consisting of deuterium, halogen, hydroxyl, amino, cyano, and oxo.

In some examples, $L^1$ is a bond, -C(=O)-, -S(=O)-, -S(=O)z-, $-C(=O)NR^{L1a}-$, $-NR^{L1a}C(=O)-$, $-S(=O)_2NR^{L1a}-$, or $-NR^{L1a}S(=O)_2-$; wherein, each $R^{L1a}$ is independently hydrogen, deuterium, $C_{1-4}$alkyl, $C_{2-4}$alkenyl, $C_{2-4}$alkynyl, $C_{1-4}$heteroalkyl, $C_{1-4}$haloalkyl, $C_{1-4}$haloheteroalkyl, or a nitrogen protecting group; wherein the alkyl, alkenyl, alkynyl, heteroalkyl, haloalkyl, haloheteroalkyl involved in $R^{L1a}$ each optionally are independently substituted by one or more substituents selected from the group consisting of deuterium, halogen, hydroxyl, amino, cyano, and oxo. In some other examples, $L^1$ is a bond, -C(=O)-, -S(=O)-, -S(=O)z-, $-C(=O)NR^{L1a}$ , $-NR^{L1a}C(=O)-$, $-S(=O)_2NR^{L1a}-$, or $-NR^{L1a}S(=O)_2-$; wherein, each $R^{L1a}$ is independently hydrogen, methyl, ethyl, ethenyl, ethynyl or trifluoromethyl. In some other examples, $L^1$ is a bond, -C(=O)-, -S(=O)-, -S(=O)z-, -C(=O)NH-, -NHC(=O)-, $-S(=O)_2NH-$ or $-NHS(=O)_2-$. In some other examples, $L^1$ is a bond, -C(=O)- or -C(=O)NH-.

In some examples, $L^2$ is a bond, alkyl, heteroalky, saturated or partially unsaturated carbocyclyl, saturated or partially unsaturated heterocyclyl, aryl or heteroaryl; wherein, the alkyl, heteroalkyl, saturated or partially unsaturated carbocyclyl, saturated or partially unsaturated heterocyclyl, aryl, or heteroaryl involved in $L^2$ each optionally are independently substituted by one or more substituents selected from the group consisting of deuterium, hydroxyl, amino, cyano, halogen, oxo, alkyl, alkenyl, alkynyl, heteroalkyl and haloheteroalkyl.

In some other examples, $L^2$ is a bond, $C_{1-6}$alkyl, $C_{1-6}$heteroalkyl, 3-6 membered saturated or partially unsaturated carbocyclyl, 3-6 membered saturated or partially unsaturated heterocyclyl, 6-10 membered aryl, or 5-10 membered heteroaryl; wherein, the alkyl, heteroalkyl, saturated or partially unsaturated carbocyclyl, saturated or partially unsaturated heterocyclyl, aryl, or heteroaryl involved in $L^2$ each optionally are independently substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl.

In some other examples, $L^2$ is a bond, $C_{1-4}$alkyl, $C_{1-4}$heteroalkyl, 3-6 membered saturated or partially unsaturated carbocyclyl, 3-6 membered saturated or partially unsaturated heterocyclyl, 6-10 membered aryl, or 5-10 membered heteroaryl; wherein, the alkyl, heteroalkyl, saturated or partially unsaturated carbocyclyl, saturated or partially unsaturated heterocyclyl, aryl, or heteroaryl involved in $L^2$ each optionally are independently substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl.

In some other examples, $L^2$ is a bond, $C_{1-4}$alkyl, $C_{1-4}$heteroalkyl, 3-6 membered saturated or partially unsaturated carbocyclyl, 3-6 membered saturated or partially unsaturated heterocyclyl, 6-10 membered aryl, or 5-10 membered heteroaryl; wherein, the alkyl, heteroalkyl, saturated or partially unsaturated carbocyclyl, saturated or partially unsaturated heterocyclyl, aryl, or heteroaryl involved in $L^2$ each optionally are independently substituted by a substituent selected from the group consisting of deuterium, hydroxyl, amino, cyano, fluoro, chloro, oxo, ethenyl, ethynyl, methoxy, ethoxy, trifluoromethoxy and methylamino.

In some other examples, $L^2$ is a bond, 3-6 membered saturated or partially unsaturated carbocyclyl or 3-6 membered saturated or partially unsaturated heterocyclyl; wherein, saturated or partially unsaturated carbocyclyl, saturated or partially unsaturated heterocyclyl involved in $L^2$ each optionally are independently substituted by one or more substituents selected from the group consisting of hydroxyl, amino, cyano, fluoro, chloro, oxo, ethenyl, ethynyl, methoxy, ethoxy, trifluoromethoxy and methylamino. In some other examples, $L^2$ is a bond, cyclohexyl, piperidyl, phenyl or pyridinyl.

In some examples, $L^3$ is a bond, aryl or heteroaryl; wherein, aryl, or heteroaryl involved in $L^3$ each optionally are independently substituted by one or more substituents selected from the group consisting of deuterium, hydroxyl, amino, cyano, halogen, alkyl, alkenyl, alkynyl, heteroalkyl, haloalkyl and haloheteroalkyl.

In some other examples, $L^3$ is a bond, 6-10 membered aryl, or 5-10 membered heteroaryl; wherein, aryl, or heteroaryl involved in $L^3$ each optionally are independently substituted by one or more substituents selected from the group consisting of deuterium, hydroxyl, amino, cyano, halogen, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl. In some other examples, $L^3$ is a bond, 6-10 membered aryl, or 5-10 membered heteroaryl; wherein, aryl, or heteroaryl involved in $L^3$ each optionally are independently substituted by a substituent selected from the group consisting of deuterium, hydroxyl, amino, cyano, fluoro, chloro, methyl, ethyl, ethenyl, ethynyl, methoxy, ethoxy, trifluoromethoxy and methylamino. In some other examples, $L^3$ is a bond, 6-10 membered aryl, or 5-10 membered heteroaryl; wherein, aryl, or heteroaryl involved in $L^3$ optionally is substituted by a substituent selected from the group consisting of hydroxyl, amino, cyano, fluoro, chloro, methyl, ethyl, ethenyl, ethynyl, methoxy, ethoxy, trifluoromethoxy and methylamino. In some other examples, $L^3$ is a bond, phenyl or pyridinyl.

In some examples, $L^4$ is a carbon chain or heterochain with 2-6 atoms; wherein, the carbon chain or heterochain involved in $L^4$ each optionally are independently substituted by one or more substituents selected from the group consisting of deuterium, hydroxyl, amino, cyano, halogen, oxo, alkenyl, alkynyl, alkyl, haloalkyl, hydroxyalkyl, aminoalkyl, alkoxyl, haloalkoxyl, hydroxyalkoxyl, aminoalkoxyl, alkylamino, and a nitrogen protecting group.

In some other examples, $L^4$ is a carbon chain or heterochain with 2-6 atoms; the carbon chain or heterochain involved in $L^4$ each optionally are independently substituted by one or more substituents selected from the group consisting of deuterium, hydroxyl, amino, cyano, halogen, oxo, $C_{2-6}$alkenyl, $C_{2-6}$alkynyl, $C_{1-6}$alkyl, $C_{1-6}$haloalkyl, $C_{1-6}$hydroxyalkyl, $C_{1-6}$aminoalkyl, $C_{1-6}$alkoxyl, $C_{1-6}$haloalkoxyl, $C_{1-6}$hydroxyalkoxyl, $C_{1-6}$aminoalkoxyl, $C_{1-6}$alkylamino, and a nitrogen protecting group. In some other examples, $L^4$ is a carbon chain or heterochain with 2-6 atoms; wherein, the carbon chain or heterochain involved in $L^4$ each optionally are independently substituted by one or more substituents selected from the group consisting of deuterium, hydroxyl, amino, cyano, halogen, oxo, $C_{2-4}$alkenyl, $C_{2-4}$alkynyl, $C_{1-4}$alkyl, $C_{1-4}$haloalkyl, $C_{1-4}$hydroxyalkyl, $C_{1-4}$aminoalkyl, $C_{1-4}$alkoxyl, $C_{1-4}$haloalkoxyl, $C_{1-4}$hydroxyalkoxyl, $C_{1-4}$aminoalkoxyl, $C_{1-4}$alkylamino, and a nitrogen protecting group. In some other examples, $L^4$ is a carbon chain or heterochain with 2-4 atoms; wherein, the carbon chain or heterochain involved in $L^4$ each optionally are independently substituted by one or more substituents selected from the group consisting of deuterium, hydroxyl, amino, cyano, halogen, oxo, ethenyl, ethynyl, methyl, ethyl, isopropyl, methoxy, ethoxy, trifluoromethoxy, methylamino, and a nitrogen protecting group.

In some other examples, $L^4$ is $-NHCH_2-$, $-CH_2NH-$, $-OCH_2-$, $-CH_2O-$, $-SCH_2-$, $-CH_2S-$, $-NHCH_2CH_2-$, $-CH_2NHCH_2-$, $-CH_2CH_2NH-$, $-OCH_2CH_2-$, $-CH_2OCH_2-$, $-CH_2CH_2O-$, $-SCH_2CH_2-$, $-CH_2SCH_2-$, $-CH_2CH_2S-$, $-NHCH_2CH_2CH_2-$, $-CH_2NHCH_2CH_2-$, $-CH_2CH_2NHCH_2-$, $-CH_2CH_2CH_2NH-$, $-OCH_2CH_2CH_2-$, $-CH_2OCH_2CH_2-$, $-CH_2CH_2OCH_2-$, $-CH_2CH_2CH_2O-$, $-SCH_2CH_2CH_2-$, $-CH_2SCH_2CH_2-$, $-CH_2CH_2SCH_2-$, $-CH_2CH_2CH_2S-$, $-CH_2OCH_2O-$, $-OCH_2OCH_2-$, $-OCH_2CH_2O-$, $-NHCH_2CH_2O-$, $-CH_2NHCH_2O-$ or $-NHCH_2OCH_2-$; wherein the hydrogen of $CH_2$ involved in $L^4$ each optionally are independently substituted by a substituent selected from the group consisting of hydrogen, deuterium, hydroxyl, amino, cyano, halogen, oxo, ethenyl, ethynyl, methyl, ethyl, isopropyl, methoxy, ethoxy, trifluoromethoxy and methylamino; the hydrogen of NH involved in $L^4$ each optionally are independently substituted by a substituent selected from the group consisting of hydrogen, deuterium, hydroxyl, cyano, halogen, oxo, ethenyl, ethynyl, methyl, ethyl, isopropyl, methoxy, ethoxy, trifluoromethoxy, and a nitrogen protecting group. In some other examples, $L^4$ is $-NHCH_2-$, $-CH_2NHCH_2-$, $-NHC(=O)-$, $-CH_2NHC(=O)-$, $-C(=O)NHCH_2-$ or $-NHCH(CH_3)-$.

In some examples, $L^5$ is $-NR^{L5a}-$, -O-, -S-, alkyl or heteroalkyl; wherein, the alkyl and heteroalkyl involved in $L^5$ each optionally are independently substituted by a substituent selected from the group consisting of deuterium, amino, hydroxyl, oxo, cyano, halogen, alkenyl and alkynyl; each $R^{L5a}$ is independently hydrogen, alkyl, alkenyl, alkynyl, heteroalkyl, haloalkyl, or a nitrogen protecting group.

In some examples, $L^5$ is $-NR^{L5a}-$, -O-, -S-, $C_{1-6}$alkyl or $C_{1-6}$heteroalkyl; wherein, the alkyl and heteroalkyl involved in $L^5$ each optionally are independently substituted by one or more substituents selected from the group consisting of deuterium, amino, hydroxyl, oxo, cyano, halogen, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; each $R^{L5a}$ is independently hydrogen, deuterium, $C_{1-6}$alkyl, $C_{2-6}$alkenyl, $C_{2-6}$alkynyl,

$C_{1-6}$heteroalkyl, $C_{1-6}$haloalkyl, or a nitrogen protecting group.

In some other examples, $L^5$ is $-NR^{L5a}$, $-CR^{L5b}R^{L5c}$-, -O-, -S-, $-CR^{L5b}R^{L5c}NR^{L5a}$-, $-NR^{L5a}CR^{L5b}R^{L5c}$-, $-CR^{L5b}R^{L5c}O$-, $-OCR^{L5b}R^{L5c}$-, $-CR^{L5b}R^{L5c}S$- or $-SCR^{L5b}R^{L5c}$-; wherein, each $R^{L5b}$ and $R^{L5c}$ are independently hydrogen, deuterium, amino, hydroxyl, oxo, cyano, halogen, $C_{1-4}$alkyl, $C_{2-4}$alkenyl, $C_{2-4}$alkynyl, $C_{1-4}$heteroalkyl, $C_{1-4}$haloalkyl, or $C_{1-4}$haloheteroalkyl; each $R^{L5a}$ is independently hydrogen, deuterium, $C_{1-4}$alkyl, $C_{2-4}$alkenyl, $C_{2-4}$alkynyl, $C_{1-4}$heteroalkyl, $C_{1-4}$haloalkyl, or a nitrogen protecting group. In some other examples, $L^5$ is -NH-, -O-, -S-, $-NHCH_2$-, $-OCH_2$-, $-SCH_2$-, $-CH_2NH$-, $-CH_2O$- or $-CH_2S$-. In some other examples, $L^5$ is -NH-.

In some examples, $L^6$ is $-NR^{L6a}$-, -O-, -S-, alkyl or heteroalkyl; wherein, the alkyl and heteroalkyl involved in $L^6$ each optionally are independently substituted by a substituent selected from the group consisting of deuterium, amino, hydroxyl, oxo, cyano, halogen, alkenyl and alkynyl; each $R^{L6a}$ is independently hydrogen, alkyl, alkenyl, alkynyl, heteroalkyl, haloalkyl, or a nitrogen protecting group.

In some other examples, $L^6$ is $-NR^{L6a}$-, -O-, -S-, $C_{1-6}$alkyl or $C_{1-6}$heteroalkyl; wherein, the alkyl and heteroalkyl involved in $L^6$ each optionally are independently substituted by one or more substituents selected from the group consisting of deuterium, amino, hydroxyl, oxo, cyano, halogen, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; each $R^{L6a}$ is independently hydrogen, deuterium, $C_{1-6}$alkyl, $C_{2-6}$alkenyl, $C_{2-6}$alkynyl, $C_{1-6}$heteroalkyl, $C_{1-6}$haloalkyl, or a nitrogen protecting group.

In some other examples, $L^6$ is $-NR^{L6a}$, $-CR^{L6b}R^{L6c}$-, -O-, -S-, $-CR^{L6b}R^{L6c}NR^{L6a}$-, $-NR^{L6a}CR^{L6b}R^{L6c}$-, $-CR^{L6b}R^{L6c}O$-, $-OCR^{L6b}R^{L6c}$-, $-CR^{L6b}R^{L6c}S$- or $-SCR^{L6b}R^{L6c}$-; wherein, each $R^{L6b}$ and $R^{L6c}$ are independently hydrogen, deuterium, amino, hydroxyl, oxo, cyano, halogen, $C_{1-4}$alkyl, $C_{2-4}$alkenyl, $C_{2-4}$alkynyl, $C_{1-4}$heteroalkyl, $C_{1-4}$haloalkyl, or $C_{1-4}$haloheteroalkyl; each $R^{L6a}$ is independently hydrogen, deuterium, $C_{1-4}$alkyl, $C_{2-4}$alkenyl, $C_{2-4}$alkynyl, $C_{1-4}$heteroalkyl, $C_{1-4}$haloalkyl, or a nitrogen protecting group. In some other examples, $L^6$ is -NH-, -O-, -S-, $-NHCH_2$-, $-OCH_2$-, $-SCH_2$-, $-CH_2NH$-, $-CH_2O$- or $-CH_2S$-. In some other examples, $L^6$ is -NH- or $-NHCH_2$-.

In some examples, $X^1$, $X^2$, $X^3$, $X^4$, $Y^1$, $Y^2$, $Y^3$, $Y^4$ and $Y^5$ each are independently CH or N.

In some examples, each $R^1$ is independently hydrogen, deuterium, amino, hydroxyl, cyano, halogen, alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl; if there are two adjacent $R^1$, the two adjacent $R^1$ and their connected atoms optionally form saturated or partially unsaturated carbocyclyl, saturated or partially unsaturated heterocyclyl, aryl or heteroaryl; wherein, the alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, heteroarylalkyl involved in $R^1$ each optionally are independently substituted by one or more substituents selected from the group consisting of deuterium, hydroxyl, amino, cyano, halogen, oxo and alkyl.

In some examples, each $R^1$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, $C_{1-6}$haloheteroalkyl, 3-6 membered saturated or partially unsaturated carbocyclyl, 3-6 membered saturated or partially unsaturated carbocyclyl$C_{1-6}$alkyl, 3-6 membered saturated or partially unsaturated heterocyclyl, 3-6 membered saturated or partially unsaturated heterocyclyl$C_{1-6}$alkyl, 6-10 membered aryl, 6-10 membered aryl$C_{1-6}$alkyl, 6-10 membered heteroaryl or 6-10 membered heteroaryl$C_{1-6}$alkyl; if there are two adjacent $R^1$, the two adjacent $R^1$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^1$ each optionally are independently substituted by one or more substituents selected from the group consisting of deuterium, hydroxyl, amino, cyano, halogen, oxo, and $C_{1-4}$alkyl.

In some other examples, each $R^1$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, or $C_{1-6}$haloheteroalkyl; if there are two adjacent $R^1$, the two adjacent $R^1$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, saturated or partially unsaturated carbocyclyl, saturated or partially unsaturated heterocyclyl, aryl, or heteroaryl involved in $R^1$ each optionally are independently substituted by one or more substituents selected from the group consisting of deuterium, hydroxyl, amino, cyano, halogen, oxo, and $C_{1-4}$alkyl.

In some other examples, each $R^1$ is independently hydrogen, deuterium, amino, hydroxyl, cyano, halogen, $C_{1-6}$alkyl, $C_{1-6}$heteroalkyl, $C_{1-6}$haloalkyl, or $C_{1-6}$haloheteroalkyl; wherein the alkyl, heteroalkyl, haloalkyl, haloheteroalkyl involved in $R^1$ each optionally are independently substituted by one or more substituents selected from the group consisting of deuterium, hydroxyl, amino, cyano, halogen, and oxo. In some other examples, each $R^1$ is independently hydrogen, deuterium, amino, hydroxyl, cyano, halogen, $C_{1-4}$alkyl, $C_{1-4}$heteroalkyl, $C_{1-4}$haloalkyl, or $C_{1-4}$haloheteroalkyl;

wherein the alkyl, heteroalkyl, haloalkyl, haloheteroalkyl involved in $R^1$ each optionally are independently substituted by one or more substituents selected from the group consisting of deuterium, hydroxyl, amino, cyano, halogen, and oxo. In some other examples, each $R^1$ is independently hydrogen, deuterium, amino, hydroxyl, cyano, fluoro, chloro, methyl, ethyl, propyl, isopropyl, *t*-butyl, methoxy, ethoxy, *i*-propoxy, *t*-butoxy, trifluoromethyl, trifluoromethoxy, $CH_3C(=O)$-, $CH_3CH_2C(=O)$-, $(CH_3)_2CHC(=O)$-, $CH_3C(=O)O$-, $CH_3CH_2C(=O)O$-, $(CH_3)_2CHC(=O)O$-, $CH_3C(=O)NH$-, $CH_3CH_2C(=O)NH$-, $(CH_3)_2CHC(=O)NH$-, $CH_3NHC(=O)$-, $CH_3CH_2NHC(=O)$-, $(CH_3)_2CHNHC(=O)$-, $CH_3ONHC(=O)$-, $CH_3CH_2ONHC(=O)$-, $(CH_3)_2CHONHC(=O)$-, $CH_3S(=O)_2$-, $CH_3CH_2S(=O)_2$-, $(CH_3)_2CHS(=O)_2$-, $CH_3S(=O)_2O$-, $CH_3CH_2S(=O)_2O$-, $(CH_3)_2CHS(=O)_2O$-, $CH_3S(=O)_2NH$-,$CH_3CH_2S(=O)_2NH$-, $(CH_3)_2CHS(=O)_2NH$-, $CH_3NHS(=O)_2$-, $CH_3CH_2NHS(=O)_2$-, $(CH_3)_2CHNHS(=O)_2$-, $CH_3C(=O)N(CH_3)$-, $CH_3CH_2C(=O)N(CH_3)$-, $(CH_3)_2CHC(=O)N(CH_3)$-, $CH_3N(CH_3)C(=O)$-, $CH_3CH_2N(CH_3)C(=O)$-, $(CH_3)_2CHN(CH_3)C(=O)$-, $CH_3ON(CH_3)C(=O)$-, $CH_3CH_2ON(CH_3)C(=O)$-, $(CH_3)_2CHON(CH_3)C(=O)$-, $CH_3S(=O)_2N(CH_3)$-, $CH_3CH_2S(=O)_2N(CH_3)$-, $(CH_3)_2CHS(=O)_2N(CH_3)$-, $CH_3N(CH_3)S(=O)_2$-, $CH_3CH_2N(CH_3)S(=O)_2$-, $(CH_3)_2CHN(CH_3)S(=O)_2$-, $CH_3C(=O)N(CH_2CH_3)$-, $CH_3CH_2C(=O)N(CH_2CH_3)$-, $(CH_3)_2CHC(=O)N(CH_2CH_3)$-, $CH_3N(CH_2CH_3)C(=O)$-, $CH_3CH_2N(CH_2CH_3)C(=O)$-, $(CH_3)_2CHN(CH_2CH_3)C(=O)$-, $CH_3ON(CH_2CH_3)C(=O)$-, $CH_3CH_2ON(CH_2CH_3)C(=O)$-, $(CH_3)_2CHON(CH_2CH_3)C(=O)$-, $CH_3S(=O)_2N(CH_2CH_3)$-, $CH_3CH_2S(=O)_2N(CH_2CH_3)$-, $(CH_3)_2CHS(=O)_2N(CH_2CH_3)$-, $CH_3N(CH_2CH_3)S(=O)_2$-, $CH_3CH_2N(CH_2CH_3)S(=O)_2$-, $(CH_3)_2CHN(CH_2CH_3)S(=O)_2$-, $(CH_3)_2P(=O)$-, $CH_3CH_2(CH_3)P(=O)$-, $(CH_3)_2CH(CH_3)P(=O)$-, $(CH_3CH_2)_2P(=O)$-, $CH_3CH_2(CH_3)P(=O)$-, or $(CH_3)_2CH(CH_3CH_2)P(=O)$-.

In some examples, each $R^2$ is independently hydrogen, deuterium, amino, hydroxyl, cyano, halogen, alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl; if there are two adjacent $R^2$, the two adjacent $R^2$ and their connected atoms optionally form saturated or partially unsaturated carbocyclyl, saturated or partially unsaturated heterocyclyl, aryl or heteroaryl; wherein, the alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^2$ each optionally are substituted by one or more substituents selected from the group consisting of hydroxyl, amino, cyano, halogen, oxo, and alkyl.

In some other examples, each $R^2$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, $C_{1-6}$haloheteroalkyl, 3-6 membered saturated or partially unsaturated carbocyclyl, 3-6 membered saturated or partially unsaturated carbocyclyl$C_{1-6}$alkyl, 3-6 membered saturated or partially unsaturated heterocyclyl, 3-6 membered saturated or partially unsaturated heterocyclyl$C_{1-6}$alkyl, 6-10 membered aryl, 6-10 membered aryl$C_{1-6}$alkyl, 6-10 membered heteroaryl or 6-10 membered heteroaryl$C_{1-6}$alkyl; if there are two adjacent $R^2$, the two adjacent $R^2$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^2$ each optionally are independently substituted by one or more substituents selected from the group consisting of deuterium, hydroxyl, amino, cyano, halogen, oxo, and $C_{1-4}$alkyl.

In some other examples, each $R^2$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, or $C_{1-6}$haloheteroalkyl; if there are two adjacent $R^2$, the two adjacent $R^2$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, saturated or partially unsaturated carbocyclyl, saturated or partially unsaturated heterocyclyl, aryl, or heteroaryl involved in $R^2$ each optionally are independently substituted by one or more substituents selected from the group consisting of deuterium, hydroxyl, amino, cyano, halogen, oxo, and $C_{1-4}$alkyl. In some other examples, each $R^2$ is independently hydrogen, deuterium, amino, hydroxyl, cyano, halogen, $C_{1-6}$alkyl, $C_{1-6}$heteroalkyl, $C_{1-6}$haloalkyl, or $C_{1-6}$haloheteroalkyl; wherein the alkyl, heteroalkyl, haloalkyl, haloheteroalkyl involved in $R^2$ each optionally are independently substituted by one or more substituents selected from the group consisting of deuterium, hydroxyl, amino, cyano, halogen, and oxo. In some other examples, each $R^2$ is independently hydrogen, deuterium, amino, hydroxyl, cyano, halogen, $C_{1-4}$alkyl, $C_{1-4}$heteroalkyl, $C_{1-4}$haloalkyl, or $C_{1-4}$haloheteroalkyl; wherein the alkyl, heteroalkyl, haloalkyl, haloheteroalkyl involved in $R^2$ each optionally are independently substituted by one or more substituents selected from the group consisting of deuterium, hydroxyl, amino, cyano, halogen, and oxo.

In some other examples, each $R^2$ is independently hydrogen, deuterium, amino, hydroxyl, cyano, fluoro, chloro, bromo, methyl, ethyl, propyl, isopropyl, *t*-butyl, methoxy, ethoxy, *i*-propoxy, *t*-butoxy, trifluoromethyl, trifluoromethoxy, $CH_3C(=O)$-, $CH_3CH_2C(=O)$-, $(CH_3)_2CHC(=O)$-, $CH_3C(=O)O$-, $CH_3CH_2C(=O)O$-, $(CH_3)_2CHC(=O)O$-, $CH_3C(=O)NH$-, $CH_3CH_2C(=O)NH$-, $(CH_3)_2CHC(=O)NH$-, $CH_3NHC(=O)$-, $CH_3CH_2NHC(=O)$-, $(CH_3)_2CHNHC(=O)$-, $CH_3ONHC(=O)$-,

$CH_3CH_2ONHC(=O)$-, $(CH_3)_2CHONHC(=O)$-, $CH_3S(=O)_2$-, $CH_3CH_2S(=O)_2$-, $(CH_3)_2CHS(=O)_2$-, $CH_3S(=O)_2O$-, $CH_3CH_2S(=O)_2O$-, $(CH_3)_2CHS(=O)_2O$-, $CH_3S(=O)_2NH$-,$CH_3CH_2S(=O)_2NH$-, $(CH_3)_2CHS(=O)_2NH$-, $CH_3NHS(=O)_2$-, $CH_3CH_2NHS(=O)_2$-, $(CH_3)_2CHNHS(=O)_2$-, $CH_3C(=O)N(CH_3)$-, $CH_3CH_2C(=O)N(CH_3)$-, $(CH_3)_2CHC(=O)N(CH_3)$-, $CH_3N(CH_3)C(=O)$-, $CH_3CH_2N(CH_3)C(=O)$-, $(CH_3)_2CHN(CH_3)C(=O)$-, $CH_3ON(CH_3)C(=O)$-, $CH_3CH_2ON(CH_3)C(=O)$-, $(CH_3)_2CHON(CH_3)C(=O)$-, $CH_3S(=O)_2N(CH_3)$-, $CH_3CH_2S(=O)_2N(CH_3)$-, $(CH_3)_2CHS(=O)_2N(CH_3)$-, $CH_3N(CH_3)S(=O)_2$-, $CH_3CH_2N(CH_3)S(=O)_2$-, $(CH_3)_2CHN(CH_3)S(=O)_2$-, $CH_3C(=O)N(CH_2CH_3)$-, $CH_3CH_2C(=O)N(CH_2CH_3)$-, $(CH_3)_2CHC(=O)N(CH_2CH_3)$-, $CH_3N(CH_2CH_3)C(=O)$-, $CH_3CH_2N(CH_2CH_3)C(=O)$-, $(CH_3)_2CHN(CH_2CH_3)C(=O)$-, $CH_3ON(CH_2CH_3)C(=O)$-, $CH_3CH_2ON(CH_2CH_3)C(=O)$-, $(CH_3)_2CHON(CH_2CH_3)C(=O)$-, $CH_3S(=O)_2N(CH_2CH_3)$-, $CH_3CH_2S(=O)_2N(CH_2CH_3)$-, $(CH_3)_2CHS(=O)_2N(CH_2CH_3)$-, $CH_3N(CH_2CH_3)S(=O)_2$-, $CH_3CH_2N(CH_2CH_3)S(=O)_2$-, $(CH_3)_2CHN(CH_2CH_3)S(=O)_2$-, $(CH_3)_2P(=O)$-, $CH_3CH_2(CH_3)P(=O)$-, $(CH_3)_2CH(CH_3)P(=O)$-, $(CH_3CH_2)_2P(=O)$-, $CH_3CH_2(CH_3)P(=O)$-, or $(CH_3)_2CH(CH_3CH_2)P(=O)$-.

In some examples, each $R^3$ is independently hydrogen, deuterium, amino, hydroxyl, cyano, halogen, alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl; if there are two adjacent $R^3$, the two adjacent $R^3$ and their connected atoms optionally form saturated or partially unsaturated carbocyclyl, saturated or partially unsaturated heterocyclyl, aryl or heteroaryl; wherein, the alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^3$ each optionally are independently substituted by one or more substituents selected from the group consisting of hydroxyl, amino, cyano, halogen, oxo and alkyl.

In some other examples, each $R^3$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, $C_{1-6}$haloheteroalkyl, 3-6 membered saturated or partially unsaturated carbocyclyl, 3-6 membered saturated or partially unsaturated carbocyclyl$C_{1-6}$alkyl, 3-6 membered saturated or partially unsaturated heterocyclyl, 3-6 membered saturated or partially unsaturated heterocyclyl$C_{1-6}$alkyl, 6-10 membered aryl, 6-10 membered aryl$C_{1-6}$alkyl, 6-10 membered heteroaryl or 6-10 membered heteroaryl$C_{1-6}$alkyl; if there are two adjacent $R^3$, the two adjacent $R^3$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^3$ each optionally are independently substituted by one or more substituents selected from the group consisting of deuterium, hydroxyl, amino, cyano, halogen, oxo, and $C_{1-4}$alkyl.

In some other examples, each $R^3$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, $C_{1-6}$haloheteroalkyl; if there are two adjacent $R^3$, the two adjacent $R^3$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, saturated or partially unsaturated carbocyclyl, saturated or partially unsaturated heterocyclyl, aryl, or heteroaryl involved in $R^3$ each optionally are independently substituted by one or more substituents selected from the group consisting of deuterium, hydroxyl, amino, cyano, halogen, oxo, and $C_{1-4}$alkyl. In some other examples, each $R^3$ is independently hydrogen, deuterium, amino, hydroxyl, cyano, halogen, $C_{1-6}$alkyl, $C_{1-6}$heteroalkyl, $C_{1-6}$haloalkyl, or $C_{1-6}$haloheteroalkyl; if there are two adjacent $R^3$, the two adjacent $R^3$ and their connected atoms optionally form 3-6 membered saturated or partially unsaturated carbocyclyl, 3-6 membered saturated or partially unsaturated heterocyclyl; wherein the alkyl, heteroalkyl, haloalkyl, haloheteroalkyl, 3-6 membered saturated or partially unsaturated carbocyclyl, 3-6 membered saturated or partially unsaturated heterocyclyl involved in $R^3$ each optionally are independently substituted by one or more substituents selected from the group consisting of deuterium, hydroxyl, amino, cyano, halogen, oxo, and $C_{1-4}$alkyl. In some other examples, each $R^3$ is independently hydrogen, deuterium, amino, hydroxyl, cyano, halogen, $C_{1-4}$alkyl, $C_{1-4}$heteroalkyl, $C_{1-4}$haloalkyl, or $C_{1-4}$haloheteroalkyl; if there are two adjacent $R^3$, the two adjacent $R^3$ and their connected atoms optionally form 3-6 membered saturated or partially unsaturated carbocyclyl, 3-6 membered saturated or partially unsaturated heterocyclyl; wherein the alkyl, heteroalkyl, haloalkyl, haloheteroalkyl, 3-6 membered saturated or partially unsaturated carbocyclyl, 3-6 membered saturated or partially unsaturated heterocyclyl involved in $R^3$ each optionally are independently substituted by one or more substituents selected from the group consisting of deuterium, hydroxyl, amino, cyano, halogen, oxo, methyl, ethyl, and isopropyl.

In some other examples, each $R^3$ is independently hydrogen, deuterium, amino, hydroxyl, cyano, fluoro, chloro, methyl, ethyl, propyl, isopropyl, *t*-butyl, methoxy, ethoxy, i-propoxy, *t*-butoxy, trifluoromethyl, trifluoromethoxy, $CH_3C(=O)$-, $CH_3CH_2C(=O)$-, $(CH_3)_2CHC(=O)$-, $CH_3C(=O)O$-, $CH_3CH_2C(=O)O$-, $(CH_3)_2CHC(=O)O$-, $CH_3C(=O)NH$-, $CH_3CH_2C(=O)NH$-, $(CH_3)_2CHC(=O)NH$-, $CH_3NHC(=O)$-, $CH_3CH_2NHC(=O)$-, $(CH_3)_2CHNHC(=O)$-, $CH_3ONHC(=O)$-,

$CH_3CH_2ONHC(=O)-$, $(CH_3)_2CHONHC(=O)-$, $CH_3S(=O)_2-$, $CH_3CH_2S(=O)_2-$, $(CH_3)_2CHS(=O)_2-$, $CH_3S(=O)_2O-$, $CH_3CH_2S(=O)_2O-$, $(CH_3)_2CHS(=O)_2O-$, $CH_3S(=O)_2NH-$, $CH_3CH_2S(=O)_2NH-$, $(CH_3)_2CHS(=O)_2NH-$, $CH_3NHS(=O)_2-$, $CH_3CH_2NHS(=O)_2-$, $(CH_3)_2CHNHS(=O)_2-$, $CH_3C(=O)N(CH_3)-$, $CH_3CH_2C(=O)N(CH_3)-$, $(CH_3)_2CHC(=O)N(CH_3)-$, $CH_3N(CH_3)C(=O)-$, $CH_3CH_2N(CH_3)C(=O)-$, $(CH_3)_2CHN(CH_3)C(=O)-$, $CH_3ON(CH_3)C(=O)-$, $CH_3CH_2ON(CH_3)C(=O)-$, $(CH_3)_2CHON(CH_3)C(=O)-$, $CH_3S(=O)_2N(CH_3)-$, $CH_3CH_2S(=O)_2N(CH_3)-$, $(CH_3)_2CHS(=O)_2N(CH_3)-$, $CH_3N(CH_3)S(=O)_2-$, $CH_3CH_2N(CH_3)S(=O)_2-$, $(CH_3)_2CHN(CH_3)S(=O)_2-$, $CH_3C(=O)N(CH_2CH_3)-$, $CH_3CH_2C(=O)N(CH_2CH_3)-$, $(CH_3)_2CHC(=O)N(CH_2CH_3)-$, $CH_3N(CH_2CH_3)C(=O)-$, $CH_3CH_2N(CH_2CH_3)C(=O)-$, $(CH_3)_2CHN(CH_2CH_3)C(=O)-$, $CH_3ON(CH_2CH_3)C(=O)-$, $CH_3CH_2ON(CH_2CH_3)C(=O)-$, $(CH_3)_2CHON(CH_2CH_3)C(=O)-$, $CH_3S(=O)_2N(CH_2CH_3)-$, $CH_3CH_2S(=O)_2N(CH_2CH_3)-$, $(CH_3)_2CHS(=O)_2N(CH_2CH_3)-$, $CH_3N(CH_2CH_3)S(=O)_2-$, $CH_3CH_2N(CH_2CH_3)S(=O)_2-$, $(CH_3)_2CHN(CH_2CH_3)S(=O)_2-$, $(CH_3)_2P(=O)-$, $CH_3CH_2(CH_3)P(=O)-$, $(CH_3)_2CH(CH_3)P(=O)-$, $(CH_3CH_2)_2P(=O)-$, $CH_3CH_z(CH_3)P(=O)-$, or $(CH_3)_2CH(CH_3CH_2)P(=O)-$; if there are two adjacent $R^3$, the two adjacent $R^3$ and their connected atoms optionally form the following groups:

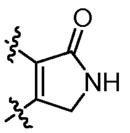, 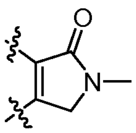, 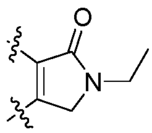, 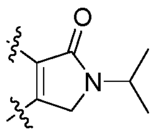, 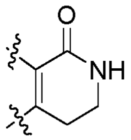, 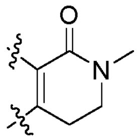, 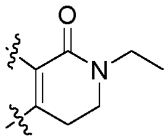,

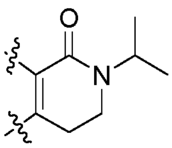, 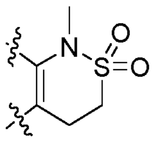 or 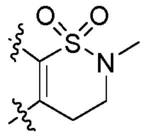.

In some examples, n, m and p each are independently 0, 1, 2, 3 or 4.

In some examples, the saturated or partially unsaturated carbocyclyl includes saturated or partially unsaturated monocyclic carbocyclyl, saturated or partially unsaturated spirocyclic carbocyclyl, saturated or partially unsaturated fused cyclic carbocyclyl, saturated or partially unsaturated bridged cyclic carbocyclyl.

In some examples, the saturated or partially unsaturated carbocyclyl is selected from formulae ii-1 to ii-6; when formulae ii-1 to ii-6 are connected to the rest of the molecule through one bond, formulae ii-1 to ii-6 are monovalent; when formulae ii-1 to ii-6 are connected to the rest of the molecule through two bonds, formulae ii-1 to ii-6 are bivalent; the saturated or partially unsaturated carbocyclyl represented by formulae ii-1 to ii-6 each optionally are independently substituted by one or more substituents of the present application;

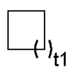
ii-1,
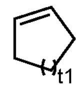
ii-2,
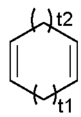
ii-3,
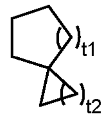
ii-4,
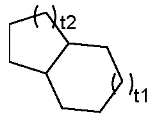
ii-5 or
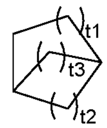
ii-6;

wherein, each t1 is independently 0, 1, 2, 3, 4, or 5; t2 and t3 each are independently 1, 2, 3, 4, or 5.

In some examples, saturated or partially unsaturated heterocyclyl includes saturated or partially unsaturated monocyclic heterocyclyl, saturated or partially unsaturated spirocyclic heterocyclyl, saturated or partially unsaturated fused cyclic heterocyclyl, saturated or partially unsaturated bridged cyclic heterocyclyl. In some examples, the saturated or partially unsaturated heterocyclyl is selected from formulae iii-1 to iii-16; when formulae iii-1 to iii-16 are connected to the rest of the molecule through one bond, formulae iii-1 to iii-16 are monovalent; when formulae iii-1 to iii-16 are connected to the rest of the molecule through two bonds, formulae iii-1 to iii-16 are bivalent; saturated or partially unsaturated heterocyclyl represented by formulae iii-1 to iii-16 each optionally are independently substituted by one or more substituents of the present application;

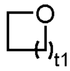 iii-1 , 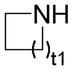 iii-2 , 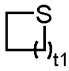 iii-3 , 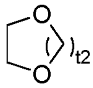 iii-4 , 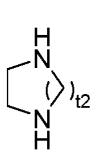 iii-5 , 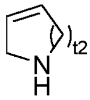 iii-6 , 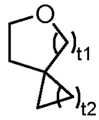 iii-7 , 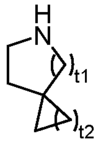 iii-8 , 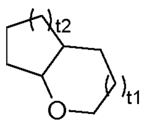 iii-9 , 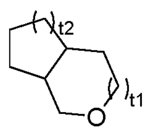 iii-10 ,

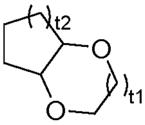 iii-11 , 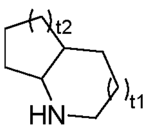 iii-12 , 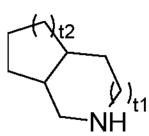 iii-13 , 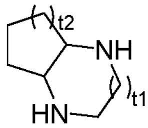 iii-14 , 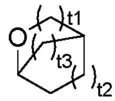 iii-15 or 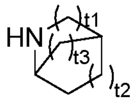 iii-16 ;

wherein, each t1 is independently 0, 1, 2, 3, 4, or 5; t2 and t3 each are independently 1, 2, 3, 4, or 5.

In some other examples, the saturated or partially unsaturated carbocyclyl is selected from the following groups; when the saturated or partially unsaturated carbocyclyl is connected to the rest of the molecule through one bond, the following groups are monovalent; when the saturated or partially unsaturated carbocyclyl is connected to the rest of the molecule through two bonds, the following groups are bivalent; the following saturated or partially unsaturated carbocyclyl each optionally are independently substituted by one or more substituents of the present application;

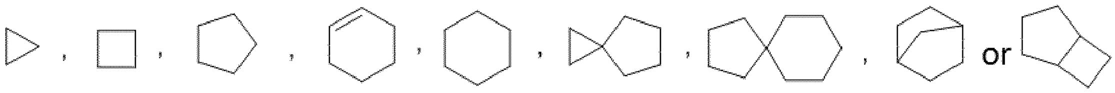

In some other examples, the saturated or partially unsaturated heterocyclyl is selected from the following groups; when the saturated or partially unsaturated heterocyclyl is connected to the rest of the molecule through one bond, the following groups are monovalent; when the saturated or partially unsaturated heterocyclyl is connected to the rest of the molecule through two bonds, the following groups are bivalent; the following saturated or partially unsaturated heterocyclyl each optionally are independently substituted by one or more substituents of the present application;

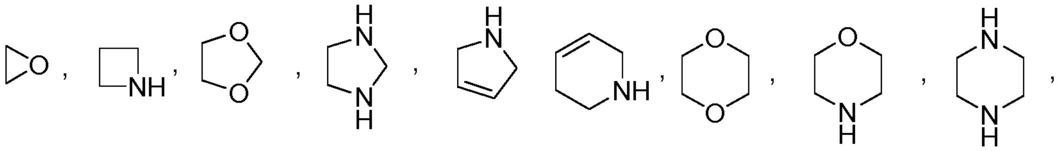

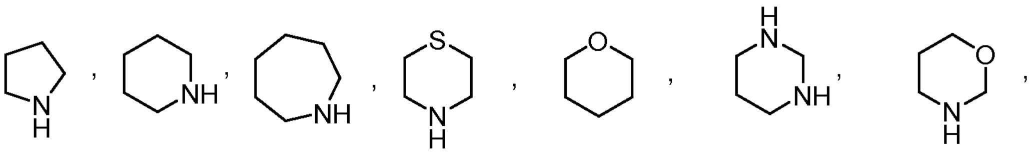

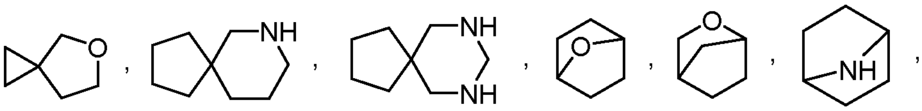

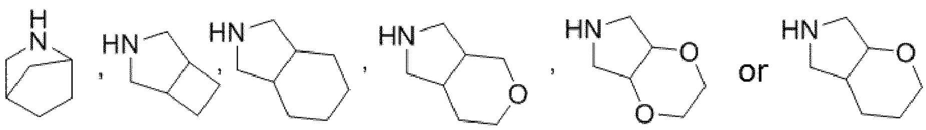

In some examples, the compound of the present application is a compound of formula II-1, or a stereoisomer,

a tautomer, a nitrogen oxide, a solvate, a metabolite, a pharmaceutically acceptable salt, or a prodrug thereof:

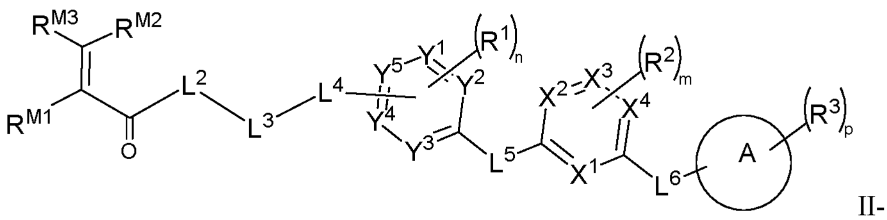

II-1

wherein, each $R^{M1}$, $R^{M2}$, $R^{M3}$, $L^2$, $L^3$, $L^4$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, A, n, m, and p are defined as described in the present application.

In some examples, the compound of the present application is a compound of formula II-2, or a stereoisomer, a tautomer, a nitrogen oxide, a solvate, a metabolite, a pharmaceutically acceptable salt, or a prodrug thereof:

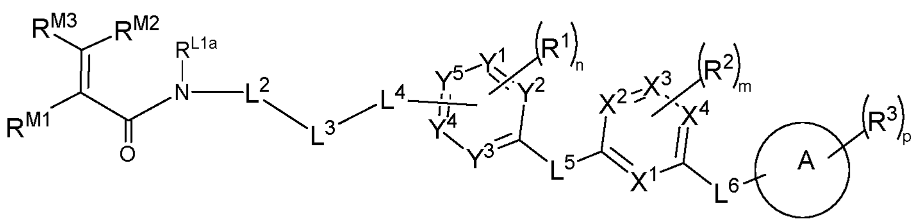

II-2

wherein, each $R^{M1}$, $R^{M2}$, $R^{M3}$, $R^{L1a}$, $L^2$, $L^3$, $L^4$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, A, n, m, and p are defined as described in the present application.

In some examples, the compound of the present application is a compound of formula II-3, or a stereoisomer, a tautomer, a nitrogen oxide, a solvate, a metabolite, a pharmaceutically acceptable salt, or a prodrug thereof:

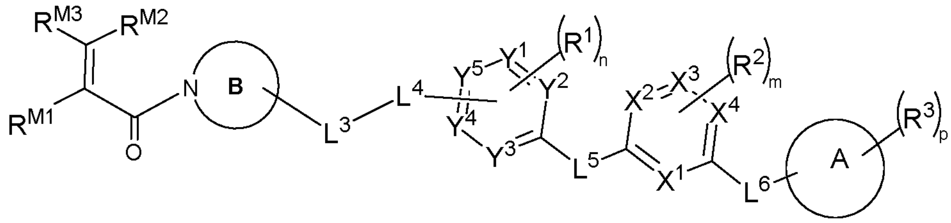

II-3

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; wherein, each $R^{M1}$, $R^{M2}$, $R^{M3}$, $L^3$, $L^4$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, A, n, m, and p are defined as described in the present application.

In some examples, the compound of the present application is a compound of formula II-4 or a stereoisomer, a tautomer, a nitrogen oxide, a solvate, a metabolite, a pharmaceutically acceptable salt, or a prodrug thereof:

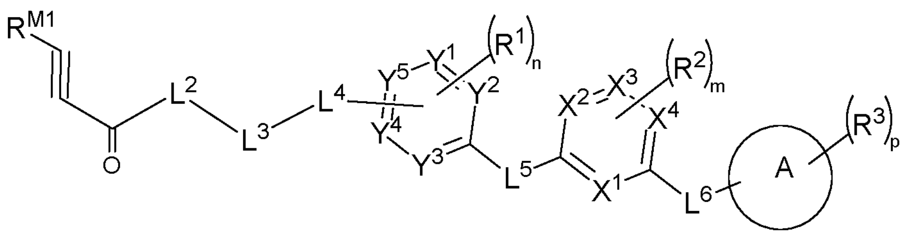

II-4

wherein, each $R^{M1}$, $L^2$, $L^3$, $L^4$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, A, n, m, and p are defined as described in the present application.

In some examples, the compound of the present application is a compound of formula II-5 or a stereoisomer,

a tautomer, a nitrogen oxide, a solvate, a metabolite, a pharmaceutically acceptable salt, or a prodrug thereof:

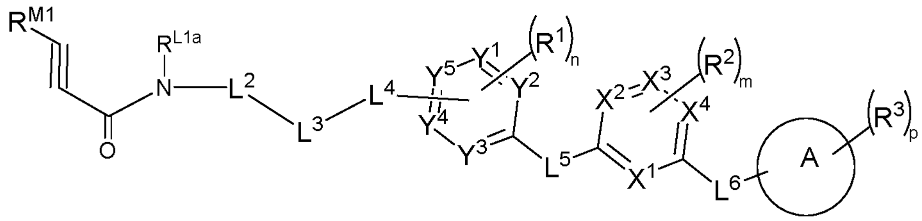

II-5

wherein, each $R^{M1}$, $R^{L1a}$, $L^2$, $L^3$, $L^4$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, A, n, m, and p are defined as described in the present application.

In some examples, the compound of the present application is a compound of formula II-6 or a stereoisomer, a tautomer, a nitrogen oxide, a solvate, a metabolite, a pharmaceutically acceptable salt, or a prodrug thereof:

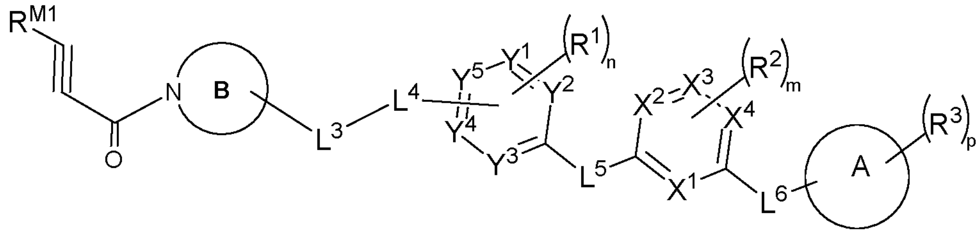

II-6

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; wherein, each $R^{M1}$, $L^3$, $L^4$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, A, n, m, and p are defined as described in the present application.

In some examples, the compound of the present application is a compound of formula III or a stereoisomer, a tautomer, a nitrogen oxide, a solvate, a metabolite, a pharmaceutically acceptable salt, or a prodrug thereof:

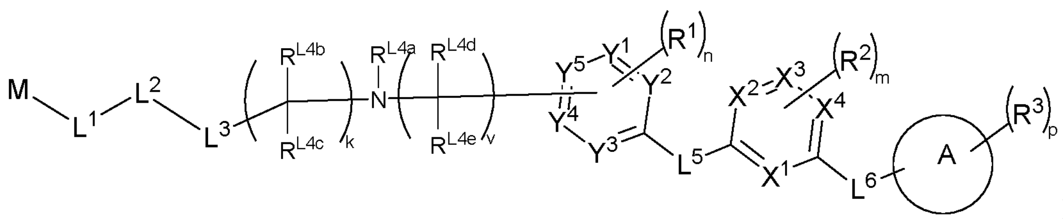

III

wherein, $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; k and v each are independently 0, 1 or 2; wherein, each M, $L^1$, $L^2$, $L^3$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, A, n, m, and p are defined as described in the present application.

In some examples, the compound of the present application is a compound of formula IV-1 or a stereoisomer, a tautomer, a nitrogen oxide, a solvate, a metabolite, a pharmaceutically acceptable salt, or a prodrug thereof:

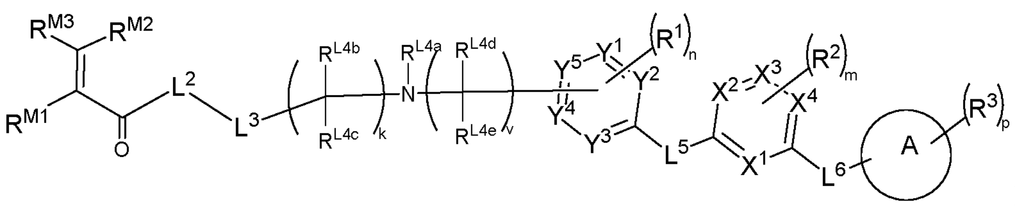

IV-1

wherein, $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; k and v each are independently 0, 1 or 2; wherein, each $R^{M1}$, $R^{M2}$, $R^{M3}$, $L^2$, $L^3$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, A, n, m, and p are defined as described in the present application.

In some examples, the compound of the present application is a compound of formula IV-2 or a stereoisomer, a tautomer, a nitrogen oxide, a solvate, a metabolite, a pharmaceutically acceptable salt, or a prodrug thereof:

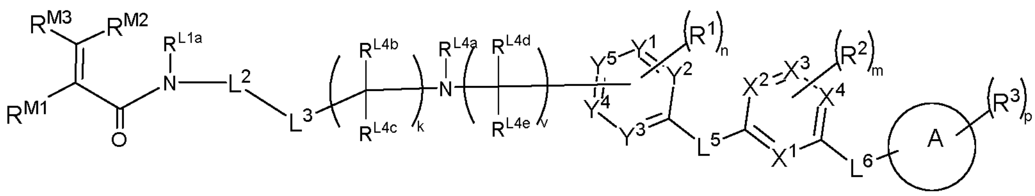

（IV-2）

wherein,

$R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group;

each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; k and v each are independently 0, 1 or 2; wherein, each $R^{M1}$, $R^{M2}$, $R^{M3}$, $R^{L1a}$, $L^2$, $L^3$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, A, n, m, and p are defined as described in the present application.

In some examples, the compound of the present application is a compound of formula IV-3 or a stereoisomer, a tautomer, a nitrogen oxide, a solvate, a metabolite, a pharmaceutically acceptable salt, or a prodrug thereof:

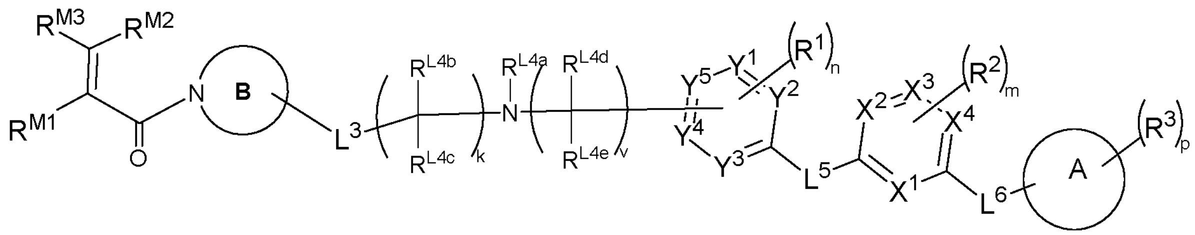

IV-3

wherein,

B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl;

$R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group;

each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo;

k and v each are independently 0, 1 or 2;

wherein, each $R^{M1}$, $R^{M2}$, $R^{M3}$, $L^3$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, A, n, m, and p are defined as described in the present application.

In some examples, the compound of the present application is a compound of formula IV-4 or a stereoisomer, a tautomer, a nitrogen oxide, a solvate, a metabolite, a pharmaceutically acceptable salt, or a prodrug thereof:

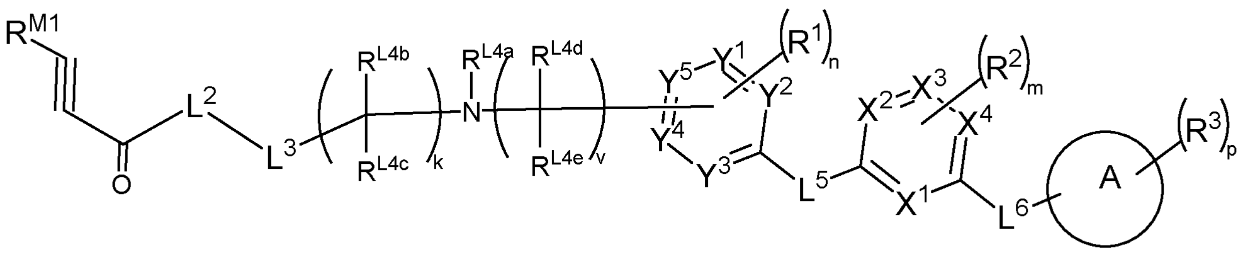

IV-4

wherein, $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; k and v each are independently 0, 1 or 2; wherein, each $R^{M1}$, $L^2$, $L^3$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, A, n, m, and p are defined as described in the present application.

In some examples, the compound of the present application is a compound of formula IV-5 or a stereoisomer, a tautomer, a nitrogen oxide, a solvate, a metabolite, a pharmaceutically acceptable salt, or a prodrug thereof:

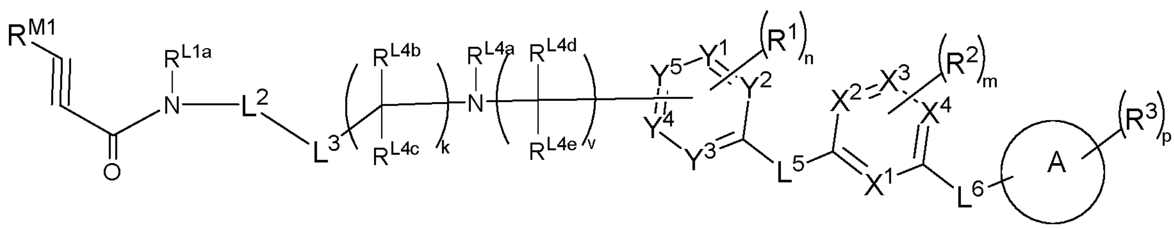

IV-5

wherein, $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; k and v each are independently 0, 1 or 2; wherein, each $R^{M1}$, $R^{L1a}$, $L^2$, $L^3$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, A, n, m, and p are defined as described in the present application.

In some examples, the compound of the present application is a compound of formula IV-6 or a stereoisomer, a tautomer, a nitrogen oxide, a solvate, a metabolite, a pharmaceutically acceptable salt, or a prodrug thereof:

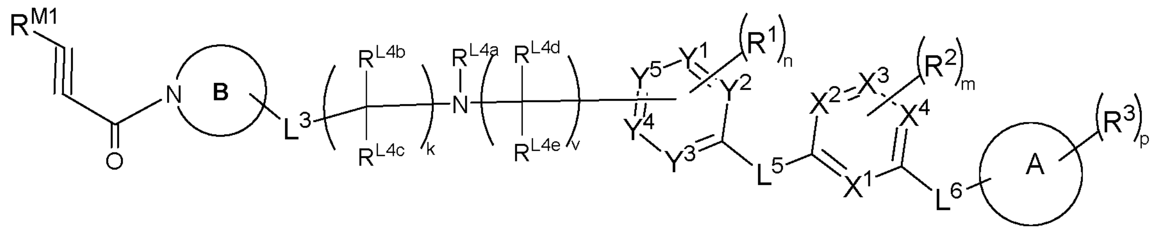

IV-6

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; k and v each are independently 0, 1 or 2; wherein, each $R^{M1}$, $L^3$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, A, n, m, and p are defined as described in the present application.

In some examples, the compound of the present application is a compound of formula V-1, or a stereoisomer, a tautomer, a nitrogen oxide, a solvate, a metabolite, a pharmaceutically acceptable salt, or a prodrug thereof:

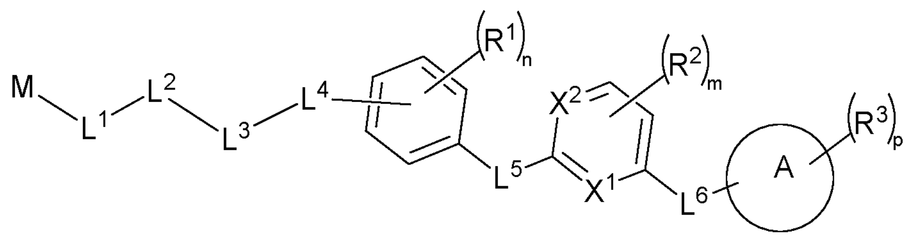

V-1

wherein, each M, $L^1$, $L^2$, $L^3$, $L^4$, $L^5$, $L^6$, $X^1$, $X^2$, $R^1$, $R^2$, $R^3$, A, n, m, and p are defined as described in the present application.

In some examples, the compound of the present application is a compound of formula V-2 or a stereoisomer, a tautomer, a nitrogen oxide, a solvate, a metabolite, a pharmaceutically acceptable salt, or a prodrug thereof:

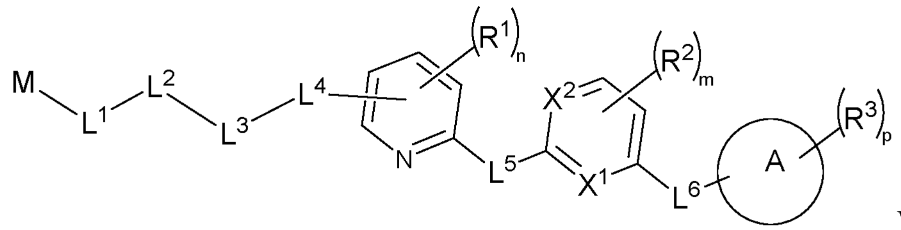

V-2

wherein, each M, $L^1$, $L^2$, $L^3$, $L^4$, $L^5$, $L^6$, $X^1$, $X^2$, $R^1$, $R^2$, $R^3$, A, n, m, and p are defined as described in the present application.

In some examples, the compound of the present application is a compound of formula V-3 or a stereoisomer, a tautomer, a nitrogen oxide, a solvate, a metabolite, a pharmaceutically acceptable salt, or a prodrug thereof:

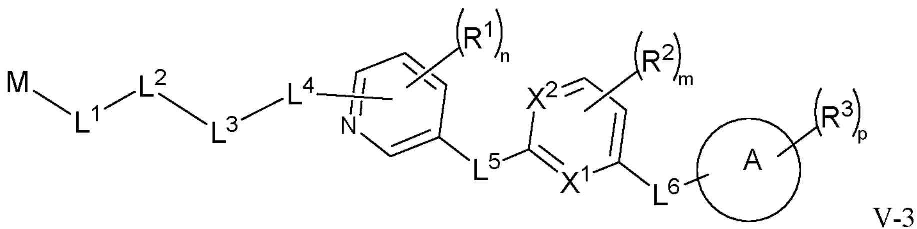

V-3

wherein, each M, $L^1$, $L^2$, $L^3$, $L^4$, $L^5$, $L^6$, $X^1$, $X^2$, $R^1$, $R^2$, $R^3$, A, n, m, and p are defined as described in the present application.

In some examples, the compound of the present application is a compound of formula V-4 or a stereoisomer, a tautomer, a nitrogen oxide, a solvate, a metabolite, a pharmaceutically acceptable salt, or a prodrug thereof:

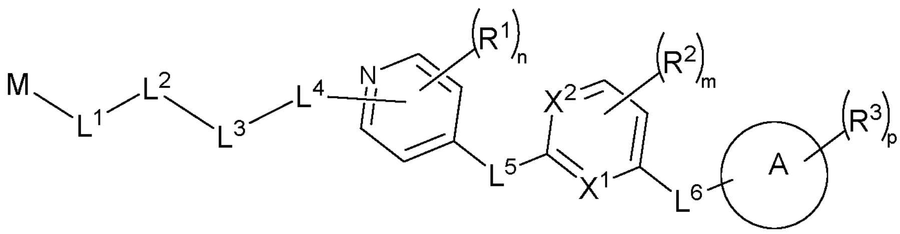

V-4

wherein, each M, $L^1$, $L^2$, $L^3$, $L^4$, $L^5$, $L^6$, $X^1$, $X^2$, $R^1$, $R^2$, $R^3$, A, n, m, and p are defined as described in the present application.

In some examples, the compound of the present application is a compound of formula V-5 or a stereoisomer, a tautomer, a nitrogen oxide, a solvate, a metabolite, a pharmaceutically acceptable salt, or a prodrug thereof:

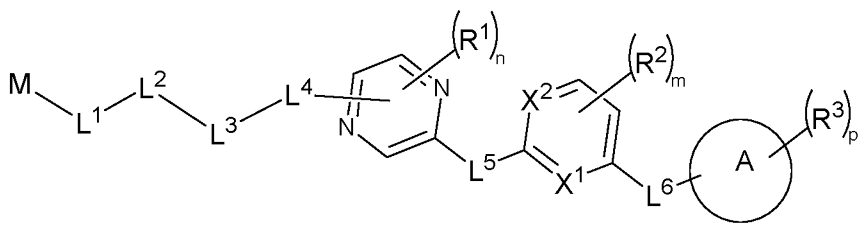

V-5

wherein, each M, $L^1$, $L^2$, $L^3$, $L^4$, $L^5$, $L^6$, $X^1$, $X^2$, $R^1$, $R^2$, $R^3$, A, n, m, and p are defined as described in the present application.

In some examples, the compound of the present application is a compound of formula V-6 or a stereoisomer, a tautomer, a nitrogen oxide, a solvate, a metabolite, a pharmaceutically acceptable salt, or a prodrug thereof:

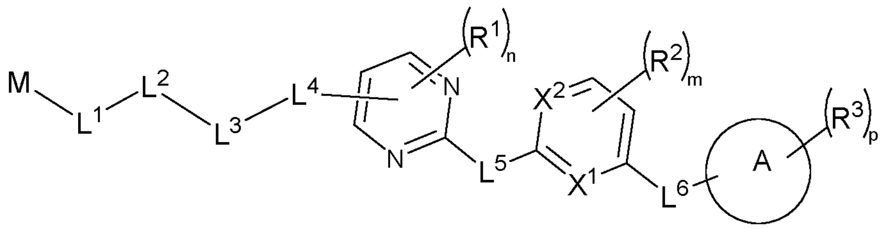

V-6

wherein, each M, $L^1$, $L^2$, $L^3$, $L^4$, $L^5$, $L^6$, $X^1$, $X^2$, $R^1$, $R^2$, $R^3$, A, n, m, and p are defined as described in the present application.

In some examples, the compound of the present application is a compound of formula V-7 or a stereoisomer, a tautomer, a nitrogen oxide, a solvate, a metabolite, a pharmaceutically acceptable salt, or a prodrug thereof:

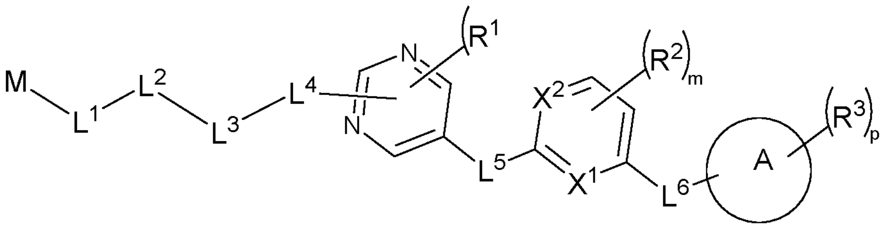

V-7

wherein, each M, $L^1$, $L^2$, $L^3$, $L^4$, $L^5$, $L^6$, $X^1$, $X^2$, $R^1$, $R^2$, $R^3$, A, n, m, and p are defined as described in the present application.

In some examples, the compound of the present application is a compound of formula VI-1 or a stereoisomer, a tautomer, a nitrogen oxide, a solvate, a metabolite, a pharmaceutically acceptable salt, or a prodrug thereof:

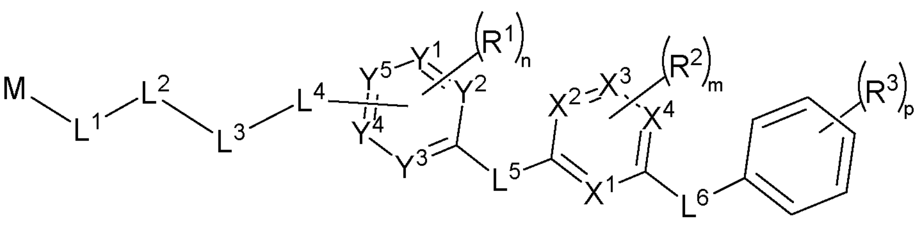

VI-1

wherein, each M, $L^1$, $L^2$, $L^3$, $L^4$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, n, m, and p are defined as described in the present application.

In some examples, the compound of the present application is a compound of formula VI-2 or a stereoisomer, a tautomer, a nitrogen oxide, a solvate, a metabolite, a pharmaceutically acceptable salt, or a prodrug thereof:

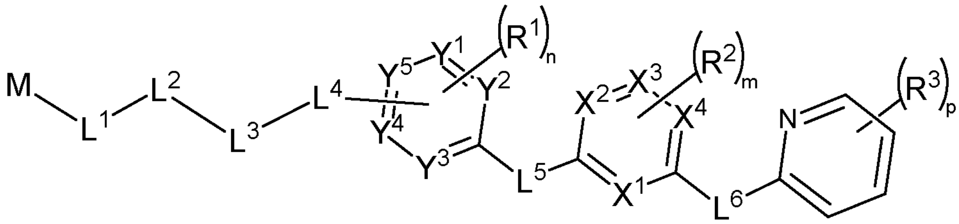

VI-2

wherein, each M, $L^1$, $L^2$, $L^3$, $L^4$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, n, m, and p are defined as described in the present application.

In some examples, the compound of the present application is a compound of formula VI-3 or a stereoisomer, a tautomer, a nitrogen oxide, a solvate, a metabolite, a pharmaceutically acceptable salt, or a prodrug thereof:

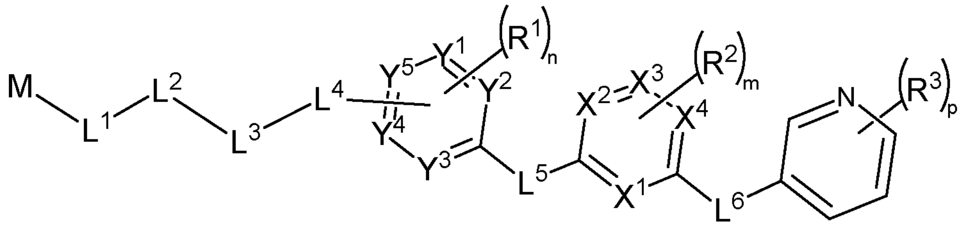

VI-3

wherein, each M, $L^1$, $L^2$, $L^3$, $L^4$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, n, m, and p are defined as described in the present application.

In some examples, the compound of the present application is a compound of formula VI-4 or a stereoisomer, a tautomer, a nitrogen oxide, a solvate, a metabolite, a pharmaceutically acceptable salt, or a prodrug thereof:

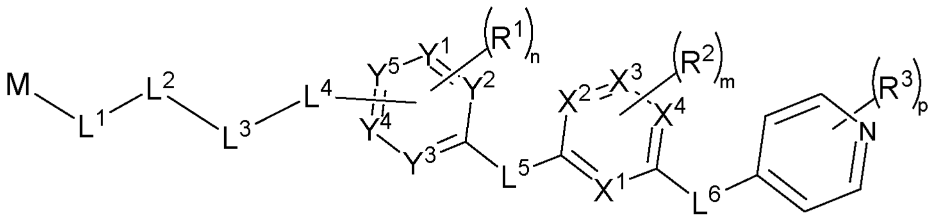

VI-4

wherein, each M, $L^1$, $L^2$, $L^3$, $L^4$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, n, m, and p are defined as described in the present application.

In some examples, the compound of the present application is a compound of formula VI-5 or a stereoisomer, a tautomer, a nitrogen oxide, a solvate, a metabolite, a pharmaceutically acceptable salt, or a prodrug thereof::

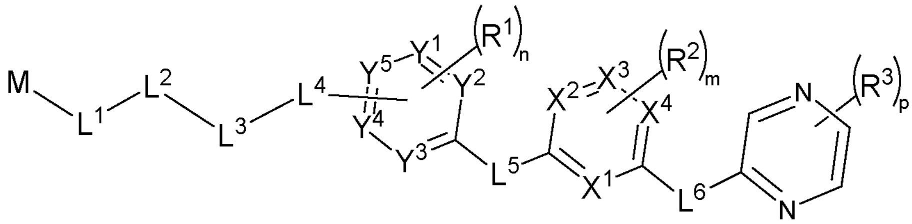

VI-5

wherein, each M, $L^1$, $L^2$, $L^3$, $L^4$, $L^5$, $L^6$, $X^1$, $X^2$, $X^3$, $X^4$, $Y^1$, $Y^2$, $Y^3$, $Y^4$, $Y^5$, $R^1$, $R^2$, $R^3$, n, m, and p are defined as described in the present application.

In some examples, the compound of the present application is a compound of formula VII-1 or a stereoisomer, a tautomer, a nitrogen oxide, a solvate, a metabolite, a pharmaceutically acceptable salt, or a prodrug thereof:

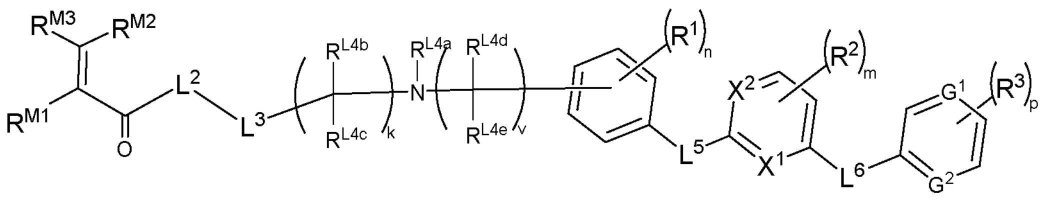

VII-1

wherein, $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; $G^1$ and $G^2$ each are independently CH or N; k and v each are independently 0, 1 or 2; wherein, each $R^{M1}$, $R^{M2}$, $R^{M3}$, $L^2$, $L^3$, $L^5$, $L^6$, $X^1$, $X^2$, $R^1$, $R^2$, $R^3$, n, m, and p are defined as described in the present application.

In some examples, the compound of the present application is a compound of formula VII-2, or a stereoisomer, a tautomer, a nitrogen oxide, a solvate, a metabolite, a pharmaceutically acceptable salt, or a prodrug thereof:

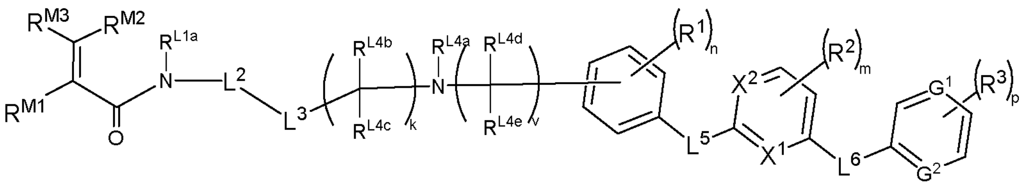

VII-2

wherein, $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; $G^1$ and $G^2$ each are independently CH or N; k and v each are independently 0, 1 or 2; wherein, each $R^{M1}$, $R^{M2}$, $R^{M3}$, $R^{L1a}$, $L^2$, $L^3$, $L^5$, $L^6$, $X^1$, $X^2$, $R^1$, $R^2$, $R^3$, n, m, and p are defined as described in the present application.

In some examples, the compound of the present application is a compound of formula VII-3, or a stereoisomer, a tautomer, a nitrogen oxide, a solvate, a metabolite, a pharmaceutically acceptable salt, or a prodrug thereof:

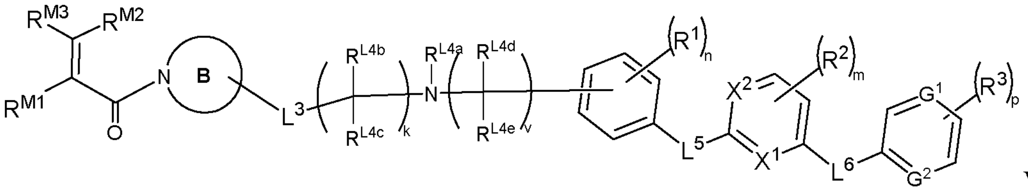

VII-3

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; G1 and G2 each are independently CH or N; k and v each are independently 0, 1 or 2; wherein, each $R^{M1}$, $R^{M2}$, $R^{M3}$, $L^3$, $L^5$, $L^6$, $X^1$, $X^2$, $R^1$, $R^2$, $R^3$, n, m, and p are defined as described in the present application.

In some examples, the compound of the present application is a compound of formula VII-4, or a stereoisomer, a tautomer, a nitrogen oxide, a solvate, a metabolite, a pharmaceutically acceptable salt, or a prodrug thereof:

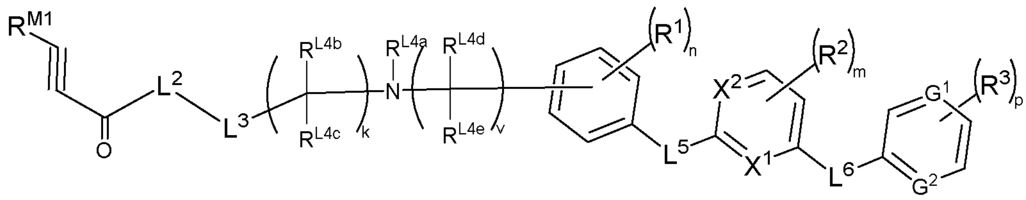

VII-4

wherein, $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; $G^1$ and $G^2$ each are independently CH or N; k and v each are independently 0, 1 or 2; wherein, each $R^{M1}$, $L^2$, $L^3$, $L^5$, $L^6$, $X^1$, $X^2$, $R^1$, $R^2$, $R^3$, n, m, and p are defined as described in the present application.

In some examples, the compound of the present application is a compound of formula VII-5, or a stereoisomer, a tautomer, a nitrogen oxide, a solvate, a metabolite, a pharmaceutically acceptable salt, or a prodrug thereof:

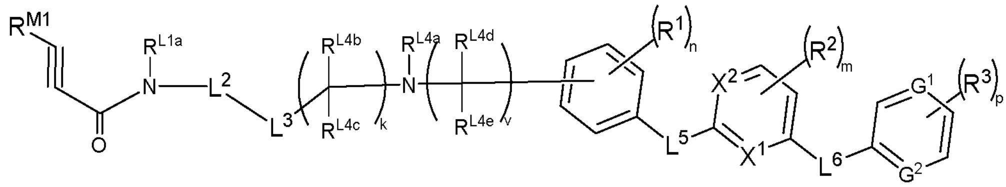

VII-5

wherein, $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; $G^1$ and $G^2$ each are independently CH or N; k and v each are independently 0, 1 or 2; wherein, each $R^{M1}$, $R^{L1a}$, $L^2$, $L^3$, $L^5$, $L^6$, $X^1$, $X^2$, $R^1$, $R^2$, $R^3$, n, m, and p are defined as described in the present application.

In some examples, the compound of the present application is a compound of formula VII-6 or a stereoisomer, a tautomer, a nitrogen oxide, a solvate, a metabolite, a pharmaceutically acceptable salt, or a prodrug thereof:

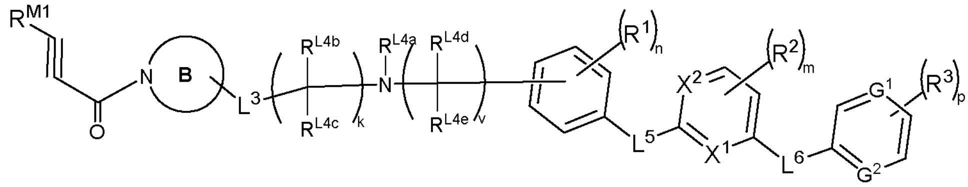

VII-6

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; $G^1$ and $G^2$ each are independently CH or N; k and v each are independently 0, 1 or 2; wherein, each $R^{M1}$, $L^3$, $L^5$, $L^6$, $X^1$, $X^2$, $R^1$, $R^2$, $R^3$, n, m, and p are defined as described in the present application.

In some examples, the compound of the present application is a compound of formula VIII-1, or a stereoisomer, a tautomer, a nitrogen oxide, a solvate, a metabolite, a pharmaceutically acceptable salt, or a prodrug thereof:

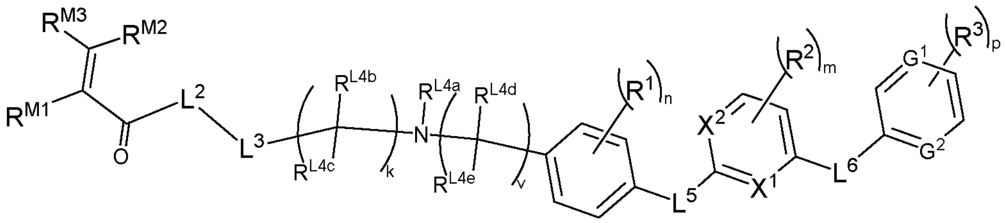

VIII-1

wherein, $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; $G^1$ and $G^2$ each are independently CH or N; k and v each are independently 0, 1 or 2; wherein, each $R^{M1}$, $R^{M2}$, $R^{M3}$, $L^2$, $L^3$, $L^5$, $L^6$, $X^1$, $X^2$, $R^1$, $R^2$, $R^3$, n, m, and p are defined as described in the present application.

In some examples, the compound of the present application is a compound of formula VIII-2, or a stereoisomer, a tautomer, a nitrogen oxide, a solvate, a metabolite, a pharmaceutically acceptable salt, or a prodrug thereof:

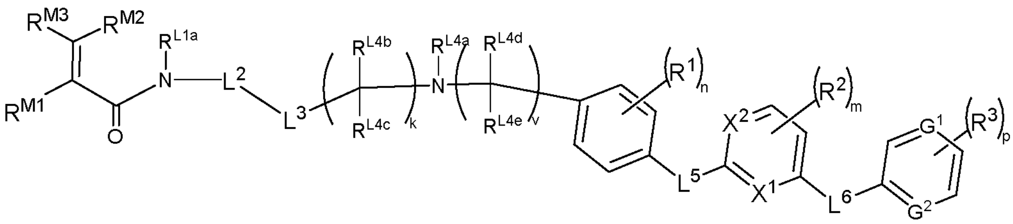

VIII-2

wherein, $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; $G^1$ and $G^2$ each are independently CH or N; k and v each are independently 0, 1 or 2; wherein, each $R^{M1}$, $R^{M2}$, $R^{M3}$, $R^{L1a}$, $L^2$, $L^3$, $L^5$, $L^6$, $X^1$, $X^2$, $R^1$, $R^2$, $R^3$, n, m, and p are defined as described in the present application.

In some examples, the compound of the present application is a compound of formula VIII-3, or a stereoisomer, a tautomer, a nitrogen oxide, a solvate, a metabolite, a pharmaceutically acceptable salt, or a prodrug thereof:

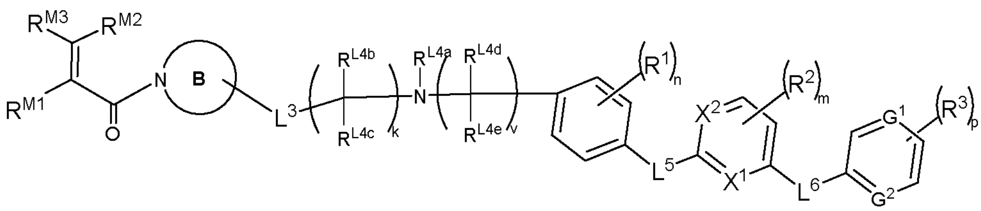

VIII-3

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; $G^1$ and $G^2$ each are independently CH or N; k and v each are independently 0, 1 or 2; wherein, each $R^{M1}$, $R^{M2}$, $R^{M3}$, $L^3$, $L^5$, $L^6$, $X^1$, $X^2$, $R^1$, $R^2$, $R^3$, n, m, and p are defined as described in the present application.

In some examples, the compound of the present application is a compound of formula VIII-4, or a stereoisomer, a tautomer, a nitrogen oxide, a solvate, a metabolite, a pharmaceutically acceptable salt, or a prodrug thereof:

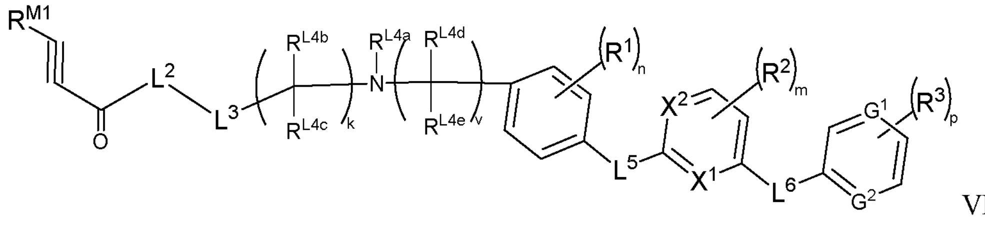

VIII-4

wherein, $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; $G^1$ and $G^2$ each are independently CH or N; k and v each are independently 0, 1 or 2; wherein, each $R^{M1}$, $L^2$, $L^3$, $L^5$, $L^6$, $X^1$, $X^2$, $R^1$, $R^2$, $R^3$, n, m, and p are defined as described in the present application.

In some examples, the compound of the present application is a compound of formula VIII-5, or a stereoisomer, a tautomer, a nitrogen oxide, a solvate, a metabolite, a pharmaceutically acceptable salt, or a prodrug thereof:

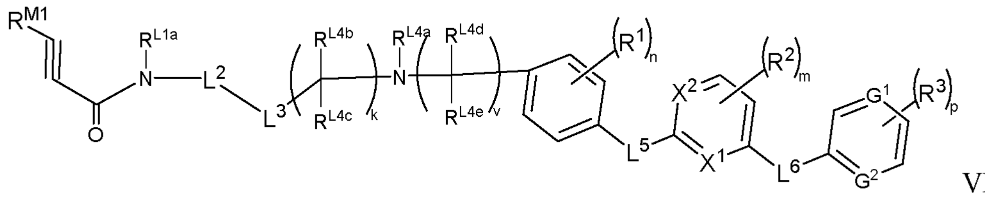

VIII-5

wherein, $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; $G^1$ and $G^2$ each are independently CH or N; k and v each are independently 0, 1 or 2; wherein, each $R^{M1}$, $R^{L1a}$, $L^2$, $L^3$, $L^5$, $L^6$, $X^1$, $X^2$, $R^1$, $R^2$, $R^3$, n, m, and p are defined as described in the present application.

In some examples, the compound of the present application is a compound of formula VIII-6, or a stereoisomer, a tautomer, a nitrogen oxide, a solvate, a metabolite, a pharmaceutically acceptable salt, or a prodrug thereof:

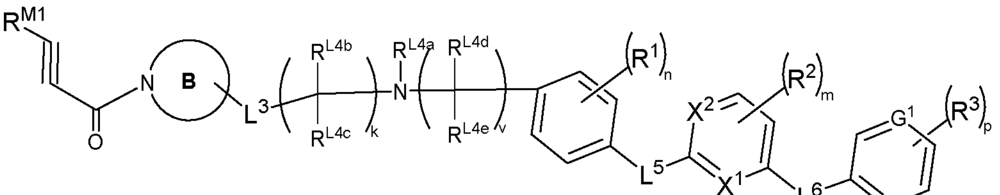

VIII-6

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; $G^1$ and $G^2$ each are independently CH or N; k and v each are independently 0, 1 or 2; wherein, each $R^{M1}$, $L^3$, $L^5$, $L^6$, $X^1$, $X^2$, $R^1$, $R^2$, $R^3$, n, m, and p are defined as described in the present application.

In one aspect, the present application provides a compound, which is a compound of formula I, or a stereoisomer, a tautomer, a nitrogen oxide, a solvate, a metabolite, a pharmaceutically acceptable salt, or a prodrug thereof:

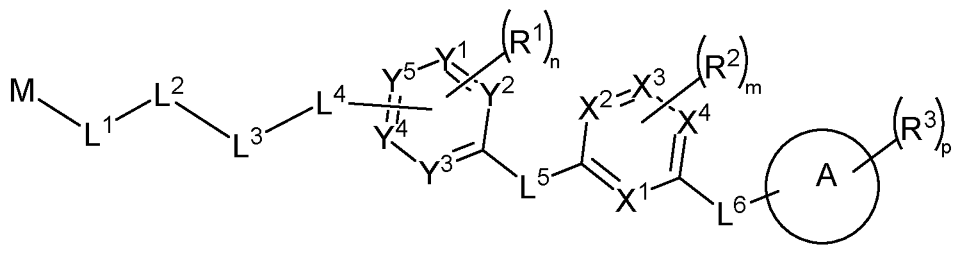

(I)

wherein,

M is a covalent warhead;

A is aryl or heteroaryl;

$L^1$ is a bond, -C(=O)-, -S(=O)-, $-S(=O)_2-$, $-C(=O)NR^{L1a}-$, $-NR^{L1a}C(=O)-$, $-S(=O)_2NR^{L1a}-$, or $-NR^{L1a}S(=O)_2-$; wherein, each $R^{L1a}$ is independently hydrogen, alkyl, alkenyl, alkynyl, heteroalkyl, haloalkyl, haloheteroalkyl, or a nitrogen protecting group; wherein the alkyl, alkenyl, alkynyl, heteroalkyl, haloalkyl, haloheteroalkyl involved in $R^{L1a}$ each optionally are independently substituted by one or more substituents selected from the group consisting of deuterium, halogen, hydroxyl, amino, cyano, and oxo;

$L^2$ is a bond, alkyl, heteroalky, saturated or partially unsaturated carbocyclyl, saturated or partially unsaturated heterocyclyl, aryl or heteroaryl; wherein, the alkyl, heteroalkyl, saturated or partially unsaturated carbocyclyl, saturated or partially unsaturated heterocyclyl, aryl, or heteroaryl involved in $L^2$ each optionally are independently substituted by one or more substituents selected from the group consisting of deuterium, hydroxyl, amino, cyano, halogen, oxo, alkyl, alkenyl, alkynyl, heteroalkyl, and haloheteroalkyl;

$L^3$ is a bond, aryl or heteroaryl; wherein, aryl, or heteroaryl involved in $L^3$ each optionally are independently substituted by one or more substituents selected from the group consisting of deuterium, hydroxyl, amino, cyano, halogen, alkyl, alkenyl, alkynyl, heteroalkyl, haloalkyl and haloheteroalkyl;

$L^4$ is a carbon chain or heterochain with 2-6 atoms; wherein, the carbon chain or heterochain involved in $L^4$ each optionally are independently substituted by one or more substituents selected from the group consisting of deuterium, hydroxyl, amino, cyano, halogen, oxo, alkenyl, alkynyl, alkyl, haloalkyl, hydroxyalkyl, aminoalkyl, alkoxyl, haloalkoxyl, hydroxyalkoxyl, aminoalkoxyl, alkylamino, and a nitrogen protecting group;

$L^5$ is $-NR^{L5a}-$, -O-, -S-, alkyl or heteroalkyl; wherein, the alkyl and heteroalkyl involved in $L^5$ each optionally are independently substituted by a substituent selected from the group consisting of deuterium, amino, hydroxyl, oxo, cyano, halogen, alkenyl and alkynyl; each $R^{L5a}$ is independently hydrogen, alkyl, alkenyl, alkynyl, heteroalkyl, haloalkyl, or a nitrogen protecting group;

$L^6$ is $-NR^{L6a}-$, -O-, -S-, alkyl or heteroalkyl; wherein, the alkyl and heteroalkyl involved in $L^6$ each optionally are independently substituted by a substituent selected from the group consisting of deuterium, amino, hydroxyl, oxo, cyano, halogen, alkenyl and alkynyl; each $R^{L6a}$ is independently hydrogen, alkyl, alkenyl, alkynyl, heteroalkyl, haloalkyl, or a nitrogen protecting group;

$X^1$, $X^2$, $X^3$, $X^4$, $Y^1$, $Y^2$, $Y^3$, $Y^4$ and $Y^5$ each are independently CH or N;

each $R^1$ is independently hydrogen, deuterium, amino, hydroxyl, cyano, halogen, alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl; if there are two adjacent $R^1$, the two adjacent $R^1$ and their connected atoms optionally form saturated or partially unsaturated carbocyclyl, saturated or partially unsaturated heterocyclyl, aryl or heteroaryl; wherein, the alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, heteroarylalkyl involved in $R^1$ each optionally are independently substituted by one or more substituents selected from the group consisting of deuterium, hydroxyl, amino, cyano, halogen, oxo and alkyl;

each $R^2$ is independently hydrogen, deuterium, amino, hydroxyl, cyano, halogen, alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl; if there are two adjacent $R^2$, the two adjacent $R^2$ and their connected atoms optionally form saturated or partially unsaturated carbocyclyl, saturated or partially unsaturated heterocyclyl, aryl or heteroaryl; wherein, the alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^2$ each

optionally are substituted by one or more hydroxyl, amino, cyano, halogen, oxo and alkyl;

each $R^3$ is independently hydrogen, deuterium, amino, hydroxyl, cyano, halogen, alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl; if there are two adjacent $R^3$, the two adjacent $R^3$ and their connected atoms optionally form saturated or partially unsaturated carbocyclyl, saturated or partially unsaturated heterocyclyl, aryl or heteroaryl; wherein, the alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^3$ each optionally are substituted by one or more hydroxyl, amino, cyano, halogen, oxo and alkyl; and m and p each are independently 0, 1, 2, 3 or 4.

In some examples, M is

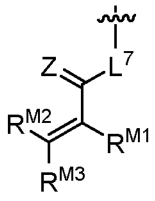
i-1
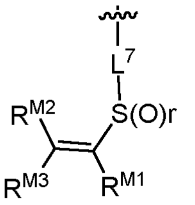
i-2
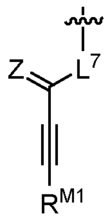
i-3
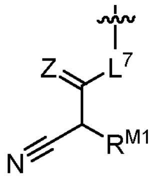
i-4
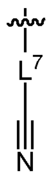
i-5
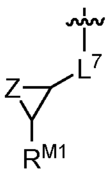
i-6
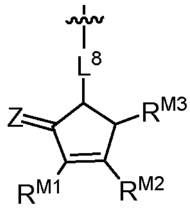
i-7
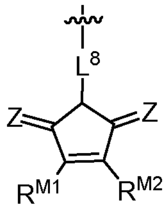
i-8
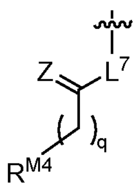
i-9
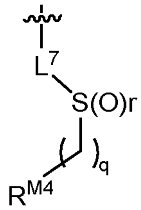
i-10
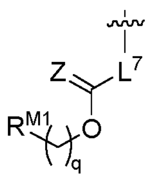
i-11
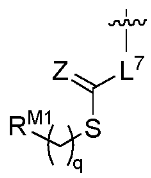
i-12
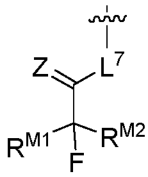
i-13
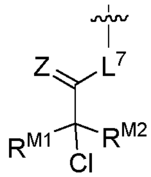
i-14
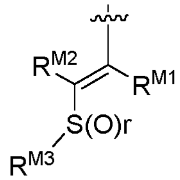
i-15
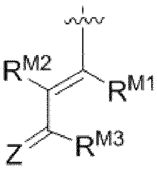
i-16
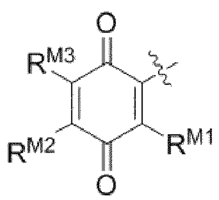
i-17
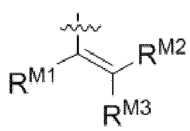
i-18
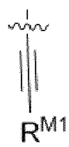
i-19 or
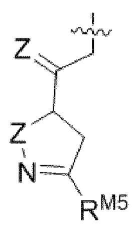
i-20 ;

wherein,

$L^7$ is a bond, -O-, -S-, -$NR^{L7a}$- or $C_{1-4}$alkyl, wherein, one or more carbon units of the $C_{1-4}$alkyl group optionally are

independently replaced with -O-, -S-, $-NR^{L7a}-$, $-NR^{L7a}C(=O)-$, $-C(=O)NR^{L7a}-$, -SC(=O)-, -C(=O)S-, -OC(=O)-, -C(=O)O-, $-NR^{L7a}C(=S)-$, $-C(=S)NR^{L7a}-$, *trans* $CR^{L7b}=CR^{L7b}-$, *cis* $CR^{L7b}=CR^{L7b}-$, -C=C-, -S(=O)-, -S(=O)O-, -OS(=O)-, $-S(=O)NR^{L7a}-$, $-NR^{L7a}S(=O)-$, $-S(=O)_2-$, $-S(=O)_2O-$, $-OS(=O)_2-$, $-S(=O)_2NR^{L7a}-$, or $-NR^{L7a}S(=O)_2-$; wherein each $R^{L7a}$ is independently hydrogen, deuterium, $C_{1-4}$alkyl, or a nitrogen protecting group; each $R^{L7b}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two adjacent $R^{L7b}$ groups bind with their connected atoms to form 3-6 membered saturated or partially unsaturated carbocyclyl or 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $L^7$, $R^{L7a}$ and $R^{L7b}$ each optionally are independently substituted by one or more substituents selected from the group consisting of deuterium, halogen, hydroxyl, amino, cyano, oxo, and $C_{1-4}$alkyl;

$L^8$ is a bond or $C_{1-4}$alkyl, wherein the alkyl involved in $L^8$ optionally is substituted by one or more substituents selected from the group consisting of deuterium, halogen, hydroxyl, amino, cyano, and oxo;

each $R^{M1}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, $-CH_2OR^{M1a}$, $-CH_2N(R^{M1a})_2$, $-CH_2SR^{M1a}$, $-OR^{M1a}$, $-N(R^{M1a})_2$, $-Si(R^{M1a})_3$ or $-SR^{M1a}$, wherein each $R^{M1a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl; or two $R^{M1a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M1}$ and $R^{M1a}$ each optionally are independently substituted by one or more substituents selected from the group consisting of deuterium, halogen, hydroxyl, amino, cyano, oxo, and $C_{1-4}$alkyl; each $R^{M2}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, $-CH_2OR^{M2a}$, $-CH_2N(R^{M2a})_2$, $-CH_2SR^{M2a}$, $-OR^{M2a}$, $-N(R^{M2a})_2$ or $-SR^{M2a}$, wherein each $R^{M2a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl; or two $R^{M2a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M2}$ and $R^{M2a}$ each optionally are independently substituted by one or more substituents selected from the group consisting of deuterium, halogen, hydroxyl, amino, cyano, oxo, and $C_{1-4}$alkyl; each $R^{M3}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, $-CH_2OR^{M3a}$, $-CH_2N(R^{M3a})_2$, $-CH_2SR^{M3a}$, $-OR^{M3a}$, $-N(R^{M3a})_2$ or $-SR^{M3a}$, wherein each $R^{M3a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl; or two $R^{M3a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M3}$ and $R^{M3a}$ each optionally are independently substituted by one or more substituents selected from the group consisting of deuterium, halogen, hydroxyl, amino, cyano, oxo, and $C_{1-4}$alkyl; Optionally, $R^{M1}$ and $R^{M3}$, or $R^{M2}$ and $R^{M3}$, or $R^{M1}$ and $R^{M2}$ are optionally connected to form saturated or partially unsaturated carbocyclyl or saturated or partially unsaturated heterocyclyl, wherein saturated or partially unsaturated carbocyclyl or saturated or partially unsaturated heterocyclyl each optionally are independently substituted by one or more substituents selected from the group consisting of deuterium, halogen, hydroxyl, amino, cyano, oxo, and $C_{1-4}$alkyl;

$R^{M4}$ is a leaving group; $R^{M5}$ is halogen; Z is O, S or $NR^Z$; wherein $R^Z$ is hydrogen, deuterium, $C_{1-4}$alkyl, or a nitrogen protecting group; alkyl involved in $R^Z$ optionally is substituted by one or more substituents selected from the group consisting of deuterium, halogen, hydroxyl, amino, cyano, and oxo; q is 0, 1, 2, 3, 4, 5 or 6; r is 1 or 2.

In some examples, M is

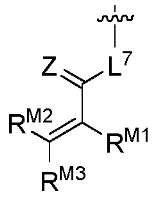 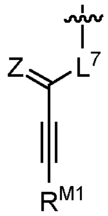 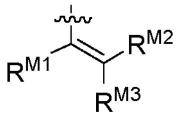 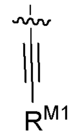

i-1 , i-3 , i-18 or i-19 ;

$L^7$ is a bond, -O-, -S-, -$NR^{L7a}$- or $C_{1-4}$alkyl, wherein, one or more carbon units of the $C_{1-4}$alkyl group optionally are independently replaced with -O-, -S-, -$NR^{L7a}$-, -$NR^{L7a}$C(=O)-, -C(=O)$NR^{L7a}$-, -SC(=O)-, -C(=O)S-, -OC(=O)-, -C(=O)O-, -$NR^{L7a}$C(=S)-, -C(=S)$NR^{L7a}$-, *trans* $CR^{L7b}$=$CR^{L7b}$-, *cis* $CR^{L7b}$=$CR^{L7b}$-, -C=C-, -S(=O)-, -S(=O)O-, -OS(=O)-, -S(=O)$NR^{L7a}$-, -$NR^{L7a}$S(=O)-, -$S(=O)_2$-, -$S(=O)_2$O-, -$OS(=O)_2$-, -$S(=O)_2NR^{L7a}$-, or -$NR^{L7a}S(=O)_2$-; wherein each $R^{L7a}$ is independently hydrogen, deuterium, $C_{1-4}$alkyl, or a nitrogen protecting group; $R^{L7b}$ is hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two adjacent $R^{L7b}$ groups optionally bind with their connected atoms to form 3-6 membered saturated or partially unsaturated carbocyclyl or 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $L^7$, $R^{L7a}$ and $R^{L7b}$ each optionally are independently substituted by one or more substituents selected from the group consisting of deuterium, halogen, hydroxyl, amino, cyano, oxo, and $C_{1-4}$alkyl;

Z is O, S or $NR^Z$; wherein $R^Z$ is hydrogen, deuterium, $C_{1-4}$alkyl, or a nitrogen protecting group; alkyl involved in $R^Z$ optionally is substituted by one or more substituents selected from the group consisting of hydrogen, deuterium, halogen, hydroxyl, amino, cyano, and oxo;

each $R^{M1}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, -$CH_2OR^{M1a}$, -$CH_2N(R^{M1a})_2$, -$CH_2SR^{M1a}$, -$OR^{M1a}$, -$N(R^{M1a})_2$, -$Si(R^{M1a})_3$ or -$SR^{M1a}$, wherein each $R^{M1a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two $R^{M1a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M1}$ and $R^{M1a}$ each optionally are independently substituted by one or more substituents selected from the group consisting of deuterium, halogen, hydroxyl, amino, cyano, oxo, and $C_{1-4}$alkyl; each $R^{M2}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, -$CH_2OR^{M2a}$, -$CH_2N(R^{M2a})_2$, -$CH_2SR^{M2a}$, -$OR^{M2a}$, -$N(R^{M2a})_2$ or -$SR^{M2a}$, wherein each $R^{M2a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two $R^{M2a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M2}$ and $R^{M2a}$ each optionally are independently substituted by one or more substituents selected from the group consisting of deuterium, halogen, hydroxyl, amino, cyano, oxo, and $C_{1-4}$alkyl; each $R^{M3}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, -$CH_2OR^{M3a}$, -$CH_2N(R^{M3a})_2$, -$CH_2SR^{M3a}$, -$OR^{M3a}$, -$N(R^{M3a})_2$ or -$SR^{M3a}$, wherein each $R^{M3a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two $R^{M3a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M3}$ and $R^{M3a}$ each optionally are independently substituted by one or more substituents selected from the group consisting of deuterium, halogen, hydroxyl, amino, cyano, oxo, and $C_{1-4}$alkyl.

In some examples, M is

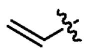, 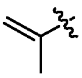, 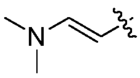, 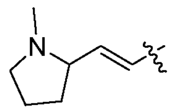, 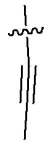, 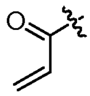, 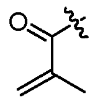, 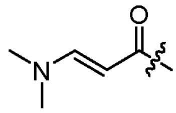,

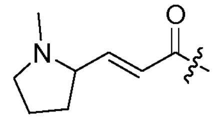 or 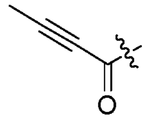.

In some examples, A is 6-10 membered aryl, or 5-10 membered heteroaryl. In some examples, A is 6-10 membered aryl or 5-10 membered azaaryl. In some examples, A is phenyl or 6-membered azaaryl. In some examples, A is phenyl, imidazolyl, pyrazolyl, triazolyl, tetraazolyl, oxazolyl, thiazolyl, furyl, thienyl, pyrrolyl, pyridinyl, pyrimidinyl, pyrazinyl, pyridazinyl, or the following groups:

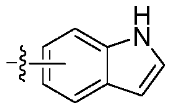, 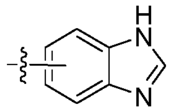, 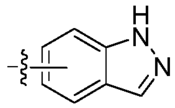, 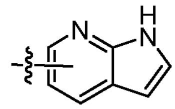, 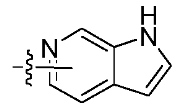, 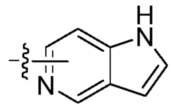,

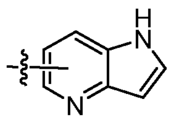, 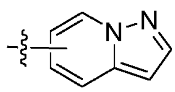, 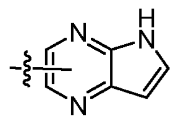, 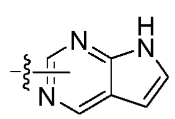, 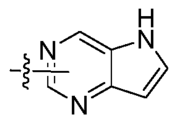, 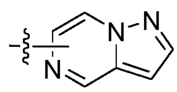,

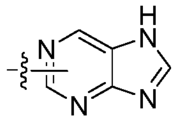, 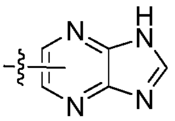, 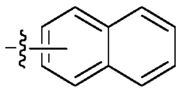, 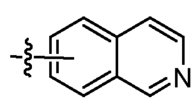, 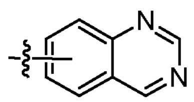 or 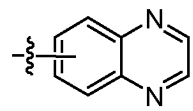.

In some examples, A is phenyl, pyridinyl, pyrimidinyl, pyrazinyl or pyridazinyl. In some examples, A is phenyl or pyrazinyl.

In some examples, L' is a bond, -C(=O)-, -S(=O)-, $-S(=O)_2-$, $-C(=O)NR^{L1a}-$, $-NR^{L1a}C(=O)-$, $-S(=O)_2NR^{L1a}-$, or $-NR^{L1a}S(=O)_2-$; wherein, each $R^{L1a}$ is independently hydrogen, deuterium, $C_{1-4}$alkyl, $C_{2-4}$alkenyl, $C_{2-4}$alkynyl, $C_{1-4}$heteroalkyl, $C_{1-4}$haloalkyl, $C_{1-4}$haloheteroalkyl, or a nitrogen protecting group; wherein the alkyl, alkenyl, alkynyl, heteroalkyl, haloalkyl, haloheteroalkyl involved in $R^{L1a}$ each optionally are independently substituted by one or more substituents selected from the group consisting of deuterium, halogen, hydroxyl, amino, cyano, and oxo; $L^2$ is a bond, $C_{1-6}$alkyl, $C_{1-6}$heteroalkyl, 3-6 membered saturated or partially unsaturated carbocyclyl, 3-6 membered saturated or partially unsaturated heterocyclyl, 6-10 membered aryl, or 5-10 membered heteroaryl; wherein, the alkyl, heteroalkyl, saturated or partially unsaturated carbocyclyl, saturated or partially unsaturated heterocyclyl, aryl, or heteroaryl involved in $L^2$ each optionally are independently substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; $L^3$ is a bond, 6-10 membered aryl, or 5-10 membered heteroaryl; wherein, the aryl, or heteroaryl involved in $L^3$ each optionally are independently substituted by one or more substituents selected from the group consisting of deuterium, hydroxyl, amino, cyano, halogen, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; $L^4$ is a carbon chain or heterochain with 2-6 atoms; wherein, the carbon chain or heterochain involved in $L^4$ each optionally are independently substituted by one or more substituents selected from the group consisting of deuterium, hydroxyl, amino, cyano, halogen, oxo, $C_{2-4}$alkenyl, $C_{2-4}$alkynyl, $C_{1-4}$alkyl, $C_{1-4}$haloalkyl, $C_{1-4}$hydroxyalkyl, $C_{1-4}$aminoalkyl, $C_{1-4}$alkoxyl, $C_{1-4}$haloalkoxyl, $C_{1-4}$hydroxyalkoxyl, $C_{1-4}$aminoalkoxyl, $C_{1-4}$alkylamino, and a nitrogen protecting group.

In some examples, $L^1$ is a bond, -C(=O)-, -S(=O)-, $-S(=O)_2-$, $-C(=O)NR^{L1a}-$, $-NR^{L1a}C(=O)-$, $-S(=O)_2NR^{L1a}-$, or $-NR^{L1a}S(=O)_2-$; wherein, each $R^{L1a}$ is independently hydrogen, methyl, ethyl, ethenyl, ethynyl or trifluoromethyl; $L^2$ is a bond, $C_{1-6}$alkyl, $C_{1-6}$heteroalkyl, 3-6 membered saturated or partially unsaturated carbocyclyl, 3-6 membered saturated or partially unsaturated heterocyclyl, 6-10 membered aryl, or 5-10 membered heteroaryl; wherein, the alkyl, heteroalkyl,

saturated or partially unsaturated carbocyclyl, saturated or partially unsaturated heterocyclyl, aryl, or heteroaryl involved in $L^2$ each optionally are independently substituted by a substituent selected from the group consisting of deuterium, hydroxyl, amino, cyano, fluoro, chloro, oxo, ethenyl, ethynyl, methoxy, ethoxy, trifluoromethoxy and methylamino; $L^3$ is a bond, 6-10 membered aryl, or 5-10 membered heteroaryl; wherein, the aryl, or heteroaryl involved in $L^3$ each optionally are independently substituted by a substituent selected from the group consisting of deuterium, hydroxyl, amino, cyano, fluoro, chloro, methyl, ethyl, ethenyl, ethynyl, methoxy, ethoxy, trifluoromethoxy and methylamino; $L^4$ is a carbon chain or heterochain with 2-4 atoms; wherein, the carbon chain or heterochain involved in $L^4$ each optionally are independently substituted by one or more substituents selected from the group consisting of deuterium, hydroxyl, amino, cyano, halogen, oxo, ethenyl, ethynyl, methyl, ethyl, isopropyl, methoxy, ethoxy, trifluoromethoxy, methylamino, and a nitrogen protecting group.

In some examples, $L^1$ is a bond, -C(=O)-, -S(=O)-, -S(=O)$_2$-, -C(=O)NH-, -NHC(=O)-, -S(=O)$_2$NH- or -NHS(=O)$_2$-; $L^2$ is a bond, 3-6 membered saturated or partially unsaturated carbocyclyl or 3-6 membered saturated or partially unsaturated heterocyclyl; wherein, saturated or partially unsaturated carbocyclyl, saturated or partially unsaturated heterocyclyl involved in $L^2$ each optionally are independently substituted by one or more substituents selected from the group consisting of hydroxyl, amino, cyano, fluoro, chloro, oxo, ethenyl, ethynyl, methoxy, ethoxy, trifluoromethoxy and methylamino; $L^3$ is a bond, 6-10 membered aryl, or 5-10 membered heteroaryl; wherein, the aryl, or heteroaryl involved in $L^3$ optionally is independently substituted by one or more substituents selected from the group consisting of hydroxyl, amino, cyano, fluoro, chloro, methyl, ethyl, ethenyl, ethynyl, methoxy, ethoxy, trifluoromethoxy and methylamino; $L^4$ is a carbon chain or heterochain with 2-4 atoms; wherein, the carbon chain or heterochain involved in $L^4$ each optionally are independently substituted by one or more substituents selected from the group consisting of deuterium, hydroxyl, amino, cyano, halogen, oxo, ethenyl, ethynyl, methyl, ethyl, isopropyl, methoxy, ethoxy, trifluoromethoxy, methylamino, and a nitrogen protecting group.

In some examples, $L^1$ is a bond, -C(=O)- or -C(=O)NH-; $L^2$ is a bond, cyclohexyl, piperidyl, phenyl or pyridinyl; $L^3$ is a bond, phenyl or pyridinyl; $L^4$ is $-NHCH_2-$, $-CH_2NH-$, $-OCH_2-$, $-CH_2O-$, $-SCH_2-$, $-CH_2S-$, $-NHCH_2CH_2-$, $-CH_2NHCH_2-$, $-CH_2CH_2NH-$, $-OCH_2CH_2-$, $-CH_2OCH_2-$, $-CH_2CH_2O-$, $-SCH_2CH_2-$, $-CH_2SCH_2-$, $-CH_2CH_2S-$, $-NHCH_2CH_2CH_2-$, $-CH_2NHCH_2CH_2-$, $-CH_2CH_2NHCH_2-$, $-CH_2CH_2CH_2NH-$, $-OCH_2CH_2CH_2-$, $-CH_2OCH_2CH_2-$, $-CH_2CH_2OCH_2-$, $-CH_2CH_2CH_2O-$, $-SCH_2CH_2CH_2-$, $-CH_2SCH_2CH_2-$, $-CH_2CH_2SCH_2-$, $-CH_2CH_2CH_2S-$, $-CH_2OCH_2O-$, $-OCH_2OCH_2-$, $-OCH_2CH_2O-$, $-NHCH_2CH_2O-$, $-CH_2NHCH_2O-$ or $-NHCH_2OCH_2-$; wherein the hydrogen of $CH_2$ involved in $L^4$ each optionally are independently substituted by a substituent selected from the group consisting of hydrogen, deuterium, hydroxyl, amino, cyano, halogen, oxo, ethenyl, ethynyl, methyl, ethyl, isopropyl, methoxy, ethoxy, trifluoromethoxy and methylamino; the hydrogen of NH involved in $L^4$ each optionally are independently substituted by a substituent selected from the group consisting of hydrogen, deuterium, hydroxyl, cyano, halogen, oxo, ethenyl, ethynyl, methyl, ethyl, isopropyl, methoxy, ethoxy, trifluoromethoxy, and a nitrogen protecting group.

In some examples, $L^1$ is a bond, -C(=O)- or -C(=O)NH-; $L^2$ is a bond, cyclohexyl, piperidyl, phenyl or pyridinyl; $L^3$ is a bond, phenyl or pyridinyl; $L^4$ is $-NHCH_2-$, $-CH_2NHCH_2-$, -NHC(=O)-, $-CH_2NHC(=O)-$, $-C(=O)NHCH_2-$, or $-NHCH(CH_3)-$.

In some examples, $L^5$ is $-NR^{L5a}-$, -O-, -S-, $C_{1-6}$alkyl or $C_{1-6}$heteroalkyl; wherein, the alkyl and heteroalkyl involved in $L^5$ each optionally are independently substituted by one or more substituents selected from the group consisting of deuterium, amino, hydroxyl, oxo, cyano, halogen, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; each $R^{L5a}$ is independently hydrogen, deuterium, $C_{1-6}$alkyl, $C_{2-6}$alkenyl, $C_{2-6}$alkynyl, $C_{1-6}$heteroalkyl, $C_{1-6}$haloalkyl, or a nitrogen protecting group; $L^6$ is $-NR^{L6a}-$, -O-, -S-, $C_{1-6}$alkyl or $C_{1-6}$heteroalkyl; wherein, the alkyl and heteroalkyl involved in $L^6$ each optionally are independently substituted by one or more substituents selected from the group consisting of deuterium, amino, hydroxyl, oxo, cyano, halogen, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; each $R^{L6a}$ is independently hydrogen, deuterium, $C_{1-6}$alkyl, $C_{2-6}$alkenyl, $C_{2-6}$alkynyl, $C_{1-6}$heteroalkyl, $C_{1-6}$haloalkyl, or a nitrogen protecting group.

In some examples, $L^5$ is $-NR^{L5a}$, $-CR^{L5b}R^{L5c}-$, -O-, -S-, $-CR^{L5b}R^{L5c}NR^{L5a}-$, $-NR^{L5a}CR^{L5b}R^{L5c}-$, $-CR^{L5b}R^{L5c}O-$, $-OCR^{L5b}R^{L5c}-$, $-CR^{L5b}R^{L5c}S-$ or $-SCR^{L5b}R^{L5c}-$; wherein, each $R^{L5b}$ and $R^{L5c}$ are independently hydrogen, deuterium, amino, hydroxyl, oxo, cyano, halogen, $C_{1-4}$alkyl, $C_{2-4}$alkenyl, $C_{2-4}$alkynyl, $C_{1-4}$heteroalkyl, $C_{1-4}$haloalkyl, or $C_{1-4}$haloheteroalkyl; each $R^{L5a}$ is independently hydrogen, deuterium, $C_{1-4}$alkyl, $C_{2-4}$alkenyl, $C_{2-4}$alkynyl, $C_{1-4}$heteroalkyl, $C_{1-4}$haloalkyl, or a nitrogen protecting group; $L^6$ is $-NR^{L6a}$, $-CR^{L6b}R^{L6c}-$, -O-, -S-, $-CR^{L6b}R^{L6c}NR^{L6a}-$, $-NR^{L6a}CR^{L6b}R^{L6c}-$, $-CR^{L6b}R^{L6c}O-$, $-OCR^{L6b}R^{L6c}-$, $-CR^{L6b}R^{L6c}S-$ or $-SCR^{L6b}R^{L6c}-$; wherein, each $R^{L6b}$ and $R^{L6c}$ are independently hydrogen, deuterium, amino, hydroxyl, oxo, cyano, halogen, $C_{1-4}$alkyl, $C_{2-4}$alkenyl, $C_{2-4}$alkynyl, $C_{1-4}$heteroalkyl, $C_{1-4}$haloalkyl, or $C_{1-4}$haloheteroalkyl; each $R^{L6a}$ is independently hydrogen, deuterium, $C_{1-4}$alkyl, $C_{2-4}$alkenyl, $C_{2-4}$alkynyl, $C_{1-4}$heteroalkyl, $C_{1-4}$haloalkyl, or a nitrogen protecting group.

In some examples, $L^5$ is -NH-, -O-, -S-, $-NHCH_2-$, $-OCH_2-$, $-SCH_2-$, $-CH_2NH-$, $-CH_2O-$ or $-CH_2S-$; $L^6$ is -NH-, -O-, -S-, $-NHCH_2-$, $-OCH_2-$, $-SCH_2-$, $-CH_2NH-$, $-CH_2O-$ or $-CH_2S-$.

In some examples, $L^5$ is -NH-; $L^6$ is -NH- or $-NHCH_2-$.

In some examples, each $R^1$ is independently hydrogen, deuterium, amino, hydroxyl, cyano, halogen, $C_{1-6}$alkyl,

$C_{2-6}$alkenyl, $C_{2-6}$alkynyl, $C_{1-6}$heteroalkyl, $C_{1-6}$haloalkyl, $C_{1-6}$haloheteroalkyl, 3-6 membered saturated or partially unsaturated carbocyclyl, 3-6 membered saturated or partially unsaturated carbocyclyl$C_{1-6}$alkyl, 3-6 membered saturated or partially unsaturated heterocyclyl, 3-6 membered saturated or partially unsaturated heterocyclyl$C_{1-6}$alkyl, 6-10 membered aryl, 6-10 membered aryl$C_{1-6}$alkyl, 6-10 membered heteroaryl or 6-10 membered heteroaryl$C_{1-6}$alkyl; if there are two adjacent $R^1$, the two adjacent $R^1$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^1$ each optionally are independently substituted by one or more substituents selected from the group consisting of deuterium, hydroxyl, amino, cyano, halogen, oxo, and $C_{1-4}$alkyl;

each $R^2$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, $C_{1-6}$haloheteroalkyl, 3-6 membered saturated or partially unsaturated carbocyclyl, 3-6 membered saturated or partially unsaturated carbocyclyl$C_{1-6}$alkyl, 3-6 membered saturated or partially unsaturated heterocyclyl, 3-6 membered saturated or partially unsaturated heterocyclyl$C_{1-6}$alkyl, 6-10 membered aryl, 6-10 membered aryl$C_{1-6}$alkyl, 6-10 membered heteroaryl or 6-10 membered heteroaryl$C_{1-6}$alkyl; if there are two adjacent $R^2$, the two adjacent $R^2$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^2$ each optionally are independently substituted by one or more substituents selected from the group consisting of deuterium, hydroxyl, amino, cyano, halogen, oxo, and $C_{1-4}$alkyl; each $R^3$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, $C_{1-6}$haloheteroalkyl, 3-6 membered saturated or partially unsaturated carbocyclyl, 3-6 membered saturated or partially unsaturated carbocyclyl$C_{1-6}$alkyl, 3-6 membered saturated or partially unsaturated heterocyclyl, 3-6 membered saturated or partially unsaturated heterocyclyl$C_{1-6}$alkyl, 6-10 membered aryl, 6-10 membered aryl$C_{1-6}$alkyl, 6-10 membered heteroaryl or 6-10 membered heteroaryl$C_{1-6}$alkyl; if there are two adjacent $R^3$, the two adjacent $R^3$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^3$ each optionally are independently substituted by one or more substituents selected from the group consisting of deuterium, hydroxyl, amino, cyano, halogen, oxo, and $C_{1-4}$alkyl.

In some examples, each $R^1$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, or $C_{1-6}$haloheteroalkyl; if there are two adjacent $R^1$, the two adjacent $R^1$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, saturated or partially unsaturated carbocyclyl, saturated or partially unsaturated heterocyclyl, aryl, or heteroaryl involved in $R^1$ each optionally are independently substituted by one or more substituents selected from the group consisting of deuterium, hydroxyl, amino, cyano, halogen, oxo, and $C_{1-4}$alkyl; each $R^2$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, or $C_{1-6}$haloheteroalkyl; if there are two adjacent $R^2$, the two adjacent $R^2$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, saturated or partially unsaturated carbocyclyl, saturated or partially unsaturated heterocyclyl, aryl, or heteroaryl involved in $R^2$ each optionally are independently substituted by one or more substituents selected from the group consisting of deuterium, hydroxyl, amino, cyano, halogen, oxo, and $C_{1-4}$alkyl; each $R^3$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, $C_{1-6}$haloheteroalkyl; if there are two adjacent $R^3$, the two adjacent $R^3$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, saturated or partially unsaturated carbocyclyl, saturated or partially unsaturated heterocyclyl, aryl, or heteroaryl involved in $R^3$ each optionally are independently substituted by one or more substituents selected from the group consisting of deuterium, hydroxyl, amino, cyano, halogen, oxo, and $C_{1-4}$alkyl.

In some examples, each $R^1$ is independently hydrogen, deuterium, amino, hydroxyl, cyano, halogen, $C_{1-6}$alkyl, $C_{1-6}$heteroalkyl, $C_{1-6}$haloalkyl, or $C_{1-6}$haloheteroalkyl; wherein the alkyl, heteroalkyl, haloalkyl, haloheteroalkyl involved in $R^1$ each optionally are independently substituted by one or more substituents selected from the group consisting of

deuterium, hydroxyl, amino, cyano, halogen, and oxo; each $R^2$ is independently hydrogen, deuterium, amino, hydroxyl, cyano, halogen, $C_{1-6}$alkyl, $C_{1-6}$heteroalkyl, $C_{1-6}$haloalkyl, or $C_{1-6}$haloheteroalkyl; wherein the alkyl, heteroalkyl, haloalkyl, haloheteroalkyl involved in $R^2$ each optionally are independently substituted by one or more substituents selected from the group consisting of deuterium, hydroxyl, amino, cyano, halogen, and oxo; each $R^3$ is independently hydrogen, deuterium, amino, hydroxyl, cyano, halogen, $C_{1-6}$alkyl, $C_{1-6}$heteroalkyl, $C_{1-6}$haloalkyl, or $C_{1-6}$haloheteroalkyl; if there are two adjacent $R^3$, the two adjacent $R^3$ and their connected atoms optionally form 3-6 membered saturated or partially unsaturated carbocyclyl, 3-6 membered saturated or partially unsaturated heterocyclyl; wherein the alkyl, heteroalkyl, haloalkyl, haloheteroalkyl, 3-6 membered saturated or partially unsaturated carbocyclyl, 3-6 membered saturated or partially unsaturated heterocyclyl involved in $R^3$ each optionally are independently substituted by one or more substituents selected from the group consisting of deuterium, hydroxyl, amino, cyano, halogen, oxo, and $C_{1-4}$alkyl.

In some examples, each $R^1$ is independently hydrogen, deuterium, amino, hydroxyl, cyano, halogen, $C_{1-4}$alkyl, $C_{1-4}$heteroalkyl, $C_{1-4}$haloalkyl, or $C_{1-4}$haloheteroalkyl; wherein the alkyl, heteroalkyl, haloalkyl, haloheteroalkyl involved in $R^1$ each optionally are independently substituted by one or more substituents selected from the group consisting of deuterium, hydroxyl, amino, cyano, halogen, and oxo; each $R^2$ is independently hydrogen, deuterium, amino, hydroxyl, cyano, halogen, $C_{1-4}$alkyl, $C_{1-4}$heteroalkyl, $C_{1-4}$haloalkyl, or $C_{1-4}$haloheteroalkyl; wherein the alkyl, heteroalkyl, haloalkyl, haloheteroalkyl involved in $R^2$ each optionally are independently substituted by one or more substituents selected from the group consisting of deuterium, hydroxyl, amino, cyano, halogen, and oxo; each $R^3$ is independently hydrogen, deuterium, amino, hydroxyl, cyano, halogen, $C_{1-4}$alkyl, $C_{1-4}$heteroalkyl, $C_{1-4}$haloalkyl, or $C_{1-4}$haloheteroalkyl; if there are two adjacent $R^3$, the two adjacent $R^3$ and their connected atoms optionally form 3-6 membered saturated or partially unsaturated carbocyclyl, 3-6 membered saturated or partially unsaturated heterocyclyl; wherein the alkyl, heteroalkyl, haloalkyl, haloheteroalkyl, 3-6 membered saturated or partially unsaturated carbocyclyl, 3-6 membered saturated or partially unsaturated heterocyclyl involved in $R^3$ each optionally are independently substituted by one or more substituents selected from the group consisting of deuterium, hydroxyl, amino, cyano, halogen, oxo, methyl, ethyl, and isopropyl.

In some examples, each $R^1$ is independently hydrogen, deuterium, amino, hydroxyl, cyano, fluoro, chloro, methyl, ethyl, propyl, isopropyl, *t*-butyl, methoxy, ethoxy, i-propoxy, *t*-butoxy, trifluoromethyl, trifluoromethoxy, $CH_3C(=O)$-, $CH_3CH_2C(=O)$-, $(CH_3)_2CHC(=O)$-, $CH_3C(=O)O$-, $CH_3CH_2C(=O)O$-, $(CH_3)_2CHC(=O)O$-, $CH_3C(=O)NH$-, $CH_3CH_2C(=O)NH$-, $(CH_3)_2CHC(=O)NH$-, $CH_3NHC(=O)$-, $CH_3CH_2NHC(=O)$-, $(CH_3)_2CHNHC(=O)$-, $CH_3ONHC(=O)$-, $CH_3CH_2ONHC(=O)$-, $(CH_3)_2CHONHC(=O)$-, $CH_3S(=O)_2$-, $CH_3CH_2S(=O)_2$-, $(CH_3)_2CHS(=O)_2$-, $CH_3S(=O)_2O$-, $CH_3CH_2S(=O)_2O$-, $(CH_3)_2CHS(=O)_2O$-, $CH_3S(=O)_2NH$-, $CH_3CH_2S(=O)_2NH$-, $(CH_3)_2CHS(=O)_2NH$-, $CH_3NHS(=O)_2$-, $CH_3CH_2NHS(=O)_2$-, $(CH_3)_2CHNHS(=O)_2$-, $CH_3C(=O)N(CH_3)$-, $CH_3CH_2C(=O)N(CH_3)$-, $(CH_3)_2CHC(=O)N(CH_3)$-, $CH_3N(CH_3)C(=O)$-, $CH_3CH_2N(CH_3)C(=O)$-, $(CH_3)_2CHN(CH_3)C(=O)$-, $CH_3ON(CH_3)C(=O)$-, $CH_3CH_2ON(CH_3)C(=O)$-, $(CH_3)_2CHON(CH_3)C(=O)$-, $CH_3S(=O)_2N(CH_3)$-, $CH_3CH_2S(=O)_2N(CH_3)$-, $(CH_3)_2CHS(=O)_2N(CH_3)$-, $CH_3N(CH_3)S(=O)_2$-, $CH_3CH_2N(CH_3)S(=O)_2$-, $(CH_3)_2CHN(CH_3)S(=O)_2$-, $CH_3C(=O)N(CH_2CH_3)$-, $CH_3CH_2C(=O)N(CH_2CH_3)$-, $(CH_3)_2CHC(=O)N(CH_2CH_3)$-, $CH_3N(CH_2CH_3)C(=O)$-, $CH_3CH_2N(CH_2CH_3)C(=O)$-, $(CH_3)_2CHN(CH_2CH_3)C(=O)$-, $CH_3ON(CH_2CH_3)C(=O)$-, $CH_3CH_2ON(CH_2CH_3)C(=O)$-, $(CH_3)_2CHON(CH_2CH_3)C(=O)$-, $CH_3S(=O)_2N(CH_2CH_3)$-, $CH_3CH_2S(=O)_2N(CH_2CH_3)$-, $(CH_3)_2CHS(=O)_2N(CH_2CH_3)$-, $CH_3N(CH_2CH_3)S(=O)_2$-, $CH_3CH_2N(CH_2CH_3)S(=O)_2$-, $(CH_3)_2CHN(CH_2CH_3)S(=O)_2$-, $(CH_3)_2P(=O)$-, $CH_3CH_2(CH_3)P(=O)$-, $(CH_3)_2CH(CH_3)P(=O)$-, $(CH_3CH_2)_2P(=O)$-, $CH_3CH_2(CH_3)P(=O)$-, or $(CH_3)_2CH(CH_3CH_2)P(=O)$-;

each $R^2$ is independently hydrogen, deuterium, amino, hydroxyl, cyano, fluoro, chloro, bromo, methyl, ethyl, propyl, isopropyl, *t*-butyl, methoxy, ethoxy, i-propoxy, *t*-butoxy, trifluoromethyl, trifluoromethoxy, $CH_3C(=O)$-, $CH_3CH_2C(=O)$-, $(CH_3)_2CHC(=O)$-, $CH_3C(=O)O$-, $CH_3CH_2C(=O)O$-, $(CH_3)_2CHC(=O)O$-, $CH_3C(=O)NH$-, $CH_3CH_2C(=O)NH$-, $(CH_3)_2CHC(=O)NH$-, $CH_3NHC(=O)$-, $CH_3CH_2NHC(=O)$-, $(CH_3)_2CHNHC(=O)$-, $CH_3ONHC(=O)$-, $CH_3CH_2ONHC(=O)$-, $(CH_3)_2CHONHC(=O)$-, $CH_3S(=O)_2$-, $CH_3CH_2S(=O)_2$-, $(CH_3)_2CHS(=O)_2$-, $CH_3S(=O)_2O$-, $CH_3CH_2S(=O)_2O$-, $(CH_3)_2CHS(=O)_2O$-, $CH_3S(=O)_2NH$-, $CH_3CH_2S(=O)_2NH$-, $(CH_3)_2CHS(=O)_2NH$-, $CH_3NHS(=O)_2$-, $CH_3CH_2NHS(=O)_2$-, $(CH_3)_2CHNHS(=O)_2$-, $CH_3C(=O)N(CH_3)$-, $CH_3CH_2C(=O)N(CH_3)$-, $(CH_3)_2CHC(=O)N(CH_3)$-, $CH_3N(CH_3)C(=O)$-, $CH_3CH_2N(CH_3)C(=O)$-, $(CH_3)_2CHN(CH_3)C(=O)$-, $CH_3ON(CH_3)C(=O)$-, $CH_3CH_2ON(CH_3)C(=O)$-, $(CH_3)_2CHON(CH_3)C(=O)$-, $CH_3S(=O)_2N(CH_3)$-, $CH_3CH_2S(=O)_2N(CH_3)$-, $(CH_3)_2CHS(=O)_2N(CH_3)$-, $CH_3N(CH_3)S(=O)_2$-, $CH_3CH_2N(CH_3)S(=O)_2$-, $(CH_3)_2CHN(CH_3)S(=O)_2$-, $CH_3C(=O)N(CH_2CH_3)$-, $CH_3CH_2C(=O)N(CH_2CH_3)$-, $(CH_3)_2CHC(=O)N(CH_2CH_3)$-, $CH_3N(CH_2CH_3)C(=O)$-, $CH_3CH_2N(CH_2CH_3)C(=O)$-, $(CH_3)_2CHN(CH_2CH_3)C(=O)$-, $CH_3ON(CH_2CH_3)C(=O)$-, $CH_3CH_2ON(CH_2CH_3)C(=O)$-, $(CH_3)_2CHON(CH_2CH_3)C(=O)$-, $CH_3S(=O)_2N(CH_2CH_3)$-, $CH_3CH_2S(=O)_2N(CH_2CH_3)$-, $(CH_3)_2CHS(=O)_2N(CH_2CH_3)$-, $CH_3N(CH_2CH_3)S(=O)_2$-, $CH_3CH_2N(CH_2CH_3)S(=O)_2$-, $(CH_3)_2CHN(CH_2CH_3)S(=O)_2$-, $(CH_3)_2P(=O)$-, $CH_3CH_2(CH_3)P(=O)$-, $(CH_3)_2CH(CH_3)P(=O)$-, $(CH_3CH_2)_2P(=O)$-, $CH_3CH_2(CH_3)P(=O)$-, or $(CH_3)_2CH(CH_3CH_2)P(=O)$-; and

each $R^3$ is independently hydrogen, deuterium, amino, hydroxyl, cyano, fluoro, chloro, methyl, ethyl, propyl, isopropyl, *t*-butyl, methoxy, ethoxy, i-propoxy, *t*-butoxy, trifluoromethyl, trifluoromethoxy, $CH_3C(=O)$-, $CH_3CH_2C(=O)$-, $(CH_3)_2CHC(=O)$-, $CH_3C(=O)O$-, $CH_3CH_2C(=O)O$-, $(CH_3)_2CHC(=O)O$-, $CH_3C(=O)NH$-, $CH_3CH_2C(=O)NH$-,

$(CH_3)_2CHC(=O)NH-$, $CH_3NHC(=O)-$, $CH_3CH_2NHC(=O)-$, $(CH_3)_2CHNHC(=O)-$, $CH_3ONHC(=O)-$, $CH_3CH_2ONHC(=O)-$, $(CH_3)_2CHONHC(=O)-$, $CH_3S(=O)_2-$, $CH_3CH_2S(=O)_2-$, $(CH_3)_2CHS(=O)_2-$, $CH_3S(=O)_2O-$, $CH_3CH_2S(=O)_2O-$, $(CH_3)_2CHS(=O)_2O-$, $CH_3S(=O)_2NH-$, $CH_3CH_2S(=O)_2NH-$, $(CH_3)_2CHS(=O)_2NH-$, $CH_3NHS(=O)_2-$, $CH_3CH_2NHS(=O)_2-$, $(CH_3)_2CHNHS(=O)_2-$, $CH_3C(=O)N(CH_3)-$, $CH_3CH_2C(=O)N(CH_3)-$, $(CH_3)_2CHC(=O)N(CH_3)-$, $CH_3N(CH_3)C(=O)-$, $CH_3CH_2N(CH_3)C(=O)-$, $(CH_3)_2CHN(CH_3)C(=O)-$, $CH_3ON(CH_3)C(=O)-$, $CH_3CH_2ON(CH_3)C(=O)-$, $(CH_3)_2CHON(CH_3)C(=O)-$, $CH_3S(=O)_2N(CH_3)-$, $CH_3CH_2S(=O)_2N(CH_3)-$, $(CH_3)_2CHS(=O)_2N(CH_3)-$, $CH_3N(CH_3)S(=O)_2-$, $CH_3CH_2N(CH_3)S(=O)_2-$, $(CH_3)_2CHN(CH_3)S(=O)_2-$, $CH_3C(=O)N(CH_2CH_3)-$, $CH_3CH_2C(=O)N(CH_2CH_3)-$, $(CH_3)_2CHC(=O)N(CH_2CH_3)-$, $CH_3N(CH_2CH_3)C(=O)-$, $CH_3CH_2N(CH_2CH_3)C(=O)-$, $(CH_3)_2CHN(CH_2CH_3)C(=O)-$, $CH_3ON(CH_2CH_3)C(=O)-$, $CH_3CH_2ON(CH_2CH_3)C(=O)-$, $(CH_3)_2CHON(CH_2CH_3)C(=O)-$, $CH_3S(=O)_2N(CH_2CH_3)-$, $CH_3CH_2S(=O)_2N(CH_2CH_3)-$, $(CH_3)_2CHS(=O)_2N(CH_2CH_3)-$, $CH_3N(CH_2CH_3)S(=O)_2-$, $CH_3CH_2N(CH_2CH_3)S(=O)_2-$, $(CH_3)_2CHN(CH_2CH_3)S(=O)_2-$, $(CH_3)_2P(=O)-$, $CH_3CH_2(CH_3)P(=O)-$, $(CH_3)_2CH(CH_3)P(=O)-$, $(CH_3CH_2)_2P(=O)-$, $CH_3CH_2(CH_3)P(=O)-$, or $(CH_3)_2CH(CH_3CH_2)P(=O)-$; if there are two adjacent $R^3$, the two adjacent $R^3$ and their connected atoms optionally form the following groups:

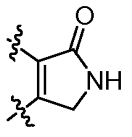 , 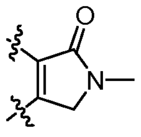 , 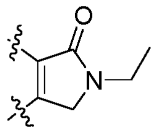 , 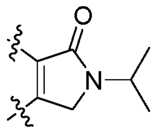 , 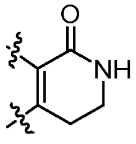 , 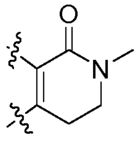 , 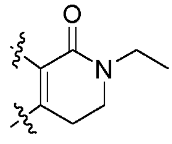 ,

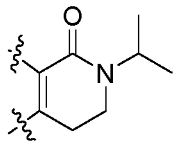 , 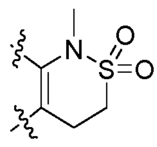 , or 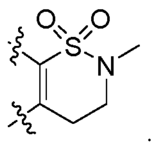 .

In some examples, the saturated or partially unsaturated carbocyclyl is selected from formulae ii-1 to ii-6; when formulae ii-1 to ii-6 are connected to the rest of the molecule through one bond, formulae ii-1 to ii-6 are monovalent; when formulae ii-1 to ii-6 are connected to the rest of the molecule through two bonds, formulae ii-1 to ii-6 are bivalent; the saturated or partially unsaturated carbocyclyl represented by formulae ii-1 to ii-6 each optionally are substituted by one or more substituents defined in claims;

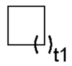
ii-1 ,
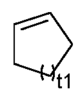
ii-2 ,
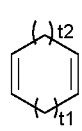
ii-3 ,
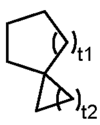
ii-4 ,
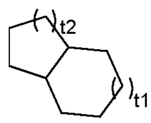
ii-5 , or
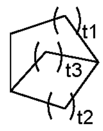
ii-6 ;

the saturated or partially unsaturated heterocyclyl is selected from formulae iii-1 to iii-16; when formulae iii-1 to iii-16 are connected to the rest of the molecule through one bond, formulae iii-1 to iii-16 are monovalent; when formulae iii-1 to iii-16 are connected to the rest of the molecule through two bonds, formulae iii-1 to iii-16 are bivalent; the saturated or partially unsaturated heterocyclyl represented by formulae iii-1 to iii-16 each optionally are substituted by one or more by one or more substituents defined in claims;

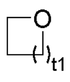
iii-1 ,
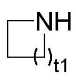
iii-2 ,
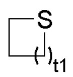
iii-3 ,
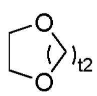
iii-4 ,
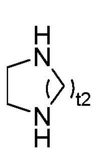
iii-5 ,
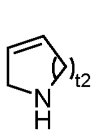
iii-6 ,
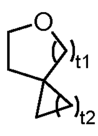
iii-7 ,
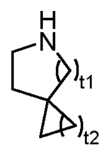
iii-8 ,
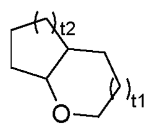
iii-9 ,
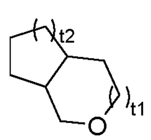
iii-10 ,

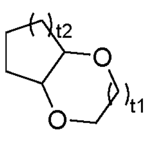
iii-11 ,
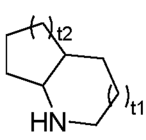
iii-12 ,
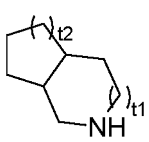
iii-13 ,
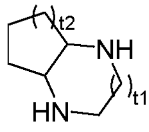
iii-14 ,
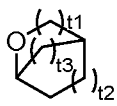
iii-15 , or
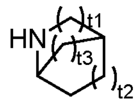
iii-16 ;

wherein, each t1 is independently 0, 1, 2, 3, 4, or 5; t2 and t3 each are independently 1, 2, 3, 4, or 5.

In some examples, the saturated or partially unsaturated carbocyclyl is selected from the following groups; when the saturated or partially unsaturated carbocyclyl is connected to the rest of the molecule through one bond, the following groups are monovalent; when the saturated or partially unsaturated carbocyclyl is connected to the rest of the molecule through two bonds, the following groups are bivalent; the following saturated or partially unsaturated carbocyclyl each optionally are substituted by one or more substituents defined in claims;

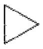 , 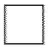 , 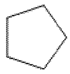 , 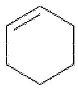 , 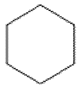 , 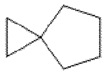 , 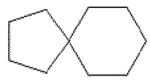 , 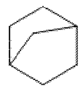 or 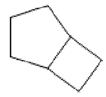 ;

the saturated or partially unsaturated heterocyclyl is selected from the following groups; when the saturated or partially unsaturated heterocyclyl is connected to the rest of the molecule through one bond, the following groups are monovalent; when the saturated or partially unsaturated heterocyclyl is connected to the rest of the molecule through two bonds, the following groups are bivalent; the following saturated or partially unsaturated heterocyclyl each optionally are substituted by one or more substituents defined in claims;

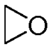 , 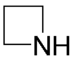 , 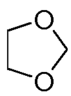 , 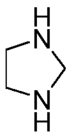 , 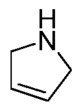 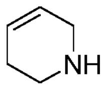 , 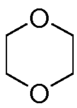 , 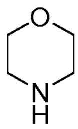 , 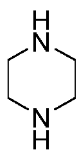 ,

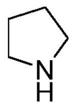 , 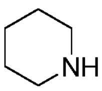 , 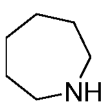 , 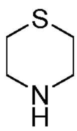 , 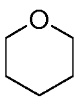 , 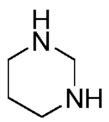 , 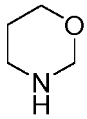 ,

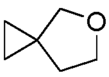 , 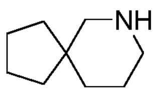 , 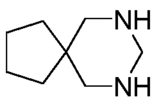 , 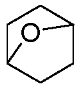 , 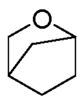 , 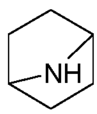 ,

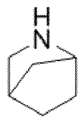 , 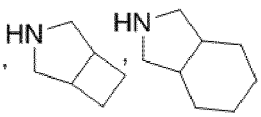 , 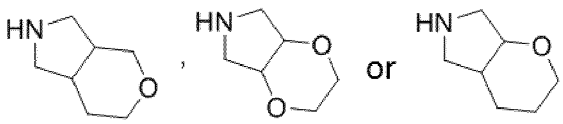 , or .

In some examples, the compound of the present application is a compound represented by any one of formulae II-1 to II-6, or a stereoisomer, a tautomer, a nitrogen oxide, a solvate, a metabolite, a pharmaceutically acceptable salt, or a prodrug thereof:

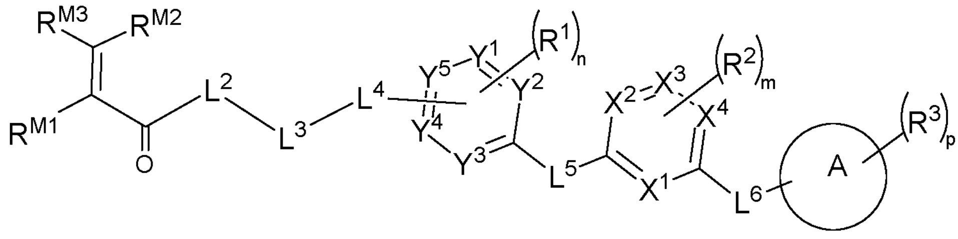

II-1,

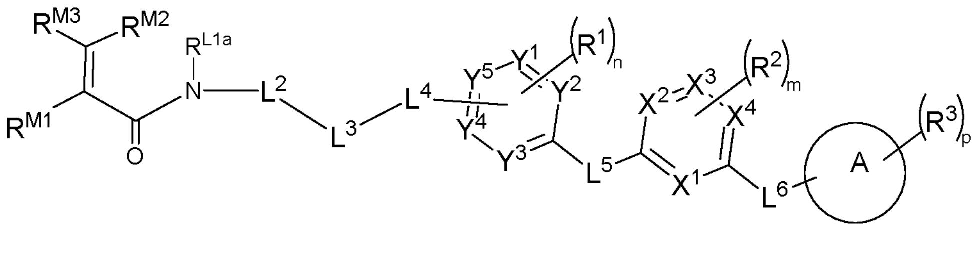

II-2,

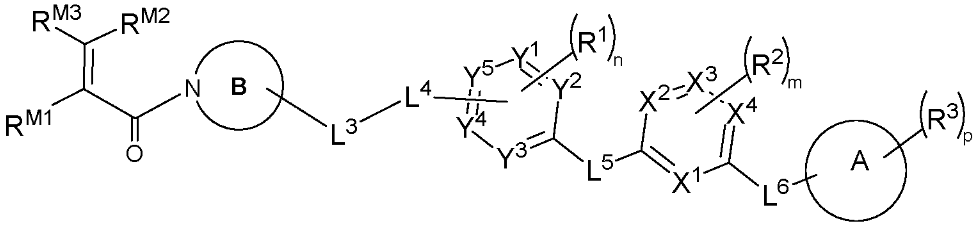

II-3,

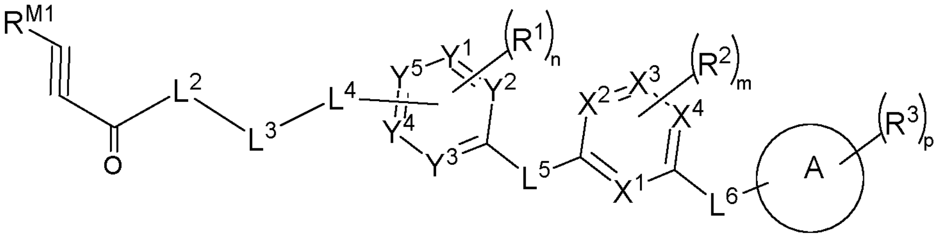

II-4,

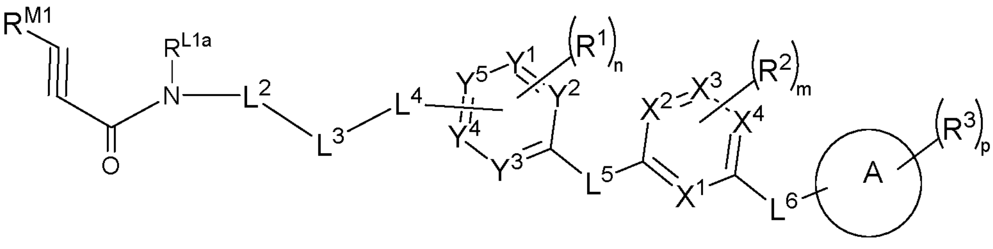

II-5, or

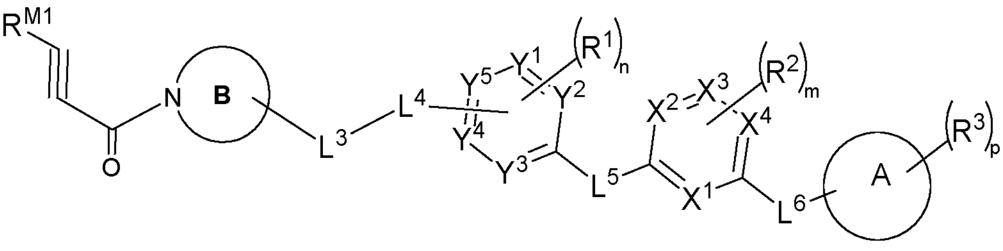

II-6

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents

selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl.

In some examples, the compound of the present application is a compound of formula III or a stereoisomer, a tautomer, a nitrogen oxide, a solvate, a metabolite, a pharmaceutically acceptable salt, or a prodrug thereof:

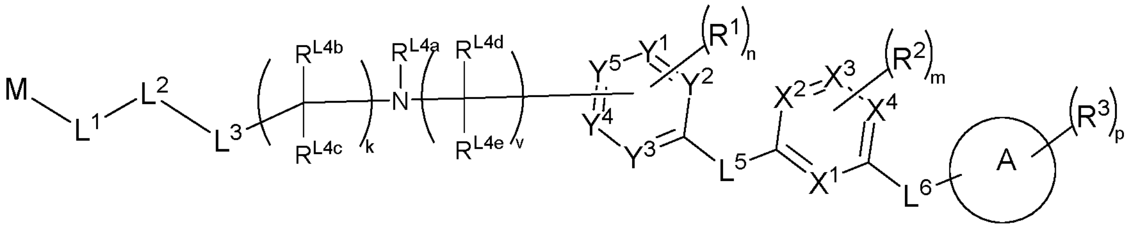

(III)

wherein, $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; k and v each are independently 0, 1 or 2.

In some examples, the compound of the present application is a compound represented by any one of formulae IV-1 to IV-6 or a stereoisomer, a tautomer, a nitrogen oxide, a solvate, a metabolite, a pharmaceutically acceptable salt, or a prodrug thereof:

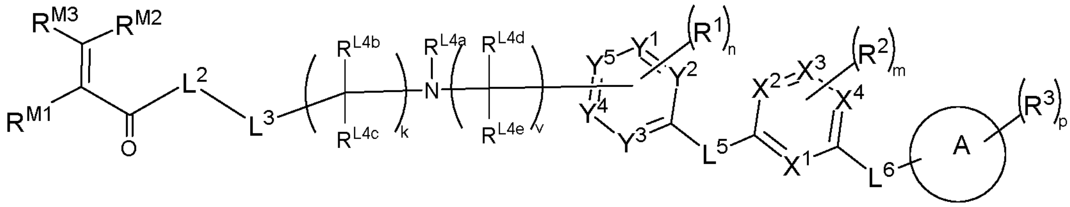

IV-1,

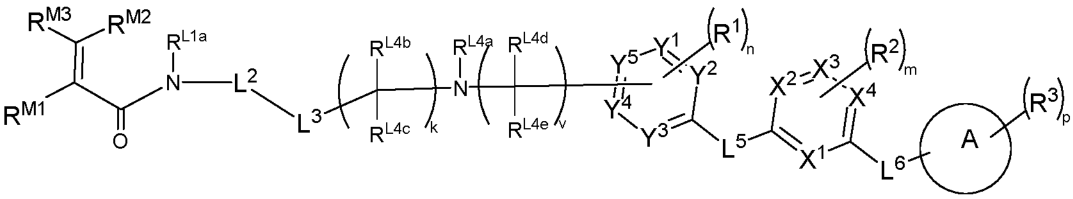

IV-2,

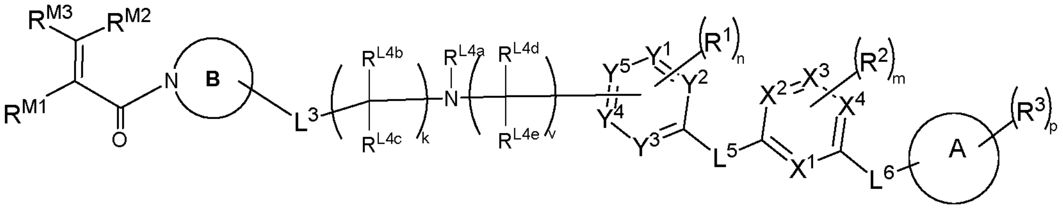

IV-3,

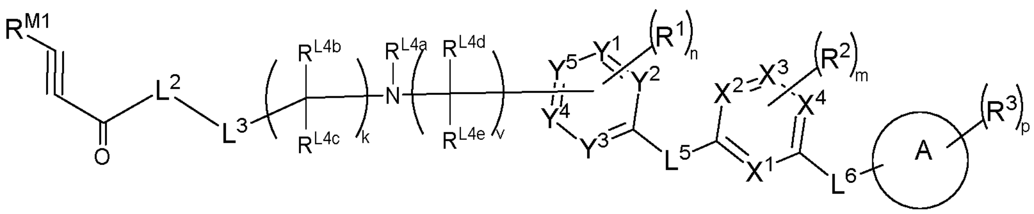

IV-4,

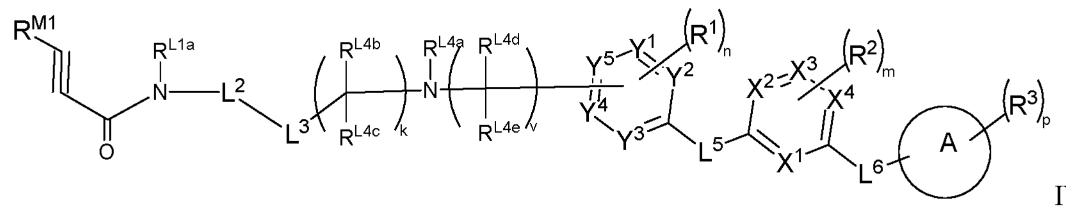

IV-5,

or

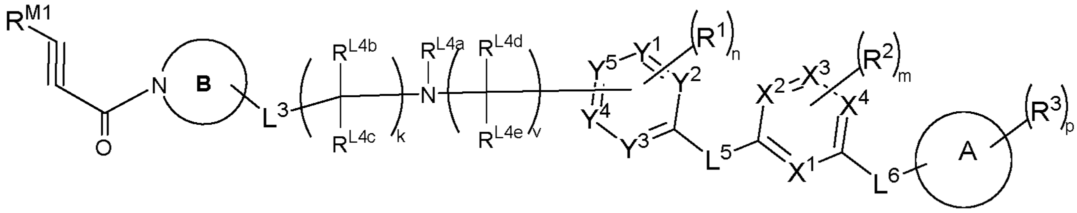

IV-6;

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; k and v each are independently 0, 1 or 2.

In some examples, the compound of the present application is a compound represented by any one of formulae V-1 to V-7 or a stereoisomer, a tautomer, a nitrogen oxide, a solvate, a metabolite, a pharmaceutically acceptable salt, or a prodrug thereof:

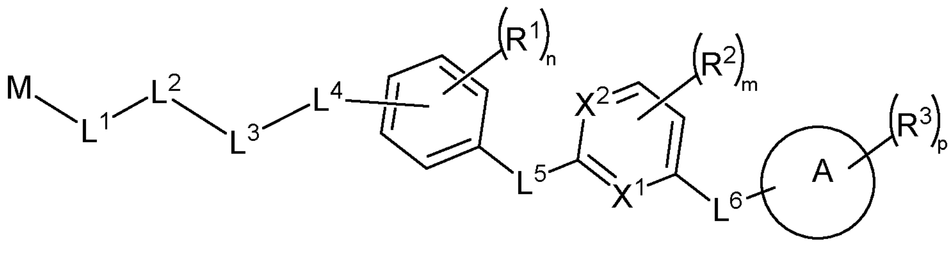

V-1,

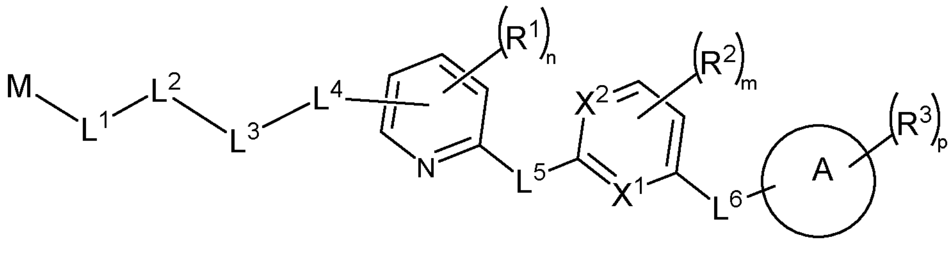

V-2,

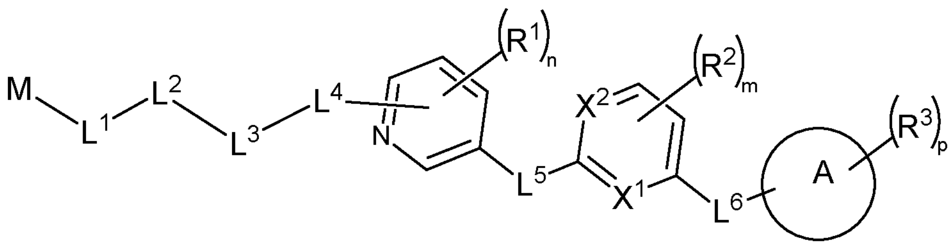

V-3,

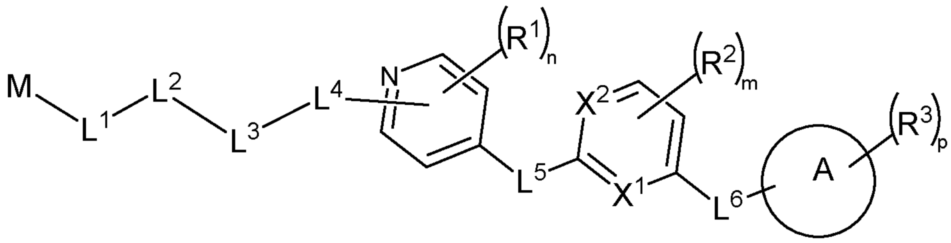

V-4,

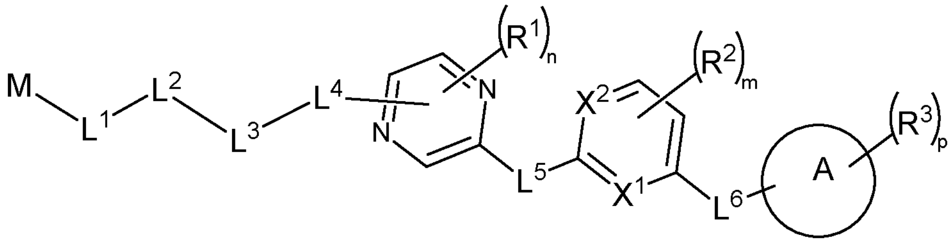

V-5,

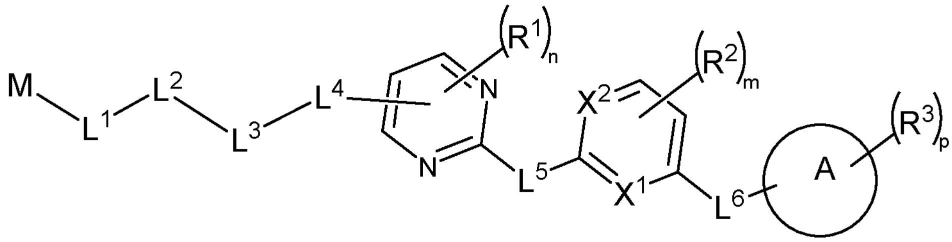

V-6,

or

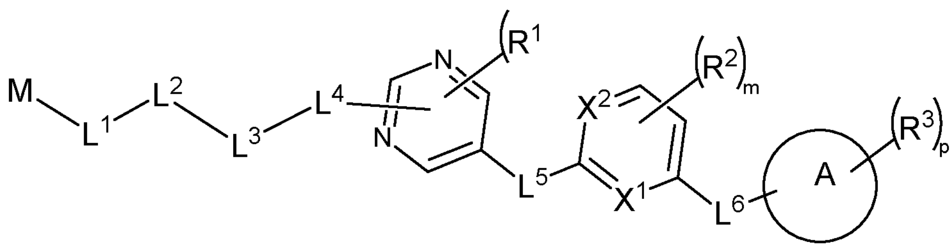

V-7.

In some examples, the compound of the present application is a compound represented by any one of formulae IV-1 to IV-5 or a stereoisomer, a tautomer, a nitrogen oxide, a solvate, a metabolite, a pharmaceutically acceptable salt, or a prodrug thereof:

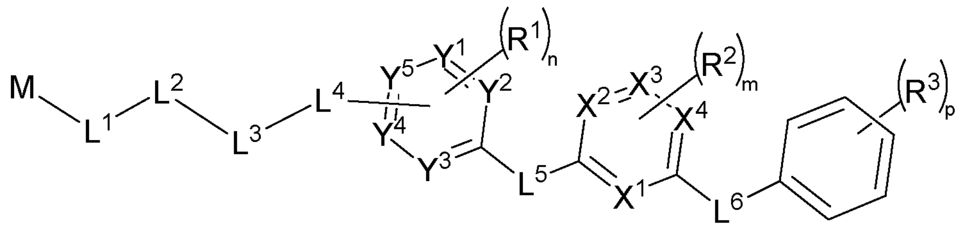
VI-1,

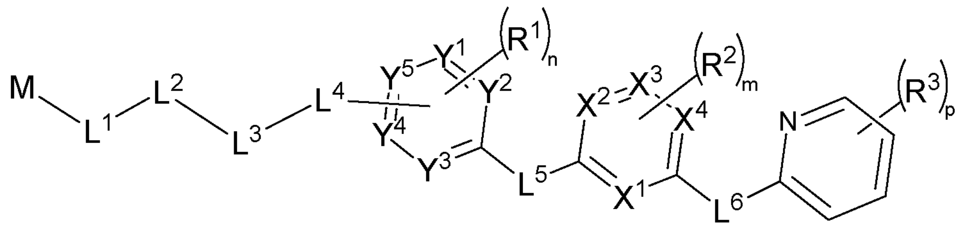
VI-2,

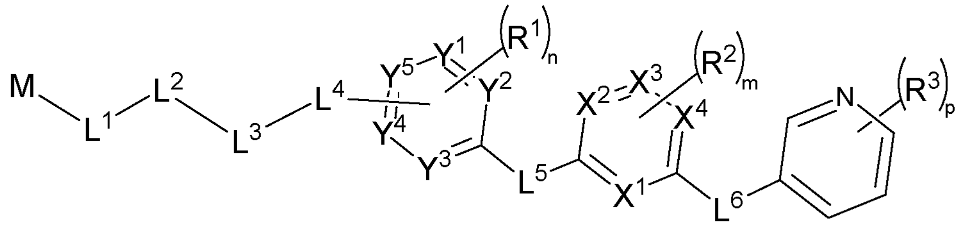
VI-3,

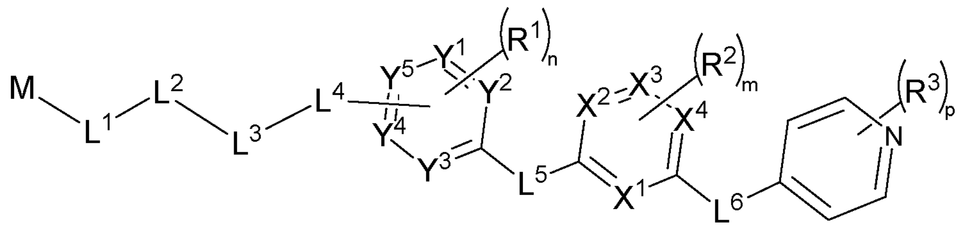
VI-4,

or

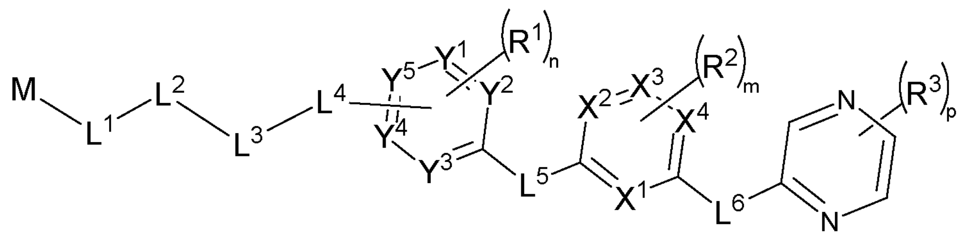
VI-5.

In some examples, the compound of the present application is a compound represented by any one of formulae VII-1 to VII-6 or a stereoisomer, a tautomer, a nitrogen oxide, a solvate, a metabolite, a pharmaceutically acceptable salt, or a prodrug thereof:

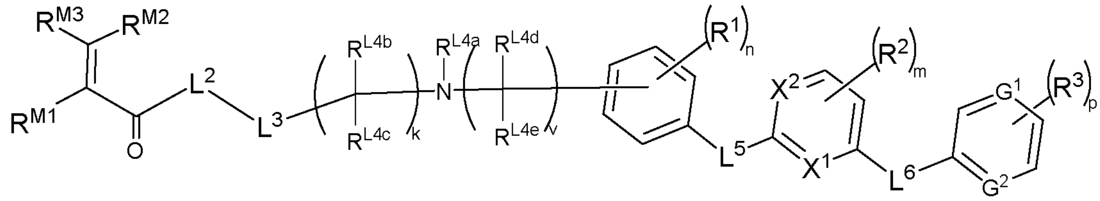
VII-1,

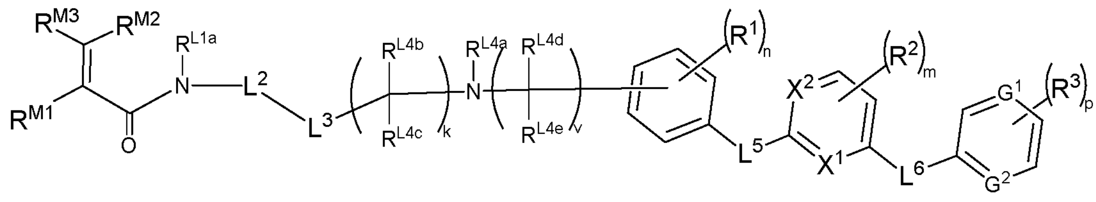

VII-2,

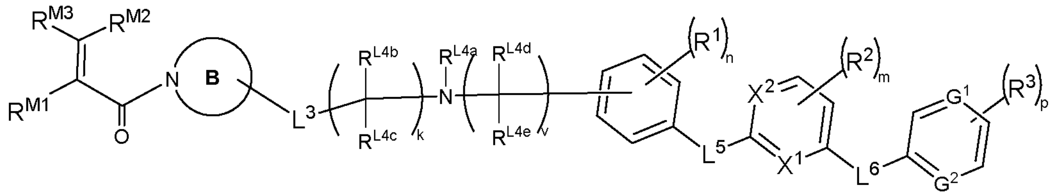

VII-3,

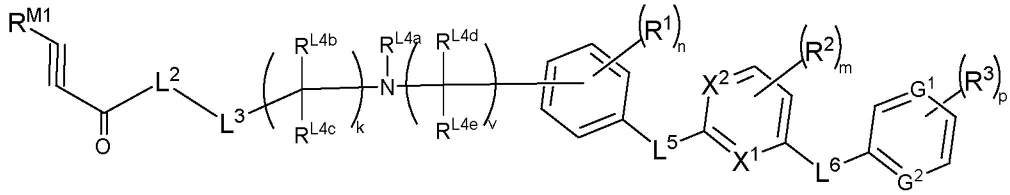

VII-4,

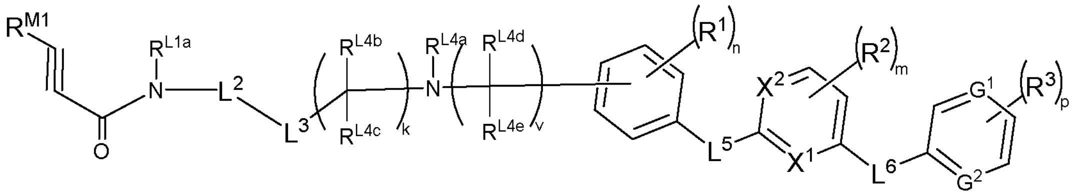

VII-5,

or

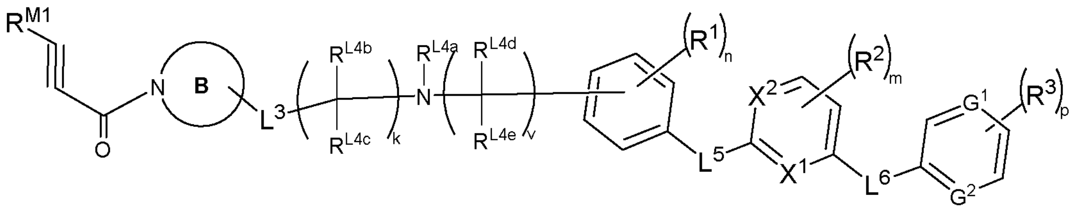

VII-6;

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; $G^1$ and $G^2$ each are independently CH or N; k and v each are independently 0, 1 or 2.

In some examples, the compound of the present application is a compound represented by any one of formulae VIII-1 to VIII-6 or a stereoisomer, a tautomer, a nitrogen oxide, a solvate, a metabolite, a pharmaceutically acceptable salt, or a prodrug thereof:

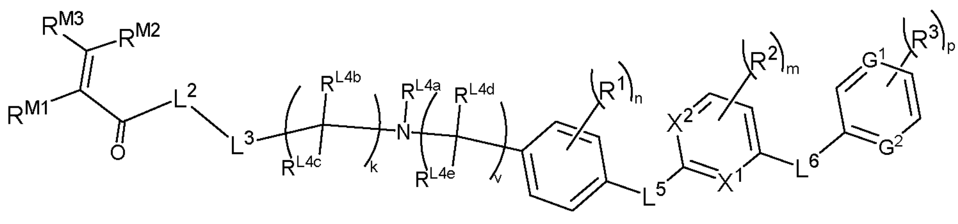

VIII-1,

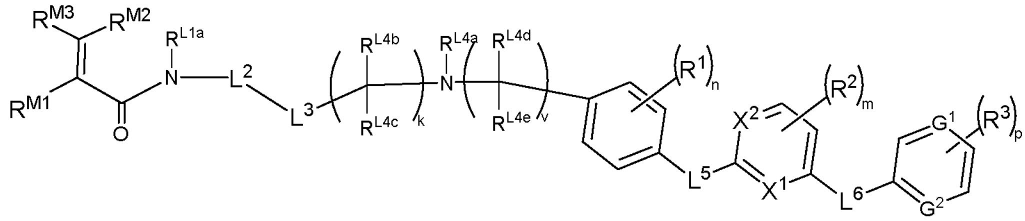

VIII-2,

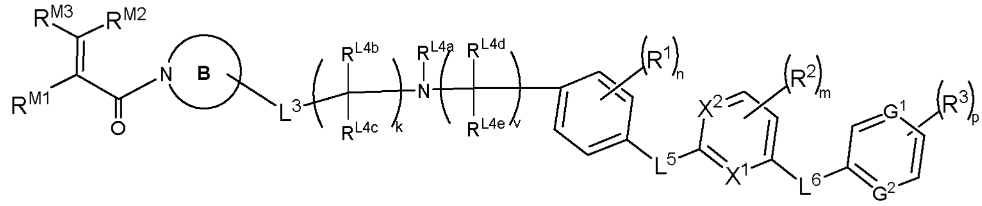

VIII-3,

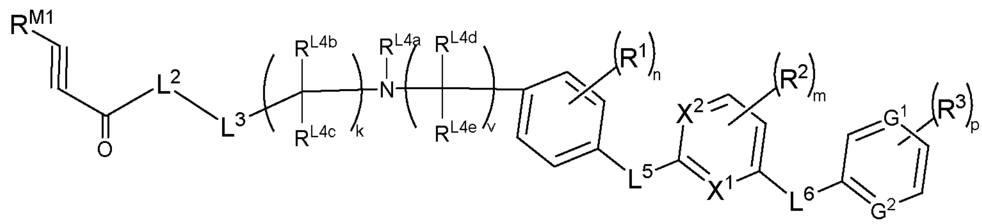

VIII-4,

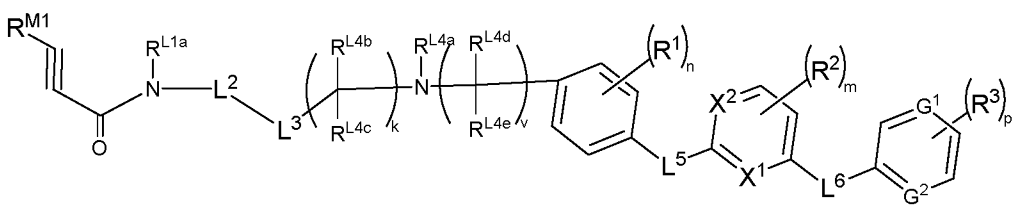

VIII-5, c

or

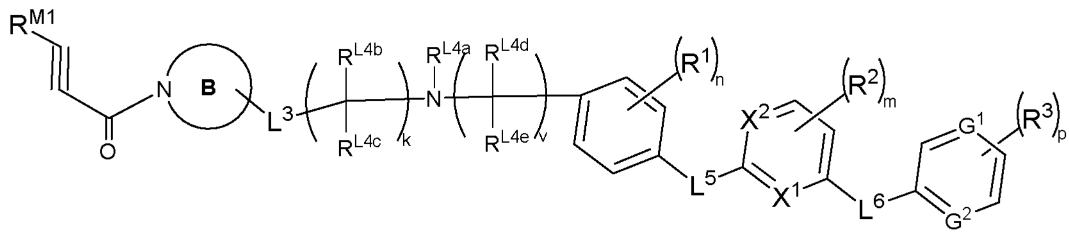

VIII-6

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; $R^{L4a}$ is hydrogen, deuterium, hydroxyl, cyano, halogen, oxo,

ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; $G^1$ and G2 each are independently CH or N; k and v each are independently 0, 1 or 2.

In some examples, the compound of the present application is the following compounds or a stereoisomer, a tautomer, a nitrogen oxide, a solvate, a metabolite, a pharmaceutically acceptable salt, or a prodrug thereof:

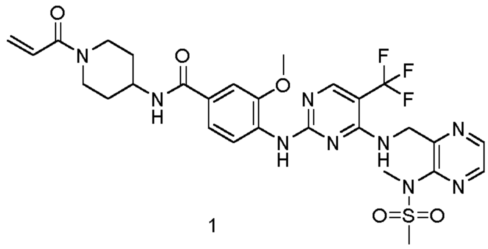

1

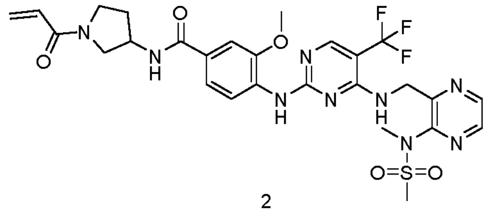

2

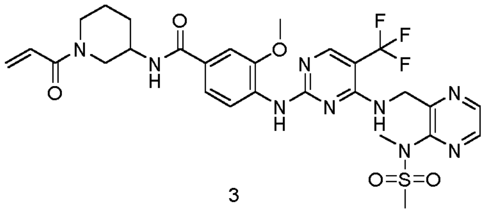

3

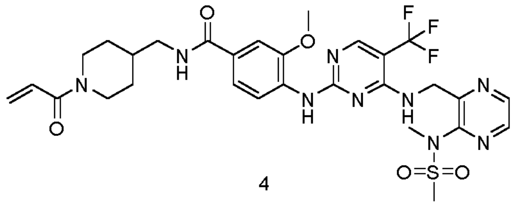

4

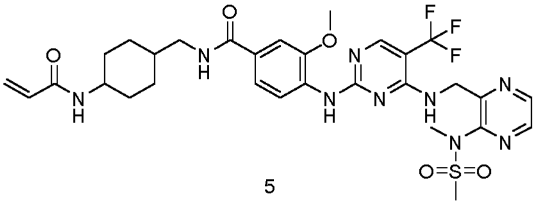

5

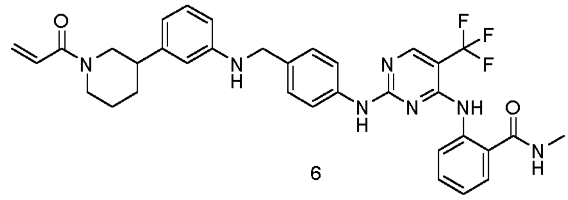

6

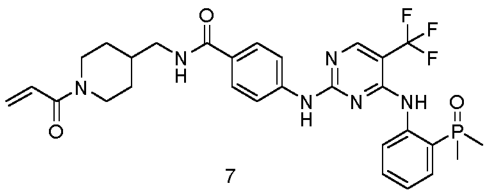

7

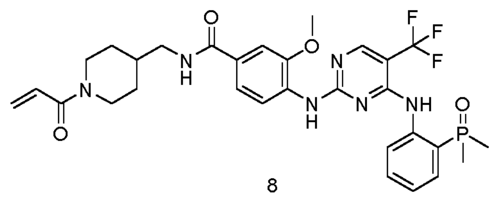

8

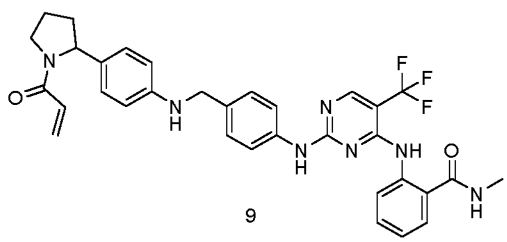

9

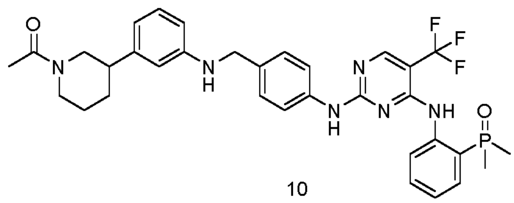

10

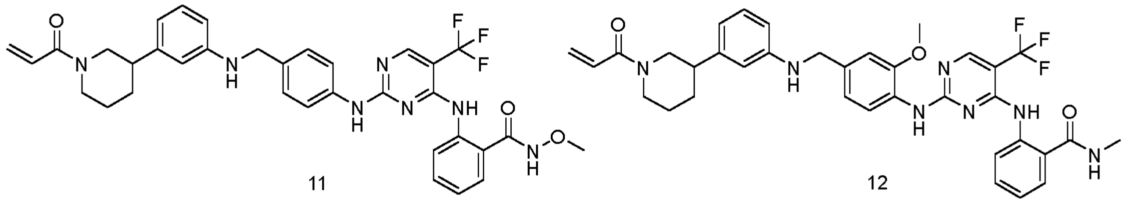

11

12

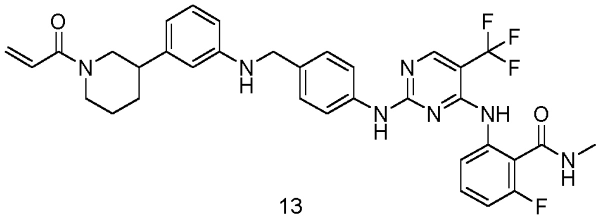

13

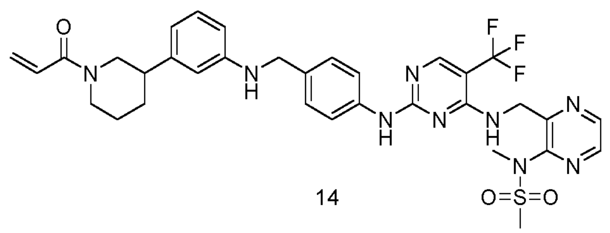

14

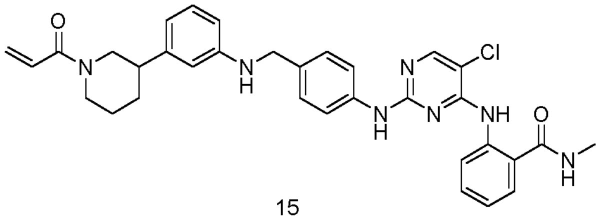

15

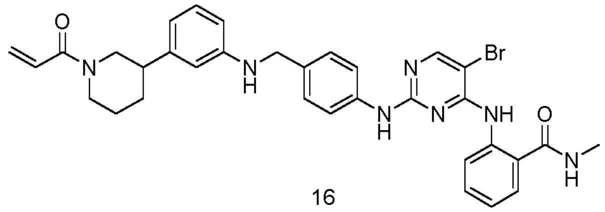

16

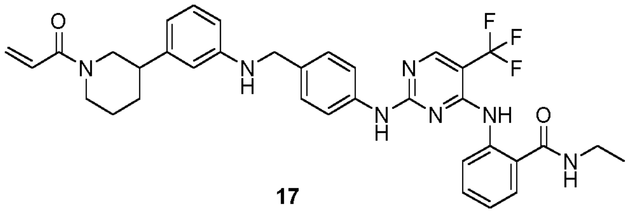

**17**

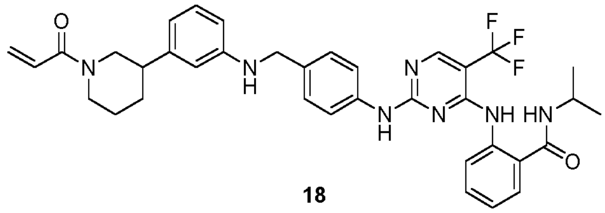

**18**

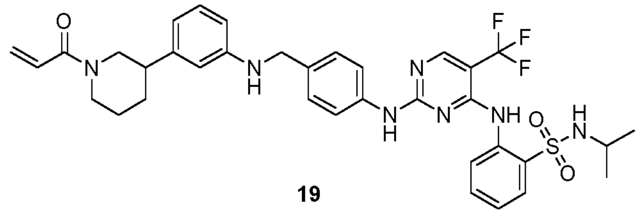

**19**

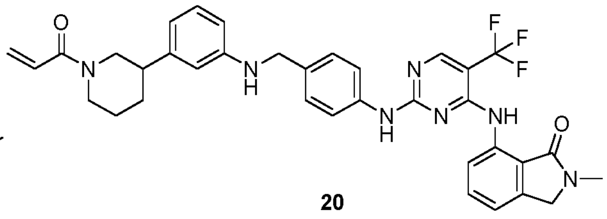

**20**

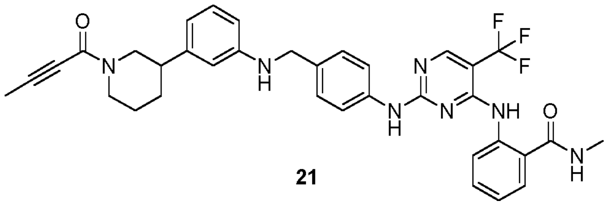

**21**

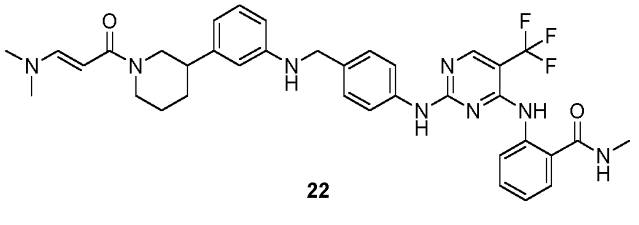

**22**

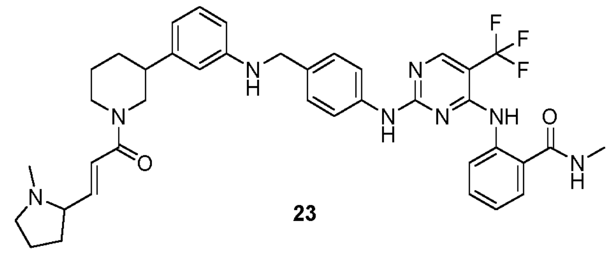

**23**

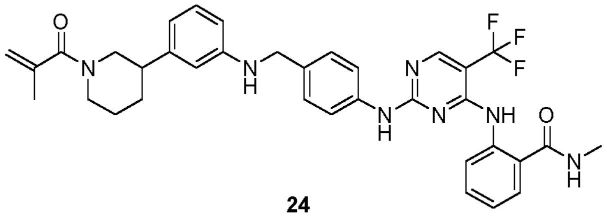

**24**

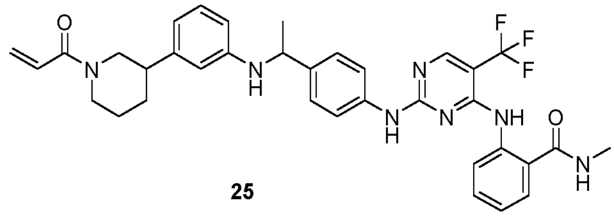

25

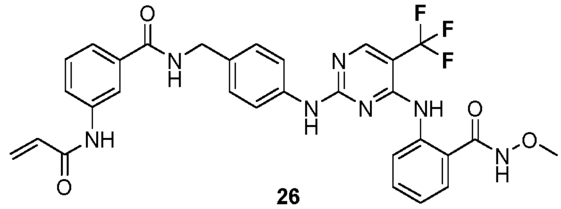

26

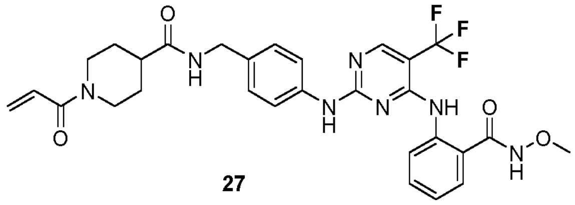

27

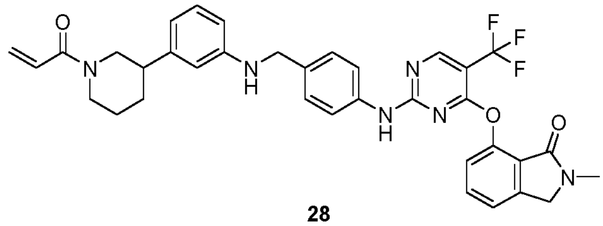

28

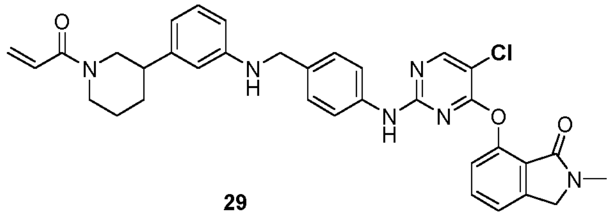

29

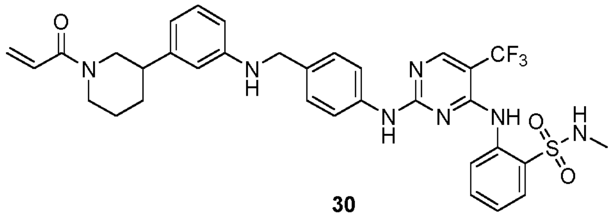

30

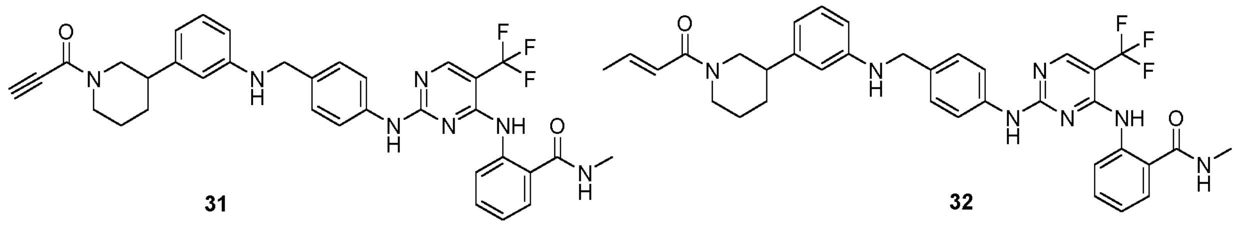

31

32

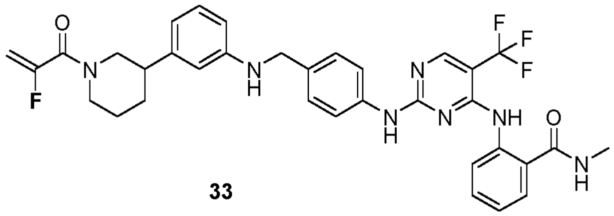

33

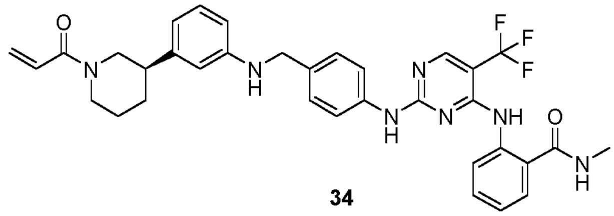

34

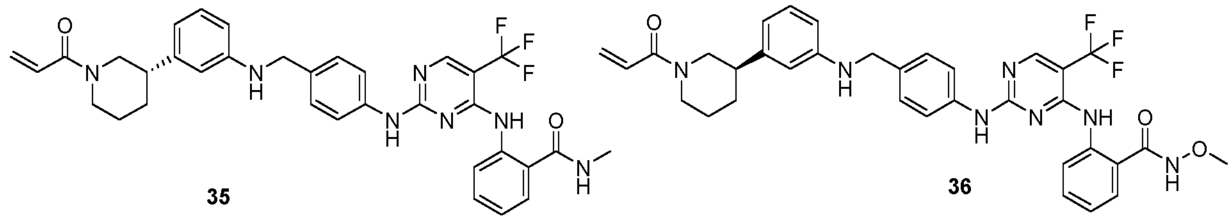

35

36

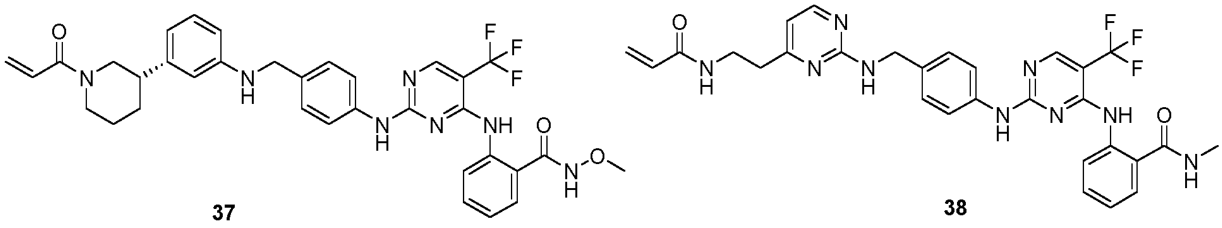

37 38

In another aspect, the present application provides a pharmaceutical composition comprising a compound of the present application and a pharmaceutically acceptable excipient. The amount of compounds in the composition of the present application can effectively treat or alleviate FAK and/or YAP related diseases in patients.

In some embodiments, the present application provides a pharmaceutical composition further comprising an additional therapeutic agent, wherein the additional therapeutic agent comprises other anticancer agents, other drugs for treating pulmonary arterial hypertension, or a combination thereof.

As described herein, the pharmaceutical composition of the present application further comprises pharmaceutically acceptable excipients suitable for a specific target dosage form, including any solvent, diluent, or other liquid excipients, dispersants or suspending agents, surfactants, isotonic agents, thickeners, emulsifiers, preservatives, solid binders or lubricants, and the like. They are described, for example in the following literature: Remington: The Science and Practice of Pharmacy, 21st edition, 2005, ed. D. B. Troy, Lippincott Williams & Wilkins, Philadelphia, and Encyclopedia of Pharmaceutical Technology, eds. J. Swarbrick and J. C. Boylan, 1988-1999, Marcel Dekker, New York. Based on the contents of the literature presented here, it is indicated that various excipients can be applied to formulations of pharmaceutically acceptable compositions and their well-known preparation methods. Except for those excipients that are incompatibility with the compound of the present application, such as any adverse biological effects generated or interactions with any other component of a pharmaceutically acceptable composition in a harmful manner, their use is also contemplated by the present application.

The pharmaceutical composition of the present application further comprises i) one or more other FAK inhibitors and/or ii) one or more other types of protein kinase inhibitors and/or one or more other types of therapeutic agents. One or more other types of protein kinase inhibitors include PYK2 or src inhibitors, while other types of therapeutic agents include other anticancer agents, other drugs for treating pulmonary arterial hypertension, etc.

The term "therapeutically effective amount" used herein refers to the total amount of active components that are sufficient to demonstrate significant benefits to patients (such as reduced viral load). When available for treatment, a therapeutically effective amount of the compounds of the present application, particularly the compound of formulae I, II-1 to II-6, III, IV-1 to IV-6, V-1 to V-6, VI-1 to VI-5, VII-1 to VII-6, VIII-1 to VIII-6, and their pharmaceutically acceptable salts, can be not only administered as unprocessed chemicals but also provided as active ingredients in pharmaceutical compositions. Therefore, the present application also provides a pharmaceutical composition comprising a therapeutically effective amount of the compound of the present application, particularly the compound of formulae I, II-1 to II-6, III, IV-1 to IV-6, V-1 to V-6, VI-1 to VI-5, VII-1 to VII-6, VIII-1 to VIII-6, or pharmaceutically acceptable salts thereof, and one or more pharmaceutically acceptable carriers, diluents, or excipients thereof.

In the case of administering a single ingredient alone, this term only pertains to that ingredient. In the case of administering in a combination, this term refers to a combined amount of active ingredients that cause therapeutic effects regardless of dosing in a combination, sequentially or simultaneously. The compound of the present application, in particular the compound of formulae I, II-1 to II-6, III, IV-1 to IV-6, V-1 to V-6, VI-1 to VI-5, VII-1 to VII-6, VIII-1 to VIII-6, and their pharmaceutically acceptable salts, are described above. In terms of compatibility with other components of the formulation and harmlessness to its recipients, the carrier, diluent, or excipient must be acceptable. According to the contents in another aspect of the present application, a method for preparing a pharmaceutical formulation is also provided, comprising mixing the compound of the present application, particularly the compound of formulae I, II-1 to II-6, III, IV-1 to IV-6, V-1 to V-6, VI-1 to VI-5, VII-1 to VII-6, VIII-1 to VIII-6, or a pharmaceutically acceptable salt thereof, with one or more pharmaceutically acceptable carriers, diluents, or excipients.

The amount of active ingredient combined with one or more excipients to prepare a single dosage form will necessarily vary depending on a host to be treated and specific administration routes. For example, formulations intended for oral administration to humans generally comprise, for example, 0.5mg to 2g active ingredient (preferably 0.5mg to 1g active ingredient, such as 0.5mg-0.5g active ingredient, more preferably 0.5 mg to 100 mg active ingredient, such as 1 mg to 30 mg) compounded with appropriate and convenient amounts of excipients that are about 5% to about 98% by weight of the total composition. The amount of the compound of formulae I, II-1 to II-6, III, IV-1 to IV-6, V-1 to V-6, VI-1 to VI-5, VII-1 to VII-6, VIII-1 to VIII-6 as active ingredients being mixed with carrier materials to prepare a single dosage form will vary depending on diseases to be treated, severity of diseases, administration time, administration route, excretion rate of the compound used, treatment time, as well as age, gender, weight and condition of the patient.

The preferred unit dosage form is a unit dosage form containing a daily dose or divided dose or an appropriate fraction of the active ingredients mentioned herein. Treatment can begin with a small dose that is clearly lower than the optimal dose of the compound, followed by increasing the dose in smaller increments until the optimal effect is achieved in this situation. Generally speaking, the most desirable concentration level for compound administrated is usually that it can provide effective results in anti-tumor effects without causing any adverse or toxic side effects.

During the treatment or prevention with the compounds of the present application, if divided doses are required, they are usually administered to obtain a daily dose of, for example, 0.1 mg/kg to 75 mg/kg body weight. In summary, lower doses may be administered when a parenteral route is adopted. Therefore, for example, in terms of intravenous or intraperitoneal administration, the commonly used dose is, for example, 0.1 mg/kg to 30 mg/kg body weight. Similarly, for inhalation administration, the dose to be used may be, for example, 0.05 mg/kg to 25 mg/kg body weight. Oral administration is also appropriate, especially in tablet form. Typically, the unit dosage form may contain approximately 0.5 mg to 0.5 g of the compound of the present application, and the unit dosage form may be administered once, twice, three or four times a day, or at a higher frequency if needed.

The pharmaceutical dosage forms of the compound of the present application and the composition thereof can be provided in the form of rapid release, controlled release, sustained release, or target drug release systems. For example, commonly used dosage forms include solutions and suspensions, (micro)emulsion, ointments, gel and patches, liposomes, tablets, sugar coated pills, soft or hard shell capsules, suppositories, ovules, implants, amorphous or crystalline powders, aerosols and freeze-dried formulations. Depending on the administration route used, special devices, such as syringes and needles, inhalers, pumps, injection pens, applicators, or special flasks may be required to deliver or apply the drug. The pharmaceutical dosage forms often consist of drugs, excipients, and container/sealing systems. One or more excipients (also known as non-active ingredients) can be added to the compound of the present application to improve or promote the manufacturing, stability, administration, and safety of drugs, and a method for achieving a desired drug release profile can be provided. Therefore, the type of excipient added to the drug depends on various factors, such as physical and chemical properties of the drug, the route of administration, and the preparation steps. There are excipients in the medicinal field, including those listed in various pharmacopoeias (see U.S. Pharmacopoeia (USP), Japanese Pharmacopoeia (JP), European Pharmacopoeia (EP), and British Pharmacopoeia (BP); publications, such as Inactive Ingredient Guide, 1996, issued from Center for Drug Evaluation and Research (CEDR) in the U.S. Food and Drug Administration (www.fda.gov); Handbook of Pharmaceutical Additives edited by Ash and Ash (2002, Synapse Information Resources, Inc., Endicott NY; etc.).

The pharmaceutical composition is suitable for administration via any suitable route, such as oral (including oral cavity or sublingual), rectal, nasal, local (including buccal, sublingual or transdermal), vaginal or parenteral (including subcutaneous, intradermal, intramuscular, intraarticular, intrasynovial, intrasternal, intrathecal, intralesional, intravenous or subdermal injection or infusion) routes. Such formulations can be prepared using any known method in the field of pharmacy, such as mixing active ingredients with carriers or excipients. Oral or injection administration is preferable.

A pharmaceutical formulation suitable for oral administration is provided in independent units, such as capsules or tablets, powder or granule, solution or suspension in aqueous or non-aqueous liquids, edible effervescent or whipping preparation, or oil-in-water emulsion or water-in-oil emulsion.

For example, for oral administration in tablet or capsule form, the pharmaceutical active component can be mixed with orally pharmaceutically acceptable non-toxic inert carriers (such as ethanol, glycerol, water, etc.). A powder formulation is prepared by crushing the compound into appropriate fine sizes and mixing with pharmaceutically acceptable carriers (edible sugars such as starch or mannitol) that are also crushed. Deodorizers, preservatives, dispersants, and colorants may also be used for oral administration.

Capsules are prepared by preparing a powdered mixture as described above and filling it into a shaped gelatin shell. Before the filling operation, glidants and lubricants (such as colloidal silica, talc powder, magnesium stearate, calcium stearate, or solid polyethylene glycol) can be added to the powdered mixture. It is also possible to add disintegrants or solubilizers (such as agar, calcium carbonate, or sodium carbonate) that may improve drug availability after taking capsules.

Moreover, suitable binders, lubricants, disintegrants, and colorants can also be blended into the mixture when needed or necessary. Suitable binders include starch, gelatin, natural sugars (such as glucose or β-lactose), corn sweeteners, natural and synthetic gums (such as arabic gum, tragacanth gum or sodium alginate), carboxymethyl cellulose, polyethylene glycol, etc. Lubricants used for these formulations include sodium oleate, sodium chloride, etc. Disintegrants include, but are not limited to, starch, methyl cellulose, agar, bentonite, xanthan gum, etc. For example, tablets are made by making a powdered mixture, granulating or prepressing, adding lubricants and disintegrants, and then pressing into a tablet. A powdered mixture is prepared by mixing appropriately crushed compounds with diluents or basic materials as described above, optionally binders (such as carboxymethyl cellulose, alginate, gelatin, or polyvinylpyrrolidone), dissolution retardants (such as paraffin), absorption accelerators (quaternary salts), and/or absorbers (such as bentonite, kaolin, or dicalcium phosphate). The powdered mixture is granulated by wetting the powdered mixture with binder (such as syrup, starch syrup, acadiamucilage, or cellulose material or polymer material solution), then passing

it through a sieve under pressure. An alternative method for granulation is to pass the powdered mixture through a tablet press, resulting in poorly formed clumps that are then crushed into particles. The particles can be lubricated by adding stearic acid, stearate salts, talc powder or mineral oil to prevent them from sticking to a die of the tablet press, then the lubricated mixture are pressed into a tablet. The compound of the present application can also be mixed with a freely flowing inert carrier and then compressed into a tablet without granulation or prepressing steps. the present application can provide protective coating materials that are transparent or opaque, comprising shellac sealing coating, sugar coating or polymer material coating, and polish coating of wax. Dyes can be added to these coating materials to distinguish unit doses from each other.

Oral liquid formulations such as solutions, syrups, and elixirs can be prepared into dosage units, thereby containing a predetermined amount of compounds. Syrups can be prepared by dissolving the compound in appropriately seasoned aqueous solutions, while elixirs can be prepared by using non-toxic solvents. Solubilizers and emulsifiers (such as ethoxylated isostearic alcohol and polyoxyethylene sorbitol ether), preservatives, flavor correction additives (such as mint oil or natural sweeteners or saccharin or other artificial sweeteners) can also be added.

If appropriate, the formulation in dosage unit for oral administration can be microencapsulated. Formulations can also be made for delayed or sustained release, such as by coating or embedding in particulate materials such as polymer, wax, and so on.

The compound of the present application, particularly the compound of formulae I, II-1 to II-6, III, IV-1 to IV-6, V-1 to V-6, VI-1 to VI-5, VII-1 to VII-6, VIII-1 to VIII-6, and their pharmaceutically acceptable salts, can also be administered through liposome delivery systems, such as small monolayer liposomes, large monolayer liposomes, and multilayer liposomes. Liposomes can be composed of various phospholipids, such as cholesterol, octadecylamine, or phosphatidylcholine.

The compound of the present application, particularly the compound of formulae I, II-1 to II-6, III, IV-1 to IV-6, V-1 to V-6, VI-1 to VI-5, VII-1 to VII-6, VIII-1 to VIII-6, and their pharmaceutically acceptable salts, can also be delivered using monoclonal antibodies as separate carriers (coupled with the compound molecule). The compound can also be coupled with soluble polymers that serve as targeting drug carriers. This type of polymer can include polyvinylpyrrolidone, pyran copolymers, polyhydroxypropyl methacrylamide phenol, polyhydroxyethyl asparagine phenol, or polyoxyethylene polylysine substituted by palmitoyl residues. In addition, the compound can be coupled with a class of biodegradable polymers, such as polylactic acid, poly ε-caprolactone, polyhydroxybutyric acid, poly ortho esters, polyacetal, polydihydropyran, a crosslinked copolymer or amphiphilic block copolymer of polycyanoacrylate and hydrogel, to achieve the controlled release of the drug.

A pharmaceutical formulation suitable for transdermal administration can serve as discrete patches to maintain close contact with the recipient's epidermis for extended periods of time. For example, active ingredients can be delivered through iontophoresis patches, as commonly seen in Pharmaceutical Research 1986, 3 (6), 318. Pharmaceutical formulations suitable for local administration can be made into ointment, cream, suspension, lotion, powder, solution, paste, gel, spray, aerosol, oil preparation or transdermal patch. Pharmaceutical formulations suitable for rectal administration can be provided as suppositories or enemas. Pharmaceutical formulations suitable for vaginal administration can be provided as vaginal suppository, vaginal plug, ointment, cream, gel, paste, foam or spray. Pharmaceutical formulations suitable for parenteral administration include aqueous and non-aqueous sterile injection solutions and aqueous and non-aqueous sterile suspensions. The aqueous and non-aqueous sterile injection solutions may contain antioxidants, buffering agents, antibacterial agents, and solutes that make the formulation isotonic with the recipient's blood. The aqueous and non-aqueous sterile suspensions may include suspending agents and thickeners. The formulation can be provided in unit dose or multi dose containers, such as sealed ampoule and small bottles, and can be stored under freeze-drying conditions until use, and added sterile liquid carriers, such as injection water, before use. The injectable solution and suspension prepared immediately before use can be prepared from sterile powder injections, granules, and tablets. Pharmaceutical formulations suitable for nasal administration (where the carrier is a solid) include coarse powders having a particle size range of, for example, 20-500μm, administered by nasal inhalation, i.e. rapidly inhaled through a nasal channel from a coarse powder container close to the nose. Suitable formulations for administration as nasal sprays or nasal drops (wherein the carrier is a liquid) include aqueous solutions or oily solutions of active ingredients. Pharmaceutical formulations suitable for inhalation administration include dry powder, aerosols, suspensions, or solution compositions. A dry powder composition to be delivered to the lungs by inhalation, typically comprises a compound of formula (I) or a pharmaceutically acceptable salt thereof as a finely ground powder, together with one or more pharmaceutically acceptable excipients as a finely ground powder. Pharmaceutically acceptable excipients that are particularly suitable for use in dry powders are known to those skilled in the art and include lactose, starch, mannitol, and mono-, di-, and polysaccharides. Fine ground powders can be prepared by, for example, micronisation and grinding. Generally speaking, compounds having a reduced size (such as micronised) can be defined by a D50 value of about 1μm to about 10μm (e.g. detected by laser diffraction).

Patients can be administered dry powder through a reservoir dry powder inhaler (RDPI), which has a reservoir suitable for storing multiple doses (unmeasured doses) of medication in the form of dry powder. RDPI typically includes

devices for measuring the dose of drugs transferred from the reservoir to delivery location each time. For example, a metering device may include a measuring cup that can be moved from a first position to a second position, where the cup can load drugs from a reservoir at the first position and prepare a metered drug dose for patient inhalation at the second position. Alternatively, dry powder can be presented as capsules (such as gelatin or plastic), cartridges, or blister packaging for multi-dose dry powder inhalers (MDPI). MDPI is an inhaler, in which the drug is contained in a multi-dose package, containing (or carrying otherwise) a multiple of limited doses (or parts thereof) of the drug. When the dry powder is presented as a blister packaging, it includes a multiple of blisters for accommodating drugs in the form of the dry powder. The blister is typically arranged in a regular manner to facilitate drug release from them. For example, blisters can usually be arranged in a circular shape on a disc shaped blister packaging, or they can be elongated, for example, in the form of threads or strips.

The aerosol can be made by suspending or dissolving the compound of formula I of the present application or a pharmaceutically acceptable salt thereof in a liquefied propellant. Suitable propellants include halogenated hydrocarbons, hydrocarbons, and other liquefied gases. Representative propellants include: trichlorofluoromethane (propellant 11), dichlorofluoromethane (propellant 12), dichlorotetrafluoroethane (propellant 114), tetrafluoroethane (HFA-134a), 1,1-difluoroethane (HFA-152a), difluoromethane (HFA-32), pentafluoroethane (HFA-12), heptafluoropropane (HFA-227a), perfluoropropane, perfluorobutane, perfluoropenane, butane, isobutane, and pentane. Typically, an aerosol containing polycrystalline substances or salts of the present application is administered to patients via a metered dose inhaler (MDI). Such a device is known to those skilled in the art. Aerosols may contain additional pharmaceutically acceptable excipients, which are typically used in conjunction with MDI, such as surfactants, lubricants, cosolvents, and other excipients to improve the physical stability of the formulation, improve valve characteristics, improve solubility, or improve taste.

It should be understood that, in addition to the ingredients specifically mentioned above, the formulation also includes other commonly used ingredients in the field related to the type of formulations, such as taste correction agents suitable for oral administration.

The compound of the present application or a composition comprising the compound of the present application is suitable for the prevention and treatment of FAK related diseases, including cancer, pulmonary arterial hypertension, immune diseases, arthritis, inflammatory bowel disease, pathological angiogenesis, etc. The compounds of the present application and their pharmaceutical compositions are particularly useful for preparing medicaments for the treatment of cancer, pulmonary arterial hypertension, and pathological angiogenesis. The compound of the present application and its pharmaceutical composition can be therapeutically used in combination with: i) one or more other FAK inhibitors and/or, ii) one or more other types of protein kinase inhibitors and/or one or more other types of therapeutic agents, which can be administered orally in the single dosage form, in separate oral dosage forms (such as sequential or non-sequential), or administered together or separately (such as sequential or non-sequential) by injection. Among them, other types of therapeutic agents include other anticancer agents and other drugs for treating pulmonary arterial hypertension.

It is expected that the compound of the present application or its pharmaceutical composition has anti-tumor properties (among other characteristics), which are believed to originate from the inhibition of FAK. For example, the compound or its pharmaceutical composition may exhibit anti-proliferative and/or pro-apoptotic and/or anti-invasive and/or anti-cell-motility and/or anti-angiogenic activity. This compound seems to be useful for treating tumors such as induced by FAK, specifically as an anticancer agent.

Therefore, it is expected that the compound of the present application or its pharmaceutical compositions can be used for the treatment of diseases or medical conditions mediated solely or partially by FAK, that is, the compounds can be used to produce FAK inhibitory effects in warm blooded animals in need of such treatment. Therefore, the compound of the present application provides a method for treating malignant cells, characterized by inhibiting FAK. Specifically, the compound of the present application or its pharmaceutical compositions can be used to generate anti-proliferative and/or pro-apoptotic and/or anti-invasive and/or anti-cell-motility and/or anti-angiogenic effects, which are mediated solely or partially through inhibition of FAK function. Specifically, it is expected that the compounds of the present application can be used for the prevention or treatment of tumors sensitive to FAK inhibition, which are related to steps such as angiogenesis, proliferation and signal transduction, and these steps cause the proliferation, invasion, migration, especially angiogenesis of these tumor cells. Therefore, the compounds of the present application can be used to treat hyperproliferative diseases, including cancer. Benign or malignant tumors may affect any tissue and include non-solid tumors such as leukemia, multiple myeloma or lymphoma, and solid tumors, such as bile duct cancer, bone cancer (including Ewing's tumor), bladder cancer, brain cancer/CNS cancer, breast cancer, colorectal cancer, endometrial cancer, gastric cancer, head and neck cancer, liver cancer, lung cancer (including non-small cell lung cancer and small cell lung cancer), neural cancer (including neuroblastoma), esophageal cancer, ovarian cancer, pancreatic cancer, prostate cancer, kidney cancer, skin cancer, testicular cancer, thyroid cancer, uterine cancer, cervical cancer, vulva cancer, and Kaposi's sarcoma. It is expected that the compounds of the present application can be used for the treatment of pathogenic angiogenesis (pathological angiogenesis), such as for the treatment of cancer described above and other

diseases that produce inappropriate or pathogenic angiogenesis, such as age-related macular degeneration (AMD) and solid tumor related cancers.

The compound of the present application or a composition comprising the compound of the present application is suitable for the prevention and treatment of YAP related diseases. Preferably, the YAP related diseases are selected from cancer that is selected from one or more of skin cancer, bone cancer, glioma, breast cancer, adrenal cancer, bladder cancer, esophageal cancer, head or neck cancer, liver cancer, parathyroid cancer, penis cancer, small intestine cancer, thyroid cancer, urethral cancer, cervical cancer, endometrial cancer, fallopian tube cancer, renal pelvis cancer, vaginal cancer, vulva cancer, chronic or acute leukemia, colon cancer, melanoma, hematological malignancy, Hodgkin’s lymphoma, lung cancer, lymphocytic lymphoma, central nervous system (CNS) tumor, ovarian cancer, pancreatic cancer, pituitary adenoma, prostatic cancer, soft tissue sarcoma, gastric cancer and uterine cancer. More preferably, the cancer is selected from one or more of lung cancer, colon cancer, ovarian cancer, prostatic cancer, and liver cancer.

The compound of the present application or a composition comprising the compound of the present application is suitable for regulating or treating FAK and YAP related diseases. Preferably, the FAK and YAP related diseases are selected from cancer that is selected from one or more of skin cancer, bone cancer, glioma, breast cancer, adrenal cancer, bladder cancer, esophageal cancer, head or neck cancer, liver cancer, parathyroid cancer, penis cancer, small intestine cancer, thyroid cancer, urethral cancer, cervical cancer, endometrial cancer, fallopian tube cancer, renal pelvis cancer, vaginal cancer, vulva cancer, chronic or acute leukemia, colon cancer, melanoma, hematological malignancy, Hodgkin’s lymphoma, lung cancer, lymphocytic lymphoma, central nervous system (CNS) tumor, ovarian cancer, pancreatic cancer, pituitary adenoma, prostatic cancer, soft tissue sarcoma, gastric cancer and uterine cancer. More preferably, the cancer is selected from one or more of lung cancer, colon cancer, ovarian cancer, prostatic cancer, and liver cancer.

The compound of the present application or pharmaceutical composition thereof can be used alone or, if necessary, in combination with other active compounds. Compounds of formulae I, II-1 to II-6, III, IV-1 to IV-6, V-1 to V-6, VI-1 to VI-5, VII-1 to VII-6, VIII-1 to VIII-6 are considered effective when used in combination with: alkylating agents, angiogenic inhibitors, antibodies, metabolic antagonists, anti-mitotic agents, anti-proliferative agents, antiviral agents, aurora kinase inhibitors, other programmed cell death promoters (such as Bcl-xL, Bcl-w, and Bfl-1) inhibitors, activators of death receptor pathways, Bcr-Abl kinase inhibitors, BiTE (Bi-specific T-cell engager) antibodies, antibody drug conjugates, biological reaction modifiers, cyclin dependent kinase inhibitors, cell cycle inhibitors, cyclooxygenase-2 inhibitors, DVD, leukemia virus oncogene homolog (ErbB2) receptor inhibitors, growth factor inhibitors, heat shock protein (HSP)-90 inhibitors, histone deacetylase (HDAC) inhibitors, hormone therapy, immunity, inhibitors of programmed cell death proteins (IAP), insert antibiotics, kinase inhibitors, kinesin inhibitors, Jak2 inhibitors, rapamycin inhibitors targeting warm blooded animals, microRNAs, mitogen-activated extracellular signal-regulated kinase inhibitors, multivalent binding proteins, nonsteroidal anti-inflammatory drugs (NSAID), poly ADP (adenosine diphosphate) ribopolymerase (PARP) inhibitors, platinum chemotherapy, polo-like kinase (Plk) inhibitors, phosphoinositol-3 kinase (PI3K) inhibitors, proteasome inhibitors, purine analogs, pyrimidine analogs, receptor tyrosine kinase inhibitors, ergot alkaloids tartrate (etinoids)/deltoids plant alkaloids, small inhibitory ribonucleic acids (siRNA), local isomerase inhibitors, ubiquitin ligase inhibitors, etc., and a combination of one or more of these agents.

In some embodiments, there are two ways for combined administration: 1) the compound or pharmaceutical composition described in the present application is separately prepared with other active drugs that can be used in combination, and the two dosage forms can be the same or different. When used, they can be used sequentially or simultaneously; when used sequentially, the first drug has not yet lost its effective effect in the body when the second drug is administered; 2) the compound or pharmaceutical composition of the present application and other active drugs that can be used in combination are made into a single formulation, so that they are administered simultaneously.

The compounds of formulae I, II-1 to II-6, III, IV-1 to IV-6, V-1 to V-6, VI-1 to VI-5, VII-1 to VII-6, VIII-1 to VIII-6, or pharmaceutically acceptable salts thereof may be administered in combination with other therapeutic agents, including other therapeutic agents that can be used for the treatment of pulmonary arterial hypertension (PHA). These therapeutic agents include vasodilators such as Floran™, Adcirca™, or Volibris™, etc.

The "effective amount or "effective dose" of the compound or pharmaceutically composition of the present application refers to an amount that is effective for the treatment or reduction of the severity of one or more of the conditions referred to in the present application. According to the method of the present application, the compound and composition can be effectively used to treat or alleviate the severity of diseases at any administered dose and by any administration route. A necessary accurate amount will vary depending on the patient’s condition, including race, age, general conditions of the patient, severity of infection, specific factors, administration mode, and so on. The compound or composition can be administered in combination with one or more other therapeutic agents, as discussed in the present application.

**General** synthesis methods

Unless specified otherwise, the compounds of the present application can generally be prepared by the methods described herein, where the substituents are defined as in formulae I, II-1 to II-6, III, IV-1 to IV-6, V-1 to V-6, VI-1 to VI-5, VII-1 to VII-6, VIII-1 to VIII-6. The following reaction scheme and examples are used to further illustrate the present application.

Technicians in the field will recognize that the chemical reactions described in the present application can be used to appropriately prepare many other compounds of the present application, and other methods for preparing compounds of the present application are considered within the scope of the present application. For example, the synthesis of compounds according to the present application which are unexemplified herein can be successfully accomplished by skilled persons in the art through modifications to the method, such as appropriate protection of interfering groups, use of other known reagents in addition to those described in the present application, or some conventional modifications to the reaction conditions. In addition, the reactions disclosed in the present application or known in the art can also adapted to the preparation of other compounds of the present application.

The structure of the compound of the present application is determined by nuclear magnetic resonance (NMR) and liquid chromatography-mass spectrometry (LC-MS). NMR was detected using Bruker AVANCE-400 and Bruker AVANCE-500 nuclear magnetic instruments, with solvents including deuterated dimethyl sulfoxide (DMSO-$d_6$), deuterated acetone ($CD_3COCD_3$), deuterated chloroform ($CDCl_3$), and deuterated methanol ($CD_3OD$). The internal standard used was tetramethyl silane (TMS), and the chemical shift was measured in parts per million (ppm) units. The liquid chromatography-mass spectrometry (LC-MS) was detected using the Agilent 1260 mass spectrometer. The HPLC determination was performed using an Agilent 1100 high-pressure chromatograph (Microsorb 5 micron C18 5 100 × 3.0mm column). Thin layer chromatography was conducted with Qingdao GF254 silica gel plate, using 0.15-0.20 mm plate for TLC and 0.4 mm-0.5 mm plate for preparative thin layer chromatography. The column chromatography is generally conducted using Qingdao silica gel of 200-300 mesh as the carrier.

Starting materials used in the examples of the present application either are known and commercially available, or have been, or can be, synthesized according to literature in the art. Unless otherwise specified, all reactions of the present application are carried out under the protection of dry inert gases (such as nitrogen or argon), with continuous magnetic stirring, and the reaction temperature is provided in Celsius degrees. The following synthesis schemes describe the steps of the processes for preparing the compounds disclosed in the present application, wherein Hal is halogen (including F, Cl, Br, I), Cy represents a structure of various cyclic system (including, but not limited to, 3-6 membered saturated or partially unsaturated carbocyclyl, 3-6 membered saturated or partially unsaturated heterocyclyl, 6-10 membered aryl, or 5-10 membered heteroaryl), Pg represents a protecting group; unless otherwise specified, A, $R^1$, $R^2$, $R^3$, $R^4$, $X^1$, $X^2$, $X^3$, $X^4$, $Y^1$, $Y^2$, $Y^3$, $Y^4$, $Y^5$, $L^1$, $L^2$, $L^5$, $L^6$, M, n, m, and p respectively have the meanings described in the present application.

Synthesis Scheme (1):

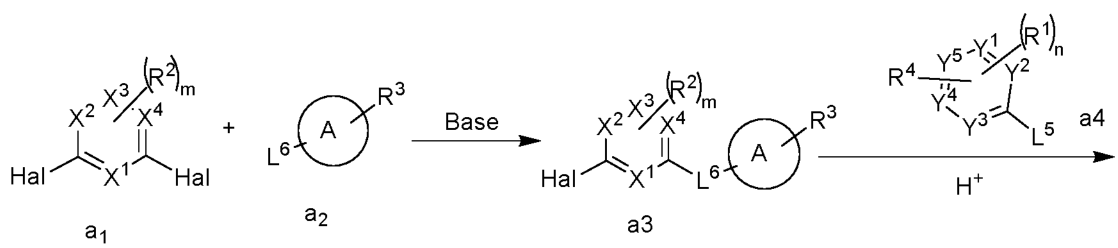

a1 a2 a3

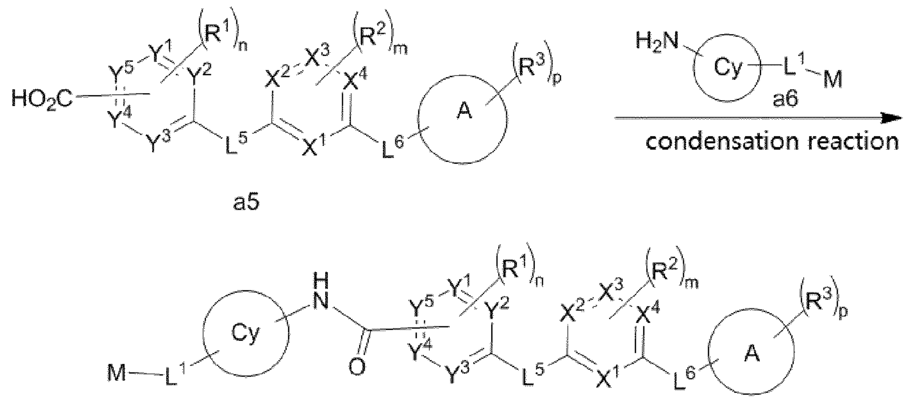


a5

a7

Compound of formula a7 can be prepared by the general method described in scheme (1), with specific steps described in the following examples in which Hal is halogen, preferably chlorine and bromine. Compound of formula a1 and Compound of formula a2 or its salt undergo a nucleophilic substitution reaction under base condition (such as DIPEA, TEA, sodium bicarbonate, sodium carbonate, etc.) in an appropriate solvent (such as DMF, ethanol, methanol) to obtain Compound of formula a3. Compound of formula a3 and Compound of formula a4 undergo a nucleophilic addition reaction in an appropriate solvent (such as isopropanol, n-butanol, 2-butanol, etc.) under acid catalytic conditions (such as HCl, TsOH, etc.) to obtain Compound of formula a5. Compound of formula a5 and Compound of formula a6 undergo a condensation reaction in an appropriate solvent (such as DMF, THF, etc.) under the action of a condensation agent(such as HATU, EDCI, HOBt, etc.) to obtain the product Compound of formula a7.

Intermediate a6 synthesis scheme (A):

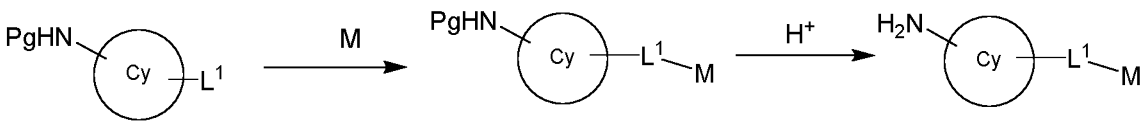


a6-1 a6-2 a6

The intermediate compound of formula a6 can be prepared by the general method described in scheme (A), with specific steps described in the following examples. A covalent warhead M is introduced onto compound of formula a6-1 which is commercially available under condensation condition (such as acyl chloride, or HATU, EDCI, HOBt, etc.) in an appropriate solvent (such as DCM, THF, DMF, etc.) to obtain Compound of formula a6-2. Compound of formula a6-2 is deprotected under acidic condition (such as HCl, etc.) to obtain the intermediate compound of formula a6.

Synthesis Scheme (2):

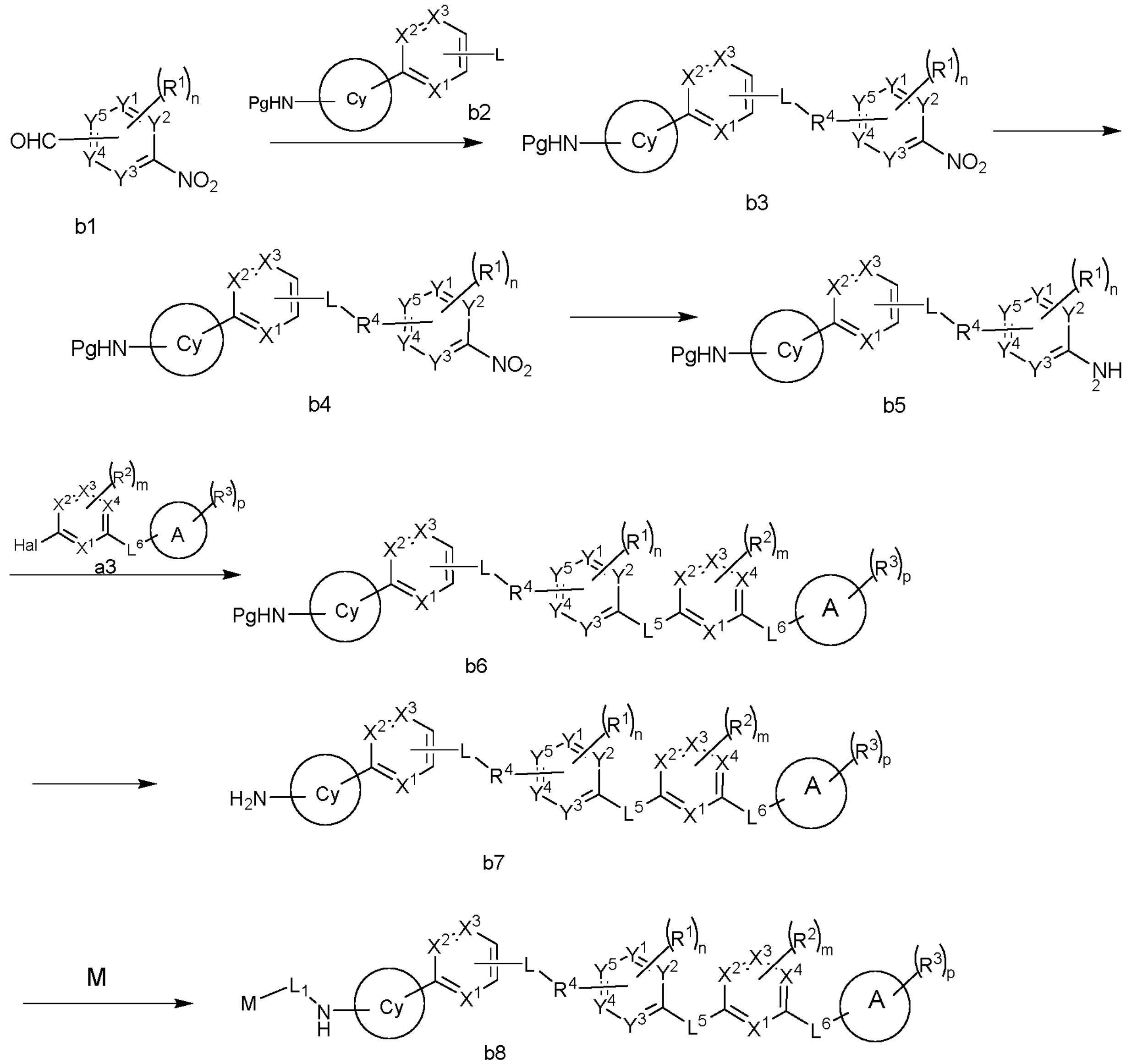


b1 b2 b3 b4 b5 a3 b6 b7 b8

Compound of formula b8 can be prepared by the general method described in scheme (2), with specific steps described in the following examples. A commercially available compound of formula b1 undergoes a reduction amination reaction with Compound of formula b2 to produce Compound of formula b3. Compound of formula b3 is reduced under Fe powder reduction condition to produce reduced product, Compound of formula b5. Compounds of formula b5 and formula a3 are reacted under acidic condition (such as HCl/Dioxane, etc.) in an appropriate solvent (such as THF, isopropanol, butanol, 2-butanol, etc.) and at an appropriate temperature, to produce Compound of formula b6. The Compound of formula b6 is deprotected to produce Compound of formula b7. A covalent warhead M is introduced under condensation condition (such as acyl chloride, or HATU, EDCI, HOBt, etc.) in an appropriate solvent (such as DCM, THF, DMF, etc.) onto the Compound of formula b7 to obtain Compound of formula b8;

Intermediate b2 synthesis scheme (B):

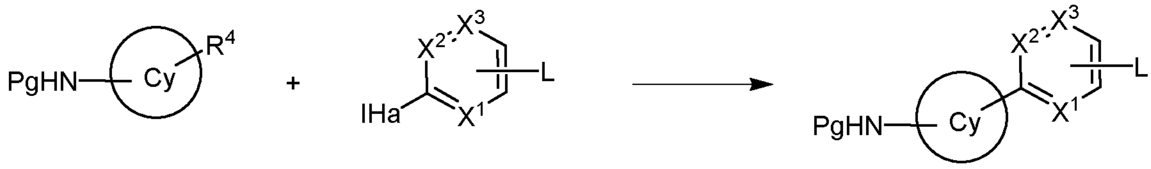

b2-1 b2-2 b2

Compound of formula b2 can be prepared by the general method described in scheme (B), with specific steps described in the following examples. Commercially available compounds of formula b2-1 and formula b2-2 undergo coupling reaction under catalytic condition such as ($Pd(dppf)Cl_2/Na_2CO_3$, or LED blue light irradiation, catalysts $Ir[dF(CF_3)ppy]_2(dtbbpy)PF_6$ and [4,4'-bis(1,1-dimethylethyl)-2,2'-bipyridine]nickel dichloride (II)), in an appropriate solvent (such as 1,4-dioxane, DMF, polyethylene glycol dimethyl ether, etc.) to obtain the intermediate compound of formula b2.

The following abbreviations are used throughout the present application:

TLC: thin layer chromatography; HCl: hydrochloric acid, hydrogen chloride; $K_2CO_3$: potassium carbonate;
LCMS: gas chromatography-mass spectrometry; Pd / C: Palladium on carbon; $K_3PO_4$:tripotassium phosphate;
ACN: acetonitrile; $Pd(OAc)_2$:palladium acetate; $NaHCO_3$:sodium bicarbonate;
FCC: flash column chromatography; $H_2$: hydrogen; $Cs_2CO_3$:cesium carbonate;
N: moles per liter; MeOH: methanol; $NaNH_2$:sodium amide;
M: moles per liter; DCM: Dichloro methane; NaH:sodium hydride;
mmol: millimolar; EA: ethyl acetate; $NaBH(AcO)_3$:sodium triacetoxyborohydride;
mL: milliliter; DIPEA: diisopropylethylamine;Xantphos:4,5-bis(diphenylphosphino)-9,9-dimethyloxanthracene;
μL: microliter; TEA: triethylamine; HOBt:1-hydroxybenzotriazole;
g: gram; DMF: *N,N*-dimethylformamide; EDCI: 1-ethyl-3(3-dimethypropyl) carbodiimide;
mg: milligram; DMSO: dimethyl sulfoxide; HCl/Dioxane: hydrogen chloride dioxane solution;
h: hour; DMAc: dimethyl acetamide; DMAP:4-dimethylaminopyridine;
min: minute; $(Boc)_2O$: di-*tert*-butyl dicarbonate;HATU:2-(7-azobenzotriazole)-*N,N,N;N'*-tetratnethylurea hexafluorophosphate;
°C: Celsius degrees; Fmoc-Cl: fluorene methoxy carbonyl chloride; LDA:lithium diisopropylamide;
psi: pascal;$Na_2SO_4$: sodium sulfate; DMSO-$d_6$:deuterated dimethyl sulfoxide;
HPLC: high performance liquid phase; ee%: percent enantiomeric excess

## BRIEF DESCRIPTION OF DRAWINGS

The above and/or additional aspects and advantages of the present application will become apparent and easy to understand from the description of examples in conjunction with the following drawings, in which:

Figure 1 shows the morphological differences between normal organoids and diffuse gastric cancer organoids. Normal organoids have a manifestation/morphology of hollow spheroid, while diffuse gastric cancer organoids have a solid, irregular grape-shaped magnification/morphology, representing one of important indicators for drug evaluation.

Figure 2 shows the effect of treatment with 500 nM compounds on the morphology of diffuse gastric cancer organoid.

Figure 3 shows the effect of treatment with 500 nM compounds on FAK signaling pathway.

Figure 4 shows the inhibitory effect of some compounds of the present application on FAK and YAP in a human diffuse gastric cancer tumor cell line SNU668 model.

Figure 5 shows the inhibitory effect of some compounds of the present application on FAK and YAP in a human diffuse gastric cancer tumor cell line SNU668 model.

Figure 6 shows the inhibitory effect of some compounds of the present application on FAK and YAP in a human diffuse gastric cancer tumor cell line SNU668 model.

## Specific Embodiments

Examples of the present application are described in detail as follows, some of which are exemplified in the accompanying drawings. The examples described below with reference to the accompanying drawings are exemplary and intended to explain the present application, cannot be understood as limitations to the present application.

### Example 1 N (1-acryloylpiperidin-4-yl)-3-methoxy-4-((4-(((3-(N methylmethanesulfonamido)pyrazin-2-yl)methyl)amin o)-5-(trifluoromethyl)pyrimidin-2-yl)amino)benzamide (1)

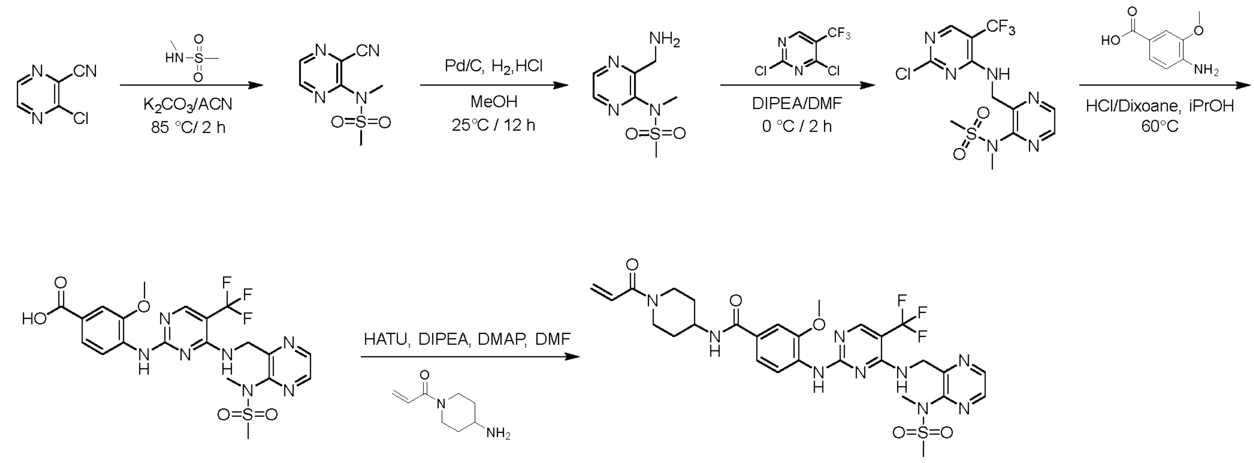


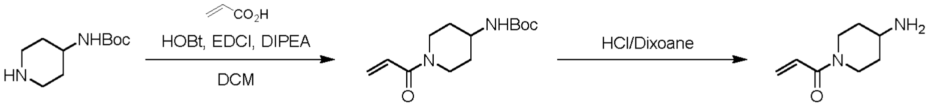


**Step 1: *N*-(3-cyanopyrazin-2-yl)-*N*-methyl methanesulfonamide:**

A solution of 3-chloropyrazin-2-nitrile (8.40 g, 60.2 mmol), *N*-methyl methanesulfonamide (8.00 g, 73.3 mmol) and $K_2CO_3$ (9.00 g, 65.1 mmol) in acetonitrile (100 mL) was stirred at 85°C for 12 hours. Upon completion of the reaction indicated by TLC (petroleum ether/ethyl acetate=1/1), the reaction solution was suction filtered and the filter cake was wash with ACN (50 mL×3). The filtrate was concentrated to obtain a crude product, which was purified by flash chromatography column (FCC, petroleum ether/ethyl acetate=3/1) to obtain N-(3-cyanopyrazin-2-yl)-N-methyl methanesulfonamide (13.3g, a crude product). MS M/Z (ESI): 213.0 $[M+H]^+$.

**Step 2: *N*-(3-(aminomethyl)pyrazin-2-yl)-*N*-methyl methanesulfonamide:**

To a mixed solution of *N*-(3-cyanopyrazin-2-yl)-*N*-methyl methanesulfonamide (13.3 g) and HCl (1 M, 2.4 mL) in MeOH (150 mL) was added 10% Pd/C (5.50 g) at room temperature. The atmosphere was replaced by Hz for three times, and the reaction solution was stirred under $H_2$ atmosphere (15psi) for 12 hours. Upon completion of the reaction indicated by LCMS, the reaction solution was suction filtered through diatomaceous earth and the filter cake was wash with MeOH (50 mL). The filtrate was concentrated to obtain a crude product, which is slurried in DCM (100 mL) and then suction filtered. The filter cake was dried to obtain N-(3-(aminomethyl)pyrazin-2-yl)-N-methyl methanesulfonamide (HCl, 5.26 g, 34.6%). MS M/Z (ESI): 217.0 $[M+H]^+$.

**Step 3: N-(3-((2-chloro-5-(trifluoromethyl)pyrimidin-4-yl)amino)methyl)pyrazin-2-yl)-N-methylmethanesulfonami de:**

A solution of N-(3-(aminomethyl)pyrazin-2-yl)-N-methylmethanesulfonamide (hydrochloride, 5.26 g, 20.83 mmol), 2,4-dichloro-5-trifluoromethyl pyrimidine (5.73 g, 20.84 mmol), and DIPEA (6.88 mL, 41.7 mmol) in DMF (60 mL) was stirred at 0°C for 2 hours. Upon completion of the reaction indicated by TLC (petroleum ether/ethyl acetate=6/1), the reaction mixture was poured into water (150 mL) and extracted with ethyl acetate (100mL×2). The combined organic

phases were washed with saturated salt water (100mL×3), dried over anhydrous $Na_2SO_4$, filtered, and rotary evaporated to obtain a crude product. The crude product was purified through ISCO (petroleum ether/ethyl acetate=10/1) to obtain *N*-(3-((2-chloro-5-(trifluoromethyl)pyrimidin-4-yl)amino)methyl)pyrazin-2-yl)-N-methylmethanesulfonamide (5.88 g). MS M/Z (ESI):397.1 $[M+H]^+$.

**Step 4: 3-methoxy-4-((4-(((3-(N methylmethanesulfonamido)pyrazin-2-yl)methyl)amino)-5-(trifluoromethyl)pyrimi din-2-yl)amino)benzoic acid:**

In isopropanol (15 mL) were dissolved *N*-(3-((2-chloro-5-(trifluoromethyl)pyrimidin-4-yl)amino)methyl)pyrazin-2-yl)-*N*-methylmethanesulfonamide (150 mg, 0.38 mmol) and 4-amino-3-methoxybenzoic acid (66.5 mg, 0.40 mmol), added with a 4N hydrochloric acid in dioxane (0.12 mL, 0.48 mmol), and allowed to react at 65°C for 4 hours. Upon completion of the reaction, the system was suction filtered to obtain 3-methoxy-4-((4-(((3-(*N*-methylmethanesulfonatnido)pyrazin-2-yl)methyl)atnino)-5-(trifluoromethyl)pyrimidin-2 -yl)amino)benzoic acid (150 mg), MS m/z (ESI): 528.1 $[M+H]^+$.

Step **5: *t*-butyl (1-acryloylpiperidin-4-yl) carbamate:**

In DCM (20 mL) were dissolved the compound *t*-butyl (piperidin-4-yl) carbamate (1.0 mg, 5.0 mmol), acrylic acid (1.8 g, 25.0 mmol), HOBt (1.35 g, 25.0 mmol), and EDCI (4.8 g, 25 mmol) at 25°C, cooled to 0°C, added slowly with DIPEA (3.1 g, 25.0 mmol) dropwise, and then warmed back to room temperature for 2 hours. Upon completion of the reaction indicated by LCMS, the reaction was quenched with water (10 mL) and extracted with EA (20 mL*2). The combined organic phases were washed with water, dried and concentrated. The residue is purified by column chromatography (petroleum ether/ethyl acetate=0-70%) to obtain *t*-butyl (1-acryloylpiperidin-4-yl) carbamate (410 mg).

**Step 6: 1-(4-aminopiperidin-1-yl)prop-2-en-1-one:**

In methanol (11 mL) were dissolved *t*-butyl (1-acetylpiperidin-4-yl) carbamate (410.0 mg, 1.6 mmol) at 0°C and added with a HCl/Dioxane (4 M, 11.0 mL) solution. The reaction was slowly warmed to 25°C and stirred for 2 hours. Upon completion of the reaction indicated by LCMS, the reaction solution was concentrated to obtain 1-(4-aminopiperidin-1-yl)prop-2-en-1-one (310.0 mg, a crude product). The crude product is used directly for the next step without purification.

**Step 7: N (1-acryloylpiperidin-4-yl)-3-methoxy-4-((4-(((3-(N methylmethanesulfonylamido)pyrazin-2-yl)methyl)am ino)-5-(trifluoromethyl)pyrimidin-2-yl)amino)benzamide:**

To DMF (6 mL) were added compound 3-methoxy-4-((4-(((3-(N methylmethanesulfonatnido)pyrazin-2-yl)methyl)atnino)-5-(trifluoromethyl)pyrimidin-2 -yl)amino)benzoic acid (20.0 mg, 0.038 mmol), 1-(4-atninopiperidin-1-yl)prop-2-en-1-one (7.0 mg, 0.046 mmol), HATU (21.1 mg, 0.057 mmol), DMAP (1.01 mg, 0.004 mmol), and DIPEA (20 μL, 0.114 mmol). The mixture was allowed to react at room temperature for 2 hours. Upon completion of the reaction indicated by LCMS, the reaction was quenched with water (10 mL), extracted with EA (20 mL×2). The combined organic phases were washed with water, dried and concentrated. The residue is purified by column chromatography to obtain *N*-(1-acryloylpiperidin-4-yl)-3-methoxy-4-((4-(((3-(N-methylmethanesulfonylatnido)pyrazin-2-yl)methyl)amino)-5-(trifluoromethyl)pyrimidin-2-yl)amino)benzamide (23.2 mg).

$^1H$ NMR (400 MHz, DMSO-$d_6$) δ 8.66 (d, J = 2.5 Hz, 1H), 8.55 (d, J = 2.5 Hz, 1H), 8.28 (s, 1H), 8.12 (d, J = 7.8 Hz, 1H), 8.08 (s, 1H), 7.99 (d, J = 8.4 Hz, 1H), 7.55 (t, J = 5.4 Hz, 1H), 7.43 (d, J = 1.9 Hz, 1H), 7.33 (dd, J = 8.4, 1.9 Hz, 1H), 6.85 (dd, J = 16.7, 10.5 Hz, 1H), 6.10 (dd, J = 16.7, 2.5 Hz, 1H), 5.68 (dd, J = 10.4, 2.5 Hz, 1H), 4.93 (d, J = 5.3 Hz, 2H), 4.44 - 4.30 (m, 4H), 4.10 - 4.01 (m, 3H), 3.88 (s, 3H), 3.23 (s, 3H), 3.19 (s, 3H), 2.78 (t, J = 12.5 Hz, 1H), 1.85 (t, J = 14.7 Hz, 3H), 1.48 - 1.38 (m, 3H). MS m/z (ESI): 664.4 $[M+H]^+$.

**Example 2**

***Preparation** of **N**-(1-acryloylpyrrolidin-3-yl)-3-methoxy-4-((4-(((3-(**N**-methylmethanesulfonylamido)pyrazin-2-yl)methyl)a mino)-5-(trifluoromethyl)pyrimidin-2-yl)amino)benzamide (2):*

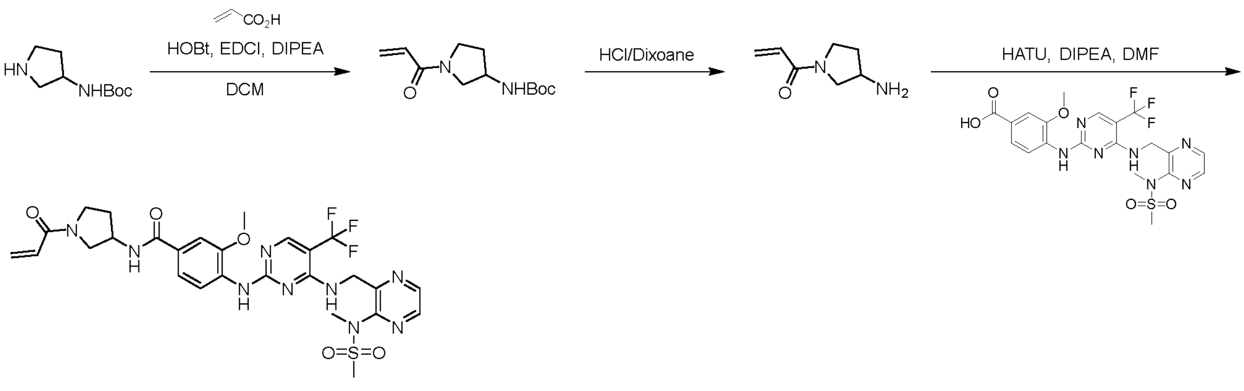


**Step 1: *t*-butyl (1-acryloylpyrrolidin-3-yl) carbamate:**

In DCM (10 mL) were dissolve the compound *t*-butyl (1-acryloylpyrioline-3-yl) carbamate (500.0 mg, 2.68 mmol), acrylic acid (966.4 mg, 13.4 mmol), HOBt (2.05 g, 13.4 mmol), and EDCI (2.57 g, 13.4 mmol) at 25°C, cooled to 0°C, added slowly with DIPEA (2.3 mL, 13.4 mmol) dropwise, and then warmed to room temperature for 2 hours. Upon completion of the reaction indicated by LCMS, the reaction was quench with water (10 mL) and extracted with EA (20 mL×2). The combined organic phases were washed with water, dried and concentrated. The residue was purified by ISCO column chromatography to obtain *t*-butyl (1-acryloylpyrrolidin-3-yl) carbamate (310.0 mg).

**Step 2: 1-(3-aminopyrrolidin-1-yl)prop-2-en-1-one:**

In methanol (8 mL) was dissolved *t*-butyl (1-acylloylpyrrolidin-3-yl) carbamate (310.0 mg, 1.29 mmol) at 0°C and added HCl/Dioxane (4M, 8mL). The reaction was allowed to warm slowly to 25°C and stirred for 2 hours. Upon completion of the reaction indicated by LCMS, the reaction solution was concentrated to obtain 1-(3-aminopyrrolidin-1-yl)prop-2-en-1-one (280.4 mg, a crude product). The crude product is used directly for the next step without purification.

**Step 3: *N*-(1-acryloylpyrrolidin-3-yl)-3-methoxy-4-((4-(((3-(N methylmethanesulfonylamido)pyrazin-2-yl)methyl)amino)-5-(trifluoromethyl)pyrimidin-2-yl)amino)benzamide:**

To DMF (10 mL) were added compound 3-methoxy-4-((4-(((3-(*N*-methylmethanesulfonamido)pyrazin-2-yl)methyl)atnino)-5-(trifluoromethyl)pyrimidin-2 -yl)amino)benzoic acid (60.0 mg, 0.11 mmol), 1-(3-aminopyrrolidin-1-yl)prop-2-en-1-one (19.6 mg, 0.14 mmol), HATU (63.0 mg, 0.17 mmol), DMAP (1.01 mg, 0.011 mmol), and DIPEA (56 μL, 0.33 mmol). The mixture was allowed to react at room temperature for 2 hours. Upon completion of the reaction indicated by LCMS, the reaction was quenched with water (10 mL) and extracted with EA (20 mL×2). The combined organic phases were washed with water, dried and concentrated. The residue was purified by column chromatography to obtain *N*-(1-acryloylpyrrolidin-3-yl)-3-methoxy-4-((4-(((3-(*N*-methylmethanesulfonylamido)pyrazin-2-yl)methyl)amino) -5-(trifluoromethyl)pyrimidin-2-yl)amino)benzamide (43.1 mg).

$^1H$ NMR (400 MHz, DMSO-$d_6$) δ 8.66 (d, J = 2.5 Hz, 1H), 8.55 (d, J = 2.5 Hz, 1H), 8.29 (s, 1H), 8.16 (d, J = 7.3 Hz, 1H), 8.09 (s, 1H), 8.01 (s, 1H), 7.55 (s, 1H), 7.43 (d, J = 1.9 Hz, 1H), 7.33 (d, J = 8.4 Hz, 1H), 6.86 - 6.68 (m, 1H), 6.13 - 6.04 (m, 1H), 5.67 (t, J = 8.6 Hz, 1H), 4.93 (d, J = 5.2 Hz, 2H), 4.39 (d, J = 35.7 Hz, 3H), 4.01 - 3.93 (m, 1H), 3.89 (s, 3H), 3.81 - 3.73 (m, 2H), 3.24 (s, 3H), 3.19 (s, 3H), 1.96 - 1.89 (m, 1H), 1.82 - 1.75 (m, 1H), 1.48 - 1.38 (m, 1H). MS m/z (ESI): 650.1 $[M+H]^+$.

**Example 3**

**Preparation of N (1-acryloyl piperidin-3-yl)-3-methoxy-4-((4-((3-(M-methylmethanesulfonylamido)pyrazin-2-yl)methyl)amino)-5-(trifluo romethyl)pyrimidin-2-yl)amino)benzamide (3)**

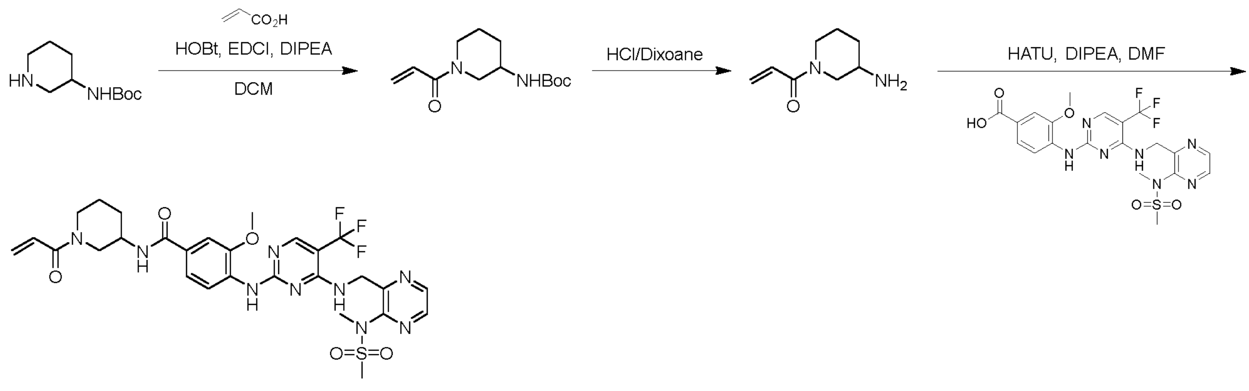


Step **1: *t*-butyl (1-acryloyl piperidin-3-yl) carbamate:**

In DCM (10 mL) were dissolved compound *t*-butyl (piperidin-3-yl) carbamate (500.0 mg, 25.0 mmol), acrylic acid (0.9 mL, 12.5 mmol), HOBt (1.91 g, 12.5 mmol), and EDCI (2.4 g, 12.5 mmol) at 25°C. The mixture was cooled to 0°C and added slowly with DIPEA (2.1 mL, 12.5 mmol) dropwise, and then warmed to room temperature for 2 hours. Upon completion of the reaction indicated by LCMS, the reaction was quenched with water (10 mL), and extracted with EA (20 mL×2). The combined organic phases were washed with water, dried and concentrated. The residue was purified by ISCO column chromatography to obtain *t*-butyl (1-acryloyl piperidin-3-yl) carbamate (150.3 mg).

Step **2: 1-(3-aminopiperidin-1-yl)prop-2-en-1-one:**

In HCl/Dioxane (4 M, 5mL) in methanol (5mL) was dissolved *t*-butyl (1-acylloyl piperidin-3-yl) carbamate (150.3 mg, 0.59 mmol) at 0°C, then warmed slowly to 25°C and stirred for 2 hours. Upon completion of the reaction indicated by LCMS, the reaction solution was concentrated to obtain 1-(3-atninopiperidin-1-yl)prop-2-en-1-one (100.1 mg, a crude product). The crude product is used directly for the next step without purification.

Step **3: N (1-acryloyl piperidin-3-yl)-3-methoxy-4-((4-((3-(7V-methylmethanesulfonylamido)pyrazin-2-yl)methyl)amino)-5-(trifluo romethyl)pyrimidin-2-yl)amino)benzamide:**

To DMF (10 mL) were added with compound 3-methoxy-4-((4-(((3-(*N*-methylmethanesulfonamido)pyrazin-2-yl)methyl)amino)-5-(trifluoromethyl)pyrimidin-2 -yl)amino)benzoic acid (60.1 mg, 0.11 mmol), 1-(3-aminopiperidin-1-yl)prop-2-en-1-one (21.0 mg, 0.14 mmol), HATU (63.0 mg, 0.17 mmol), DMAP (1.01 mg, 0.009 mmol), and DIPEA (56 μL, 0.33 mmol). The mixture was allowed to react at room temperature for 2 hours, and Upon completion of the reaction indicated by LCMS, quenched with water (10 mL) and extracted with EA (20 mL×2). The combined organic phases were washed with water, dried and concentrated. The residue was purified by column chromatography to obtain N (1-acryloyl piperidin-3-yl)-3-methoxy-4-((4-((3-(N methylmethanesulfonylatnido)pyrazin-2-yl)methyl)atnino)-5-(trifluoromethyl)pyrimidin-2-yl)amino)benzamide (46.5 mg).

$^1H$ NMR (500 MHz, DMSO-$d_6$) δ 8.66 (d, J = 2.4 Hz, 1H), 8.55 (d, J = 2.5 Hz, 1H), 8.40 (dd, J = 20.9, 6.6 Hz, 1H), 8.28 (s, 1H), 8.10 (s, 1H), 8.03 - 7.97 (m, 1H), 7.54 (t, J = 5.8 Hz, 1H), 7.44 (t, J = 2.0 Hz, 1H), 7.35 (dd, J = 8.4, 1.8 Hz, 1H), 6.59 (ddd, J = 24.7, 16.8, 10.3 Hz, 1H), 6.14 (ddd, J = 16.8, 7.0, 2.4 Hz, 1H), 5.67 (td, J = 10.5, 2.4 Hz, 1H), 4.93 (d, J = 5.2 Hz, 2H), 4.47 (dq, J = 39.9, 6.1 Hz, 1H), 3.76 - 3.54 (m, 2H), 3.50 - 3.39 (m, 2H), 3.23 (s, 3H), 3.19 (s, 3H), 2.24 - 2.07 (m, 1H), 2.04 - 1.89 (m, 1H). MS m/z (ESI): 664.1 $[M+H]^+$.

**Example 4**

**Preparation of *N*-((1-acryloylpiperidin-4-yl)methyl)-3-methoxy-4-((4-((3-(*N*-methylmethanesulfonylamido)pyrazin-2-yl)me thyl)amino)-5-(trifluoromethyl)pyrimidin-2-yl)amino)benzamide *(4)*:**

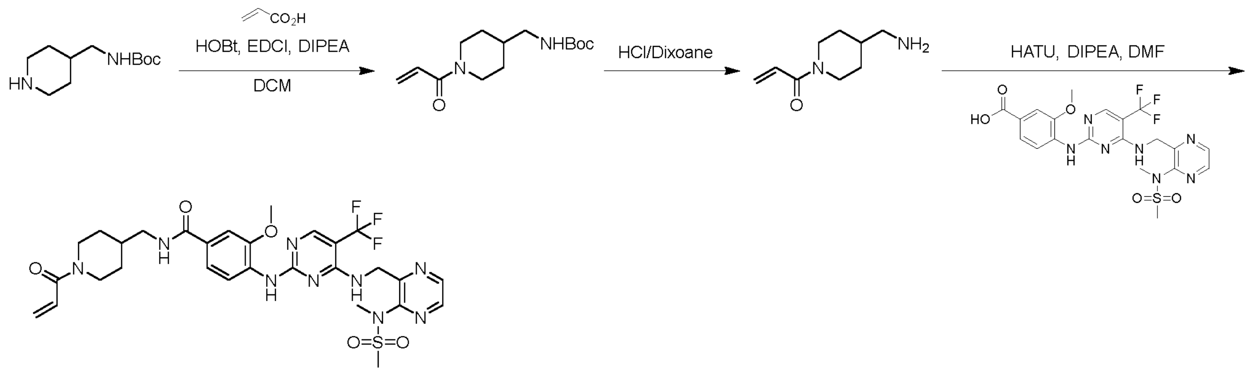


**Step 1: *t*-butyl ((1-acryloylpiperidin-4-yl)methyl)carbamate:**

In DCM (10 mL) were dissolved compound *t*-butyl (piperidin-4-ylmethyl)carbamate (500.5 mg, 2.34 mmol), acrylic acid (1.06 g, 11.68 mmol), HOBt (1.8 g, 11.68 mmol), and EDCI (2.2 g, 11.68 mmol) at 25°C. The mixture was cooled to 0°C, added slowly with DIPEA (1.9 mL, 11.68 mmol) dropwise, and then warmed to room temperature for 2 hours. Upon completion of the reaction indicated by LCMS, the reaction was quenched with water (10 mL) and extracted with EA (20 mL×2). The combined organic phases were washed with water, dried and concentrated. The residue was purified by column chromatography to obtain *t*-butyl ((1-acryloylpiperidin-4-yl)methyl)carbamate (163.2 mg).

Step **2: 1-(4-(aminomethyl)piperidin-1-yl)prop-2-en-1-one:**

In methanol (5 mL) was dissolved *t*-butyl ((1-acryloylpiperidin-4-yl)methyl)carbatnate (163.2 mg, 0.61 mmol) at 0°C and added with HCl/Dioxane (4 M, 5 mL). The mixture was warmed slowly to 25°C and stirred for 2 hours. Upon completion of the reaction indicated by LCMS, the reaction solution was concentrated to obtain 1-(4-(aminomethyl)piperidin-1-yl)prop-2-en-1-one (130.3 mg, a crude product). The crude product is used directly for the next reaction without purification.

Step **3: N ((1-acryloylpiperidin-4-yl)methyl)-3-methoxy-4-((4-((3-(N methylmethanesulfonylamido)pyrazin-2-yl)me thyl)amino)-5-(trifluoromethyl)pyrimidin-2-yl)amino)benzamide:**

To DMF (10 mL) were added compound 3-methoxy-4-((4-(((3-(A-methylmethanesulfonamido)pyrazin-2-yl)methyl)amino)-5-(trifluoromethyl)pyrimidin-2 -yl)amino)benzoic acid (60 mg, 0.11 mmol), 1-(4-(aminomethyl)piperidin-1-yl)prop-2-en-1-one (23.5 mg, 0.14 mmol), HATU (63.0 mg, 0.17 mmol), DMAP (1.01 mg, 0.011 mmol), and DIPEA (56 μL, 0.33 mmol). The mixture was allowed to react at room temperature for 2 hours. Upon completion of the reaction indicated by LCMS, the reaction was quenched with water (10 mL) and extracted with EA (20 mL×2). The combined organic phases were washed with water, dried and concentrated. The residue was purified by column chromatography to obtain *N*-(1-acryloyl piperidin-3-yl)-3-methoxy-4-((4-((3-(*N*-methylmethanesulfonylamido)pyrazin-2-yl)methyl)amino)-5-(trifluoromet hyl)pyrimidin-2-yl)amino)benzamide (32.1 mg).

$^1$H NMR (400 MHz, DMSO-$d_6$) δ 8.66 (d, J = 2.5 Hz, 1H), 8.55 (d, J = 2.5 Hz, 1H), 8.28 (d, J = 0.9 Hz, 1H), 8.10 (d, J = 7.8 Hz, 1H), 8.07 (s, 1H), 8.00 (d, J = 8.4 Hz, 1H), 7.53 (t, J = 5.3 Hz, 1H), 7.43 (d, J = 1.9 Hz, 1H), 7.34 (dd, J = 8.5, 1.8 Hz, 1H), 6.84 (dd, J = 16.7, 10.5 Hz, 1H), 6.10 (dd, J = 16.7, 2.5 Hz, 1H), 5.67 (dd, J = 10.4, 2.5 Hz, 1H), 4.93 (d, J = 5.3 Hz, 2H), 4.40 (d, J = 13.0 Hz, 1H), 4.12 - 4.00 (m, 2H), 3.88 (s, 3H), 3.23 (s, 3H), 3.18 (s, 3H), 2.79 (t, J = 12.6 Hz, 1H), 1.85 (s, 2H), 1.49 - 1.38 (m, 3H). MS m/z (ESI): 678.4 [M+H]$^+$.

**Example 5**

**Preparation of *N*-((4-acrylamidocyclohexyl)methyl)-3-methoxy-4-((4-(((3-(*N*-methylmethanesulfonylamido)pyrazin-2-yl)m ethyl)amino)-5-(trifluoromethyl)pyrimidin-2-yl)amino)benzamide (5):**

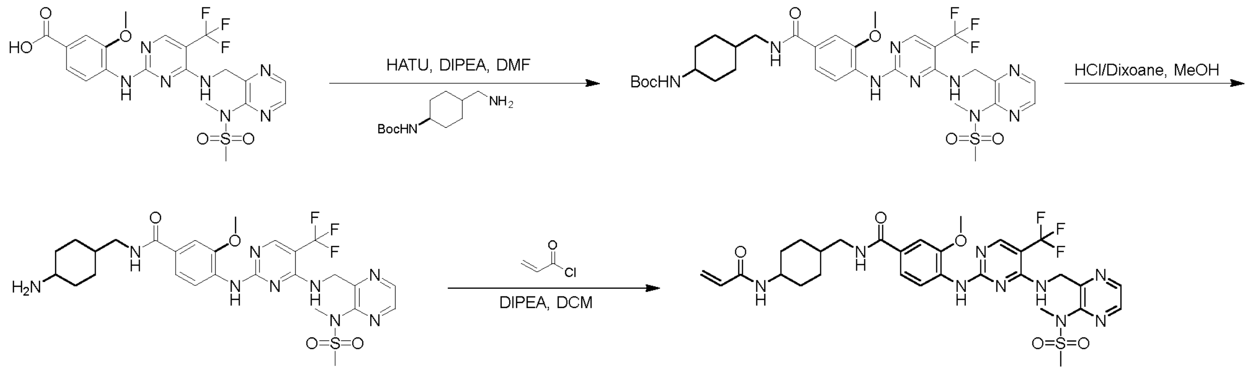


**Step 1: *t*-butyl (4-((3-methoxy-4-(((4-(((3-(*N*-methylmethanesulfonylamido)pyrazin-2-yl)methyl)amino)-5-(trifluoromethyl) pyrimidin-2-yl)amino)benzamido)methyl)cyclohexyl)carbamate:**

To DMF (10 mL) were added compound 3-methoxy-4-((4-(((3-(N-methylmethanesulfonamido)pyrazin-2-yl)methyl)amino)-5-(trifluoromethyl)pyrimidin-2 -yl)amino)benzoic acid (102.1 mg, 0.19 mmol), *t*-butyl (4-(aminomethyl)cyclohexyl)carbamate (66.3 mg, 0.29 mmol), HATU (110.3 mg, 0.29 mmol), DMAP (3 mg, 0.02 mmol), and DIPEA (0.1 mL, 0.57 mmol). The mixture was allowed to react at room temperature for 2 hours. Upon completion of the reaction indicated by LCMS, the reaction was quenched with water (10 mL) and extracted with EA (20 mL×2). The combined organic phases were washed with water, dried and concentrated. The residue was purified by column chromatography (petroleum ether/ether acetate=0-100%) to obtain *t*-butyl (4-((3-methoxy-4-(((4-(((3-(*N*-methylmethanesulfonylatnido)pyrazin-2-yl)methyl)amino)-5-(trifluoromethyl)pyri midin-2-yl) amino)benzamido)methyl)cyclohexyl)carbamate (90.3 mg). MS m/z (ESI): 738.6 [M+H]$^+$.

**Step 2: *N*-((4-aminocyclohexyl)methyl)-3-methoxy-4-((4-((3-(N-methylmethanesulfonylamido)pyrazin-2-yl)methyl) amino)-5-(trifluoromethyl)pyrimidin-2-yl)amino)benzamide:**

In methanol (3mL) was dissolved *t*-butyl (4-((3-methoxy-4-(((4-(((3-(*N*-methylmethanesulfonylamido)pyrazin-2-yl)methyl)amino)-5-(trifhioromethyl)pyri midin-2-yl)amino)benzamido)methyl)cyclohexyl)carbamate (90.3 mg, 0.12 mmol) at 0°C and added with HCl/Dioxane (4 M, 3mL). The mixture was warmed slowly to 25°C and stirred for 2 hours. Upon completion of the reaction indicated by LCMS, the reaction solution was concentrated to obtain *N*-((4-aminocyclohexyl)methyl)-3-methoxy-4-((4-((3-(*N*-methylmethanesulfonylatnido)pyrazin-2-yl)methyl)amin o)-5-(trifluoromethyl)pyrimidin-2-yl)amino)benzamide (80.6 mg, a crude product). The crude product is used directly for the next step without purification. MS m/z (ESI): 638.2 [M+H]$^+$.

**Step 2: *N*-((4-acrylamidocyclohexyl)methyl)-3-methoxy-4-((4-(((3-(N methylmethanesulfonylamido)pyrazin-2-yl)m ethyl)amino)-5-(trifluoromethyl)pyrimidin-2-yl)amino)benzamide:**

To dichloromethane (6 mL) were added *N*-((4-aminocyclohexyl)methyl)-3-methoxy-4-((4-((3-(*N*-methylmethanesulfonylamido)pyrazin-2-yl)methyl)amin o)-5-(trifluoromethyl)pyrimidin-2-yl)amino)benzamide (80.6 mg, a crude product), *N*, *N*-diisopropylethylamine (64 μL, 3.4 mmol). The mixture was added with acryloyl chloride (24 μL, 1.7 mmol) dropwise at 0°C, and then warmed to room temperature and allowed to react for 2 hours. Upon completion of the reaction indicated by TLC, the reaction was quenched with water (10 mL) and extracted with DCM (10 mL × 3). The combined organic phases were washed with water, dried and concentrated. The residue was purified by column chromatography to obtain N-((4-acrylamidocyclohexyl)methyl)-3-methoxy-4-((4-(((3-(*N*-methylmethanesulfonylamido)pyrazin-2-yl)methyl )amino)-5-(trifluoromethyl)pyrimidin-2-yl)amino)benzamide (35.1 mg).

$^1$H NMR (400 MHz, DMSO-$d_6$) δ 8.66 (d, J = 2.4 Hz, 1H), 8.55 (d, J = 2.5 Hz, 1H), 8.38 (t, J = 5.7 Hz, 1H), 8.28 (s, 1H), 8.08 (s, 1H), 7.98 (d, J = 8.6 Hz, 1H), 7.54 (s, 1H), 7.45 (d, J = 1.9 Hz, 1H), 7.33 (d, J = 8.5 Hz, 1H), 6.80 (dd, J = 16.7, 10.5 Hz, 1H), 6.07 (dd, J = 16.7, 2.5 Hz, 1H), 5.64 (dd, J = 10.4, 2.5 Hz, 1H), 4.93 (d, J = 5.1 Hz, 2H), 4.40 (d, J = 13.1 Hz, 1H), 4.04 (d, J = 12.9 Hz, 1H), 3.88 (s, 3H), 3.23 (d, J = 1.7 Hz, 3H), 3.19 (s, 3H), 3.15 (s, 1H), 3.02 (t, J = 13.7 Hz, 1H), 2.66 - 2.58 (m, 0H), 2.02 - 1.95 (m, 0H), 1.82 (s, 1H), 1.72 (d, J = 13.1 Hz, 2H), 1.10 - 0.98 (m, 2H). MS m/z (ESI): 692.2 [M+H]$^+$.

**Example 6**

**Preparation of 2-((2-((4-(((3-(1-acryloylpiperidin-3-yl)phenyl)amino)methyl)phenyl)amino)-5-(trifluoromethyl)pyrimidin-4 -yl)amino)-N-methylbenzamide (6)**

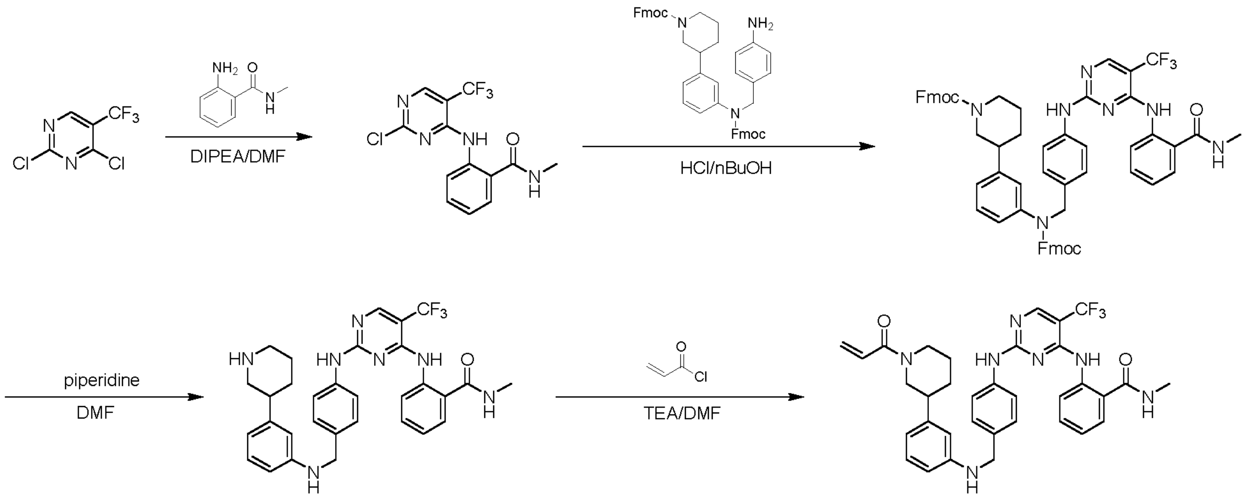


**Step 1: synthesis of 2-((2-chloro-5-(trifluoromethyl)pyrimidin-4-yl)amino)-7V-methylbenzamide**

In DMF (5 mL) was dissolved 2,4-dichloro-5-(trifluoromethyl)pyrimidine (1.6 g, 7.37 mmol), added with DIPEA (2.2 mL, 14.75 mmol) and 2-amino-N-methylbenzamide (1.0 g, 6.70 mmol) in ice bath, and stirred at 60°C for 2 hours. Upon completion of the reaction, the mixture was added with water, and extracted with EA. The combined organic phases were dried and concentrated. The residue was purified by column chromatography to obtain 189 mg 2-((2-chloro-5-(trifluoromethyl)pyrimidin-4-yl)amino)-*N*-methylbenzamide, MS m/z (ESI): 331.0 $[M+H]^+$.

**Step 2: synthesis of (9H-fluoren-9-yl)methyl 3-(3-(((((9H-fluoren-9-yl)methoxy)carbonyl)(4-(((4-((2-(methylcarbamoyl)phenyl)amino))-5-(trifluoromethy l)pyrimidin-2-yl)amino)benzyl)amino)phenyl) piperidine-1-carboxylate**

In *n*-butanol (10 mL) were dissolved (9H-fluoren-9-yl)methyl 3-(3-(((((((9H-fluoren-9-yl)methoxy)carbonyl))(4-aminobenzyl)amino)phenyl) piperidine-1-carboxylic acid (377 mg, 0.52 mmol), and 2-((2-chloro-5-(trifluoromethyl)pyrimidin-4-yl)amino)-*N*-methylbenzamide (189mg, 0.57 mmol), added with hydrochloric acid in dioxane (4N, 40 μL), and allowed to react overnight at 70°C. Upon completion of the reaction, the reaction solution was concentrated, and the residue was purified by column chromatography to obtain 500 mg (9H-fluoren-9-yl)methyl 3-(3-(((((9H-fluoren-9-yl)methoxy)carbonyl)(4-(((4-((2-(methylcarbamoyl)phenyl)amino))-5-(trifluoromethyl)pyr imidin-2-yl)amino)benzyl)amino)phenyl) piperidine-1-carboxylate, MS m/z (ESI): 1020.4 $[M+H]^+$.

**Step 3: synthesis of *N*-methyl-2-((2-((4-(((3-(piperidin-3-yl)phenyl)amino)methyl)phenyl)amino)-5-(trifluoromethyl)pyrimidin-4 -yl)amino) benzamide**

In DMF (10 mL) was dissolved (9H-fluoren-9-yl)methyl 3-(3-(((((9H-fluoren-9-yl)methoxy)carbonyl)(4-(((4-((2-(methylcarbamoyl)phenyl)amino))-5-(trifluoromethyl)pyr imidin-2-yl)amino)benzyl)amino)phenyl) piperidine-1-carboxylate (500 mg, 0.49 mmol), added with piperidine (2.0 mL) in ice bath and allowed to react at room temperature for 20 minutes. Upon completion of the reaction, the reaction was added with water, extracted with DCM. The combined organic phases were dried and concentrated. The residue was purified by column chromatography to obtain 240 mg *N*-methyl-2-((2-((4-(((3-(pipen*din-3-yl)phenyl)amino)methyl)phenyl)amino)-5-(trifluoromethyl)pyrimidin-4-yl)a mino) benzamide, MS m/z (ESI): 576.1 $[M+H]^+$.

**Step 4: synthesis of 2-((2-((4-(((3-(1-acryloylpiperidin-3-yl)phenyl)amino)methyl)phenyl)amino)-5-(trifluoromethyl)pyrimidin-4 -yl)amino)-*N*-methylbenzamide**

In DMF (6 mL) was dissolved *N*-methyl-2-((2-((4-(((3-(pipen*din-3-yl)phenyl)amino)methyl)phenyl)amino)-5-(trifluoromethyl)pyrimidin-4-yl)a mino) benzamide (120 mg, 0.21 mmol), added with triethylamine (87 μL, 0.63 mmol) in ice bath, then added with acryloyl chloride (16.96 μL, 0.21mmol) dropwise. The reaction was allowed to proceed at room temperature for 10 minutes. Upon completion of the reaction, the reaction was added with water and extracted with DCM. The combined organic phases were dried and concentrated, and the residue was purified by column chromatography to obtain 43 mg 2-((2-((4-(((3-(1-acryloylpiperidin-3-yl)phenyl)amino)methyl)phenyl)amino)-5-(trifluoromethyl)pyrimidin-4-yl)a mino)-N-methylbenzamide.

$^1$H NMR (500 MHz, DMSO-$d_6$) δ 11.34 (s, 1H), 9.79 (s, 1H), 8.71 (d, *J* = 4.7 Hz, 1H), 8.41 (s, 1H), 7.69 (d, *J* = 7.6 Hz, 1H), 7.56 (s, 2H), 7.26 (m, 3H), 7.11 (m, 1H), 6.98 (t, *J* = 7.8 Hz, 1H), 6.86 - 6.78 (m, 1H), 6.51 (s, 1H), 6.44 (d, *J* = 7.0 Hz, 2H), 6.15 - 6.01 (m, 2H), 5.66 (d, *J* = 9.9 Hz, 1H), 5.59 (d, *J* = 11.7 Hz, 1H), 4.44 (d, *J* = 11.7 Hz, 1H), 4.20 (d, *J* = 5.9 Hz, 2H), 4.07 (d, *J* = 13.5 Hz, 1H), 3.92 (d, *J* = 12.7 Hz, 1H), 3.04 (t, *J* = 13.1 Hz, 1H), 2.77 (d, *J* = 4.5 Hz, 3H), 2.65 - 2.56 (m, 1H), 1.86 (m, 1H), 1.80 - 1.58 (m, 3H). MS m/z (ESI): 630.2 [M+H]$^+$.

**Example 7**

**Preparation of *N*-((1-acryloylpiperidin-4-yl)methyl)-4-((4-((2-(dimethylphosphoryl)phenyl)amino)-5-(trifluoromethyl)pyri midin-2-yl)amino)benzamide (7)**

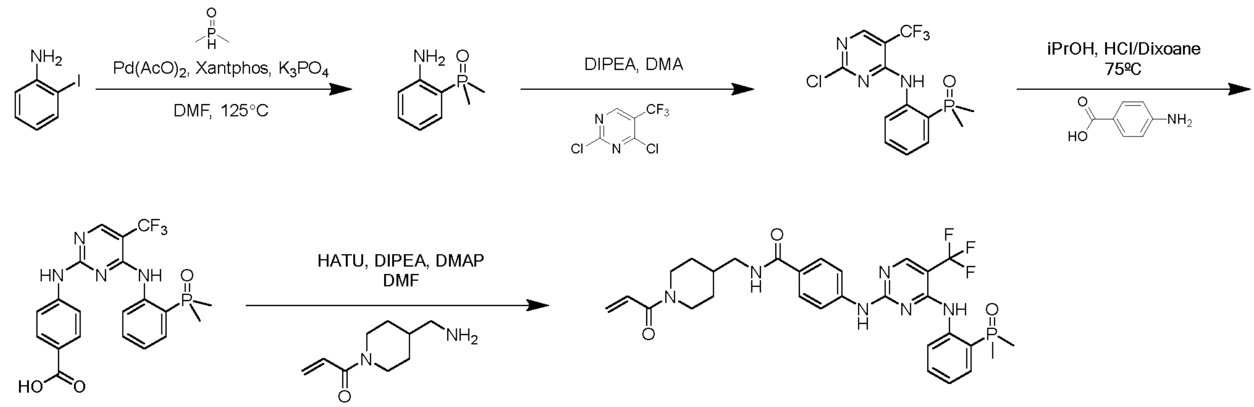


**Step** 1: synthesis of (2-aminophenyl)dimethylphosphine oxide

In DMF (100 mL) were dissolved compound 2-iodoaniline (10 g, 45.7 mmol) and dimethylphosphine oxide (4.3 g, 54.8 mmol). The reaction system was added respectively with Pd(OAc)$_2$ (1.03 g, 4.57 mol), $K_3PO_4$ (11.63 g, 54.8 mmol), and Xanthphos (2.64 g, 4.57 mol) and protected with nitrogen atmosphere. The reaction temperature was slowly heated to 125°C and stirred under reflux for 6 hours. Upon completion of the reaction indicated by TLC, the reaction was quenched with water, suction filtered through diatomaceous earth, and extracted with EA (20mL×2). The organic phases were combined and washed with saturated brine (30mL×2). After evaporation under reduced pressure, the residue was purified by ISCO column chromatography to obtain 7.1 g of (2-aminophenyl) dimethylphosphine oxide, MS m/z (ESI): 170 [M+H]$^+$.

**Step 2: synthesis of (2-((2-chloro-5-(trifluoromethyl)pyrimidin-4-yl)amino)phenyl)dimethylphosphine oxide**

In DMF (50 mL) was dissolved 2,4-dichloropyrimidine-5-trifluoromethyl (2.56 g, 11.8 mmol), added with (2-aminophenyl) dimethylphosphine oxide (2.0 g, 11.8 mmol), and then added slowly with DIPEA (2.0 mL, 11.8 mmol) in ice bath. The mixture was heated to 50°C, and allowed to react for 1 hour. Upon completion of the reaction, the reaction was quenched with water, extracted with dichloromethane, dried and concentrated. The residue was purified by column chromatography to obtain 920 mg 2-((2-chloro-5-(trifluoromethyl)pyrimidin-4-yl)amino)phenyl)dimethylphosphine oxide, MS m/z (ESI): 350 [M+H]$^+$.

**Step 3: synthesis of 4-((4-((2-(dimethylphosphoryl)phenyl)amino)-5-(trifluoromethyl)pyrimidin-2-yl)amino)benzoic acid**

In isopropanol (25 mL) were dissolved 2-((2-chloro-5-(trifluoromethyl)pyrimidin-4-yl)amino)phenyl)dimethylphosphine oxide (300 mg, 0.86 mmol) and p-aminobenzoic acid (130 mg, 0.95 mmol), added with hydrochloric acid in dioxane (4 N, 1.3 mL, 5.2 mmol), and allowed to react at 65°C for 4 hours. Upon completion of the reaction, the system was suction filtered to obtain 276 mg 4-((4-((2-(dimethylphosphoryl)phenyl)amino)-5-(trifluoromethyl)pyrimidin-2-yl)amino)benzoic acid, MS m/z (ESI): 451 $[M+H]^+$.

Step **4: *N*-((1-acryloylpiperidin-4-yl)methyl)-4-((4-((2-(dimethylphosphoryl)phenyl)amino)-5-(trifluoromethyl)pyri midin-2-yl)amino)benzamide**

To DMF (6 mL) were added with 4-((4-((2-(dimethylphosphoryl)phenyl)amino)-5-(trifluoromethyl)pyrimidin-2-yl)amino)benzoic acid (40.1 mg, 0.089 mmol), 1-(4-(aminomethyl)piperidin-1-yl)prop-2-en-1-one (22.8 mg, 0.18 mmol), HATU (51.9 mg, 0.14 mmol), DMAP (1.01 mg, 0.009 mmol), and DIPEA (46 μL, 0.27 mmol). The mixture was allowed to react at room temperature for 2 hours. Upon completion of the reaction indicated by LCMS, the reaction was quenched with water (10 mL) and extracted with EA (20 mL×2). The combined organic phases were washed with water, dried and concentrated. The residue was purified by column chromatography to obtain *N*-((1-acryloylpiperidin-4-yl)methyl)-4-((4-((2-(dimethylphosphoryl)phenyl)amino)-5-(trifluoromethyl)pyrimidin-2-yl)amino)benzamide (11.2 mg).

$^1H$ NMR (400 MHz, DMSO-$d_6$) δ 10.59 (s, 1H), 10.04 (s, 1H), 8.52 - 8.46 (m, 1H), 8.34 (t, J = 5.8 Hz, 1H), 8.19 (s, 1H), 7.74 - 7.62 (m, 4H), 7.60 (t, J = 7.8 Hz, 1H), 7.31 (t, J = 7.5 Hz, 1H), 6.80 (dd, J = 16.7, 10.5 Hz, 1H), 6.07 (dd, J = 16.7, 2.5 Hz, 1H), 5.64 (dd, J = 10.5, 2.5 Hz, 1H), 4.40 (d, J = 12.9 Hz, 1H), 4.04 (d, J = 13.7 Hz, 1H), 3.14 (s, 2H), 3.01 (t, J = 12.8 Hz, 1H), 2.62 (t, J = 12.5 Hz, 1H), 1.87 - 1.78 (m, 1H), 1.75 (s, 3H), 1.72 (s, 3H), 1.05 (t, J = 12.0 Hz, 1H). MS m/z(ESI): 601.4 $[M+H]^+$.

**Example 8**

**Preparation of (*N*-((1-acryloylpiperidin-4-yl)methyl)-4-((4-((2-(dimethylphosphoryl)phenyl)amino)-5-(trifluoromethyl)pyri midin-2-yl)amino)-3-methoxybenzamide (8)**

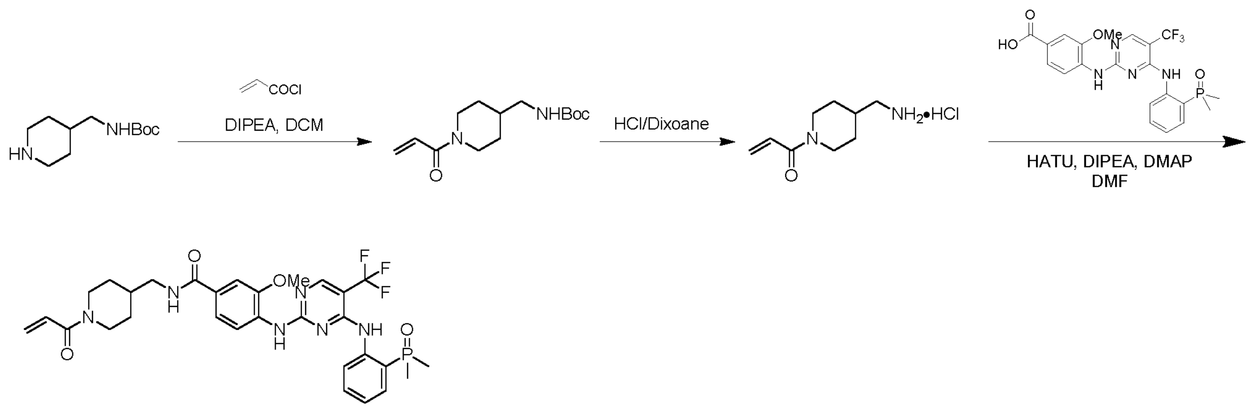


Step **1: synthesis of *t*-butyl ((1-acryloylpiperidin-4-yl)methyl)carbamate**

To dichloromethane (20 mL) were added *t*-butyl (piperidin-4-ylmethyl)carbamate (600 mg, 2.8 mmol) and *N,N*-diisopropylethylamine (1 mL, 5.6 mmol). The reaction system was added with acetyl chloride (0.3 mL, 3.4 mmol) dropwise at 0°C, then warmed to room temperature and allowed to react for 2 hours. Upon completion of the reaction indicated by LCMS, the reaction was quenched with water (10 mL), and extracted with DCM (20 mL×3). The combined organic phases were washed with water, dried and concentrated. The residue was purified by column chromatography to obtain 720 mg *t*-butyl ((1-acryloylpiperidin-4-yl)methyl)carbatnate.

**Step 2: synthesis of 1-(4-(aminomethyl)piperidin-1-yl)prop-2-en-1-one hydrochloride**

To dichloromethane (10 mL) was added with *t*-butyl ((1-acryloylpiperidin-4-yl)methyl)carbamate (360 mg, 1.34

mmol), followed by 4M hydrochloric acid in 1,4-dioxane (1 mL, 4 mmol) dropwise at 0°C. The mixture was warmed to room temperature and allowed to react for 2 hours. Upon completion of the reaction indicated by LCMS, the reaction system is filtered and the resulting solid is dried under vacuum to obtain 260 mg 1-(4-(aminomethyl)piperidin-1-yl)prop-2-en-1-one hydrochloride.

**Step 3: synthesis of *N*-((1-acryloylpiperidin-4-yl)methyl)-4-((4-((2-(dimethylphosphoryl)phenyl)amino)-5-(trifluoromethyl)pyri midin-2-yl)amino)-3-methoxybenzamide**

To *N,N*-dimethylformamide (3 mL) were added with 4-((4-((2-(dimethylphosphoryl)phenyl)amino)-5-(trifluoromethyl)pyrimidin-2-yl)amino)-3-methoxybenzoic acid (48 mg, 0.1 mmol), 1-(4-(aminomethyl)piperidin-1-yl)prop-2-en-1-one hydrochloride (41 mg, 0.2 mmol), 2-(7-azobenzotriazole)-*N,N,N;*N'-tetramethylurea hexafluorophosphate (76 mg, 0.2 mmol), *N,N*-diisopropylethylamine (0.07 mL, 0.4 mmol), and 4-dimethylaminopyridine (1.2 mg, 0.01 mmol). Then reaction was allowed to proceed at room temperature for 16 hours. Upon completion of the reaction indicated by LCMS, the reaction was quenched with water (10 mL) and extracted with EA (20 mL×3). The combined organic phases were washed with water, dried and concentrated. The residue was purified by ISCO column chromatography to obtain *N*-((1-acryloylpiperidin-4-yl)methyl)-4-((4-((2-(dimethylphosphoryl)phenyl)amino)-5-(trifluoromethyl)pyrimidin-2-yl)amino)-3-methoxybenzamide (50 mg).

$^1H$ NMR (400 MHz, DMSO-$d_6$) δ 10.73 (s, 1H), 8.61 (s, 1H), 8.50 (t, J = 5.9 Hz, 1H), 8.44 (s, 1H), 8.16 (s, 1H), 7.85 (d, J = 8.4 Hz, 1H), 7.64 - 7.56 (m, 1H), 7.53 (d, J = 1.8 Hz, 1H), 7.46 (t, J = 7.9 Hz, 1H), 7.38 (dd, J = 8.3, 1.8 Hz, 1H), 7.22 (t, J = 7.5 Hz, 1H), 6.80 (dd, J = 16.7, 10.5 Hz, 1H), 6.07 (dd, J = 16.7, 2.5 Hz, 1H), 5.64 (dd, J = 10.4, 2.5 Hz, 1H), 3.88 (s, 3H), 3.18 (s, 2H), 3.02 (t, J = 12.7 Hz, 1H), 2.63 (t, J = 12.7 Hz, 1H), 1.85 (d, J = 4.0 Hz, 1H), 1.73 (d, J = 13.6 Hz, 8H), 1.14 - 1.00 (m, 4H). MS m/z (ESI): 631.2 $[M+H]^+$.

**Example 9**

**Preparation of 2-((2-((4-(((4-(1-acryloylpyrrolidin-2-yl)phenyl)amino)methyl)phenyl)amino)-5-(trifluoromethyl)pyrimidin-4-yl)amino)-N-methylbenzamide (9)**

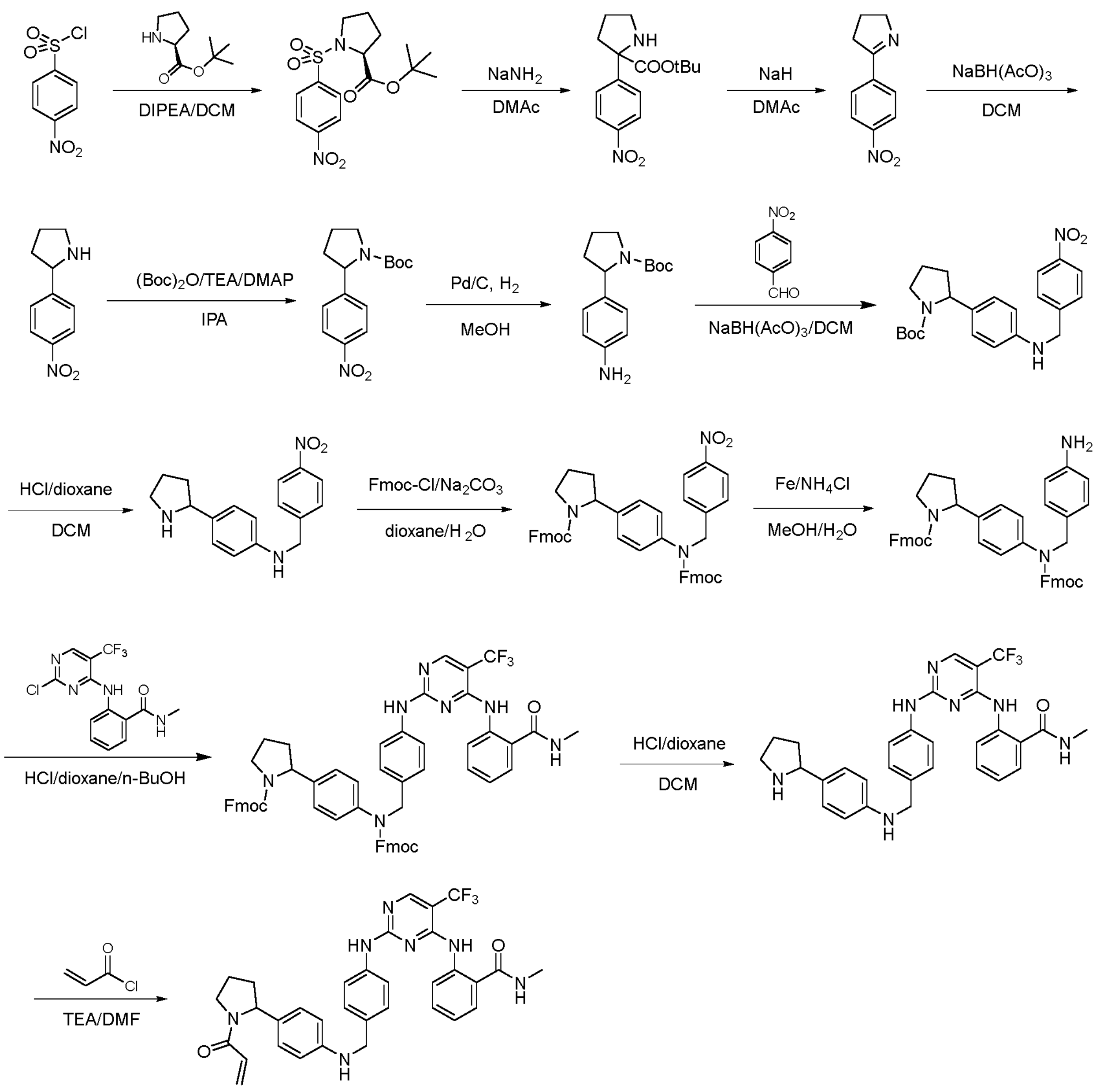


**Step 1: synthesis of *t*-butyl ((4-nitrophenyl)sulfonyl)-L-prolinate**

In DCM (100mL) were dissolved *t*-butyl L-prolinate hydrochloride (10.3 g, 49.59 mmol), DIPEA (22.37mL, 135.37 mmol), added with 4-nitrobenzenesulfonyl chloride (10g, 45.12 mmol) in ice bath and allowed to react at room temperature for 2 hours. The mixture was added with water and extracted with DCM. The combined organic phases were washed with water, dried and concentrated. The residue was purified by column chromatography to obtain 8.6g of *t*-butyl ((4-nitrophenyl)sulfonyl)-L-prolinate, MS m/z (ESI): 300.9[M+H-56]$^+$.

**Step 2: synthesis of *t*-butyl 2-(4-nitrophenyl)pyrrolidine-2-carboxylate**

In DMAc (80mL) was dissolved *t*-butyl ((4-nitrophenyl)sulfonyl)-L-prolinate (8.6 g, 24.16 mmol), added under $N_2$ protection with $NaNH_2$ (1.41g, 36.15 mmol) in batches in ice bath. After reaction for 3 hours at room temperature, the mixture was quenched with saturated aqueous solution of ammonium chloride, and extracted with EA. The combined organic phases were washed with water, dried and concentrated. The residue was purified by column chromatography to obtain 4.6 g of *t*-butyl 2-(4-nitrophenyl)pyrrolidine-2-carboxylate, MS m/z (ESI): 293.1[M+H]$^+$.

**Step 3: synthesis of 5-(4-nitrophenyl)-3,4-dihydro-2H--pyrrole**

In DMAc (40 mL) was dissolved *t*-butyl 2-(4-nitrophenyl)pyrrolidine-2-carboxylate (4 g, 13.68 mmol), added under $N_2$ protection with NaH (0.98g, 24.63 mmol) in batches in ice bath. After 30 minutes of reaction at room temperature, the mixture was allowed to react overnight at 50°C. The reaction was quenched with saturated aqueous solution of ammonium chloride and extracted with EA. The combined organic phases were washed with water, dried and concentrated. The residue was purified by column chromatography to obtain 0.727 g of 5-(4-nitrophenyl)-3,4-dihydro-2H-pyrrole, MS m/z (ESI): 191.0$[M+H]^+$.

**Step 4: synthesis of 2-(4-nitrophenyl)-pyrrolidine**

In DCM (15mL) was dissolved 5-(4-nitrophenyl)-3,4-dihydro-2H-pyrrole (0.72g, 3.79 mmol), added with $NaBH(AcO)_3$ (3.3g, 15.57 mmol), and stirred overnight at room temperature. Upon completion of the reaction, the reaction was quenched with aqueous solution of ammonium chloride and extracted with EA. The combined organic phases were washed with water, dried and concentrated. The residue was purified by column chromatography to obtain 0.44g of 2-(4-nitrophenyl)-pyrrolidine, MS m/z (ESI): 193.0$[M+H]^+$.

**Step 5: synthesis of *t*-butyl 2-(4-nitrophenyl)pyrrolidine-1-carboxylate**

In isopropanol (4 mL) was dissolved 2-(4-nitrophenyl)-pyrrolidine (0.44g, 2.29 mmol), added with TEA (0.96 mL, 6.87 mmol), added with $(Boc)_2O$ (0.8mL, 3.44 mmol) and DMAP (2.8mg, 0.02 mmol) in ice bath. The mixture was allowed to react at room temperature for 1 hour. Upon completion of the reaction, the reaction was added with water, extracted with EA. The combined organic phases were dried and concentrated. The residue was purified by column chromatography to obtain 0.62 g of *t*-butyl 2-(4-nitrophenyl)pyrrolidine-1-carboxylate, MS m/z (ESI): 237.1 $[M+H-56]^+$.

**Step 6: synthesis of *t*-butyl 2-(4-aminophenyl)pyrrolidine-1-carboxylate**

In methanol (12 mL) were dissolved *t*-butyl 2-(4-nitrophenyl)pyrrolidine-1-carboxylate (0.62 g, 2.05 mmol) and 10% Pd/C (0.12 g), bubbled with hydrogen gas, and allowed to react overnight at room temperature. Upon completion of the reaction, the mixture was filtered, and the filtrate was concentrated to obtain a crude product, which was directly used in the next step.

**Step 7: synthesis of *t*-butyl 2-(4-((4-nitrobenzyl)amino)phenyl)pyrrolidine-1-carboxylate**

In dichloromethane (10 mL) were dissolved *t*-butyl 2-(4-aminophenyl)pyrrolidine-1-carboxylate (0.42g, 1.60 mmol) and 4-nitrobenzaldehyde (0.256 g, 1.69 mmol). The mixture was allowed to react at room temperature for 2 hours, then added with $NaBH(AcO)_3$ (1 g, 4.72 mmol) and stirred at room temperature for 1 hour. Upon completion of reaction, the reaction was quenched with saturated aqueous solution of ammonium chloride and extracted with EA. The combined organic phases were washed with water, dried and concentrated. The residue was purified by column chromatography to obtain 0.64 g of *t*-butyl 2-(4-((4-nitrobenzyl)amino)phenyl)pyrrolidine-1-carboxylate, MS m/z (ESI): 398.2$[M+H-56]^+$.

**Step 8: synthesis of *N*-(4-nitrobenzyl)-4-(pyrrolidin-2-yl)aniline**

In dichloromethane (10 mL) was dissolved *t*-butyl 2-(4-((4-nitrobenzyl)amino)phenyl)pyrrolidine-1-carboxylate (0.64g, 1.61 mmol), added with 4 N hydrochloric acid in dioxane (4 mL) dropwise in ice bath, and stirred at room temperature for 30 minutes. Upon completion of the reaction, the reaction solution was concentrated to obtain a crude product, which was directly used in the next step.

**Step 9: synthesis of (9H-fluoren-9-yl)methyl 2-(4-(((((9H-fluoren-9-yl)methoxy)carbonyl)(4-nitrobenzyl)amino)phenyl)pyrrolidine-1-carboxylate**

In dioxane (3.8 mL) and water (3.8 mL) was dissolved the crude product obtained in the last step, added with sodium carbonate (0.54 g, 5.10 mmol), then added with Fmoc-Cl (0.99 g, 3.83 mmol) in ice bath, and stirred at room temperature for 1 hour. Upon the complete reaction, the reaction was added with small amount of water, and then added with 1 N hydrochloric acid solution to adjust to acidic pH, and then extracted with ethyl acetate. The combined organic phases were washed with water, dried and concentrated. The residue was purified by column chromatography to obtain 1.0 g of (9H-fluoren-9-yl)methyl 2-(4-(((((9H-fluoren-9-yl)methoxy)carbonyl)(4-nitrobenzyl)amino)phenyl)pyrrolidine-1-carboxylate, MS m/z (ESI): 742.3 $[M+H]^+$.

**Step 10: synthesis of (9H-fluoren-9-yl)methyl 2-(4-(((((9H-fluoren-9-yl)methoxy)carbonyl)(4-aminobenzyl)amino)phenyl)pyrrolidine-1-carboxylate**

In methanol (100 mL) and water (25 mL) were dissolved (9H-fluoren-9-yl)methyl 2-(4-(((((9H-fluoren-9-yl)methoxy)carbonyl)(4-nitrobenzyl)amino)phenyl)pyrrolidine-1-carboxylate (1.0g, 1.35 mmol), iron powder (189 mg, 3.38 mmol), and ammonium chloride (181 mg, 3.38 mmol). The mixture was refluxed at 70°C overnight. Upon completion of the reaction, the mixture was filtered. The filtrate was concentrated and the residue was purified by column chromatography to obtain 505mg of (9H-fluoren-9-yl)methyl

2-(4-(((((9H-fluoren-9-yl)methoxy)carbonyl)(4-aminobenzyl)amino)phenyl)pyrrolidine-1-carboxylate, MS m/z (ESI): 712.3 $[M+H]^+$.

**Step 11: synthesis of (9H-fluoren-9-yl)methyl 2-(4-(((((9H-fluoren-9-yl)methoxy)carbonyl)(4-((4-((2-(methylcarbamoyl)phenyl)amino)-5-(trifluoromethyl) pyrimidin-2-yl)amino)benzyl)amino)phenyl)pyrrolidine-1-carboxylate**

In *n*-butanol (10 mL) were dissolved (9H-fluoren-9-yl)methyl 2-(4-(((((9H-fluoren-9-yl)methoxy)carbonyl)(4-aminobenzyl)amino)phenyl)pyrrolidine-1-carboxylate (340 mg, 0.48 mmol) and 2-((2-chloro-5-(trifluoromethyl)pyrimidin-4-yl)amino)-N-methylbenzamide (150 mg, 0.45 mmol), added with 4N hydrochloric acid in dioxane (34 μL), and allowed to react at 70°C for 4 hours. Upon the complete of reaction, the reaction solution is concentrated and the residue was purified by column chromatography to obtain 382 mg (9H-fluoren-9-yl)methyl 2-(4-(((((9H-fluoren-9-yl)methoxy)carbonyl)(4-((4-((2-(methylcarbamoyl)phenyl)amino)-5-(trifluoromethyl)pyri midin-2-yl)amino)benzyl)amino)phenyl)pyrrolidine-1-carboxylate, MS m/z (ESI): 1006.4 $[M+H]^+$.

**Step 12: synthesis of N-methyl-2-((2-((4-(((4-(pyrrolidin-2-yl)phenyl)amino)methyl)phenyl)amino)-5-(trifluoromethyl)pyrimidin-4-yl)amino) benzamide**

In DMF (8 mL) was dissolved (9H-fluoren-9-yl)methyl 2-(4-(((((9H-fluoren-9-yl)methoxy)carbonyl)(4-((4-((2-(methylcarbamoyl)phenyl)amino)-5-(trifluoromethyl)pyri midin-2-yl)amino)benzyl)amino)phenyl)pyrrolidine-1-carboxylate (382 mg, 0.38 mmol), added with piperidine (1.6 mL) in ice bath and allowed to react at room temperature for 20 minutes. Upon the complete of reaction, the reaction was added with water, extracted with DCM, dried and concentrated. The residue was purified by column chromatography to obtain 180mg of N-methyl-2-((2-((4-(((4-(pyrrolidin-2-yl)phenyl)amino)methyl)phenyl)amino)-5-(trifluoromethyl)pyrimidin-4-yl) amino) benzamide, MS m/z (ESI): 562.2 [M+H]+.

**Step 13: synthesis of 2-((2-((4-(((4-(1-acryloylpyrrolidin-2-yl)phenyl)amino)methyl)phenyl)amino)-5-(trifluoromethyl)pyrimidin-4-yl)amino)-*N*-methylbenzamide**

In DMF (4 mL) was dissolved N-methyl-2-((2-((4-(((4-(pyrrolidin-2-yl)phenyl)amino)methyl)phenyl)amino)-5-(trifluoromethyl)pyrimidin-4-yl) amino) benzamide (68 mg, 0.12 mmol), added with triethylamine (50.68 μL, 0.36 mmol) in ice bath followed by acryloyl chloride (11.23 μL, 0.15 mmol). The mixture was allowed to react at room temperature for 10 minutes. Upon completion of the reaction, the reaction was added with water, extracted with DCM, dried and concentrated. The residue was purified by column chromatography to obtain 12 mg 2-((2-((4-(((4-(1-acryloylpyrrolidin-2-yl)phenyl)amino)methyl)phenyl)amino)-5-(trifluoromethyl)pyrimidin-4-yl)a mino)-N-methylbenzamide.

$^1$H NMR (400 MHz, DMSO-$d_6$) δ 11.33 (s, 1H), 9.78 (s, 1H), 8.71 (d, J = 4.3 Hz, 1H), 8.40 (s, 1H), 7.68 (d, *J* = 7.9 Hz, 1H), 7.55 (d, *J* = 7.3 Hz, 2H), 7.31 (s, 1H), 7.23 (d, *J* = 8.6 Hz, 2H), 7.16 - 7.06 (m, 1H), 6.84 (d, *J* = 8.4 Hz, 2H), 6.54 (d, *J* = 8.4 Hz, 2H), 6.10 (m, 3H), 5.63 (d, *J* = 10.7 Hz, 1H), 5.40 (d, *J* = 7.6 Hz, 1H), 4.98 (t, *J* = 8.9 Hz, 1H), 4.16 (s, 2H), 3.67 - 3.52 (m, 2H), 2.76 (d, *J* = 4.5 Hz, 3H), 1.74 (m, 4H). MS m/z (ESI): 616.3 $[M+H]^+$.

**Example 10**

**Preparation of 1-(3-(3-((4-((4-((2-(dimethylphosphoryl)phenyl)amino)-5-(trifluoromethyl)pyrimidin-2-yl)amino)benzyl)ami no)phenyl)piperidin-1-yl)prop-2-en-1-one (10)**

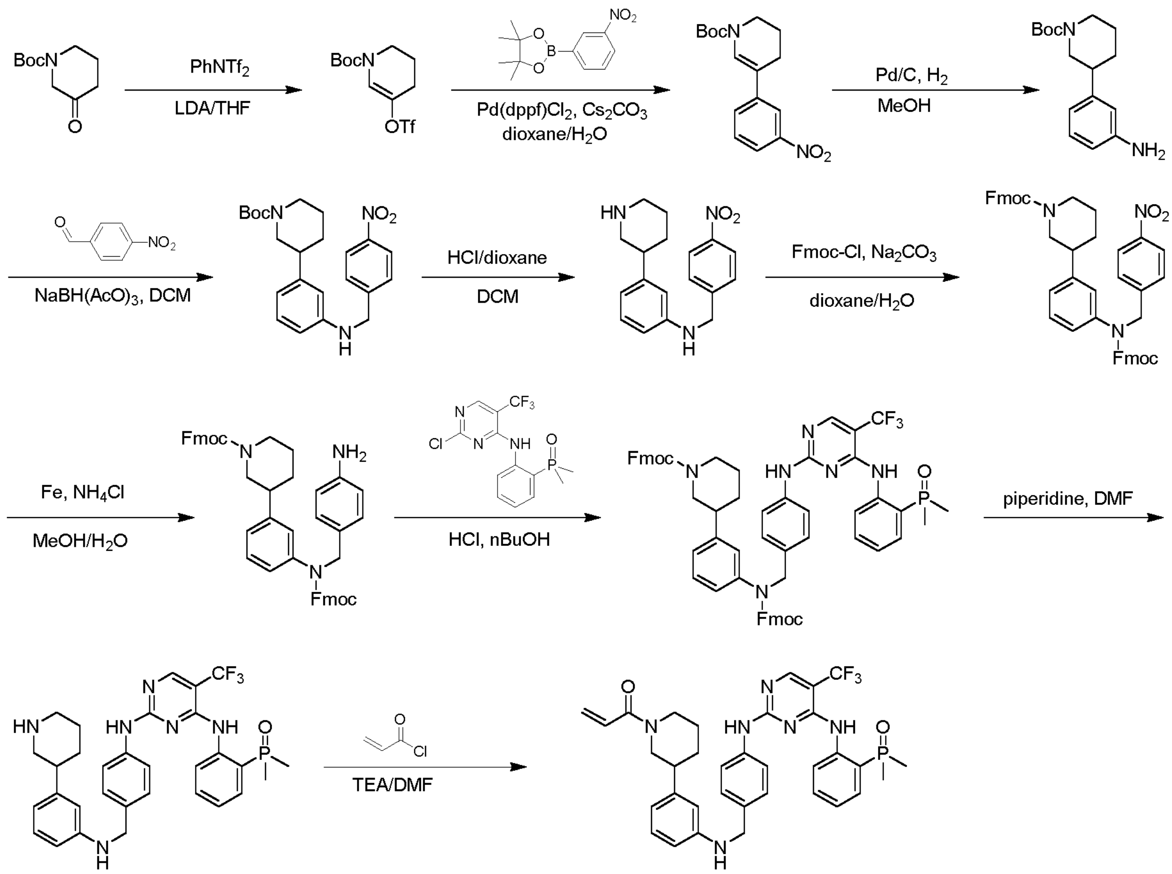


**Step 1: synthesis of 5-((((trifluoromethyl)sulfonyl)oxy)*t*-butyl-3,4-dihydropyridine-1(2H)-carboxylate**

In tetrahydrofuran (100 mL) was dissolved *t*-butyl 3-oxo piperidine-1-carboxylate (4.95g, 24.84 mmol), cooled to -78°C, and added with 25 mL solution of LDA in tetrahydrofuran dropwise. The mixture was stirred for 30 minutes, then added with 1,1,1-trifluoro-N phenyl-N ((trifluoromethyl)sulfonyl)methylsulfonatnide (10.65g, 29.81 mmol). The mixture was stirred at room temperature for 3h. Upon completion of the reaction, the reaction system was quenched with saturated NaHCOs solution, concentrated, and extracted with DCM. The combined organic phases were washed with water, dried and concentrated. The residue was purified by column chromatography to obtain 2.37 g 5-((((trifluoromethyl)sulfonyl)oxy)*t*-butyl-3,4-dihydropyridine-1(2H)-carboxylate, MS m/z (ESI): 231.9 $[M+H-100]^+$.

**Step 2: synthesis of *t*-butyl 5-(3-nitrophenyl)-3,4-dihydropyridine-1(2H)-carboxylate**

In dioxane (60 mL) and water (20 mL) were added with 5-((((trifluoromethyl)sulfonyl)oxy)*t*-butyl-3,4-dihydropyridine-1(2H)-carboxylate (2.3 g, 6.95 mmol), 4,4,5,5-tetratnethyl-2-(3-nitrophenyl)-1,3,2-dioxaborolane (3.46 g, 13.9 mmol), $Pd(dppf)Cl_2$ (0.57g, 0.69 mmol), and $Cs_2CO_3$ (6.80g, 20.84 mmol). The mixture was stirred overnight at 80°C under nitrogen protection. Upon completion of the reaction, the mixture was cooled to room temperature, and concentrated. The residue was added with ethanol and filtered to remove insoluble matters, and then concentrated. The residue was purified by column chromatography to obtain 1.2 g *t*-butyl 5-(3-nitrophenyl)-3,4-dihydropyridine-1(2H)-carboxylate, MS m/z (ESI): 248.9 $[M+H-56]^+$.

**Step 3: synthesis of *t*-butyl 3-(3-aminophenyl) piperidine-1-carboxylate**

In methanol (24 mL) were dissolved *t*-butyl 5-(3-nitrophenyl)-3,4-dihydropyridine-1(2H)-carboxylate (1.2 g, 3.94 mmol) and Pd/C (0.24 g), bubbled with hydrogen and allowed to react at room temperature overnight. Upon completion of the reaction, the mixture was filtered and the filtrate was concentrated to obtain a crude product, which was used directly for the next step.

**Step 4: synthesis of *t*-butyl 3-(3-(((4-nitrobenzyl)amino)phenyl) piperidine-1-carboxylate**

In dichloromethane (16 mL) were dissolved the crude product obtained in the last step and 4-nitrobenzaldehyde (0.46 g, 3.04 mmol). The mixture was allowed to react at room temperature for 2h, and then added with $NaBH(AcO)_3$ (1.8 g, 8.69 mmol), and stirred at room temperature for 1 hour. Upon completion of the reaction, the reaction was quenched with saturated solution of ammonium chloride, and extracted with DCM. The combined organic phases were washed with water, dried and concentrated. The residue was purified by column chromatography to obtain 1 g 3-(3-(((4-nitrobenzyl)amino)phenyl) piperidine-1-carboxylate *t*-butyl, MS m/z (ESI): 356.0[M+H-56]$^+$.

**Step 5: synthesis of *N*-(4-nitrobenzyl)-3-(piperidin-3-yl)aniline**

In dichloromethane (10 mL) was dissolved *t*-butyl 3-(3-(((4-nitrobenzyl)amino)phenyl) piperidine-1-carboxylate (1.00 g, 2.43 mmol), added with hydrochloric acid in dioxane (4 N, 4 mL) in ice bath dropwise, and stirred at room temperature for 30 min. Upon completion of the reaction, the mixture was concentrated to obtain a crude product, which was used directly for the next step.

**Step 6: synthesis of (9H-fluoren-9-yl)methyl 3-(3-((((((9H-fluoren-9-yl)methoxy)carbonyl)(4-nitrobenzyl)amino)phenyl) piperidine-1-carboxylic acid**

In dioxane (5 mL) and water (5 mL) were added the crude product obtained in the last step, added with sodium carbonate (0.68g, 6.43 mmol), added with Fmoc-Cl (0.86g, 3.32 mmol) in ice bath, and stirred at room temperature for 2 h. Upon completion of the reaction, the mixture was added with small amount of water, adjusted to acidic pH by 1N hydrochloric acid solution, and then extracted with ethyl acetate. The combined organic phases were washed with water, dried and concentrated. The residue was purified by column chromatography to obtain 1.1 g (9H-fluoren-9-yl)methyl 3-(3-((((((9H-fluoren-9-yl)methoxy)carbonyl)(4-nitrobenzyl)amino)phenyl) piperidine-1-carboxylic acid, MS m/z (ESI): 756.1 [M+H]$^+$.

**Step 7: synthesis of (9H-fluoren-9-yl)methyl 3-(3-(((((((9H-fluoren-9-yl)methoxy)carbonyl))(4-aminobenzyl)amino)phenyl) piperidine-1-carboxylic acid**

In methanol (100 mL) and water (25 mL) were dissolved (9H-fluoren-9-yl)methyl 3-(3-((((((9H-fluoren-9-yl)methoxy)carbonyl)(4-nitrobenzyl)amino)phenyl) piperidine-1-carboxylic acid (1.10 g, 1.46 mmol), iron powder (174 mg, 3.11 mmol), ammonium chloride (196 mg, 3.67 mmol), and refluxed at 70°C overnight. Upon completion of the reaction, the mixture was filtered and the filtrate was concentrated. The residue was purified by column chromatography to obtain 370 mg (9H-fluoren-9-yl)methyl 3-(3-(((((((9H-fluoren-9-yl)methoxy)carbonyl))(4-aminobenzyl)amino)phenyl) piperidine-1-carboxylic acid, MS m/z (ESI): 726.2 [M+H]$^+$.

**Step 8: synthesis of (9H-fluoren-9-yl)methyl 3-(3-((((((9H-fluoren-9-yl)methoxy)carbonyl))(4-((4-(((2-(dimethylphosphoryl)phenyl)amino)-5-(trifluorome thyl)pyrimidin-2-yl)amino)benzyl)amino)phenyl) piperidine-1-carboxylic acid**

In n-butanol (10 mL) were dissolved (9H-fluoren-9-yl)methyl 3-(3-(((((((9H-fluoren-9-yl)methoxy)carbonyl))(4-aminobenzyl)amino)phenyl) piperidine-1-carboxylic acid (224 mg, 0.31 mmol), (2-((2-chloro-5-(trifluoromethyl)pyrimidin-4-yl)amino)phenyl)dimethylphosphine oxide(102mg, 0.29mmol), added with hydrochloric acid in dioxane (4 N, 22 $\mu$L), and allowed to react at 70°C for 4 h. Upon completion of the reaction, the mixture was concentrated. The residue was purified by column chromatography to obtain 314 mg (9H-fluoren-9-yl)methyl 3-(3-((((((9H-fluoren-9-yl)methoxy)carbonyl))(4-((4-(((2-(dimethylphosphoryl)phenyl)atnino)-5-(trifluoromethyl) pyrimidin-2-yl)amino)benzyl)amino)phenyl) piperidine-1-carboxylic acid, MS m/z (ESI): 1069.4 [M+H]+.

**Step 9: synthesis of dimethyl(2-((2-((4-(((3-(piperidin-3-yl)phenyl)amino)methyl)phenyl)amino)-5-(trifluoromethyl)pyrimidin-4 -yl)amino)phenyl)phosphine oxide**

In DMF (7 mL) was dissolved (9H-fluoren-9-yl)methyl 3-(3-((((((9H-fluoren-9-yl)methoxy)carbonyl))(4-((4-(((2-(dimethylphosphoryl)phenyl)atnino)-5-(trifluoromethyl) pyrimidin-2-yl)amino)benzyl)amino)phenyl) piperidine-1-carboxylic acid (314 mg, 0.29 mmol), added with piperidine (1.4 mL) in ice bath, and allowed to react at room temperature for 20 min. Upon completion of the reaction, the reaction was added with water, extracted with DCM, dried and concentrated. The residue was purified by column chromatography to obtain 174 mg dimethyl(2-((2-((4-(((3-(piperidin-3-yl)phenyl)amino)methyl)phenyl)amino)-5-(trifluoromethyl)pyrimidin-4-yl)a mino)phenyl)phosphine oxide, MS m/z

(ESI): 594.3 $[M+H]^+$.

**Step 10: synthesis of 1-(3-(3-((4-((4-((2-(dimethylphosphoryl)phenyl)amino)-5-(trifluoromethyl)pyrimidin-2-yl)amino)benzyl)ami no)phenyl)piperidin-1-yl)prop-2-en-1-one**

In DMF (4 mL) was dissolved dimethyl (2-((2-((4-(((3-(piperidin-3-yl)phenyl)amino)methyl)phenyl)amino)-5-(trifluoromethyl)pyrimidin-4-yl)amino)phe nyl)phosphine oxide (160 mg, 0.27 mmol), added with triethylamine (0.11 mL, 0.81 mmol) in ice bath, then added with acryloyl chloride (21.89 μL, 0.27 mmol) dropwise, and allowed to react at room temperature for 10 minutes. Upon completion of the reaction, the reaction was added with water, extracted with DCM, dried and concentrated. The residue was purified by column chromatography to obtain 24 mg 1-(3-(3-((4-((4-((2-(dimethylphosphoryl)phenyl)amino)-5-(trifluoromethyl)pyrimidin-2-yl)amino)benzyl)amino)p henyl)piperidin-1 -yl)prop-2-en-1 -one.

$^1H$ NMR (400 MHz, DMSO) δ 10.59 (s, 1H), 9.76 (s, 1H), 8.41 (s, 1H), 8.20 (s, 1H), 7.59 (m, 1H), 7.51 (d, *J* = 5.8 Hz, 2H), 7.41 (s, 1H), 7.26 - 7.11 (m, 3H), 6.98 (t, *J* = 7.8 Hz, 1H), 6.51 (s, 1H), 6.43 (d, *J* = 6.9 Hz, 2H), 6.15 - 6.00 (m, 2H), 5.66 (d, *J*= 8.3 Hz, 1H), 5.59 (d, *J*= 10.8 Hz, 1H), 4.44 (d, *J* = 13.3 Hz, 1H), 4.18 (d, *J* = 5.6 Hz, 2H), 4.06 (d, *J*= 15.6 Hz, 1H), 3.92 (d, *J* = 13.7 Hz, 1H), 3.10 - 2.99 (m, 1H), 2.62 (m, 1H), 1.86 (d, *J*= 14.3 Hz, 2H), 1.72 (d, *J*= 13.6 Hz, 6H), 1.51 - 1.36 (m, 2H). MS m/z (ESI): 649.3 $[M+H]^+$.

**Example 11**

**2-((2-((4-(((3-(1-acryloylpiperidin-3-yl)phenyl)amino)methyl)phenyl)amino)-5-(trifluoromethyl)pyrimidin-4 -yl)amino)-N-methoxybenzamide Preparation of (11)**

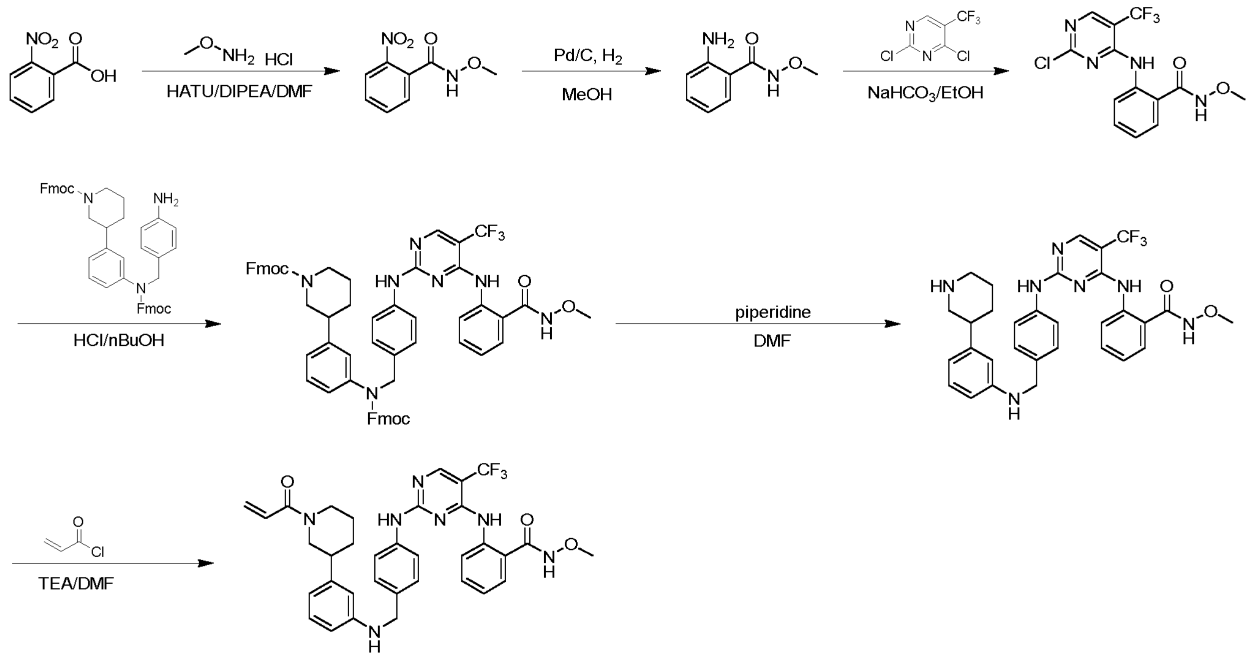


**Step 1: synthesis of *N*-methoxy-2-nitrobenzamide**

In DMF (10 mL) was dissolved N-methoxy-2-nitrobenzamide (1 g, 5.98 mmol), added with HATU (3.4 g, 8.98 mmol) and DIPEA (1.98 mL, 11.97mmol) in ice bath, and allowed to react at room temperature for 1 h, and then added with methoxyamine hydrochloride (1 g, 11.97 mmol) and allowed to react at room temperature overnight. Upon completion of the reaction, the reaction was added with water, extracted with DCM, dried and concentrated. The residue was purified by column chromatography to obtain 1.45 g N-methoxy-2-nitrobenzamide, MS m/z (ESI): 197.0 $[M+H]^+$.

**Step 2: synthesis of 2-amino-*N*-methoxybenzamidobenzamide**

In methanol (24 mL) were dissolved N-methoxy-2-nitrobenzamide (1.17 g, 5.97 mmol) and Pd/C (0.24 g)

bubbled with hydrogen, and allowed to react at room temperature overnight. Upon completion of the reaction, the mixture was filtered and the filtrate was concentrated to obtain a crude product, which was used directly for the next step.

**Step 3: synthesis of 2-((2-chloro-5-(trifluoromethyl)pyrimidin-4-yl)amino)-N-methoxybenzamide**

In ethanol (10 mL) was dissolved 2,4-dichloro-5-(trifluoromethyl)pyrimidine (722 mg, 3.33 mmol) added with NaHCOs (466 mg, 5.55 mmol), then added with N-methoxy-2-nitrobenzamide (460 mg, 2.77 mmol) in ethanol dropwise, and allowed to react at room temperature overnight. Upon completion of the reaction, the reaction was added with water, extracted with DCM, dried and concentrated. The residue was purified by column chromatography to obtain 280mg 2-((2-chloro-5-(trifluoromethyl)pyrimidin-4-yl)amino)-N-methoxybenzamide, MS m/z (ESI): 347.0 $[M+H]^+$.

**Step 4: synthesis of (9H-fluoren-9-yl)methyl 3-(3-(((((9H-fluoren-9-yl)methoxy)carbonyl)(4-(((4-((2-(methoxycarbamoyl)phenyl)amino)-)-5-(trifluoromet hyl)pyrimidin-2-yl)amino)benzyl)amino)phenyl) piperidine-1-carboxylate**

In n-butanol (10 mL) were dissolved (9H-fluoren-9-yl)methyl 3-(3-(((((9H-fluoren-9-yl)methoxy)carbonyl)(4-aminobenzyl)amino)phenyl) piperidine-1-carboxylate (272 mg, 0.38 mmol), and 2-((2-chloro-5-(trifluoromethyl)pyrimidin-4-yl)amino)-N-methoxybenzamide (153 mg, 0.44 mmol), added with hydrogen chloride in dioxane (4 N, 28 μL), and allowed to react at 70°C overnight. Upon completion of the reaction, the mixture was concentrated. The residue was purified by column chromatography to obtain 321 mg (9H-fluoren-9-yl)methyl 3-(3-(((((9H-fluoren-9-yl)methoxy)carbonyl)(4-(((4-((2-(methoxycarbamoyl)phenyl)amino)-)-5-(trifluoromethyl) pyrimidin-2-yl)amino)benzyl)amino)phenyl) piperidine-1-carboxylate, MS m/z (ESI): 1036.4 $[M+H]^+$.

**Step 5: synthesis of *N*-methoxy-2-((2-((4-(((3-(piperidin-3-yl)phenyl)amino)methyl)phenyl)amino)-5-(trifluoromethyl)pyrimidin -4-yl)amino) benzamide**

In DMF (7 mL) was dissolved (9H-fluoren-9-yl)methyl 3-(3-(((((9H-fluoren-9-yl)methoxy)carbonyl)(4-(((4-((2-(methoxycarbamoyl)phenyl)amino)-)-5-(trifluoromethyl) pyrimidin-2-yl)amino)benzyl)amino)phenyl) piperidine-1-carboxylate (321 mg, 0.31 mmol), added with piperidine (1.4 mL) in ice bath, and allowed to react at room temperature for 20 min. Upon completion of the reaction, the reaction was added with water, extracted with DCM, dried and concentrated. The residue was purified by column chromatography to obtain 87 mg N methoxy-2-((2-((4-(((3-(piperidin-3-yl)phenyl)atnino)methyl)phenyl)amino)-5-(trifluoromethyl)pyrimidin-4-yl) amino) benzamide, MS m/z (ESI): 592.3 $[M+H]^+$.

**Step 6: synthesis of 2-((2-((4-(((3-(1-acryloylpiperidin-3-yl)phenyl)amino)methyl)phenyl)amino)-5-(trifluoromethyl)pyrimidin-4 -yl)amino)-N-methoxybenzamide**

In DMF (4 mL) was dissolved *N*-methoxy-2-((2-((4-(((3-(piperidin-3-yl)phenyl)amino)methyl)phenyl)amino)-5-(trifluoromethyl)pyrimidin-4-yl) amino) benzamide (60mg, 0.10 mmol), added with triethylamine (30 μL, 0.30 mmol) in ice bath, then added with acryloyl chloride (8.11 μL, 0.10 mmol) dropwise, and allowed to react at room temperature for 10 minutes. Upon completion of the reaction, the reaction was added with water, extracted with DCM, dried and concentrated. The residue was purified by column chromatography to obtain 17 mg 2-((2-((4-(((3 -(1 -acryloylpiperidin-3 -yl)phenyl)amino)methyl)phenyl)amino)-5 -(trifluoromethyl)pyrimidin-4-yl)a mino)-N-methoxybenzamide.

$^1$H NMR (400 MHz, DMSO-$d_6$) δ 11.95 (s, 1H), 10.60 (s, 1H), 9.81 (s, 1H), 8.41 (s, 1H), 7.55 (d, *J* = 6.9 Hz, 3H), 7.35 (s, 1H), 7.24 (d, *J* = 8.1 Hz, 2H), 7.15 - 7.05 (m, 1H), 6.97 (t, *J* = 7.8 Hz, 1H), 6.86 - 6.65 (m, 1H), 6.50 (s, 1H), 6.42 (d, *J* = 6.3 Hz, 2H), 6.15 - 5.97 (m, 2H), 5.64 (d, *J* = 10.4 Hz, 1H), 5.58 (d, *J* = 11.2 Hz, 1H), 4.43 (d, *J* = 12.0 Hz, 1H), 4.19 (d, *J* = 5.9 Hz, 2H), 4.05 (d, *J* = 14.0 Hz, 1H), 3.96 - 3.84 (m, 1H), 3.69 (s, 3H), 3.09 - 2.96 (m, 1H), 2.61 (m, 1H), 1.84 (m, 1H), 1.79 - 1.55 (m, 3H). MS m/z (ESI): 646.3 $[M+H]^+$.

**Example 12**

**Preparation of 2-(((2-((4-(((3-(1-acryloylpiperidin-3-yl)phenyl)amino)methyl)-2-methoxyphenyl)amino)-5-(trifluoromethyl) pyrimidin-4-yl)amino)-N-methylbenzamide (12)**

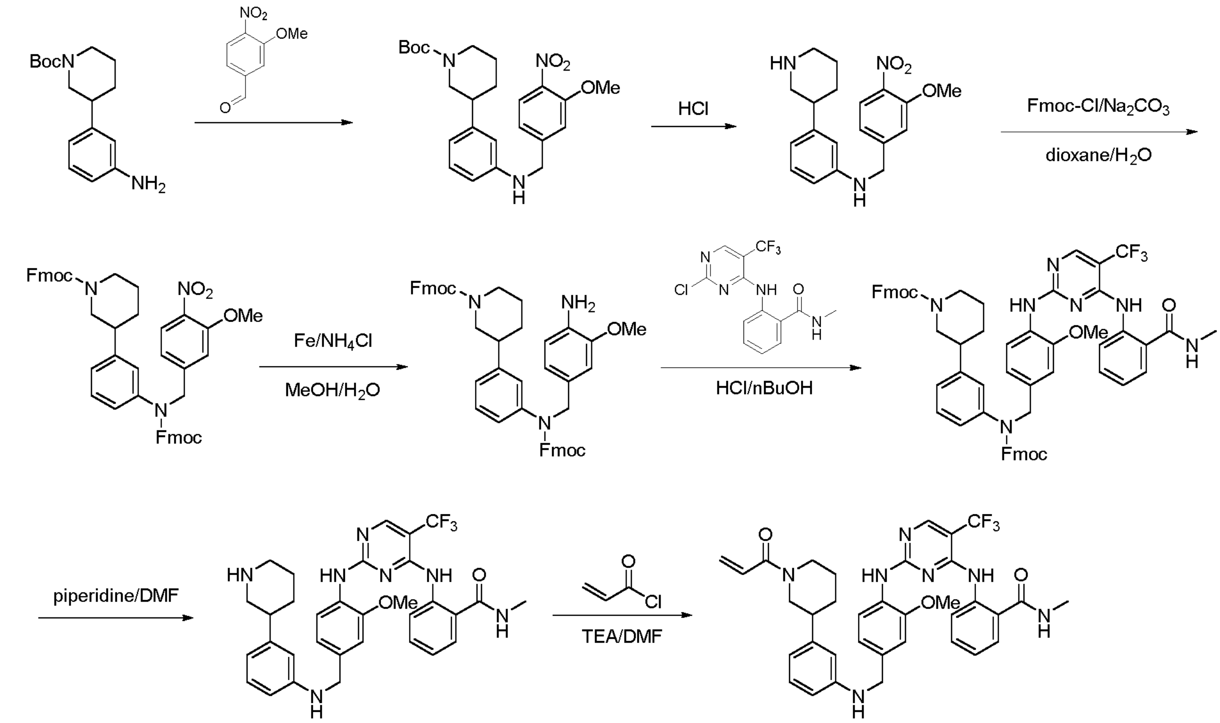


**Step 1: synthesis of *t*-butyl 3-(3-((3-methoxy-4-nitrobenzyl)amino)phenyl) piperidine-1-carboxylate**

In dichloromethane (10 mL) was dissolved *t*-butyl 3-(3-aminophenyl) piperidine-1-carboxylate (0.40 g, 1.45 mmol), 3-methoxy-4-nitrobenzaldehyde (0.28g, 1.52 mmol), allowed to react at room temperature for 2h, and then added with $NaBH(AcO)_3$ (0.92 g, 4.34 mmol), stirred at room temperature for 1 h. Upon completion of the reaction, the reaction was quenched with saturated solution of ammonium chloride, extracted with DCM. The combined organic phases were washed with water, dried and concentrated. The residue was purified by column chromatography to obtain 0.64 g 3-(3-((3-methoxy-4-nitrobenzyl)amino)phenyl) piperidine-1-carboxylate *t*-butyl, MS m/z (ESI): 386.1[M+H-56]$^+$.

**Step 2: synthesis of N-(3-methoxy-4-nitrobenzyl)-3-(piperidin-3-yl)aniline**

In dichloromethane (7 mL) was dissolved *t*-butyl 3-(3-((3-methoxy-4-nitrobenzyl)amino)phenyl) piperidine-1-carboxylate(0.64 g, 1.45 mmol), added dropwise with hydrochloric acid in dioxane (4 N, 3 mL) in ice bath, and stirred at room temperature for 30 min. Upon completion of the reaction, the mixture was concentrated to obtain a crude product, which was used directly for the next step.

**Step 3: synthesis of (9H-fluoren-9-yl)methyl 3-(3-(((((9H-fluoren-9-yl)methoxy)carbonyl)(3-methoxy-4-nitrobenzyl)amino)phenyl) piperidine-1-carboxylate**

In dioxane (3.2 mL) and water (3.2mL) was dissolved the crude product obtained in the last step, added with sodium carbonate (0.40 g, 3.77 mmol), and then added with Fmoc-Cl (0.73 g, 2.82 mmol) in ice bath, and stirred at room temperature for 1 hour. Upon completion of the reaction, the mixture was added with small amount of water, adjusted to acidic pH by adding 1N hydrochloric acid solution, extracted with ethyl acetate. The combined organic phases were washed with water, dried and concentrated. The residue was purified by column chromatography to obtain 1.1g (9H-fluoren-9-yl)methyl 3-(3-(((((9H-fluoren-9-yl)methoxy)carbonyl)(3-methoxy-4-nitrobenzyl)amino)phenyl) piperidine-1-carboxylate, MS m/z (ESI): 786.3 [M+H]$^+$.

**Step 4: synthesis of (9H-fluoren-9-yl)methyl 3-(3-(((((9H-fluoren-9-yl)methoxy)carbonyl)(4-amino-3-methoxybenzyl)amino)phenyl) piperidine-1-carboxylate**

In methanol (100 mL) and water (25 mL) were dissolved (9H-fluoren-9-yl)methyl 3-(3-(((((9H-fluoren-9-yl)methoxy)carbonyl)(3-methoxy-4-nitrobenzyl)amino)phenyl)piperidine-1-carboxylate (1.1g, 1.40mmol), iron powder (220 mg, 3.93 mmol), and ammonium chloride (210 mg, 3.93 mmol), and refluxed at 70°C overnight. Upon completion of the reaction, the mixture was filtered and the filtrate was concentrated, the residue was purified by column chromatography

to obtain 600 mg (9H-fluoren-9-yl)methyl 3-(3-(((((9H-fluoren-9-yl)methoxy)carbonyl)(4-amino-3-methoxybenzyl)amino)phenyl) piperidine-1-carboxylate, MS m/z (ESI): 756.3 $[M+H]^+$.

**Step 5: synthesis of (9H-fluoren-9-yl)methyl 3-(3-(((((9H-fluoren-9-yl)methoxy)carbonyl)(3-methoxy-4-((4-((2-(methylcarbamoyl))phenyl))amino)-5-(trif luoromethyl)pyrimidin-2-yl)amino)benzyl)amino)phenyl) piperidine-1-carboxylate**

In n-butanol (10 mL) were dissolved (9H-fluoren-9-yl)methyl 3-(3-(((((9H-fluoren-9-yl)methoxy)carbonyl)(4-amino-3-methoxybenzyl)amino)phenyl) piperidine-1-carboxylate (336 mg, 0.45 mmol) and 2-((2-chloro-5-(trifluoromethyl)pyrimidin-4-yl)amino)-N-methylbenzamide (147 mg, 0.45 mmol), added with 4N hydrochloric acid in dioxane (33μL), allowed to react at 70°C for 4 h. Upon completion of the reaction, the mixture was concentrated. The residue was purified by column chromatography to obtain 457 mg (9H-fluoren-9-yl)methyl 3-(3-(((((9H-fluoren-9-yl)methoxy)carbonyl)(3-methoxy-4-((4-((2-(methylcarbamoyl))phenyl))amino)-5-(trifluor omethyl)pyrimidin-2-yl)amino)benzyl)amino)phenyl) piperidine-1-carboxylate, MS m/z (ESI): 1050.4 $[M+H]^+$.

**Step 6: synthesis of 2-((2-((2-methoxy-4-(((3-(piperidin-3-yl)phenyl)amino)methyl)phenyl)amino)-5-(trifluoromethyl)pyrimidin-4-yl)amino)-N-methylbenzamide**

In DMF (7 mL) was dissolved (9H-fluoren-9-yl)methyl 3-(3-(((((9H-fluoren-9-yl)methoxy)carbonyl)(3-methoxy-4-((4-((2-(methylcarbamoyl))phenyl))amino)-5-(trifluor omethyl)pyrimidin-2-yl)amino)benzyl)amino)phenyl) piperidine-1-carboxylate (457 mg, 0.44 mmol), added with piperidine (1.4 mL) in ice bath, and allowed to react at room temperature for 20 min. Upon completion of the reaction, the reaction was added with water, extracted with DCM, dried and concentrated. The residue was purified by column chromatography to obtain 135 mg 2-((2-((2-methoxy-4-(((3-(piperidin-3-yl)phenyl)amino)methyl)phenyl)amino)-5-(trifluoromethyl)pyrimidin-4-yl) amino)-N-methylbenzamide, MS m/z (ESI): 606.3 $[M+H]^+$.

**Step 7: synthesis of 2-(((2-((4-(((3-(1-acryloylpiperidin-3-yl)phenyl)amino)methyl)-2-methoxyphenyl)amino)-5-(trifluoromethyl) pyrimidin-4-yl)amino)-N-methylbenzamide**

In DMF (4mL) was dissolved 2-((2-((2-methoxy-4-(((3-(piperidin-3-yl)phenyl)amino)methyl)phenyl)amino)-5-(trifluoromethyl)pyrimidin-4-yl) amino)-N-methylbenzamide (120 mg, 0.20 mmol), added with triethylamine (82.7 μL, 0.60 mmol) in ice bath, then added dropwise with acryloyl chloride (16.11 μL, 0.20 mmol), and allowed to react at room temperature for 10 minutes. Upon completion of the reaction, the reaction was added with water, extracted with DCM, dried and concentrated. The residue was purified by column chromatography to obtain 80 mg 2-(((2-((4-(((3-(1-acryloylpiperidin-3-yl)phenyl)amino)methyl)-2-methoxyphenyl)amino)-5-(trifluoromethyl)pyri midin-4-yl)amino)-N-methylbenzamide.

$^1H$ NMR (400 MHz, DMSO-$d_6$) δ 11.44 (d, *J* = 14.3 Hz, 1H), 8.75 (s, 1H), 8.68 (d, *J* = 4.5 Hz, 1H), 8.34 (s, 2H), 7.65 (d, *J* = 7.8 Hz, 1H), 7.51 (d, *J* = 8.0 Hz, 1H), 7.12 (d, *J* = 1.7 Hz, 2H), 6.99 (t, *J* = 7.8 Hz, 2H), 6.92 (d, *J* = 8.0 Hz, 1H), 6.55 (s, 1H), 6.45 (d, *J* = 7.6 Hz, 2H), 6.19 (s, 1H), 6.13 - 5.99 (m, 1H), 5.66 (d, *J* = 10.6 Hz, 1H), 5.58 (d, *J* = 10.7 Hz, 1H), 4.44 (d, *J* = 12.6 Hz, 1H), 4.26 (d, *J* = 5.9 Hz, 2H), 4.04 (m, 1H), 3.91 (d, *J* = 12.4 Hz, 1H), 3.76 (s, 3H), 3.04 (t, *J* = 12.7 Hz, 1H), 2.76 (d, *J* = 4.5 Hz, 3H), 2.69 - 2.54 (m, 1H), 1.89 - 1.55 (m, 4H). MS m/z (ESI): 660.3 $[M+H]^+$.

**Example 13**

**Preparation of 2-((2-((4-(((3-(1-acryloylpiperidin-3-yl)phenyl)amino)methyl)phenyl)amino)-5-(trifluoromethyl)pyrimidin-4 -yl)amino)-6 -fluoro-N methylbenzamide (13)**

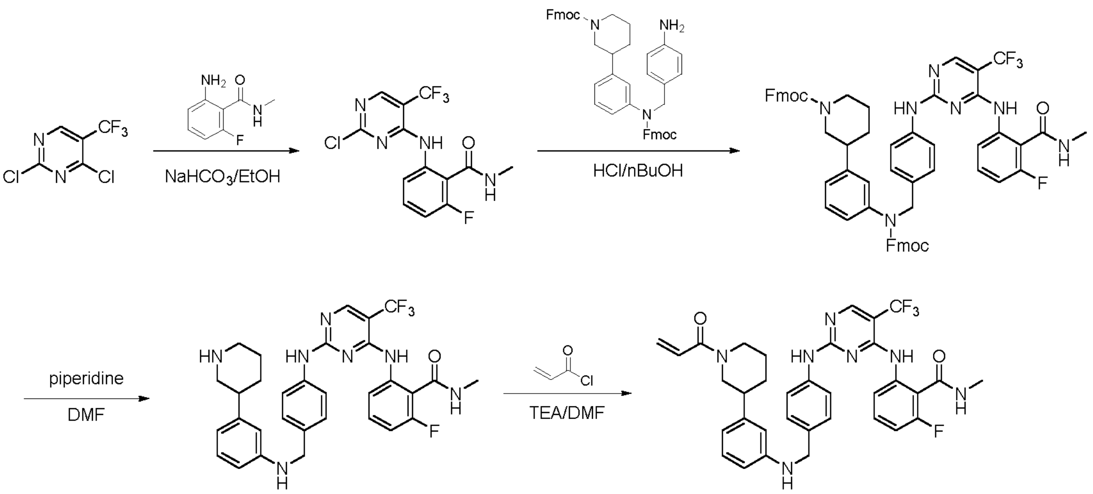


**Step 1: synthesis of 2-((2-chloro-5-(trifluoromethyl)pyrimidin-4-yl)amino)-6-fluoro-*N*-methylbenzamide**

In ethanol (10 mL) was dissolved 2,4-dichloro-5-(trifluoromethyl)pyrimidine (813 mg, 3.75mmol), added with NaHCOs (450mg, 5.36mmol), then added dropwise with 2-amino-6-fluoro-N-methylbenzamide (450mg, 2.68mmol) in ethanol, and allowed to react at room temperature overnight. Upon completion of the reaction, the reaction was added with water, extracted with DCM, dried and concentrated. The residue was purified by column chromatography to obtain 220mg 2-((2-chloro-5-(trifluoromethyl)pyrimidin-4-yl)amino)-6-fluoro-N-methylbenzamide, MS m/z (ESI): 349.0 $[M+H]^+$.

**Step 2: synthesis of (9H-fluoren-9-yl)methyl**

**3-(3-(((((9H-fluoren-9-yl)methoxy)carbonyl)(4-((4-((3-fluoro-2-(methylcarbamoyl))phenyl))amino)-5-(triflu oromethyl)pyrimidin-2-yl)amino)benzyl)amino)phenyl) piperidine-1-carboxylate**

In n-butanol (10 mL) were dissolved (9H-fluoren-9-yl)methyl 3-(3-(((((9H-fluoren-9-yl)methoxy)carbonyl)(4-aminobenzyl)amino)phenyl) piperidine-1-carboxylate (334 mg, 0.46 mmol) and 2-((2-chloro-5-(trifluoromethyl)pyrimidin-4-yl)amino)-6-fluoro-N-methylbenzamide (160 mg, 0.46 mmol), added with hydrochloric acid in dioxane (4 N, 34.5 μL), and allowed to react at 70°C overnight. Upon completion of the reaction, the mixture was concentrated. The residue was purified by column chromatography to obtain 450 mg (9H-fluoren-9-yl)methyl 3-(3-(((((9H-fluoren-9-yl)methoxy)carbonyl)(4-((4-((3-fluoro-2-(methylcarbamoyl))phenyl))amino)-5-(trifluorom ethyl)pyrimidin-2-yl)amino)benzyl)amino)phenyl) piperidine-1-carboxylate, MS m/z (ESI): 1038.4 $[M+H]^+$.

**Step 3: synthesis of**

**2-fluoro-N-methyl-6-((2-(((4-(((3-(piperidin-3-yl)phenyl)amino)methyl)phenyl)amino)-5-(trifluoromethyl)py rimidin-4-yl)amino)benzamide**

In DMF (7 mL) was dissolved (9H-fluoren-9-yl)methyl 3-(3-(((((9H-fluoren-9-yl)methoxy)carbonyl)(4-((4-((3-fluoro-2-(methylcarbamoyl))phenyl))amino)-5-(trifluorom ethyl)pyrimidin-2-yl)amino)benzyl)amino)phenyl) piperidine-1-carboxylate (450 mg, 0.43 mmol), added with piperidine (1.4 mL) in ice bath, and allowed to react at room temperature for 20 min. Upon completion of the reaction, the reaction was added with water, extracted with DCM, dried and concentrated. The residue was purified by column chromatography to obtain 160 mg N-methoxy-2-((2-((4-(((3-(piperidin-3-yl)phenyl)amino)methyl)phenyl)amino)-5-(trifluoromethyl)pyrimidin-4-yl)amino) benzamide, MS m/z (ESI): 594.3 $[M+H]^+$.

**Step 4: synthesis of 2-((2-((4-(((3-(1-acryloylpiperidin-3-yl)phenyl)amino)methyl)phenyl)amino)-5-(trifluoromethyl)pyrimidin-4 -yl)amino)-6 -fluoro-N-methylbenzamide**

In DMF (4 mL) was dissolved 2-fluoro-N-methyl-6-((2-(((4-(((3-(piperidin-3-yl)phenyl)atnino)methyl)phenyl)amino)-5-(trifluoromethyl)pyrimi din-4-yl)amino)benzamide (120mg, 0.20mmol), added with triethylamine (84μL, 0.60mmol) in ice bath, then added dropwise with acryloyl chloride (16 μL, 0.20 mmol), and allowed to react at room temperature for 10 minutes. Upon completion of the reaction, the reaction was added with water, extracted with DCM, dried and

concentrated. The residue was purified by column chromatography to obtain 40 mg 2-((2-((4-(((3 -(1 -acryloylpiperidin-3 -yl)phenyl)amino)methyl)phenyl)amino)-5 -(trifluoromethyl)pyrimidin-4-yl)a mino)-6 -fluoro-N-methylbenzamide.

$^1$H NMR (400 MHz, DMSO-$d_6$) δ 9.82 (s, 1H), 9.70 (s, 1H), 8.59 (d, *J* = 5.1 Hz, 1H), 8.42 (s, 1H), 7.76 - 7.62 (m, 1H), 7.53 (d, *J* = 7.9 Hz, 2H), 7.31 (s, 1H), 7.23 (d, *J* = 8.1 Hz, 2H), 7.08 - 6.91 (m, 2H), 6.51 (s, 1H), 6.43 (t, *J* = 7.0 Hz, 2H), 6.19 - 5.98 (m, 2H), 5.66 (d, *J* = 10.7 Hz, 1H), 5.59 (d, *J* = 10.6 Hz, 1H), 4.44 (m, 1H), 4.19 (d, *J* = 5.9 Hz, 2H), 4.06 (d, *J* = 13.8 Hz, 1H), 3.92 (d, *J* = 13.5 Hz, 1H), 3.04 (t, *J* = 12.8 Hz, 1H), 2.76 (d, *J* = 4.6 Hz, 3H), 2.60 (t, *J* = 12.1 Hz, 1H), 1.90 - 1.56 (m, 4H). MS m/z (ESI): 648.3 [M+H]$^+$.

**Example 14**

**Preparation of N-(3-(((2-((4-(((3-(1-acryloylpiperidin-3-yl)phenyl)amino)methyl)phenyl)amino)-5-(trifluoromethyl)pyrimid in-4-yl) amino) methyl) pyrazin-2-yl)-N-methylmethanesulfonamide (14)**

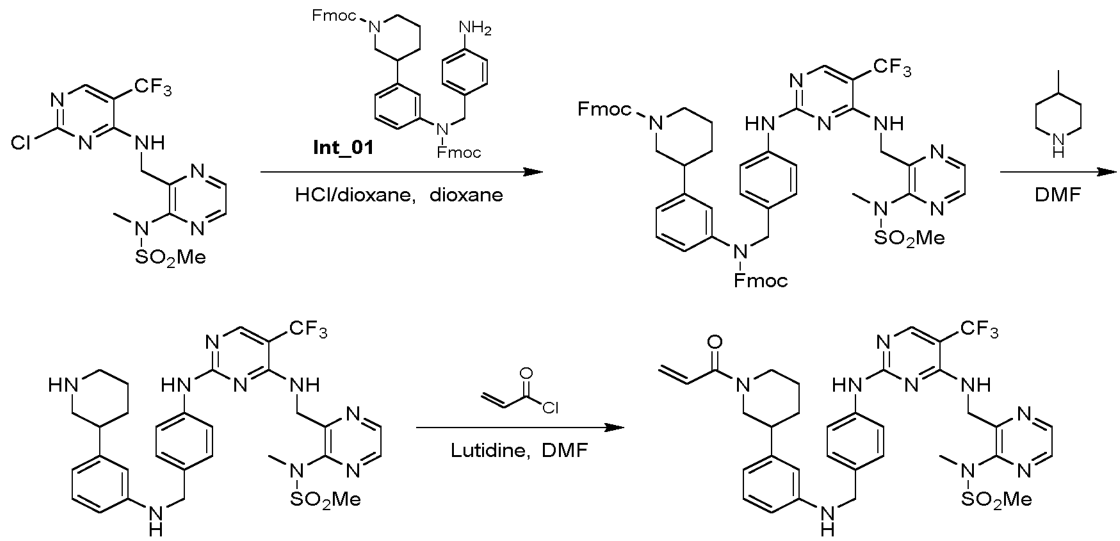


**Step 1: (9H-fluoren-9-yl)methyl 3-(3-((((9H-fluoren-9-yl)methoxy)carbonyl)(4-((4-(((3-(N-methylmethylsulfonamido)pyrazin- 2-yl)methyl) amino)-5-(trifluoromethyl) pyrimidin-2-yl)amino) benzyl) amino) phenyl) piperidine-1-carboxylate**

In 1,4-dioxane (12 ml) was dissolved N-(3-(((2-chloro-5-(trifluoromethyl)pyrimidin-4-yl)amino)methyl)pyrazin-2-yl)-N-methylmethanesulfonamide (380.51 mg, 0.96 mmol), added with (9H-fluoren-9-yl)methyl 3-(3-(((((9H-fluoren-9-yl)methoxy)carbonyl)(4-aminobenzyl)amino)phenyl) piperidine-1-carboxylate (663 mg, 0.91 mmol) and HCl/dioxane (68.50 μL), and allowed to react under nitrogen protection at 75°C for 16 hours. Upon completion of the reaction, the mixture was cooled to room temperature, concentrated under vacuum to obtain a residue, which was added with water (20mL), extracted with DCM (20mL×3). The organic phases were combined and washed with saturated brine, dried over $Na_2SO_4$ and concentrated to obtain a residue. The residue was purified by column chromatography (petroleum ether: ethyl acetate=1: 1) to obtain (9H-fluoren-9-yl)methyl 3-(3-((((9H-fluoren-9-yl)methoxy)carbonyl)(4-((4-(((3-(N-methylmethylsulfonamido)pyrazin-2-yl)methyl) amino)-5-(trifluoromethyl) pyrimidin-2-yl)amino) benzyl) amino) phenyl) piperidine-1-carboxylate (700 mg, 0.64 mmol, yield: 70.56%). MS m/z (ESI): 1087.1[M+H]$^+$.

**Step 2: N-methyl-N-(3-(((2-((4-(((3-(piperidin-3-yl)phenyl)amino)methyl)phenyl)amino)-5-(trifluoromethyl)pyrimid ine-4-)yl)amino) methyl) pyrazin-2-yl) methanesulfonamide**

In DMF (7ml) was dissolved (9H-fluoren-9-yl)methyl 3-(3-((((9H-fluoren-9-yl)methoxy)carbonyl)(4-((4-(((3-(N-methylmethylsulfonamido)pyrazin-2-yl)methyl)amino) -5-(trifluoromethyl)pyrimidin-2-yl)amino)benzyl)amino)phenyl)piperidine-1-carboxylate (700 mg, 0.64 mmol), added slowly with 4-methyl piperidine (1.4 mL) dropwise at 0°C, warmed to room temperature and stirred for 1h. Upon completion of the reaction indicated by TLC (petroleum ether: ethyl acetate = 1: 1), the reaction was diluted with DCM (30 mL), added with water (30 mL), and extracted with DCM (30 mL×3). The combined organic phases were dried over $Na_2SO_4$ and concentrated to obtain a residue, which was

purified by column chromatography (dichloromethane: methanol=10: 1), to obtain N-methyl-N-(3-(((2-((4-(((3-(piperidin-3-yl)phenyl)amino)methyl)phenyl)amino)-5-(trifluoromethyl)pyrimidine-4 -)yl)amino) methyl) pyrazin-2-yl) methanesulfonamide (335 mg, 0.52 mmol, yield: 81.00%). MS m/z (ESI): 642.3 $[M+H]^+$.

**Step 3:**

**N-(3-(((2-((4-(((3-(1-acryloylpiperidin-3-yl)phenyl)amino)methyl)phenyl)amino)-5-(trifluoromethyl)pyrimid in-4-yl) amino) methyl) pyrazin-2-yl)-N-methylmethanesulfonamide**

In DMF (30 ml) was added N-methyl-N-(3-(((2-((4-(((3-(piperidin-3-yl)phenyl)amino)methyl)phenyl)amino)-5-(trifluoromethyl)pyrimidine-4 -)yl)amino) methyl) pyrazin-2-yl) methanesulfonamide (300 mg, 0.47 mmol), added with DIEA(193.15 μL, 1.17 mmol), and then added slowly with acryloyl chloride (41.56 μL, 0.51 mmol) in DMF (1 mL) at 0°C dropwise, warmed to room temperature and stirred for 1 hour. Upon completion of the reaction, the reaction was diluted with DCM (30 mL), added with water (30 mL), and extracted with DCM (30 mL×3). The combined organic phases were dried over $Na_2SO_4$ and concentrated to obtain a residue. The residue was purified by column chromatography (petroleum ether: ethyl acetate=1: 2) to obtain N-(3-(((2-((4-(((3-(1-acryloylpiperidin-3-yl)phenyl)amino)methyl)phenyl)amino)-5-(trifluoromethyl)pyrimidin-4-yl)amino)methyl)pyrazin-2-yl)-N-methylmethanesulfonamide (186.1 mg, 0.26 mmol, yield: 56.32%).

$^1$H NMR (DMSO-$d_6$) δ: 9.55 (br s, 1H), 8.65 (d, *J*=2.2 Hz, 1H), 8.53 (d, *J*=2.2 Hz, 1H), 8.22 (s, 1H), 7.43 (br s, 2H), 7.30 (br s, 1H), 7.14 (br d, *J*=8.0 Hz, 2H), 6.95 (t, *J*=7.8 Hz, 1H), 6.69-6.86 (m, 1H), 6.47 (s, 1H), 6.35-6.43 (m, 2H), 5.99-6.11 (m, 2H), 5.62 (br dd, *J*=19.5, 10.8 Hz, 1H), 4.94 (br d, *J*=4.8 Hz, 2H), 4.38-4.49 (m, 1H), 4.11 (br d, *J*=5.4 Hz, 2H), 3.87-4.08 (m, 1H), 3.10-3.21 (m, 6H), 2.96-3.10 (m, 1H), 2.53-2.63 (m, 1H), 2.38-2.45 (m, 1H), 1.84 (br d, *J*=12.5 Hz, 1H), 1.56-1.75 (m, 2H), 1.29-1.49 (m, 1H). MS m/z (ESI): 696.3 $[M+H]^+$

**Example 15**

**Preparation of 2-(2-(4-((3-(1-acryloylpiperidin-3-yl)phenyl)amino)methyl)phenyl)amino)-5-chloropyrimidin-4-yl)amino)-N -methylbenzamide (15)**

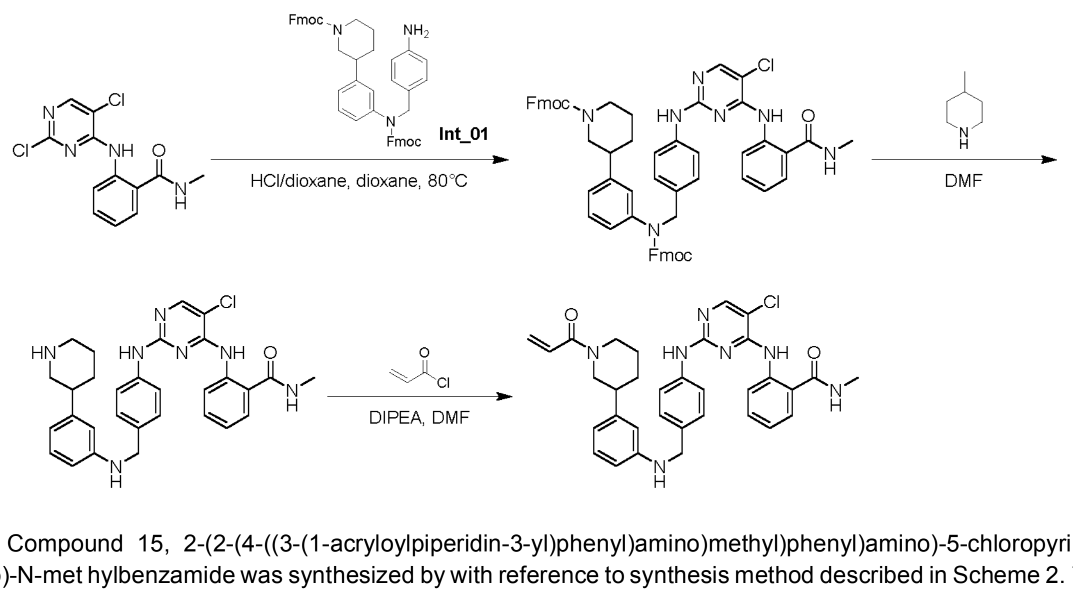


Compound 15, 2-(2-(4-((3-(1-acryloylpiperidin-3-yl)phenyl)amino)methyl)phenyl)amino)-5-chloropyrimidin-4-yl)amino)-N-met hylbenzamide was synthesized by with reference to synthesis method described in Scheme 2. $^1$H NMR (DMSO-$d_6$) δ: 11.60 (br d, J=6.8 Hz, 1H), 9.42 (s, 1H), 8.59-8.82 (m, 2H), 8.20 (s, 1H), 7.74 (d, J=7.6 Hz, 1H), 7.58 (d, J=8.4 Hz, 1H), 7.31-7.40 (m, 1H), 7.27 (d, J=8.5 Hz, 1H), 7.10 (br t, J=7.6 Hz, 1H), 6.98 (t, J=7.8 Hz, 1H), 6.67-6.87 (m, 1H), 6.52 (br s, 1H), 6.44 (br d, J=7.0 Hz, 1H), 6.11 (br s, 1H), 6.00-6.10 (m, 1H), 5.56-5.70 (m, 1H), 4.45 (br d, J=13.0 Hz, 1H), 4.20 (brd, J=5.5 Hz, 1H), 3.85-4.11 (m, 1H), 2.98-3.10 (m, 1H), 2.80 (d, J=4.5 Hz, 1H), 2.61 (br t, J=12.0 Hz, 1H), 2.38-2.45 (m, 1H), 1.86 (br d, J=13.1 Hz, 1H), 1.70-1.78 (m, 1H), 1.59-1.68 (m, 1H), 1.36-1.49 (m, 1H). **MS m/z (ESI):** 596.3 $[M+H]^+$.

**Example 16**

**Preparation of 2-((2-((4-(((3-(1-acryloylpiperidin-3-yl)phenyl)amino)methyl)phenyl)amino)-5-bromopyrimidin-4-yl)amino) -N-methylbenzamide (16)**

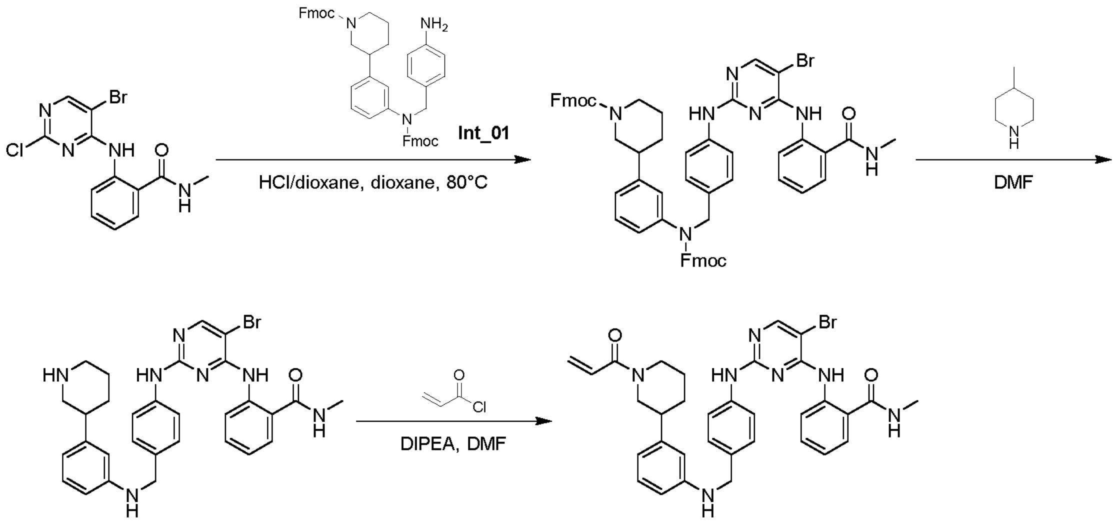


**Step 1: (9H-fluoren-9-yl)methyl 3-(3-((((9H-fluoren-9-yl)methoxy)carbonyl)(4-((5-bromo-4-((2-(methylcarbamoyl)phenyl)))amino)pyrimidi n-2-yl)amino)benzyl)amino)phenyl) piperidine-1-carboxylate**

In 1,4-dioxane (22ml) were dissolved 2-((5-bromo-2-chloropyrimidin-4-yl)amino)-N-methylbenzamide (750 mg, 2.20 mmol) and (9H-fluoren-9-yl)methyl 3-(3-(((((9H-fluoren-9-yl)methoxy)carbonyl)(4-aminobenzyl)amino)phenyl)piperidine-1-carboxylate (1.75 g, 2.42 mmol). The mixture was cooled to 0°C and added under stirring with 4M hydrochloric acid in dioxane (0.17 mL, 0.66 mmol) dropwise. After the addition, the mixture was heated to 80°C to react overnight. The reaction mixture was cooled to room temperature and concentrated under reduced pressure to remove solvent, and purified by column chromatography (ethyl acetate: petroleum ether=1/2) to obtain (9H-fluoren-9-yl)methyl 3-(3-((((9H-fluoren-9-yl)methoxy)carbonyl)(4-((5-bromo-4-((2-(methylcarbamoyl)phenyl)))amino)pyrimidin-2-yl )amino)benzyl)amino)phenyl) piperidine-1-carboxylate (450 mg, 0.44 mmol, 20.00%).

**Step 2: 2-((5-bromo-2-((4-(((3-(piperidin-3-yl)phenyl)amino)methyl)phenyl)amino)pyrimidin-4-yl)amino)-N-methyl benzamide**

In N,N-dimethylformamide (6 ml) was dissolved (9H-fluoren-9-yl)methyl 3-(3-((((9H-fluoren-9-yl)methoxy)carbonyl)(4-((5-bromo-4-((2-(methylcarbamoyl)phenyl)))amino)pyrimidin-2-yl )amino)benzyl)amino)phenyl) piperidine-1-carboxylate (450 mg, 0.44 mmol), cooled in ice bath and added with 4-methyl piperidine (1.2 mL) dropwise under stirring. After the addition, the reaction was warmed to room temperature and stirred for 2 h, added with 50 mL water, and extracted with ethyl acetate (50 mL×3). The organic phases were combined and washed with saturated sodium chloride solution (80 mL), dried over anhydrous sodium sulfate and filtered. The filtrate was concentrated under reduced pressure, and the residue was purified by column chromatography (methanol: dichloromethane=1/10) to obtain 2-((5-bromo-2-((4-(((3-(piperidin-3-yl)phenyl)amino)methyl)phenyl)amino)pyrimidin-4-yl)amino)-N-methylbenz amide (171 mg, 0.29 mmol, 66.26%). **MS m/z (ESI):** 587.3 $[M+H]^+$.

**Step 3: 2-((2-((4-(((3-(1-acryloylpiperidin-3-yl)phenyl)amino)methyl)phenyl)amino)-5-bromopyrimidin-4-yl)amino) -N-methylbenzamide**

In N,N-dimethylformamide (5 ml) were dissolved 2-((5-bromo-2-((4-(((3-(piperidin-3-yl)phenyl)amino)methyl)phenyl)amino)pyrimidin-4-yl)amino)-N-methylbenz amide (150 mg, 0.26 mmol) and N,N-diisopropylethylamine (0.11 mL, 0.78 mmol), cooled in ice bath and added with acryloyl chloride (25.5 mg, 0.29 mmol) in DMF (0.5 mL) dropwise under stirring. After the addition, the reaction was warmed to room temperature and stirred for 1 h, quenched with 30 mL water, and extracted with ethyl acetate (25 mL×3). The organic phases were combined and washed with saturated

sodium chloride solution (30 mL), dried over anhydrous sodium sulfate, and filtered. The filtrate was concentrated under reduced pressure, and the residue was purified by column chromatography (ethyl acetate: petroleum ether=4/1) to obtain 2-((2-((4-(((3-(1-acryloylpiperidin-3-yl)phenyl)amino)methyl)phenyl)amino)-5-bromopyrimidin-4-yl)amino)-N-m ethylbenzamide (31.1 mg, 0.05 mmol, 18.67%).

$^1$H NMR (DMSO-$d_6$) δ: 11.37 (br d, *J*=7.6 Hz, 1H), 9.43 (s, 1H), 8.73 (br q, *J*=4.5 Hz, 1H), 8.64 (br d, *J*=6.9 Hz, 1H), 8.27 (s,1H), 7.72 (d, *J*=7.6 Hz, 1H), 7.52-7.65 (m, *J*=8.4 Hz, 2H), 7.35 (br d, *J*=7.3 Hz, 1H), 7.22-7.30 (m, *J*=8.4 Hz, 2H), 7.10 (br t,*J*=7.3 Hz, 1H), 6.99 (t, *J*=7.8 Hz, 1H), 6.67-6.87 (m, 1H), 6.53 (br s, 1H), 6.44 (br d, *J*=7.1 Hz, 2H), 6.00-6.18 (m, 2H),5.55-5.72 (m, 1H), 4.45 (br d, *J*=11.5 Hz, 1H), 4.20 (br d, *J*=5.6 Hz, 2H), 4.07 (br d, *J*=13.4 Hz, 1H), 2.97-3.12 (m, 1H), 2.81(d, *J*=4.5 Hz, 3H), 2.52-2.70 (m, 1H), 2.38-2.49 (m, 1H), 1.86 (brd, *J*=12.9 Hz, 1H), 1.59-1.80 (m, 2H), 1.33-1.50 (m, 1H). MS m/z (ESI): 640.2 [M+H]$^+$.

**Example 17**

**Preparation of 2-((2-((4-(((3-(1-acryloylpiperidin-3-yl)phenyl)amino)methyl)phenyl)amino)-5-(trifluoromethyl)pyrimidin-4 -yl)amino)-N-ethylbenzamide (17)**

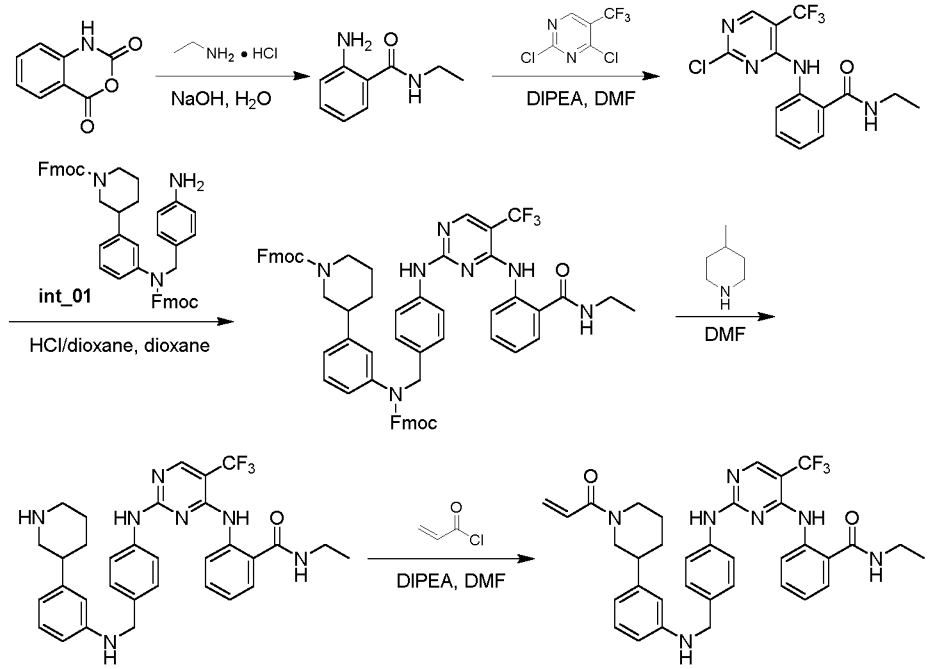


**Step 1: 2-amino-N-ethylbenzamide**

In water (60 ml) was dissolved NaOH (3.63 g, 90.83 mmol), added with ethyl amine hydrochloride (4.10 g, 90.83 mmol) at 0°C under nitrogen protection, and stirred for 20 minutes. The mixture was added with 2H-benzo[d][1,3] oxazin-2,4(1H)-dione (10 g, 60.55 mmol), and allowed to react at 0°C for 30 minutes. The reaction was allowed to warm to room temperature and react for 5 hours. Upon completion of the reaction, the reaction system was filtered, and the filter cake was washed with water (50 mL×3) and lyophilized to obtain the target compound 2-amino-N-ethylbenzamide (10 g, 60.89 mmol, yield: 99.34%). **MS m/z (ESI)**: 165.1 [M+H]$^+$.

**Step 2: 2-((2-chloro-5-(trifluoromethyl)pyrimidin-4-yl)amino)-N-ethylbenzamide**

In DMF (50 ml) were dissolved 2-amino-N-ethylbenzamide (3.78 g, 23.04 mmol) and 2,4-dichloro-5-(trifluoromethyl)pyrimidine (3.11 mL, 23.04 mmol), added under nitrogen protection with DIPEA (3.8mL, 23.04 mmol) at 0°C, and allowed to react at room temperature for 2 hours. The reaction was diluted with DCM (20 mL), added with water (50 mL), and extracted with DCM (50 mL×3). The combined organic phases were dried over $Na_2SO_4$ and concentrated to obtain a residue, which was purified by column chromatography (petroleum ether: ethyl acetate=1: 1) to obtain 2-((2-chloro-5-(trifluoromethyl)pyrimidin-4-yl)amino)-N-ethylbenzamide (5 g, 14.50 mmol, yield: 62.94%).

**Step 3: (9H-fluoren-9-yl)methyl 3-(3-((((9H-fluoren-9-yl)methoxy)carbonyl)(4-((4-((2-(ethylcarbamoyl)phenyl)amino)-)5-(trifluoromethyl)p yrimidin-2-yl)amino)benzyl)amino)phenyl)piperidine-1-carboxylate**

In 1,4-dioxane (4ml) was dissolved 2-((2-chloro-5-(trifluoromethyl)pyrimidin-4-yl)amino)-N-ethylbenzamide (249 mg, 0.72 mmol), added with (9H-fluoren-9-yl)methyl 3-(3-(((((9H-fluoren-9-yl)methoxy)carbonyl)(4-aminobenzyl)amino)phenyl) piperidine-1-carboxylate (500 mg, 0.69 mmol) and 4M hydrochloric acid in dioxane (52 μL), and allowed to react under nitrogen protection at 100°C for 16 hours. Upon completion of the reaction, the mixture was cooled to room temperature, concentrated under vacuum to obtain a residue, which was added with water (10mL) and extracted with DCM (20mL×3). The organic phases were combined and washed with saturated brine, dried over $Na_2SO_4$ and concentrated to obtain a residue, which was purified by column chromatography (petroleum ether: ethyl acetate=1: 1) to obtain (9H-fluoren-9-yl)methyl 3-(3-((((9H-fluoren-9-yl)methoxy)carbonyl)(4-((4-((2-(ethylcarbamoyl)phenyl)amino)-) 5-(trifluoromethyl)pyrimidin-2-yl)amino)benzyl)amino)phenyl) piperidine-1-carboxylate (190mg, 0.18 mmol, yield: 38%).

**Step 4: N-ethyl-2-((2-((4-(((3-(piperidin-3-yl)phenyl)amino)methyl)phenyl)amino)-5-(trifluoromethyl)pyrimidin-4-y l)amino)benzamide**

In DMF (5ml) was dissolved (9H-fluoren-9-yl)methyl 3-(3-((((9H-fluoren-9-yl)methoxy)carbonyl)(4-((4-((2-(ethylcarbamoyl)phenyl)amino)-)5-(trifluoromethyl)pyrimi din-2-yl)amino)benzyl)amino)phenyl)piperidine-1-carboxylate (500 mg, 0.48 mmol), added slowly with 4-methyl piperidine (1 mL) dropwise at 0°C, warmed to room temperature and stirred for 3 hours. Upon completion of the reaction indicated by TLC (petroleum ether: ethyl acetate = 1: 1), the reaction was diluted with DCM (5 mL), added with water (5 mL), and extracted with DCM (10 mL×3). The combined organic phases were dried over $Na_2SO_4$ and concentrated to obtain a residue, which was purified by column chromatography (dichloromethane: methanol = 10: 1) to obtain N-ethyl-2-((2-((4-(((3-(piperidin-3-yl)phenyl)amino)methyl)phenyl)amino)-5-(trifluoromethyl)pyrimidin-4-yl)ami no) benzamide (190 mg, 0.32 mmol, yield: 67.28%).

**Step 5: 2-((2-((4-(((3-(1-acryloylpiperidin-3-yl)phenyl)amino)methyl)phenyl)amino)-5-(trifluoromethyl)pyrimidin-4 -yl)amino)-N-ethylbenzamide**

In DMF (30ml) was dissolved N-ethyl-2-((2-((4-(((3-(piperidin-3-yl)phenyl)amino)methyl)phenyl)amino)-5-(trifluoromethyl)pyrimidin-4-yl)ami no) benzamide (150 mg, 0.25 mmol), added with DIEA (105.10 μL, 0.64 mmol), added slowly with acryloyl chloride (22.61 μL, 0.28 mmol) in DMF (3 mL) dropwise at 0°C, warmed to room temperature and stirred for 1 hour. Upon completion of the reaction, the reaction was diluted with DCM (30 mL), added with water (30 mL), and extracted with DCM (30 mL×3). The combined organic phases were dried over $Na_2SO_4$ and concentrated to obtain a residue, which was purified by column chromatography (petroleum ether: ethyl acetate = 1: 2) to obtain 2-((2-((4-(((3-(1-acryloylpiperidin-3-yl)phenyl)amino)methyl)phenyl)amino)-5-(trifluoromethyl)pyrimidin-4-yl)a mino)-N-ethylbenzamide (86 mg, 0.13 mmol, yield: 51.76%).

$^1H$ NMR (DMSO-$d_6$) δ: 11.14-11.30 (m, 1H), 9.82 (br s, 1H), 8.76 (t, *J*=5.4 Hz, 1H), 8.42 (s, 2H), 7.69 (d, *J*=7.8 Hz, 1H), 7.57 (br d, *J*=7.6 Hz, 2H), 7.30-7.43 (m, 1H), 7.26 (br d, *J*=8.5 Hz, 2H), 7.12 (br t, *J*=7.6 Hz, 1H), 7.01 (br t, *J*=7.6 Hz, 1H), 6.65-6.88 (m, 1H), 6.55 (br dd, *J*=4.5, 3.0 Hz, 1H), 6.37-6.51 (m, 2H), 6.01-6.12 (m, 1H), 5.57-5.69 (m, 1H), 4.45 (br d, *J*=13.3 Hz, 1H), 4.22 (s, 2H), 3.86-4.13 (m, 1H), 3.21-3.32 (m, 2H), 2.99-3.11 (m, 1H), 2.57-2.69 (m, 1H), 2.36-2.48 (m, 1H), 1.83-1.90 (m, 1H), 1.60-1.79 (m, 2H), 1.35-1.49 (m, 1H), 1.11 (t, *J*=7.2 Hz, 3H). **MS m/z (ESI):** 644.4 $[M+H]^+$.

**Example 18**

**Preparation of 2-((2-((4-(((3-(1-acryloylpiperidin-3-yl)phenyl)amino)methyl)phenyl)amino)-5-(trifluoromethyl)pyrimidin-4 -yl)amino)-N-isopropylbenzamide (18)**

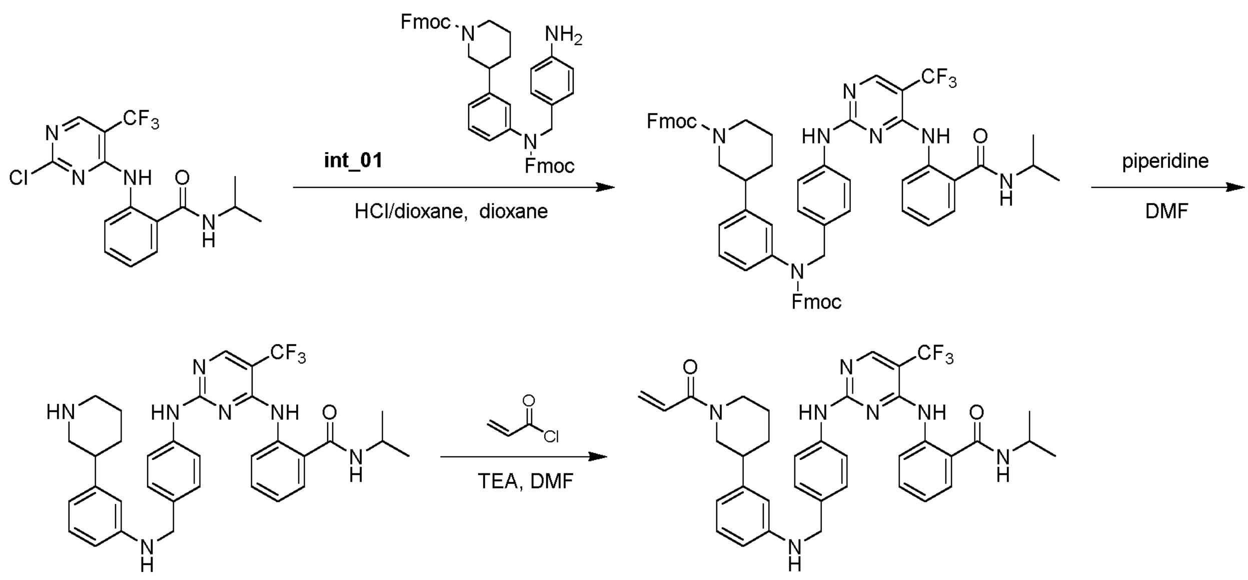


**Step 1: (9H-fluoren-9-yl)methyl 3-(3-((((9H-fluoren-9-yl)methoxy)carbonyl)(4-((4-((2-(isopropylcarbamoyl)phenyl)amino)-) 5-(trifluoromethyl)pyrimidin-2-yl)amino)benzyl)amino)phenyl) piperidine-1-carboxylate**

In 1,4-dioxane (13ml) was dissolved 2-((2-chloro-5-(trifluoromethyl)pyrimidin-4-yl)amino)-N-isopropylbenzamide (259.47 mg, 0.72 mmol), added with (9H-fluoren-9-yl)methyl 3-(3-(((((9H-fluoren-9-yl)methoxy)carbonyl)(4-aminobenzyl)amino)phenyl) piperidine-1-carboxylate (500 mg, 0.69 mmol) and HCl/dioxane (0.05 mL), and allowed to react under nitrogen protection at 80°C for 16 hours. Upon completion of the reaction, the mixture was cooled to room temperature, concentrated under vacuum to obtain a residue, which was added with water (20mL) and extracted with DCM (20mL×3). The organic phases were combined and washed with saturated brine, dried over $Na_2SO_4$ and concentrated to obtain a residue, which was purified by column chromatography (petroleum ether: ethyl acetate=1: 1) to obtain (9H-fluoren-9-yl)methyl 3-(3-((((9H-fluoren-9-yl)methoxy)carbonyl)(4-((4-((2-(isopropylcarbamoyl)phenyl)amino)-)

5-(trifluoromethyl)pyrimidin-2-yl)amino)benzyl)amino)phenyl) piperidine-1-carboxylate (630 mg, 0.60 mmol, yield: 87.26%). **MS m/z (ESI):** 1049.3[M+H]$^+$.

**Step 2: N-isopropyl-2-((2-((4-(((3-(piperidin-3-yl)phenyl)amino)methyl)phenyl)amino)-5-(trifluoromethyl)pyrimidi n-4-yl)amino) benzamide**

In DMF (10 ml) was dissolved (9H-fluoren-9-yl)methyl 3-(3-((((9H-fluoren-9-yl)methoxy)carbonyl)(4-((4-((2-(isopropylcarbamoyl)phenyl)amino)-)

5-(trifluoromethyl)pyrimidin-2-yl)amino)benzyl)amino)phenyl) piperidine-1-carboxylate (600 mg, 0.57 mmol), added slowly with piperidine(2 mL) dropwise at 0°C, warmed to room temperature and stirred for 1 hour. Upon completion of the reaction indicated by TLC (petroleum ether: ethyl acetate = 1: 1), the reaction was diluted with DCM (30 mL), added with water (30 mL) and extracted with DCM (30 mL×3). The combined organic phases were dried over $Na_2SO_4$ and concentrated to obtain a residue, which was purified by column chromatography (dichloromethane: methanol=10: 1) to obtain N-isopropyl-2-((2-((4-(((3-(piperidin-3-yl)phenyl)amino)methyl)phenyl)amino)-5-(trifluoromethyl)pyrimidin-4-yl )amino) benzamide (240 mg, 0.40 mmol, yield: 69.45%). **MS m/z (ESI):** 604.4 [M+H]$^+$.

**Step 3: 2-((2-((4-(((3-(1-acryloylpiperidin-3-yl)phenyl)amino)methyl)phenyl)amino)-5-(trifluoromethyl)pyrimidin-4 -yl)amino)-N-isopropylbenzamide**

In DMF (19 ml) was dissolved N-isopropyl-2-((2-((4-(((3-(piperidin-3-yl)phenyl)amino)methyl)phenyl)amino)-5-(trifluoromethyl)pyrimidin-4-yl )amino) benzamide (190 mg, 0.31 mmol), added with triethylamine (109.37 μL, 0.79 mmol), then added slowly with acryloyl chloride (27.98 μL, 0.35 mmol) in DMF (1 mL) dropwise at 0°C, warmed to room temperature and stirred for 1 hour. Upon completion of the reaction, the reaction was diluted with DCM (30 mL), added with water (30 mL) and extracted with DCM (30 mL×3). The combined organic phases were dried over $Na_2SO_4$ and concentrated to obtain a residue, which was purified by column chromatography (petroleum ether: ethyl acetate=1: 2) to obtain 2-((2-((4-(((3-(1-acryloylpiperidin-3-yl)phenyl)amino)methyl)phenyl)amino)-5-(trifluoromethyl)pyrimidin-4-yl)amino)-N-isopropylbenzamide (66.4 mg, 0.10 mmol, yield: 31.84%).

$^1H$ NMR (DMSO-$d_6$) δ: 11.05 (br s, 1H), 9.81 (br s, 1H), 8.51 (br d, *J*=7.5 Hz, 1H), 8.37-8.46 (s, 1H), 7.68 (br d, *J*=7.5 Hz, 1H), 7.57 (br d, *J*=6.6 Hz, 2H), 7.31 (m, 1H), 7.25 (br d, *J*=7.9 Hz, 2H), 7.12 (br t, *J*=7.1 Hz, 1H), 6.99 (br t, *J*=7.8 Hz, 1H), 6.65-6.91 (m, 1H), 6.52 (br s, 1H), 6.44 (br d, *J*=6.6 Hz, 2H), 6.02-6.21 (m, 2H), 5.57-5.71 (m, 1H), 4.45 (br d, *J*=11.4 Hz, 1H), 4.21 (br d, *J*=5.3 Hz, 2H), 3.99-4.14 (m, 1H), 3.93 (br d, *J*=13.1 Hz, 1H), 3.05 (br t, *J*=12.4 Hz, 1H), 2.56-2.66 (m, 1H), 2.43 (m, 1H), 1.86 (m, 1H), 1.60-1.80 (m, 2H), 1.43 (m, 1H), 1.15 (br d, *J*=6.5 Hz, 6H). **MS m/z (ESI):** 658.4 $[M+H]^+$.

**Example** 19

**Preparation of 2-((2-((4-(((3-(1-acryloylpiperidin-3-yl)phenyl)amino)methyl) phenyl) amino)-5-(trifluoromethyl) pyrimidin-4-yl)amino)-N-isopropylbenzenesulfonamide (19)**

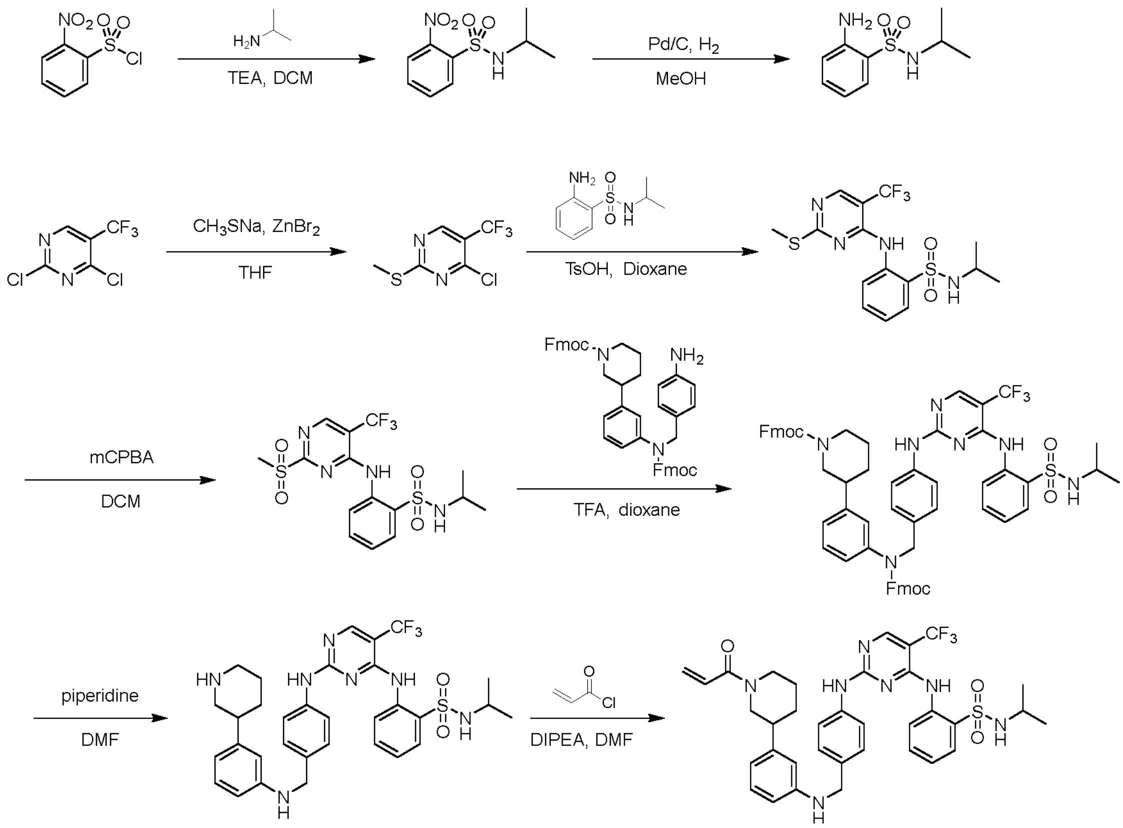


Step **1: N-isopropyl-2-nitrobenzenesulfonamide**

Into a round bottom flask was added 2-nitrobenzenesulfonyl chloride (11 g, 49.64 mmol), added with dichloromethane (100 mL) as solvent, and then added slowly with 2-propanamine (3.5 g, 59.21 mmol) and triethylamine (9.59 mL, 69.18 mmol), and stirred at room temperature for 4 hours. Upon completion of the reaction, the mixture was added with water and extracted with ethyl acetate. The organic phases were combined, dried over anhydrous sodium sulfate, and filtered. The filtrate was concentrated by rotary evaporation, and the residue was purified by silica gel column chromatography to obtain N-isopropyl-2-nitrobenzenesulfonamide (12 g, yield: 98.98%). MS m/z (ESI): 245.0 $[M+H]^+$.

Step **2: 2-amino-N-isopropylbenzenesulfonamide**

In methanol (100 ml) was dissolved N-isopropyl-2-nitrobenzenesulfonamide (7 g, 28.66 mmol), then added with Pd/C (10%) (1.4 g, 13.16 mmol). The atmosphere was replaced with hydrogen for three times and kept under hydrogen protection, and stirred overnight. Upon completion of the reaction, the mixture was filtered through filter paper and the filtrate was concentrated by rotary evaporation to obtain 2-amino-N-isopropylbenzenesulfonamide (5.6 g, yield: 91.19%). MS m/z (ESI): 215.0 $[M+H]^+$.

Step **3: 4-chloro-2-(methylthio)-5-(trifluoromethyl)pyrimidine**

In THF (150 mL) were dissolved2,4-dichloro-5-(trifluoromethyl)pyrimidine (10 g, 46 mmol), zinc bromide (18 g, 78 mmol). The atmosphere was replaced with nitrogen for three times and kept under nitrogen protection. The mixture was stirred in ice bath for 2 hours, and then added with sodium methylsulfide (3.4 g, 48 mmol) while kept in ice bath, stirred at room temperature overnight. Upon completion of the reaction, the mixture was evaporated by rotary evaporation to remove most of THF, added with water and extracted with dichloromethane. The organic phases were combined, dried over anhydrous sodium sulfate, and filtered. The filtrate was concentrated by rotary evaporation, and the residue was purified by silica gel column chromatography to obtain 4-chloro-2-(methylthio)-5-(trifluoromethyl)pyrimidine (10 g, yield: 95%).

Step **4: N-isopropyl-2-((2-(methylthio)-5-(trifluoromethyl)pyrimidin-4-yl)amino)benzenesulfonamide**

In dioxane (20 mL) was dissolved 4-chloro-2-(methylthio)-5-(trifluoromethyl)pyrimidine (693 mg, 3.03 mmol), then added with 2-amino-N-isopropylbenzenesulfonamide (500 mg, 2.33 mmol) and TsOH (521.98 mg, 3.03 mmol). The atmosphere was replaced with nitrogen for three times and kept under nitrogen protection, and the mixture was refluxed with stirring at 100°C overnight. Upon completion of the reaction, the mixture was added with water and extracted with dichloromethane. The organic phases were combined, dried over anhydrous sodium sulfate, and filtered. The filtrate was concentrated by rotary evaporation, and the residue was purified by silica gel column chromatography to obtain N-isopropyl-2-((2-(methylthio)-5-(trifluoromethyl)pyrimidin-4-yl)amino)benzenesulfonamide (192.7 mg, yield: 15.64%). MS m/z (ESI): 407.1 $[M+H]^+$.

Step **5: N-isopropyl-2-((2-(methylsulfonyl)-5-(trifluoromethyl)pyrimidin-4-yl)amino)benzenesulfonamide**

In DCM (10 mL) was dissolved N-isopropyl-2-((2-(methylthio)-5-(trifluoromethyl)pyrimidin-4-yl)amino)benzenesulfonamide (192 mg, 0.47 mmol). The atmosphere was replaced with nitrogen for three times and kept under nitrogen protection. The reaction mixture was added with mCPBA (163.03 mg, 0.94 mmol) in batch and cooled in ice bath, stirred at room temperature for 1 hour. Upon completion of the reaction, the mixture was quenched by adding saturated sodium thiosulfate aqueous solution, and then washed with saturated sodium bicarbonate aqueous solution and saturated brine, and extracted with dichloromethane. The organic phases were combined, dried over anhydrous sodium sulfate, and filtered. The filtrate was concentrated by rotary evaporation, and the residue was purified by silica gel column chromatography to obtain N-isopropyl-2-((2-(methylsulfonyl)-5-(trifluoromethyl)pyrimidin-4-yl)amino)benzenesulfonamide (178 mg, yield: 85.94%). MS m/z (ESI): 439.1 $[M+H]^+$.

Step **6: methyl (9H-fluoren-9-yl)methyl3-(((9H-fluoren-9-yl)methoxy)carbonyl)(4-((2-(N-isopropylsulfoamido)phenyl)amin o)-5-(trifluoromethyl)pyrimidin-2-yl)amino)benzyl)aminophenyl)piperidine-1-carboxylate**

In dioxane (3 mL) were dissolved N-isopropyl-2-((2-(methylsulfonyl)-5-(trifluoromethyl)pyrimidin-4-yl)amino)benzenesulfonamide (168 mg, 0.38 mmol), methyl (9H-fluoren-9-yl)3-(3-(((9H-fluoren-9-yl) methoxy) carbonyl) (4-aminobenzyl) amino) phenyl) piperidine-1-carboxylate (278.14 mg, 0.38 mmol), and TFA (0.29 mL, 3.83 mmol). The atmosphere was replaced with nitrogen for three times, and the reaction was allowed to proceed under nitrogen protection at 100°C, with refluxing and stirring overnight. Upon completion of the reaction, the mixture was extracted with dichloromethane and water. The organic phases were combined, dried over anhydrous sodium sulfate, and filtered. The filtrate was concentrated by rotary evaporation, and the residue was purified by silica gel column chromatography to obtain methyl (9H-fluoren-9-yl)methyl 3-(((9H-fluoren-9-yl)methoxy)carbonyl)(4-((2-(N-isopropylsulfoamido)phenyl)amino)-5-(trifluoromethyl)pyrimid in-2-yl)amino)benzyl)aminophenyl)piperidine-1-carboxylate (292 mg, yield: 70.29%). MS m/z (ESI): 1085.0 $[M+H]^+$.

Step **7: N-isopropyl-2-((2-((4-(((3-(piperidin-3-yl)phenyl)amino)methyl)phenyl)amino)-5-(trifluoromethyl)pyrimidi n-4-yl)amino)benzenesulfonamide**

In N,N-dimethylformamide (2 mL) was dissolved methyl (9H-fluoren-9-yl)methyl3-(((9H-fluoren-9-yl)methoxy)carbonyl)(4-((2-(N-isopropylsulfoamido)phenyl)amino)-5-(trifluoromethyl)pyrimidin-2-yl)amino)benzyl)aminophenyl)piperidine-1-carboxylate (292 mg, 0.27 mmol). The atmosphere was replaced with nitrogen for three times, and the reaction was cooled in ice bath and added with piperidine (0.4 mL) under nitrogen protection, stirred at room temperature for 1 hour. Upon completion of the reaction, the mixture was extracted with dichloromethane and water. The organic phases were combined, dried over anhydrous sodium sulfate, and filtered. The filtrate was concentrated by rotary evaporation, and the residue was purified by silica gel column chromatography to obtain N-isopropyl-2-((2-((4-(((3-(piperidin-3-yl)phenyl)amino)methyl)phenyl)amino)-5-(trifluoromethyl)pyrimidin-4-yl )amino)benzenesulfonamide (83.4 mg, yield: 48.41%). MS m/z (ESI): 640.3 [M+H]$^+$.

Step **8: 2-((2-((4-(((3-(1-acryloylpiperidin-3-yl)phenyl)amino)methyl)phenyl)amino)-5-(trifluoromethyl)pyrimidin-4 -yl)amino)-N-isopropylbenzenesulfonamide**

In DMF (3 mL) was dissolved N-isopropyl-2-((2-((4-(((3-(piperidin-3-yl)phenyl)amino)methyl)phenyl)amino)-5-(trifluoromethyl)pyrimidin-4-yl )amino)benzenesulfonamide (78 mg, 0.12 mmol). The mixture was cooled in ice bath and added with DIPEA (0.05 mL, 0.30 mmol) and acryloyl chloride (0.01 mL, 0.13 mmol), and stirred in ice bath for 0.5 hours. Upon completion of the reaction, the mixture was extracted with dichloromethane and water. The organic phases were combined, dried over anhydrous sodium sulfate, and filtered. The filtrate was concentrated by rotary evaporation, and the residue was purified by silica gel column chromatography to obtain 2-((2-((4-(((3-(1-acryloylpiperidin-3-yl)phenyl)amino)methyl)phenyl)amino)-5-(trifluoromethyl)pyrimidin-4-yl)a mino)-N-isopropylbenzenesulfonamide(49mg, yield: 57.93%).

$^1$H NMR (DMSO-d$_6$, 400 MHz) $\delta$: 9.87 (br s, 1H), 8.92 (br s, 1H), 8.48 (s, 1H), 8.13 (br s, 1H), 7.84 (d, 1H, J=7.4 Hz), 7.79 (br s, 1H), 7.50 (br s, 3H), 7.29 (br t, 1H, J=7.5 Hz), 7.17 (br d, 2H, J=6.3 Hz), 6.98 (t, 1H, J=7.8 Hz), 6.7-6.9 (m, 1H), 6.50 (s, 1H), 6.43 (br t, 2H, J=8.1 Hz), 6.0-6.1 (m, 2H), 5.6-5.7 (m, 1H), 4.45 (br d, 1H, J=12.3 Hz), 4.17 (br d, 2H, J=5.8 Hz), 3.9-4.1 (m, 1H), 3.27 (br dd, 1H, J=6.3, 12.6 Hz), 3.0-3.1 (m, 1H), 2.60 (br t, 1H, J=12.1 Hz), 2.4-2.5 (m, 1H), 1.86 (br d, 1H, J=12.3 Hz), 1.6-1.8 (m, 2H), 1.4-1.5 (m, 1H), 0.89 (br d, 6H, J=6.4 Hz). MS m/z (ESI): 694.4 [M+H]$^+$.

**Example 20**

**Preparation of 7-((2-((4-(((3-(1-acryloylpiperidin-3-yl)phenyl)amino)methyl)phenyl)amino)-5-(trifluoromethyl)pyrimidin-4 -yl)amino)-2-methylisoindolin-1-one (20)**

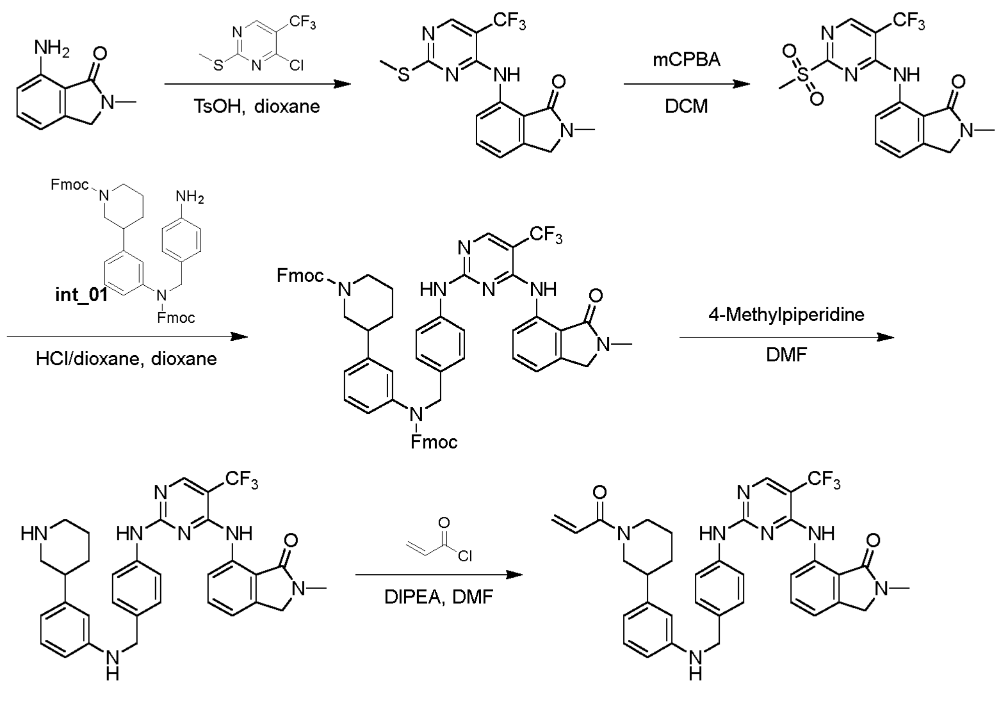


**Step 1: 2-methyl-7-((2-(methylthio)-5-(trifluoromethyl)pyrimidin-4-yl)amino)isoindolin-1-one**

In 1,4-dioxane (36 ml) was dissolved 7-amino-2-methylisoindolin-1-one (1.0 g, 6.17 mmol), added with 4-chloro-2-(methylthio)-5-(trifluoromethyl)pyrimidine (1.83 g, 8.02 mmol) and TsOH (1.17 g, 6.17 mmol), and stirred at 90°C overnight. Upon completion of the reaction, the mixture was cooled to room temperature, concentrated under vacuum to obtain a residue, which was purified by column chromatography (petroleum ether: ethyl acetate=1: 1) to obtain intermediate 2-methyl-7-((2-(methylthio)-5-(trifluoromethyl)pyrimidin-4-yl)amino)isoindolin-1-one (570 mg, 1.60 mmol, yield: 26.09%). **MS m/z (ESI):** 355.1 [M+H]$^+$.

**Step 2: 2-methyl-7-((2-(methylsulfonyl)-5-(trifluoromethyl)pyrimidin-4-yl)amino)isoindolin-1-one**

In dichloromethane (20 ml) was dissolved 2-methyl-7-((2-(methylthio)-5-(trifluoromethyl)pyrimidin-4-yl)amino)isoindolin-1-one (570 mg, 1.61 mmol), added with m-chloroperoxy benzoic acid (653 mg, 3.22 mmol) at 0°C, and stirred under nitrogen protection at room temperature for 1 hour. Upon completion of the reaction, the reaction was quenched by adding saturated sodium thiosulfate solution, added with water (20mL) and extracted with DCM (20mL×3). The organic phases were combined and washed with saturated brine, dried over $Na_2SO_4$ and concentrated to obtain a residue, which was purified by column chromatography (petroleum ether: ethyl acetate=1: 1) to obtain 2-methyl-7-((2-(methylsulfonyl)-5-(trifluoromethyl)pyrimidin-4-yl)amino)isoindolin-1-one (540 mg, 1.40 mmol, yield: 86.89%). **MS m/z (ESI):** 387.5[M+H]$^+$.

**Step 3: (9H-fluoren-9-yl)methyl 3-(3-((((9H-fluoren-9-yl)methoxy)carbonyl)(4-((4-((2-methyl-3-oxoisoindolin-4-))yl)amino)-5-(trifluorometh yl) pyrimidin-2-yl)amino) benzyl) amino) phenyl) piperidine-1-carboxylate**

In 1,4-dioxane (20 ml) was dissolved 2-methyl-7-((2-(methylsulfonyl)-5-(trifluoromethyl)pyrimidin-4-yl)amino)isoindolin-1-one (300 mg, 0.78 mmol), added with (9H-fluoren-9-yl)methyl 3-(3-(((((9H-fluoren-9-yl)methoxy)carbonyl)(4-aminobenzyl)amino)phenyl) piperidine-1-carboxylate (563 mg, 0.78 mmol) and trifluoroacetic acid (0.6 mL, 7.76 mmol), and allowed to react under nitrogen protection at 100°C overnight. Upon completion of the reaction, the mixture was cooled to room temperature, concentrated under vacuum to obtain a residue, which was added with water (20mL), and extracted with DCM (20mL×3). The organic phases were combined and washed with saturated brine, dried over $Na_2SO_4$ and concentrated to obtain a residue, which was purified by column chromatography (petroleum ether: ethyl acetate=1: 1) to obtain (9H-fluoren-9-yl)methyl 3-(3-((((9H-fluoren-9-yl)methoxy)carbonyl)(4-((4-((2-methyl-3-oxoisoindolin-4-))yl)amino)-5-(trifluoromethyl) pyrimidin-2-yl)amino) benzyl) amino) phenyl) piperidine-1-carboxylate (640 mg, 0.62 mmol, yield: 79.88%). **MS m/z (ESI):** 1032.7[M+H]$^+$.

**Step 4: 2-methyl-7-((2-((4-(((3-(piperidin-3-yl)phenyl)amino)methyl)phenyl)amino)-5-(trifluoromethyl)pyrimidin-4 -yl)amino) isoindolin-1-one**

In DMF (12 ml) was dissolved (9H-fluoren-9-yl)methyl 3-(3-((((9H-fluoren-9-yl)methoxy)carbonyl)(4-((4-((2-methyl-3-oxoisoindofin-4-))yl)amino)-5-(trifluoromethyl)p yrimidin-2-yl)amino)benzyl)amino)phenyl)piperidine-1-carboxylate (640 mg, 0.62 mmol), added slowly with 4-methyl piperidine(2.4 mL) dropwise at 0°C, warmed to room temperature and stirred for 1 hour. Upon completion of the reaction indicated by TLC (petroleum ether: ethyl acetate = 1: 1), the reaction was diluted with DCM (30 mL), added with water (30 mL) and extracted with DCM (30 mL×3). The combined organic phases were dried over $Na_2SO_4$ and concentrated to obtain a residue, which was purified by column chromatography (dichloromethane: methanol=10: 1) to obtain 2-methyl-7-((2-((4-(((3-(piperidin-3-yl)phenyl)amino)methyl)phenyl)amino)-5-(trifluoromethyl)pyrimidin-4-yl)a mino) isoindolin-1-one (360 mg, 0.61 mmol, yield: 98.80%). **MS m/z (ESI):** 588.2 [M+H]$^+$.

**Step 5: 7-((2-((4-(((3-(1-acryloylpiperidin-3-yl)phenyl)amino)methyl)phenyl)amino)-5-(trifluoromethyl)pyrimidin-4 -yl)amino)-2 -methylisoindolin-1-one**

In DMF (30 ml) was dissolved 2-methyl-7-((2-((4-(((3-(piperidin-3-yl)phenyl)amino)methyl)phenyl)amino)-5-(trifluoromethyl)pyrimidin-4-yl)a mino) isoindolin-1-one (320 mg, 0.54 mmol), added slowly at 0°C with acryloyl chloride (48.7 μL, 0.60 mmol) in DMF (3 mL) dropwise, warmed to room temperature and stirred for 1 hour. Upon completion of the reaction, the reaction was diluted with DCM (30 mL), added with water (30 mL) and extracted with DCM (30 mL×3). The combined organic phases were dried over $Na_2SO_4$ and concentrated to obtain a residue, which was purified by column chromatography (petroleum ether: ethyl acetate=1: 2) to obtain 7-((2-((4-(((3-(1-acryloylpiperidin-3-yl)phenyl)amino)methyl)phenyl)amino)-5-(trifluoromethyl)pyrimidin-4-yl)a mino)-2 -methylisoindolin-1-one (83.84 mg, 0.13 mmol, yield: 23.76%).

$^1$H NMR (DMSO-$d_6$) δ: 10.59 (br s, 1H), 9.90 (s, 1H), 8.46 (s, 1H), 7.58 (br s, 2H), 7.35 (br d, J=8.1 Hz, 2H), 7.16 (br s, 1H), 7.00 (t, J=7.8 Hz, 1H), 6.62-6.88 (m, 1H), 6.49-6.57 (m, 1H), 6.45 (br d, J=6.9 Hz, 2H), 6.18 (br s, 1H), 5.98-6.14 (m, 1H), 5.51-5.72 (m, 1H), 4.47 (s, 2H), 4.43 (br s, 1H), 4.26 (br d, J=5.4 Hz, 2H), 3.83-4.15 (m, 1H), 3.07 (s, 3H), 3.00-3.06 (m, 1H), 2.55-2.69 (m, 1H), 2.37-2.48 (m, 1H), 1.78-1.92 (m, 1H), 1.56-1.78 (m, 2H), 1.29-1.51 (m, 1H). **MS m/z (ESI):** 642.7 [M+H]$^+$.

**Example 21**

**Preparation of 7-((2-((4-(((3-(1-acryloylpiperidin-3-yl)phenyl)amino)methyl)phenyl)amino)-5-(trifluoromethyl)pyrimidin-4 -yl)amino)-2 -methylisoindolin-1-one (21)**

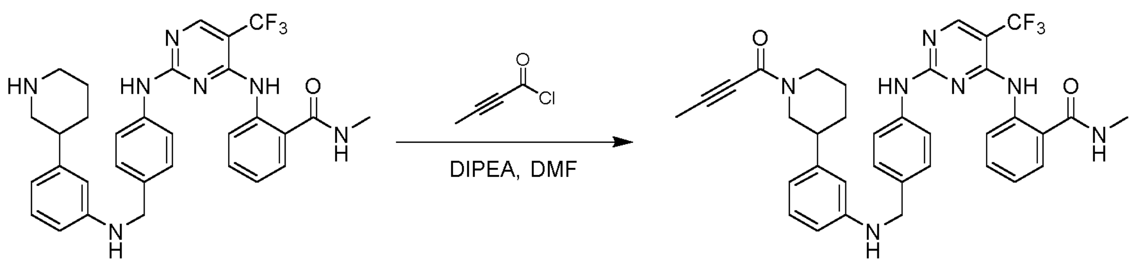


**Step: 2-((2-((4-(((3-(1-(2-butynoyl)piperidin-3-yl)phenyl)amino)methyl)phenyl)amino)-5-(trifluoromethyl)pyrimi din-4-yl)amino)-N-methylbenzamide**

In N,N-dimethylformamide (4 ml) were dissolved N-methyl-2-((2-((4-(((3-(piperidin-3-yl)phenyl)amino)methyl)phenyl)amino)-5-(trifluoromethyl)pyrimidin-4-yl)a mino) benzamide (100 mg, 0.17 mmol) and N,N-diisopropylethylamine (76 μL, 0.43 mmol), cooled in ice bath and added with butynoyl chloride (22.7 mg, 0.22 mmol) in DMF (0.5 mL) dropwise under stirring. After the addition, the reaction was warmed to room temperature and stirred for 1 h, and then quenched by adding 30 mL water, and extracted with ethyl acetate (25 mL×3). The organic phases were combined and washed with saturated sodium chloride solution (30 mL), dried over anhydrous sodium sulfate, and filtered. The filtrate was concentrated under reduced pressure, and the residue was purified by column chromatography (ethyl acetate: petroleum ether=4/1) to obtain 2-((2-((4-(((3-(1-(2-butynoyl)piperidin-3-yl)phenyl)atnino)methyl)phenyl)amino)-5-(trifluoromethyl)pyrimidin-4-yl)amino)-N-methylbenzamide (28.5 mg, 0.04 mmol, yield: 26.13%).

$^1$H NMR (DMSO-$d_6$) δ: 11.35 (br s, 1H), 9.81 (br s, 1H), 8.73 (br d, *J*=4.4 Hz, 1H), 8.42 (s, 2H), 7.70 (br d, *J*=7.9 Hz, 1H),7.51-7.64 (m, 2H), 7.22-7.42 (m, 3H), 7.12 (br t, *J*=7.4 Hz, 1H), 6.96-7.03 (m, 1H), 6.53 (br d, *J*=4.8 Hz, 1H), 6.40-6.48 (m,2H), 6.06-6.22 (m, 1H), 4.25-4.37 (m, 1H), 4.18-4.25 (m, 3H), 3.06-3.20 (m, 1H), 2.78 (d, *J*=4.5 Hz, 3H), 2.58-2.73 (m, 1H),2.32-2.44 (m, 1H), 1.92-2.06 (m, 3H), 1.69-1.88 (m, 2H), 1.59-1.69 (m, 1H), 1.33-1.53 (m, 1H). **MS m/z (ESI):** 642.7 [M+H]$^+$.

**Example 24**

**Preparation of 2-((2-((4-(((3-(1-methylacryloylpiperidin-3-yl)phenyl)amino)methyl)phenyl)amino)-5-(trifluoromethyl) pyrimidin-4-yl)amino)-N-methylbenzamide (24)**

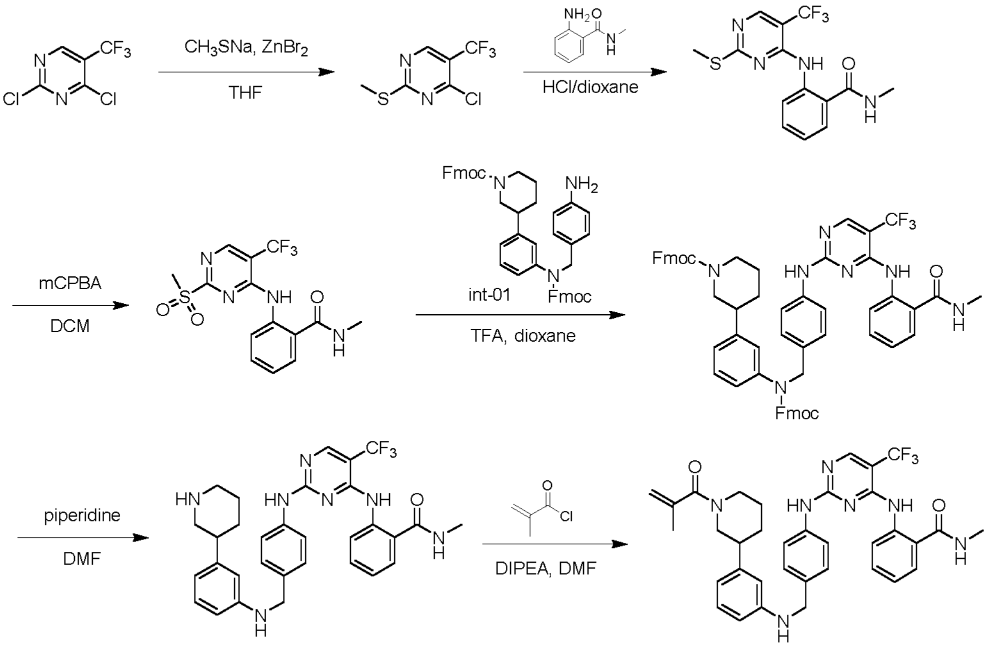


**Step 1: 4-chloro-2-(methylthio)-5-(trifluoromethyl)pyrimidine**

In THF (150 mL) were dissolved 2,4-dichloro-5-(trifluoromethyl)pyrimidine (10 g, 46 mmol and zinc bromide (18 g, 78 mmol). The atmosphere was replaced with nitrogen for three times and kept under nitrogen protection. The mixture was stirred in ice bath for 2 hours, then added with Sodium thiomethoxide (3.4 g, 48 mmol) in ice bath and stirred at room temperature overnight. Upon completion of the reaction, the mixture was evaporated by rotary evaporation to remove most of THF, added with water and extracted with dichloromethane. The organic phases were combined, dried over anhydrous sodium sulfate, and filtered. The filtrate was concentrated by rotary evaporation, and the residue was purified by silica gel column chromatography to obtain 4-chloro-2-(methylthio)-5-(trifluoromethyl)pyrimidine (10 g, yield: 95%).

**Step 2: N-methyl-2-((2-(methylthio)-5-(trifluoromethyl)pyrimidin-4-yl)amino)benzamide**

In dioxane (100 mL) were dissolved 4-chloro-2-(methylthio)-5-(trifluoromethyl)pyrimidine (9 g, 39 mmol), 2-amino-N-methylbenzamide (4.5 g, 30 mmol), and hydrochloric acid in dioxane (2.2 mL, 9 mmol). The atmosphere was replaced with nitrogen for three times and kept under nitrogen protection. The reaction was refluxed at 100°C under stirring overnight. Upon completion of the reaction, the mixture was evaporated by rotary evaporation to remove most of solvent, added with water and extracted with dichloromethane. The organic phases were combined, dried over anhydrous sodium sulfate, and filtered. The filtrate was concentrated by rotary evaporation, and the residue was purified by silica gel column chromatography to obtain N-methyl-2-((2-(methylthio)-5-(trifluoromethyl)pyrimidin-4-yl)amino)benzamide (6 g, yield: 60%). MS m/z (ESI): 343.1 $[M+H]^+$.

**Step 3: N-methyl-2-((2-(methylsulfonyl)-5-(trifluoromethyl)pyrimidin-4-yl)amino)benzamide**

In DCM (40 mL) was dissolved N-methyl-2-((2-(methylthio)-5-(trifluoromethyl)pyrimidin-4-yl)amino)benzamide (620 mg, 1.8 mmol). The atmosphere was replaced with nitrogen for three times and kept under nitrogen protection. The mixture was added with mCPBA (735 mg, 3.6 mmol) in ice bath in batch, stirred at room temperature for 1 hour. Upon completion of the reaction, the reaction was quenched by adding saturated sodium thiosulfate aqueous solution, and then washed with saturated sodium bicarbonate aqueous solution and saturated brine, and then extracted with dichloromethane. The organic phases were combined, dried over anhydrous sodium sulfate, and filtered. The filtrate was concentrated by rotary evaporation, and the residue was purified by silica gel column chromatography to obtain N-methyl-2-((2-(methylsulfonyl)-5-(trifluoromethyl)pyrimidin-4-yl)amino)benzamide (400 mg, yield: 59%). MS m/z (ESI): 375.05 $[M+H]^+$.

**Step 4: methyl (9H-fluoren-9-yl)3-(3-(((9H-fluoren-9-yl)methoxy)carbonyl)(4-((2-(methylcarbamoyl)phenyl)amino)-5-(trifl uoromethyl)pyrimidin-2-yl)amino)benzyl)aminophenyl)piperidine-1-carboxylate**

In dioxane (100 mL) were dissolved N-methyl-2-((2-(methylsulfonyl)-5-(trifluoromethyl)pyrimidin-4-yl)amino)benzamide (1.9 g, 5.1 mmol), methyl (9H-fluoren-9-yl)3-(3-(((9H-fluoren-9-yl) methoxy) carbonyl) (4-aminobenzyl) amino) phenyl) piperidine-1-carboxylate (3.7 g, 5.1 mmol), and TFA (3.8 mL, 51 mmol). The atmosphere was replaced with nitrogen for three times and refluxed under nitrogen protection at 100°C with stirring overnight. Upon completion of the reaction, the mixture was extracted with dichloromethane and water. The organic phases were combined, dried over anhydrous sodium sulfate, and filtered. The filtrate was concentrated by rotary evaporation, and the residue was purified by silica gel column chromatography to obtain methyl (9H-fluoren-9-yl)3-(3-(((9H-fluoren-9-yl)methoxy)carbonyl)(4-((2-(methylcarbamoyl)phenyl)amino)-5-(trifluoro methyl)pyrimidin-2-yl)amino)benzyl)aminophenyl)piperidine-1-carboxylate (5 g, yield: 96%).

**Step 5: N-methyl-2-((2-((4-(((3-(piperidin-3-yl)phenyl) amino) methyl) phenyl) amino)-5-(trifluoromethyl) pyrimidin-4-yl)amino) benzamide**

In N,N-dimethylformamide (30 mL) was dissolved methyl (9H-fluoren-9-yl)3-(3-(((9H-fluoren-9-yl) methoxy) carbonyl)
(4-((2-(methylcarbamoyl)phenyl)amino)-5-(trifluoromethyl)pyrimidin-2-yl)amino)benzyl)aminophenyl)piperidine -1-carboxylate (5 g, 4.9 mmol). The atmosphere was replaced with nitrogen for three times, and the reaction was added with piperidine (6 mL) under nitrogen protection in ice bath, stirred at room temperature for 1 hour. Upon completion of the reaction, the mixture was extracted with dichloromethane and water. The organic phases were combined, dried over anhydrous sodium sulfate, and filtered. The filtrate was concentrated by rotary evaporation, and the residue was purified by silica gel column chromatography to obtain N-methyl-2-((2-((4-(((3-(piperidin-3-yl)phenyl)amino)methyl)phenyl)amino)-5-(trifluoromethyl)pyrimidin-4-yl)a mino)benzamide (1.85 g, yield: 66%). MS m/z (ESI): 576.3 $[M+H]^+$.

**Step 6: 2-((2-((4-(((3-(1-methylacryloylpiperidin-3-yl)phenyl)amino)methyl)phenyl)amino)-5-(trifluoromethyl)pyri midin-4-yl)amino)-N-methylbenzamide**

In DMF (20 mL) was dissolved N-methyl-2-((2-((4-(((3-(piperidin-3-yl)phenyl) amino) methyl) phenyl) amino)-5-(trifluoromethyl) pyrimidin-4-yl)amino) benzamide (200 mg, 0.35 mmol). The mixture was cooled in ice bath and added with DIPEA (113 mg, 0.86 mmol), 2-methylacryloyl chloride (39.7 mg, 0.38 mmol), and allowed to react in ice bath with stirring for 2 hours. Upon completion of the reaction, the mixture was extracted with dichloromethane and water. The organic phases were combined, dried over anhydrous sodium sulfate, and filtered. The filtrate was concentrated by rotary evaporation, and the residue was purified by silica gel column chromatography to obtain 2-((2-((4-(((3 -(1 -methylacryloylpiperidin-3 -yl)phenyl)amino)methyl)phenyl)amino)-5 -(trifluoromethyl)pyrimidin-4-yl)amino)-N-methylbenzamide (81.9 mg, yield: 36%).

$^1$H NMR (DMSO-$d_6$, 400 MHz) δ: 11.36 (br s, 1H), 9.81 (br s, 1H), 8.72 (br d, 1H, J=4.5 Hz), 8.42 (s, 2H), 7.70 (d, 1H, J=7.9 Hz), 7.57 (br d, 2H, J=7.8 Hz), 7.26 (br d, 3H, J=8.3 Hz), 7.10 (br s, 1H), 6.99 (t, 1H, J=7.8 Hz), 6.45 (br d, 3H, J=7.9 Hz), 6.17 (br s, 1H), 5.0-5.2 (m, 1H), 4.94 (br s, 1H), 4.37 (br s, 1H), 4.21 (s, 2H), 3.7-3.9 (m, 1H), 2.98 (br s, 1H), 2.78 (d, 3H, J=4.4 Hz), 2.60 (br s, 1H), 2.45 (br t, 1H, J=11.1 Hz), 1.6-1.9 (m, 6H), 1.43 (br d, 1H, J=11.3 Hz).MS m/z (ESI): 644.4 $[M+H]^+$.

**Example 30**

**Preparation of 2-((2-((4-(((3-(1-acryloylpiperidin-3-yl)phenyl)amino)methyl)phenyl)amino)-5-(trifluoromethyl)pyrimidin-4 -yl)amino)-N-methylbenzenesulfonamide (30)**

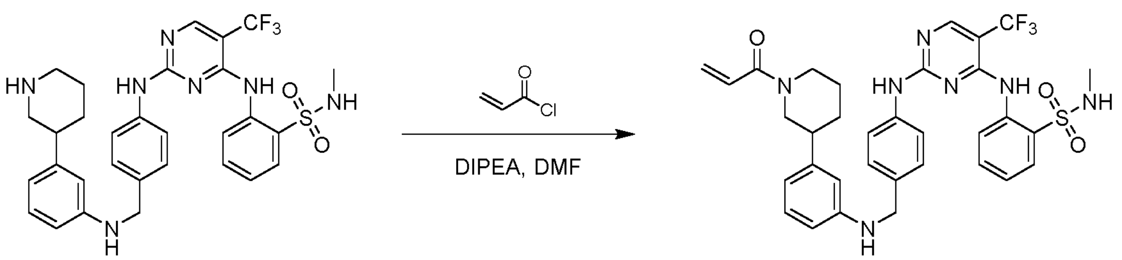


**Step: 2-((2-((4-(((3-(1-acryloylpiperidin-3-yl)phenyl)amino)methyl)phenyl)amino)-5-(trifluoromethyl)pyrimidin-4-yl)amino)-N-methylbenzenesulfonamide**

In N,N-dimethylformamide (3 ml) were dissolved N-methyl-2-((2-((4-(((3-(piperidin-3-yl)phenyl)amino)methyl)phenyl)amino)-5-(trifluoromethyl)pyrimidin-4-yl)a mino) benzenesulfonamide (100 mg, 0.16 mmol) and N,N-diisopropylethylamine (73 μL, 0.41 mmol), added with acryloyl chloride (17.8 mg, 0.20 mmol) in DMF (0.5 mL) in ice bath dropwise under stirring. After the addition, the reaction was warmed to room temperature and stirred for 1 h, quenched with 30 mL water, and extracted with ethyl acetate (25 mL×3). The organic phases were combined and washed with saturated sodium chloride solution (30 mL), dried over anhydrous sodium sulfate, and filtered. The filtrate was concentrated under reduced pressure, and the residue was purified by column chromatography (ethyl acetate: petroleum ether=4/1) to obtain 2-((2-((4-(((3 -(1 -acryloylpiperidin-3 -yl)phenyl)amino)methyl)phenyl)amino)-5 -(trifluoromethyl)pyrimidin-4-yl)a mino)-N-methylbenzenesulfonamide (36.0 mg, 0.05 mmol, yield: 32.40%).

$^1$H NMR (DMSO-$d_6$) δ: 9.78 (br s, 1H), 8.92 (br s, 1H), 8.40 (s, 1H), 8.13 (br s, 1H), 7.73 (d, *J*=7.9 Hz, 1H), 7.57 (br s, 1H), 7.35-7.53 (m, 3H), 7.25 (t, *J*=7.6 Hz, 1H), 7.13 (br d, *J*=7.1 Hz, 2H), 6.92 (t, *J*=7.8 Hz, 1H), 6.60-6.83 (m, 1H), 6.44 (s, 1H), 6.36 (br t, *J*=7.0 Hz, 2H), 5.95-6.08 (m, 2H), 5.50-5.64 (m, 1H), 4.38 (br d, *J*=11.5 Hz, 1H), 4.11 (br d, *J*=5.8 Hz, 2H), 3.80-4.06 (m, 1H), 2.86-3.09 (m, 1H), 2.54 (br t, *J*=12.1 Hz, 1H), 2.30-2.41 (m, 4H), 1.74-1.84 (m, 1H), 1.52-1.71 (m, 2H), 1.36 (br d, *J*=11.0 Hz, 1H). **MS m/z (ESI):** 666.3 [M+H]$^+$.

**Example 31**

**Preparation of N-methyl-2-((2-((4-(((3-(1-propionypiperidin-3-yl)phenyl)amino)methyl)phenyl)amino)-5-(trifluoromethyl) pyrimidin-4-yl)amino)benzamide (31)**

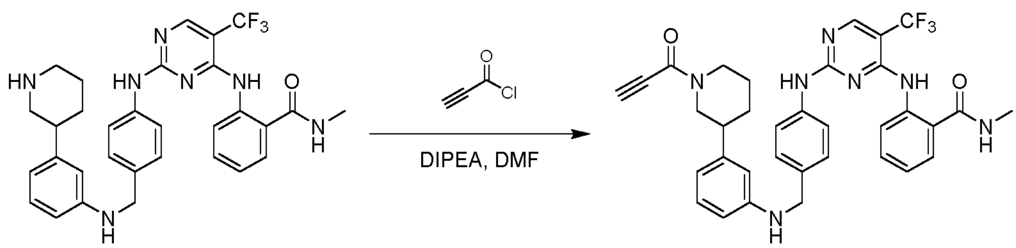


**Step: N-methyl-2-((2-((4-(((3-(1-propionypiperidin-3-yl)phenyl)amino)methyl)phenyl)amino)-5-(trifluoromethyl) pyrimidin-4-yl)amino)benzamide**

In N,N-dimethylformamide (5 ml) were dissolved N-methyl-2-((2-((4-(((3-(piperidin-3-yl)phenyl)amino)methyl)phenyl)amino)-5-(trifluoromethyl)pyrimidin-4-yl)a mino) benzamide (120 mg, 0.21 mmol) and N,N-diisopropylethylamine (109μL, 0.63 mmol). The mixture was cooled in ice bath and added with butynoyl chloride (17.6 mg, 0.25 mmol) solution in DMF (0.5 mL) dropwise under stirring. After the addition, the reaction was warmed back to room temperature and stirred for 1 h. the reaction was quenched by adding 30 mL water, and extracted with ethyl acetate (25 mL×3). The organic phases were combined and washed with saturated sodium chloride solution (30 mL), dried over anhydrous sodium sulfate, and filtered. The filtrate was concentrated under reduced pressure, and the residue was purified by column chromatography (ethyl acetate: petroleum ether=4/1) to obtain N-methyl-2-((2-((4-(((3-(1-propionypiperidin-3-yl)phenyl)amino)methyl)phenyl)amino)-5-(trifluoromethyl)pyrim idin-4-yl)amino)benzamide (53.4 mg, 0.09 mmol, yield: 40.90%).

$^1$H NMR (DMSO-$d_6$) δ: 11.35 (br s, 1H), 9.81 (br s, 1H), 8.73 (br d, *J*=4.6 Hz, 1H), 8.36-8.55 (m, 2H), 7.70 (d,

*J*=7.9 Hz,1H), 7.57 (br d, *J*=7.6 Hz, 2H), 7.22-7.40 (m, 3H), 7.12 (t, *J*=7.5 Hz, 1H), 6.99 (td, *J*=7.8, 4.4 Hz, 1H), 6.53 (br d, *J*=5.6 Hz,1H), 6.40-6.49 (m, 2H), 6.10-6.19 (m, 1H), 4.46-4.56 (m, 1H), 4.22-4.37 (m, 2H), 4.21 (br d, *J*=5.6 Hz, 2H), *3.07-3.28* (*m,*1H)*, 2.78* (d, *J*=4.5 Hz, 3H)*, 2.63-2.75* (m, 1H)*,* 2.36-2.48 (m, 1H), 1.87 (br d, *J*=13.3 Hz, 1H), 1.61-1.80 (m, 2H), 1.35-1.55(m, 1H). **MS m/z (ESI):** 628.3 [M+H]+.

**Example 32**

**Preparation of (E)-2-((2-((4-(((3-(1-(but-2-enoyl)piperidin-3-yl)phenyl)amino)methyl)phenyl)amino)-5-(trifluoromethyl)py rimidin-4-yl)amino)-N-methylbenzamide (32)**

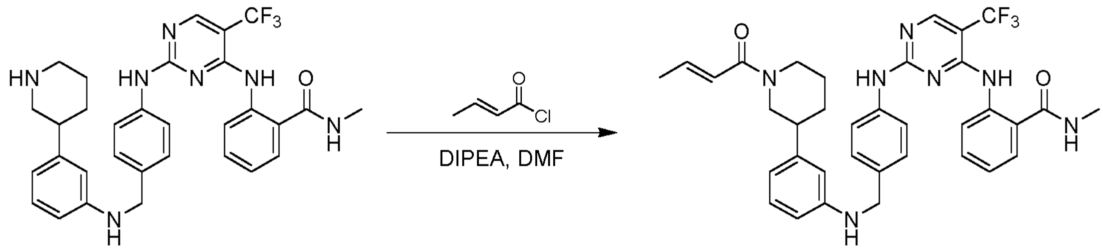


**Step: (E)-2-((2-((4-(((3-(1-(but-2-enoyl)piperidin-3-yl)phenyl)amino)methyl)phenyl)amino)-5-(trifluoromethyl)py rimidin-4-yl)amino)-N-methylbenzamide**

Into N,N-dimethylformamide (4ml) were dissolved N-methyl-2-((2-((4-(((3-(piperidin-3-yl)phenyl)amino)methyl)phenyl)amino)-5-(trifluoromethyl)pyrimidin-4-yl)a mino) benzamide (100 mg, 0.17 mmol) and N,N-diisopropylethylamine (76 μL, 0.43 mmol), added with butenoyl chloride (20.0 mg, 0.19 mmol) in DMF (0.5 mL) dropwise in ice bath under stirring. After the addition, the reaction was warmed to room temperature and stirred for 1 h, quenched by adding 30 mL water, and extracted with ethyl acetate (25 mL×3). The organic phases were combined and washed with saturated sodium chloride solution (30 mL), dried over anhydrous sodium sulfate, and filtered. The filtrate was concentrated under reduced pressure, and the residue was purified by column chromatography (ethyl acetate: petroleum ether=4/1) to obtain (E)-2-((2-((4-(((3-(1-(but-2-enoyl)piperidin-3-yl)phenyl)amino)methyl)phenyl)amino)-5-(trifluoromethyl)pyrimid in-4-yl)amino)-N-methylbenzamide (60.7 mg, 0.09 mmol, yield: 55.47%).

$^1$H NMR (DMSO-$d_6$) δ: 11.35 (br s, 1H), 9.81 (br s, 1H), 8.73 (br d, *J*=4.5 Hz, 1H), 8.42 (s, 2H), 7.70 (d, *J*=7.9 Hz, 1H), 7.57(br d, *J*=7.5 Hz, 2H), 7.32 (br s, 1H), 7.26 (br d, *J*=8.3 Hz, 2H), 7.12 (t, *J*=7.5 Hz, 1H), 6.99 (t, *J*=7.8 Hz, 1H), 6.64 (br d,*J*=5.6 Hz, 1H), 6.52 (br s, 1H), 6.43 (br d, *J*=7.0 Hz, 2H), 6.12 (br t, *J*=5.8 Hz, 1H), 4.45 (br s, 1H), 4.21 (br d, *J*=5.6 Hz, 2H),3.90-4.15 (m, 1H), 3.03 (q, *J*=12.8 Hz, 1H), 2.78 (d, *J*=4.5 Hz, 3H), 2.52-2.61 (m, 1H), 2.41 (br d, *J*=9.8 Hz, 1H), 1.70-1.90(m, 5H), 1.58-1.69 (m, 1H), 1.41 (br d, *J*=10.8 Hz, 1H). **MS m/z (ESI):** 644.4 [M+H]+.

**Example 33**

**Preparation** of **2-((2-((4-(((3-(1-(2-fluoroacryloyl)piperidin-3-yl)phenyl)amino)methyl)phenyl)amino)-5-(trifluoromethyl)py rimidin-4-yl)amino)-N-methylbenzamide (33)**

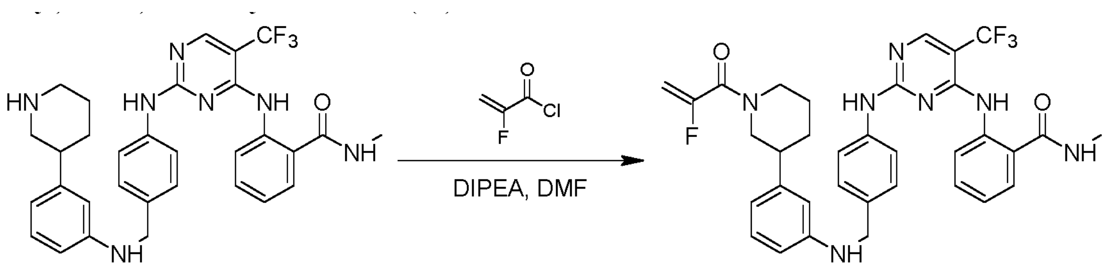


In N,N-dimethylformamide (5ml) were dissolved N-methyl-2-((2-((4-(((3-(piperidin-3-yl)phenyl)amino)methyl)phenyl)amino)-5-(trifluoromethyl)pyrimidin-4-yl)a mino) benzamide (150 mg, 0.26 mmol) and *N*,*N*-diisopropylethylamine (0.11 mL, 0.78 mmol), added with 2-fluoroacryloyl chloride (31.3 mg, 0.29 mmol) in DMF (0.5 mL) in ice bath dropwise under stirring. After the addition, the reaction was warmed to room temperature and stirred for 1 h, quenched by adding 30 mL water, and extracted with ethyl acetate (25 mL×3). The organic phases were combined and washed with saturated sodium chloride solution (30 mL), dried over anhydrous sodium sulfate, and filtered. The filtrate was concentrated under reduced pressure, and the residue was purified by column chromatography (ethyl acetate: petroleum ether=4/1) to obtain

2-((2-((4-(((3-(1-acryloylpiperidin-3-yl)phenyl)amino)methyl)phenyl)amino)-5-bromopyrimidin-4-yl)amino)-N-m ethylbenzamide (30.5 mg, 0.05 mmol, 18.11%).

$^1$H NMR (DMSO-$d_6$) δ: 11.37 (br s, 1H), 9.84 (br s, 1H), 8.73 (br d, *J*=4.5 Hz, 1H), 8.43 (s, 2H), 7.65-7.76 (m, 1H), 7.58 (brd, *J*=7.6 Hz, 2H), 7.33 (br s, 1H), 7.27 (br d, *J*=8.3 Hz, 2H), 7.12 (br t, *J*=7.5 Hz, 1H), 7.05 (br t, *J*=7.7 Hz, 1H), 6.50-6.66 (m,3H), 5.03-5.29 (m, 4H), 4.25 (br s, 2H), 3.90 (br s, 1H), 3.02-3.20 (m, 1H), 2.78 (d, *J*=4.5 Hz, 3H), 2.72 (br s, 1H), 1.61-2.05(m, 4H), 1.40-1.56 (m, 1H). **MS m/z (ESI):** 648.7 $[M+H]^+$.

**Example 34**

**Preparation of (R)-2-((2-((4-(((3-(1-acryloylpiperidin-3-yl)phenyl)amino)methyl)phenyl)amino)-5-(trifluoromethyl)pyrimid in-4-yl)amino)-N-methylbenzamide (34)**

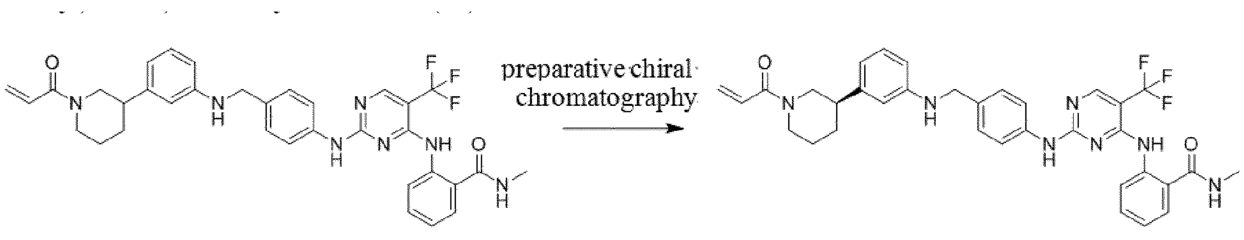


**7.9 g compound obtained in Example 6 was subjected to separation by preparative chiral chromatography, to obtain the product 3.6g, with HPLC purity 100%, chiral purity 100% (ee%).**

$^1$H NMR (DMSO-d6) δ: 11.36 (br s, 1H), 9.81 (br s, 1H), 8.73 (br d, J=4.4 Hz, 1H), 8.37-8.59 (m, 2H), 7.71 (d, J=7.8 Hz, 1H), 7.58 (br d, J=7.8 Hz, 2H), 7.20-7.42 (m, 3H), 7.12 (br t, J=7.5 Hz, 1H), 7.00 (t, J=7.8 Hz, 1H), 6.67-6.90 (m, 1H), 6.53 (br s, 1H), 6.45 (br d, J=7.4 Hz, 2H), 6.00-6.20 (m, 2H), 5.58-5.70 (m, 1H), 4.46 (br d, J=12.0 Hz, 1H), 4.22 (br s, 2H), 4.07 (br d, J=13.3 Hz, 1H), 3.05 (br t, J=12.5 Hz, 1H), 2.79 (d, J=4.5 Hz, 3H), 2.55-2.70 (m, 1H), 2.39-2.49 (m, 1H), 1.87 (br d, J=13.3 Hz, 1H), 1.60-1.77 (m, 2H), 1.34-1.50 (m, 1H)

**Example 35**

**Preparation of (S)-2-((2-((4-(((3-(1-acryloylpiperidin-3-yl)phenyl)amino)methyl)phenyl)amino)-5-(trifluoromethyl)pyrimid in-4-yl)amino)-N-methylbenzamide (35)**

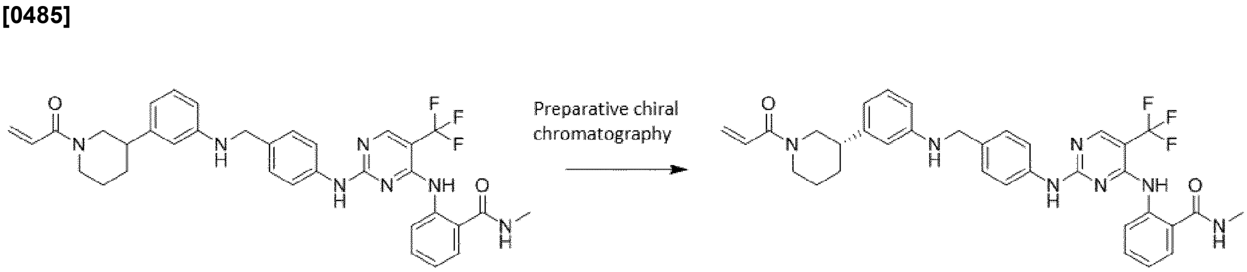


**7.9 g compound obtained in Example 6 was subjected to separation by preparative chiral chromatography, to obtain the product 2.9g, with HPLC purity 99.8%, chiral purity 99.82% (ee%).**

$^1$H NMR (DMSO-d6) δ: 11.36 (br s, 1H), 9.81 (br s, 1H), 8.73 (brd, J=4.4 Hz, 1H), 8.35-8.59 (m, 2H), 7.71 (d, J=7.9 Hz, 1H), 7.58 (br d, J=7.6 Hz, 2H), 7.33 (br s, 1H), 7.27 (br d, J=8.3 Hz, 2H), 7.12 (br t, J=7.5 Hz, 1H), 7.00 (t, J=7.8 Hz, 1H), 6.68-6.89 (m, 1H), 6.53 (br s, 1H), 6.45 (br d, J=7.4 Hz, 2H), 6.01-6.17 (m, 2H), 5.58-5.70 (m, 1H), 4.46

(br d, J=11.9 Hz, 1H), 4.21 (br s, 2H), 4.08 (br d, J=12.6 Hz, 1H), 3.05 (br t, J=12.6 Hz, 1H), 2.79 (d, J=4.4 Hz, 3H), 2.57-2.66 (m, 1H), 2.39-2.49 (m, 1H), 1.87 (br d, J=12.3 Hz, 1H), 1.60-1.79 (m, 2H), 1.35-1.50 (m, 1H)

**Example 36**

**Preparation of (R) 2-((2-((4-(((3-(1-acryloylpiperidin-3-yl)phenyl)amino)methyl)phenyl)amino)-5-(trifluoromethyl)pyrimidin-4 -yl)amino)-N-methoxybenzamide (36)**

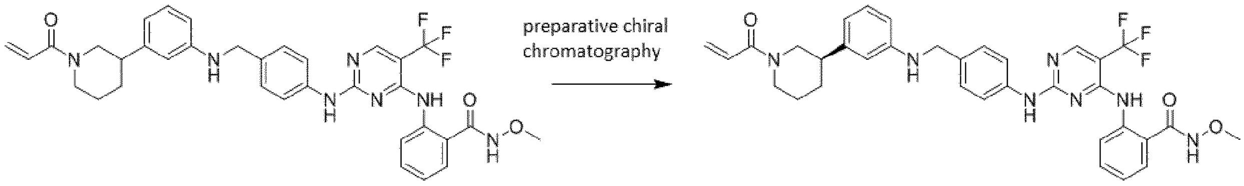


About 120mg of compound obtained in Example 11 was subjected to separation by preparative chiral chromatography, to obtain product 43.3mg, with HPLC purity 99.2%, chiral purity 100% (ee%). MS m/z (ESI): 646.5 [M+H]$^+$.

**Example 37**

**Preparation of (S) 2-((2-((4-(((3-(1-acryloylpiperidin-3-yl)phenyl)amino)methyl)phenyl)amino)-5-(trifluoromethyl)pyrimidin-4 -yl)amino)-N-methoxybenzamide (37)**

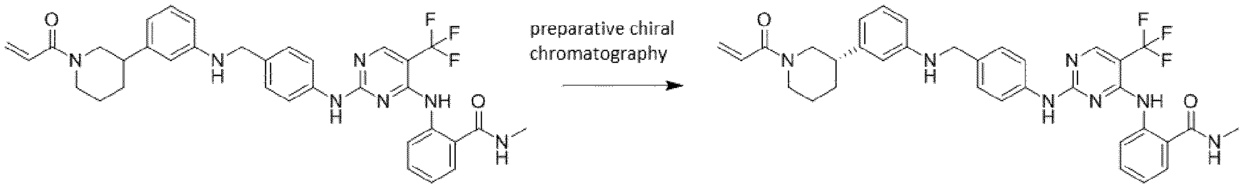


About 120mg of compound obtained in Example 11 was subjected to separation by preparative chiral chromatography, to obtain product 43.4mg, with HPLC purity 96.8%, chiral purity 99.4% (ee%). MS m/z (ESI): 646.4 [M+H]$^+$.

**Example 38**

**Preparation of 2-((2-((4-(((4-(2-acrylamidoethyl)pyrimidin-2-yl)amino)methyl)phenyl)amino)-5-(trifluoromethyl)pyrimidin -4-yl)amino)-N-methylbenzamide (38)**

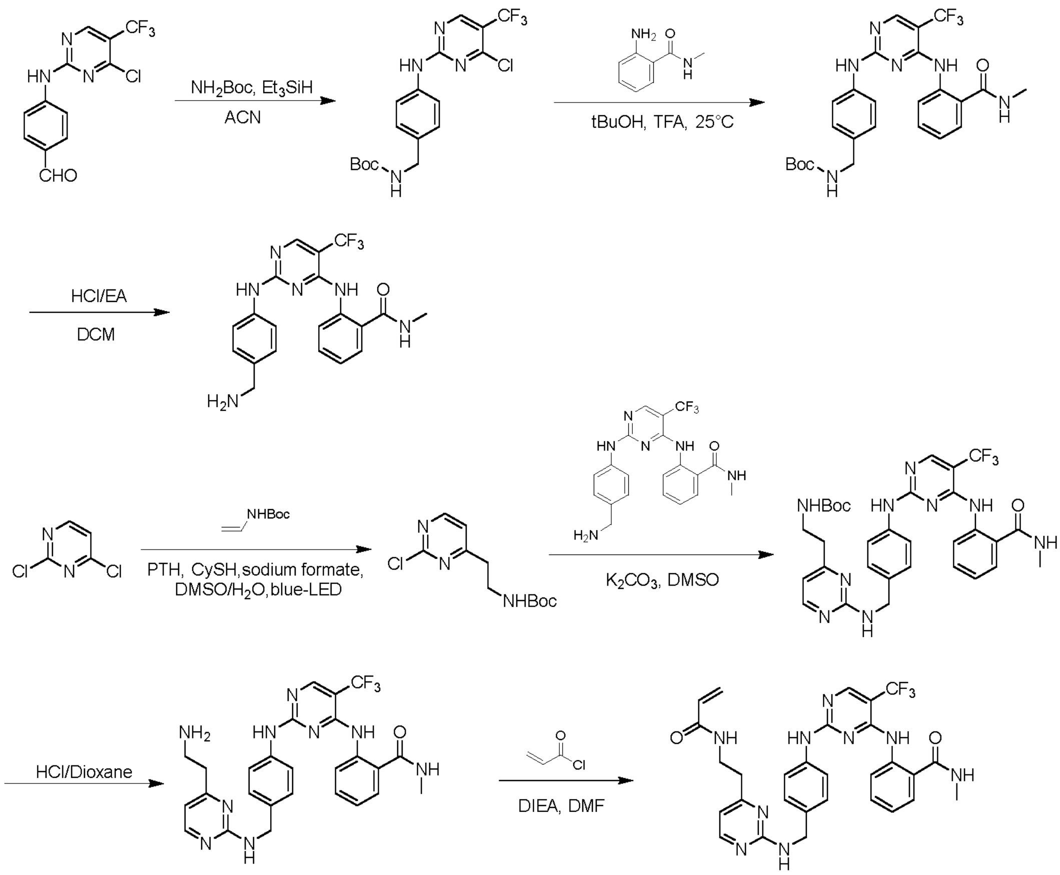


**Step 1: synthesis of *tert*-butyl (4-((4-chloro-5-(trifluoromethyl)pyrimidin-2-yl)amino)benzyl)carbamate**

At room temperature, in a solvent mixture comprising 15 mL acetonitrile and 5 mL dichloromethane were dissolved 4-((4-chloro-5-(trifluoromethyl)pyrimidin-2-yl)amino)benzaldehyde (1 g, 3.32 mmol), *tert*-butyl carbamate (1.17 g, 9.95 mmol) and triethyl silane (1.61 mL, 9.95 mmol), added with trifluoroacetic acid (0.49 mL, 6.63 mmol) dropwise under stirring. After the addition, the mixture was continuously stirred overnight, and then cooled to room temperature. The mixture was concentrated under reduced pressure to remove solvent, added with 40 mL water, and extracted with ethyl acetate (30 mL×3). The organic phases were combined and washed with saturated sodium chloride solution (80 mL), dried over anhydrous sodium sulfate, and filtered. The filtrate was concentrated under reduced pressure, and the residue was purified by column chromatography (ethyl acetate: petroleum ether=1/2) to obtain *tert*-butyl (4-((4-chloro-5-(trifluoromethyl)pyrimidin-2-yl)amino)benzyl) carbamate (0.8 g, 1.99 mmol, yield: 59.91%). **MS m/z (ESI)**: 347.0 $[M-56+H]^+$.

**Step 2: synthesis of *tert*-butyl (4-((2-(methylcarbamoyl)phenyl)amino)-5-(trifluoromethyl)pyrimidin-2-yl)amino)benzyl)carbamate**

In *t*-butanol (10 ml) were dissolved *tert*-butyl (4-((4-chloro-5-(trifluoromethyl)pyrimidin-2-yl)amino)benzyl) carbamate (780 mg, 1.94 mmol) and 2-amino-N-methylbenzamide (320 mg, 2.13 mmol), added with trifluoroacetic acid (144 μL, 1.94 mmol) dropwise under stirring. After the addition, the mixture was continuously stirred overnight. Upon completion of the reaction indicated by TLC, the mixture was directly filtered, and the filter cake was washed with ethyl acetate (10 mL×3) to obtain *tert*-butyl (4-((2-(methylcarbamoyl)phenyl)amino)-5-(trifluoromethyl)pyrimidin-2-yl)amino)benzyl)carbamate (790 mg, 1.53 mmol, yield: 78.98%) as a yellow solid. **MS m/z (ESI)**: 517.3 $[M+H]^+$.

**Step 3: 2-((2-((4-(aminomethyl)phenyl)amino)-5-(trifluoromethyl)pyrimidin-4-yl)amino)-N-methylbenzamide**

Dichloromethane (5 ml) was added in *tert*-butyl (4-((2-(methylcarbamoyl)phenyl)amino)-5-(trifluoromethyl)pyrimidin-2-yl)amino)benzyl) carbamate (790 mg, 1.53 mmol), added with 10 mL 2M hydrochloric acid in ethyl acetate dropwise with stirring. After the addition, the mixture was continuously stirred at room temperature for 3 h. Upon completion of the reaction indicated by TLC, the mixture was directly filtered, and the filter cake was washed with dichloromethane

(80 mL×3) to obtain 2-((2-((4-(aminomethyl)phenyl)amino)-5-(trifluoromethyl)pyrimidin-4-yl)amino)-N-methylbenzamide (690 mg, 1.52 mmol, yield: 99.62%) as a white solid. **MS m/z (ESI)**: 417.2 $[M+H]^+$.

**Step 4: *tert*-butyl (2-(2-chloropyrimidin-4-yl)ethyl) carbamate**

Into a 40 mL vial were added photocatalyst 10-phenyl phenothiazine (13.9mg, 0.05 mmol), 2,4-dichloropyrimidine (91.46 μL, 1.01 mmol), *tert*-butyl N-ethenyl carbamate (173.6 mg, 1.21 mmol) and sodium formate (205.4 mg, 3.02 mmol). The vial was sealed and added with 10 mL DMSO, 0.5 mL water and cyclohexyl mercaptan (6.16 μL, 0.05 mmol) under nitrogen protection. Before sealing with sealing film, the solution was degassed under stirring by nitrogen gas flushing for 10 minutes. The reaction was stirred and irradiated with a 40W blue LED light for 3 hours. Upon completion of the reaction indicated by TLC, the mixture was poured into 100 ml water and extracted with ethyl acetate (80 mL×3). The organic phases were combined and washed with saturated sodium chloride solution (100 mL), dried over anhydrous sodium sulfate, and filtered. The filtrate was concentrated under reduced pressure, and the residue was purified by column chromatography (ethyl acetate: petroleum ether=1/4) to obtain *tert*-butyl (2-(2-chloropyrimidin-4-yl)ethyl)carbamate (173 mg, 0.67 mmol, yield: 66.67%). **MS m/z (ESI)**: 202.0$[M-56+H]^+$.

**Step 5: *t*-butyl (2-(2-((4-((4-((2-(methylcarbamoyl)phenyl)amino)-5-(trifluoromethyl)pyrimidin-2-yl)amino)benzyl)amino)p yrimidin-4-yl)ethyl)carbamate**

In N,N-dimethylsulfoxide (20 ml) were dissolved 2-((2-((4-(aminomethyl)phenyl)amino)-5-(trifluoromethyl)pyrimidin-4-yl)amino)-N-methylbenzamide (200 mg, 0.48 mmol), *tert*-butyl (2-(2-chloropyrimidin-4-yl)ethyl) carbamate (123.8 mg, 0.48 mmol) and potassium carbonate (265.5 mg, 1.92 mmol). The mixture was heated to 120°C and stirred overnight. Upon completion of the reaction indicated by TLC, the reaction mixture was poured into 100 mL water, and extracted with ethyl acetate (80 mL×3). The organic phases were combined and washed with saturated sodium chloride solution (100 mL), dried over anhydrous sodium sulfate, and filtered. The filtrate was concentrated under reduced pressure, and the residue was purified by column chromatography (ethyl acetate: petroleum ether=1/4) to obtain *t*-butyl (2-(2-((4-((4-((2-(methylcarbamoyl)phenyl)amino)-5-(trifluoromethyl)pyrimidin-2-yl)amino)benzyl)amino)pyrimi din-4-yl)ethyl)carbamate (180 mg, 0.28 mmol, yield: 58.77%). **MS m/z (ESI)**: 638.3 $[M+H]^+$.

**Step 6: 2-((2-((4-(((4-(2-aminoethyl)pyrimidin-2-yl)amino)methyl)phenyl)amino)-5-(trifluoromethyl) pyrimidin-4-yl)amino)-N-methylbenzamide**

Dichloromethane (1 ml) was added in *t*-butyl (2-(2-((4-((4-((2-(methylcarbamoyl)phenyl)amino)-5-(trifluoromethyl)pyrimidin-2-yl)amino)benzyl)amino)pyrimi din-4-yl)ethyl)carbamate (180 mg, 0.28 mmol). The mixture was added with 10 mL 4 M hydrochloric acid in 1,4-dioxane dropwise under stirring. After the addition, the mixture was stirred at room temperature for 2 h. Upon completion of the reaction indicated by TLC, the solvent is removed by concentration of the reaction mixture under reduced pressure to obtain 2-((2-((4-(((4-(2-aminoethyl)pyrimidin-2-yl)amino)methyl)phenyl)amino)-5-(trifluoromethyl)pyrimidin-4-yl)amin o)-N-methylbenzamide (150 mg, 0.28 mmol, yield: 98.85%) as a white solid. **MS m/z (ESI)**: 538.2 $[M+H]^+$.

**Step 7: 2-((2-((4-(((4-(2-acrylamidoethyl)pyrimidin-2-yl)amino)methyl)phenyl)amino)-5-(trifluoromethyl) pyrimidin-4-yl)amino)-N-methylbenzamide**

In DMF (10 ml) were dissolved 2-((2-((4-(((4-(2-aminoethyl)pyrimidin-2-yl)amino)methyl)phenyl)amino)-5-(trifluoromethyl)pyrimidin-4-yl)amin o)-N-methylbenzamide (150 mg, 0.28 mmol) and N,N-diisopropylethylamine (210.8 μL, 1.19 mmol). The mixture was stirred in ice bath for 10 minutes and added with 0.22 mL acryloyl chloride in DMF (obtained by dissolving 70 μL acryloyl chloride in 0.5 mL DMF) dropwise under stirring. After the addition, the mixture was stirred in ice bath for 1h and allowed to warm to room temperature. Upon depletion of the starting material indicated by TLC, the reaction mixture was quenched by pouring into 80 mL water, and then extracted with ethyl acetate (40 mL×3). The organic phases were combined and washed twice with 10% sodium chloride solution (80 mL), and then washed once with saturated sodium chloride solution (80 mL), dried over anhydrous sodium sulfate, and filtered. The filtrate was concentrated under reduced pressure, and the residue was purified by preparative reverse phase chromatography to obtain 2-((2-((4-(((4-(2-acrylamidoethyl)pyrimidin-2-yl)amino)methyl)phenyl)amino)-5-(trifluoromethyl)pyrimidin-4-yl) amino)-N-methylbenzamide (57 mg, 0.09 mmol, yield: 31.73%).

$^1$H NMR (DMSO-$d_6$) δ: 11.37 (br s, 1H), 9.82 (br s, 1H), 8.73 (br d, J = 4.5 Hz, 1H), 8.42 (s, 2H), 8.23 (d, J = 5.3Hz, 1H), 8.16 (br s, 1H), 8.05 (br s, 1H), 7.71 (d, J = 7.5 Hz, 1H), 7.56 (br d, J = 7.4 Hz, 2H), 7.38 (br s, 1H), 7.24 (br d, J =8.3 Hz, 2H), 7.14 (t, J = 7.6 Hz, 1H), 6.62 (br d, J = 4.4 Hz, 1H), 6.17 (dd, J = 9.9, 17.1 Hz, 1H), 6.06 (dd, J = 2.4, 17.1 Hz,1H), 5.56 (dd, J = 2.4, 9.9 Hz, 1H), 4.49 (br s, 2H), 3.49 (q, J = 6.8 Hz, 2H), 2.81 - 2.73 (m, 5H). **MS m/z (ESI)**:

592.2[M+H]+.

Compounds synthesized according to the above methods are summarized in the following table 1.

Table 1

| | **Pyrimidine or pyridine derivatives and pharmaceutical uses thereof** | **Pyrimidine or pyridine derivatives and pharmaceutical uses thereof** | **Pyrimidine or pyridine derivatives and pharmaceutical uses thereof** |
|---|---|---|---|
| **Compound No.** | **Structure** | Synthesis method | MS m/z (ESI) $[M+H]^+$ |
| **22** | 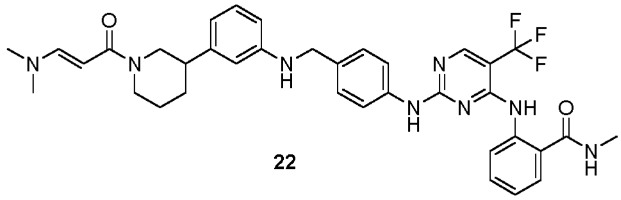 **22** | **2** | 673.3 |
| **23** | 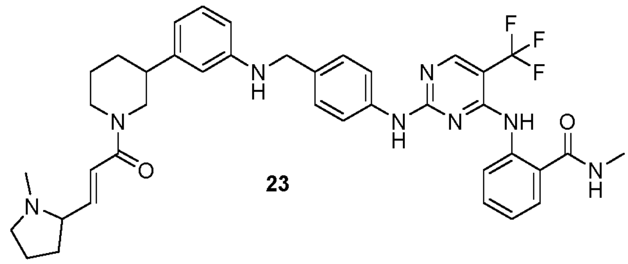 **23** | **2** | 713.4 |
| **25** | 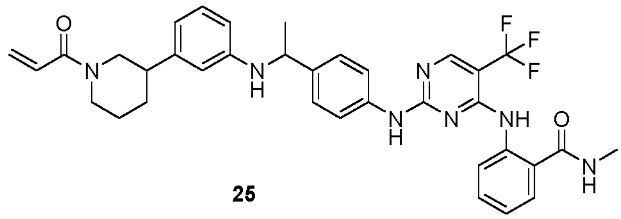 **25** | **2** | 644.3 |
| **26** | 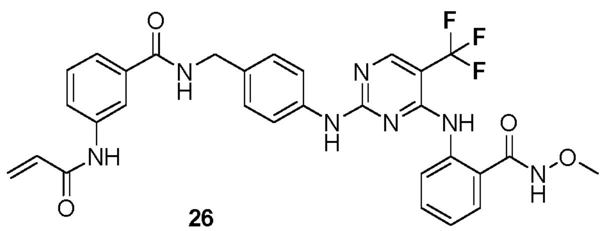 **26** | **2** | 606.2 |
| **27** | 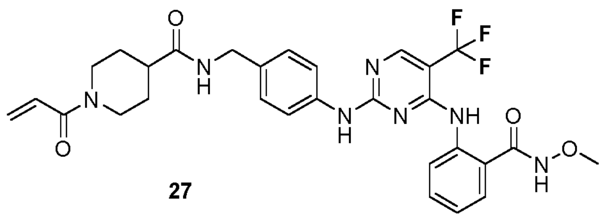 **27** | **2** | 598.2 |

(continued)

| | Pyrimidine or pyridine derivatives and pharmaceutical uses thereof | Pyrimidine or pyridine derivatives and pharmaceutical uses thereof | Pyrimidine or pyridine derivatives and pharmaceutical uses thereof |
|---|---|---|---|
| **Compound No.** | **Structure** | Synthesis method | MS m/z (ESI) $[M+H]^+$ |
| **28** | 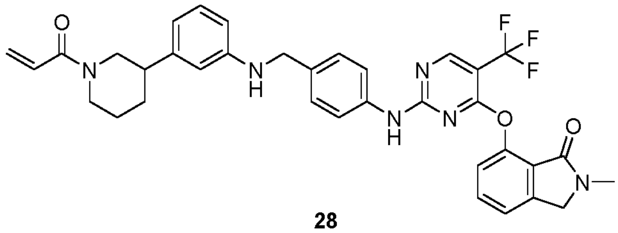 **28** | **2** | 643.3 |
| **29** | 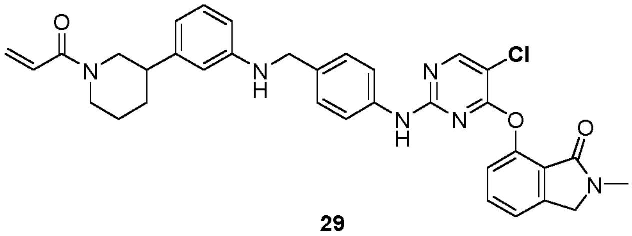 **29** | **2** | 609.2 |

**Biological evaluation of compounds**

**Test Example 1: *In vitro* enzymatic inhibitory activity of compounds of the present application**

1. Reagents, consumables, and instruments used are listed in Table 2.

Table 2

| **Reagent** | Supplier |
|---|---|
| HEPES | Life Technologies |
| BRIJ 35 detergent (10%) | Merck |
| MgCl2 | Sigma |
| HTRF KinEASE-TK kit | Cisbio |
| FAK | Signalchem |
| ATP | Promega |
| DTT (DL-Dithiothreitol) | Sigma |
| **Consumables** | Supplier |
| Topseal A | Perkin Elmer |
| 96 Well Plates | Nunc |
| ProxiPlate-384 Plus | Perkin Elmer |
| **Instruments** | Supplier |
| Plate reader | Perkin Elmer |
| Centrifuge | Eppendorf |

**2. Experimental Procedure**

1) To 384-well dilution plate was added 50 μL of compound.
2) Samples in each column were serially diluted with DMSO at a ratio of 1:3, with 10 samples diluted each time, as well as a control containing only DMSO.
3) 0.1 μL of the diluted solution of the compound in each row was transferred to a 384-well assay plate, with 2 replicates for each column.
4) To the assay plate was added 5 μL of 2X enzyme solution, centrifuged at 1000 rpm for 1 minute, and incubated at 25°C for 15 minutes.
5) To a 384-well assay plate was added 5 μL of 2X substrate solution.
6) Incubation at 25°C for 60 minutes.
7) To assay plate were added 5 μL of Sa-XL665 solution and 5μL of TK antibody-Eu3 $^{+}$, and centrifuged at 1000 rpm for 1minute.
2) Incubation at 25°C for 60 minutes.
8) Acquisition of fluorescence signals using Envision 2104 Reader.

**3. Data analysis**

1) For each screening plate, Means and Standard deviation (SD) of DMSO and 100 nM Defactinib (as control) were calculated.
2) Inhibition percentage of compounds (%inh) = 100×(maximum value-measured value)/( maximum value - minimum value).
3) IC50 is calculated by the nonlinear regression equation of XLfit 5.3.1, using the following formula:

$$Y=Bottom + (Top-Bottom)/(1+10^((LogIC50-X)*HillSlope))$$

X: Log(concentration of compound)
Y: inhibition rate(% inh)
Top and Bottom have the same unit as Y
logIC50 has the same unit as X
HillSlope: slope coefficient or slope.

**4. Experimental results**

The data of FAK kinase activity and IC50 activity of the specific compound are shown in Table 3.

Table 3

| Compound No. | IC50 (nM) |
|---|---|
| 1 | 1.133 |
| 2 | 1.177 |
| 3 | 1.595 |
| 4 | 1.32 |
| 5 | 0.9 |
| 6 | 2.68 |
| 7 | 3.58 |
| 8 | 5.79 |
| 9 | 3.71 |
| 10 | 15.78 |

(continued)

| Compound No. | IC50 (nM) |
|---|---|
| 11 | 5.67 |
| 12 | 3.41 |
| 13 | 19.77 |
| 14 | 4.36 |
| 15 | 7.84 |
| 16 | 6.73 |
| 17 | 6.13 |
| 18 | 17.88 |
| 19 | 10.43 |
| 20 | 4.98 |
| 21 | 4.33 |
| 24 | 2.93 |
| 30 | 3.75 |
| 31 | 3.93 |
| 32 | 3.43 |
| 33 | 5.11 |
| 34 | 1.27 |
| 35 | 1.45 |
| 36 | 0.97 |
| 37 | 1.35 |

**Test Example 2: *In vitro* pharmacodynamic evaluation of compounds in a diffuse-type gastric cancer (DGC) organoid model**

Experimental procedures: Organoids were amplified in 6-well plates (7 Matrigel aliquots per well) for 2-3 days prior to evaluation. After passage, 1000 cells were inoculated in 5 μl Matrigel aliquot into each well containing 100 μl 50% L-WRN medium in a 96-well plate. Organoids were cultured for 24 hours, followed by addition of the compounds. Compounds were dissolved in DMSO as stock solution (with a final concentration of 10uM), which was then diluted with DMSO to 1000X concentration of the testing concentration. Culture medium was added to the stock solution to dilute to the testing concentration. The diluted solution was added to the culture plate. Once compound was added, culture medium was no longer added, and no further compound added. After the organoids were cultured for a specified number of days, the effects of compounds were evaluated by the following three methods, each using DMSO as negative control.

1. Cell morphology was observed with microscope, and results were shown in Figure 1. There was a difference in cell morphology between normal cells and the diffuse-type gastric cancer (DGC) organoid model. Normal organoids have hollow spheroid shape, while organoids of diffuse-type gastric cancer have solid grape-like irregular shape. Therefore, the observations of the effect of drugs on cell morphology may be one of the important characteristics for evaluating drug efficacy. Results of the effects of different compounds on cell morphology are shown in Figure 2. The compound of the present application can restore cell morphology by treating the DGC organoid model at a concentration of 500nM, in which compounds 9, 11, and 12 have significantly better effects than defactinib and IN-10018.

2. After cell culture in accordance with the above procedure and culture with compounds for 48 hours, the cells were lysed, and proteins were extracted and tested by Western Blot. After gel imaging, gray values of protein bands were analyzed to investigate the effects of the compounds on phosphorylated FAK and activated YAP in the organoid

model. (For detailed methods, please refer to Haisheng Zhang, Cancer Discovery, 2020).

The experimental results are shown in Figure 3, indicating that compounds 6, 9, 11, 12, and 13 of the present application can inhibit the phosphorylation of FAK (p-FAK 397) and meanwhile inhibit the activated YAP (non-p-YAP) in the organoid model in a dose-dependent manner, and are superior to IN-10018 and compound Ref-1. The structural formula of Ref-1 is as follows:

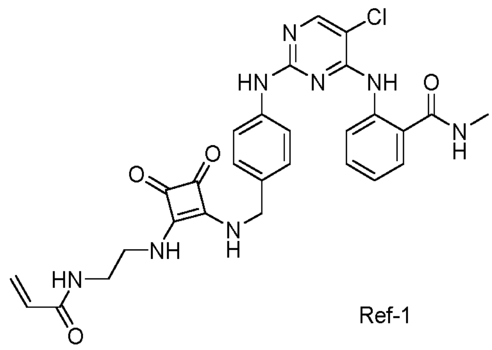

Ref-1

Therefore, the compounds of the present application can achieve the goal of treating diseases, especially cancer, by inhibiting FAK kinases and/or inhibiting activated YAP.

**Test Example 3: Time-dependent FAK kinase inhibition activity test**

This test process was the same as **Test Example 1**, except that step 4) in Experimental Procedure was modified as follows: 5μL 2X enzyme solution was added to the assay plate, centrifuged at 1000 rpm for 1 minute, and incubate at 25°C for 0 minutes, 15 minutes, and 60 minutes, respectively, with other operations being the same. The experimental results show that, compared to the reference compound defactinib, compound 6 of the present application has a longer effective inhibition time on the kinase activity of FAK. At 60 minutes, the IC50 of compound 6 is 0.6625nM.

**Test Example 4: *In vitro* pharmacodynamic evaluation (detection of cell proliferation inhibition) of compounds in human diffuse-type gastric cancer cell lines SNU-668 or NUGC4 model**

1. Experimental procedure

1) cell plating

a. SNU-668 cells (catalog number: 00668, KCLB) or NUGC4 cells (catalog number: 0834, JCRB) were allowed to grow to 70%-80% confluence, the supernatant of the culture medium was pipetitted off, rinsed once with PBS and then pipetitted off;
b. To a 10 cm dish was added 1mL of 0.25% trypsin, votexed to homogenity and then place into incubator for digestion for 2-3 min;
c. The culture dish is removed from the incubator added with 2-3 mL complete culture medium to terminate the digestion, pipetitted into a 15mL centrifuge tube, and then centrifuged in centrifuge at 1000 rpm for 5 minutes;
d. The supernatant was pipetitted off, and the cells were resuspended in 1mL of complete culture medium, and then counted;
e. 1000 cells were plated into each well of 96-well plate, and then cultured overnight in a cell incubator;

2) Cell administration with compounds

a. Compounds to be tested were dissolved and votexed to homogeneity;
b. Each compound was serielly diluted in 1:3 ratio from 1mM stock solution to 9 gradient concentrations;
c. The plate seeded with cells was taken out from the incubator, pipetitted off culture medium, and then added slowly

in each well with 100μL fresh culture medium by multi-channel pipette. Wells are labeled by corresponding compounds and concentrations, and then gently added with 100 μL compound-containing culture medium along the well wall by multi-channel pipette;

d. After the addition to all wells, the plate was gently shaken to allow homogeneous spread of the compound, and then put back to the incubator, with time recorded;

3) CELL TITER-GLO test

4 days after the addition of compounds, the culture medium was removed and mixed at 100μL from each well with cell titer-glo testing liquid in 1:1 ratio. The mixture was incubated with shaking at room temperature for 10 min and then counted with Plate reader.

2. Experimental conclusions

The experimental results (see Table 4 for details) indicate that some compounds of the present application can inhibit cell growth of a tumor cell line model of human diffuse-type gastric cancer, with inhibitory activity superior to FAK inhibitors Defactinib and IN10018 in clinical phases.

Table 4

| Example | SNU668 cell proliferation $IC_{50}$ (μM) | NUGC4 cell proliferation $IC_{50}$ (μM) |
|---|---|---|
| Defactinib | | 1.37 |
| IN10018 | 8.4 | 3.50 |
| 6 | 0.05 | 0.02 |
| 11 | | 0.09 |
| 15 | 0.11 | |
| 16 | 0.06 | |
| 17 | <0.01 | |
| 18 | 0.14 | |
| 19 | 0.21 | 0.09 |
| 20 | | 0.34 |
| 21 | | 0.04 |
| 24 | | 0.02 |
| 30 | 0.09 | |
| 31 | <0.01 | 0.02 |
| 32 | | 0.04 |
| 33 | | 0.02 |
| 34 | | 0.08 |
| 36 | | 0.09 |
| 37 | | 0.12 |

**Test Example 5: *In vivo* pharmacokinetic evaluation of compounds**

1. Experimental design

Three male SD rats were administered with compounds in a single dose of 5 mg/kg or 3 mg/kg by gavage, and blood samples were collected before administration and at 0.25, 0.5, 1, 2, 4, 6, 8, and 24 h after administration.

2. Formulation preparation

Preparation of solutions of compounds to be dosed at 5 mg/kg: 5.5 mg compound (converted based on purity) was accurately weighed and added to 0.55mL of DMSO. Clear solutions were obtained after stirring and sonication. 0.5mL of each of the above solutions was added with 1 mL of Solutol, stirred for 1 minute; and then added with 8.5 mL of physiological saline and stirred for 1 minute, to obtain a clear solution at 0.5mg/mL concentration.

Preparation of a solution of compounds to be dosed at 3 mg/kg: 3.6 mg compound (converted according to purity) was accurately weighed and added to 0.6 mL of DMSO. Clear solutions were obtained after stirring and sonication. 0.5mL of each of the above solutions was added with 1 mL of Solutol and stirred for 1 minute; and then added with 8.5 mL of physiological saline and stirred for 1 minute, to obtain a clear solution at 0.3mg/mL concentration.

3. Animal manipulation

Rats were administered the formulations prepared in step 2 by gavage at a dose of 10mL/kg. The rats were fasted overnight before the administration and resumed feeding 4 hours after administration. Throughout the entire experiment, the animals were free to access drinking water.

4. Analysis methods

The plasma concentration was measured using LC-MS/MS method.

5. Experimental results

The experimental results show that the compound of the present application leads to good pharmacokinetic parameters in rats, as shown in Table 5.

Table 5

| Compound No. | 35 | 36 | 37 | Defactinib |
|---|---|---|---|---|
| Administration route | gavage | gavage | gavage | gavage |
| Dose (mg/kg) | 5 | 3 | 3 | 5 |
| Pharmacokinetic parameters | - | - | - | - |
| $T_{1/2}$ (h) | 0.96 | 1.20 | 1.07 | 1.20 |
| $T_{max}$ (h) | 2.67 | 2.67 | 2.67 | 4.00 |
| $C_{max}$ (nM) | 130.31 | 146.17 | 87.17 | 99.51 |
| $AUC_{0\text{-}last}$ (nM·h) | 540.10 | 688.72 | 360.55 | 424.2 |
| $AUC_{0\text{-}inf}$ (nM·h) | 548.39 | 710.50 | 368.24 | 440.54 |

**Test Example 6: *In vitro* pharmacodynamic evaluation of the compound of the present application in a human diffuse type gastric cancer cell line SNU-668 model (detection of FAK and YAP activity by Western blot)**

1. Experimental procedure

1) Cell plating

a. SNU-668 cells (catalog number: 00668, KCLB) were allowed to grow to 70%-80% confluence, the supernatant of the culture medium was pipetitted off, rinsed once with PBS and then pipetitted off;
b. To a 10 cm dish was added 2mL trypsin, and digested in an incubator for 3-5min;
c. The culture dish is removed from the incubator added with 5mL 1640 medium (containing 10% FBS) to terminate the digestion, pipetitted into a 15mL centrifuge tube, and then centrifuged in centrifuge at 1000 rpm for 5 minutes;
d. The supernatant was pipetitted off, added with 5mL 1640 medium (containing 10% FBS). ; the cells were resuspended thoroughly and mix well, then counted;

e. $1.5\times10^5$ cells were seeded into each well of 12-well plate, and then cultured in a cell incubator for 24h.

2) Cell treatment with compounds

a. Stock solutions of the compounds to be tested (1 mM) were diluted in DMSO, and blown and nixed several times to homogeneity;

b. The plate seeded with cells was taken out from the incubator, gently added in wells with 1ul 1mM stocks (1000X diluted) along the well wall, and then gently shaken immediately to homogeneity;

c. After the addition to all wells, the plate was gently shaken to allow homogeneous spread of the compound, and then put back to the incubator, with time recorded; Samples were collected after 24 hours.

3) Protein extraction

a. 24 hours after the addition of compounds, the culture medium was removed and the cells were rinsed with PBS, and then the PBS was removed;

b. The cells were lysed with a RIPA lysis solution containing phosphatase inhibitors and PMSF, centrifuged at 4°C for 10min at 12000rpm. The supernatant was taken and tested for concentrations of proteins. After adjusting the concentrations of proteins, 4× loading buffer was added for protein digestion.

4) Western blot assay

SDS-PAGE electrophoresis: 80v, 30min, 120v, 60min; transferring: 80v, 60min; blocking: 5% skimmed milk 1H, Primary antibody: P-FAKY397 (1: 1000, CST), non-p-YAP (1: 2000, Abcam), p-YAP (1: 1000, CST), actin (1: 10000, CST), formulated in.TBST containing 5% BSA, incubation overnight at 4°C; Secondary antibody (Goat-anti-mouse-IgG and Goat-anti-rabbit-IgG,1:3000, Proteintech): formulated in TBST containing 5% skimmed milk, incubation at room temperature for 1 h. Developed with Thermo developer.

2. Experimental conclusion

Results are shown in Figures 4, 5, and 6, indicating that the compounds of the present application can inhibit the phosphorylation of FAK (inhibiting FAK kinase activity, reducing phosphorylated FAK) and meanwhile reduce activated YAP (inhibiting YAP activity, reducing non-phosphorylated YAP (non-p-YAP)) in the human diffuse-type gastric cancer cell line SNU668 model, while Defactinib and IN10018 have no inhibiting effect on YAP activity.

Although examples of the present application have been shown and described above, it can be understood that the above examples are illustrative and cannot be understood as a limitation of the present application. Ordinary technical personnel in the art may make changes, modifications, substitutions, and variations to the above examples within the scope of the present application.

## Claims

1. A compound, which is a compound of formula I, or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof:

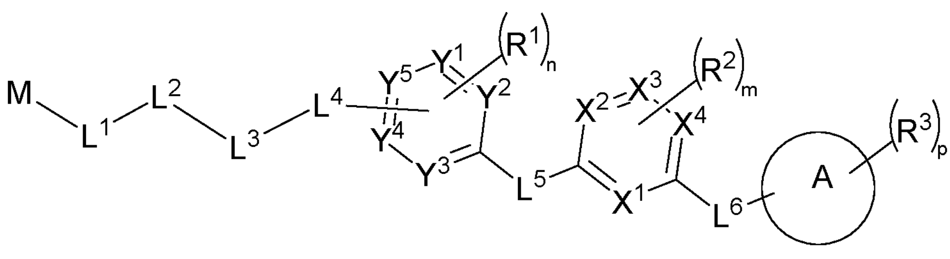

I

wherein,

M is a covalent warhead;

A is aryl or heteroaryl;

$L^1$ is a bond, -C(=O)-, -S(=O)-, $-S(=O)_2-$, $-C(=O)NR^{L1a}-$, $-NR^{L1a}C(=O)-$, $-S(=O)_2NR^{L1a}-$, or $-NR^{L1a}S(=O)_2-$; wherein each $R^{L1a}$ is independently hydrogen, alkyl, alkenyl, alkynyl, heteroalkyl, haloalkyl, haloheteroalkyl, or a nitrogen protecting group; wherein the alkyl, alkenyl, alkynyl, heteroalkyl, haloalkyl, and haloheteroalkyl involved in $R^{L1a}$ each optionally are independently substituted by one or more substituents selected from the group consisting of deuterium, halogen, hydroxyl, amino, cyano, and oxo;

$L^2$ is a bond, alkyl, heteroalky, saturated or partially unsaturated carbocyclyl, saturated or partially unsaturated heterocyclyl, aryl, or heteroaryl; wherein the alkyl, heteroalkyl, saturated or partially unsaturated carbocyclyl, saturated or partially unsaturated heterocyclyl, aryl, or heteroaryl involved in $L^2$ each optionally are independently substituted by one or more substituents selected from the group consisting of deuterium, hydroxyl, amino, cyano, halogen, oxo, alkyl, alkenyl, alkynyl, heteroalkyl, and haloheteroalkyl;

$L^3$ is a bond, aryl, or heteroaryl; wherein, the aryl, or heteroaryl involved in $L^3$ each optionally are independently substituted by one or more substituents selected from the group consisting of deuterium, hydroxyl, amino, cyano, halogen, alkyl, alkenyl, alkynyl, heteroalkyl, haloalkyl, and haloheteroalkyl;

$L^4$ is a carbon chain, or heterochain with 2-6 atoms; wherein, the carbon chain or heterochain involved in $L^4$ each optionally are independently substituted by one or more substituents selected from the group consisting of deuterium, hydroxyl, amino, cyano, halogen, oxo, alkenyl, alkynyl, alkyl, haloalkyl, hydroxyalkyl, aminoalkyl, alkoxyl, haloalkoxyl, hydroxyalkoxyl, aminoalkoxyl, alkylamino, and a nitrogen protecting group;

$L^5$ is $-NR^{L5a}-$, -O-, -S-, alkyl, or heteroalkyl; wherein, the alkyl and heteroalkyl involved in $L^5$ each optionally are independently substituted by a substituent selected from the group consisting of deuterium, amino, hydroxyl, oxo, cyano, halogen, alkenyl, and alkynyl; each $R^{L5a}$ is independently hydrogen, alkyl, alkenyl, alkynyl, heteroalkyl, haloalkyl, or a nitrogen protecting group;

$L^6$ is $-NR^{L6a}-$, -O-, -S-, alkyl or heteroalkyl; wherein, the alkyl and heteroalkyl involved in $L^6$ each optionally are independently substituted by a substituent selected from the group consisting of deuterium, amino, hydroxyl, oxo, cyano, halogen, alkenyl and alkynyl; each $R^{L6a}$ is independently hydrogen, alkyl, alkenyl, alkynyl, heteroalkyl, haloalkyl, or a nitrogen protecting group;

$X^1$, $X^2$, $X^3$, $X^4$, $Y^1$, $Y^2$, $Y^3$, $Y^4$, and $Y^5$ each are independently CH or N;

each $R^1$ is independently hydrogen, deuterium, amino, hydroxyl, cyano, halogen, alkyl, alkenyl, alkynyl, heteroalkyl, haloalkyl, haloheteroalkyl, alkoxyl, haloalkoxyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl; if there are two adjacent $R^1$, the two adjacent $R^1$ and their connected atoms optionally form saturated or partially unsaturated carbocyclyl, saturated or partially unsaturated heterocyclyl, aryl, or heteroaryl; wherein, the alkyl, alkenyl, alkynyl, heteroalkyl, haloalkyl, haloheteroalkyl, alkoxyl, haloalkoxyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, and heteroarylalkyl involved in $R^1$ each optionally are independently substituted by one or more substituents selected from the group consisting of deuterium, hydroxyl, amino, cyano, halogen, oxo, and alkyl;

each $R^2$ is independently hydrogen, deuterium, amino, hydroxyl, cyano, halogen, alkyl, alkenyl, alkynyl, heteroalkyl, haloalkyl, haloheteroalkyl, alkoxyl, haloalkoxyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl; if there are two adjacent $R^2$, the two adjacent $R^2$ and their connected atoms optionally form saturated or partially unsaturated carbocyclyl, saturated or partially unsaturated heterocyclyl, aryl or heteroaryl; wherein, the alkyl, alkenyl, alkynyl, heteroalkyl, haloalkyl, haloheteroalkyl, alkoxyl, haloalkoxyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^2$ each optionally are independently substituted by one or more substituents selected from the group consisting of hydroxyl, amino, cyano, halogen, oxo, and alkyl;

each $R^3$ is independently hydrogen, deuterium, amino, hydroxyl, cyano, halogen, alkyl, alkenyl, alkynyl, heteroalkyl, haloalkyl, haloheteroalkyl, alkoxyl, haloalkoxyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl; if there are two adjacent $R^3$, the two adjacent $R^3$ and their connected atoms optionally form saturated or partially unsaturated carbocyclyl, saturated or partially unsaturated heterocyclyl, aryl or heteroaryl; wherein, the alkyl, alkenyl, alkynyl, heteroalkyl, haloalkyl,

haloheteroalkyl, alkoxyl, haloalkoxyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^3$ each optionally are independently substituted by one or more substituents selected from the group consisting of hydroxyl, amino, cyano, halogen, oxo, and alkyl; and

n, m, and p each are independently 0, 1, 2, 3, or 4.

**2.** The compound according to claim 1, or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof, wherein,

M is

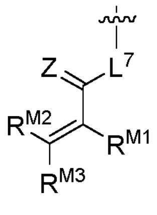
i-1

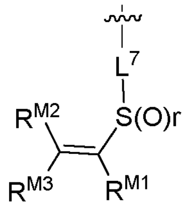
i-2

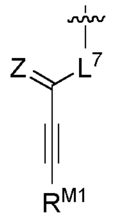
i-3

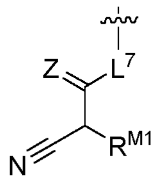
i-4

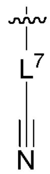
i-5

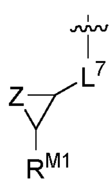
i-6

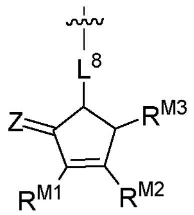
i-7

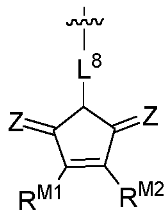
i-8

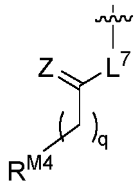
i-9

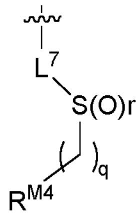
i-10

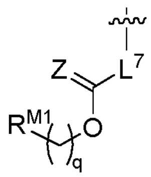
i-11

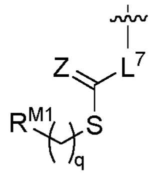
i-12

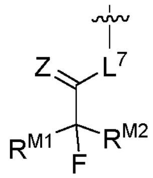
i-13

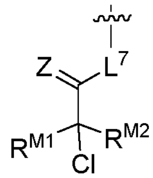
i-14

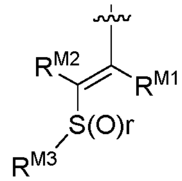
i-15

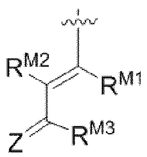
i-16

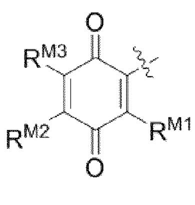
i-17

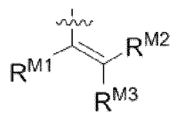
i-18

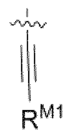
i-19 or

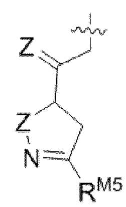
i-20 ;

wherein,

$L^7$ is a bond, -O-, -S-, $-NR^{L7a}-$ or $C_{1-4}$alkyl, wherein, one or more carbon units of the $C_{1-4}$alkyl group optionally are independently replaced with -O-, -S-, $-NR^{L7a}-$, $-NR^{L7a}C(=O)-$, $-C(=O)NR^{L7a}-$, -SC(=O)-, -C(=O)S-, -OC(=O)-, -C(=O)O-, $-NR^{L7a}C(=S)-$, $-C(=S)NR^{L7a}-$, *trans* $CR^{L7b}=CR^{L7b}-$, *cis* $CR^{L7b}=CR^{L7b}-$, -C≡C-, -S(=O)-, -S(=O)O-, -OS(=O)-, $-S(=O)NR^{L7a}-$, $-NR^{L7a}S(=O)-$, $-S(=O)_2-$, $-S(=O)_2O-$, $-OS(=O)_2-$, $-S(=O)_2NR^{L7a}-$, or $-NR^{L7a}S(=O)_2-$;

wherein each $R^{L7a}$ is independently hydrogen, deuterium, $C_{1-4}$alkyl, or a nitrogen protecting group; each $R^{L7b}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two adjacent $R^{L7b}$ groups bind with their connected atoms to form 3-6 membered saturated or partially unsaturated carbocyclyl or 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $L^7$, $R^{L7a}$ and $R^{L7b}$ each optionally are independently substituted by one or more substituents selected from the group consisting of deuterium, halogen, hydroxyl, amino, cyano, oxo, and $C_{1-4}$alkyl;

$L^8$ is a bond or $C_{1-4}$alkyl, wherein the alkyl involved in $L^8$ is optionally substituted by one or more substituents selected from the group consisting of deuterium, halogen, hydroxyl, amino, cyano, and oxo;

each $R^{M1}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, $-CH_2OR^{41a}$, $-CH_2N(R^{M1a})_2$, $-CH_2SR^{M1a}$, $-OR^{M1a}$, $-N(R^{M1a})_2$, $-Si(R^{M1a})_3$, or $-SR^{M1a}$, wherein each $R^{M1a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl; or two $R^{M1a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M1}$ and $R^{M1a}$ each optionally are independently substituted by one or more substituents selected from the group consisting of deuterium, halogen, hydroxyl, amino, cyano, oxo, and $C_{1-4}$alkyl;

each $R^{M2}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, $-CH_2ORM^{2a}$, $-CH_2N(R^{M2a})_2$, $-CH_2SR^{M2a}$, $-OR^{M2a}$, $-N(R^{M2a})_2$ or $-SR^{M2a}$, wherein each $R^{M2a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl; or two $R^{M2a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M2}$ and $R^{M2a}$ each optionally are independently substituted by one or more substituents selected from the group consisting of deuterium, halogen, hydroxyl, amino, cyano, oxo, and $C_{1-4}$alkyl;

each $R^{M3}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, $-CH_2ORM^{3a}$, $-CH_2N(R^{M3a})_2$, $-CH_2SR^{M3a}$, $-OR^{M3a}$, $-N(R^{M3a})_2$ or $-SR^{M3a}$, wherein each $R^{M3a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl; or two adjacent $R^{M3a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M3}$ and $R^{M3a}$ each optionally are independently substituted by one or more substituents selected from the group consisting of deuterium, halogen, hydroxyl, amino, cyano, oxo, and $C_{1-4}$alkyl;

optionally, $R^{M1}$ and $R^{M3}$, or $R^{M2}$ and $R^{M3}$, or $R^{M1}$ and $R^{M2}$ optionally connect to form saturated or partially unsaturated carbocyclyl or saturated or partially unsaturated heterocyclyl, wherein saturated or partially unsaturated carbocyclyl or saturated or partially unsaturated heterocyclyl each optionally are independently substituted by one or more substituents selected from the group consisting of deuterium, halogen, hydroxyl, amino, cyano, oxo, and $C_{1-4}$alkyl;

$R^{M4}$ is a leaving group;

$R^{M5}$ is halogen;

Z is O, S or $NR^Z$; wherein $R^Z$ is hydrogen, deuterium, $C_{1-4}$alkyl, or a nitrogen protecting group; the involved in $R^Z$ optionally is substituted by one or more substituents selected from the group consisting of deuterium, halogen, hydroxyl, amino, cyano, and oxo;

q is 0, 1, 2, 3, 4, 5 or 6;

r is 1 or 2; or

M is

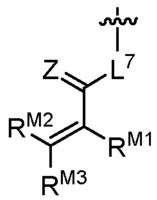
i-1 ,
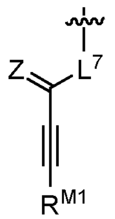
i-3 ,
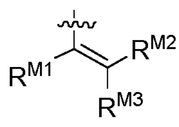
i-18 or
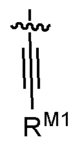
i-19 ;

$L^7$ is a bond, -O-, -S-, -$NR^{L7a}$- or $C_{1-4}$alkyl, wherein, one or more carbon units of the $C_{1-4}$alkyl group optionally are independently replaced with -O-, -S-, -$NR^{L7a}$-, -$NR^{L7a}$C(=O)-, -C(=O)$NR^{L7a}$-, -SC(=O)-, -C(=O)S-, -OC(=O)-, -C(=O)O-, -$NR^{L7a}$C(=S)-, -C(=S)$NR^{L7a}$-, *trans* $CR^{L7b}$=$CR^{L7b}$-, *cis* $CR^{L7b}$=$CR^{L7b}$-, -C≡C-, -S(=O)-, -S(=O)O-, -OS(=O)-, -S(=O)$NR^{L7a}$-, -$NR^{L7a}$S(=O)-, -$S(=O)_2$-, -$S(=O)_2$O-, -$OS(=O)_2$-, -$S(=O)_2NR^{L7a}$-, or -$NR^{L7a}S(=O)_2$-; wherein each $R^{L7a}$ is independently hydrogen, deuterium, $C_{1-4}$alkyl, or a nitrogen protecting group; each $R^{L7b}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two adjacent $R^{L7b}$ groups optionally bind with their connected atoms to form 3-6 membered saturated or partially unsaturated carbocyclyl or 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $L^7$, $R^{L7a}$ and $R^{L7b}$ each optionally are independently substituted by one or more substituents selected from the group consisting of deuterium, halogen, hydroxyl, amino, cyano, oxo, and $C_{1-4}$alkyl;

Z is O, S or $NR^Z$; wherein $R^Z$ is hydrogen, deuterium, $C_{1-4}$alkyl, or a nitrogen protecting group; alkyl involved in $R^Z$ optionally is substituted by one or more substituents selected from the group consisting of hydrogen, deuterium, halogen, hydroxyl, amino, cyano, and oxo;

each $R^{M1}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, -$CH_2OR^{M1a}$, -$CH_2N(R^{M1a})_2$, -$CH_2SR^{M1a}$, -$OR^{M1a}$, -$N(R^{M1a})_2$, -$Si(R^{M1a})_3$ or -$SR^{M1a}$, wherein each $R^{M1a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two $R^{M1a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M1}$ and $R^{M1a}$ each optionally are independently substituted by one or more substituents selected from the group consisting of deuterium, halogen, hydroxyl, amino, cyano, oxo, and $C_{1-4}$alkyl;

each $R^{M2}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, -$CH_2ORM^{2a}$, -$CH_2N(R^{M2a})_2$, -$CH_2SR^{M2a}$, -$OR^{M2a}$, -$N(R^{M2a})_2$ or -$SR^{M2a}$, wherein each $R^{M2a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two $R^{M2a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M2}$ and $R^{M2a}$ each optionally are independently substituted by one or more substituents selected from the group consisting of deuterium, halogen, hydroxyl, amino, cyano, oxo, and $C_{1-4}$alkyl;

each $R^{M3}$ is independently hydrogen, deuterium, halogen, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, 5-10 membered heteroaryl, -CN, -$CH_2ORM^{3a}$, -$CH_2N(R^{M3a})_2$, -$CH_2SR^{M3a}$, -$OR^{M3a}$, -$N(R^{M3a})_2$ or -$SR^{M3a}$, wherein each $R^{M3a}$ is independently hydrogen, deuterium, $C_{1-4}$alkyl, $C_{2-4}$alkenyl, $C_{2-4}$alkynyl, 3-6 membered saturated or partially unsaturated carbocyclyl, 3-6 membered saturated or partially unsaturated heterocyclyl, 6-10 membered aryl, or 5-10 membered heteroaryl, or two adjacent $R^{M3a}$ groups optionally bind with their connected atoms to form 3-6 membered saturated or partially unsaturated heterocyclyl; wherein the alkyl, alkenyl, alkynyl, saturated or partially unsaturated carbocyclyl, saturated or partially unsaturated heterocyclyl, aryl, or heteroaryl involved in each of $R^{M3}$ and $R^{M3a}$ each optionally are independently

substituted by one or more substituents selected from the group consisting of deuterium, halogen, hydroxyl, amino, cyano, oxo, and $C_{1-4}$alkyl; or

M is 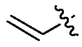, 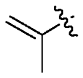, 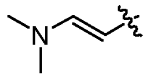, 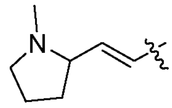, 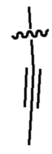, 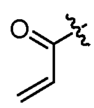, 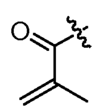, 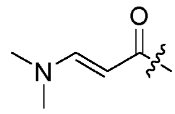,

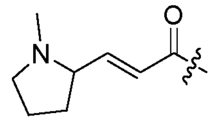 or 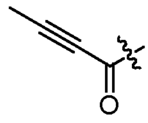.

3. The compound according to claim 1, or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof, wherein, A is 6-10 membered aryl, or 5-10 membered heteroaryl; or A is 6-10 membered aryl or 5-10 membered azaaryl; or A is phenyl or 6-membered azaaryl; or A is phenyl, imidazolyl, pyrazolyl, triazolyl, tetraazolyl, oxazolyl, thiazolyl, furyl, thienyl, pyrrolyl, pyridinyl, pyrimidinyl, pyrazinyl, pyridazinyl, or the following groups:

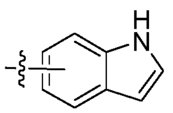, 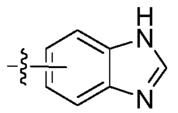, 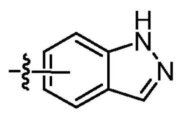, 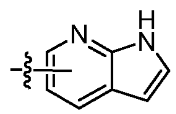, 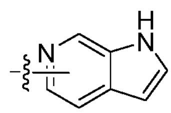, 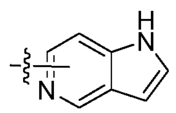,

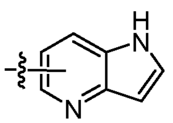, 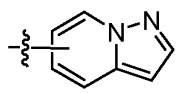, 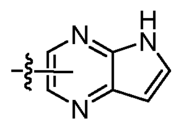, 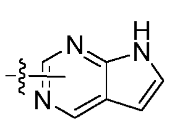, 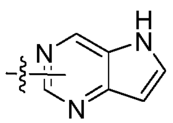, 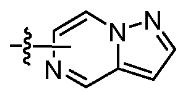,

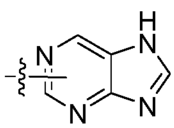, 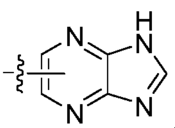, 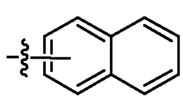, 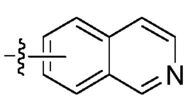, 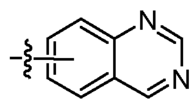 or 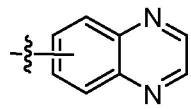,

in which 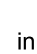 means an end of A connected to $L^6$;
or A is phenyl, pyridinyl, pyrimidinyl, pyrazinyl or pyridazinyl;
or A is phenyl or pyrazinyl.

4. The compound according to claim 1, or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof, wherein,

$L^1$ is a bond, -C(=O)-, -S(=O)-, $-S(=O)_2-$, $-C(=O)NR^{L1a}-$, $-NR^{L1a}C(=O)-$, $-S(=O)_2NR^{L1a}-$, or $-NR^{L1a}S(=O)_2-$; wherein, each $R^{L1a}$ is independently hydrogen, deuterium, $C_{1-4}$alkyl, $C_{2-4}$alkenyl, $C_{2-4}$alkynyl, $C_{1-4}$heteroalkyl, $C_{1-4}$haloalkyl, $C_{1-4}$haloheteroalkyl, or a nitrogen protecting group; wherein the alkyl, alkenyl, alkynyl, heteroalkyl, haloalkyl, and haloheteroalkyl involved in $R^{L1a}$ each optionally are independently substituted by one or more substituents selected from the group consisting of deuterium, halogen, hydroxyl, amino, cyano, and oxo; $L^2$ is a bond, $C_{1-6}$alkyl, $C_{1-6}$heteroalkyl, 3-6 membered saturated or partially unsaturated carbocyclyl, 3-6 membered saturated or partially unsaturated heterocyclyl, 6-10 membered aryl, or 5-10 membered heteroaryl; wherein, the alkyl, heteroalkyl, saturated or partially unsaturated carbocyclyl, saturated or partially unsaturated heterocyclyl, aryl, or heteroaryl involved in $L^2$ each optionally are independently substituted by one or more substituents selected from the group consisting of deuterium, hydroxyl, amino, cyano, halogen, oxo, $C_{1-4}$alkyl, $C_{2-4}$alkenyl,

$C_{2-4}$alkynyl, $C_{1-4}$heteroalkyl, $C_{1-4}$haloalkyl, and $C_{1-4}$haloheteroalkyl; $L^3$ is a bond, 6-10 membered aryl, or 5-10 membered heteroaryl; wherein, aryl, or heteroaryl involved in $L^3$ each optionally are independently substituted by one or more substituents selected from the group consisting of deuterium, hydroxyl, amino, cyano, halogen, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; $L^4$ is a carbon chain or heterochain with 2-6 atoms; wherein, the carbon chain or heterochain involved in $L^4$ each optionally are independently substituted by one or more substituents selected from the group consisting of deuterium, hydroxyl, amino, cyano, halogen, oxo, $C_{2-4}$alkenyl, $C_{2-4}$alkynyl, $C_{1-4}$alkyl, $C_{1-4}$haloalkyl, $C_{1-4}$hydroxyalkyl, $C_{1-4}$aminoalkyl, $C_{1-4}$alkoxyl, $C_{1-4}$haloalkoxyl, $C_{1-4}$hydroxyalkoxyl, $C_{1-4}$aminoalkoxyl, $C_{1-4}$alkylamino, and a nitrogen protecting group; or

$L^1$ is a bond, -C(=O)-, -S(=O)-, -$S(=O)_2$-, -C(=O)$NR^{L1a}$-, -$NR^{L1a}$C(=O)-, -$S(=O)_2NR^{L1a}$-, or -$NR^{L1a}S(=O)_2$-; wherein, each $R^{L1a}$ is independently hydrogen, methyl, ethyl, ethenyl, ethynyl or trifluoromethyl; $L^2$ is a bond, $C_{1-6}$alkyl, $C_{1-6}$heteroalkyl, 3-6 membered saturated or partially unsaturated carbocyclyl, 3-6 membered saturated or partially unsaturated heterocyclyl, 6-10 membered aryl, or 5-10 membered heteroaryl; wherein, the alkyl, heteroalkyl, saturated or partially unsaturated carbocyclyl, saturated or partially unsaturated heterocyclyl, aryl, or heteroaryl involved in $L^2$ each optionally are independently substituted by a substituent selected from the group consisting of deuterium, hydroxyl, amino, cyano, fluoro, chloro, oxo, ethenyl, ethynyl, methoxy, ethoxy, trifluoromethoxy and methylamino; $L^3$ is a bond, 6-10 membered aryl, or 5-10 membered heteroaryl; wherein, aryl, or heteroaryl involved in $L^3$ each optionally are independently substituted by a substituent selected from the group consisting of deuterium, hydroxyl, amino, cyano, fluoro, chloro, methyl, ethyl, ethenyl, ethynyl, methoxy, ethoxy, trifluoromethoxy and methylamino; $L^4$ is a carbon chain or heterochain with 2-4 atoms; wherein, the carbon chain or heterochain involved in $L^4$ each optionally are independently substituted by one or more substituents selected from the group consisting of deuterium, hydroxyl, amino, cyano, halogen, oxo, ethenyl, ethynyl, methyl, ethyl, isopropyl, methoxy, ethoxy, trifluoromethoxy, methylamino, and a nitrogen protecting group; or

$L^1$ is a bond, -C(=O)-, -S(=O)-, -$S(=O)_2$-, -C(=O)NH-, -NHC(=O)-, -$S(=O)_2$NH- or -NH$S(=O)_2$-; $L^2$ is a bond, 3-6 membered saturated or partially unsaturated carbocyclyl or 3-6 membered saturated or partially unsaturated heterocyclyl; wherein, saturated or partially unsaturated carbocyclyl, saturated or partially unsaturated heterocyclyl involved in $L^2$ each optionally are independently substituted by one or more substituents selected from the group consisting of hydroxyl, amino, cyano, fluoro, chloro, oxo, ethenyl, ethynyl, methoxy, ethoxy, trifluoromethoxy and methylamino; $L^3$ is a bond, 6-10 membered aryl, or 5-10 membered heteroaryl; wherein, aryl, or heteroaryl involved in $L^3$ each optionally are independently substituted by a substituent selected from the group consisting of hydroxyl, amino, cyano, fluoro, chloro, methyl, ethyl, ethenyl, ethynyl, methoxy, ethoxy, trifluoromethoxy and methylamino; $L^4$ is a carbon chain or heterochain with 2-4 atoms; wherein, the carbon chain or heterochain involved in $L^4$ each optionally are independently substituted by one or more substituents selected from the group consisting of deuterium, hydroxyl, amino, cyano, halogen, oxo, ethenyl, ethynyl, methyl, ethyl, isopropyl, methoxy, ethoxy, trifluoromethoxy, methylamino, and a nitrogen protecting group; or

$L^1$ is a bond, -C(=O)- or -C(=O)NH-; $L^2$ is a bond, cyclohexyl, piperidyl, phenyl or pyridinyl; $L^3$ is a bond, phenyl or pyridinyl; $L^4$ is $-NHCH_2-$, $-CH_2NH-$, $-OCH_2-$, $-CH_2O-$, $-SCH_2-$, $-CH_2S-$, $-NHCH_2CH_2-$, $-CH_2NHCH_2-$, $-CH_2CH_2NH-$, $-OCH_2CH_2-$, $-CH_2OCH_2-$, $-CH_2CH_2O-$, $-SCH_2CH_2-$, $-CH_2SCH_2-$, $-CH_2CH_2S-$, $-NHCH_2CH_2CH_2-$, $-CH_2NHCH_2CH_2-$, $-CH_2CH_2NHCH_2-$, $-CH_2CH_2CH_2NH-$, $-OCH_2CH_2CH_2-$, $-CH_2OCH_2CH_2-$, $-CH_2CH_2OCH_2-$, $-CH_2CH_2CH_2O-$, $-SCH_2CH_2CH_2-$, $-CH_2SCH_2CH_2-$, $-CH_2CH_2SCH_2-$, $-CH_2CH_2CH_2S-$, $-CH_2OCH_2O-$, $-OCH_2OCH_2-$, $-OCH_2CH_2O-$, $-NHCH_2CH_2O-$, $-CH_2NHCH_2O-$ or $-NHCH_2OCH_2-$; wherein the hydrogen of $CH_2$ involved in $L^4$ each optionally are replaced with a substituent selected from the group consisting of hydrogen, deuterium, hydroxyl, amino, cyano, halogen, oxo, ethenyl, ethynyl, methyl, ethyl, isopropyl, methoxy, ethoxy, trifluoromethoxy and methylamino; the hydrogen of NH involved in $L^4$ each optionally are replaced with a substituent selected from the group consisting of hydrogen, deuterium, hydroxyl, cyano, halogen, oxo, ethenyl, ethynyl, methyl, ethyl, isopropyl, methoxy, ethoxy, trifluoromethoxy, and a nitrogen protecting group; or

$L^1$ is a bond, -C(=O)- or -C(=O)NH-; $L^2$ is a bond, cyclohexyl, piperidyl, phenyl or pyridinyl; $L^3$ is a bond, phenyl or pyridinyl; $L^4$ is $-NHCH_2-$, $-CH_2NHCH_2-$, -NHC(=O)-, $-CH_2NHC(=O)-$, $-C(=O)NHCH_2-$, or $-NHCH(CH_3)-$.

**5.** The compound according to claim 1, or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof, wherein

$L^5$ is -$NR^{L5a}$-, -O-, -S-, $C_{1-6}$alkyl or $C_{1-6}$heteroalkyl; wherein, the alkyl and heteroalkyl involved in $L^5$ each optionally are independently substituted by one or more substituents selected from the group consisting of deuterium, amino, hydroxyl, oxo, cyano, halogen, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; each $R^{L5a}$ is independently hydrogen, deuterium, $C_{1-6}$alkyl, $C_{2-6}$alkenyl, $C_{2-6}$alkynyl, $C_{1-6}$heteroalkyl, $C_{1-6}$haloalkyl, or a nitrogen protecting group; and

$L^6$ is $-NR^{L6a}-$, -O-, -S-, $C_{1-6}$alkyl or $C_{1-6}$heteroalkyl; wherein, the alkyl and heteroalkyl involved in $L^6$ each optionally are independently substituted by one or more substituents selected from the group consisting of deuterium, amino, hydroxyl, oxo, cyano, halogen, $C_{1-4}$alkyl, $C_{2-4}$alkenyl, $C_{2-4}$alkynyl, $C_{1-4}$heteroalkyl, $C_{1-4}$haloalkyl, and $C_{1-4}$haloheteroalkyl; each $R^{L6a}$ is independently hydrogen, deuterium, $C_{1-6}$alkyl, $C_{2-6}$alkenyl, $C_{2-6}$alkynyl, $C_{1-6}$heteroalkyl, $C_{1-6}$haloalkyl, or a nitrogen protecting group;

or, $L^5$ is $-NR^{L5a}$, $-CR^{L5b}R^{L5c}-$, -O-, -S-, $-CR^{L5b}R^{L5c}NR^{L5a}-$, $-NR^{L5a}CR^{L5b}R^{L5c}-$, $-CR^{L5b}R^{L5c}O-$, $-OCR^{L5b}R^{L5c}-$, $-CR^{L5b}R^{L5c}S-$ or $-SCR^{L5b}R^{L5c}-$; wherein, each $R^{L5b}$ and $R^{L5c}$ are independently hydrogen, deuterium, amino, hydroxyl, oxo, cyano, halogen, $C_{1-4}$alkyl, $C_{2-4}$alkenyl, $C_{2-4}$alkynyl, $C_{1-4}$heteroalkyl, $C_{1-4}$haloalkyl, or $C_{1-4}$haloheteroalkyl; each $R^{L5a}$ is independently hydrogen, deuterium, $C_{1-4}$alkyl, $C_{2-4}$alkenyl, $C_{2-4}$alkynyl, $C_{1-4}$heteroalkyl, $C_{1-4}$haloalkyl, or a nitrogen protecting group; and

$L^6$ is $-NR^{L6a}$, $-CR^{L6b}R^{L6c}-$, -O-, -S-, $-CR^{L6b}R^{L6c}NR^{L6a}-$, $-NR^{L6a}CR^{L6b}R^{L6c}-$, $-CR^{L6b}R^{L6c}O-$, $-OCR^{L6b}R^{L6c}-$, $-CR^{L6b}R^{L6c}S-$ or $-SCR^{L6b}R^{L6c}-$; wherein, each $R^{L6b}$ and $R^{L6c}$ are independently hydrogen, deuterium, amino, hydroxyl, oxo, cyano, halogen, $C_{1-4}$alkyl, $C_{2-4}$alkenyl, $C_{2-4}$alkynyl, $C_{1-4}$heteroalkyl, $C_{1-4}$haloalkyl, or $C_{1-4}$haloheteroalkyl; each $R^{L6a}$ is independently hydrogen, deuterium, $C_{1-4}$alkyl, $C_{2-4}$alkenyl, $C_{2-4}$alkynyl, $C_{1-4}$heteroalkyl, $C_{1-4}$haloalkyl, or a nitrogen protecting group;

or, $L^5$ is -NH-, -O-, -S-, $-NHCH_2-$, $-OCH_2-$, $-SCH_2-$, $-CH_2NH-$, $-CH_2O-$ or $-CH_2S-$; $L^6$ is -NH-, -O-, -S-, $-NHCH_2-$, $-OCH_2-$, $-SCH_2-$, $-CH_2NH-$, $-CH_2O-$ or $-CH_2S-$; or

$L^5$ is -NH-, and $L^6$ is -NH- or $-NHCH_2-$.

**6.** The compound according to any one of claims 1 to 5, or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof, wherein

each $R^1$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, $C_{1-6}$haloheteroalkyl, $C_{1-6}$alkoxyl, $C_{1-6}$haloalkoxyl, 3-6 membered saturated or partially unsaturated carbocyclyl, 3-6 membered saturated or partially unsaturated carbocyclyl$C_{1-6}$alkyl, 3-6 membered saturated or partially unsaturated heterocyclyl, 3-6 membered saturated or partially unsaturated heterocyclyl$C_{1-6}$alkyl, 6-10 membered aryl, 6-10 membered aryl$C_{1-6}$alkyl, 6-10 membered heteroaryl or 6-10 membered heteroaryl$C_{1-6}$alkyl; if there are two adjacent $R^1$, the two adjacent $R^1$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, alkoxyl, haloalkoxyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^1$ each optionally are independently substituted by one or more substituents selected from the group consisting of deuterium, hydroxyl, amino, cyano, halogen, oxo, and $C_{1-4}$alkyl;

each $R^2$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, $C_{1-6}$haloheteroalkyl, $C_{1-6}$alkoxyl, $C_{1-6}$haloalkoxyl, 3-6 membered saturated or partially unsaturated carbocyclyl, 3-6 membered saturated or partially unsaturated carbocyclyl$C_{1-6}$alkyl, 3-6 membered saturated or partially unsaturated heterocyclyl, 3-6 membered saturated or partially unsaturated heterocyclyl$C_{1-6}$alkyl, 6-10 membered aryl, 6-10 membered aryl$C_{1-6}$alkyl, 6-10 membered heteroaryl or 6-10 membered heteroaryl$C_{1-6}$alkyl; if there are two adjacent $R^2$, the two adjacent $R^2$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, alkoxyl, haloalkoxyl, saturated or partially unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^2$ each optionally are independently substituted by one or more substituents selected from the group consisting of deuterium, hydroxyl, amino, cyano, halogen, oxo, and $C_{1-4}$alkyl; and

each $R^3$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, $C_{1-6}$haloheteroalkyl, $C_{1-6}$alkoxyl, $C_{1-6}$haloalkoxyl, 3-6 membered saturated or partially unsaturated carbocyclyl, 3-6 membered saturated or partially unsaturated carbocyclyl$C_{1-6}$alkyl, 3-6 membered saturated or partially unsaturated heterocyclyl, 3-6 membered saturated or partially unsaturated heterocyclyl$C_{1-6}$alkyl, 6-10 membered aryl, 6-10 membered aryl$C_{1-6}$alkyl, 6-10 membered heteroaryl or 6-10 membered heteroaryl$C_{1-6}$alkyl; if there are two adjacent $R^3$, the two adjacent $R^3$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, alkoxyl, haloalkoxyl, saturated or partially

unsaturated carbocyclyl, saturated or partially unsaturated carbocyclylalkyl, saturated or partially unsaturated heterocyclyl, saturated or partially unsaturated heterocyclylalkyl, aryl, arylalkyl, heteroaryl, or heteroarylalkyl involved in $R^3$ each optionally are independently substituted by one or more substituents selected from the group consisting of deuterium, hydroxyl, amino, cyano, halogen, oxo, and $C_{1-4}$alkyl;

alternatively, each $R^1$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, $C_{1-6}$haloheteroalkyl or $C_{1-6}$alkoxyl, $C_{1-6}$haloalkoxy; if there are two adjacent $R^1$, the two adjacent $R^1$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, alkoxyl, haloalkoxyl, saturated or partially unsaturated carbocyclyl, saturated or partially unsaturated heterocyclyl, aryl, or heteroaryl involved in $R^1$ each optionally are independently substituted by one or more substituents selected from the group consisting of deuterium, hydroxyl, amino, cyano, halogen, oxo, and $C_{1-4}$alkyl; each $R^2$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, $C_{1-6}$haloheteroalkyl or $C_{1-6}$alkoxyl, $C_{1-6}$haloalkoxy; if there are two adjacent $R^2$, the two adjacent $R^2$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, alkoxyl, haloalkoxyl, saturated or partially unsaturated carbocyclyl, saturated or partially unsaturated heterocyclyl, aryl, or heteroaryl involved in $R^2$ each optionally are independently substituted by one or more substituents selected from the group consisting of deuterium, hydroxyl, amino, cyano, halogen, oxo, and $C_{1-4}$alkyl; and each $R^3$ is independently hydrogen, deuterium, amino, hydroxyl, cyano, halogen, $C_{1-6}$alkyl, $C_{2-6}$alkenyl, $C_{2-6}$alkynyl, $C_{1-6}$heteroalkyl, $C_{1-6}$haloalkyl, $C_{1-6}$haloheteroalkyl or $C_{1-6}$alkoxyl, $C_{1-6}$haloalkoxy; if there are two adjacent $R^3$, the two adjacent $R^3$ and their connected atoms optionally form 3-6 membered saturated or partially unsaturated carbocyclyl, 3-6 membered saturated or partially unsaturated heterocyclyl, 6-10 membered aryl or 6-10 membered heteroaryl; wherein, the alkyl, alkenyl, alkynyl, heteroalkyl, haloalkyl, haloheteroalkyl, alkoxyl, haloalkoxyl, saturated or partially unsaturated carbocyclyl, saturated or partially unsaturated heterocyclyl, aryl, or heteroaryl involved in $R^3$ each optionally are independently substituted by one or more substituents selected from the group consisting of deuterium, hydroxyl, amino, cyano, halogen, oxo, and $C_{1-4}$alkyl; or

each $R^1$ is independently hydrogen, deuterium, amino, hydroxyl, cyano, halogen, $C_{1-6}$alkyl, $C_{1-6}$heteroalkyl, $C_{1-6}$haloalkyl, or $C_{1-6}$haloheteroalkyl; wherein the alkyl, heteroalkyl, haloalkyl, haloheteroalkyl involved in $R^1$ each optionally are independently substituted by one or more substituents selected from the group consisting of deuterium, hydroxyl, amino, cyano, halogen, and oxo; each $R^2$ is independently hydrogen, deuterium, amino, hydroxyl, cyano, halogen, $C_{1-6}$alkyl, $C_{1-6}$heteroalkyl, $C_{1-6}$haloalkyl, or $C_{1-6}$haloheteroalkyl; wherein the alkyl, heteroalkyl, haloalkyl, haloheteroalkyl involved in $R^2$ each optionally are independently substituted by one or more substituents selected from the group consisting of deuterium, hydroxyl, amino, cyano, halogen, and oxo; each $R^3$ is independently hydrogen, deuterium, amino, hydroxyl, cyano, halogen, $C_{1-6}$alkyl, $C_{1-6}$heteroalkyl, $C_{1-6}$haloalkyl, or $C_{1-6}$haloheteroalkyl; if there are two adjacent $R^3$, the two adjacent $R^3$ and their connected atoms optionally form 3-6 membered saturated or partially unsaturated carbocyclyl, 3-6 membered saturated or partially unsaturated heterocyclyl; wherein the alkyl, heteroalkyl, haloalkyl, haloheteroalkyl, 3-6 membered saturated or partially unsaturated carbocyclyl, 3-6 membered saturated or partially unsaturated heterocyclyl involved in $R^3$ each optionally are independently substituted by one or more substituents selected from the group consisting of deuterium, hydroxyl, amino, cyano, halogen, oxo, and $C_{1-4}$alkyl; or

each $R^1$ is independently hydrogen, deuterium, amino, hydroxyl, cyano, halogen, $C_{1-4}$alkyl, $C_{1-4}$heteroalkyl, $C_{1-4}$haloalkyl, or $C_{1-4}$haloheteroalkyl; wherein the alkyl, heteroalkyl, haloalkyl, haloheteroalkyl involved in $R^1$ each optionally are independently substituted by one or more substituents selected from the group consisting of deuterium, hydroxyl, amino, cyano, halogen, and oxo; each $R^2$ is independently hydrogen, deuterium, amino, hydroxyl, cyano, halogen, $C_{1-4}$alkyl, $C_{1-4}$heteroalkyl, $C_{1-4}$haloalkyl, or $C_{1-4}$haloheteroalkyl; wherein the alkyl, heteroalkyl, haloalkyl, haloheteroalkyl involved in $R^2$ each optionally are independently substituted by one or more substituents selected from the group consisting of deuterium, hydroxyl, amino, cyano, halogen, and oxo; and each $R^3$ is independently hydrogen, deuterium, amino, hydroxyl, cyano, halogen, $C_{1-4}$alkyl, $C_{1-4}$heteroalkyl, $C_{1-4}$haloalkyl, or $C_{1-4}$haloheteroalkyl; if there are two adjacent $R^3$, the two adjacent $R^3$ and their connected atoms optionally form 3-6 membered saturated or partially unsaturated carbocyclyl, 3-6 membered saturated or partially unsaturated heterocyclyl; wherein the alkyl, heteroalkyl, haloalkyl, haloheteroalkyl, 3-6 membered saturated or partially unsaturated carbocyclyl, 3-6 membered saturated or partially unsaturated heterocyclyl involved in $R^3$ each optionally are independently substituted by one or more substituents selected from the group consisting of deuterium, hydroxyl, amino, cyano, halogen, oxo, methyl, ethyl, and isopropyl; or

each $R^1$ is independently hydrogen, deuterium, amino, hydroxyl, cyano, fluoro, chloro, methyl, ethyl, propyl,

isopropyl, *t*-butyl, methoxy, ethoxy, i-propoxy, *t*-butoxy, trifluoromethyl, trifluoromethoxy, $CH_3C(=O)$-, $CH_3CH_2C(=O)$-, $(CH_3)_2CHC(=O)$-, $CH_3C(=O)O$-, $CH_3CH_2C(=O)O$-, $(CH_3)_2CHC(=O)O$-, $CH_3C(=O)NH$-, $CH_3CH_2C(=O)NH$-, $(CH_3)_2CHC(=O)NH$-, $CH_3NHC(=O)$-, $CH_3CH_2NHC(=O)$-, $(CH_3)_2CHNHC(=O)$-, $CH_3ONHC(=O)$-, $CH_3CH_2ONHC(=O)$-, $(CH_3)_2CHONHC(=O)$-, $CH_3S(=O)_2$-, $CH_3CH_2S(=O)_2$-, $(CH_3)_2CHS(=O)_2$-, $CH_3S(=O)_2O$-, $CH_3CH_2S(=O)_2O$-, $(CH_3)_2CHS(=O)_2O$-, $CH_3S(=O)_2NH$-, $CH_3CH_2S(=O)_2NH$-, $(CH_3)_2CHS(=O)_2NH$-, $CH_3NHS(=O)_2$-, $CH_3CH_2NHS(=O)_2$-, $(CH_3)_2CHNHS(=O)_2$-, $CH_3C(=O)N(CH_3)$-, $CH_3CH_2C(=O)N(CH_3)$-, $(CH_3)_2CHC(=O)N(CH_3)$-, $CH_3N(CH_3)C(=O)$-, $CH_3CH_2N(CH_3)C(=O)$-, $(CH_3)_2CHN(CH_3)C(=O)$-, $CH_3ON(CH_3)C(=O)$-, $CH_3CH_2ON(CH_3)C(=O)$-, $(CH_3)_2CHON(CH_3)C(=O)$-, $CH_3S(=O)_2N(CH_3)$-, $CH_3CH_2S(=O)_2N(CH_3)$-, $(CH_3)_2CHS(=O)_2N(CH_3)$-, $CH_3N(CH_3)S(=O)_2$-, $CH_3CH_2N(CH_3)S(=O)_2$-, $(CH_3)_2CHN(CH_3)S(=O)_2$-, $CH_3C(=O)N(CH_2CH_3)$-, $CH_3CH_2C(=O)N(CH_2CH_3)$-, $(CH_3)_2CHC(=O)N(CH_2CH_3)$-, $CH_3N(CH_2CH_3)C(=O)$-, $CH_3CH_2N(CH_2CH_3)C(=O)$-, $(CH_3)_2CHN(CH_2CH_3)C(=O)$-, $CH_3ON(CH_2CH_3)C(=O)$-, $CH_3CH_2ON(CH_2CH_3)C(=O)$-, $(CH_3)_2CHON(CH_2CH_3)C(=O)$-, $CH_3S(=O)_2N(CH_2CH_3)$-, $CH_3CH_2S(=O)_2N(CH_2CH_3)$-, $(CH_3)_2CHS(=O)_2N(CH_2CH_3)$-, $CH_3N(CH_2CH_3)S(=O)_2$-, $CH_3CH_2N(CH_2CH_3)S(=O)_2$-, $(CH_3)_2CHN(CH_2CH_3)S(=O)_2$-, $(CH_3)_2P(=O)$-, $CH_3CH_2(CH_3)P(=O)$-, $(CH_3)_2CH(CH_3)P(=O)$-, $(CH_3CH_2)_2P(=O)$-, $CH_3CH_2(CH_3)P(=O)$-, or $(CH_3)_2CH(CH_3CH_2)P(=O)$-;

each $R^2$ is independently hydrogen, deuterium, amino, hydroxyl, cyano, fluoro, chloro, bromo, methyl, ethyl, propyl, isopropyl, *t*-butyl, methoxy, ethoxy, i-propoxy, *t*-butoxy, trifluoromethyl, trifluoromethoxy, $CH_3C(=O)$-, $CH_3CH_2C(=O)$-, $(CH_3)_2CHC(=O)$-, $CH_3C(=O)O$-, $CH_3CH_2C(=O)O$-, $(CH_3)_2CHC(=O)O$-, $CH_3C(=O)NH$-, $CH_3CH_2C(=O)NH$-, $(CH_3)_2CHC(=O)NH$-, $CH_3NHC(=O)$-, $CH_3CH_2NHC(=O)$-, $(CH_3)_2CHNHC(=O)$-, $CH_3ONHC(=O)$-, $CH_3CH_2ONHC(=O)$-, $(CH_3)_2CHONHC(=O)$-, $CH_3S(=O)_2$-, $CH_3CH_2S(=O)_2$-, $(CH_3)_2CHS(=O)_2$-, $CH_3S(=O)_2O$-, $CH_3CH_2S(=O)_2O$-, $(CH_3)_2CHS(=O)_2O$-, $CH_3S(=O)_2NH$-, $CH_3CH_2S(=O)_2NH$-, $(CH_3)_2CHS(=O)_2NH$-, $CH_3NHS(=O)_2$-, $CH_3CH_2NHS(=O)_2$-, $(CH_3)_2CHNHS(=O)_2$-, $CH_3C(=O)N(CH_3)$-, $CH_3CH_2C(=O)N(CH_3)$-, $(CH_3)_2CHC(=O)N(CH_3)$-, $CH_3N(CH_3)C(=O)$-, $CH_3CH_2N(CH_3)C(=O)$-, $(CH_3)_2CHN(CH_3)C(=O)$-, $CH_3ON(CH_3)C(=O)$-, $CH_3CH_2ON(CH_3)C(=O)$-, $(CH_3)_2CHON(CH_3)C(=O)$-, $CH_3S(=O)_2N(CH_3)$-, $CH_3CH_2S(=O)_2N(CH_3)$-, $(CH_3)_2CHS(=O)_2N(CH_3)$-, $CH_3N(CH_3)S(=O)_2$-, $CH_3CH_2N(CH_3)S(=O)_2$-, $(CH_3)_2CHN(CH_3)S(=O)_2$-, $CH_3C(=O)N(CH_2CH_3)$-, $CH_3CH_2C(=O)N(CH_2CH_3)$-, $(CH_3)_2CHC(=O)N(CH_2CH_3)$-, $CH_3N(CH_2CH_3)C(=O)$-, $CH_3CH_2N(CH_2CH_3)C(=O)$-, $(CH_3)_2CHN(CH_2CH_3)C(=O)$-, $CH_3ON(CH_2CH_3)C(=O)$-, $CH_3CH_2ON(CH_2CH_3)C(=O)$-, $(CH_3)_2CHON(CH_2CH_3)C(=O)$-, $CH_3S(=O)_2N(CH_2CH_3)$-, $CH_3CH_2S(=O)_2N(CH_2CH_3)$-, $(CH_3)_2CHS(=O)_2N(CH_2CH_3)$-, $CH_3N(CH_2CH_3)S(=O)_2$-, $CH_3CH_2N(CH_2CH_3)S(=O)_2$-, $(CH_3)_2CHN(CH_2CH_3)S(=O)_2$-, $(CH_3)_2P(=O)$-, $CH_3CH_2(CH_3)P(=O)$-, $(CH_3)_2CH(CH_3)P(=O)$-, $(CH_3CH_2)_2P(=O)$-, $CH_3CH_2(CH_3)P(=O)$-, or $(CH_3)_2CH(CH_3CH_2)P(=O)$-; and

each $R^3$ is independently hydrogen, deuterium, amino, hydroxyl, cyano, fluoro, chloro, methyl, ethyl, propyl, isopropyl, *t*-butyl, methoxy, ethoxy, i-propoxy, *t*-butoxy, trifluoromethyl, trifluoromethoxy, $CH_3C(=O)$-, $CH_3CH_2C(=O)$-, $(CH_3)_2CHC(=O)$-, $CH_3C(=O)O$-, $CH_3CH_2C(=O)O$-, $(CH_3)_2CHC(=O)O$-, $CH_3C(=O)NH$-, $CH_3CH_2C(=O)NH$-, $(CH_3)_2CHC(=O)NH$-, $CH_3NHC(=O)$-, $CH_3CH_2NHC(=O)$-, $(CH_3)_2CHNHC(=O)$-, $CH_3ONHC(=O)$-, $CH_3CH_2ONHC(=O)$-, $(CH_3)_2CHONHC(=O)$-, $CH_3S(=O)_2$-, $CH_3CH_2S(=O)_2$-, $(CH_3)_2CHS(=O)_2$-, $CH_3S(=O)_2O$-, $CH_3CH_2S(=O)_2O$-, $(CH_3)_2CHS(=O)_2O$-, $CH_3S(=O)_2NH$-, $CH_3CH_2S(=O)_2NH$-, $(CH_3)_2CHS(=O)_2NH$-, $CH_3NHS(=O)_2$-, $CH_3CH_2NHS(=O)_2$-, $(CH_3)_2CHNHS(=O)_2$-, $CH_3C(=O)N(CH_3)$-, $CH_3CH_2C(=O)N(CH_3)$-, $(CH_3)_2CHC(=O)N(CH_3)$-, $CH_3N(CH_3)C(=O)$-, $CH_3CH_2N(CH_3)C(=O)$-, $(CH_3)_2CHN(CH_3)C(=O)$-, $CH_3ON(CH_3)C(=O)$-, $CH_3CH_2ON(CH_3)C(=O)$-, $(CH_3)_2CHON(CH_3)C(=O)$-, $CH_3S(=O)_2N(CH_3)$-, $CH_3CH_2S(=O)_2N(CH_3)$-, $(CH_3)_2CHS(=O)_2N(CH_3)$-, $CH_3N(CH_3)S(=O)_2$-, $CH_3CH_2N(CH_3)S(=O)_2$-, $(CH_3)_2CHN(CH_3)S(=O)_2$-, $CH_3C(=O)N(CH_2CH_3)$-, $CH_3CH_2C(=O)N(CH_2CH_3)$-, $(CH_3)_2CHC(=O)N(CH_2CH_3)$-, $CH_3N(CH_2CH_3)C(=O)$-, $CH_3CH_2N(CH_2CH_3)C(=O)$-, $(CH_3)_2CHN(CH_2CH_3)C(=O)$-, $CH_3ON(CH_2CH_3)C(=O)$-, $CH_3CH_2ON(CH_2CH_3)C(=O)$-, $(CH_3)_2CHON(CH_2CH_3)C(=O)$-, $CH_3S(=O)_2N(CH_2CH_3)$-, $CH_3CH_2S(=O)_2N(CH_2CH_3)$-, $(CH_3)_2CHS(=O)_2N(CH_2CH_3)$-, $CH_3N(CH_2CH_3)S(=O)_2$-, $CH_3CH_2N(CH_2CH_3)S(=O)_2$-, $(CH_3)_2CHN(CH_2CH_3)S(=O)_2$-, $(CH_3)_2P(=O)$-, $CH_3CH_2(CH_3)P(=O)$-, $(CH_3)_2CH(CH_3)P(=O)$-, $(CH_3CH_2)_2P(=O)$-, $CH_3CH_2(CH_3)P(=O)$-, or $(CH_3)_2CH(CH_3CH_2)P(=O)$-; if there are two adjacent $R^3$, the two adjacent $R^3$ and their connected atoms optionally form the following groups:

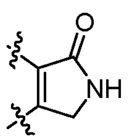, 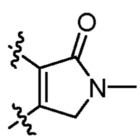, 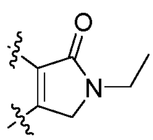, 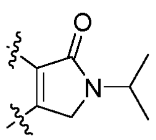, 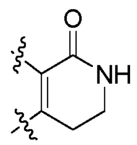, 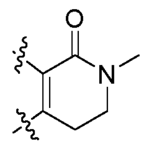, 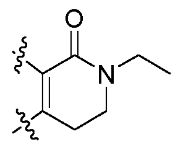,

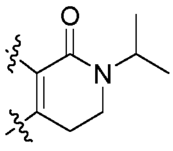 , 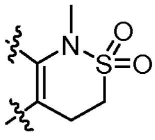 or 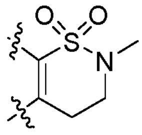 .

7. The compound according to any one of claims 1 to 6, or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof, wherein,

the saturated or partially unsaturated carbocyclyl is selected from formulae ii-1 to ii-6; when formulae ii-1 to ii-6 are connected to the rest of the molecule through one bond, formulae ii-1 to ii-6 are monovalent; when formulae ii-1 to ii-6 are connected to the rest of the molecule through two bonds, formulae ii-1 to ii-6 are bivalent; the saturated or partially unsaturated carbocyclyl represented by formulae ii-1 to ii-6 each optionally are independently substituted by one or more substituents defined by the preceding corresponding claims;

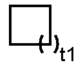 ii-1 , 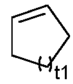 ii-2 , 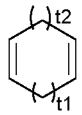 ii-3 , 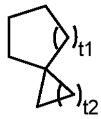 ii-4 , 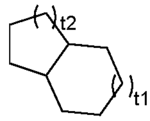 ii-5 or 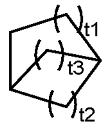 ii-6 ;

the saturated or partially unsaturated heterocyclyl is selected from formulae iii-1 to iii-16; when formulae iii-1 to iii-16 are connected to the rest of the molecule through one bond, formulae iii-1 to iii-16 are monovalent; when formulae iii-1 to iii-16 are connected to the rest of the molecule through two bonds, formulae iii-1 to iii-16 are bivalent; the saturated or partially unsaturated heterocyclyl represented by formulae iii-1 to iii-16 each optionally are independently substituted by one or more substituents defined by the preceding corresponding claims;

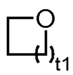 iii-1 , 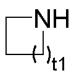 iii-2 , 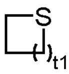 iii-3 , 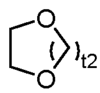 iii-4 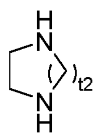 iii-5 , 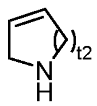 iii-6 , 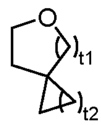 iii-7 , 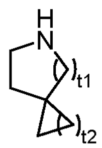 iii-8 , 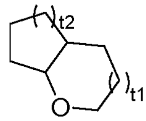 iii-9 , 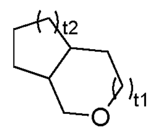 iii-10 ,

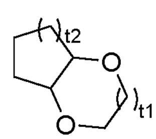 iii-11 , 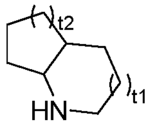 iii-12 , 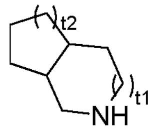 iii-13 , 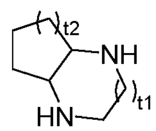 iii-14 , 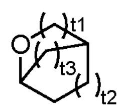 iii-15 or 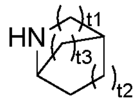 iii-16 ;

wherein, each t1 is independently 0, 1, 2, 3, 4, or 5; each t2 and t3 are independently 1, 2, 3, 4, or 5; or the saturated or partially unsaturated carbocyclyl is selected the following groups; when the saturated or partially unsaturated carbocyclyl is connected to the rest of the molecule through one bond, the following groups are monovalent; when the saturated or partially unsaturated carbocyclyl is connected to the rest of the molecule through two bonds, the following groups are bivalent; the following saturated or partially unsaturated carbocyclyl each optionally are independently substituted by one or more substituents defined by the preceding corresponding claims;

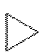 , 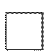 , 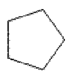 , 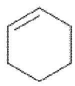 , 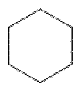 , 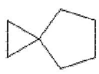 , 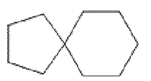 , 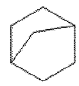 or 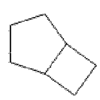 ;

the saturated or partially unsaturated heterocyclyl is selected the following groups; when the saturated or partially unsaturated heterocyclyl is connected to the rest of the molecule through one bond, the following groups are monovalent; when the saturated or partially unsaturated heterocyclyl is connected to the rest of the molecule through two bonds, the following groups are bivalent; the following saturated or partially unsaturated heterocyclyl each optionally are independently substituted by one or more substituents defined by the preceding corresponding claims;

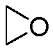 , 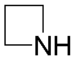 , 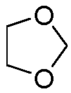 , 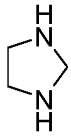 , 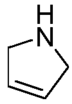 , 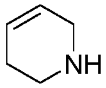 , 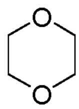 , 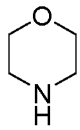 , 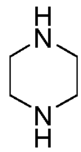 ,

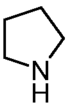 , 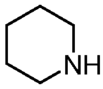 , 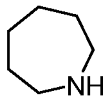 , 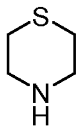 , 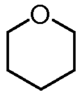 , 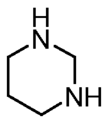 , 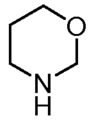 ,

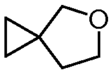 , 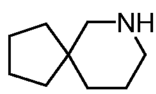 , 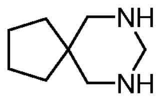 , 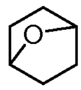 , 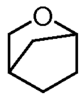 , 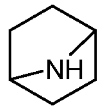 ,

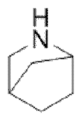 , 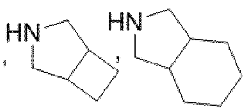 , 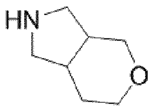 , 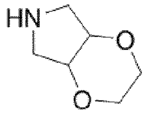 or 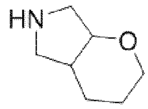 .

**8.** The compound according to any one of claims 1 to 7, which is a compound represented by any one of formulae II-1 to II-6 or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof:

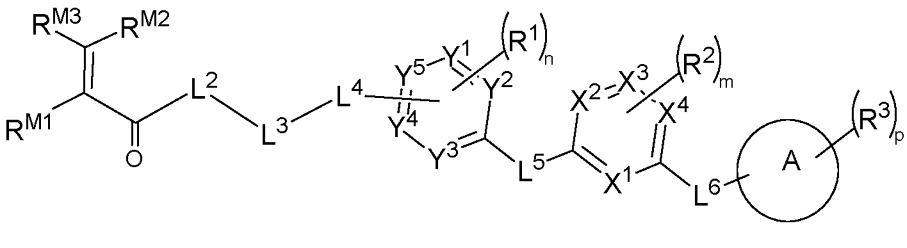

II-1,

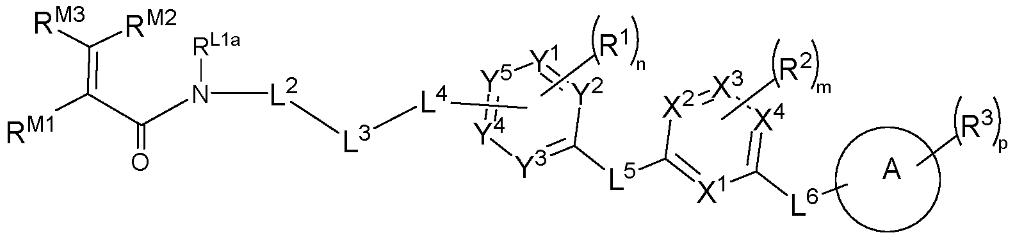

II-2,

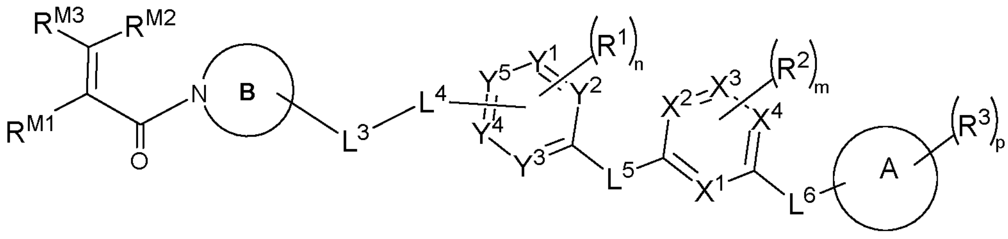

II-3,

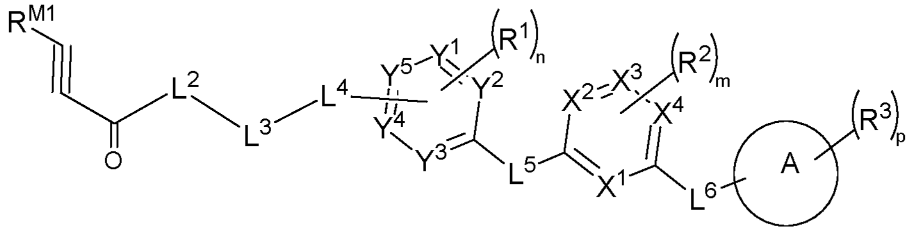

II-4,

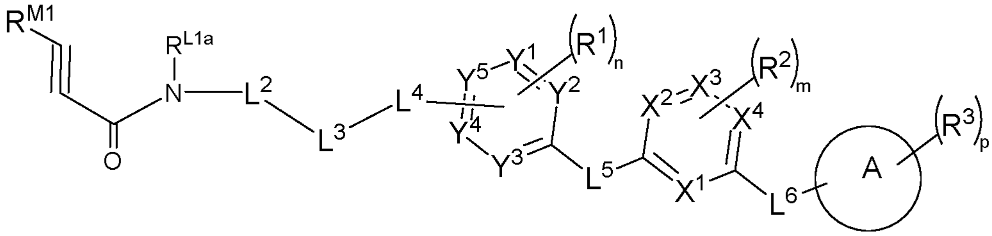

II-5,

or

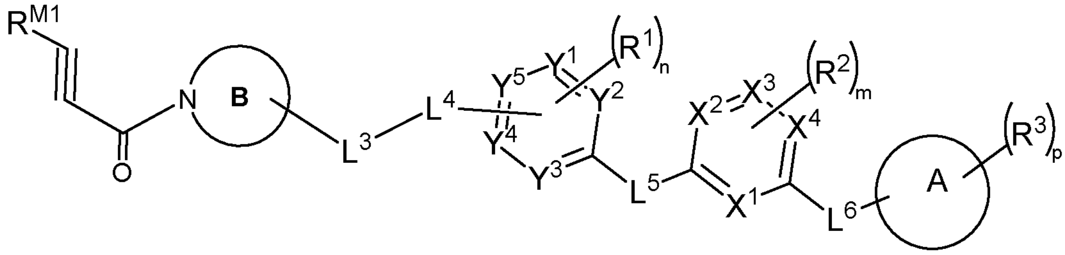

II-6;

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, $C_{1-4}$haloheteroalkyl, $C_{1-4}$alkoxy and $C_{1-4}$haloalkoxy.

**9.** The compound according to any one of claims 1 to 7, which is a compound of formula III or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof:

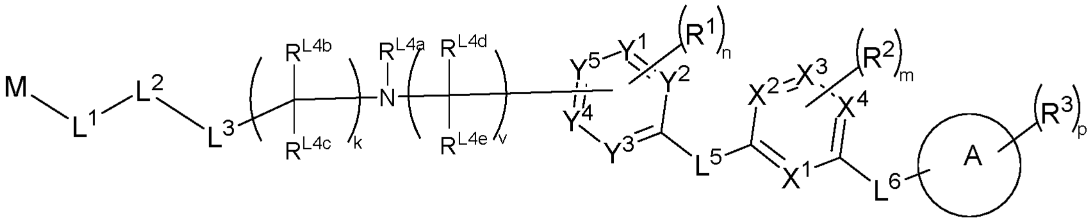

III

wherein, $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; k and v each are independently 0, 1 or 2.

**10.** The compound according to any one of claims 1 to 7, which is a compound represented by any one of formulae IV-1 to IV-6 or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof:

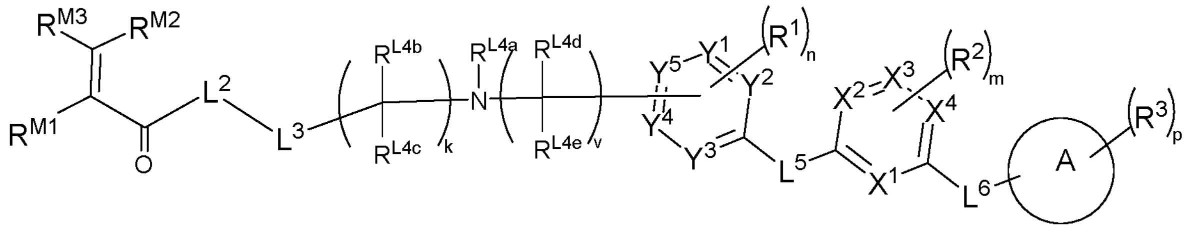

IV-1,

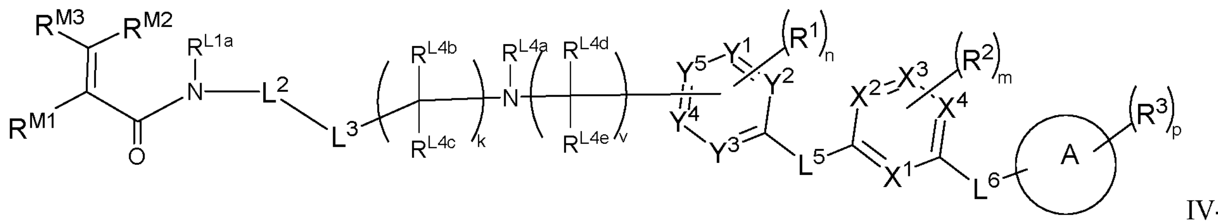

IV-2,

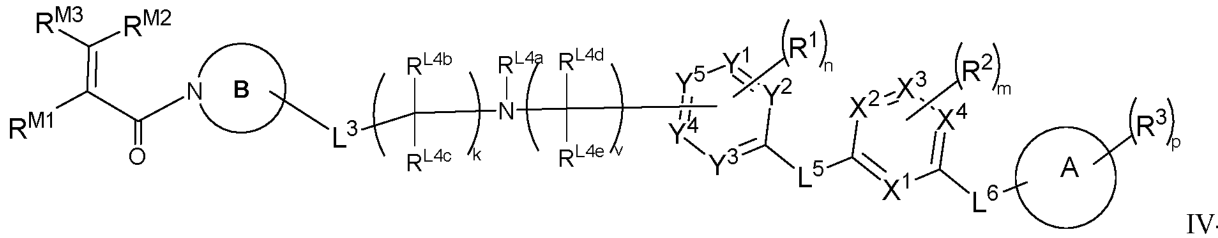

IV-3,

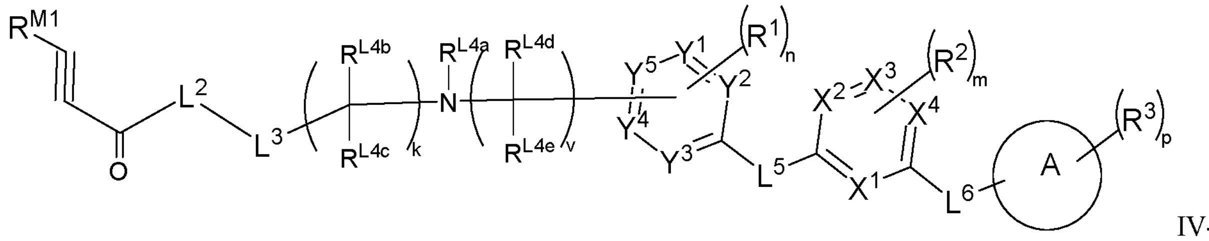

IV-4,

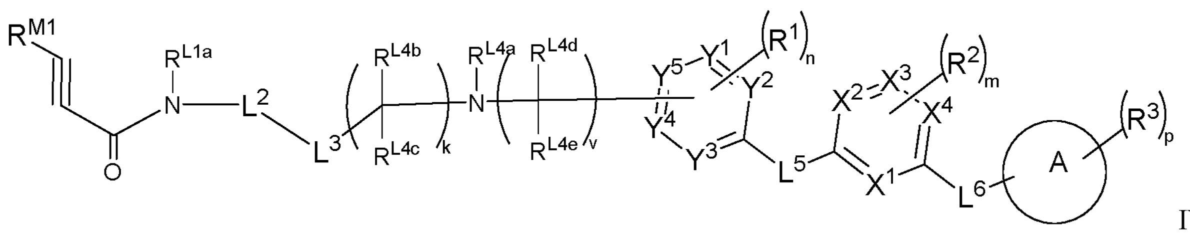

IV-5,

or

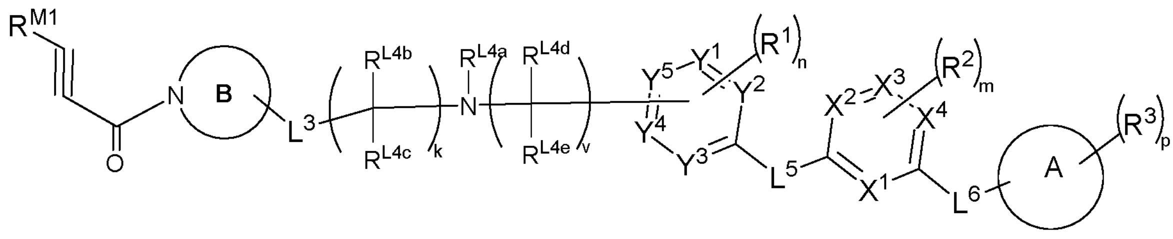

IV-6;

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, $C_{1-4}$haloheteroalkyl, $C_{1-4}$alkoxy and $C_{1-4}$haloalkoxy; $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; k and v each are independently 0, 1 or 2.

**11.** The compound according to any one of claims 1 to 7, which is a compound represented by any one of formulae V-1 to V-7 or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof:

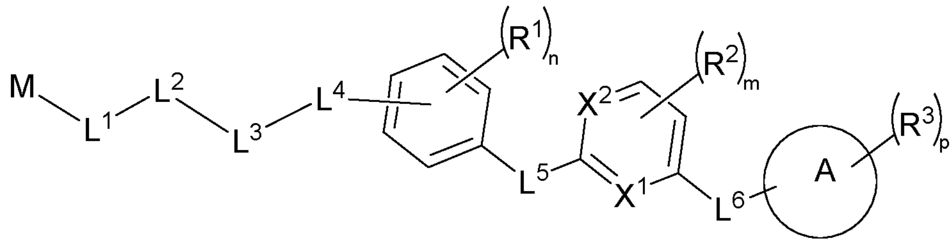

V-1,

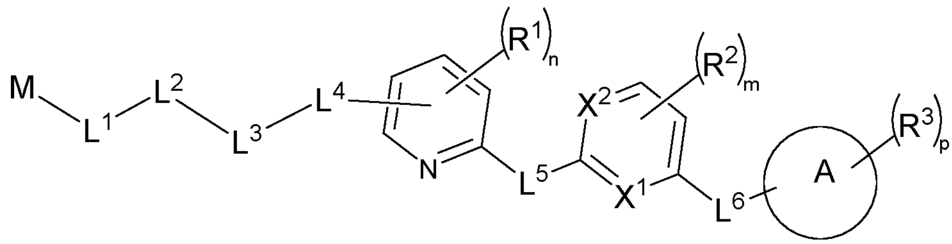

V-2,

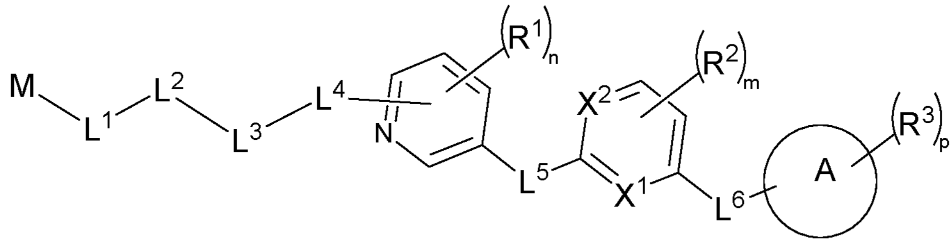

V-3,

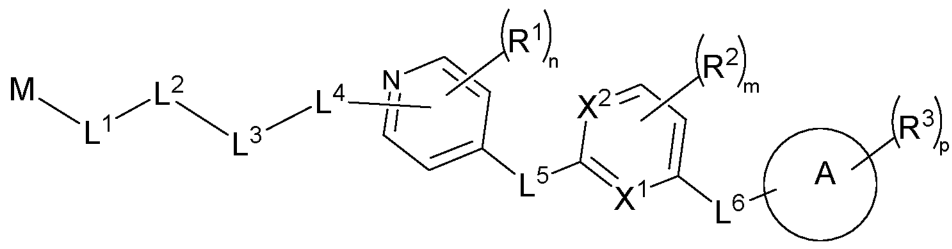

V-4,

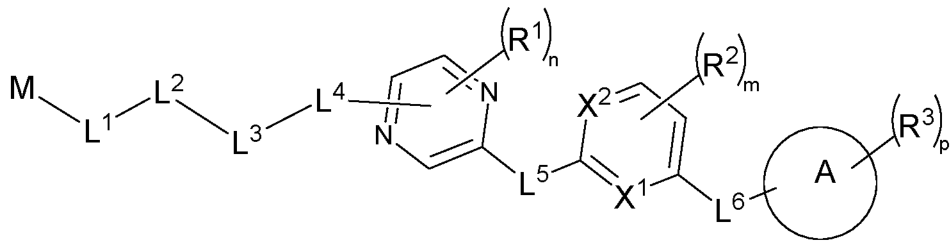

V-5,

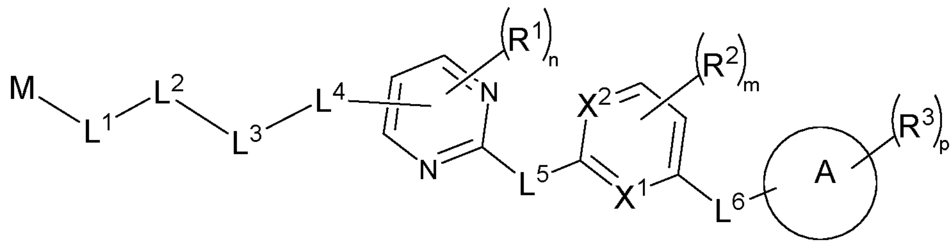

V-6,

or

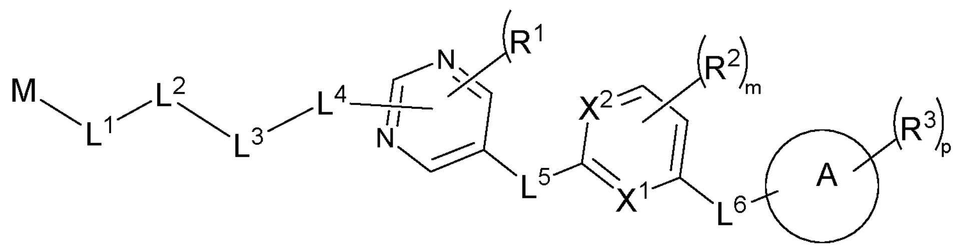

V-7.

**12.** The compound according to any one of claims 1 to 7, which is a compound represented by any one of formulae VI-1 to VI-5 or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof:

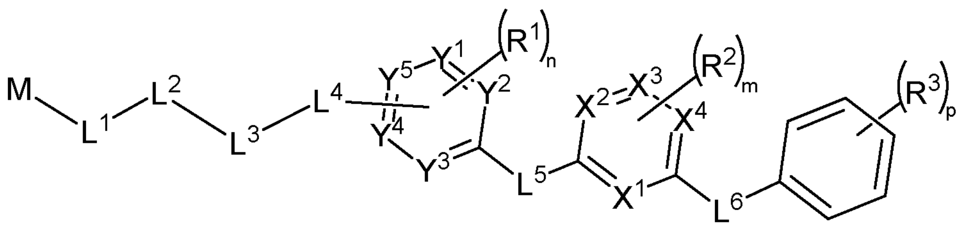

VI-1,

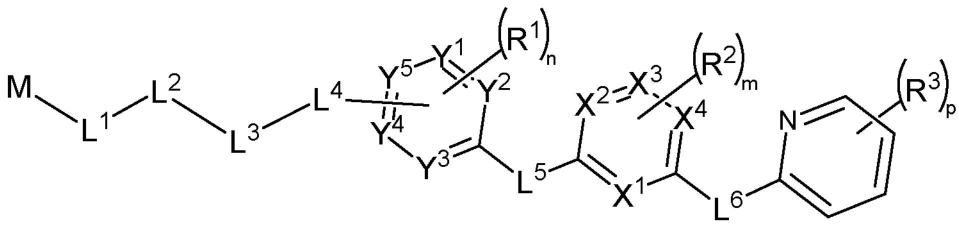

VI-2,

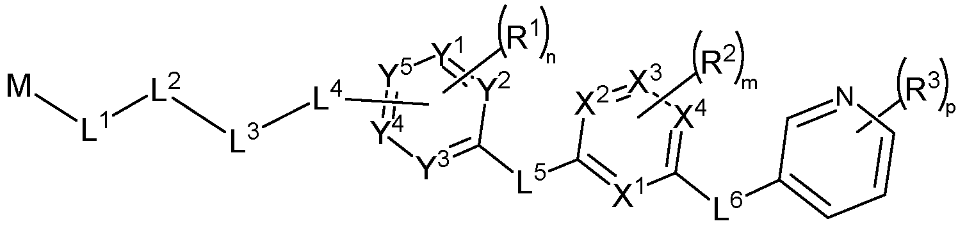

VI-3,

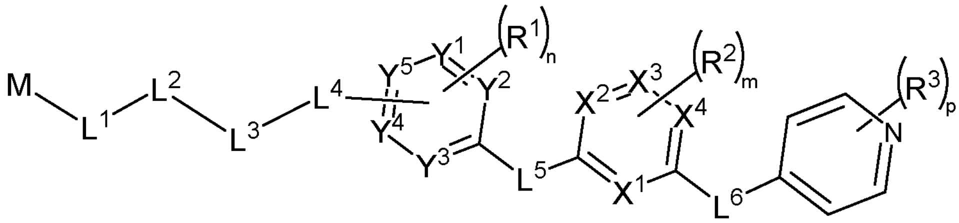

VI-4,

or

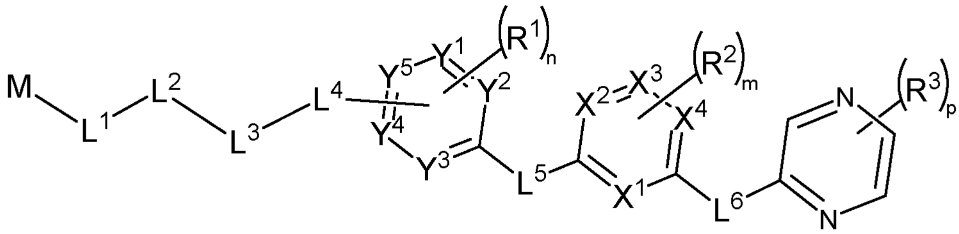

VI-5.

**13.** The compound according to any one of claims 1 to 7, which is a compound represented by any one of formulae VII-1 to VII-6 or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof:

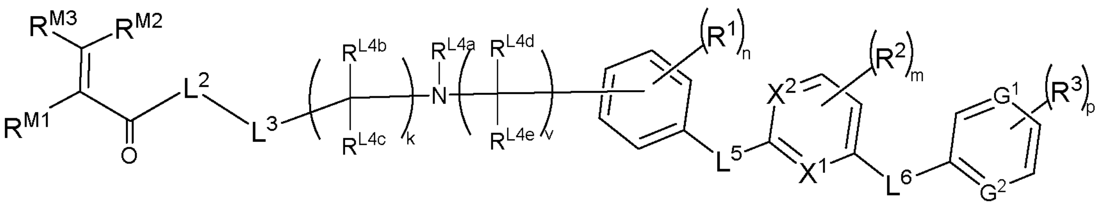

VII-1,

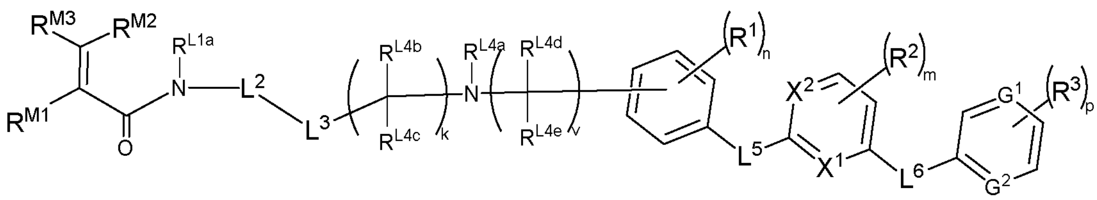

VII-2,

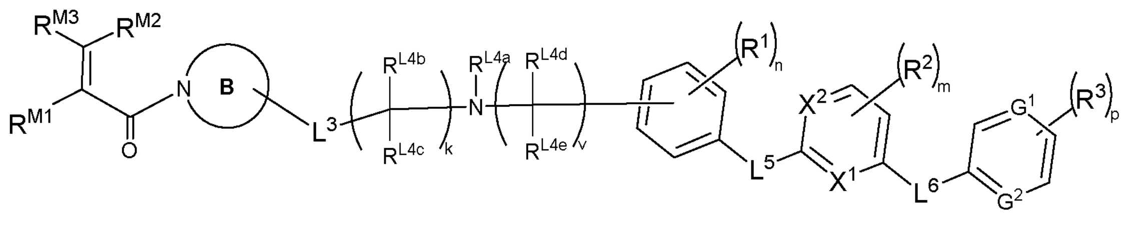

VII-3,

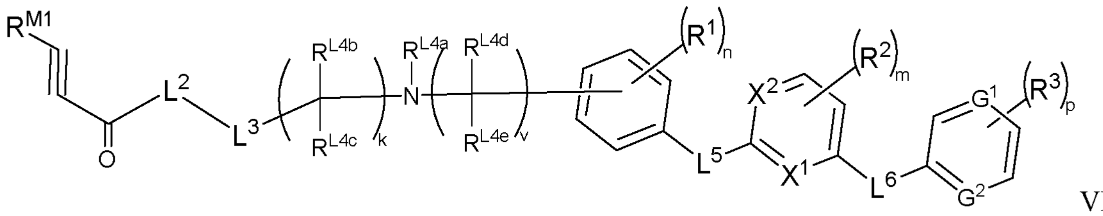

VII-4,

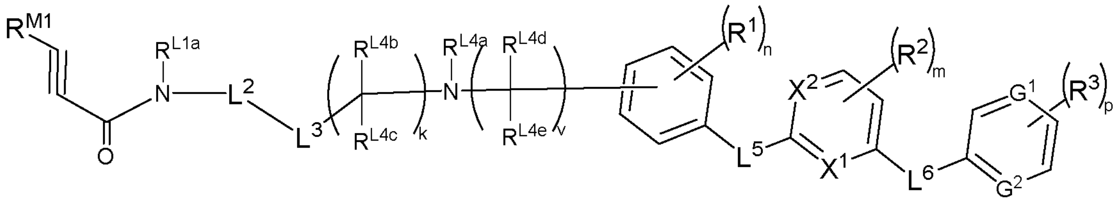

VII-5,

or

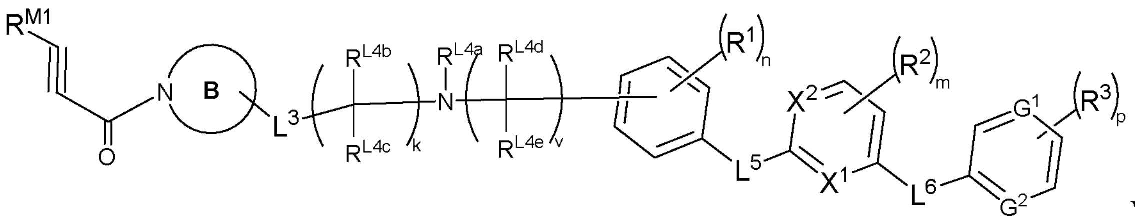

VII-6;

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, $C_{1-4}$haloheteroalkyl, $C_{1-4}$alkoxy and $C_{1-4}$haloalkoxy; $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; $G^1$ and $G^2$ each are independently CH or N; k and v each are independently 0, 1 or 2.

**14.** The compound according to any one of claims 1 to 7, which is a compound represented by any one of formulae VIII-1 to VIII-6 or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof:

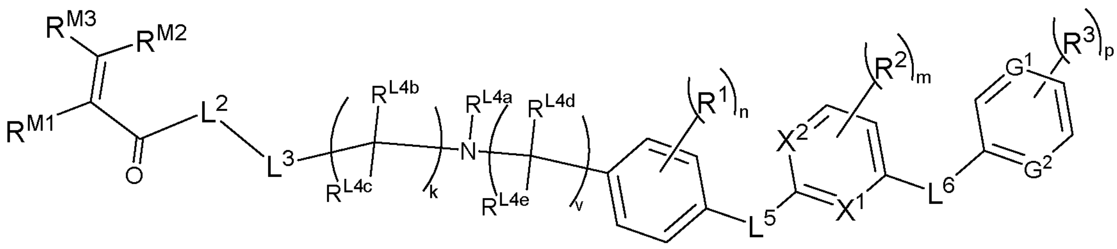

VIII-1,

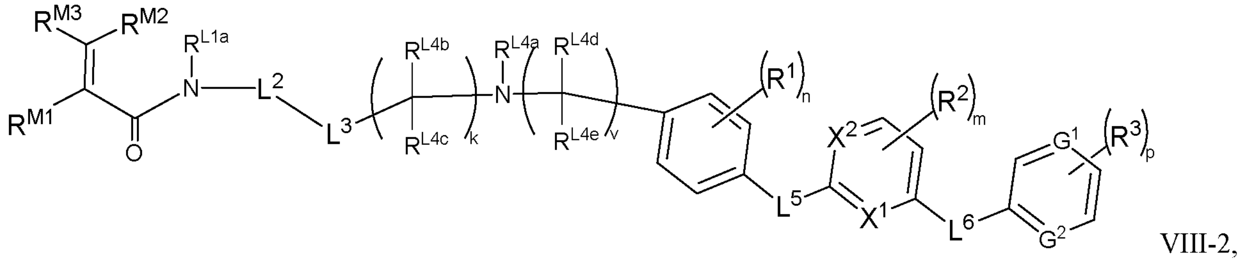

VIII-2,

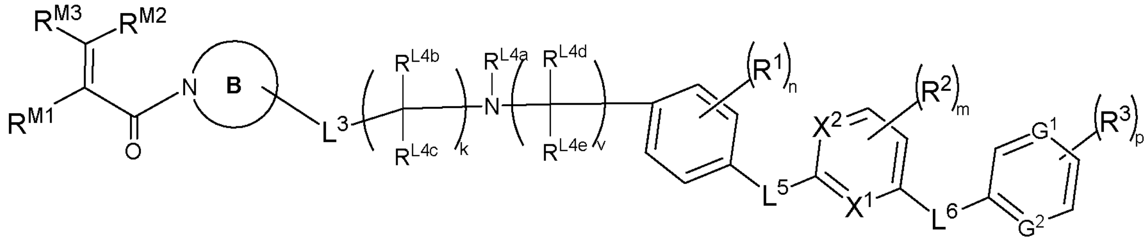

VIII-3,

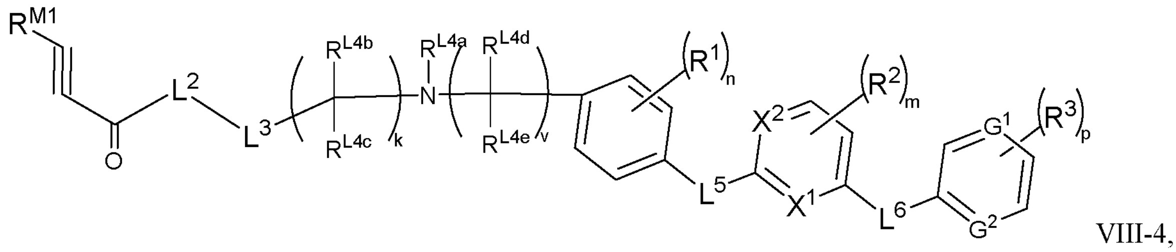

VIII-4,

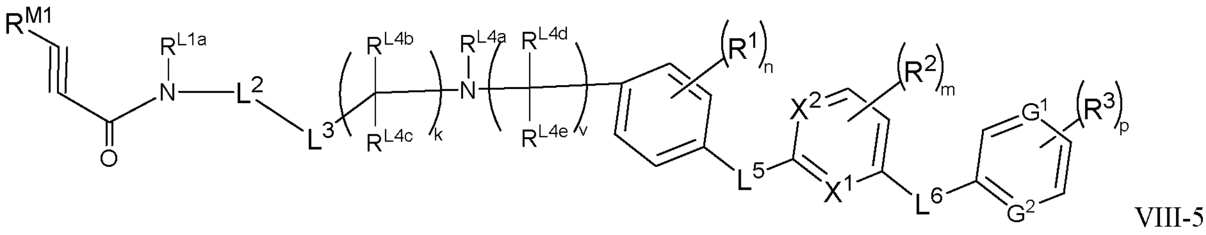

VIII-5,

or

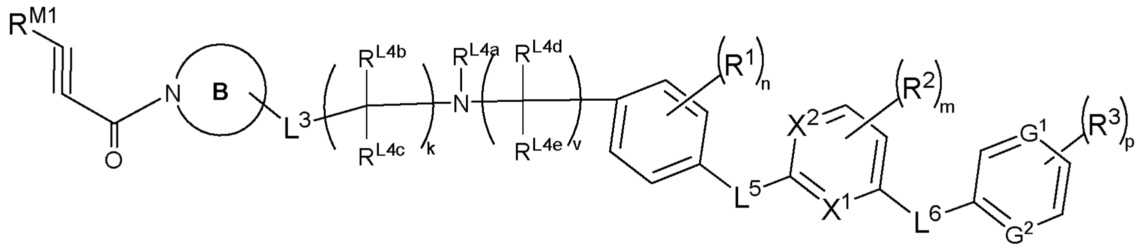

VIII-6;

wherein, B is a 3-6 membered saturated or partially unsaturated nitrogen-containing heterocyclyl, the 3-6 membered saturated or partially unsaturated nitrogen-containing heterocyclyl optionally is substituted by one or more substituents selected from the group consisting of deuterium, hydroxyl, amino, cyano, halogen, oxo, $C_{1-4}$alkyl, $C_{2-4}$alkenyl, $C_{2-4}$alkynyl, $C_{1-4}$heteroalkyl, $C_{1-4}$haloalkyl, $C_{1-4}$haloheteroalkyl, $C_{1-4}$alkoxy and $C_{1-4}$haloalkoxy; $R^{L4a}$ is hydrogen, deuterium, hydroxyl, cyano, halogen, oxo, ethenyl, ethynyl, methyl, ethyl, isopropyl, methoxy, ethoxy, trifluoromethoxy, or a nitrogen protecting group; each $R^{L4b}$, $R^{L4c}$, $R^{L4d}$ and $R^{L4e}$ are independently hydrogen, deuterium, hydroxyl, amino, cyano, halogen, ethenyl, ethynyl, methyl, ethyl, isopropyl, methoxy, ethoxy, trifluoromethoxy, or methylamino; wherein, $R^{L4b}$ and $R^{L4c}$ optionally form oxo; $R^{L4d}$ and $R^{L4e}$ optionally form oxo; $G^1$ and $G^2$ each are independently CH or N; k and v each are independently 0, 1 or 2.

**15.** The compound according to any one of claims 1 to 14, which is the following compound or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof:

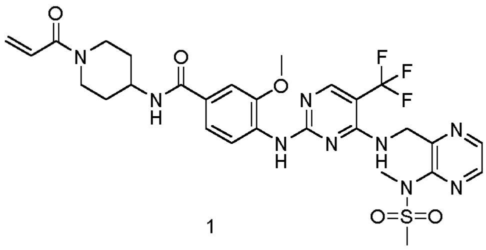

1

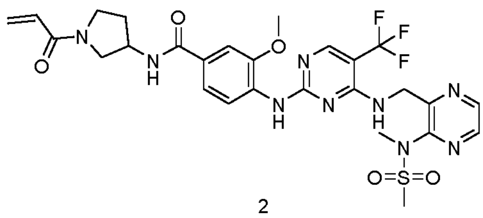

2

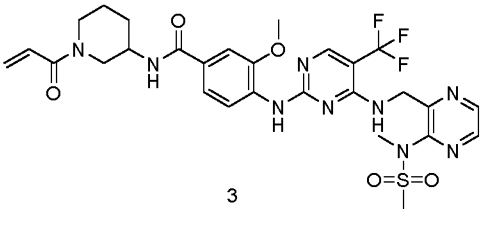

3

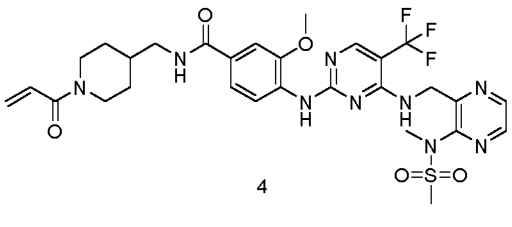

4

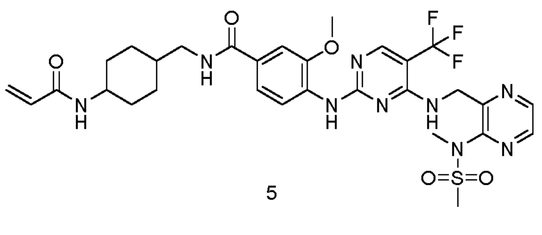

5

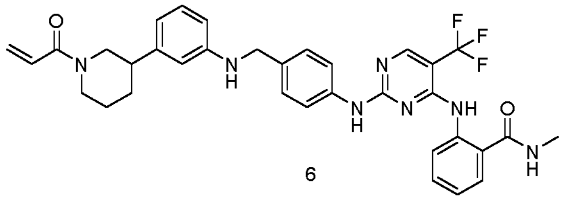

6

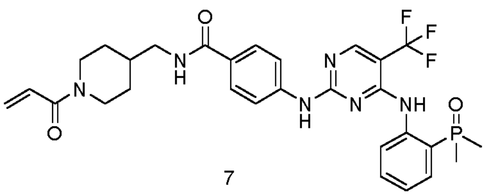

7

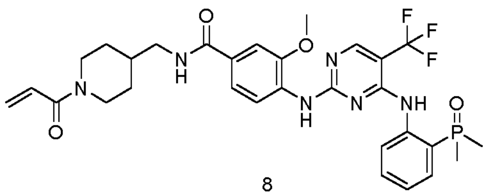

8

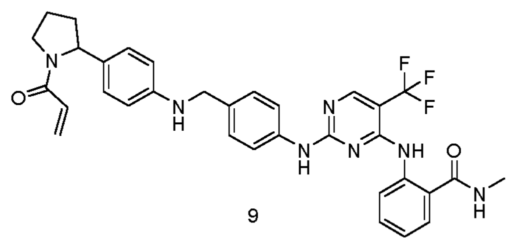
9

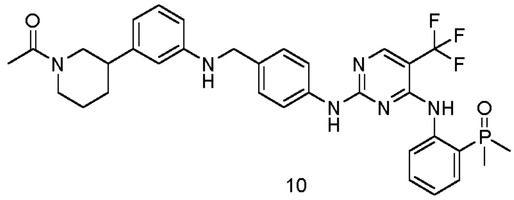
10

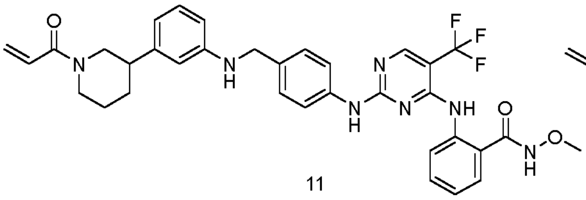
11

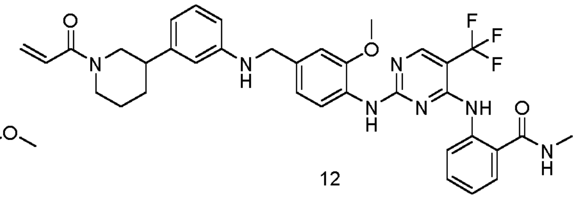
12

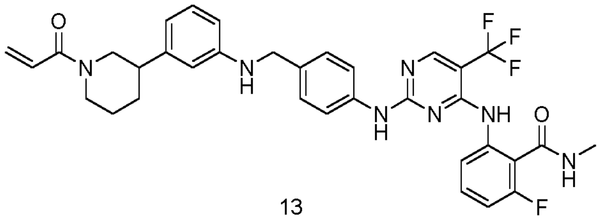
13

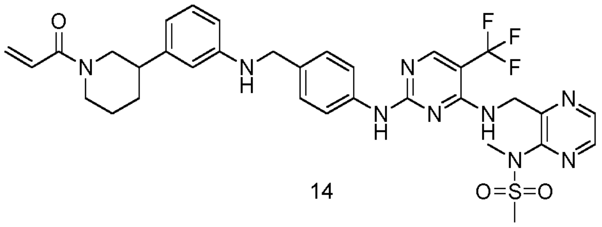
14

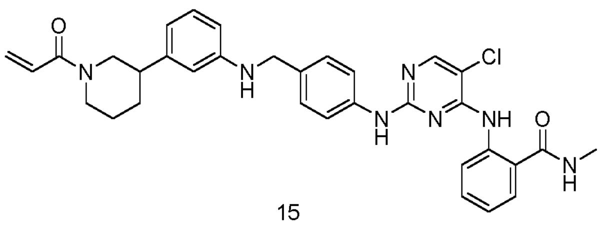
15

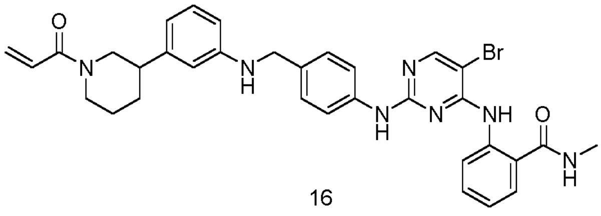
16

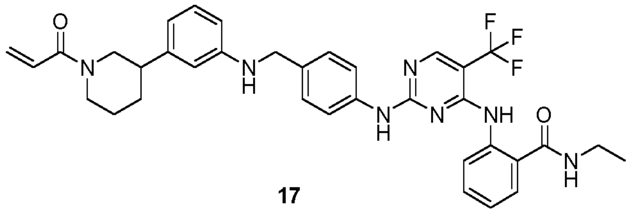
**17**

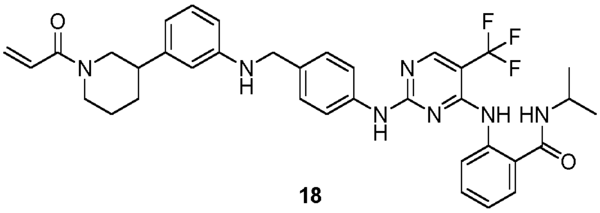
**18**

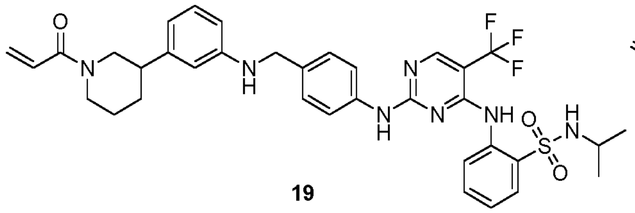
**19**

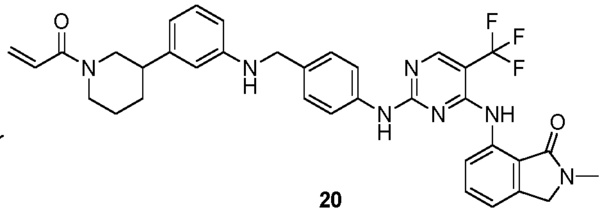
**20**

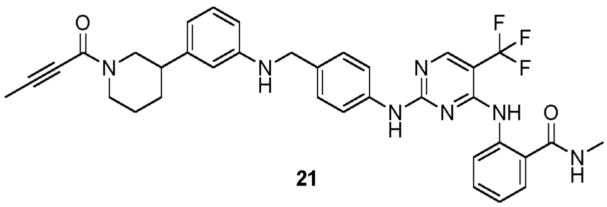

21

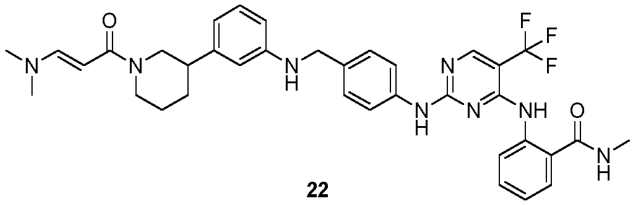

22

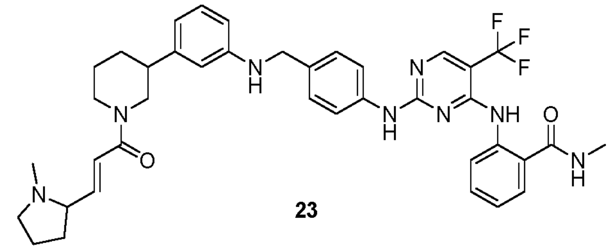

23

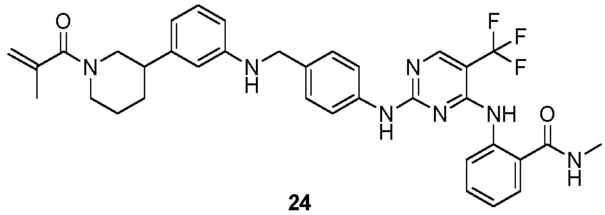

24

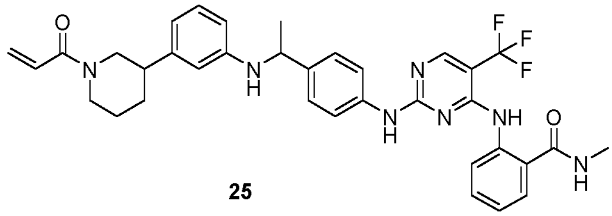

25

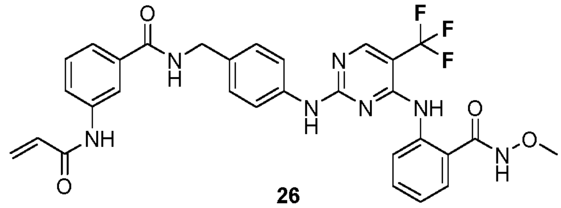

26

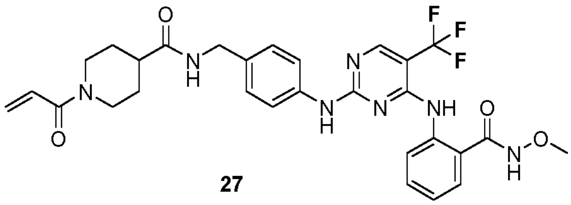

27

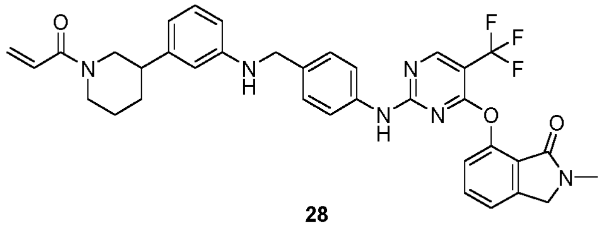

28

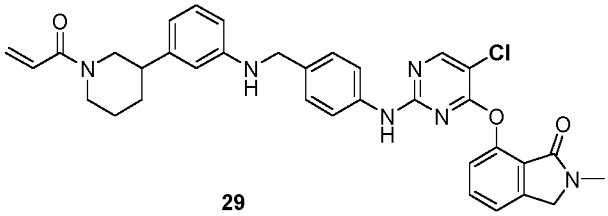

29

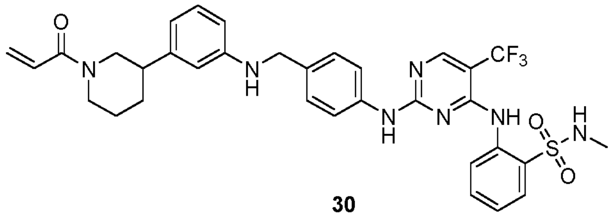

30

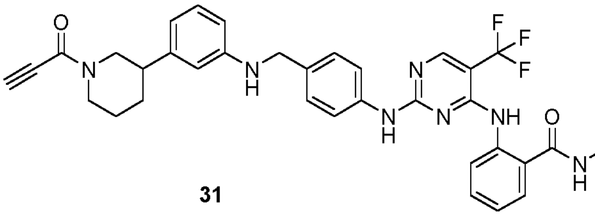

31

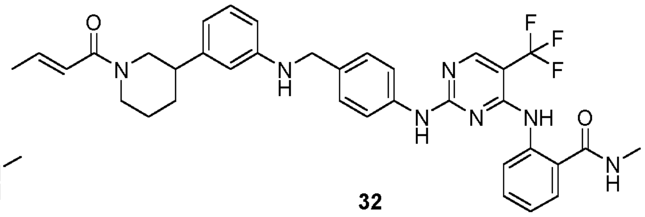

32

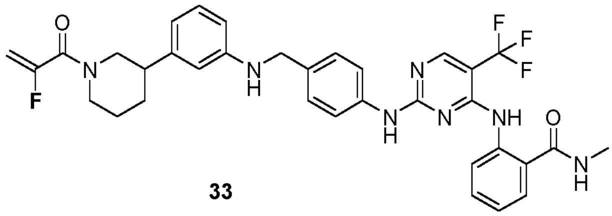

33

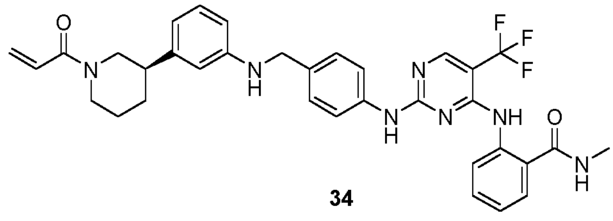

34

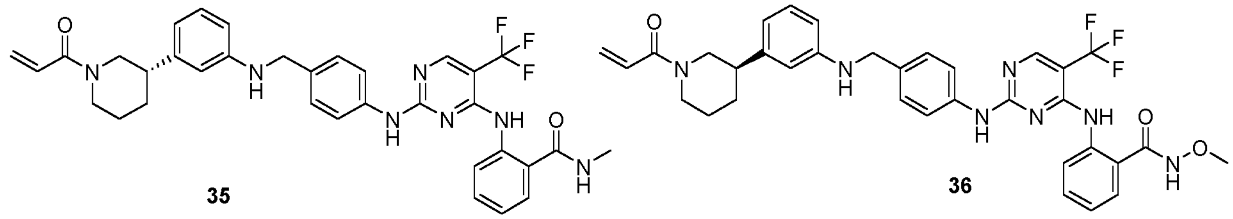

35

36

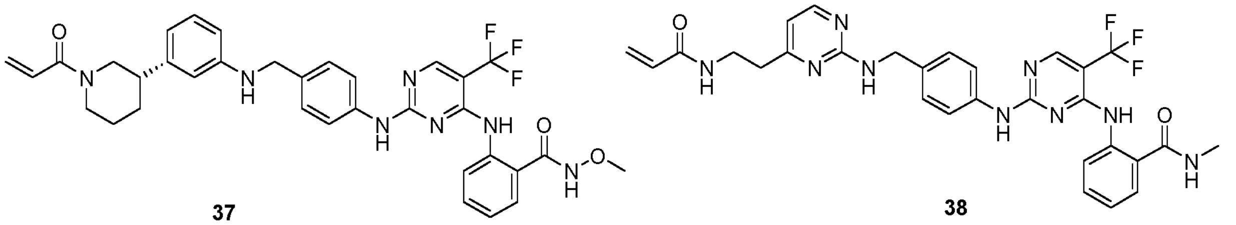

37

38

.

**16.** A pharmaceutical composition comprising the compound according to any one of claims 1-15 or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof, and a pharmaceutically acceptable excipient.

**17.** Use of the compound according to any one of claims 1-15 or the pharmaceutical composition according to claim 16 in the preparation of a medicament for the prevention or treatment of a FAK related disease.

**18.** The use according to claim 17, wherein the FAK related disease is selected from cancer, pulmonary arterial hypertension or pathological angiogenesis, preferably, the cancer includes lung cancer, bone cancer, pancreatic cancer, skin cancer, head and neck cancer, skin or intraocular melanoma, uterine cancer, ovarian cancer, rectal cancer, cancer in the anal region, gastric cancer, colon cancer, breast cancer, fallopian tube cancer, endometrial cancer, cervical cancer, sheath cancer, vulva cancer, Hodgkin's disease, esophageal cancer, small intestine cancer, endocrine system cancer, thyroid cancer, parathyroid cancer, adrenal cancer, soft tissue sarcoma, urethral cancer, penis cancer, prostatic cancer, chronic or acute leukemia, lymphocytic lymphoma, bladder cancer, kidney or ureter cancer, renal cell cancer, renal pelvis cancer, central nervous system (CNS) tumor, primary CNS lymphoma, spinal axis cancer, brain stem glioma, pituitary adenoma, or a combination of the aforementioned cancers.

**19.** Use of the compound according to any one of claims 1-15 or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof or the pharmaceutical composition according to claim 16 in the preparation of a medicament for regulating or treating a YAP related disease; preferably, the YAP related disease is a cancer selected from one or more of skin cancer, bone cancer, glioma, breast cancer, adrenal cancer, bladder cancer, esophageal cancer, head or neck cancer, liver cancer, parathyroid cancer, penis cancer, small intestine cancer, thyroid cancer, urethral cancer, cervical cancer, endometrial cancer, fallopian tube cancer, renal pelvis cancer, vaginal cancer, vulva cancer, chronic or acute leukemia, colon cancer, melanoma, hematological malignancy, Hodgkin's lymphoma, lung cancer, lymphocytic lymphoma, central nervous system (CNS) tumor, ovarian cancer, pancreatic cancer, pituitary adenoma, prostatic cancer, soft tissue sarcoma, gastric cancer and uterine cancer; more preferably, the cancer is selected from one or more of lung cancer, colon cancer, ovarian cancer, prostatic cancer, and liver cancer.

**20.** Use of the compound according to any one of claims 1-15 or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof or the pharmaceutical composition according to claim 16 in the preparation of a medicament for regulating or treating a FAK and YAP related disease, preferably the FAK and YAP related disease is selected from cancer.

**21.** A method for regulating the Hippo-YAP signaling pathway, comprising administering the compound according to any one of claims 1-15 or a stereoisomer, a tautomer, an enantiomer, a diastereomer, a racemate, a geometric isomer, a nitrogen oxide, a solvate, a hydrate, a crystal form, an ester, an isotope labeled compound (preferably a deuterate), a metabolite, a pharmaceutically acceptable salt, or a prodrug thereof or the pharmaceutical composition according to claim 16.

**22.** Use of the compound according to any one of claims 1-15 or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof or the pharmaceutical composition according to claim 16 in the preparation of a Hippo-YAP signaling pathway inhibitor.

**23.** A method for preventing or treating a YAP related disease, comprising administering the compound according to any one of claims 1-15 or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof or the pharmaceutical composition according to claim 16;
preferably, the YAP related disease includes one or more of skin cancer, bone cancer, glioma, breast cancer, adrenal cancer, bladder cancer, esophageal cancer, head or neck cancer, liver cancer, parathyroid cancer, penis cancer, small intestine cancer, thyroid cancer, urethral cancer, cervical cancer, endometrial cancer, fallopian tube cancer, renal pelvis cancer, vaginal cancer, vulva cancer, chronic or acute leukemia, colon cancer, melanoma, hematological malignancy, Hodgkin’s lymphoma, lung cancer, lymphocytic lymphoma, central nervous system (CNS) tumor, ovarian cancer, pancreatic cancer, pituitary adenoma, prostatic cancer, soft tissue sarcoma, gastric cancer and uterine cancer.

**24.** A method for preventing or treating a FAK related disease, comprising administering the compound according to any one of claims 1-15 or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof, or the pharmaceutical composition according to claim 16; preferably, the FAK related disease is selected from cancer, pulmonary arterial hypertension, or pathological angiogenesis.

**25.** A method for inhibiting FAK kinase and/or YAP protein activity in a cell or subject, comprising contacting the cell with the compound according to any one of claims 1-15 or a stereoisomer, a tautomer, an enantiomer, a diastereomer, a racemate, a geometric isomer, a nitrogen oxide, a solvate, a hydrates, a crystal form, an ester, an isotope labeled compound (preferably a deuterate), a metabolite, a pharmaceutically acceptable salt, or a prodrug thereof, or the pharmaceutical composition according to claim 16; or administering to the subject the compound according to any one of claims 1-15 or a stereoisomer, a tautomer, an enantiomer, a diastereomer, a racemate, a geometric isomer, a nitrogen oxide, a solvate, a hydrate, a crystal form, an ester, an isotope labeled compound (preferably a deuterate), a metabolite, a pharmaceutically acceptable salt, or a prodrug thereof, or the pharmaceutical composition according to claim 16.

**26.** A method for preparing the compound according to any one of claims 1-15, comprising one or more of steps (1) to (3), or one or more of steps (4) to (9):
(1) reacting the compound of formula a1 with compound a2 to produce the compound of formula a3; (2) reacting the compound of formula a3 with compound a4 to produce the compound of formula a5; (3) reacting the compound of formula a5 with the compound of formula a6 to produce the compound of formula a7;

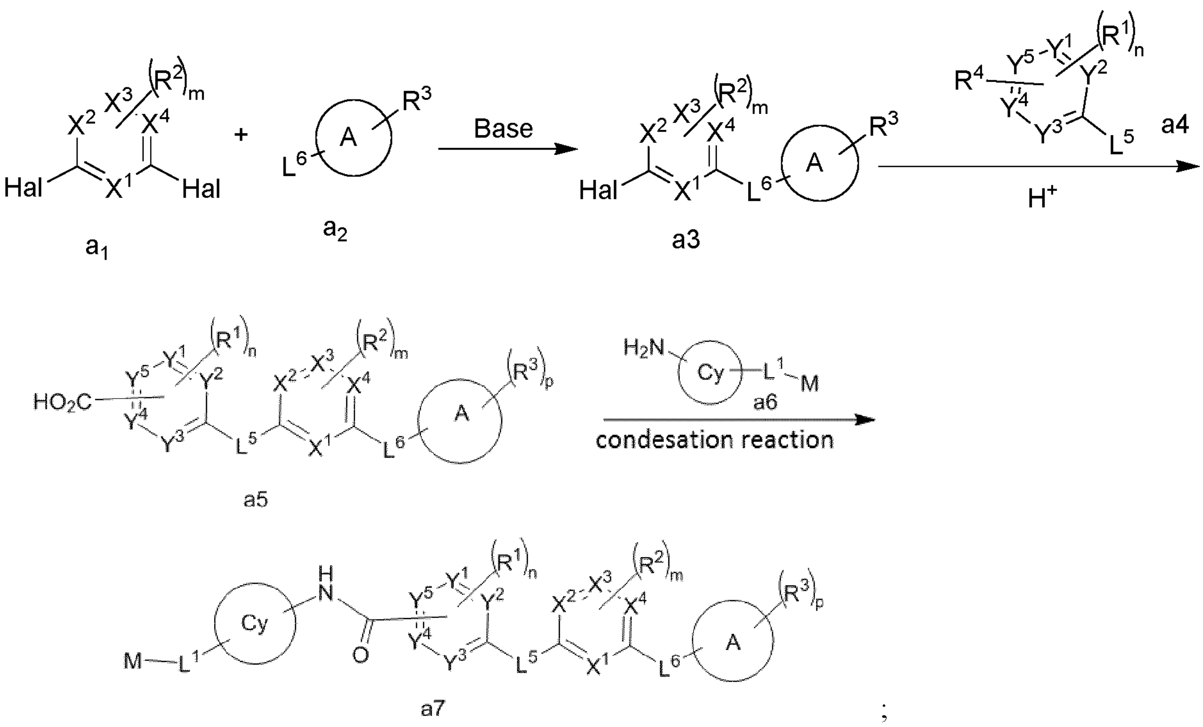


;

(4) reacting the compound of formula b1 with the compound of formula b2 to produce the compound of formula b3; (5) converting the compound of formula b3 into the compound of formula b4; (6) converting the compound of formula b4 into the compound of formula b5; (7) reacting the compound of formula b5 with the compound of formula b3 to produce the compound of formula b6; (8) converting the compound of formula b6 into the compound of formula b7; (9) converting the compound of formula b7 into the compound of formula b8,

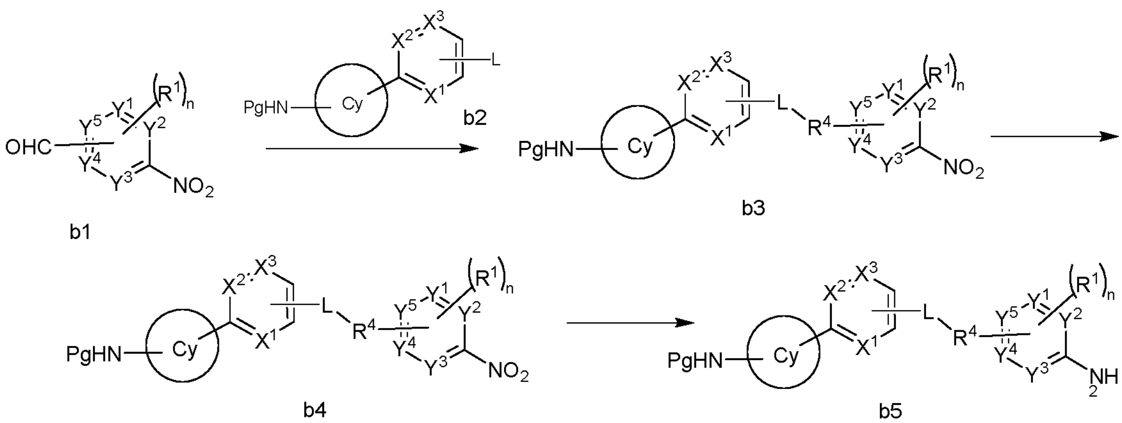

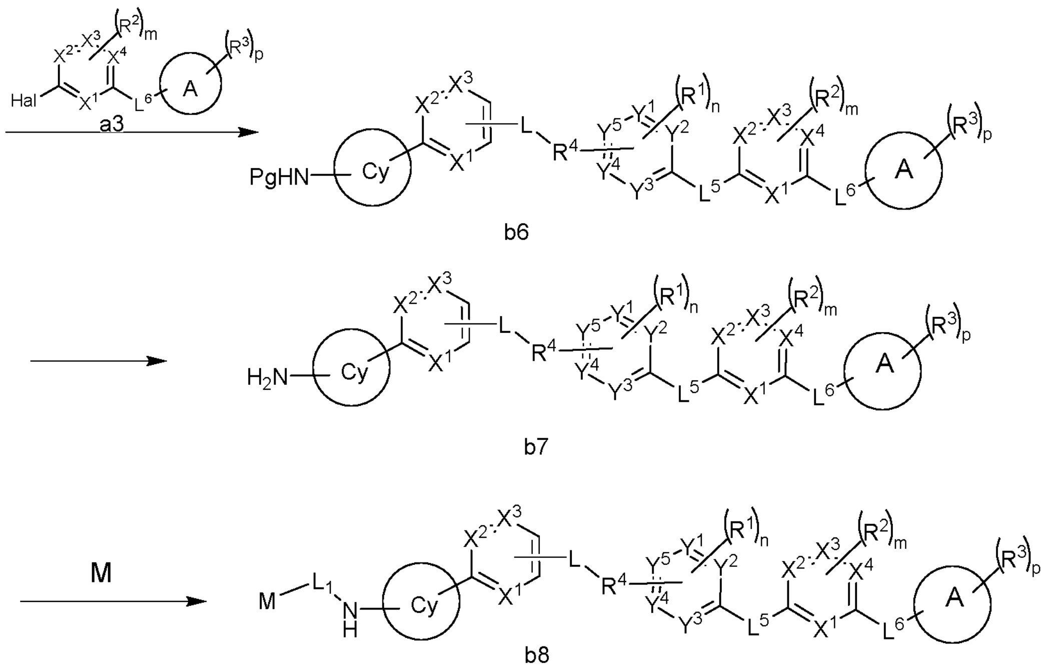


b6

b7

b8

wherein Hal is halogen, Cy represents a structure of various ring systems, preferably 3-6 membered saturated or partially unsaturated carbocyclyl, 3-6 membered saturated or partially unsaturated heterocyclyl, 6-10 membered aryl, or 5-10 membered heteroaryl, Pg represents a protecting group; A, $R^1$, $R^2$, $R^3$, $R^4$, $X^1$, $X^2$, $X^3$, $X^4$, $Y^1$, $Y^2$, $Y^3$, $Y^4$, $Y^5$, $L^1$, $L^2$, $L^5$, $L^6$, M, n, m, and p are defined as in claim 1.

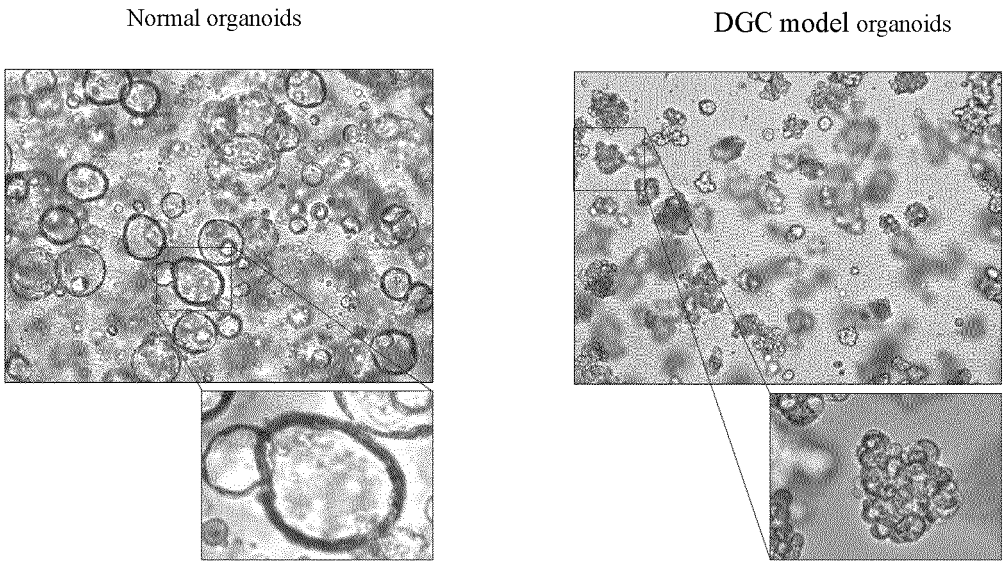


Figure 1

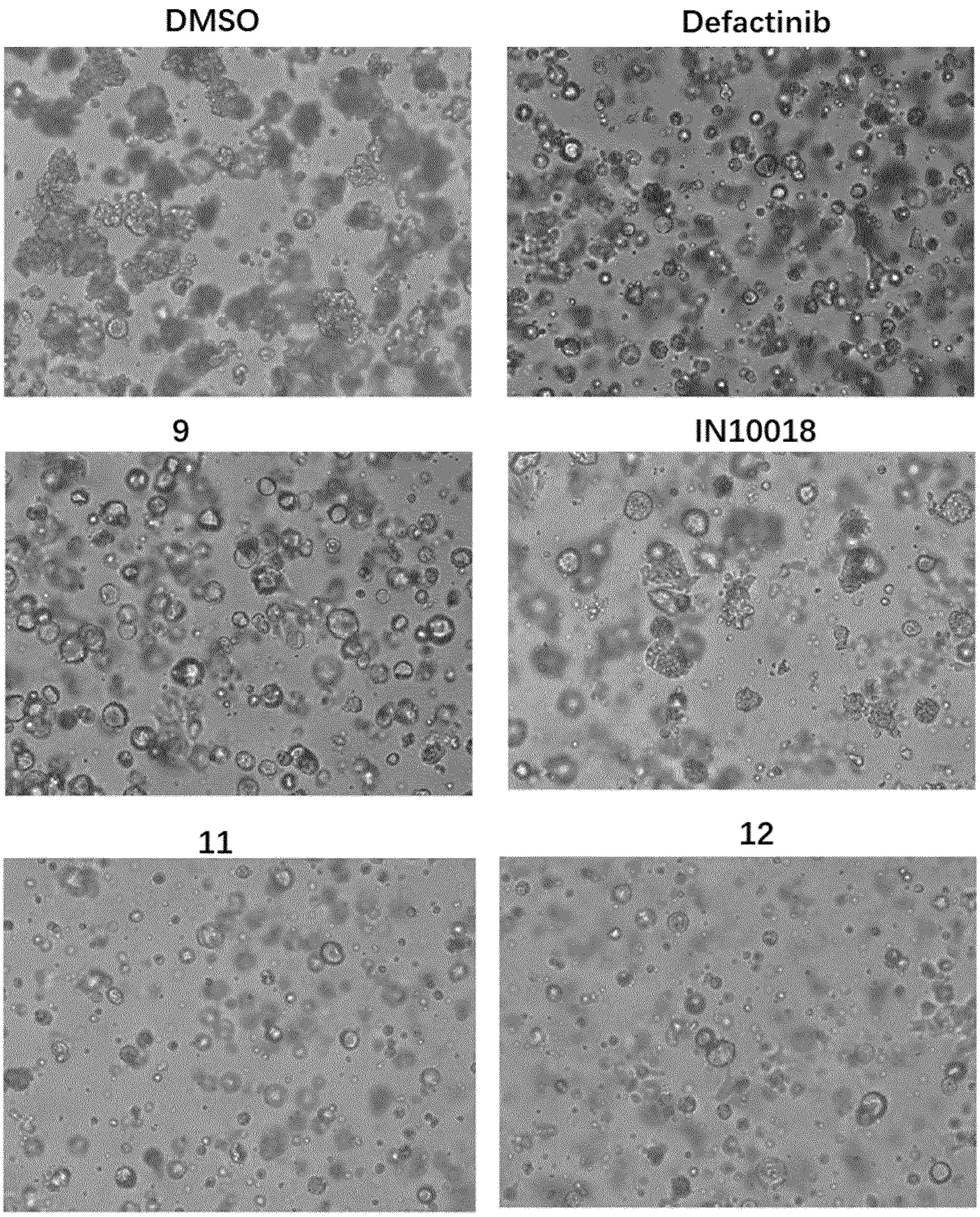


Figure 2

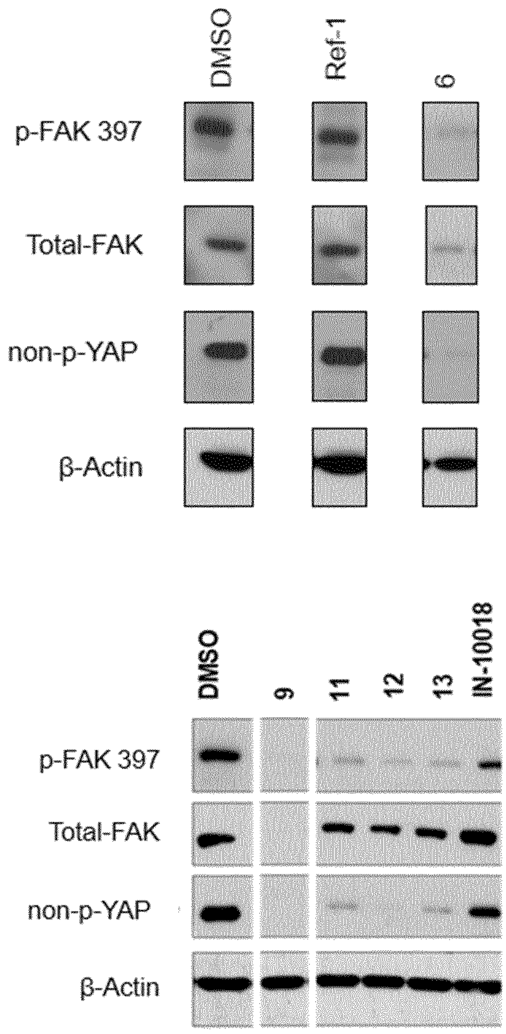


Figure 3

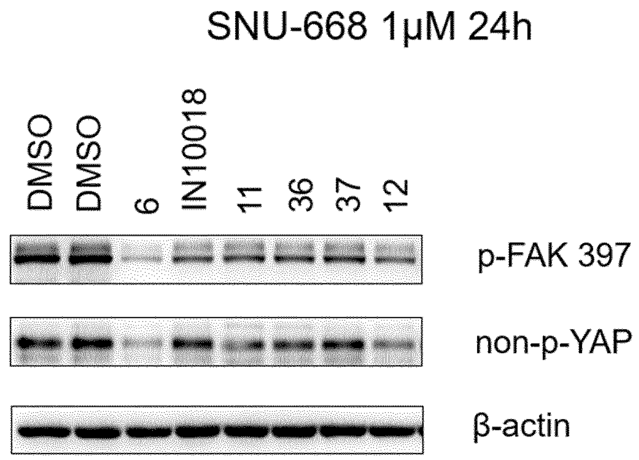


Figure 4

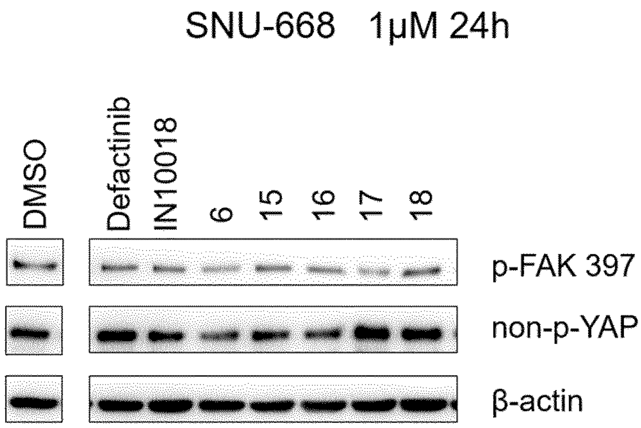


Figure 5

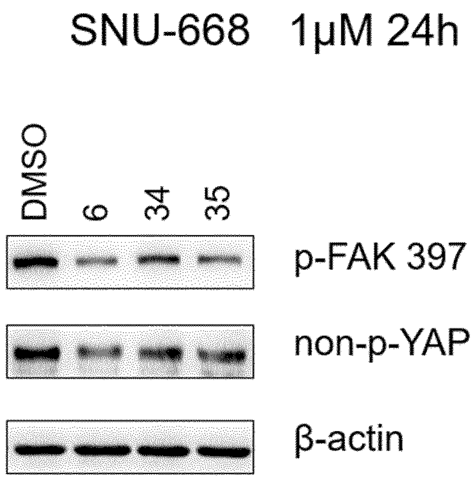


Figure 6

**INTERNATIONAL SEARCH REPORT**

International application No.
**PCT/CN2022/138383**

**A. CLASSIFICATION OF SUBJECT MATTER**

C07D401/12(2006.01)i;A61K31/506(2006.01)i;A61P35/00(2006.01)i

According to International Patent Classification (IPC) or to both national classification and IPC

**B. FIELDS SEARCHED**

Minimum documentation searched (classification system followed by classification symbols)

C07D,A61K,A61P

Documentation searched other than minimum documentation to the extent that such documents are included in the fields searched

Electronic data base consulted during the international search (name of data base and, where practicable, search terms used)

WPI; CNABS; EPTXT; USTXT; WOTXT; JPTXT; CNTXT; CNKI, Registry(STN), Caplus(STN), Marpat(STN): 希格生科, 张海生, 卓鉥, 胡亚兵, 代长贵, 程辉敏, 牛春意, 方磊, 陈誉, YAP, FAK, tumo?r, cancer, pyrimidine, 黏着斑激酶, 肿瘤, 癌, 嘧啶, 结构式检索, search for structural formula

**C. DOCUMENTS CONSIDERED TO BE RELEVANT**

| Category* | Citation of document, with indication, where appropriate, of the relevant passages | Relevant to claim No. |
|---|---|---|
| X | CN 102105150 A (ARIAD PHARMACEUTICALS, INC.) 22 June 2011 (2011-06-22) description, pages 1-2, paragraph [0333], page 180, table and embodiments | 1-26 |
| X | CN 103641833 A (IRM LLC) 19 March 2014 (2014-03-19) description, pages 1-3 and 7 and embodiments | 1-26 |
| X | CN 101921236 A (NOVARTIS AG) 22 December 2010 (2010-12-22) description, pages 1-3 and 10 and embodiments | 1-26 |
| X | CN 102482277 A (DANA-FARBER CANCER INSTITUTE, INC.) 30 May 2012 (2012-05-30) description, pages 3-7, paragraphs [0428] and [0782], embodiments and table 2, and claim 119 | 1-26 |
| X | CN 111683662 A (CHEN, Zhihong) 18 September 2020 (2020-09-18) description, pages 1-2 and embodiments | 1-26 |
| X | CN 1335838 A (ASTRAZENECA AB) 13 February 2002 (2002-02-13) description, pages 2-6 and 57 and embodiments | 1-26 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 February 2023** | **15 February 2023** |
| Name and mailing address of the ISA/CN<br>**China National Intellectual Property Administration (ISA/CN)**<br>**China No. 6, Xitucheng Road, Jimenqiao, Haidian District, Beijing 100088**<br>Facsimile No. **(86-10)62019451** | Authorized officer<br><br>Telephone No. |

Form PCT/ISA/210 (second sheet) (July 2022)

**INTERNATIONAL SEARCH REPORT**

International application No. **PCT/CN2022/138383**

**C. DOCUMENTS CONSIDERED TO BE RELEVANT**

| Category* | Citation of document, with indication, where appropriate, of the relevant passages | Relevant to claim No. |
|---|---|---|
| X | YEN-PON, Expédite et al. "Structure-Based Design, Synthesis, and Characterization of the First Irreversible Inhibitor of Focal Adhesion Kinase" *ACS Chem. Biol.,* Vol. vol. 13, 13 June 2018 (2018-06-13), pp. 2067-2073 | 1-14 |
| X | LI, Bo et al. "Design, Synthesis, and Biological Evaluation of Covalent Inhibitors of Focal Adhesion Kinase (FAK) against Human Malignant Glioblastoma" *J. Med. Chem,* Vol. vol. 63, 29 October 2020 (2020-10-29), pp. 12707-12724 | 1-14 |

Form PCT/ISA/210 (second sheet) (July 2022)

**INTERNATIONAL SEARCH REPORT**

International application No.
**PCT/CN2022/138383**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **21, 23-25**
because they relate to subject matter not required to be searched by this Authority, namely:

The subject matter of claims 21 and 23-25 relates to a disease treatment method for the human or animal body, belonging to the case stipulated by PCT Rule 39.1(iv).

The search is carried out on the basis of the use of the foregoing compound in the preparation of a drug for a corresponding disease.

2. ☑ Claims Nos.: **1-14,16-26**
because they relate to parts of the international application that do not comply with the prescribed requirements to such an extent that no meaningful international search can be carried out, specifically:

The scope of the structures defined in claims 1-14 is very broad. The compounds involved cover a large number of known compounds in the prior art. Claims 16-26 essentially refer to any one of said claims, which makes it difficult for the examiner to perform an exhaustive search of the prior art for the scope of the current claims. The present search report is provided on the basis of the structure of the parent ring of formula V-1 in claim 11 (and wherein X1 and X2 are N, formula A being a unit or a multi-membered ring comprising a benzene ring or a pyrazine fragment, and specifying that L5 and L6 are NH, and L4 is a heterochain).

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/138383**

| Patent document cited in search report | Publication date (day/month/year) | Patent family member(s) | Publication date (day/month/year) |
|---|---|---|---|
| CN 102105150 A | 22 June 2011 | LTPA 2019510 I1 | 10 June 2019 |
| | | LTC 2300013 I2 | 11 April 2022 |
| | | HUS 1900029 I1 | 28 April 2020 |
| | | NO 2300013 T3 | 03 February 2018 |
| | | US 2020317705 A1 | 08 October 2020 |
| | | JP 2022116057 A | 09 August 2022 |
| | | US 2012202776 A1 | 09 August 2012 |
| | | US 9012462 B2 | 21 April 2015 |
| | | KR 20160132127 A | 16 November 2016 |
| | | KR 101860057 B1 | 21 May 2018 |
| | | CY 1119534 T1 | 04 April 2018 |
| | | IL 208716 A0 | 30 December 2010 |
| | | IL 208716 B | 28 February 2018 |
| | | US 2015225436 A1 | 13 August 2015 |
| | | US 2020048288 A1 | 13 February 2020 |
| | | EP 2300013 A1 | 30 March 2011 |
| | | EP 2300013 A4 | 12 September 2012 |
| | | EP 2300013 B1 | 06 September 2017 |
| | | MX 353308 B | 08 January 2018 |
| | | CA 2723961 A1 | 26 November 2009 |
| | | CA 2723961 C | 21 March 2017 |
| | | IL 257083 A | 29 March 2018 |
| | | IL 257083 B | 29 August 2019 |
| | | JP 2017186345 A | 12 October 2017 |
| | | JP 6271064 B2 | 31 January 2018 |
| | | BRPI 0908637 A2 | 23 October 2018 |
| | | BRPI 0908637 B1 | 17 November 2020 |
| | | BRPI 0908637 B8 | 25 May 2021 |
| | | NO 2019024 I1 | 20 May 2019 |
| | | LUC 00120 I1 | 24 May 2019 |
| | | LUC 00120 I2 | 27 December 2019 |
| | | HUE 035029 T2 | 28 March 2018 |
| | | JP 2020125308 A | 20 August 2020 |
| | | SI 2300013 T1 | 30 March 2018 |
| | | WO 2009143389 A1 | 26 November 2009 |
| | | EP 3210609 A1 | 30 August 2017 |
| | | HRP 20171534 T1 | 26 January 2018 |
| | | HRP 20171534 T8 | 24 August 2018 |
| | | ES 2645689 T3 | 07 December 2017 |
| | | US 2017218000 A1 | 03 August 2017 |
| | | KR 20180014882 A | 09 February 2018 |
| | | KR 101956261 B1 | 08 March 2019 |
| | | EA 201071339 A1 | 30 June 2011 |
| | | EA 029131 B1 | 28 February 2018 |
| | | LT 2300013 T | 27 December 2017 |
| | | AU 2009248923 A1 | 26 November 2009 |
| | | AU 2009248923 B2 | 29 January 2015 |
| | | PT 2300013 T | 31 October 2017 |
| | | NL 300990 I1 | 29 May 2019 |
| | | NL 300990 I2 | 11 July 2019 |

Form PCT/ISA/210 (patent family annex) (July 2022)

**INTERNATIONAL SEARCH REPORT**
**Information on patent family members**

International application No.
**PCT/CN2022/138383**

| Patent document cited in search report | Publication date (day/month/year) | Patent family member(s) | Publication date (day/month/year) |
|---|---|---|---|
| | | JP 2018065864 A | 26 April 2018 |
| | | JP 6483233 B2 | 13 March 2019 |
| | | JP 2019094344 A | 20 June 2019 |
| | | JP 6690032 B2 | 28 April 2020 |
| | | HK 1158497 A1 | 20 July 2012 |
| | | PL 2300013 T3 | 30 May 2018 |
| | | DK 2300013 T3 | 04 December 2017 |
| | | KR 20110010801 A | 07 February 2011 |
| | | KR 101781605 B1 | 25 September 2017 |
| | | MX 2010012703 A | 21 December 2010 |
| | | JP 2015163621 A | 10 September 2015 |
| | | JP 6190415 B2 | 30 August 2017 |
| | | JP 2011523646 A | 18 August 2011 |
| CN 103641833 A | 19 March 2014 | NO 20160333 A1 | 29 February 2016 |
| | | ZA 200903601 B | 26 January 2011 |
| | | AU 2007333394 A1 | 19 June 2008 |
| | | AU 2007333394 B2 | 03 February 2011 |
| | | AU 2007333394 C1 | 18 August 2011 |
| | | JP 2012229240 A | 22 November 2012 |
| | | JP 5513558 B2 | 04 June 2014 |
| | | PT 2091918 E | 24 November 2014 |
| | | AU 2010210018 A1 | 02 September 2010 |
| | | AU 2010210018 B2 | 02 February 2012 |
| | | BRPI 0720264 A2 | 10 May 2011 |
| | | BRPI 0720264 B1 | 03 March 2022 |
| | | IL 198936 A0 | 17 February 2010 |
| | | NO 20092472 L | 02 September 2009 |
| | | NO 338069 B1 | 25 July 2016 |
| | | ME 00811 B | 20 March 2012 |
| | | TN 2009000225 A1 | 18 October 2010 |
| | | ECSP 099500 A | 28 August 2009 |
| | | DK 2091918 T3 | 01 December 2014 |
| | | PL 2091918 T3 | 27 February 2015 |
| | | LTC 2091918 I2 | 10 April 2017 |
| | | JP 2013144683 A | 25 July 2013 |
| | | HUS 1500049 I1 | 30 October 2017 |
| | | KR 20090087127 A | 14 August 2009 |
| | | KR 101149295 B1 | 05 July 2012 |
| | | JP 2010512329 A | 22 April 2010 |
| | | JP 5208123 B2 | 12 June 2013 |
| | | CA 2671744 A1 | 19 June 2008 |
| | | CA 2671744 C | 28 August 2012 |
| | | EP 2091918 A2 | 26 August 2009 |
| | | EP 2091918 B1 | 27 August 2014 |
| | | CO 6231028 A2 | 20 December 2010 |
| | | GT 200900147 A | 27 January 2010 |
| | | SMAP 200900058 A | 19 January 2010 |
| | | SMP 200900058 B | 12 November 2010 |
| | | CR 10832 A | 14 July 2009 |
| | | AU 2010210019 A1 | 02 September 2010 |

Form PCT/ISA/210 (patent family annex) (July 2022)

**INTERNATIONAL SEARCH REPORT**
**Information on patent family members**

International application No.
**PCT/CN2022/138383**

| Patent document cited in search report | Publication date (day/month/year) | Patent family member(s) | Publication date (day/month/year) |
|---|---|---|---|
| | | AU 2010210019 B2 | 02 August 2012 |
| | | AU 2010210019 C1 | 14 February 2013 |
| | | EA 200970557 A1 | 30 December 2009 |
| | | EA 017405 B1 | 28 December 2012 |
| | | EA 017405 B9 | 30 May 2014 |
| | | NO 2016014 I1 | |
| | | NO 2016014 I2 | |
| | | NL 300763 I2 | |
| | | NL 300763 I1 | |
| | | MA 30923 B1 | 02 November 2009 |
| | | SV 2009003290 A | 17 August 2010 |
| | | WO 2008073687 A2 | 19 June 2008 |
| | | WO 2008073687 A3 | 31 July 2008 |
| | | MX 2009006081 A | 17 June 2009 |
| CN 101921236 A | 22 December 2010 | KR 20050109987 A | 22 November 2005 |
| | | KR 101148261 B1 | 21 May 2012 |
| | | CY 1114238 T1 | 31 August 2016 |
| | | WO 2004080980 A1 | 23 September 2004 |
| | | NO 20054726 D0 | 13 October 2005 |
| | | NO 20054726 L | 08 December 2005 |
| | | NO 331977 B1 | 14 May 2012 |
| | | IS 8062 A | 06 October 2005 |
| | | IS 2961 B | 15 February 2017 |
| | | EP 1606265 A1 | 21 December 2005 |
| | | EP 1606265 B1 | 02 February 2011 |
| | | PL 1606265 T3 | 29 July 2011 |
| | | DK 2275413 T3 | 03 June 2013 |
| | | NZ 542219 A | 31 January 2009 |
| | | PL 2275413 T3 | 30 August 2013 |
| | | MA 27668 A1 | 01 December 2005 |
| | | SI 2275413 T1 | 31 July 2013 |
| | | ES 2409885 T3 | 28 June 2013 |
| | | PT 1606265 E | 05 May 2011 |
| | | PT 2275413 E | 21 June 2013 |
| | | DE 602004031287 D1 | 17 March 2011 |
| | | US 2006247241 A1 | 02 November 2006 |
| | | US 7964592 B2 | 21 June 2011 |
| | | BRPI 0408347 A | 21 March 2006 |
| | | BRPI 0408347 B1 | 06 August 2019 |
| | | BRPI 0408347 B8 | 25 May 2021 |
| | | ECSP 056019 A | 27 January 2006 |
| | | TNSN 05225 A1 | 11 June 2007 |
| | | BR 122019010008 B1 | 17 September 2019 |
| | | BR 122019010008 B8 | 27 July 2021 |
| | | US 2011201606 A1 | 18 August 2011 |
| | | US 8263590 B2 | 11 September 2012 |
| | | IL 170546 A | 31 December 2013 |
| | | GB 0305929 D0 | 23 April 2003 |
| | | MXPA 05009883 A | 05 December 2005 |
| | | AT 497495 T | 15 February 2011 |

Form PCT/ISA/210 (patent family annex) (July 2022)

**INTERNATIONAL SEARCH REPORT**
**Information on patent family members**

International application No.
**PCT/CN2022/138383**

| Patent document cited in search report | Publication date (day/month/year) | Patent family member(s) | Publication date (day/month/year) |
|---|---|---|---|
| | | EP 2275413 A1 | 19 January 2011 |
| | | EP 2275413 B1 | 06 March 2013 |
| | | AU 2004220338 A1 | 23 September 2004 |
| | | AU 2004220338 B2 | 09 October 2008 |
| | | JP 2006520354 A | 07 September 2006 |
| | | JP 4796487 B2 | 19 October 2011 |
| | | CA 2518932 A1 | 23 September 2004 |
| | | CA 2518932 C | 04 December 2012 |
| CN 102482277 A | 30 May 2012 | WO 2010129053 A2 | 11 November 2010 |
| | | WO 2010129053 A3 | 24 March 2011 |
| | | WO 2010129053 A8 | 15 December 2011 |
| | | KR 20120047208 A | 11 May 2012 |
| | | KR 101705158 B1 | 09 February 2017 |
| | | IL 216140 A0 | 31 January 2012 |
| | | IL 216140 B | 29 March 2018 |
| | | JP 2012526113 A | 25 October 2012 |
| | | JP 5918693 B2 | 18 May 2016 |
| | | ES 2659725 T3 | 19 March 2018 |
| | | EP 2440559 A2 | 18 April 2012 |
| | | EP 2440559 A4 | 21 November 2012 |
| | | EP 2440559 B1 | 10 January 2018 |
| | | US 2012094999 A1 | 19 April 2012 |
| | | US 9908884 B2 | 06 March 2018 |
| | | CA 2760794 A1 | 11 November 2010 |
| | | CA 2760794 C | 25 July 2017 |
| | | US 2019040065 A1 | 07 February 2019 |
| CN 111683662 A | 18 September 2020 | EP 3675860 A1 | 08 July 2020 |
| | | EP 3675860 A4 | 14 April 2021 |
| | | US 2020190068 A1 | 18 June 2020 |
| | | US 11440904 B2 | 13 September 2022 |
| | | WO 2019046163 A1 | 07 March 2019 |
| CN 1335838 A | 13 February 2002 | US 6593326 B1 | 15 July 2003 |
| | | PT 1140860 E | 31 January 2005 |
| | | AU 1874300 A | 31 July 2000 |
| | | AU 763091 B2 | 10 July 2003 |
| | | GB 9828511 D0 | 17 February 1999 |
| | | IL 143806 A0 | 21 April 2002 |
| | | ES 2228145 T3 | 01 April 2005 |
| | | KR 20010099900 A | 09 November 2001 |
| | | KR 100663773 B1 | 03 January 2007 |
| | | EP 1140860 A1 | 10 October 2001 |
| | | EP 1140860 B1 | 22 September 2004 |
| | | ZA 200104413 B | 29 August 2002 |
| | | DK 1140860 T3 | 10 January 2005 |
| | | NO 20013038 D0 | 19 June 2001 |
| | | NO 20013038 L | 22 August 2001 |
| | | NO 319815 B1 | 19 September 2005 |
| | | BR 9916590 A | 23 October 2001 |
| | | IL 143806 A | 15 June 2009 |
| | | AT 277020 T | 15 October 2004 |

Form PCT/ISA/210 (patent family annex) (July 2022)

**INTERNATIONAL SEARCH REPORT**
**Information on patent family members**

International application No.
**PCT/CN2022/138383**

| Patent document cited in search report | Publication date (day/month/year) | Patent family member(s) | | | Publication date (day/month/year) |
|---|---|---|---|---|---|
| | | CA | 2352896 | A1 | 06 July 2000 |
| | | DE | 69920509 | D1 | 28 October 2004 |
| | | DE | 69920509 | T2 | 09 March 2006 |
| | | WO | 0039101 | A1 | 06 July 2000 |
| | | NZ | 512118 | A | 29 August 2003 |
| | | JP | 2002533446 | A | 08 October 2002 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202111522765


### Non-patent literature cited in the description


- **JERRY MARCH.** Advanced Organic Chemistry. WileyInterscience
- **L. W. DEADY.** *Syn. Comm.,* 1977, 7509-514
- **S. M. BERGE et al.** *J. Pharmaceutical Sciences,* 1977, vol. 66, 1-19
- Pro-drugs as Novel Delivery Systems. **T. HIGUCHI ; V STELLA.** A. C. S. Symposium Series. vol. 14
- Bioreversible Carriers in Drug Design. American Pharmaceutical Association and Pergamon Press, 1987
- **J. RAUTIO et al.** Prodrugs: Design and Clinical Applications. *Nature Review Drug Discovery,* 2008, vol. 7, 255-270
- **S. J. HECKER et al.** Prodrugs of Phosphates and Phosphonates. *Journal of Medicinal Chemistry,* 2008, vol. 51, 2328-2345
- Remington''s Pharmaceutical Sciences. Mack Publishing Company
- The Handbook of Pharmaceutical Additives. Gower Publishing Limited
- The Handbook of Pharmaceutical Excipients. the American Pharmaceutical Association and the Pharmaceutical Press
- Remington: The Science and Practice of Pharmacy. Lippincott Williams & Wilkins, 2005
- Encyclopedia of Pharmaceutical Technology. Marcel Dekker, 1988
- Inactive Ingredient Guide. Center for Drug Evaluation and Research (CEDR) in the U.S. Food and Drug Administration, 1996
- Handbook of Pharmaceutical Additives. Synapse Information Resources, Inc, 2002
- *Pharmaceutical Research,* 1986, vol. 3 (6), 318